(19) Europäisches Patentamt
European Patent Office
Office européen des brevets

(11) EP 4 371 979 A1

# (12) EUROPEAN PATENT APPLICATION

published in accordance with Art. 153(4) EPC

(43) Date of publication:
**22.05.2024 Bulletin 2024/21**

(21) Application number: **22842196.2**

(22) Date of filing: **15.07.2022**

(51) International Patent Classification (IPC):
*C07D 271/06* (2006.01) *A01N 43/836* (2006.01) *A01P 3/00* (2006.01) *C07C 259/18* (2006.01) *C07D 413/04* (2006.01) *C07D 413/12* (2006.01)

(52) Cooperative Patent Classification (CPC):
**A01N 43/82; A01P 3/00; C07C 259/18; C07D 271/06; C07D 413/04; C07D 413/12**

(86) International application number:
**PCT/JP2022/027824**

(87) International publication number:
**WO 2023/286855 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.07.2021 JP 2021117258**

(71) Applicant: **Kumiai Chemical Industry Co., Ltd.**
**Taito-ku**
**Tokyo 110-8782 (JP)**

(72) Inventors:
- **TANAKA, Kosuke**
  **Tokyo 110-8782 (JP)**
- **HORINOUCHI, Kento**
  **Tokyo 110-8782 (JP)**
- **OE, Takuto**
  **Tokyo 110-8782 (JP)**
- **YOKOCHI, Shunsuke**
  **Tokyo 110-8782 (JP)**
- **KANEKO, Isao**
  **Tokyo 110-8782 (JP)**

(74) Representative: **Kraus & Lederer PartGmbB**
**Thomas-Wimmer-Ring 15**
**80539 München (DE)**

# (54) FORMAMIDE DERIVATIVE AND AGRICULTURAL OR HORTICULTURAL PLANT DISEASE CONTROL AGENT

(57) The purpose of the present invention is to provide an excellent agricultural or horticultural plant disease control agent. Provided are: a formamide derivative represented by general formula [I] and the like (in the formula, $X^1$, $X^2$, $X^3$, and $X^4$ each independently represents a carbon atom and the like, Y represents an oxygen atom or a sulfur atom, $R^1$ represents a halogen atom, and $R^2$ represents a hydrogen atom, a $C_1$-$C_6$ alkyl group, and the like) or an agriculturally acceptable salt thereof; and an agricultural or horticultural plant disease control agent containing the same as an active ingredient.

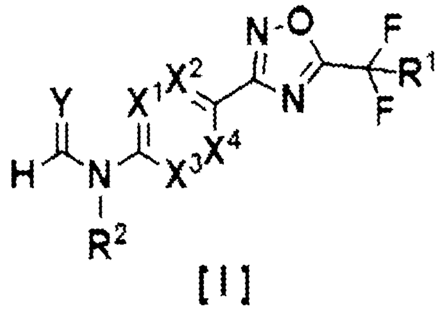

[I]

EP 4 371 979 A1

## Description

Technical Field

The present invention relates to a novel formamide derivative or an agriculturally acceptable salt thereof, an agricultural and horticultural plant disease control agent containing the derivative or the salt thereof as an active ingredient, and a production intermediate of the derivative or the salt thereof.

Background Art

Patent Document 1 describes an amide derivative containing aryl oxadiazole.
Patent Documents 2 to 6 describe an amide derivative containing aryl oxadiazole and heteroaryl oxadiazole.
Patent Documents 7 and 8 describe an aniline derivative containing aryl oxadiazole and heteroaryl oxadiazole.
Patent Document 9 describes a di-substituted amide derivative containing aryl oxadiazole.
In the patent documents described above, a formamide derivative of the present invention is not disclosed at all.

Citation List

Patent Document

Patent Document 1: JP 63-162680 A
Patent Document 2: WO 2017/081309 A
Patent Document 3: WO 2017/081310 A
Patent Document 4: WO 2009/019656 A
Patent Document 5: WO 2013/080120 A
Patent Document 6: WO 2017/222951 A
Patent Document 7: WO 2020/070610 A
Patent Document 8: WO 2020/254494 A
Patent Document 9: WO 2017/110862 A

Summary of the Invention

Problems to be Solved by the Invention

In cultivation of agricultural and horticultural crops, a large number of control agents are used for diseases of crops. However, there are few control agents according to the background art which fully satisfy the requirements for use as a control agent from the viewpoint of an insufficient control effect thereof, limited use thereof due to the emergence of agent-resistant pathogens, phytotoxic or contaminating effects on plants, or toxicity to humans, domestic animals, and fish, and an adverse effect on the environment. Therefore, there is a strong demand for the development of a control agent that has few such defects and can be safely used.

Means for Solving the Problems

As a result of conducting intensive studies, the present inventors have found that an agent containing a formamide derivative of formula [I] or an agriculturally acceptable salt thereof as an active ingredient has a high control effect on diseases of useful crops, thereby completing the present invention.
That is, the gist of the present invention is as follows.

(1) A formamide derivative of formula [I] or an agriculturally acceptable salt thereof,

[Chem. 1]

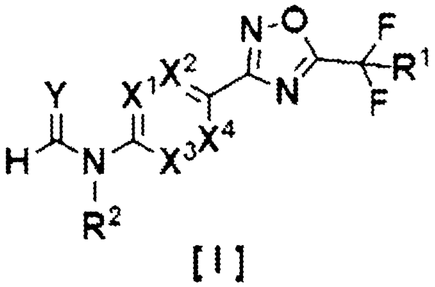

[I]

wherein in the formula [I],

$X^1$, $X^2$, $X^3$, and $X^4$ each independently represent a carbon atom (the carbon atom is optionally substituted with $R^3$) or a nitrogen atom,

Y represents an oxygen atom or a sulfur atom,

$R^1$ represents a hydrogen atom or a halogen atom,

$R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C3-C6)cycloalkyl (the (C3-C6)cycloalkyl is optionally mono-substituted or poly-substituted with $R^4$), a (C6-C12)aryl (the (C6-C12)aryl is optionally mono-substituted or poly-substituted with $R^5$), a 3-to 6-membered heterocyclyl (the 3- to 6-membered heterocyclyl is optionally mono-substituted or poly-substituted with $R^5$), or a 5- to 12-membered heteroaryl (the 5- to 12-membered heteroaryl is optionally mono-substituted or poly-substituted with $R^5$),

$R^3$ each independently represent a hydrogen atom, a halogen atom, or a (C1-C6)alkyl,

$R^4$ each independently represent a hydrogen atom, a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alky-

nylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$),

a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5- to 12-membered heteroaryloxy (the 5- to 12-membered heteroaryloxy is optionally mono-substituted or poly-substituted with $R^5$), a 5-to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3-to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, an amino, a hydroxy, a thiol, a formyl, an oxo, a (C1-C6)alkyl, a (C2-C6)alkenyl, a (C2-C6)alkynyl, a (C3-C6)cycloalkyl, a (C1-C6)haloalkyl, a (C1-C6)alkoxy, a (C1-C6)haloalkoxy, a (C1-C6)alkoxy (C1-C6)alkyl, a (C1-C6)haloalkoxy (C1-C6)alkyl, a cyano (C1-C6)alkyl, a cyano (C3-C6)cycloalkyl, a (C1-C6)alkylthio, a (C1-C6)haloalkylthio, a (C1-C6)alkylsulfinyl, a (C1-C6)haloalkylsulfinyl, a (C1-C6)alkylsulfonyl, a (C1-C6)haloalkylsulfonyl, a (C1-C6)alkylcarbonylthio, a (C1-C6)alkylamino (the amino moiety is optionally substituted with $R^6$), a (C1-C6)haloalkylamino (the amino moiety is optionally substituted with $R^6$), a formylamino (the amino moiety is optionally substituted with $R^6$), a (C1-C6)alkylcarbonylamino (the amino moiety is optionally substituted with $R^6$), a carbamoyl, a (C1-C6)alkylaminocarbonyl (the amino moiety is optionally substituted with $R^6$), a carboxyl, a (C1-C6)alkylcarbonyl, a (C1-C6)haloalkylcarbonyl, a (C1-C6)alkoxycarbonyl, a hydroxyimino (C1-C6)alkyl, a (C1-C6)alkoxyimino (C1-C6)alkyl, a (C6-C12)aryl (the (C6-C12)aryl is optionally mono-substituted or poly-substituted with $R^7$), a (C6-C12)aryl (C1-C6)alkyl (the (C6-C12)aryl moiety is optionally mono-substituted or poly-substituted with $R^7$), a (C6-C12)arylcarbonyl (the (C6-C12)arylcarbonyl is optionally mono-substituted or poly-substituted with $R^7$), a (C6-C12)aryloxy (the (C6-C12)aryloxy is optionally mono-substituted or poly-substituted with $R^7$), a (C6-C12)arylthio (the (C6-C12)arylthio is optionally mono-substituted or poly-substituted with $R^7$), a (C6-C12)arylsulfinyl (the (C6-C12)arylsulfinyl is optionally mono-substituted or poly-substituted with $R^7$), a (C6-C12)arylsulfonyl (the (C6-C12)arylsulfonyl is optionally mono-substituted or poly-substituted with $R^7$), a (C6-C12)arylamino (the (C6-C12)aryl moiety is optionally mono-substituted or poly-substituted with $R^7$, and the amino moiety is optionally substituted with $R^6$), a 3- to 6-membered heterocyclyl (the 3- to 6-membered heterocyclyl is optionally mono-substituted or poly-substituted with $R^7$), a 3- to 6-membered heterocyclyl (C1-C6)alkyl (the 3- to 6-membered heterocyclyl moiety is optionally mono-substituted or poly-substituted with $R^7$), a 3-to 6-membered heterocyclylcarbonyl (the 3- to 6-membered heterocyclylcarbonyl is optionally mono-substituted or poly-substituted

with $R^7$), a 3- to 6-membered heterocyclyloxy (the 3- to 6-membered heterocyclyloxy is optionally mono-substituted or poly-substituted with $R^7$), a 3- to 6-membered heterocyclylthio (the 3- to 6-membered heterocyclylthio is optionally mono-substituted or poly-substituted with $R^7$), a 3-to 6-membered heterocyclylsulfinyl (the 3- to 6-membered heterocyclylsulfinyl is optionally mono-substituted or poly-substituted with $R^7$), 3- to 6-membered heterocyclylsulfonyl (the 3- to 6-membered heterocyclylsulfonyl is optionally mono-substituted or poly-substituted with $R^7$), a 3- to 6-membered heterocyclylamino (the 3- to 6-membered heterocyclyl moiety is optionally mono-substituted or poly-substituted with $R^7$, and the amino moiety is optionally substituted with $R^6$), a 5- to 12-membered heteroaryl (the 5- to 12-membered heteroaryl is optionally mono-substituted or poly-substituted with $R^7$), a 5-to 12-membered heteroaryl (C1-C6)alkyl (the 5- to 12-membered heteroaryl moiety is optionally mono-substituted or poly-substituted with $R^7$), a 5- to 12-membered heteroarylcarbonyl (the 5- to 12-membered heteroarylcarbonyl is optionally mono-substituted or poly-substituted with $R^7$), a 5- to 12-membered heteroaryloxy (the 5- to 12-membered heteroaryloxy is optionally mono-substituted or poly-substituted with $R^7$), a 5-to 12-membered heteroarylthio (the 5- to 12-membered heteroarylthio is optionally mono-substituted or poly-substituted with $R^7$), a 5- to 12-membered heteroarylsulfinyl (the 5- to 12-membered heteroarylsulfinyl is optionally mono-substituted or poly-substituted with $R^7$), a 5- to 12-membered heteroarylsulfonyl (the 5- to 12-membered heteroarylsulfonyl is optionally mono-substituted or poly-substituted with $R^7$), or a 5- to 12-membered heteroarylamino (the 5- to 12-membered heteroaryl moiety is optionally mono-substituted or poly-substituted with $R^7$, and the amino moiety is optionally substituted with $R^6$),

$R^6$ each independently represent a hydrogen atom, a (C1-C6)alkyl, a (C1-C6)haloalkyl, a (C3-C6)cycloalkyl, a (C1-C6)alkoxy (C1-C6)alkyl, a (C1-C6)haloalkoxy (C1-C6)alkyl, a cyano (C1-C6)alkyl, a (C1-C6)alkylcarbonyl, a (C1-C6)haloalkylcarbonyl, a (C1-C6)alkoxycarbonyl, or a (C6-C12)arylcarbonyl (the (C6-C12)arylcarbonyl is optionally mono-substituted or poly-substituted with $R^7$),

$R^7$ each independently represent a hydrogen atom, a halogen atom, a cyano, a (C1-C6)alkyl, or a (C1-C6)alkoxy, and

$R^8$ each independently represent a halogen atom, a cyano, a (C1-C6)haloalkyl, a (C3-C6)cycloalkyl, a (C1-C6)alkoxy, a (C1-C6)alkylcarbonyl, a (C1-C6)alkoxycarbonyl, a (C1-C6)haloalkoxycarbonyl, a (C6-C12)aryl (the (C6-C12)aryl is optionally mono-substituted or poly-substituted with $R^7$), a 3-to 6-membered heterocyclyl (the 3- to 6-membered heterocyclyl is optionally mono-substituted or poly-substituted with $R^7$), or a 5- to 12-membered heteroaryl (the 5- to 12-membered heteroaryl is optionally mono-substituted or poly-substituted with $R^7$).

(2) The formamide derivative of formula [I] or the agriculturally acceptable salt thereof according to (1), wherein in the formula [I], $R^2$ represents a (C1-C6)alkyl (the (C1-C6)alkyl is optionally mono-substituted or poly-substituted with $R^4$), a (C3-C6)cycloalkyl (the (C3-C6)cycloalkyl is optionally mono-substituted or poly-substituted with $R^4$), or a 3- to 6-membered heterocyclyl (the 3- to 6-membered heterocyclyl is optionally mono-substituted or poly-substituted with $R^5$), and

$R^4$ each independently represent a (C3-C6)cycloalkyl, a (C3-C6)halocycloalkyl, a (C6-C12)aryl (the (C6-C12)aryl is optionally mono-substituted or poly-substituted with $R^5$), a 3-to 6-membered heterocyclyl (the 3- to 6-membered heterocyclyl is optionally mono-substituted or poly-substituted with $R^5$), a 5- to 12-membered heteroaryl (the 5- to 12-membered heteroaryl is optionally mono-substituted or poly-substituted with $R^5$), or a 1,3-benzodioxole (the 1,3-benzodioxole is optionally mono-substituted or poly-substituted with $R^5$).

(3) The formamide derivative of formula [I] or the agriculturally acceptable salt thereof according to (2), wherein in the formula [I], $R^2$ represents a (C1-C6)alkyl (the (C1-C6)alkyl is optionally mono-substituted or poly-substituted with $R^4$), and

$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl.

(4) The formamide derivative of formula [I] or the agriculturally acceptable salt thereof according to (1), wherein in the formula [I], $R^4$ each independently represent a halogen atom, a cyano, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted

or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally

mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

(5) The formamide derivative of formula [I] or the agriculturally acceptable salt thereof according to (4), wherein in the formula [I], $R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C6-C12)aryl (the (C6-C12)aryl is optionally mono-substituted or poly-substituted with $R^5$), or a 3- to 6-membered heterocyclyl (the 3- to 6-membered heterocyclyl is optionally mono-substituted or poly-substituted with $R^5$),

$R^4$ each independently represent a halogen atom, a cyano, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl, and

$R^6$ each independently represent a hydrogen atom, a (C1-C6)alkyl, a (C1-C6)haloalkylcarbonyl, or a (C1-C6)alkoxycarbonyl.

(6) The formamide derivative of formula [I] or the agriculturally acceptable salt thereof according to (2) or (3), wherein in the formula [I], $X^1$, $X^2$, $X^3$, and $X^4$ each independently represent a carbon atom (the carbon atom is optionally substituted with $R^3$), and $R^1$ is a halogen atom.

(7) The formamide derivative of formula [I} or the agriculturally acceptable salt thereof according to (2) or (3), wherein in the formula [I], $R^3$ is a hydrogen atom.

(8) The formamide derivative of formula[I] or the agriculturally acceptable salt thereof according to (2) or (3), wherein in the formula [I], $R^2$ represents a (C1-C6)alkyl mono-substituted or poly-substituted with substituent(s) selected from a substituent group $R^4$.

(9) The formamide derivative of formula [I] or the agriculturally acceptable salt thereof according to (4) or (5), wherein in the formula [I], $X^1$, $X^2$, $X^3$, and $X^4$ each independently represent a carbon atom (the carbon atom is optionally substituted with $R^3$), and $R^1$ is a halogen atom.

(10) The formamide derivative of formula [I] or the agriculturally acceptable salt thereof according to (4) or (5), wherein in the formula [I], $R^3$ is a hydrogen atom.

(11) The formamide derivative of formula [I] or the agriculturally acceptable salt thereof according to (4), wherein in

the formula [I], $R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C3-C6)cycloalkyl (the (C3-C6)cycloalkyl is optionally mono-substituted or poly-substituted with $R^4$), a (C6-C12)aryl (the (C6-C12)aryl is optionally mono-substituted or poly-substituted with $R^5$), or a 5- to 12-membered heteroaryl (the 5- to 12-membered heteroaryl is optionally mono-substituted or poly-substituted with $R^5$).

(12) The formamide derivative of formula [I] or the agriculturally acceptable salt thereof according to (4), wherein in the formula [I], $R^2$ represents a hydrogen atom or a (C1-C6)alkyl mono-substituted or poly-substituted with substituent(s) selected from a substituent group $R^4$.

(13) An agricultural and horticultural plant disease control agent containing the formamide derivative of formula [I] or the agriculturally acceptable salt thereof according to any one of (1) to (12) as an active ingredient.

(14) The agricultural and horticultural plant disease control agent according to (13), in which phytopathogenic fungi are basidiomycetes, ascomycetes, deuteromycetes, oomycetes, and/or zygomycetes.

(15) An agricultural and horticultural plant disease control agent composition containing the formamide derivative of formula [I] or the agriculturally acceptable salt thereof according to any one of (1) to (12), and an agriculturally acceptable carrier.

(16) A method for controlling a disease of a useful plant, comprising a step of spraying an effective amount of the formamide derivative of formula [I] or the agriculturally acceptable salt thereof according to any one of (1) to (12) on the useful plant, a part of the useful plant, or a place where the useful plant grows.

(17) The method according to (16), in which phytopathogenic fungi are basidiomycetes, ascomycetes, deuteromycetes, oomycetes, and/or zygomycetes.

(18) A use of the formamide derivative of formula [I] or the agriculturally acceptable salt thereof according to any one of (1) to (12) as an agricultural and horticultural plant disease control agent for a useful plant.

(19) An aniline derivative of formula [II] or an agriculturally acceptable salt thereof,

[Chem. 2]

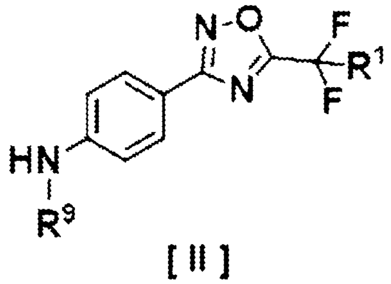

[II]

wherein in the formula [II],

$R^1$ represents a hydrogen atom or a halogen atom,

$R^9$ represents a (C3-C6)alkyl, a (C1-C6)alkyl mono-substituted or poly-substituted with substituent(s) selected from a substituent group $R^{10}$, a (C2-C6)alkenyl mono-substituted or poly-substituted with substituent(s) selected from a substituent group $R^{11}$, or a (C2-C6)alkynyl mono-substituted or poly-substituted with substituent(s) selected from a substituent group $R^{12}$,

$R^{10}$ and $R^{12}$ each independently represent a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or

poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^{11}$ each independently represent a halogen atom, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl,

a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted

with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the

3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, an amino, a hydroxy, a thiol, a formyl, an oxo, a (C1-C6)alkyl, a (C2-C6)alkenyl, a (C2-C6)alkynyl, a (C3-C6)cycloalkyl, a (C1-C6)haloalkyl, a (C1-C6)alkoxy, a (C1-C6)haloalkoxy, a (C1-C6)alkoxy (C1-C6)alkyl, a (C1-C6)haloalkoxy (C1-C6)alkyl, a cyano (C1-C6)alkyl, a cyano (C3-C6)cycloalkyl, a (C1-C6)alkylthio, a (C1-C6)haloalkylthio, a (C1-C6)alkylsulfinyl, a (C1-C6)haloalkylsulfinyl, a (C1-C6)alkylsulfonyl, a (C1-C6)haloalkylsulfonyl, a (C1-C6)alkylcarbonylthio, a (C1-C6)alkylamino (the amino moiety is optionally substituted with $R^6$), a (C1-C6)haloalkylamino (the amino moiety is optionally substituted with $R^6$), a formylamino (the amino moiety is optionally substituted with $R^6$), a (C1-C6)alkylcarbonylamino (the amino moiety is optionally substituted with $R^6$), a carbamoyl, a (C1-C6)alkylaminocarbonyl (the amino moiety is optionally substituted with $R^6$), a carboxyl, a (C1-C6)alkylcarbonyl, a (C1-C6)haloalkylcarbonyl, a (C1-C6)alkoxycarbonyl, a hydroxyimino (C1-C6)alkyl, a (C1-C6)alkoxyimino (C1-C6)alkyl, a (C6-C12)aryl (the (C6-C12)aryl is optionally mono-substituted or poly-substituted with $R^7$), a (C6-C12)aryl (C1-C6)alkyl (the (C6-C12)aryl moiety is optionally mono-substituted or poly-substituted with $R^7$), a (C6-C12)arylcarbonyl (the (C6-C12)arylcarbonyl is optionally mono-substituted or poly-substituted with $R^7$), a (C6-C12)aryloxy (the (C6-C12)aryloxy is optionally mono-substituted or poly-substituted with $R^7$), a (C6-C12)arylthio (the (C6-C12)arylthio is optionally mono-substituted or poly-substituted with $R^7$), a (C6-C12)arylsulfinyl (the (C6-C12)arylsulfinyl is optionally mono-substituted or poly-substituted with $R^7$), a (C6-C12)arylsulfonyl (the (C6-C12)arylsulfonyl is optionally mono-substituted or poly-substituted with $R^7$), a (C6-C12)arylamino (the (C6-C12)aryl moiety is optionally mono-substituted or poly-substituted with $R^7$, and the amino moiety is optionally substituted with $R^6$), a 3- to 6-membered heterocyclyl (the 3- to 6-membered heterocyclyl is optionally mono-substituted or poly-substituted with $R^7$), a 3- to 6-membered heterocyclyl (C1-C6)alkyl (the 3- to 6-membered heterocyclyl moiety is optionally mono-substituted or poly-substituted with $R^7$), a 3-to 6-membered heterocyclylcarbonyl (the 3- to 6-membered heterocyclylcarbonyl is optionally mono-substituted or poly-substituted with $R^7$), a 3- to 6-membered heterocyclyloxy (the 3- to 6-membered heterocyclyloxy is optionally mono-substituted or poly-substituted with $R^7$), a 3- to 6-membered heterocyclylthio (the 3- to 6-membered heterocyclylthio is optionally mono-substituted or poly-substituted with $R^7$), a 3-to 6-membered heterocyclylsulfinyl (the 3- to 6-membered heterocyclylsulfinyl is optionally mono-substituted or poly-substituted with $R^7$), 3- to 6-membered heterocyclylsulfonyl (the 3- to 6-membered heterocyclylsulfonyl is optionally mono-substituted or poly-substituted with $R^7$), a 3- to 6-membered heterocyclylamino (the 3- to 6-membered heterocyclyl moiety is optionally mono-substituted or poly-substituted with $R^7$, and the amino moiety is optionally substituted with $R^6$), a 5- to 12-membered heteroaryl (the 5- to 12-membered heteroaryl is optionally mono-substituted or poly-substituted with $R^7$), a 5-to 12-membered heteroaryl (C1-C6)alkyl (the 5- to 12-membered heteroaryl moiety is optionally mono-substituted or poly-substituted with $R^7$), a 5- to 12-membered heteroarylcarbonyl (the 5- to 12-membered heteroarylcarbonyl is optionally mono-substituted or poly-substituted with $R^7$), a 5- to 12-membered heteroaryloxy (the 5- to 12-membered heteroaryloxy is optionally mono-substituted or poly-substituted with $R^7$), a 5-to 12-membered heteroarylthio (the 5- to 12-membered heteroarylthio is optionally mono-substituted or poly-substituted with $R^7$), a 5- to 12-membered heteroarylsulfinyl (the 5- to 12-membered heteroarylsulfinyl is optionally mono-substituted or poly-substituted with $R^7$), a 5- to 12-membered heteroarylsulfonyl (the 5- to 12-membered heteroarylsulfonyl is optionally mono-substituted or poly-substituted with $R^7$), or a 5- to 12-membered heteroarylamino (the 5- to 12-membered heteroaryl moiety is optionally mono-substituted or poly-substituted with $R^7$, and the amino moiety is optionally substituted with $R^6$),

$R^6$ each independently represent a hydrogen atom, a (C1-C6)alkyl, a (C1-C6)haloalkyl, a (C3-C6)cycloalkyl, a (C1-C6)alkoxy (C1-C6)alkyl, a (C1-C6)haloalkoxy (C1-C6)alkyl, a cyano (C1-C6)alkyl, a (C1-C6)alkylcarbonyl, a (C1-C6)haloalkylcarbonyl, a (C1-C6)alkoxycarbonyl, or a (C6-C12)arylcarbonyl (the (C6-C12)arylcarbonyl is optionally mono-substituted or poly-substituted with $R^7$),

$R^7$ each independently represent a hydrogen atom, a halogen atom, a cyano, a (C1-C6)alkyl, or a (C1-C6)alkoxy, and

$R^8$ each independently represent a halogen atom, a cyano, a (C1-C6)haloalkyl, a (C3-C6)cycloalkyl, a (C1-C6)alkoxy, a (C1-C6)alkylcarbonyl, a (C1-C6)alkoxycarbonyl, a (C1-C6)haloalkoxycarbonyl, a (C6-C12)aryl (the (C6-C12)aryl is optionally mono-substituted or poly-substituted with $R^7$), a 3-to 6-membered heterocyclyl (the 3- to 6-membered heterocyclyl is optionally mono-substituted or poly-substituted with $R^7$), or a 5- to 12-membered heteroaryl (the 5- to 12-membered heteroaryl is optionally mono-substituted or poly-substituted with $R^7$).

(20) The aniline derivative of formula [II] or the agriculturally acceptable salt thereof according to (19), wherein in the formula [II], $R^9$ represents a (C1-C6)alkyl mono-substituted or poly-substituted with substituent(s) selected from a substituent group $R^{10}$, and

$R^{10}$ each independently represent a (C3-C6)cycloalkyl, a (C3-C6)halocycloalkyl, a (C6-C12)aryl (the (C6-C12)aryl is optionally mono-substituted or poly-substituted with $R^5$), a 3-to 6-membered heterocyclyl (the 3- to 6-membered heterocyclyl is optionally mono-substituted or poly-substituted with $R^5$), a 5- to 12-membered heteroaryl (the 5- to 12-membered heteroaryl is optionally mono-substituted or poly-substituted with $R^5$), or a 1,3-benzodioxole (the 1,3-benzodioxole is optionally mono-substituted or poly-substituted with $R^5$).

(21) The aniline derivative of formula [II] or the agriculturally acceptable salt thereof according to (20), wherein in the formula [II], $R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl.

(22) The aniline derivative of formula [II] or the agriculturally acceptable salt thereof according to (19), wherein in the formula [II], $R^{10}$ and $R^{12}$ each independently represent a halogen atom, a cyano, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a

(C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to

6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^{11}$ each independently represent a halogen atom, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to

12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally

mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

(23) The aniline derivative of formula [II] or the agriculturally acceptable salt thereof according to (22), wherein in the formula [II], $R^{10}$ and $R^{12}$ each independently represent a halogen atom, a cyano, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with $R^6$), or a pentafluorosulfanyl,

$R^{11}$ each independently represent a halogen atom, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally

mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl, and

$R^6$ each independently represent a hydrogen atom, a (C1-C6)alkyl, a (C1-C6)haloalkylcarbonyl, or a (C1-C6)alkoxycarbonyl.

(24) An amidoxime derivative of formula [III] or an agriculturally acceptable salt thereof,

[Chem. 3]

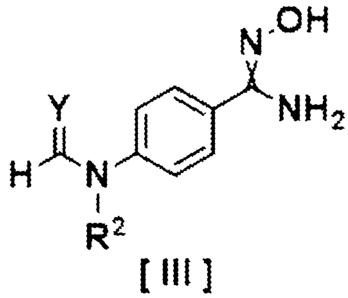

[III]

wherein in the formula [III],

Y represents an oxygen atom or a sulfur atom,

$R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C3-C6)cycloalkyl (the (C3-C6)cycloalkyl is optionally mono-substituted or poly-substituted with $R^4$), a (C6-C12)aryl (the (C6-C12)aryl is optionally mono-substituted or poly-substituted with $R^5$), a 3-to 6-membered heterocyclyl (the 3- to 6-membered heterocyclyl is optionally mono-substituted or poly-substituted with $R^5$), or a 5- to 12-membered heteroaryl (the 5- to 12-membered heteroaryl is optionally mono-substituted or poly-substituted with $R^5$),

$R^4$ each independently represent a hydrogen atom, a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is

optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, an amino, a hydroxy, a thiol, a formyl, an oxo, a (C1-C6)alkyl, a (C2-C6)alkenyl, a (C2-C6)alkynyl, a (C3-C6)cycloalkyl, a (C1-C6)haloalkyl, a (C1-C6)alkoxy, a (C1-C6)haloalkoxy, a (C1-C6)alkoxy (C1-C6)alkyl, a (C1-C6)haloalkoxy (C1-C6)alkyl, a cyano (C1-C6)alkyl, a cyano (C3-C6)cycloalkyl, a (C1-C6)alkylthio, a (C1-C6)haloalkylthio, a (C1-C6)alkylsulfinyl, a (C1-C6)haloalkylsulfinyl, a (C1-C6)alkylsulfonyl, a (C1-C6)haloalkylsulfonyl, a (C1-C6)alkylcarbonylthio, a (C1-C6)alkylamino (the amino moiety is optionally substituted with $R^6$), a (C1-C6)haloalkylamino (the amino moiety is optionally substituted with $R^6$), a formylamino (the amino moiety is optionally substituted with $R^6$), a (C1-C6)alkylcarbonylamino (the amino moiety is optionally substituted with $R^6$), a carbamoyl, a (C1-C6)alkylaminocarbonyl (the amino moiety is optionally substituted with $R^6$), a carboxyl, a (C1-C6)alkylcarbonyl, a (C1-C6)haloalkylcarbonyl, a (C1-C6)alkoxycarbonyl, a hydroxyimino (C1-C6)alkyl, a (C1-C6)alkoxyimino (C1-C6)alkyl, a (C6-C12)aryl (the (C6-C12)aryl is optionally mono-substituted or poly-substituted with $R^7$), a (C6-C12)aryl (C1-C6)alkyl (the (C6-C12)aryl moiety is optionally mono-substituted or poly-substituted with $R^7$), a (C6-C12)arylcarbonyl (the (C6-C12)arylcarbonyl is optionally mono-substituted or poly-substituted with $R^7$), a (C6-C12)aryloxy (the (C6-C12)aryloxy is optionally mono-substituted or poly-substituted with $R^7$), a (C6-C12)arylthio (the (C6-C12)arylthio is optionally mono-substituted or poly-substituted with $R^7$), a (C6-C12)arylsulfinyl (the (C6-C12)arylsulfinyl is optionally mono-substituted or poly-substituted with $R^7$), a (C6-C12)arylsulfonyl (the (C6-C12)arylsulfonyl is optionally mono-substituted or poly-substituted with $R^7$), a (C6-C12)arylamino (the (C6-C12)aryl moiety is optionally mono-substituted or poly-substituted with $R^7$, and the amino moiety is optionally substituted with $R^6$), a 3- to 6-membered heterocyclyl (the 3- to 6-membered heterocyclyl is optionally mono-substituted or poly-substituted with $R^7$), a 3- to 6-membered heterocyclyl (C1-C6)alkyl (the 3- to 6-membered heterocyclyl moiety is optionally mono-substituted or poly-substituted with $R^7$), a 3-to 6-membered heterocyclylcarbonyl (the 3- to 6-membered heterocyclylcarbonyl is optionally mono-substituted or poly-substituted with $R^7$), a 3- to 6-membered heterocyclyloxy (the 3- to 6-membered heterocyclyloxy is optionally mono-substituted or poly-substituted with $R^7$), a 3- to 6-membered heterocyclylthio (the 3- to 6-membered heterocyclylthio is optionally mono-substituted or poly-substituted with $R^7$), a 3-to 6-membered heterocyclylsulfinyl (the 3- to 6-membered heterocyclylsulfinyl is optionally mono-substituted or poly-substituted with $R^7$), 3- to 6-membered heterocyclylsulfonyl (the 3- to 6-membered heterocyclylsulfonyl is optionally mono-substituted or poly-substituted with $R^7$), a 3- to 6-membered heterocyclylamino (the 3- to 6-membered heterocyclyl moiety is optionally mono-substituted or poly-substituted with $R^7$, and the amino moiety is optionally substituted with $R^6$), a 5- to 12-membered heteroaryl (the 5- to 12-membered heteroaryl is optionally mono-substituted or poly-substituted with $R^7$), a 5-to 12-membered heteroaryl (C1-C6)alkyl (the 5- to 12-membered heteroaryl moiety is optionally mono-substituted or poly-substituted with $R^7$), a 5- to 12-membered heteroarylcarbonyl (the 5- to 12-membered heteroarylcarbonyl is optionally mono-substituted or poly-substituted with $R^7$), a 5- to 12-membered heteroaryloxy (the 5- to 12-membered heteroaryloxy is optionally mono-substituted or poly-substituted with $R^7$), a 5-to 12-membered heteroarylthio (the 5- to 12-membered heteroarylthio is optionally mono-substituted or poly-substituted with $R^7$), a 5- to 12-membered heteroarylsulfinyl (the 5- to 12-membered heteroarylsulfinyl is optionally mono-substituted or poly-substituted with $R^7$), a 5- to 12-membered heteroarylsulfonyl (the 5- to 12-membered heteroarylsulfonyl is optionally mono-substituted or poly-substituted with $R^7$), or a 5- to 12-membered heteroarylamino (the 5- to 12-membered heteroaryl moiety is optionally mono-substituted or poly-substituted with $R^7$, and the amino moiety is optionally substituted with $R^6$),

$R^6$ each independently represent a hydrogen atom, a (C1-C6)alkyl, a (C1-C6)haloalkyl, a (C3-C6)cycloalkyl, a (C1-C6)alkoxy (C1-C6)alkyl, a (C1-C6)haloalkoxy (C1-C6)alkyl, a cyano (C1-C6)alkyl, a (C1-C6)alkylcarbonyl, a (C1-C6)haloalkylcarbonyl, a (C1-C6)alkoxycarbonyl, or a (C6-C12)arylcarbonyl (the (C6-C12)arylcarbonyl is optionally mono-substituted or poly-substituted with $R^7$),
$R^7$ each independently represent a hydrogen atom, a halogen atom, a cyano, a (C1-C6)alkyl, or a (C1-C6)alkoxy, and
$R^8$ each independently represent a halogen atom, a cyano, a (C1-C6)haloalkyl, a (C3-C6)cycloalkyl, a (C1-C6)alkoxy, a (C1-C6)alkylcarbonyl, a (C1-C6)alkoxycarbonyl, a (C1-C6)haloalkoxycarbonyl, a (C6-C12)aryl (the (C6-C12)aryl is optionally mono-substituted or poly-substituted with $R^7$), a 3-to 6-membered heterocyclyl (the 3- to 6-membered heterocyclyl is optionally mono-substituted or poly-substituted with $R^7$), or a 5- to 12-membered heteroaryl (the 5- to 12-membered heteroaryl is optionally mono-substituted or poly-substituted with $R^7$).

(25) The amidoxime derivative of formula [III] or the agriculturally acceptable salt thereof according to (24), wherein in the formula [III], $R^2$ represents a (C1-C6)alkyl (the (C1-C6)alkyl is optionally mono-substituted or poly-substituted with $R^4$), a (C3-C6)cycloalkyl (the (C3-C6)cycloalkyl is optionally mono-substituted or poly-substituted with $R^4$), or a 3- to 6-membered heterocyclyl (the 3- to 6-membered heterocyclyl is optionally mono-substituted or poly-substituted with $R^5$), and
$R^4$ each independently represent a (C3-C6)cycloalkyl, a (C3-C6)halocycloalkyl, a (C6-C12)aryl (the (C6-C12)aryl is optionally mono-substituted or poly-substituted with $R^5$), a 3-to 6-membered heterocyclyl (the 3- to 6-membered heterocyclyl is optionally mono-substituted or poly-substituted with $R^5$), a 5- to 12-membered heteroaryl (the 5- to 12-membered heteroaryl is optionally mono-substituted or poly-substituted with $R^5$), or a 1,3-benzodioxole (the 1,3-benzodioxole is optionally mono-substituted or poly-substituted with $R^5$).
(26) The amidoxime derivative of formula [III] or the agriculturally acceptable salt thereof according to (25), wherein in the formula [III], $R^2$ represents a (C1-C6)alkyl (the (C1-C6)alkyl is optionally mono-substituted or poly-substituted with $R^4$), and $R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl.
(27) The amidoxime derivative of formula [III] or the agriculturally acceptable salt thereof according to (24), wherein in the formula [III], $R^4$ each independently represent a halogen atom, a cyano, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-

C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted

with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

(28) The amidoxime derivative of formula [III] or the agriculturally acceptable salt thereof according to (27), wherein in the formula [III], $R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C6-C12)aryl (the (C6-C12)aryl is optionally mono-substituted or poly-substituted with $R^5$), or a 3- to 6-membered heterocyclyl (the 3- to 6-membered heterocyclyl is optionally mono-substituted or poly-substituted with $R^5$),

$R^4$ each independently represent a halogen atom, a cyano, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the

(C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl, and

$R^6$ each independently represent a hydrogen atom, a (C1-C6)alkyl, a (C1-C6)haloalkylcarbonyl, or a (C1-C6)alkoxycarbonyl.

## Effects of the Invention

The compound of the present invention is useful as an active ingredient of an agricultural chemical such as an agricultural and horticultural plant disease control agent.

An agricultural and horticultural plant disease control agent containing the compound of the present invention can exhibit an excellent control effect on a wide range of plant diseases caused by basidiomycetes, ascomycetes, deuteromycetes, oomycetes, zygomycetes, and the like, and can also control fungi that have acquired chemical resistance.

## Description of Embodiments

Symbols and terms used in the present specification will be described.

In the present invention, the "halogen atom" refers to a fluorine atom, a chlorine atom, a bromine atom, or an iodine atom.

In the present invention, the expression of "(C1-C6)" or the like indicates that the number of carbon atoms of the subsequent substituent is 1 to 6 in this case.

In the present invention, unless otherwise specified, the "(C1-C6)alkyl" refers to a linear or branched alkyl group having 1 to 6 carbon atoms, and examples thereof include methyl, ethyl, n-propyl, isopropyl, n-butyl, sec-butyl, isobutyl, tert-butyl, n-pentyl, 1-methylbutyl, 2-methylbutyl, 3-methylbutyl, 1-ethylpropyl, 1,1-dimethylpropyl, 1,2-dimethylpropyl, neopentyl, n-hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethyl-1-methylpropyl, and 1-ethyl-2-methylpropyl groups.

In the present invention, unless otherwise specified, the "(C3-C6)alkyl" refers to a linear or branched alkyl group having 3 to 6 carbon atoms, and examples thereof include groups excluding methyl and ethyl among the "(C1-C6)alkyl".

In the present invention, unless otherwise specified, the "(C2-C6)alkenyl" refers to a linear or branched alkenyl group having 2 to 6 carbon atoms, and examples thereof include vinyl, 1-propenyl, isopropenyl, 2-propenyl, 1-butenyl, 1-methyl-1-propenyl, 2-butenyl, 1-methyl-2-propenyl, 3-butenyl, 2-methyl-1-propenyl, 2-methyl-2-propenyl, 1,3-butadienyl, 1-pentenyl, 1-ethyl-2-propenyl, 2-pentenyl, 1-methyl-1-butenyl, 3-pentenyl, 1-methyl-2-butenyl, 4-pentenyl, 1-methyl-3-butenyl, 3-methyl-1-butenyl, 1,2-dimethyl-2-propenyl, 1,1-dimethyl-2-propenyl, 2-methyl-2-butenyl, 3-methyl-2-butenyl, 1,2-dimethyl-1-propenyl, 2-methyl-3-butenyl, 3-methyl-3-butenyl, 1,3-pentadienyl, 1-vinyl-2-propenyl, 1-hexenyl, 1-propyl-2-propenyl, 2-hexenyl, 1-methyl-1-pentenyl, 1-ethyl-2-butenyl, 3-hexenyl, 4-hexenyl, 5-hexenyl, 1-methyl-4-pentenyl, 1-ethyl-3-butenyl, 1-(isobutyl)vinyl, 1-ethyl-1-methyl-2-propenyl, 1-ethyl-2-methyl-2-propenyl, 1-(isopropyl)-2-propenyl, 2-methyl-2-pentenyl, 3-methyl-3-pentenyl, 4-methyl-3-pentenyl, 1,3-dimethyl-2-butenyl, 1,1-dimethyl-3-butenyl, 3-methyl-4-pentenyl, 4-methyl-4-pentenyl, 1,2-dimethyl-3-butenyl, 1,3-dimethyl-3-butenyl, 1,1,2-trimethyl-2-propenyl, 1,5-hexadienyl, 1-vinyl-3-butenyl, and 2,4-hexadienyl groups.

In the present invention, unless otherwise specified, the "(C2-C6)alkynyl" refers to a linear or branched alkynyl group having 2 to 6 carbon atoms, and examples thereof include ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 1-(n-propyl)-2-propynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, 1-ethyl-3-butynyl, 1-ethyl-1-methyl-2-propynyl, 1-(isopropyl)-2-propynyl, 1,1-dimethyl-2-butynyl, and 2,2-dimethyl-3-butynyl groups.

In the present invention, unless otherwise specified, the "(C3-C6)cycloalkyl" refers to a cycloalkyl group having 3 to 6 carbon atoms, and the ring may be optionally substituted with an alkyl group in a range of a specific number of carbon atoms. Examples thereof include cyclopropyl, 1-methylcyclopropyl, 2-methylcyclopropyl, 2,2-dimethylcyclopropyl, cyclobutyl, 1-methylcyclobutyl, cyclopentyl, 1-methylcyclopentyl, and cyclohexyl groups.

In the present invention, unless otherwise specified, the "(C6-C12)aryl" refers to an aromatic hydrocarbon group having 6 to 12 carbon atoms, and examples thereof include phenyl, biphenyl, naphthalen-1-yl, and naphthalen-2-yl groups.

In the present invention, unless otherwise specified, the "3- to 6-membered heterocyclyl" refers to a 3- to 6-membered non-aromatic hetero ring containing one or more atoms selected from an oxygen atom, a nitrogen atom, a sulfur atom, and a boron atom in addition to a carbon atom, and examples thereof include aziridinyl, oxylanyl, oxetanyl, thietanyl, tetrahydrofuranyl, tetrahydropyranyl, azetidinyl, pyrrolidinyl, piperidinyl, piperazinyl, dihydroisoxazolyl, dioxaborolanyl, dioxolanyl, dioxanyl, and morpholinyl groups.

In the present invention, unless otherwise specified, the "5- to 12-membered heteroaryl" refers to a 5- to 12-membered monocyclic or condensed bicyclic aromatic hetero ring containing one or more heteroatoms selected from a nitrogen atom, an oxygen atom, and a sulfur atom in addition to a carbon atom. Examples of the monocyclic hetero ring include pyridyl, pyrimidinyl, pyrazinyl, pyridazinyl, triazolyl, thienyl, furanyl, pyrrolyl, pyrazolyl, thiazolyl, isothiazolyl, oxazolyl, isooxazolyl, imidazolyl, triazolyl, thiadiazolyl, oxadiazolyl, and tetrazolyl groups. The condensed bicyclic aromatic hetero ring has a condensed bicyclic ring moiety formed by condensing one of the monocyclic groups and the phenyl ring or any one of the heteroaromatic monocyclic groups to form a $C_8$-$C_{10}$ bicyclic group, and examples thereof include indolyl, benzoimidazoly, indazolyl, benzotriazolyl, isoquinolinyl, quinolinyl, benzothiazolyl, benzofuranyl, benzothienyl, benzoisoxazolyl, pyrazolopyridyl, pyrazolopyrimidinyl, pyrrolopyridyl, triazoropyridyl, triazolopyrimidinyl, quinazolinyl, quinoxalinyl, and cinnolinyl groups.

In the present invention, unless otherwise specified, the "tri((C1-C6)alkyl)silyl" refers to a ((C1-C6)alkyl)$_3$-Si- whose alkyl moiety is as defined above, in which three alkyl groups may be the same as or different from each other, and examples thereof include trimethylsilyl, triethylsilyl, triisopropylsilyl, dimethylisopropylsilyl, and tertbutyldimethylsilyl groups.

In the present invention, unless otherwise specified, the "(C1-C6)haloalkyl" refers to a linear or branched alkyl group having 1 to 6 carbon atoms that is substituted with 1 to 13 same or different halogen atoms, and examples thereof include fluoromethyl, difluoromethyl, trifluoromethyl, chloromethyl, dichloromethyl, trichloromethyl, bromomethyl, dibromomethyl, tribromomethyl, iodomethyl, chlorodifluoromethyl, dichlorofluoromethyl, 1-fluoroethyl, 2-fluoroethyl, 1,1-difluoroethyl, 2,2-difluoroethyl, 2,2,2-trifluoroethyl, 1,1,2,2-tetrafluoroethyl, pentafluoroethyl, 1-chloroethyl, 2-chloroethyl, 1,1-dichloroethyl, 2,2-dichloroethyl, 2,2,2-trichloroethyl, 1,1,2,2-tetrachloroethyl, pentachloroethyl, 1-bromoethyl, 2-bromoethyl, 2,2,2-tribromoethyl, 1-iodoethyl, 2-iodoethyl, 2-chloro-2,2-difluoroethyl, 2,2-dichloro-2-fluoroethyl, 1-fluoropropyl, 2-fluoropropyl, 3-fluoropropyl, 1,1-difluoropropyl, 2,2-difluoropropyl, 3,3-difluoropropyl, 3,3,3-trifluoropropyl, 2,2,3,3,3-pentafluoropropyl, 1,1,2,3,3,3-hexafluoropropyl, heptafluoropropyl, 1-fluoro-2-propyl, 2-fluoro-2-propyl, 1,1-difluoro-2-propyl, 1,2-difluoro-2-propyl, 1,3-difluoro-2-propyl, 1,2,3-trifluoro-2-propyl, 1,1,3,3-tetrafluoro-2-propyl, 1,1,1,3,3,3-hexafluoro-2-propyl, heptafluoro-2-propyl, 1-chloropropyl, 2-chloropropyl, 3-chloropropyl, 1,1-dichloropropyl, 2,2-dichloropropyl, 3,3-dichloropropyl, 3,3,3-trichloropropyl, 2,2,3,3,3-pentachloropropyl, heptachloropropyl, 1-chloro-2-propyl, 2-chloro-2-propyl, 1,1-dichloro-2-propyl, 1,2-dichloro-2-propyl, 1,3-dichloro-2-propyl, 1,2,3-trichloro-2-propyl, 1,1,3,3-tetrachloro-2-propyl, 1,1,1,3,3,3-hexachloro-2-propyl, heptachloro-2-propyl, 1-bromopropyl, 2-bromopropyl, 3-bromopropyl, 1-bromo-2-propyl, 2-bromo-2-propyl, 1-iodopropyl, 2-iodopropyl, 3-iodopropyl, 1-iodo-2-propyl, 2-iodo-2-propyl, 1-fluorobutyl, 2-fluorobutyl, 3-fluorobutyl, 4-fluorobutyl, 4,4-difluorobutyl, 4,4,4-trifluorobutyl, 4,4,4-trifluoro-3-methylbutyl, 3,3,4,4,4-pentafluorobutyl, 2,2,3,4,4,4-hexafluorobutyl, 2,2,3,3,4,4,4-heptafluorobutyl, nonafluorobutyl, 1,1,1-trifluoro-2-butyl, 4,4,4-trifluoro-2-butyl, 3,3,4,4,4-pentafluoro-2-butyl, nonafluoro-2-butyl, 1,1,1,3,3,3-hexafluoro-2-(trifluoromethyl)-2-propyl, 1-chlorobutyl, 2-chlorobutyl, 3-chlorobutyl, 4-chlorobutyl, 4,4-dichlorobutyl, 4,4,4-trichlorobutyl, nonachlorobutyl, 1,1,1-trichloro-2-butyl, 4,4,4-trichloro-2-butyl, nonachloro-2-butyl, 1-bromobutyl, 2-bromobutyl, 3-bromobutyl, 4-bromobutyl, 1-iodobutyl, 2-iodobutyl, 3-iodobutyl, 4-iodobutyl, 4-chloro-1,1,2,2,3,3,4,4-octafluorobutyl, 4-bromo-1,1,2,2,3,3,4,4-octafluorobutyl, 1-fluoropentyl, 2-fluoropentyl, 3-fluoropentyl, 4-fluoropentyl, 5-fluoropentyl, 5,5,5-trifluoropentyl, 4,4,5,5,5-pentafluoropentyl, 3,3,4,4,5,5,5-heptafluoropentyl, 2,2,3,3,4,4,5,5-octafluoropentyl, 2,2,3,3,4,4,5,5,5-nonafluoropentyl, undecafluoropentyl, 1-chloropentyl, 2-chloropentyl, 3-chloropentyl, 4-chloropentyl,

5-chloropentyl, 5,5,5-trichloropentyl, 4,4,5,5,5-pentachloropentyl, 3,3,4,4,5,5,5-heptachloropentyl, 2,2,3,3,4,4,5,5,5-nonachloropentyl, undecachloropentyl, 1-bromopentyl, 2-bromopentyl, 3-bromopentyl, 4-bromopentyl, 5-bromopentyl, 5-iodopentyl, 1-fluorohexyl, 2-fluorohexyl, 3-fluorohexyl, 4-fluorohexyl, 5-fluorohexyl, 6-fluorohexyl, 6,6,6-trifluorohexyl, 5,5,6,6,6-pentafluorohexyl, 4,4,5,5,6,6,6-heptafluorohexyl, 3,3,4,4,5,5,6,6,6-nonafluorohexyl, 2,2,3,3,4,4,5,5,6,6,6-undecafluorohexyl, 2,2,3,3,4,4,5,5,6,6-decafluorohexyl, tridecafluorohexyl, 1-chlorohexyl, 2-chlorohexyl, 3-chlorohexyl, 4-chlorohexyl, 5-chlorohexyl, 6-chlorohexyl, 5-bromohexyl, 6-bromohexyl, 5-iodohexyl, and 6-iodohexyl groups.

In the present invention, unless otherwise specified, the "(C3-C6)halocycloalkyl" refers to a cycloalkyl group having 3 to 6 carbon atoms that is substituted with the same or different 1 to 11 halogen atoms, and examples thereof include 1-fluorocyclopropyl, 2-fluorocyclopropyl, 2,2-difluorocyclopropyl, 2,2,3,3-tetrafluorocyclopropyl, 1-chlorocyclopropyl, 2-chlorocyclopropyl, 2,2-dichlorocyclopropyl, 2,2,3,3-tetrachlorocyclopropyl, 2,2-dibromocyclopropyl, 2,2-diiodocyclopropyl, 1-fluorocyclobutyl, 2-fluorocyclobutyl, 3-fluorocyclobutyl, 3,3-difluorocyclobutyl, heptafluorocyclobutyl, 2-chlorocyclobutyl, 3-chlorocyclobutyl, 3,3-dichlorocyclobutyl, 3,3-dibromocyclobutyl, 3,3-diiodocyclobutyl, 1-fluorocyclopentyl, 2-fluorocyclopentyl, 3-fluorocyclopentyl, 2,2-difluorocyclopentyl, 3,3-difluorocyclopentyl, nonafluorocyclopentyl, 2,2-dichlorocyclopentyl, 3,3-dichlorocyclopentyl, 2,2-dibromocyclopentyl, 3,3-dibromocyclopentyl, 2,2-diiodocyclopentyl, 3,3-diiodocyclopentyl, 1-fluorocyclohexyl, 2-fluorocyclohexyl, 3-fluorocyclohexyl, 4-fluorocyclohexyl, 2,2-difluorocyclohexyl, 3,3-difluorocyclohexyl, 4,4-difluorocyclohexyl, 1-chlorocyclohexyl, 2-chlorocyclohexyl, 3-chlorocyclohexyl, 4-chlorocyclohexyl, 2,2-dichlorocyclohexyl, 3,3-dichlorocyclohexyl, 4,4-dichlorocyclohexyl, 3,3-dibromocyclohexyl, 4,4-dibromocyclohexyl, 3,3-diiodocyclohexyl, and 4,4-diiodocyclohexyl groups.

In the present invention, unless otherwise specified, the "(C1-C6)alkylamino" refers to a ((C1-C6)alkyl)-NH-group whose alkyl moiety is as defined above, and examples thereof include methylamino, ethylamino, n-propylamino, and isopropyl amino groups.

In the present invention, unless otherwise specified, the "(C2-C6)alkenylamino" refers to a ((C2-C6)alkenyl)-NH-group whose alkenyl moiety is as defined above, and examples thereof include vinylamino, 1-propenylamino, isopropenylamino, 2-propenylamino, 1-butenylamino, 1-methyl-1-propenylamino, 2-butenylamino, 1-methyl-2-propenylamino, 3-butenylamino, 2-methyl-1-propenylamino, 2-methyl-2-propenylamino, 1,3-butadienylamino, 1-pentenylamino, 1-ethyl-2-propenylamino, 2-pentenylamino, 1-methyl-1-butenylamino, 3-pentenylamino, 1-methyl-2-butenylamino, 4-pentenylamino, 1-methyl-3-butenylamino, 3-methyl-1-butenylamino, 1,2-dimethyl-2-propenylamino, 1,1-dimethyl-2-propenylamino, 2-methyl-2-butenylamino, 3-methyl-2-butenylamino, 1,2-dimethyl-1-propenylamino, 2-methyl-3-butenylamino, 3-methyl-3-butenylamino, 1,3-pentadienylamino, 1-vinyl-2-propenylamino, 1-hexenylamino, 1-propyl-2-propenylamino, 2-hexenylamino, 1-methyl-1-pentenylamino, 1-ethyl-2-butenylamino, 3-hexenylamino, 4-hexenylamino, 5-hexenylamino, 1-methyl-4-pentenylamino, 1-ethyl-3-butenylamino, 1-(isobutyl)vinylamino, 1-ethyl-1-methyl-2-propenylamino, 1-ethyl-2-methyl-2-propenylamino, 1-(isopropyl)-2-propenylamino, 2-methyl-2-pentenylamino, 3-methyl-3-pentenylamino, 4-methyl-3-pentenylamino, 1,3-dimethyl-2-butenylamino, 1,1-dimethyl-3-butenylamino, 3-methyl-4-pentenylamino, 4-methyl-4-pentenylamino, 1,2-dimethyl-3-butenylamino, 1,3-dimethyl-3-butenylamino, 1,1,2-trimethyl-2-propenylamino, 1,5-hexadienylamino, 1-vinyl-3-butenylamino, and 2,4-hexadienylamino groups.

In the present invention, unless otherwise specified, the "(C2-C6)alkynylamino" refers to a ((C2-C6)alkynyl)-NH-group whose alkynyl moiety is as defined above, and examples thereof include ethynylamino, 1-propynylamino, 2-propynylamino, 1-methyl-2-propynylamino, 1-ethyl-2-propynylamino, 1-butynylamino, 2-butynylamino, 3-butynylamino, 1-methyl-2-butynylamino, 1-pentynylamino, 2-pentynylamino, 3-pentynylamino, 4-pentynylamino, and 4,4-dimethyl-2-pentynylamino groups.

In the present invention, unless otherwise specified, the "(C3-C6)cycloalkylamino" refers to a ((C3-C6)cycloalkyl)-NH-group whose cycloalkyl moiety is as defined above, and examples thereof include cyclopropylamino, cyclobutylamino, cyclopentylamino, and cyclohexylamino groups.

In the present invention, unless otherwise specified, the "(C1-C6)alkylcarbonyl" refers to a ((C1-C6)alkyl)-C(=O)-group whose alkyl moiety is as defined above, and examples thereof include acetyl, propionyl, 2-methylpropionyl, 2,2-dimethylpropionyl, butanoyl, pivaloyl, 2-methylbutanoyl, 3-methylbutanoyl, 2-ethylbutanoyl, 2,2-dimethylbutanoyl, 2,3-dimethylbutanoyl, 3,3-dimethylbutanoyl, pentanoyl, 2-methylpentanoyl, 3-methylpentanoyl, 4-methylpentanoyl, and hexanoyl groups.

In the present invention, unless otherwise specified, the "(C2-C6)alkenylcarbonyl" refers to a ((C2-C6)alkenyl)-C(=O)-group whose alkenyl moiety is as defined above, and examples thereof include vinylcarbonyl, 1-propenylcarbonyl, isopropenylcarbonyl, 2-propenylcarbonyl, 1-methyl-2-propenylcarbonyl, 2-methyl-2-propenylcarbonyl, 2-butenylcarbonyl, 3-butenylcarbonyl, 3-methyl-2-butenylcarbonyl, 2-methyl-2-butenylcarbonyl, 2-pentenylcarbonyl, 3-pentenylcarbonyl, 4-pentenylcarbonyl, 2-hexenylcarbonyl, 3-hexenylcarbonyl, 4-hexenylcarbonyl, and 5-hexenylcarbonyl groups.

In the present invention, unless otherwise specified, the "(C2-C6)alkynylcarbonyl" refers to a ((C2-C6)alkynyl)-C(=O)-group whose alkynyl moiety is as defined above, and examples thereof include ethynylcarbonyl, 1-propynylcarbonyl, 2-propynylcarbonyl, 1-methyl-2-propynylcarbonyl, 1-ethyl-2-propynylcarbonyl, 1-butynylcarbonyl, 2-butynylcarbonyl, 3-butynylcarbonyl, 1-methyl-2-butynylcarbonyl, 1-pentynylcarbonyl, 2-pentynylcarbonyl, 3-pentynylcarbonyl,

4-pentynylcarbonyl, and 4,4-dimethyl-2-pentynylcarbonyl groups.

In the present invention, unless otherwise specified, the "(C3-C6)cycloalkylcarbonyl" refers to a ((C3-C6)cycloalkyl)-C(=O)- group whose cycloalkyl moiety is as defined above, and examples thereof include cyclopropylcarbonyl, cyclobutylcarbonyl, cyclopentylcarbonyl, and cyclohexylcarbonyl groups.

In the present invention, unless otherwise specified, the "(C6-C12)arylcarbonyl" refers to a ((C6-C12)aryl)-C(=O)- group whose aryl moiety is as defined above, and examples thereof include benzoyl, biphenylcarbonyl, naphthalen-1-ylcarbonyl, and naphthalen-2-ylcarbonyl groups.

In the present invention, unless otherwise specified, the "3- to 6-membered heterocyclylcarbonyl" refers to a (3- to 6-membered heterocyclyl)-C(=O)- group whose heterocyclyl moiety is as defined above, and examples thereof include aziridinylcarbonyl, oxylanylcarbonyl, oxetanylcarbonyl, thietanylcarbonyl, tetrahydrofuranylcarbonyl, tetrahydropyranylcarbonyl, azetidinylcarbonyl, pyrrolidinylcarbonyl, piperidinylcarbonyl, piperazinylcarbonyl, dihydroisoxazolylcarbonyl, dioxaborolanylcarbonyl, dioxolanylcarbonyl, dioxanylcarbonyl, and morpholinylcarbonyl groups.

In the present invention, unless otherwise specified, the "5- to 12-membered heteroarylcarbonyl" refers to a (5- to 12-membered heteroaryl)-C(=O)- group whose heteroaryl moiety is as defined above, and examples thereof include pyridylcarbonyl, pyrimidinylcarbonyl, pyrazinylcarbonyl, pyridadinylcarbonyl, triazinylcarbonyl, thienylcarbonyl, furanylcarbonyl, pyrrolylcarbonyl, pyrazolylcarbonyl, thiazolylcarbonyl, isothiazolylcarbonyl, oxazolylcarbonyl, isoxazolylcarbonyl, imidazolylcarbonyl, triazolylcarbonyl, thiadiazolylcarbonyl, oxadiazolylcarbonyl, tetrazolylcarbonyl, indolylcarbonyl, benzoimidazolylcarbonyl, indazolylcarbonyl, benzotriazolylcarbonyl, isoquinolinylcarbonyl, quinolinylcarbonyl, benzothiazolylcarbonyl, benzofuranylcarbonyl, benzothienylcarbonyl, benzoisoxazolylcarbonyl, pyrazolopyridylcarbonyl, pyrazolopyrimidinylcarbonyl, pyrrolopyridylcarbonyl, triazolopyridylcarbonyl, triazolopyrimidinylcarbonyl, quinazolinylcarbonyl, quinoxalinylcarbonyl, and cinnolinylcarbonyl groups.

In the present invention, unless otherwise specified, the "(C1-C6)alkoxycarbonyl" refers to a ((C1-C6)alkyl)-O-C(=O)-group whose alkyl moiety is as defined above, and examples thereof include methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentoxycarbonyl, 1-methyl butoxycarbonyl, 2-methyl butoxycarbonyl, 3-methyl butoxycarbonyl, 1-ethylpropoxycarbonyl, 1,1-dimethylpropoxycarbonyl, 1,2-dimethylpropoxycarbonyl, and 2,2-dimethylpropoxycarbonyl groups.

In the present invention, unless otherwise specified, the "(C2-C6)alkenyloxycarbonyl" refers to a ((C2-C6)alkenyl)-O-C(=O)- group whose alkenyl moiety is as defined above, and examples thereof include vinyloxycarbonyl, 1-propenyloxycarbonyl, isopropenyloxycarbonyl, 2-propenyloxycarbonyl, 1-methyl-2-propenyloxycarbonyl, 2-methyl-2-propenyloxycarbonyl, 2-butenyloxycarbonyl, 3-butenyloxycarbonyl, 3-methyl-2-butenyloxycarbonyl, 2-methyl-2-butenyloxycarbonyl, 2-pentenyloxycarbonyl, 3-pentenyloxycarbonyl, 4-pentenyloxycarbonyl, 2-hexenyloxycarbonyl, 3-hexenyloxycarbonyl, 4-hexenyloxycarbonyl, and 5-hexenyloxycarbonyl groups.

In the present invention, unless otherwise specified, the "(C2-C6)alkynyloxycarbonyl" refers to a ((C2-C6)alkynyl)-O-C(=O)- group whose alkynyl moiety is as defined above, and examples thereof include ethynyloxycarbonyl, 1-propynyloxycarbonyl, 2-propynyloxycarbonyl, 1-methyl-2-propynyloxycarbonyl, 1-ethyl-2-propynyloxycarbonyl, 1-butynyloxycarbonyl, 2-butynyloxycarbonyl, 3-butynyloxycarbonyl, 1-methyl-2-butynyloxycarbonyl, 1-pentynyloxycarbonyl, 2-pentynyloxycarbonyl, 3-pentynyloxycarbonyl, 4-pentynyloxycarbonyl, and 4,4-dimethyl-2-pentynyloxycarbonyl groups.

In the present invention, unless otherwise specified, the "(C3-C6)cycloalkyloxycarbonyl" refers to a ((C3-C6)cycloalkyl)-O-C(=O)- group whose cycloalkyl moiety is as defined above, and examples thereof include cyclopropyloxycarbonyl, cyclobutyloxycarbonyl, cyclopentyloxycarbonyl, and cyclohexyloxycarbonyl groups.

In the present invention, unless otherwise specified, the "(C6-C12)aryloxycarbonyl" refers to a ((C6-C12)aryl)-O-C(=O)-group whose aryl moiety is as defined above, and examples thereof include phenyloxycarbonyl, biphenyloxycarbonyl, naphthalen-1-yloxycarbonyl, and naphthalen-2-yloxycarbonyl groups.

In the present invention, unless otherwise specified, the "3- to 6-membered heterocyclyloxycarbonyl" refers to a (3- to 6-membered heterocyclyl)-O-C(=O)- group whose heterocyclyl moiety is as defined above, and examples thereof include aziridinyloxycarbonyl, oxylanyloxycarbonyl, oxetanyloxycarbonyl, thietanyloxycarbonyl, tetrahydrofuranyloxycarbonyl, tetrahydropyranyloxycarbonyl, azetidinyloxycarbonyl, pyrrolidinyloxycarbonyl, piperidinyloxycarbonyl, piperazinyloxycarbonyl, dihydroisoxazolyloxycarbonyl, dioxaborolanyloxycarbonyl, dioxolanyloxycarbonyl, dioxanyloxycarbonyl, and morpholinyloxycarbonyl groups.

In the present invention, unless otherwise specified, the "5- to 12-membered heteroaryloxycarbonyl" refers to a (5- to 12-membered heteroaryl)-O-C(=O)- group whose heteroaryl moiety is as defined above, and examples thereof include pyridyloxycarbonyl, pyrimidinyloxycarbonyl, pyrazinyloxycarbonyl, pyridadinyloxycarbonyl, triazinyloxycarbonyl, thienyloxycarbonyl, furanyloxycarbonyl, pyrrolyloxycarbonyl, pyrazolyloxycarbonyl, thiazolyloxycarbonyl, isothiazolyloxycarbonyl, oxazolyloxycarbonyl, isoxazolyloxycarbonyl, imidazolyloxycarbonyl, triazolyloxycarbonyl, thiadiazolyloxycarbonyl, oxadiazolyloxycarbonyl, tetrazolyloxycarbonyl, indolyloxycarbonyl, benzoimidazolyloxycarbonyl, indazolyloxycarbonyl, benzotriazolyloxycarbonyl, isoquinolinyloxycarbonyl, quinolinyloxycarbonyl, benzothiazolyloxycarbonyl, benzofuranyloxycarbonyl, benzothienyloxycarbonyl, benzoisooxazolyloxycarbonyl, pyrazolopyridyloxycarbonyl, pyrazolopyrimidinyloxycarbonyl, pyrrolopyridyloxycarbonyl, triazolopyridyloxycarbonyl, triazolopyrimidinyloxycarbonyl, quinazolinyloxycarbonyl, quinoxalinyloxycarbonyl, and cinnolinyloxycarbonyl groups.

In the present invention, unless otherwise specified, the "(C1-C6)alkylaminocarbonyl" refers to a ((C1-C6)alkyl)-NH-C(=O)- group whose alkyl moiety is as defined above, and examples thereof include methylaminocarbonyl, ethylaminocarbonyl, n-propylaminocarbonyl, isopropylaminocarbonyl, n-butylaminocarbonyl, isobutylaminocarbonyl, sec-butylaminocarbonyl, tert-butylaminocarbonyl, n-pentylaminocarbonyl, 1-methylbutylaminocarbonyl, 2-methylbutylaminocarbonyl, 3-methylbutylaminocarbonyl, 1-ethylpropylaminocarbonyl, 1,1-dimethylpropylaminocarbonyl, 1,2-dimethylpropylaminocarbonyl, 2,2-dimethylpropylaminocarbonyl, and n-hexylaminocarbonyl groups.

In the present invention, unless otherwise specified, the "(C2-C6)alkenylaminocarbonyl" refers to a ((C2-C6)alkenyl)-NH-C(=O)- group whose alkenyl moiety is as defined above, and examples thereof include vinylaminocarbonyl, 1-propenylaminocarbonyl, isopropenylaminocarbonyl, 2-propenylaminocarbonyl, 1-methyl-2-propenylaminocarbonyl, 2-methyl-2-propenylaminocarbonyl, 2-butenylaminocarbonyl, 3-butenylaminocarbonyl, 3-methyl-2-butenylaminocarbonyl, 2-methyl-2-butenylaminocarbonyl, 2-pentenylaminocarbonyl, 3-pentenylaminocarbonyl, 4-pentenylaminocarbonyl, 2-hexenylaminocarbonyl, 3-hexenylaminocarbonyl, 4-hexenylaminocarbonyl, and 5-hexenylaminocarbonyl groups.

In the present invention, unless otherwise specified, the "(C2-C6)alkynylaminocarbonyl" refers to a ((C2-C6)alkynyl)-NH-C(=O)- group whose alkynyl moiety is as defined above, and examples thereof include ethynylaminocarbonyl, 1-propynylaminocarbonyl, 2-propynylaminocarbonyl, 1-methyl-2-propynylaminocarbonyl, 1-ethyl-2-propynylaminocarbonyl, 1-butynylaminocarbonyl, 2-butynylaminocarbonyl, 3-butynylaminocarbonyl, 1-methyl-2-butynylaminocarbonyl, 1-pentynylaminocarbonyl, 2-pentynylaminocarbonyl, 3-pentynylaminocarbonyl, 4-pentynylaminocarbonyl, and 4,4-dimethyl-2-pentynylaminocarbonyl groups.

In the present invention, unless otherwise specified, the "(C3-C6)cycloalkylaminocarbonyl" refers to a ((C3-C6)cycloalkyl)-NH-C(=O)- group whose cycloalkyl moiety is as defined above, and examples thereof include cyclopropylaminocarbonyl, cyclobutylaminocarbonyl, cyclopentylaminocarbonyl, and cyclohexylaminocarbonyl groups.

In the present invention, unless otherwise specified, the "(C6-C12)arylaminocarbonyl" refers to a ((C6-C12)aryl)-NH-C(=O)- group whose aryl moiety is as defined above, and examples thereof include phenylaminocarbonyl, biphenylaminocarbonyl, naphthalen-1-ylaminocarbonyl, and naphthalen-2-ylaminocarbonyl groups.

In the present invention, unless otherwise specified, the "3- to 6-membered heterocyclylaminocarbonyl" refers to a (3-to 6-membered heterocyclyl)-NH-C(=O)- group whose heterocyclyl moiety is as defined above, and examples thereof include aziridinylaminocarbonyl, oxylanylaminocarbonyl, oxetanylaminocarbonyl, thietanylaminocarbonyl, tetrahydrofuranylaminocarbonyl, tetrahydropyranylaminocarbonyl, azetidinylaminocarbonyl, pyrrolidinylaminocarbonyl, piperidinylaminocarbonyl, piperazinylaminocarbonyl, dihydroisoxazolylaminocarbonyl, dioxaborolanylaminocarbonyl, dioxolanylaminocarbonyl, dioxanylaminocarbonyl, and morpholinylaminocarbonyl groups.

In the present invention, unless otherwise specified, the "5- to 12-membered heteroarylaminocarbonyl" refers to a (5- to 12-membered heteroaryl)-NH-C(=O)- group whose heteroaryl moiety is as defined above, and examples thereof include pyridylaminocarbonyl, pyrimidinylaminocarbonyl, pyrazinylaminocarbonyl, pyridadinylaminocarbonyl, triazinylaminocarbonyl, thienylaminocarbonyl, furanylaminocarbonyl, pyrrolylaminocarbonyl, pyrazolylaminocarbonyl, thiazolylaminocarbonyl, isothiazolylaminocarbonyl, oxazolylaminocarbonyl, isoxazolylaminocarbonyl, imidazolylaminocarbonyl, triazolylaminocarbonyl, thiadiazolylaminocarbonyl, oxadiazolylaminocarbonyl, tetrazolylaminocarbonyl, indolylaminocarbonyl, benzoimidazolylaminocarbonyl, indazolylaminocarbonyl, benzotriazolylaminocarbonyl, isoquinolinylaminocarbonyl, quinolinylaminocarbonyl, benzothiazolylaminocarbonyl, benzofuranylaminocarbonyl, benzothienylaminocarbonyl, benzoisooxazolylaminocarbonyl, pyrazolopyridylaminocarbonyl, pyrazolopyrimidinylaminocarbonyl, pyrrolopyridylaminocarbonyl, triazolopyridylaminocarbonyl, triazolopyrimidinylaminocarbonyl, quinazolinylaminocarbonyl, quinoxalinylaminocarbonyl, and cinnolinylaminocarbonyl groups.

In the present invention, unless otherwise specified, the "(C1-C6)alkylcarbonylamino" refers to a ((C1-C6)alkyl)-C(=O)-NH- group whose alkyl moiety is as defined above, and examples thereof include acetylamino, propionylamino, butyrylamino, and isobutyrylamino groups.

In the present invention, unless otherwise specified, the "(C1-C6)alkoxycarbonylamino" refers to a ((C1-C6)alkyl)-O-C(=O)-NH- group whose alkyl moiety is as defined above, and examples thereof include methoxycarbonylamino, ethoxycarbonylamino, isopropoxycarbonylamino, tert-butoxycarbonylamino, and isobutoxycarbonylamino groups.

In the present invention, unless otherwise specified, the "(C2-C6)alkenylcarbonylamino" refers to a ((C2-C6)alkenyl)-C(=O)-NH- group whose alkenyl moiety is as defined above, and examples thereof include vinylcarbonylamino, 1-propenylcarbonylamino, isopropenylcarbonylamino, 2-propenylcarbonylamino, 1-methyl-2-propenylcarbonylamino, 2-methyl-2-propenylcarbonylamino, 2-butenylcarbonylamino, 3-butenylcarbonylamino, 3-methyl-2-butenylcarbonylamino, 2-methyl-2-butenylcarbonylamino, 2-pentenylcarbonylamino, 3-pentenylcarbonylamino, 4-pentenylcarbonylamino, 2-hexenylcarbonylamino, 3-hexenylcarbonylamino, 4-hexenylcarbonylamino, and 5-hexenylcarbonylamino groups.

In the present invention, unless otherwise specified, the "(C2-C6)alkynylcarbonylamino" refers to a ((C2-C6)alkynyl)-C(=O)-NH- group whose alkynyl moiety is as defined above, and examples thereof include ethynylcarbonylamino, 1-propynylcarbonylamino, 2-propynylcarbonylamino, 1-methyl-2-propynylcarbonylamino, 1-ethyl-2-propynylcarbonylamino, 1-butynylcarbonylamino, 2-butynylcarbonylamino, 3-butynylcarbonylamino, 1-methyl-2-butynylcarbonylamino, 1-pentynylcarbonylamino, 2-pentynylcarbonylamino, 3-pentynylcarbonylamino, 4-pentynylcarbonylamino, and 4,4-dimethyl-2-pentynylcarbonylamino groups.

In the present invention, unless otherwise specified, the "(C3-C6)cycloalkylcarbonylamino" refers to a ($C_3$-$C_6$ cycloalkyl)-C(=O)-NH- group whose cycloalkyl moiety is as defined above, and examples thereof include cyclopropylcarbonylamino, cyclobutylcarbonylamino, cyclopentylcarbonylamino, and cyclohexylcarbonylamino groups.

In the present invention, unless otherwise specified, the "(C6-C12)arylcarbonylamino" refers to a ((C6-C12)aryl)-C(=O)-NH- group whose aryl moiety is as defined above, and examples thereof include benzoylamino, biphenylcarbonylamino, naphthalen-1-ylcarbonylamino, and naphthalen-2-ylcarbonylamino groups.

In the present invention, unless otherwise specified, the "3- to 6-membered heterocyclylcarbonylamino" refers to a (3-to 6-membered heterocyclyl)-C(=O)-NH- group whose heterocyclyl moiety is as defined above, and examples thereof include aziridinylcarbonylamino, oxylanylcarbonylamino, oxetanylcarbonylamino, thietanylcarbonylamino, tetrahydrofuranylcarbonylamino, tetrahydropyranylcarbonylamino, azetidinylcarbonylamino, pyrrolidinylcarbonylamino, piperidinylcarbonylamino, piperazinylcarbonylamino, dihydroisoxazolylcarbonylamino, dioxaborolanylcarbonylamino, dioxolanylcarbonylamino, dioxanylcarbonylamino, and morpholinylcarbonylamino groups.

In the present invention, unless otherwise specified, the "5- to 12-membered heteroarylcarbonylamino" refers to a (5- to 12-membered heteroaryl)-C(=O)-NH- group whose heteroaryl moiety is as defined above, and examples thereof include pyridylcarbonylamino, pyrimidinylcarbonylamino, pyrazinylcarbonylamino, pyridadinylcarbonylamino, triazinylcarbonylamino, thienylcarbonylamino, furanylcarbonylamino, pyrrolylcarbonylamino, pyrazolylcarbonylamino, thiazolylcarbonylamino, isothiazolylcarbonylamino, oxazolylcarbonylamino, isoxazolylcarbonylamino, imidazolylcarbonylamino, triazolylcarbonylamino, thiadiazolylcarbonylamino, oxadiazolylcarbonylamino, tetrazolylcarbonylamino, indolylcarbonylamino, benzoimidazolylcarbonylamino, indazolylcarbonylamino, benzotriazolylcarbonylamino, isoquinolinylcarbonylamino, quinolinylcarbonylamino, benzothiazolylcarbonylamino, benzofuranylcarbonylamino, benzothienylcarbonylamino, benzoisooxazolylcarbonylamino, pyrazolopyridylcarbonylamino, pyrazolopyrimidinylcarbonylamino, pyrrolopyridylcarbonylamino, triazolopyridylcarbonylamino, triazolopyrimidinylcarbonylamino, quinazolinylcarbonylamino, quinoxalinylcarbonylamino, and cinnolinylcarbonylamino groups.

In the present invention, unless otherwise specified, "(C1-C6)alkylcarbonyloxy" refers to a ((C1-C6)alkyl)-C(=O)-O- group whose alkyl moiety is as defined above, and examples thereof include methylcarbonyloxy, ethylcarbonyloxy, n-propylcarbonyloxy, isopropylcarbonyloxy, n-butylcarbonyloxy, isobutylcarbonyloxy, sec-butylcarbonyloxy, and tert-butylcarbonyloxy groups.

In the present invention, unless otherwise specified, the "(C2-C6)alkenylcarbonyloxy" refers to a ((C2-C6)alkenyl)-C(=O)-O- group whose alkenyl moiety is as defined above, and examples thereof include vinylcarbonyloxy, 1-propenylcarbonyloxy, isopropenylcarbonyloxy, 2-propenylcarbonyloxy, 1-methyl-2-propenylcarbonyloxy, 2-methyl-2-propenylcarbonyloxy, 2-butenylcarbonyloxy, 3-butenylcarbonyloxy, 3-methyl-2-butenylcarbonyloxy, 2-methyl-2-butenylcarbonyloxy, 2-pentenylcarbonyloxy, 3-pentenylcarbonyloxy, 4-pentenylcarbonyloxy, 2-hexenylcarbonyloxy, 3-hexenylcarbonyloxy, 4-hexenylcarbonyloxy, and 5-hexenylcarbonyloxy groups.

In the present invention, unless otherwise specified, the "(C2-C6)alkynylcarbonyloxy" refers to a ((C2-C6)alkynyl)-C(=O)-O- group whose alkynyl moiety is as defined above, and examples thereof include ethynylcarbonyloxy, 1-propynylcarbonyloxy, 2-propynylcarbonyloxy, 1-methyl-2-propynylcarbonyloxyl, 1-ethyl-2-propynylcarbonyloxy, 1-butynylcarbonyloxy, 2-butynylcarbonyloxy, 3-butynylcarbonyloxy, 1-methyl-2-butynylcarbonyloxy, 1-pentynylcarbonyloxy, 2-pentynylcarbonyloxy, 3-pentynylcarbonyloxy, 4-pentynylcarbonyloxy, and 4,4-dimethyl-2-pentynylcarbonyloxy groups.

In the present invention, unless otherwise specified, the "(C3-C6)cycloalkylcarbonyloxy" refers to a ((C3-C6)cycloalkyl)-C(=O)-O- group whose cycloalkyl moiety is as defined above, and examples thereof include cyclopropylcarbonyloxy, cyclobutylcarbonyloxy, cyclopentylcarbonyloxy, and cyclohexylcarbonyloxy groups.

In the present invention, unless otherwise specified, the "(C6-C12)arylcarbonyloxy" refers to a ((C6-C12)aryl)-C(=O)-O-group whose aryl moiety is as defined above, and examples thereof include benzoyloxy, biphenylcarbonyloxy, naphthalen-1-ylcarbonyloxy, and naphthalen-2-ylcarbonyloxy groups.

In the present invention, unless otherwise specified, the "3- to 6-membered heterocyclylcarbonyloxy" refers to a (3- to 6-membered heterocyclyl)-C(=O)-O- group whose heterocyclyl moiety is as defined above, and examples thereof include aziridinylcarbonyloxy, oxylanylcarbonyloxy, oxetanylcarbonyloxy, thietanylcarbonyloxy, tetrahydrofuranylcarbonyloxy, tetrahydropyranylcarbonyloxy, azetidinylcarbonyloxy, pyrrolidinylcarbonyloxyy, piperidinylcarbonyloxy, piperazinylcarbonyloxy, dihydroisoxazolylcarbonyloxy, dioxaborolanylcarbonyloxy, dioxolanylcarbonyloxy, dioxanylcarbonyloxyl, and morpholinylcarbonyloxy groups.

In the present invention, unless otherwise specified, the "5- to 12-membered heteroarylcarbonyloxy" refers to a (5- to 12-membered heteroaryl)-C(=O)-O- group whose heteroaryl moiety is as defined above, and examples thereof include pyridylcarbonyloxy, pyrimidinylcarbonyloxy, pyrazinylcarbonyloxy, pyridadinylcarbonyloxy, triazinylcarbonyloxy,

thienylcarbonyloxy, furanylcarbonyloxy, pyrrolylcarbonyloxy, pyrazolylcarbonyloxy, thiazolylcarbonyloxy, isothiazolylcarbonyloxy, oxazolylcarbonyloxy, isoxazolylcarbonyloxy, imidazolylcarbonyloxy, triazolylcarbonyloxy, thiadiazolylcarbonyloxy, oxadiazolylcarbonyloxy, tetrazolylcarbonyloxy, indolylcarbonyloxy, benzoimidazolylcarbonyloxy, indazolylcarbonyloxy, benzotriazolylcarbonyloxy, isoquinolinylcarbonyloxy, quinolinylcarbonyloxy, benzothiazolylcarbonyloxy, benzofuranylcarbonyloxy, benzothienylcarbonyloxy, benzoisooxazolylcarbonyloxy, pyrazolopyridylcarbonyloxy, pyrazolopyrimidinylcarbonyloxy, pyrrolopyridylcarbonyloxy, triazolopyridylcarbonyloxy, triazolopyrimidinylcarbonyloxy, quinazolinylcarbonyloxy, quinoxalinylcarbonyloxy, and cinnolinylcarbonyloxy groups.

In the present invention, unless otherwise specified, the "(C1-C6)alkoxy" refers to a ((C1-C6)alkyl)-O- group whose alkyl moiety is as defined above, and examples thereof include methoxy, ethoxy, n-propoxy, isopropoxy, n-butoxy, isobutoxy, sec-butoxy, tert-butoxy, n-pentoxy, 1-methylbutoxy, 2-methylbutoxy, 3-methylbutoxy, 1-ethylpropoxy, 1,1-dimethylpropoxy, 1,2-dimethylpropoxy, and n-hexyloxy groups.

In the present invention, unless otherwise specified, the "(C1-C6)haloalkoxy" refers to a ((C1-C6)haloalkyl)-O-group whose haloalkyl moiety is as defined above, and examples thereof include difluoromethoxy, dichloromethoxy, trifluoromethoxy, trichloromethoxy, tribromomethoxy, chlorodifluoromethoxy, bromodifluoromethoxy, 2-fluoroethoxy, 1-chloroethoxy, 2-chloroethoxy, 1-bromoethoxy, 2-bromoethoxy, 2,2-difluoroethoxy, 1,2-dichloroethoxy, 2,2-dichloroethoxy, 2,2,2-trifluoroethoxy, 2,2,2-trichloroethoxy, 1,1,2,2-tetrafluoroethoxy, pentafluoroethoxy, 2-bromo-2-chloroethoxy, 2-chloro-1,1,2,2-tetrafluoroethoxy, 1-chloro-1,2,2,2-tetrafluoroethoxy, 1-chloropropoxy, 2-chloropropoxy, 3-chloropropoxy, 2-bromopropoxy, 3-bromopropoxy, 2-bromo-1-methylethoxy, 3-iodopropoxy, 2,3-dichloropropoxy, 2,3-dibromopropoxy, 3,3,3-trifluoropropoxy, 3,3,3-trifluoro-2-propoxy, 3,3,3-trichloropropoxy, 3-bromo-3,3-difluoropropoxy, 2,2-difluoropropoxy, 3,3-dichloro-3-fluoropropoxy, 2,2,3,3-tetrafluoropropoxy, 1-bromo-3,3,3-trifluoropropoxy, 2,2,3,3,3-pentafluoropropoxy, 2,2,2-trifluoro-1-trifluoromethylethoxy, heptafluoropropoxy, heptafluoro-2-propoxy, 1,2,2,2-tetrafluoro-1-trifluoromethylethoxy, 1,1,2,3,3,3-hexafluoropropoxy, 2-chlorobutoxy, 3-chlorobutoxy, 4-chlorobutoxy, 2-chloro-1,1-dimethylethoxy, 4-bromobutoxy, 3-bromo-2-methylpropoxy, 2-bromo-1,1-dimethylethoxy, 2,2-dichloro-1,1-dimethylethoxy, 2-chloro-1-chloromethyl-2-methylethoxy, 4,4,4-trifluorobutoxy, 3,3,3-trifluoro-1-methylpropoxy, 3,3,3-trifluoro-2-methylpropoxy, 2,3,4-trichlorobutoxy, 2,2,2-trichloro-1,1-dimethylethoxy, 4-chloro-4,4-difluorobutoxy, 4,4-dichloro-4-fluorobutoxy, 4-bromo-4,4-difluorobutoxy, 2,4-dibromo-4,4-difluorobutoxy, 3,4-dichloro-3,4,4-trifluorobutoxy, 3,3-dichloro-4,4,4-trifluorobutoxy, 4-bromo-3,3,4,4-tetrafluorobutoxy, 4-bromo-3-chloro-3,4,4-trifluorobutoxy, 2,2,3,3,4,4-hexafluorobutoxy, 2,2,3,4,4,4-hexafluorobutoxy, 2,2,2-trifluoro-1-methyl-1-trifluoromethylethoxy, 3,3,3-trifluoro-2-trifluoromethylpropoxy, 2,2,3,3,4,4,4-heptafluorobutoxy, 3,3,4,4,4-pentafluoro-2-butoxy, 2,3,3,3-tetrafluoro-2-trifluoromethylpropoxy, 1,1,2,2,3,3,4,4-octafluorobutoxy, nonafluorobutoxy, perfluoro-tert-butoxy, 4-chloro-1,1,2,2,3,3,4,4-octafluorobutoxy, 5,5,5-trifluoropentoxy, 4,4,5,5,5-pentafluoropentoxy, 3,3,4,4,5,5,5-heptafluoropentoxy, 3,3,4,4,5,5,5-heptafluoro-2-pentoxy, 2, 2, 3, 3, 4, 4, 5, 5, 5-nonafluoropentoxy, 2,2,3,3,4,4,5,5-octafluoropentoxy, perfluoropentoxy, 4,4,5,5,5-pentafluoro-2-butoxy, 2,2-bis(trifluoromethyl)propoxy, 2, 2, 3, 3, 4, 4, 5, 5, 6, 6, 6-undecafluorohexyloxy, 3,3,4,4,5,5,6,6,6-nonafluorohexyloxy, 4,4,5,5,6,6,6-heptafluorohexyloxy, 2,2,3,3,4,4,5,5,6,6-decafluorohexyloxy, 4,4,4-trifluoro-3,3-bis(trifluoromethyl)butyloxy, and perfluorohexyloxy groups.

In the present invention, unless otherwise specified, the "(C2-C6)alkenyloxy" refers to a ((C2-C6)alkenyl)-O-group whose alkenyl moiety is as defined above, and examples thereof include vinyloxy, 1-propenyloxy, isopropenyloxy, 2-propenyloxy, 1-methyl-2-propenyloxy, 2-methyl-2-propenyloxy, 2-butenyloxy, 3-butenyloxy, 3-methyl-2-butenyloxy, 2-methyl-2-butenyloxy, 2-pentenyloxy, 3-pentenyloxy, 4-pentenyloxy, 2-hexenyloxy, 3-hexenyloxy, 4-hexenyloxy, and 5-hexenyloxy groups.

In the present invention, unless otherwise specified, the "(C2-C6)alkynyloxy" refers to a ((C2-C6)alkynyl)-O-group whose alkynyl moiety is as defined above, and examples thereof include ethynyloxy, 1-propynyloxy, 2-propynyloxy, 1-methyl-2-propynyloxy, 1-ethyl-2-propynyloxy, 1-butynyloxy, 2-butynyloxy, 3-butynyloxy, 1-methyl-2-butynyloxy, 1-pentynyloxy, 2-pentynyloxy, 3-pentynyloxy, 4-pentynyloxy, and 4,4-dimethyl-2-pentynyloxy groups.

In the present invention, unless otherwise specified, the "(C3-C6)cycloalkyloxy" refers to a ((C3-C6)cycloalkyl)-O-group whose cycloalkyl moiety is as defined above, and examples thereof include cyclopropyloxy, cyclobutyloxy, cyclopentyloxy, and cyclohexyloxy groups.

In the present invention, unless otherwise specified, the "(C1-C6)alkylsulfonylamino" refers to a ((C1-C6)alkyl)-S(=O)$_2$-NH- group whose alkyl moiety is as defined above, and examples thereof include methylsulfonylamino, ethylsulfonylamino, n-propylsulfonylamino, isopropylsulfonylamino, and tert-butylsulfonylamino groups.

In the present invention, unless otherwise specified, the "(C2-C6)alkenylsulfonylamino" refers to a ((C2-C6)alkenyl)-S(=O)$_2$-NH- group whose alkenyl moiety is as defined above, and examples thereof include vinylsulfonylamino, 1-propenylsulfonylamino, isopropenylsulfonylamino, 2-propenylsulfonylamino, 1-methyl-2-propenylsulfonylamino, 2-methyl-2-propenylsulfonylamino, 2-butenylsulfonylamino, 3-butenylsulfonylamino, 3-methyl-2-butenylsulfonylamino, 2-methyl-2-butenylsulfonylamino, 2-pentenylsulfonylamino, 3-pentenylsulfonylamino, 4-pentenylsulfonylamino, 2-hexenylsulfonylamino, 3-hexenylsulfonylamino, 4-hexenylsulfonylamino, and 5-hexenylsulfonylamino groups.

In the present invention, unless otherwise specified, the "(C2-C6)alkynylsulfonylamino" refers to a ((C2-C6)alkynyl)-S(=O)$_2$-NH- group whose alkynyl moiety is as defined above, and examples thereof include ethynylsulfonylamino,

1-propynylsulfonylamino, 2-propynylsulfonylamino, 1-methyl-2-propynylsulfonylamino, 1-ethyl-2-propynylsulfonylamino, 1-butynylsulfonylamino, 2-butynylsulfonylamino, 3-butynylsulfonylamino, 1-methyl-2-butynylsulfonylamino, 1-pentynylsulfonylamino, 2-pentynylsulfonylamino, 3-pentynylsulfonylamino, 4-pentynylsulfonylamino, and 4,4-dimethyl-2-pentynylsulfonylamino groups.

In the present invention, unless otherwise specified, the "(C3-C6)cycloalkylsulfonylamino" refers to a ((C3-C6)cycloalkyl)-S(=O)$_2$-NH- group whose cycloalkyl moiety is as defined above, and examples thereof include cyclopropylsulfonylamino, cyclobutylsulfonylamino, cyclopentylsulfonylamino, and cyclohexylsulfonylamino groups.

In the present invention, unless otherwise specified, the "(C6-C12)arylsulfonylamino" refers to a ((C6-C12)aryl)-S(=O)$_2$-NH- group whose aryl moiety is as defined above, and examples thereof include phenylsulfonylamino, biphenylsulfonylamino, naphthalen-1-ylsulfonylamino, and naphthalen-2-ylsulfonylamino groups.

In the present invention, unless otherwise specified, the "3- to 6-membered heterocyclylsulfonylamino" refers to a (3-to 6-membered heterocyclyl)-S(=O)$_2$-NH- group whose heterocyclyl moiety is as defined above, and examples thereof include aziridinylsulfonylamino, oxiranylsulfonylamino, oxetanylsulfonylamino, thietanylsulfonylamino, tetrahydrofuranylsulfonylamino, tetrahydropyranylsulfonylamino, azetidinylsulfonylamino, pyrrolidinylsulfonylamino, piperidinylsulfonylamino, piperazinylsulfonylamino, dihydroisoxazolylsulfonylamino, dioxaborolanylsulfonylamino, dioxolanylsulfonylamino, dioxanylsulfonylamino, and morpholinylsulfonylamino groups.

In the present invention, unless otherwise specified, the "5- to 12-membered heteroarylsulfonylamino" refers to a (5- to 12-membered heteroaryl)-S(=O)$_2$-NH- group whose heteroaryl moiety is as defined above, and examples thereof include pyridylsulfonylamino, pyrimidinylsulfonylamino, pyrazinylsulfonylamino, pyridadinylsulfonylamino, triazinylsulfonylamino, thienylsulfonylamino, furanylsulfonylamino, pyrrolylsulfonylamino, pyrazolylsulfonylamino, thiazolylsulfonylamino, isothiazolylsulfonylamino, oxazolylsulfonylamino, isoxazolylsulfonylamino, imidazolylsulfonylamino, triazolylsulfonylamino, thiadiazolylsulfonylamino, oxadiazolylsulfonylamino, tetrazolylsulfonylamino, indolylsulfonylamino, benzoimidazolylsulfonylamino, indazolylsulfonylamino, benzotriazolylsulfonylamino, isoquinolinylsulfonylamino, quinolinylsulfonylamino, benzothiazolylsulfonylamino, benzofuranylsulfonylamino, benzothienylsulfonylamino, benzoisooxazolylsulfonylamino, pyrazolopyridylsulfonylamino, pyrazolopyrimidinylsulfonylamino, pyrrolopyridylsulfonylamino, triazolopyridylsulfonylamino, triazolopyrimidinylsulfonylamino, quinazolinylsulfonylamino, quinoxalinylsulfonylamino, and cinnolinylsulfonylamino groups.

In the present invention, unless otherwise specified, the "(C1-C6)alkylaminosulfonyl" refers to a ((C1-C6)alkyl)-NH-S(=O)$_2$- group whose alkyl moiety is as defined above, and examples thereof include methylaminosulfonyl, ethylaminosulfonyl, n-propylaminosulfonyl, isopropylaminosulfonyl, and tert-butylaminosulfonyl groups.

In the present invention, unless otherwise specified, the "(C2-C6)alkenylaminosulfonyl" refers to a ((C2-C6)alkenyl)-NH-S(=O)$_2$- group whose alkenyl moiety is as defined above, and examples thereof include vinylaminosulfonyl, 1-propenylaminosulfonyl, isopropenylaminosulfonyl, 2-propenylaminosulfonyl, 1-methyl-2-propenylaminosulfonyl, 2-methyl-2-propenylaminosulfonyl, 2-butenylaminosulfonyl, 3-butenylaminosulfonyl, 3-methyl-2-butenylaminosulfonyl, 2-methyl-2-butenylaminosulfonyl, 2-pentenylaminosulfonyl, 3-pentenylaminosulfonyl, 4-pentenylaminosulfonyl, 2-hexenylaminosulfonyl, 3-hexenylaminosulfonyl, 4-hexenylaminosulfonyl, and 5-hexenylaminosulfonyl groups.

In the present invention, unless otherwise specified, the "(C2-C6)alkynylaminosulfonyl" refers to a ((C2-C6)alkynyl)-NH-S(=O)$_2$- group whose alkynyl moiety is as defined above, and examples thereof include ethynylaminosulfonyl, 1-propynylaminosulfonyl, 2-propynylaminosulfonyl, 1-methyl-2-propynylaminosulfonyl, 1-ethyl-2-propynylaminosulfonyl, 1-butynylaminosulfonyl, 2-butynylaminosulfonyl, 3-butynylaminosulfonyl, 1-methyl-2-butynylaminosulfonyl, 1-pentynylaminosulfonyl, 2-pentynylaminosulfonyl, 3-pentynylaminosulfonyl, 4-pentynylaminosulfonyl, and 4,4-dimethyl-2-pentynylaminosulfonyl groups.

In the present invention, unless otherwise specified, the "(C3-C6)cycloalkylaminosulfonyl" refers to a ((C3-C6)cycloalkyl)-NH-S(=O)$_2$- group whose cycloalkyl moiety is as defined above, and examples thereof include cyclopropylaminosulfonyl, cyclobutylaminosulfonyl, cyclopentylaminosulfonyl, and cyclohexylaminosulfonyl groups.

In the present invention, unless otherwise specified, the "(C6-C12)arylaminosulfonyl" refers to a ((C6-C12)aryl)-NH-S(=O)$_2$- group whose aryl moiety is as defined above, and examples thereof include phenylaminosulfonyl, biphenylaminosulfonyl, naphthalen-1-ylaminosulfonyl, and naphthalen-2-ylaminosulfonyl groups.

In the present invention, unless otherwise specified, the "3- to 6-membered heterocyclylaminosulfonyl" refers to a (3-to 6-membered heterocyclyl)-NH-S(=O)$_2$- group whose heterocyclyl moiety is as defined above, and examples thereof include aziridinylaminosulfonyl, oxiranylaminosulfonyl, oxetanylaminosulfonyl, thietanylaminosulfonyl, tetrahydrofuranylaminosulfonyl, tetrahydropyranylaminosulfonyl, azetidinylaminosulfonyl, pyrrolidinylaminosulfonyl, piperidinylaminosulfonyl, piperazinylaminosulfonyl, dihydroisoxazolylaminosulfonyl, dioxaborolanylaminosulfonyl, dioxolanylaminosulfonyll, dioxanylaminosulfonyl, and morpholinylaminosulfonyl groups.

In the present invention, unless otherwise specified, the "5- to 12-membered heteroarylaminosulfonyl" refers to a (5- to 12-membered heteroaryl)-NH-S(=O)$_2$- group whose heteroaryl moiety is as defined above, and examples thereof include pyridylaminosulfonyl, pyrimidinylaminosulfonyl, pyrazinylaminosulfonyl, pyridadinylaminosulfonyl, triazinylaminosulfonyl, thienylaminosulfonyl, furanylaminosulfonyl, pyrrolylaminosulfonyl, pyrazolylaminosulfonyl, thiazolylaminosulfonyl, isothiazolylaminosulfonyl, oxazolylaminosulfonyl, isoxazolylaminosulfonyl, imidazolylaminosulfonyl, triazolylaminosulfonyl, thiadiazolylaminosulfonyl, oxadiazolylaminosulfonyl, tetrazolylaminosulfonyl, indolylaminosulfonyl, benzoimidazolylaminosulfonyl, indazolylaminosulfonyl, benzotriazolylaminosulfonyl, isoquinolinylaminosulfonyl, quinolinylaminosulfonyl, benzothiazolylaminosulfonyl, benzofuranylaminosulfonyl, benzothienylaminosulfonyl, benzoisooxazolylaminosulfonyl, pyrazolopyridylaminosulfonyl, pyrazolopyrimidinylaminosulfonyl, pyrrolopyridylaminosulfonyl, triazolopyridylaminosulfonyl, triazolopyrimidinylaminosulfonyl, quinazolinylaminosulfonyl, quinoxalinylaminosulfonyl, and cinnolinylaminosulfonyl groups.

In the present invention, unless otherwise specified, "(C1-C6)alkylcarbonylthio" refers to a ((C1-C6)alkyl)-C(=O)-S-group whose alkyl moiety is as defined above, and examples thereof include methylcarbonylthio, ethylcarbonylthio, n-propylcarbonylthio, isopropylcarbonylthio, n-butylcarbonylthio, isobutylcarbonylthio, sec-butylcarbonylthio, and tert-butylcarbonylthio groups.

In the present invention, unless otherwise specified, the "(C1-C6)alkylthio group" refers to a ((C1-C6)alkyl)-S-group whose alkyl moiety is as defined above, and examples thereof include methylthio, ethylthio, n-propylthio, isopropylthio, n-butylthio, isobutylthio, sec-butylthio, and tert-butylthio groups.

In the present invention, unless otherwise specified, the "(C1-C6)alkylsulfinyl" refers to a ($C_1$-$C_6$ alkyl)-S(=O)-group whose alkyl moiety is as defined above, and examples thereof include methylsulfinyl, ethylsulfinyl, n-propylsulfinyl, isopropylsulfinyl, n-butylsulfinyl, isobutylsulfinyl, sec-butylsulfinyl, and tert-butylsulfinyl groups.

In the present invention, unless otherwise specified, the "(C1-C6)alkylsulfonyl" refers to a ((C1-C6)alkyl)-S(=O)$_2$-group whose alkyl moiety is as defined above, and examples thereof include methylsulfonyl, ethylsulfonyl, n-propylsulfonyl, isopropylsulfonyl, n-butylsulfonyl, isobutylsulfonyl, sec-butylsulfonyl, and tert-butylsulfonyl groups.

In the present invention, unless otherwise specified, the "(C1-C6)haloalkylthio" refers to a ((C1-C6)haloalkyl)-S-group whose haloalkyl moiety is as defined above, and examples thereof include fluoromethylthio, difluoromethylthio, trifluoromethylthio, trichloromethylthio, 2,2,2-trifluoroethylthio, pentafluoroethylthio, 2,2,2-trichloroethylthio, 3,3,3-trifluoropropylthio, 1,1,2,3,3,3-hexafluoropropyl, heptafluoropropylthio, 1,1,1,3,3,3-hexafluoropropan-2-ylthio, heptafluoropropan-2-ylthio, and 4,4,4-trifluorobutylthio groups.

In the present invention, unless otherwise specified, the "(C1-C6)haloalkylsulfinyl" refers to a ((C1-C6)haloalkyl)-S(=O)- group whose haloalkyl moiety is as defined above, and examples thereof include difluoromethylsulfinyl, trifluoromethylsulfinyl, trichloromethylsulfinyl, 2,2,2-trifluoroethylsulfinyl, 2,2,2-trichloroethylsulfinyl, pentafluoroethylsulfinyl, 3,3,3-trifluoropropylsulfinyl, heptafluoropropylsulfinyl, and heptafluoro-2-propylsulfinyl groups.

In the present invention, unless otherwise specified, the "(C1-C6)haloalkylsulfonyl" refers to a ($C_1$-$C_6$ haloalkyl)-S(=O)$_2$- group whose haloalkyl moiety is as defined above, and examples thereof include difluoromethylsulfonyl, trifluoromethylsulfonyl, trichloromethylsulfonyl, 2,2,2-trifluoroethylsulfonyl, pentafluoroethylsulfonyl, 3,3,3-trifluoropropylsulfonyl, heptafluoropropylsulfonyl, and heptafluoro-2-propylsulfonyl groups.

In the present invention, unless otherwise specified, the "(C2-C6)alkenylthio" refers to a ((C2-C6)alkenyl)-S-group whose alkenyl moiety is as defined above, and examples thereof include vinylthio, 1-propenylthio, isopropenylthio, 2-propenylthio, 1-methyl-2-propenylthio, 2-methyl-2-propenylthio, 2-butenylthio, 3-butenylthio, 3-methyl-2-butenylthio, 2-methyl-2-butenylthio, 2-pentenylthio, 3-pentenylthio, 4-pentenylthio, 2-hexenylthio, 3-hexenylthio, 4-hexenylthio, and 5-hexenylthio groups.

In the present invention, unless otherwise specified, the "(C2-C6)alkenylsulfinyl" refers to a ((C2-C6)alkenyl)-S(=O)-group whose alkenyl moiety is as defined above, and examples thereof include vinylsulfinyl, 1-propenylsulfinyl, isopropenylsulfinyl, 2-propenylsulfinyl, 1-methyl-2-propenylsulfinyl, 2-methyl-2-propenylsulfinyl, 2-butenylsulfinyl, 3-butenylsulfinyl, 3-methyl-2-butenylsulfinyl, 2-methyl-2-butenylsulfinyl, 2-pentenylsulfinyl, 3-pentenylsulfinyl, 4-pentenylsulfinyl, 2-hexenylsulfinyl, 3-hexenylsulfinyl, 4-hexenylsulfinyl, and 5-hexenylsulfinyl groups.

In the present invention, unless otherwise specified, the "(C2-C6)alkenylsulfonyl" refers to a ((C2-C6)alkenyl)-S(=O)$_2$-group whose alkenyl moiety is as defined above, and examples thereof include vinylsulfonyl, 1-propenylsulfonyl, isopropenylsulfonyl, 2-propenylsulfonyl, 1-methyl-2-propenylsulfonyl, 2-methyl-2-propenylsulfonyl, 2-butenylsulfonyl, 3-butenylsulfonyl, 3-methyl-2-butenylsulfonyl, 2-methyl-2-butenylsulfonyl, 2-pentenylsulfonyl, 3-pentenylsulfonyl, 4-pentenylsulfonyl, 2-hexenylsulfonyl, 3-hexenylsulfonyl, 4-hexenylsulfonyl, and 5-hexenylsulfonyl groups.

In the present invention, unless otherwise specified, the "(C2-C6)alkynylthio" refers to a ((C2-C6)alkynyl)-S-group whose alkynyl moiety is as defined above, and examples thereof include ethynylthio, 1-propynylthio, 2-propynylthio, 1-methyl-2-propynylthio, 1-ethyl-2-propynylthio, 1-butynylthio, 2-butynylthio, 3-butynylthio, 1-methyl-2-butynylthio, 1-pentynylthio, 2-pentynylthio, 3-pentynylthio, 4-pentynylthio, and 4,4-dimethyl-2-pentynylthio groups.

In the present invention, unless otherwise specified, the "(C2-C6)alkynylsulfinyl" refers to a ((C2-C6)alkynyl)-S(=O)-group whose alkynyl moiety is as defined above, and examples thereof include ethynylsulfinyl, 1-propynylsulfinyl, 2-propynylsulfinyl, 1-methyl-2-propynylsulfinyl, 1-ethyl-2-propynylsulfinyl, 1-butynylsulfinyl, 2-butynylsulfinyl, 3-butynylsulfinyl, 1-methyl-2-butynylsulfinyl, 1-pentynylsulfinyl, 2-pentynylsulfinyl, 3-pentynylsulfinyl, 4-pentynylsulfinyl, and 4,4-dimethyl-2-pentynylsulfinyl groups.

In the present invention, unless otherwise specified, the "(C2-C6)alkynylsulfonyl" refers to a ($C_2$-$C_6$ alky-

nyl)-$S(=O)_2$-group whose alkynyl moiety is as defined above, and examples thereof include ethynylsulfonyl, 1-propynylsulfonyl, 2-propynylsulfonyl, 1-methyl-2-propynylsulfonyl, 1-ethyl-2-propynylsulfonyl, 1-butynylsulfonyl, 2-butynylsulfonyl, 3-butynylsulfonyl, 1-methyl-2-butynylsulfonyl, 1-pentynylsulfonyl, 2-pentynylsulfonyl, 3-pentynylsulfonyl, 4-pentynylsulfonyl, and 4,4-dimethyl-2-pentynylsulfonyl groups.

In the present invention, unless otherwise specified, the "(C3-C6)cycloalkylthio" refers to a ((C3-C6)cycloalkyl)-S-group whose cycloalkyl moiety is as defined above, and examples thereof include cyclopropylthio, cyclobutylthio, cyclopentylthio, and cyclohexylthio groups.

In the present invention, unless otherwise specified, the "(C3-C6)cycloalkylsulfinyl" refers to a ((C3-C6)cycloalkyl)-S(=O)- group whose cycloalkyl moiety is as defined above, and examples thereof include cyclopropylsulfinyl, cyclobutylsulfinyl, cyclopentylsulfinyl, and cyclohexylsulfinyl groups.

In the present invention, unless otherwise specified, the "(C3-C6)cycloalkylsulfonyl" refers to a ((C3-C6)cycloalkyl)-$S(=O)_2$- group whose cycloalkyl moiety is as defined above, and examples thereof include cyclopropylsulfonyl, cyclobutylsulfonyl, cyclopentylsulfonyl, and cyclohexylsulfonyl groups.

In the present invention, unless otherwise specified, the "(C6-C12)aryloxy" refers to a ((C6-C12)aryl)-O- group whose aryl moiety is as defined above, and examples thereof include phenyloxy, biphenyloxy, naphthalen-1-yloxy, and naphthalen-2-yloxy groups.

In the present invention, unless otherwise specified, the "(C6-C12)arylthio" refers to a ((C6-C12)aryl)-S- group whose aryl moiety is as defined above, and examples thereof include phenylthio, biphenylthio, naphthalen-1-ylthio, and naphthalen-2-ylthio groups.

In the present invention, unless otherwise specified, the "(C6-C12)arylsulfinyl" refers to a ((C6-C12)aryl)-S(=O)- group whose aryl moiety is as defined above, and examples thereof include phenylsulfinyl, biphenylsulfinyl, naphthalen-1-ylsulfinyl, and naphthalen-2-ylsulfinyl groups.

In the present invention, unless otherwise specified, the "(C6-C12)arylsulfonyl" refers to a ((C6-C12)aryl)-$S(=O)_2$- group whose aryl moiety is as defined above, and examples thereof include phenylsulfonyl, biphenylsulfonyl, naphthalen-1-ylsulfonyl, and naphthalen-2-ylsulfonyl groups.

In the present invention, unless otherwise specified, the "(C6-C12)arylamino" refers to a ((C6-C12)aryl)-NH- group whose aryl moiety is as defined above, and examples thereof include phenylamino, biphenylamino, naphthalen-1-ylamino, and naphthalen-2-ylamino groups.

In the present invention, unless otherwise specified, the "3- to 6-membered heterocyclyloxy" refers to a (3- to 6-membered heterocyclyl)-O- group whose heterocyclyl moiety is as defined above, and examples thereof include aziridinyloxy, oxylanyloxy, oxetanyloxy, thietanyloxy, tetrahydrofuranyloxy, tetrahydropyranyloxy, azetidinyloxy, pyrrolidinyloxy, piperidinyloxy, piperazinyloxy, dihydroisoxazolyloxy, dioxaborolanyloxy, dioxolanyloxy, dioxanyloxy, and morpholinyloxy groups.

In the present invention, unless otherwise specified, the "3- to 6-membered heterocyclylthio" refers to a (3- to 6-membered heterocyclyl)-S- group whose heterocyclyl moiety is as defined above, and examples thereof include aziridinylthio, oxylanylthio, oxetanylthio, thietanylthio, tetrahydrofuranylthio, tetrahydropyranylthio, azetidinylthio, pyrrolidinylthio, piperidinylthio, piperazinylthio, dihydroisoxazolylthio, dioxaborolanylthio, dioxolanylthio, dioxanylthio, and morpholinylthio groups.

In the present invention, unless otherwise specified, the "3- to 6-membered heterocyclylsulfinyl" refers to a (3- to 6-membered heterocyclyl)-S(=O)- group whose heterocyclyl moiety is as defined above, and examples thereof include aziridinylsulfinyl, oxylanylsulfinyl, oxetanylsulfinyl, thietanylsulfinyl, tetrahydrofuranylsulfinyl, tetrahydropyranylsulfinyl, azetidinylsulfinyl, pyrrolidinylsulfinyl, piperidinylsulfinyl, piperazinylsulfinyl, dihydroisoxazolylsulfinyl, dioxaborolanylsulfinyl, dioxolanylsulfinyl, dioxanylsulfinyl, and morpholinylsulfinyl groups.

In the present invention, unless otherwise specified, the "3- to 6-membered heterocyclylsulfonyl" refers to a (3- to 6-membered heterocyclyl)-$S(=O)_2$- group whose heterocyclyl moiety is as defined above, and examples thereof include aziridinylsulfonyl, oxylanylsulfonyl, oxetanylsulfonyl, thietanylsulfonyl, tetrahydrofuranylsulfonyl, tetrahydropyranylsulfonyl, azetidinylsulfonyl, pyrrolidinylsulfonyl, piperidinylsulfonyl, piperazinylsulfonyl, dihydroisoxazolylsulfonyl, dioxaborolanylsulfonyl, dioxolanylsulfonyl, dioxanylsulfonyl, and morpholinylsulfonyl groups.

In the present invention, unless otherwise specified, the "3- to 6-membered heterocyclylamino" refers to a (3- to 6-membered heterocyclyl)-NH- group whose heterocyclyl moiety is as defined above, and examples thereof include aziridinylamino, oxylanylamino, oxetanylamino, thietanylamino, tetrahydrofuranylamino, tetrahydropyranylamino, azetidinylamino, pyrrolidinylamino, piperidinylamino, piperazinylamino, dihydroisoxazolylamino, dioxaborolanylamino, dioxolanylamino, dioxanylamino, and morpholinylamino groups.

In the present invention, unless otherwise specified, the "5- to 12-membered heteroaryloxy" refers to a (5- to 12-membered heteroaryl)-O- group whose heteroaryl moiety is as defined above, and examples thereof include pyridyloxy, pyrimidinyloxy, pyrazinyloxy, pyridadinyloxy, triazinyloxy, thienyloxy, furanyloxy, pyrrolyloxy, pyrazolyloxy, thiazolyloxy, isothiazolyloxy, oxazolyloxy, isoxazolyloxy, imidazolyloxy, triazolyloxy, thiadiazolyloxy, oxadiazolyloxy, tetrazolyloxy, indolyloxy, benzoimidazolyloxy, indazolyloxy, benzotriazolyloxy, isoquinolinyloxy, quinolinyloxy, benzothiazolyloxy,

benzofuranyloxy, benzothienyloxy, benzoisooxazolyloxy, pyrazolopyridyloxy, pyrazolopyrimidinyloxy, pyrrolopyridyloxy, triazolopyridyloxy, triazolopyrimidinyloxy, quinazolinyloxy, quinoxalinyloxy, and cinnolinyloxy groups.

In the present invention, unless otherwise specified, the "5- to 12-membered heteroarylthio" refers to a (5- to 12-membered heteroaryl)-S- group whose heteroaryl moiety is as defined above, and examples thereof include pyridylthio, pyrimidinylthio, pyrazinylthio, pyridadinylthio, triazinylthio, thienylthio, furanylthio, pyrrolylthio, pyrazolylthio, thiazolylthio, isothiazolylthio, oxazolylthio, isoxazolylthio, imidazolylthio, triazolylthio, thiadiazolylthio, oxadiazolylthio, tetrazolylthio, indolylthio, benzoimidazolylthio, indazolylthio, benzotriazolylthio, isoquinolinylthio, quinolinylthio, benzothiazolylthio, benzofuranylthio, benzothienylthio, benzoisooxazolylthio, pyrazolopyridylthio, pyrazolopyrimidinylthio, pyrrolopyridylthio, triazolopyridylthio, triazolopyrimidinylthio, quinazolinylthio, quinoxalinylthio, and cinnolinylthio groups.

In the present invention, unless otherwise specified, the "5- to 12-membered heteroarylsulfinyl" refers to a (5- to 12-membered heteroaryl)-S(=O)- group whose heteroaryl moiety is as defined above, and examples thereof include pyridylsulfinyl, pyrimidinylsulfinyl, pyrazinylsulfinyl, pyridadinylsulfinyl, triazinylsulfinyl, thienylsulfinyl, furanylsulfinyl, pyrrolylsulfinyl, pyrazolylsulfinyl, thiazolylsulfinyl, isothiazolylsulfinyl, oxazolylsulfinyl, isoxazolylsulfinyl, imidazolylsulfinyl, triazolylsulfinyl, thiadiazolylsulfinyl, oxadiazolylsulfinyl, tetrazolylsulfinyl, indolylsulfinyl, benzoimidazolylsulfinyl, indazolylsulfinyl, benzotriazolylsulfinyl, isoquinolinylsulfinyl, quinolinylsulfinyl, benzothiazolylsulfinyl, benzofuranylsulfinyl, benzothienylsulfinyl, benzoisoxazolylsulfinyl, pyrazolopyridylsulfinyl, pyrazolopyrimidinylsulfinyl, pyrrolopyridylsulfinyl, triazolopyridylsulfinyl, triazolopyrimidinylsulfinyl, quinazolinylsulfinyl, quinoxalinylsulfinyl, and cinnolinylsulfinyl groups.

In the present invention, unless otherwise specified, the "5- to 12-membered heteroarylsulfonyl" refers to a (5- to 12-membered heteroaryl)-$S(=O)_2$- group whose heteroaryl moiety is as defined above, and examples thereof include pyridylsulfonyl, pyrimidinylsulfonyl, pyrazinylsulfonyl, pyridadinylsulfonyl, triazinylsulfonyl, thienylsulfonyl, furanylsulfonyl, pyrrolylsulfonyl, pyrazolylsulfonyl, thiazolylsulfonyl, isothiazolylsulfonyl, oxazolylsulfonyl, isoxazolylsulfonyl, imidazolylsulfonyl, triazolylsulfonyl, thiadiazolylsulfonyl, oxadiazolylsulfonyl, tetrazolylsulfonyl, indolylsulfonyl, benzoimidazolylsulfonyl, indazolylsulfonyl, benzotriazolylsulfonyl, isoquinolinylsulfonyl, quinolinylsulfonyl, benzothiazolylsulfonyl, benzofuranylsulfonyl, benzothienylsulfonyl, benzoisooxazolylsulfonyl, pyrazolopyridylsulfonyl, pyrazolopyrimidinylsulfonyl, pyrrolopyridylsulfonyl, triazolopyridylsulfonyl, triazolopyrimidinylsulfonyl, quinazolinylsulfonyl, quinoxalinylsulfonyl, and cinnolinylsulfonyl groups.

In the present invention, unless otherwise specified, the "5- to 12-membered heteroarylamino" refers to a (5- to 12-membered heteroaryl)-NH- group whose heteroaryl moiety is as defined above, and examples thereof include pyridylamino, pyrimidinylamino, pyrazinylamino, pyridadinylamino, triazinylamino, thienylamino, furanylamino, pyrrolylamino, pyrazolylamino, thiazolylamino, isothiazolylamino, oxazolylamino, isoxazolylamino, imidazolylamino, triazolylamino, thiadiazolylamino, oxadiazolylamino, tetrazolylamino, indolylamino, benzoimidazolylamino, indazolylamino, benzotriazolylamino, isoquinolinylamino, quinolinylamino, benzothiazolylamino, benzofuranylamino, benzothienylamino, benzoisooxazolylamino, pyrazolopyridylamino, pyrazolopyrimidinylamino, pyrrolopyridylamino, triazolopyridylamino, triazolopyrimidinylamino, quinazolinylamino, quinoxalinylamino, and cinnolinylamino groups.

In the present invention, unless otherwise specified, the "(C2-C6)alkenyloxyimino (C1-C6)alkyl" refers to a ((C2-C6)alkenyl)-O-N=((C1-C6)alkyl) group whose alkenyl moiety and alkyl moiety are as defined above, and examples thereof include 1-(vinyloxyimino)methyl, 1-(vinyloxyimino)ethyl, 1-(vinyloxyimino)propyl, 1-(vinyloxyimino)butyl, 1-(2-propenyloxyimino)methyl, 1-(2-propenyloxyimino)ethyl, 1-(2-propenyloxyimino)propyl, and 1-(2-propenyloxyimino)butyl groups.

In the present invention, unless otherwise specified, the "(C2-C6)alkynyloxyimino (C1-C6)alkyl" refers to a ((C2-C6)alkynyl)-O-N=((C1-C6)alkyl) group whose alkynyl moiety and alkyl moiety are as defined above, and examples thereof include 1-(ethynyloxyimino)methyl, 1-(2-propynyloxyimino)methyl, 1-(ethynyloxyimino)ethyl, 1-(2-propynyloxyimino)ethyl, 1-(2-propynyloxyimino)propyl, and 1-(ethynyloxyimino)propyl groups.

In the present invention, unless otherwise specified, the "(C3-C6)cycloalkyloxyimino (C1-C6)alkyl group" refers to a ((C3-C6)cycloalkyl)-O-N=((C1-C6)alkyl) group whose cycloalkyl moiety and alkyl moiety are as defined above, and examples thereof include 1-(cyclopropyloxyimino)methyl, 1-(cyclopropyloxyimino)ethyl, 2-(cyclopropyloxyimino)ethyl, 1-(cyclopropyloxyimino)propyl, and 2-(cyclopropyloxyimino)propyl groups.

In the present invention, unless otherwise specified, the "(C6-C12)aryloxyimino (C1-C6)alkyl" refers to a ((C6-C12)aryl)-O-N=((C1-C6)alkyl) group whose aryl moiety and alkyl moiety are as defined above, and examples thereof include 1-(phenoxyimino)methyl, 1-(phenoxyimino)ethyl, and 2-(phenoxyimino)ethyl groups.

In the present invention, unless otherwise specified, the "3- to 6-membered heterocyclyloxyimino (C1-C6)alkyl" refers to a (3- to 6-membered heterocyclyl)-O-N=((C1-C6)alkyl) group whose heterocyclyl moiety and alkyl moiety are as defined above, and examples thereof include 1-(aziridinyloxyimino)ethyl, 1-(oxylanyloxyimino)ethyl, 1-(oxetanyloxyimino)ethyl, 1-(thietanyloxyimino)ethyl, 1-(tetrahydrofuranyloxyimino)ethyl, 1-(tetrahydropyranyloxyimino)ethyl, 1-(azetidinyloxyimino)ethyl, 1-(pyrrolidinyloxyimino)ethyl, 1-(piperidinyloxyimino)ethyl, and 1-(piperazinyloxyimino)ethyl groups.

In the present invention, unless otherwise specified, the "5- to 12-membered heteroaryloxyimino (C1-C6)alkyl" refers to a (5- to 12-membered heteroaryl)-O-N=((C1-C6)alkyl) group whose heteroaryl moiety and alkyl moiety are as

defined above, and examples thereof include 1-(pyridyloxyimino)ethyl, 1-(pyrimidinyloxyimino)ethyl, 1-(pyrazinyloxyimino)ethyl, 1-(pyridadinyloxyimino)ethyl, 1-(triazinyloxyimino)ethyl, 1-(thienyloxyimino)ethyl, 1-(furanyloxyimino)ethyl, 1-(pyrrolyloxyimino)ethyl, 1-(pyrazolyloxyimino)ethyl, 1-(thiazolyloxyimino)ethyl, 1-(isothiazolyloxyimino)ethyl, 1-(oxazolyloxyimino)ethyl, 1-(isoxazolyloxyimino)ethyl, 1-(imidazolyloxyimino)ethyl, 1-(triazolyloxyimino)ethyl, 1-(thiadiazolyloxyimino)ethyl, 1-(oxadiazolyloxyimino)ethyl, and 1-(tetrazolyloxyimino)ethyl groups.

In the present invention, unless otherwise specified, the "(C2-C6)alkenyloxycarbonyloxy" refers to a ((C2-C6)alkenyl)-O-C(=O)-O- group whose alkenyl moiety is as defined above, and examples thereof include vinyloxycarbonyloxy, 1-propenyloxycarbonyloxy, isopropenyloxycarbonyloxy, 2-propenyloxycarbonyloxy, 1-methyl-2-propenyloxycarbonyloxy, 2-methyl-2-propenyloxycarbonyloxy, 2-butenyloxycarbonyloxy, 3-butenyloxycarbonyloxy, 3-methyl-2-butenyloxycarbonyloxy, 2-methyl-2-butenyloxycarbonyloxy, 2-pentenyloxycarbonyloxy, 3-pentenyloxycarbonyloxy, 4-pentenyloxycarbonyloxy, 2-hexenyloxycarbonyloxy, 3-hexenyloxycarbonyloxy, 4-hexenyloxycarbonyloxy, and 5-hexenyloxycarbonyloxy groups.

In the present invention, unless otherwise specified, the "(C2-C6)alkynyloxycarbonyloxy" refers to a ((C2-C6)alkynyl)-O-C(=O)-O- group whose alkynyl moiety is as defined above, and examples thereof include ethynyloxycarbonyloxy, 1-propynyloxycarbonyloxy, 2-propynyloxycarbonyloxy, 1-methyl-2-propynyloxycarbonyloxy, 1-ethyl-2-propynyloxycarbonyloxy, 1-butynyloxycarbonyloxy, 2-butynyloxycarbonyloxy, 3-butynyloxycarbonyloxy, 1-methyl-2-butynyloxycarbonyloxy, 1-pentynyloxycarbonyloxy, 2-pentynyloxycarbonyloxy, 3-pentynyloxycarbonyloxy, 4-pentynyloxycarbonyloxy, and 4,4-dimethyl-2-pentynyloxycarbonyloxy groups.

In the present invention, unless otherwise specified, the "(C3-C6)cycloalkyloxycarbonyloxy" refers to a ((C3-C6)cycloalkyl)-O-C(=O)-O- group whose cycloalkyl moiety is as defined above, and examples thereof include cyclopropyloxycarbonyloxy, cyclobutyloxycarbonyloxy, cyclopentyloxycarbonyloxy, and cyclohexyloxycarbonyloxy groups.

In the present invention, unless otherwise specified, the "(C6-C12)aryloxycarbonyloxy" refers to a ((C6-C12)aryl)-O-C(=O)-O- group whose aryl moiety is as defined above, and examples thereof include phenyloxycarbonyl, biphenyloxycarbonyloxy, naphthalen-1-yloxycarbonyloxy, and naphthalen-2-yloxycarbonyloxy groups.

In the present invention, unless otherwise specified, the "3- to 6-membered heterocyclyloxycarbonyloxy" refers to a (3-to 6-membered heterocyclyl)-O-C(=O)-O- group whose heterocyclyl moiety is as defined above, and examples thereof include aziridinyloxycarbonyloxy, oxiranyloxycarbonyloxy, oxetanyloxycarbonyloxy, thietanyloxycarbonyloxy, tetrahydrofuranyloxycarbonyloxy, tetrahydropyranyloxycarbonyloxy, azetidinyloxycarbonyloxy, pyrrolidinyloxycarbonyloxy, piperidinyloxycarbonyloxy, piperazinyloxycarbonyloxy, dihydroisoxazolyloxycarbonyloxy, dioxaborolanyloxycarbonyloxy, dioxolanyloxycarbonyloxy, dioxanyloxycarbonyloxy, and morpholinyloxycarbonyloxy groups.

In the present invention, unless otherwise specified, the "5- to 12-membered heteroaryloxycarbonyloxy" refers to a (5-to 12-membered heteroaryl)-O-C(=O)-O- group whose heteroaryl moiety is as defined above, and examples thereof include pyridyloxycarbonyloxy, pyrimidinyloxycarbonyloxy, pyrazinyloxycarbonyloxy, pyridadinyloxycarbonyloxy, triazinyloxycarbonyloxy, thienyloxycarbonyloxy, furanyloxycarbonyloxy, pyrrolyloxycarbonyloxy, pyrazolyloxycarbonyloxy, thiazolyloxycarbonyloxy, isothiazolyloxycarbonyloxy, oxazolyloxycarbonyloxy, isoxazolyloxycarbonyloxy, imidazolyloxycarbonyloxy, triazolyloxycarbonyloxy, thiadiazolyloxycarbonyloxy, oxadiazolyloxycarbonyloxy, tetrazolyloxycarbonyloxy, indolyloxycarbonyloxy, benzoimidazolyloxycarbonyloxy, indazolyloxycarbonyloxy, benzotriazolyloxycarbonyloxy, isoquinolinyloxycarbonyloxy, quinolinyloxycarbonyloxy, benzothiazolyloxycarbonyloxy, benzofuranyloxycarbonyloxy, benzothienyloxycarbonyloxy, benzoisooxazolyloxycarbonyloxy, pyrazolopyridyloxycarbonyloxy, pyrazolopyrimidinyloxycarbonyloxy, pyrrolopyridyloxycarbonyloxy, triazolopyridyloxycarbonyloxy, triazolopyrimidinyloxycarbonyloxy, quinazolinyloxycarbonyloxy, quinoxalinyloxycarbonyloxy, and cinnolinyloxycarbonyloxy groups.

In the present invention, unless otherwise specified, the "(C1-C6)alkylaminocarbonylamino" refers to a (C1-C6)alkyl)-NH-C(=O)-NH- group whose alkyl moiety is as defined above, and examples thereof include methylaminocarbonylamino, ethylaminocarbonylamino, n-propylaminocarbonylamino, isopropylaminocarbonylamino, and tert-butylaminocarbonylamino groups.

In the present invention, unless otherwise specified, the "(C2-C6)alkenylaminocarbonylamino" refers to a ((C2-C6)alkenyl)-NH-C(=O)-NH- group whose alkenyl moiety is as defined above, and examples thereof include vinylaminocarbonylamino, 1-propenylaminocarbonylamino, isopropenylaminocarbonylamino, 2-propenylaminocarbonylamino, 1-methyl-2-propenylaminocarbonylamino, 2-methyl-2-propenylaminocarbonylamino, 2-butenylaminocarbonylamino, 3-butenylaminocarbonylamino, 3-methyl-2-butenylaminocarbonylamino, 2-methyl-2-butenylaminocarbonylamino, 2-pentenylaminocarbonylamino, 3-pentenylaminocarbonylamino, 4-pentenylaminocarbonylamino, 2-hexenylaminocarbonylamino, 3-hexenylaminocarbonylamino, 4-hexenylaminocarbonylamino, and 5-hexenylaminocarbonylamino groups.

In the present invention, unless otherwise specified, the "(C2-C6)alkynylaminocarbonylamino" refers to a ((C2-C6)alkynyl)-NH-C(=O)-NH- group whose alkynyl moiety is as defined above, and examples thereof include ethynylaminocarbonylamino, 1-propynylaminocarbonylamino, 2-propynylaminocarbonylamino, 1-methyl-2-propynylaminocarbonylamino, 1-ethyl-2-propynylaminocarbonylamino, 1-butynylaminocarbonylamino, 2-butynylaminocarbonylamino, 3-butynylaminocarbonylamino, 1-methyl-2-butynylaminocarbonylamino, 1-pentynylaminocarbonylamino, 2-pentynylaminocarbonylamino, 3-pentynylaminocarbonylamino, 4-pentynylaminocarbonylamino, and 4,4-dimethyl-2-pentynylaminocarbonylamino groups.

In the present invention, unless otherwise specified, the "(C3-C6)cycloalkylaminocarbonylamino" refers to a ((C3-C6)cycloalkyl)-NH-C(=O)-NH- group whose cycloalkyl moiety is as defined above, and examples thereof include cyclopropylaminocarbonylamino, cyclobutylaminocarbonylamino, cyclopentylaminocarbonylamino, and cyclohexylaminocarbonylamino groups.

In the present invention, unless otherwise specified, the "(C6-C12)arylaminocarbonylamino" refers to a ((C6-C12)aryl)-NH-C(=O)-NH- group whose aryl moiety is as defined above, and examples thereof include phenylaminocarbonylamino, biphenylaminocarbonylamino, naphthalen-1-ylaminocarbonylamino, and naphthalen-2-ylaminocarbonylamino groups.

In the present invention, unless otherwise specified, the "3- to 6-membered heterocyclylaminocarbonylamino" refers to a (3- to 6-membered heterocyclyl)-NH-C(=O)-NH- group whose heterocyclyl moiety is as defined above, and examples thereof include aziridinylaminocarbonylamino, oxiranylaminocarbonylamino, oxetanylaminocarbonylamino, thietanylaminocarbonylamino, tetrahydrofuranylaminocarbonylamino, tetrahydropyranylaminocarbonylamino, azetidinylaminocarbonylamino, pyrrolidinylaminocarbonylamino, piperidinylaminocarbonylamino, piperazinylaminocarbonylamino, dihydroisoxazolylaminocarbonylamino, dioxaborolanylaminocarbonylamino, dioxolanylaminocarbonylamino, dioxanylaminocarbonylamino, and morpholinylaminocarbonylamino groups.

In the present invention, unless otherwise specified, the "5- to 12-membered heteroarylaminocarbonylamino" refers to a (5- to 12-membered heteroaryl)-NH-C(=O)-NH- group whose heteroaryl moiety is as defined above, and examples thereof include pyridylaminocarbonylamino, pyrimidinylaminocarbonylamino, pyrazinylaminocarbonylamino, pyridadinylaminocarbonylamino, triazinylaminocarbonylamino, thienylaminocarbonylamino, furanylaminocarbonylamino, pyrrolylaminocarbonylamino, pyrazolylaminocarbonylamino, thiazolylaminocarbonylamino, isothiazolylaminocarbonylamino, oxazolylaminocarbonylamino, isoxazolylaminocarbonylamino, imidazolylaminocarbonylamino, triazolylaminocarbonylamino, thiadiazolylaminocarbonylamino, oxadiazolylaminocarbonylamino, tetrazolylaminocarbonylamino, indolylaminocarbonylamino, benzoimidazolylaminocarbonylamino, indazolylaminocarbonylamino, benzotriazolylaminocarbonylamino, isoquinolinylaminocarbonylamino, quinolinylaminocarbonylamino, benzothiazolylaminocarbonylamino, benzofuranylaminocarbonylamino, benzothienylaminocarbonylamino, benzoisooxazolylaminocarbonylamino, pyrazolopyridylaminocarbonylamino, pyrazolopyrimidinylaminocarbonylamino, pyrrolopyridylaminocarbonylamino, triazolopyridylaminocarbonylamino, triazolopyrimidinylaminocarbonylamino, quinazolinylaminocarbonylamino, quinoxalinylaminocarbonylamino, and cinnolinylaminocarbonylamino groups.

In the present invention, unless otherwise specified, the "(C1-C6)alkoxy (C1-C6)alkyl" refers to a ((C1-C6)alkoxy)-((C1-C6)alkyl)- group whose alkoxy moiety and alkyl moiety are as defined above, and examples thereof include methoxymethyl, ethoxymethyl, n-propoxymethyl, isopropoxymethyl, tert-butoxymethyl, 1-methoxyethyl, 1-methoxy-1-methylethyl, 2-methoxyethyl, 1-ethoxyethyl, 2-ethoxyethyl, 1-isopropoxyethyl, 1-methoxypropyl, 2-methoxypropyl, 1-ethoxypropyl, 1-methoxybutyl, and 1-ethoxybutyl groups.

In the present invention, unless otherwise specified, the "(C1-C6)haloalkoxy (C1-C6)alkyl" refers to a ((C1-C6)haloalkyl)-O-((C1-C6)alkyl)- group whose haloalkyl moiety and alkyl moiety are as defined above, and examples thereof include 2-(difluoromethoxy)ethyl, 2-(trifluoromethoxy)ethyl, 2-(2,2-difluoroethoxy)ethyl, 2-(2,2,2-trifluoroethoxy)ethyl, 2-(3,3-difluoropropioxy)ethyl, 2-(3,3,3-trifluoropropioxy)ethyl, 3-(difluoromethoxy)propyl, 3-(trifluoromethoxy)propyl, 3-(2,2-difluoroethoxy)propyl, 3-(2,2,2-trifluoroethoxy)propyl, 3-(3,3-difluoropropioxy)propyl, 3-(3,3,3-trifluoropropioxy)propyl, 4-(trifluoromethoxy)butyl, and 5-(trifluoromethoxy)pentyl groups.

In the present invention, unless otherwise specified, the "cyano (C1-C6)alkyl" refers to a (cyano)-((C1-C6)alkyl)-group whose alkyl moiety is as defined above, and examples thereof include cyanomethyl, 1-cyanoethyl, 2-cyanoethyl, 1-cyanopropyl, 3-cyanopropyl, 2-cyano-2-propyl, 1-cyanobutyl, 4-cyanobutyl, 5-cyanopentyl, and 6-cyanohexyl groups.

In the present invention, unless otherwise specified, the "cyano (C3-C6)cycloalkyl" refers to a (cyano)-((C3-C6)cycloalkyl)- group whose cycloalkyl moiety is as defined above, and examples thereof include 1-cyanocyclopropyl, 2-cyanocyclopropyl, 1-cyanomethylcyclopropyl, 1-cyanocyclobutyl, 3-cyanocyclobutyl, 1-cyanocyclopentyl, and 1-cyanocyclohexyl groups.

In the present invention, unless otherwise specified, the "(C1-C6)haloalkylamino" refers to a ((C1-C6)haloalkyl)-NH-group whose haloalkyl moiety is as defined above, and examples thereof include trifluoromethylamino, 2-fluoroethylamino, 2,2-difluoroethylamino, 2,2,2-trifluoroethylamino, 2,2,2-trichloroethylamino, pentafluoroethylamino, 3,3,3-trifluoropropylamino, and 1,1,1,3,3,3-hexafluoro-2-propylamino groups.

In the present invention, unless otherwise specified, the "(C1-C6)haloalkylcarbonyl" refers to a ((C1-C6)haloalkyl)-C(=O)- group whose haloalkyl moiety is as defined above, and examples thereof include fluoroacetyl, difluoroacetyl, trifluoroacetyl, chloroacetyl, trichloroacetyl, tribromoacetyl, 3,3,3-trifluoropropionyl, 3,3-difluoropropionyl, and 4,4,4-trifluorobutyryl groups.

In the present invention, unless otherwise specified, the "hydroxyimino (C1-C6)alkyl" refers to a HO-N=((C1-C6)alkyl) group whose alkyl moiety is as defined above, and examples thereof include 1-(hydroxyimino)methyl, 1-(hydroxyimino)ethyl, 2-(hydroxyimino)ethyl, 1-(hydroxyimino)propyl, 2-(hydroxyimino)propyl, and 3-(hydroxyimino)propyl

groups.

In the present invention, unless otherwise specified, the "(C1-C6)alkoxyimino (C1-C6)alkyl" refers to a ((C1-C6)alkyl)-O-N=((C1-C6)alkyl) group whose alkyl moiety is as defined above, and examples thereof include 1-(methoxyimino)methyl, 1-(methoxyimino)ethyl, 2-(methoxyimino)ethyl, 1-(methoxyimino)propyl, 2-(methoxyimino)propyl, 3-(methoxyimino)propyl, 1-(ethoxyimino)methyl, 1-(ethoxyimino)ethyl, 2-(ethoxyimino)ethyl, 1-(ethoxyimino)propyl, 2-(ethoxyimino)propyl, 3-(ethoxyimino)propyl, 1-(isopropoxyimino)ethyl, and 2-(isopropoxymino)ethyl groups.

In the present invention, unless otherwise specified, the "(C6-C12)aryl (C1-C6)alkyl" refers to a ((C6-C12)aryl)-((C1-C6)alkyl) group whose aryl moiety and alkyl moiety are as defined above, and examples thereof include benzyl, 1-phenylethyl, 2-phenylethyl, 1-phenylpropyl, 2-phenylpropyl, and 3-phenylpropyl groups.

In the present invention, unless otherwise specified, the "3- to 6-membered heterocyclyl (C1-C6)alkyl" refers to a (3-to 6-membered heterocyclyl)-((C1-C6)alkyl) group whose heterocyclyl moiety and alkyl moiety are as defined above, and examples thereof include aziridinylmethyl, oxylanylmethyl, oxetanylmethyl, thietanylmethyl, tetrahydrofuranylmethyl, tetrahydropyranylmethyl, azetidinylmethyl, pyrrolidinylmethyl, piperidinylmethyl, piperazinylmethyl, dihydroisoxazolylmethyl, dioxaborolanylmethyl, dioxolanylmethyl, dioxanylmethyl, and morpholinylmethyl groups.

In the present invention, unless otherwise specified, the "5- to 12-membered heteroaryl (C1-C6)alkyl" refers to a (5- to 12-membered heteroaryl)-((C1-C6)alkyl) group whose heteroaryl moiety and alkyl moiety are as defined above, and examples thereof include pyridylmethyl, pyrimidinylmethyl, pyrazinylmethyl, pyridadinylmethyl, triazinylmethyl, thienylmethyl, furanylmethyl, pyrrolylmethyl, pyrazolylmethyl, thiazolylmethyl, isothiazolylmethyl, oxazolylmethyl, isoxazolylmethyl, imidazolylmethyl, triazolylmethyl, thiadiazolylmethyl, oxadiazolylmethyl, tetrazolylmethyl, indolylmethyl, benzoimidazolylmethyl, indazolylmethyl, benzotriazolylmethyl, isoquinolinylmethyl, quinolinylmethyl, benzothiazolylmethyl, benzofuranylmethyl, benzothienylmethyl, benzoisooxazolylmethyl, pyrazolopyridylmethyl, pyrazolopyrimidinylmethyl, pyrrolopyridylmethyl, triazolopyridylmethyl, triazolopyrimidinylmethyl, quinazolinylmethyl, quinoxalinylmethyl, and cinnolinylmethyl groups.

In the present invention, unless otherwise specified, "(C1-C6)alkylsulfonyloxy" refers to a ((C1-C6)alkyl)-S(=O)$_2$-O- group whose alkyl moiety is as defined above, and examples thereof include methylsulfonyloxy, ethylsulfonyloxy, n-propylsulfonyloxy, isopropylsulfonyloxy, n-butylsulfonyloxy, isobutylsulfonyloxy, sec-butylsulfonyloxy, and tert-butylsulfonyloxy groups.

In the present invention, unless otherwise specified, the "(C1-C6)haloalkylsulfonyloxy" refers to a ((C1-C6)haloalkyl)-S(=O)$_2$-O- group whose haloalkyl moiety is as defined above, and examples thereof include difluoromethylsulfonyloxy, trifluoromethylsulfonyloxy, trichloromethylsulfonyloxy, chlorodifluoromethylsulfonyloxy, 2,2,2-trifluoroethylsulfonyloxy, 2,2,2-trichloroethylsulfonyloxy, pentafluoroethylsulfonyloxy, 3,3,3-trifluoropropylsulfonyloxy, heptafluoropropylsulfonyloxy, and heptafluoro-2-propylsulfonyloxy groups.

In the present invention, unless otherwise specified, "(C1-C6)alkoxycarbonyloxy" refers to a ((C1-C6)alkyl)-O-C(=O)-O-group whose alkyl moiety is as defined above, and examples thereof include methoxycarbonyloxy, ethoxycarbonyloxy, n-propoxycarbonyloxy, isopropoxycarbonyloxy, n-butoxycarbonyloxy, isobutoxycarbonyloxy, sec-butoxycarbonyloxy, tert-butoxycarbonyloxy, n-pentoxycarbonyloxy, 1-methylbutoxycarbonyloxy, 2-methylbutoxycarbonyloxy, 3-methylbutoxycarbonyloxy, 1-ethylpropoxycarbonyloxy, 1,1-dimethylpropoxycarbonyloxy, 1,2-dimethylpropoxycarbonyloxy, 2,2-dimethylpropoxycarbonyloxy, and n-hexoxycarbonyloxy groups.

In the present invention, unless otherwise specified, the "(C1-C6)haloalkylcarbonyloxy" refers to a ((C1-C6)haloalkyl)-C(=O)-O- group whose alkyl moiety is as defined above, and examples thereof include difluoromethylcarbonyloxy, trifluoromethylcarbonyloxy, trichloromethylcarbonyloxy, chlorodifluoromethylcarbonyloxy, 2,2,2-trifluoroethylcarbonyloxy, 2,2,2-trichloroethylcarbonyloxy, pentafluoroethylcarbonyloxy, 3,3,3-trifluoropropylcarbonyloxy, heptafluoropropylcarbonyloxy, and heptafluoro-2-propylcarbonyloxy groups.

In the present invention, unless otherwise specified, the "(C1-C6)haloalkoxycarbonyloxy" refers to a ((C1-C6)haloalkyl)-O-C(=O)-O- group whose haloalkyl moiety is as defined above, and examples thereof include difluoromethoxycarbonyloxy, dichloromethoxycarbonyloxy, trifluoromethoxycarbonyloxy, trichloromethoxycarbonyloxy, tribromomethoxycarbonyloxy, chlorodifluoromethoxycarbonyloxy, bromodifluoromethoxycarbonyloxy, 2-fluoroethoxycarbonyloxy, 1-chloroethoxycarbonyloxy, 2-chloroethoxycarbonyloxy, 1-bromoethoxycarbonyloxy, 2-bromoethoxycarbonyloxy, 2,2-difluoroethoxycarbonyloxy, 1,2-dichloroethoxycarbonyloxy, 2,2-dichloroethoxycarbonyloxy, 2,2,2-trifluoroethoxycarbonyloxy, 2,2,2-trichloroethoxycarbonyloxy, 1,1,2,2-tetrafluoroethoxycarbonyloxy, pentafluoroethoxycarbonyloxy, 2-bromo-2-chloroethoxycarbonyloxy, 2-chloro-1,1,2,2-tetrafluoroethoxycarbonyloxy, 1-chloro-1,2,2,2-tetrafluoroethoxycarbonyloxy, 1-chloropropoxycarbonyloxy, 2-chloropropoxycarbonyloxy, 3-chloropropoxycarbonyloxy, 2-bromopropoxycarbonyloxy, 3-bromopropoxycarbonyloxy, 2-bromo-1-methylethoxycarbonyloxy, 3-iodopropoxycarbonyloxy, 2,3-dichloropropoxycarbonyloxy, 2,3-dibromopropoxycarbonyloxy, 3,3,3-trifluoropropoxycarbonyloxy, 3,3,3-trifluoro-2-propoxycarbonyloxy, 3,3,3-trichloropropoxycarbonyloxy, 3-bromo-3,3-difluoropropoxycarbonyloxy, 2,2-difluoropropoxycarbonyloxy, 3,3-dichloro-3-fluoropropoxycarbonyloxy, 2,2,3,3-tetrafluoropropoxycarbonyloxy, 1-bromo-3,3,3-trifluoropropoxycarbonyloxy, 2,2,3,3,3-pentafluoropropoxycarbonyloxy, 2,2,2-trifluoro-1-trifluoromethylethoxycarbonyloxy, heptafluoropropoxycarbonyloxy, heptafluoro-2-propoxycarbonyloxy, 1,2,2,2-tetrafluoro-1-trifluoromethylethoxycarbonyloxy, 1,1,2,3,3,3-hexafluoropropoxycarbonyloxy, 2-chlorobutoxycarbonyloxy, 3-chlorobutoxycarbonyloxy, 4-chlorobutoxycarbonyloxy, 2-chloro-1,1-dimethylethoxycarbonyloxy, 4-bromobutoxycarbonyloxy, 3-bromo-2-methylpropoxycarbonyloxy, 2-bromo-1,1-dimethylethoxycarbonyloxy, 2,2-dichloro-1,1-dimethylethoxycarbonyloxy, 2-chloro-1-chloromethyl-2-methylethoxycarbonyloxy, 4,4,4-trifluorobutoxycarbonyloxy, 3,3,3-trifluoro-1-methylpropoxycarbonyloxy, 3,3,3-trifluoro-2-methylpropoxycarbonyloxy, 2,3,4-trichlorobutoxycarbonyloxy, 2,2,2-trichloro-1,1-dimethylethoxycarbonyloxy, 4-chloro-4,4-difluorobutoxycarbonyloxy, 4,4-dichloro-4-fluorobutoxycarbonyloxy, 4-bromo-4,4-difluorobutoxycarbonyloxy, 2,4-dibromo-4,4-difluorobutoxycarbonyloxy, 3,4-dichloro-3,4,4-trifluorobutoxycarbonyloxy, 3,3-dichloro-4,4,4-trifluorobutoxycarbonyloxy, 4-bromo-3,3,4,4-tetrafluorobutoxycarbonyloxy, 4-bromo-3-chloro-3,4,4-trifluorobutoxycarbonyloxy, 2,2,3,3,4,4-hexafluorobutoxycarbonyloxy, 2,2,3,4,4,4-hexafluorobutoxycarbonyloxy, 2,2,2-trifluoro-1-methyl-1-trifluoromethylethoxycarbonyloxy, 3,3,3-trifluoro-2-trifluoromethylpropoxycarbonyloxy, 2,2,3,3,4,4,4-heptafluorobutoxycarbonyloxy, 3,3,4,4,4-pentafluoro-2-butoxycarbonyloxy, 2,3,3,3-tetrafluoro-2-trifluoromethylpropoxycarbonyloxy, 1,1,2,2,3,3,4,4-octafluorobutoxycarbonyloxy, nonafluorobutoxycarbonyloxy, perfluoro-tert-butoxycarbonyloxy, 4-chloro-1,1,2,2,3,3,4,4-octafluorobutoxycarbonyloxy, 5,5,5-trifluoropentoxycarbonyloxy, 4,4,5,5,5-pentafluoropentoxycarbonyloxy, 3,3,4,4,5,5,5-heptafluoropentoxycarbonyloxy, 3,3,4,4,5,5,5-heptafluoro-2-pentoxycarbonyloxy, 2,2,3,3,4,4,5,5,5-nonafluoropentoxycarbonyloxy, 2,2,3,3,4,4,5,5-octafluoropentoxycarbonyloxy, perfluoropentoxycarbonyloxy, 4,4,5,5,5-pentafluoro-2-butoxycarbonyloxy, 2,2-bis(trifluoromethyl)propoxycarbonyloxy, 2,2,3,3,4,4,5,5,6,6,6-undecafluorohexyloxycarbonyloxy, 3,3,4,4,5,5,6,6,6-nonafluorohexyloxycarbonyloxy, 4,4,5,5,6,6,6-heptafluorohexyloxycarbonyloxy, 2,2,3,3,4,4,5,5,6,6-decafluorohexyloxycarbonyloxy, 4,4,4-trifluoro-3,3-bis(trifluoromethyl)butyloxycarbonyloxy, and perfluorohexyloxycarbonyloxy groups.

In the present invention, the expression of "is optionally mono-substituted or poly-substituted with $R^4$" refers to "is optionally substituted with one or two or more $R^4$". In a case of being substituted with two or more $R^4$, the two or more $R^4$ may be the same as or different from each other, and $R^4$ each independently represent a hydrogen atom, a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety

is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

In the present invention, the expression of "is optionally mono-substituted or poly-substituted with $R^5$" refers to "is optionally substituted with one or two or more $R^5$". In a case of being substituted with two or more $R^5$, the two or more $R^5$ may be the same as or different from each other, and $R^5$ each independently represent a hydrogen atom, a halogen atom, a cyano, a nitro, an amino, a hydroxy, a thiol, a formyl, an oxo, a (C1-C6)alkyl, a (C2-C6)alkenyl, a (C2-C6)alkynyl, a (C3-C6)cycloalkyl, a (C1-C6)haloalkyl, a (C1-C6)alkoxy, a (C1-C6)haloalkoxy, a (C1-C6)alkoxy (C1-C6)alkyl, a (C1-C6)haloalkoxy (C1-C6)alkyl, a cyano (C1-C6)alkyl, a cyano (C3-C6)cycloalkyl, a (C1-C6)alkylthio, a (C1-C6)haloalkylthio, a (C1-C6)alkylsulfinyl, a (C1-C6)haloalkylsulfinyl, a (C1-C6)alkylsulfonyl, a (C1-C6)haloalkylsulfonyl, a (C1-C6)alkylcarbonylthio, a (C1-C6)alkylamino (the amino moiety is optionally substituted with $R^6$), a (C1-C6)haloalkylamino (the amino moiety is optionally substituted with $R^6$), a formylamino (the amino moiety is optionally substituted with $R^6$), a (C1-C6)alkylcarbonylamino (the amino moiety is optionally substituted with $R^6$), a carbamoyl, a (C1-C6)alkylaminocarbonyl (the amino moiety is optionally substituted with $R^6$), a carboxyl, a (C1-C6)alkylcarbonyl, a (C1-C6)haloalkylcarbonyl, a (C1-C6)alkoxycarbonyl, a hydroxyimino (C1-C6)alkyl, a (C1-C6)alkoxyimino (C1-C6)alkyl, a (C6-C12)aryl (the (C6-C12)aryl is optionally mono-substituted or poly-substituted with $R^7$), a (C6-C12)aryl (C1-C6)alkyl (the (C6-C12)aryl moiety is optionally mono-substituted or poly-substituted with $R^7$), a (C6-C12)arylcarbonyl group (the (C6-C12)aryl moiety may be mono-substituted or poly-substituted with $R^7$), a (C6-C12)aryloxy (the (C6-C12)aryl moiety is optionally mono-substituted or poly-substituted with $R^7$), a (C6-C12)arylthio group (the (C6-C12)aryl moiety is optionally mono-substituted or poly-substituted with $R^7$), a (C6-C12)arylsulfinyl (the (C6-C12)aryl moiety is optionally mono-substituted or poly-substituted with $R^7$), a (C6-C12)arylsulfonyl (the (C6-C12)aryl moiety is optionally mono-substituted or poly-substituted with $R^7$), a (C6-C12)arylamino (the (C6-C12)aryl moiety is optionally mono-substituted or poly-substituted with $R^7$, and the amino moiety is optionally substituted with $R^6$), a 3- to 6-membered heterocyclyl (the 3- to 6-membered heterocyclyl is optionally mono-substituted or poly-substituted with $R^7$), a 3- to 6-membered heterocyclyl (C1-C6)alkyl (the 3- to 6-membered heterocyclyl moiety is optionally mono-substituted or poly-substituted with $R^7$), a 3- to 6-membered heterocyclylcarbonyl (the 3- to 6-membered heterocyclyl moiety is optionally mono-substituted or poly-substituted with $R^7$), a 3- to 6-membered heterocyclyloxy (the 3- to 6-membered heterocyclyl moiety is optionally mono-substituted or poly-substituted with $R^7$), a 3-to 6-membered heterocyclylthio (the 3- to 6-membered heterocyclyl moiety is optionally mono-substituted or poly-substituted with $R^7$), a 3- to 6-membered heterocyclylsulfinyl (the 3- to 6-membered heterocyclyl moiety is optionally mono-substituted or poly-substituted with $R^7$), 3- to 6-membered heterocyclylsulfonyl (the 3- to 6-membered heterocyclyl moiety is optionally mono-substituted or poly-substituted with $R^7$), a 3- to 6-membered heterocyclylamino (the 3- to 6-membered heterocyclyl moiety is optionally mono-substituted or poly-substituted with $R^7$, and the amino moiety is optionally substituted with $R^6$), a 5- to 12-membered heteroaryl (the 5- to 12-membered heteroaryl is optionally mono-substituted or poly-substituted with $R^7$), a 5- to 12-membered heteroaryl (C1-C6)alkyl (the 5- to 12-membered heteroaryl moiety is optionally mono-substituted or poly-substituted with $R^7$), a 5-to 12-membered heteroarylcarbonyl (the 5- to 12-membered heteroaryl moiety is optionally mono-substituted or poly-substituted with $R^7$), a 5- to 12-membered heteroaryloxy (the 5-to 12-membered heteroaryl moiety is optionally mono-substituted or poly-substituted with $R^7$), a 5- to 12-membered heteroarylthio (the 5- to 12-membered heteroaryl moiety is optionally mono-substituted

or poly-substituted with $R^7$), a 5-to 12-membered heteroarylsulfinyl (the 5- to 12-membered heteroaryl moiety is optionally mono-substituted or poly-substituted with $R^7$), a 5- to 12-membered heteroarylsulfonyl (the 5- to 12-membered heteroaryl moiety is optionally mono-substituted or poly-substituted with $R^7$), or a 5- to 12-membered heteroarylamino (the 5- to 12-membered heteroaryl moiety is optionally mono-substituted or poly-substituted with $R^7$, and the amino moiety is optionally substituted with $R^6$).

In the present invention, the expression of "is optionally mono-substituted or poly-substituted with $R^7$" refers to "is optionally substituted with one or two or more $R^7$". In a case of being substituted with two or more $R^7$, the two or more $R^7$ may be the same as or different from each other, and $R^7$ each independently represent a hydrogen atom, a halogen atom, a cyano, a (C1-C6)alkyl, or a (C1-C6)alkoxy.

In the present invention, the expression of "is optionally mono-substituted or poly-substituted with $R^8$" refers to "is optionally substituted with one or two or more $R^8$". In a case of being substituted with two or more $R^8$, the two or more $R^8$ may be the same as or different from each other, and $R^8$ each independently represent a halogen atom, a cyano, a (C1-C6)haloalkyl, a (C3-C6)cycloalkyl, a (C1-C6)alkoxy, a (C1-C6)alkylcarbonyl, a (C1-C6)alkoxycarbonyl, a (C1-C6)haloalkoxycarbonyl, a (C6-C12)aryl (the (C6-C12)aryl is optionally mono-substituted or poly-substituted with $R^7$), a 3-to 6-membered heterocyclyl (the 3- to 6-membered heterocyclyl is optionally mono-substituted or poly-substituted with $R^7$), or a 5- to 12-membered heteroaryl (the 5- to 12-membered heteroaryl is optionally mono-substituted or poly-substituted with $R^7$) .

In the present invention, the expression of "mono-substituted or poly-substituted with substituent(s) selected from a substituent group $R^4$" refers to "substituted with one or two or more substituents selected from a substituent group $R^4$". In a case of being substituted with two or more substituents selected from the substituent group $R^4$, the two or more substituents selected from the substituent group $R^4$ may be the same as or different from each other, and $R^4$ each independently represent a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is

optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$),
a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3-to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a

(C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

In the present invention, the expression of "mono-substituted or poly-substituted with substituent(s) selected from a substituent group $R^{10}$" refers to "substituted with one or two or more substituents selected from a substituent group $R^{10}$". In a case of being substituted with two or more substituents selected from the substituent group $R^{10}$, the two or more substituents selected from the substituent group $R^{10}$ may be the same as or different from each other, and $R^{10}$ each independently represent a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally

substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

In the present invention, the expression of "mono-substituted or poly-substituted with substituent(s) selected from a substituent group $R^{11}$" refers to "substituted with one or two or more substituents selected from a substituent group $R^{11}$". In a case of being substituted with two or more substituents selected from the substituent group $R^{11}$, the two or more substituents selected from the substituent group $R^{11}$ may be the same as or different from each other, and $R^{11}$ each independently represent a halogen atom, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the

amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally sub-

stituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

In the present invention, the expression of "mono-substituted or poly-substituted with substituent(s) selected from a substituent group $R^{12}$" refers to "substituted with one or two or more substituents selected from a substituent group $R^{12}$". In a case of being substituted with two or more substituents selected from the substituent group $R^{12}$, the two or more substituents selected from the substituent group $R^{12}$ may be the same as or different from each other, and $R^{12}$ each independently represent a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3-to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3-to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5-to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

In the present invention, the agriculturally acceptable salt or salt means a salt of any compound of the present invention of formula [I], [II], or [III], which contains a nitrogen-containing heterocyclic group, a hydroxyl group, a carboxyl group, an amino group, or the like in a structure thereof, with metals or organic bases or mineral acids or organic acids. Examples of the metal include alkali metals such as sodium and potassium and alkaline earth metals such as magnesium and calcium, examples of the organic base include triethylamine and diisopropylamine, examples of the mineral acid include hydrochloric acid, hydrobromic acid, and sulfuric acid, and examples of the organic acid include formic acid, acetic acid, methanesulfonic acid, 4-toluenesulfonic acid, and trifluoromethanesulfonic acid.

Next, representative examples of the compound included in the formamide derivative of the present invention of formula [I] are shown in Tables 1 to 119, examples of the representative compounds included in the aniline derivative of the present invention of formula [II] are shown in Tables 120 to 160, and examples of the representative compounds included in the amidoxime derivative of the present invention of formula [III] are shown in Tables 161 to 202. However, the compounds included in the present invention are not limited thereto. In addition, the compound numbers in the tables are referred to in the following description.

The compounds included in the formamide derivative, the aniline derivative, or the amidoxime derivative of the present invention can have geometrical isomers of E-body and Z-body depending on the kind of the substituent, and the present invention encompasses such E-body, Z-body, or a mixture containing E-body and Z-body in any ratio. In addition, the compounds included in the present invention can have optical isomers caused by the existence of one or two or more asymmetric carbon atoms and asymmetric sulfur atoms, and the present invention encompasses any optically active substances, racemic bodies, or diastereomers.

In the present specification, the following notations in the tables represent the corresponding groups, respectively, as follows.

Me: methyl;
Et: ethyl;
n-Pr: n-propyl;
i-Pr: isopropyl;
n-Bu: n-butyl
i-Bu: isobutyl
s-Bu: sec-butyl;
t-Bu: tert-butyl;
n-Pen: n-pentyl;
n-Hex: n-hexyl;
c-Pr: cyclopropyl;
c-Bu: cyclobutyl;
c-Pen: cyclopentyl;
c-Hex: cyclohexyl;
c-Pr(2,2-$Cl_2$): 2,2-dichlorocyclopropyl;
$CF_3$: trifluoromethyl;
$CHF_2$: difluoromethyl;
COMe: acetyl;
$CO_2Me$: methyl ester;
TMS: trimethylsilyl;
Ph(2-F): 2-fluorophenyl;
Ph(2,3-$F_2$): 2,3-difluorophenyl;
pyridin-2-yl(3-F): 3-fluoro-pyridin-2-yl;

[Table 1]

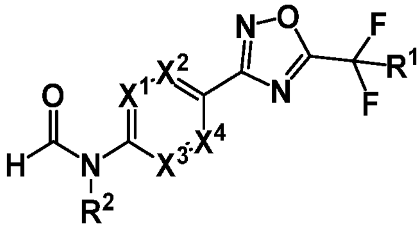

| Compound Number | $R^1$ | $X^1$ | $X^2$ | $X^3$ | $X^4$ | $R^2$ |
|---|---|---|---|---|---|---|
| A-0001 | F | CH | CH | CH | CH | H |
| A-0002 | F | CH | CH | CH | CH | Me |
| A-0003 | F | CH | CH | CH | CH | Et |
| A-0004 | F | CH | CH | CH | CH | n-Pr |
| A-0005 | F | CH | CH | CH | CH | i-Pr |
| A-0006 | F | CH | CH | CH | CH | n-Bu |
| A-0007 | F | CH | CH | CH | CH | i-Bu |
| A-0008 | F | CH | CH | CH | CH | s-Bu |
| A-0009 | F | CH | CH | CH | CH | t-Bu |
| A-0010 | F | CH | CH | CH | CH | $CH_2$t-Bu |
| A-0011 | F | CH | CH | CH | CH | n-Pen |
| A-0012 | F | CH | CH | CH | CH | n-Hex |
| A-0013 | F | CH | CH | CH | CH | $CH=CH_2$ |
| A-0014 | F | CH | CH | CH | CH | $CH_2CH=CH_2$ |
| A-0015 | F | CH | CH | CH | CH | $CH_2CH=CHCH_2Cl$ |

(continued)

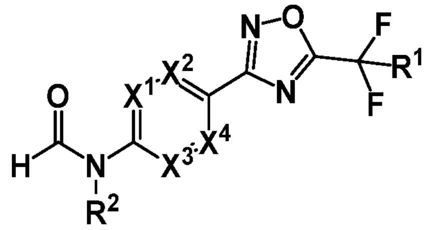

| Compound Number | $R^1$ | $X^1$ | $X^2$ | $X^3$ | $X^4$ | $R^2$ |
|---|---|---|---|---|---|---|
| A-0016 | F | CH | CH | CH | CH | $CH_2CH=CHCH_2NMe_2$ |
| A-0017 | F | CH | CH | CH | CH | $C\equiv CH$ |
| A-0018 | F | CH | CH | CH | CH | $CH_2C\equiv CH$ |
| A-0019 | F | CH | CH | CH | CH | c-Pr |
| A-0020 | F | CH | CH | CH | CH | c-Bu |
| A-0021 | F | CH | CH | CH | CH | c-Pen |
| A-0022 | F | CH | CH | CH | CH | c-Hex |
| A-0023 | F | CH | CH | CH | CH | $CH_2$c-Pr |
| A-0024 | F | CH | CH | CH | CH | $CH_2$c-Bu |
| A-0025 | F | CH | CH | CH | CH | $CH_2$c-Pen |
| A-0026 | F | CH | CH | CH | CH | $CH_2$c-Hex |
| A-0027 | F | CH | CH | CH | CH | $CH_2$c-Pr(2,2-$Cl_2$) |
| A-0028 | F | CH | CH | CH | CH | $CF_3$ |
| A-0029 | F | CH | CH | CH | CH | $CH_2CF_3$ |
| A-0030 | F | CH | CH | CH | CH | $CH_2CH_2CF_3$ |
| A-0031 | F | CH | CH | CH | CH | $CHF_2$ |
| A-0032 | F | CH | CH | CH | CH | $CH_2CHF_2$ |
| A-0033 | F | CH | CH | CH | CH | $CH_2CH_2CHF_2$ |
| A-0034 | F | CH | CH | CH | CH | $CH_2COMe$ |
| A-0035 | F | CH | CH | CH | CH | $CH_2CO_2Me$ |
| A-0036 | F | CH | CH | CH | CH | $CH(CH_3)CO_2Me$ |

[Table 2]

| Compound Number | $R^1$ | $X^1$ | $X^2$ | $X^3$ | $X^4$ | $R^2$ |
|---|---|---|---|---|---|---|
| A-0037 | F | CH | CH | CH | CH | $C(CH_3)(CH_3)CO_2Me$ |
| A-0038 | F | CH | CH | CH | CH | $CH_2CO_2Et$ |
| A-0039 | F | CH | CH | CH | CH | $CH(CH_3)CO_2Et$ |
| A-0040 | F | CH | CH | CH | CH | $C(CH_3)(CH_3)CO_2Et$ |
| A-0041 | F | CH | CH | CH | CH | $CH_2CONH_2$ |

(continued)

| Compound Number | $R^1$ | $X^1$ | $X^2$ | $X^3$ | $X^4$ | $R^2$ |
|---|---|---|---|---|---|---|
| A-0042 | F | CH | CH | CH | CH | $CH_2CONHMe$ |
| A-0043 | F | CH | CH | CH | CH | $CH(CH_3)CONHMe$ |
| A-0044 | F | CH | CH | CH | CH | $C(CH_3)(CH_3)CONHMe$ |
| A-0045 | F | CH | CH | CH | CH | $CH_2CONHEt$ |
| A-0046 | F | CH | CH | CH | CH | $CH(CH_3)CONHEt$ |
| A-0047 | F | CH | CH | CH | CH | $C(CH_3)(CH_3)CONHEt$ |
| A-0048 | F | CH | CH | CH | CH | $CH_2NHCHO$ |
| A-0049 | F | CH | CH | CH | CH | $CH(CH_3)NHCHO$ |
| A-0050 | F | CH | CH | CH | CH | $C(CH_3)(CH_3)NHCHO$ |
| A-0051 | F | CH | CH | CH | CH | $CH_2NHCOMe$ |
| A-0052 | F | CH | CH | CH | CH | $CH(CH_3)NHCOMe$ |
| A-0053 | F | CH | CH | CH | CH | $C(CH_3)(CH_3)NHCOMe$ |
| A-0054 | F | CH | CH | CH | CH | $CH_2NHCOEt$ |
| A-0055 | F | CH | CH | CH | CH | $CH(CH_3)NHCOEt$ |
| A-0056 | F | CH | CH | CH | CH | $C(CH_3)(CH_3)NHCOEt$ |
| A-0057 | F | CH | CH | CH | CH | $CH_2CN$ |
| A-0058 | F | CH | CH | CH | CH | $CH_2CH_2CN$ |
| A-0059 | F | CH | CH | CH | CH | $CH_2$c-Pr(1-CN) |
| A-0060 | F | CH | CH | CH | CH | $CH_2SCN$ |
| A-0061 | F | CH | CH | CH | CH | $CH_2CH_2SCN$ |
| A-0062 | F | CH | CH | CH | CH | $CH_2CH_2CH_2CH_2CH_2SCN$ |
| A-0063 | F | CH | CH | CH | CH | $CH_2CH_2CH_2CH_2CH_2CH_2SCN$ |
| A-0064 | F | CH | CH | CH | CH | $CH_2OCH_3$ |
| A-0065 | F | CH | CH | CH | CH | $CH_2OCH_2CH_3$ |
| A-0066 | F | CH | CH | CH | CH | $CH_2CH_2OCH_3$ |
| A-0067 | F | CH | CH | CH | CH | $CH_2CH_2OCH_2CH_3$ |
| A-0068 | F | CH | CH | CH | CH | $CH_2OCF_3$ |
| A-0069 | F | CH | CH | CH | CH | $CH_2OCH_2CF_3$ |
| A-0070 | F | CH | CH | CH | CH | $CH_2CH_2OCF_3$ |
| A-0071 | F | CH | CH | CH | CH | $CH_2CH_2OCH_2CF_3$ |
| A-0072 | F | CH | CH | CH | CH | $CH_2SCH_3$ |
| A-0073 | F | CH | CH | CH | CH | $CH_2SCH_2CH_3$ |
| A-0074 | F | CH | CH | CH | CH | $CH_2CH_2SCH_3$ |
| A-0075 | F | CH | CH | CH | CH | $CH_2CH_2SCH_2CH_3$ |

(continued)

| Compound Number | $R^1$ | $X^1$ | $X^2$ | $X^3$ | $X^4$ | $R^2$ |
|---|---|---|---|---|---|---|
| A-0076 | F | CH | CH | CH | CH | $CH_2SCF_3$ |
| A-0077 | F | CH | CH | CH | CH | $CH_2SCH_2CF_3$ |
| A-0078 | F | CH | CH | CH | CH | $CH_2CH_2SCF_3$ |

[Table 3]

| Compound Number | $R^1$ | $X^1$ | $X^2$ | $X^3$ | $X^4$ | $R^2$ |
|---|---|---|---|---|---|---|
| A-0079 | F | CH | CH | CH | CH | $CH_2CH_2SCH_2CF_3$ |
| A-0080 | F | CH | CH | CH | CH | $CH_2CH_2CH_2CH_2CH_2SCF_3$ |
| A-0081 | F | CH | CH | CH | CH | $CH_2CH_2CH_2CH_2CH_2CH_2SCF_3$ |
| A-0082 | F | CH | CH | CH | CH | Ph |
| A-0083 | F | CH | CH | CH | CH | pyridin-2-yl |
| A-0084 | F | CH | CH | CH | CH | pyridin-3-yl |
| A-0085 | F | CH | CH | CH | CH | pyridin-4-yl |
| A-0086 | F | CH | CH | CH | CH | pyrimidin-2-yl |
| A-0087 | F | CH | CH | CH | CH | pyrimidin-4-yl |
| A-0088 | F | CH | CH | CH | CH | pyrimidin-5-yl |
| A-0089 | F | CH | CH | CH | CH | pyridazin-3-yl |
| A-0090 | F | CH | CH | CH | CH | pyridazin-4-yl |
| A-0091 | F | CH | CH | CH | CH | pyrazin-2-yl |
| A-0092 | F | CH | CH | CH | CH | azetidin-3-yl |
| A-0093 | F | CH | CH | CH | CH | azetidin-3-yl(1-Me) |
| A-0094 | F | CH | CH | CH | CH | pyrrolidin-3-yl |
| A-0095 | F | CH | CH | CH | CH | pyrrolidin-3-yl(1-Me) |
| A-0096 | F | CH | CH | CH | CH | $CH_2Ph$ |
| A-0097 | F | CH | CH | CH | CH | $CH_2CH_2Ph$ |
| A-0098 | F | CH | CH | CH | CH | $CH_2CH_2CH_2Ph$ |
| A-0099 | F | CH | CH | CH | CH | $CH(CH_3)Ph$ |
| A-0100 | F | CH | CH | CH | CH | $C(CH_3)(CH_3)Ph$ |
| A-0101 | F | CH | CH | CH | CH | 1-Ph-c-Pr |
| A-0102 | F | CH | CH | CH | CH | 1-Ph-c-Bu |
| A-0103 | F | CH | CH | CH | CH | $CH_2COPh$ |
| A-0104 | F | CH | CH | CH | CH | $CH_2CO_2Ph$ |
| A-0105 | F | CH | CH | CH | CH | $CH_2CONHPh$ |
| A-0106 | F | CH | CH | CH | CH | $CH_2NHCOPh$ |
| A-0107 | F | CH | CH | CH | CH | $CH(CH_3)$pyridin-2-yl |

(continued)

| Compound Number | $R^1$ | $X^1$ | $X^2$ | $X^3$ | $X^4$ | $R^2$ |
|---|---|---|---|---|---|---|
| A-0108 | F | CH | CH | CH | CH | $C(CH_3)(CH_3)$pyridin-2-yl |
| A-0109 | F | CH | CH | CH | CH | 1-(pyridin-2-yl)-c-Pr |
| A-0110 | F | CH | CH | CH | CH | 1-(pyridin-2-yl)-c-Bu |
| A-0111 | F | CH | CH | CH | CH | $CH(CH_3)$pyridin-3-yl |
| A-0112 | F | CH | CH | CH | CH | $C(CH_3)(CH_3)$pyridin-3-yl |
| A-0113 | F | CH | CH | CH | CH | 1-(pyridin-3-yl)-c-Pr |
| A-0114 | F | CH | CH | CH | CH | 1-(pyridin-3-yl)-c-Bu |
| A-0115 | F | CH | CH | CH | CH | $CH(CH_3)$pyridin-4-yl |
| A-0116 | F | CH | CH | CH | CH | $C(CH_3)(CH_3)$pyridin-4-yl |
| A-0117 | F | CH | CH | CH | CH | 1-(pyridin-4-yl)-c-Pr |
| A-0118 | F | CH | CH | CH | CH | 1-(pyridin-4-yl)-c-Bu |
| A-0119 | F | CH | CH | CH | CH | $CH(CH_3)$pyrimidin-2-yl |
| A-0120 | F | CH | CH | CH | CH | $C(CH_3)(CH_3)$pyrimidin-2-yl |

[Table 4]

| Compound Number | $R^1$ | $X^1$ | $X^2$ | $X^3$ | $X^4$ | $R^2$ |
|---|---|---|---|---|---|---|
| A-0121 | F | CH | CH | CH | CH | 1-(pyrimidin-2-yl)-c-Pr |
| A-0122 | F | CH | CH | CH | CH | 1 -(pyrimidin-2-yl)-c-Bu |
| A-0123 | F | CH | CH | CH | CH | $CH(CH_3)$pyrimidin-4-yl |
| A-0124 | F | CH | CH | CH | CH | $C(CH_3)(CH_3)$pyrimidin-4-yl |
| A-0125 | F | CH | CH | CH | CH | 1-(pyrimidin-4-yl)-c-Pr |
| A-0126 | F | CH | CH | CH | CH | 1 -(pyrimidin-4-yl)-c-Bu |
| A-0127 | F | CH | CH | CH | CH | $CH(CH_3)$pyrimidin-5-yl |
| A-0128 | F | CH | CH | CH | CH | $C(CH_3)(CH_3)$pyrimidin-5-yl |
| A-0129 | F | CH | CH | CH | CH | 1-(pyrimidin-5-yl)-c-Pr |
| A-0130 | F | CH | CH | CH | CH | 1 -(pyrimidin-5-yl)-c-Bu |
| A-0131 | F | CH | CH | CH | CH | $CH(CH_3)$pyridazin-3-yl |
| A-0132 | F | CH | CH | CH | CH | $C(CH_3)(CH_3)$pyridazin-3-yl |
| A-0133 | F | CH | CH | CH | CH | 1-(pyridazin-3-yl)-c-Pr |
| A-0134 | F | CH | CH | CH | CH | 1-(pyridazin-3-yl)-c-Bu |
| A-0135 | F | CH | CH | CH | CH | $CH(CH_3)$pyridazin-4-yl |
| A-0136 | F | CH | CH | CH | CH | $C(CH_3)(CH_3)$pyridazin-4-yl |
| A-0137 | F | CH | CH | CH | CH | 1-(pyridazin-4-yl)-c-Pr |
| A-0138 | F | CH | CH | CH | CH | 1-(pyridazin-4-yl)-c-Bu |
| A-0139 | F | CH | CH | CH | CH | $CH(CH_3)$pyrazin-2-yl |

(continued)

| Compound Number | $R^1$ | $X^1$ | $X^2$ | $X^3$ | $X^4$ | $R^2$ |
|---|---|---|---|---|---|---|
| A-0140 | F | CH | CH | CH | CH | $C(CH_3)(CH_3)$pyrazin-2-yl |
| A-0141 | F | CH | CH | CH | CH | 1-(pyrazin-2-yl)-c-Pr |
| A-0142 | F | CH | CH | CH | CH | 1-(pyrazin-2-yl)-c-Bu |
| A-0143 | F | CH | CH | CH | CH | $CH_2OPh$ |
| A-0144 | F | CH | CH | CH | CH | $CH_2CH_2OPh$ |
| A-0145 | F | CH | CH | CH | CH | $CH(OH)Ph$ |
| A-0146 | F | CH | CH | CH | CH | $CH(CH_2OH)Ph$ |
| A-0147 | F | CH | CH | CH | CH | $CH_2CH(OH)Ph$ |
| A-0148 | F | CH | CH | CH | CH | $CH_2SPh$ |
| A-0149 | F | CH | CH | CH | CH | $CH_2SOPh$ |
| A-0150 | F | CH | CH | CH | CH | $CH_2SO_2Ph$ |
| A-0151 | F | CH | CH | CH | CH | $CH_2CH_2SPh$ |
| A-0152 | F | CH | CH | CH | CH | $CH_2CH_2SOPh$ |
| A-0153 | F | CH | CH | CH | CH | $CH_2CH_2SO_2Ph$ |
| A-0154 | F | CH | CH | CH | CH | $CH_2NHPh$ |
| A-0155 | F | CH | CH | CH | CH | $CH_2CH_2NHPh$ |
| A-0156 | F | CH | CH | CH | CH | $CH_2Ph(2\text{-}F)$ |
| A-0157 | F | CH | CH | CH | CH | $CH_2Ph(3\text{-}F)$ |
| A-0158 | F | CH | CH | CH | CH | $CH_2Ph(4\text{-}F)$ |
| A-0159 | F | CH | CH | CH | CH | $CH_2Ph(2\text{-}Cl)$ |
| A-0160 | F | CH | CH | CH | CH | $CH_2Ph(3\text{-}Cl)$ |
| A-0161 | F | CH | CH | CH | CH | $CH_2Ph(4\text{-}Cl)$ |
| A-0162 | F | CH | CH | CH | CH | $CH_2Ph(2\text{-}Br)$ |

[Table 5]

| Compound Number | $R^1$ | $X^1$ | $X^2$ | $X^3$ | $X^4$ | $R^2$ |
|---|---|---|---|---|---|---|
| A-0163 | F | CH | CH | CH | CH | $CH_2Ph(3\text{-}Br)$ |
| A-0164 | F | CH | CH | CH | CH | $CH_2Ph(4\text{-}Br)$ |
| A-0165 | F | CH | CH | CH | CH | $CH_2Ph(2\text{-}I)$ |
| A-0166 | F | CH | CH | CH | CH | $CH_2Ph(3\text{-}I)$ |
| A-0167 | F | CH | CH | CH | CH | $CH_2Ph(4\text{-}I)$ |
| A-0168 | F | CH | CH | CH | CH | $CH_2Ph(2\text{-}Me)$ |
| A-0169 | F | CH | CH | CH | CH | $CH_2Ph(3\text{-}Me)$ |
| A-0170 | F | CH | CH | CH | CH | $CH_2Ph(4\text{-}Me)$ |

(continued)

| Compound Number | $R^1$ | $X^1$ | $X^2$ | $X^3$ | $X^4$ | $R^2$ |
|---|---|---|---|---|---|---|
| A-0171 | F | CH | CH | CH | CH | $CH_2Ph(2\text{-}Et)$ |
| A-0172 | F | CH | CH | CH | CH | $CH_2Ph(3\text{-}Et)$ |
| A-0173 | F | CH | CH | CH | CH | $CH_2Ph(4\text{-}Et)$ |
| A-0174 | F | CH | CH | CH | CH | $CH_2Ph(2\text{-}n\text{-}Pr)$ |
| A-0175 | F | CH | CH | CH | CH | $CH_2Ph(3\text{-}n\text{-}Pr)$ |
| A-0176 | F | CH | CH | CH | CH | $CH_2Ph(4\text{-}n\text{-}Pr)$ |
| A-0177 | F | CH | CH | CH | CH | $CH_2Ph(2\text{-}i\text{-}Pr)$ |
| A-0178 | F | CH | CH | CH | CH | $CH_2Ph(3\text{-}i\text{-}Pr)$ |
| A-0179 | F | CH | CH | CH | CH | $CH_2Ph(4\text{-}i\text{-}Pr)$ |
| A-0180 | F | CH | CH | CH | CH | $CH_2Ph(2\text{-}s\text{-}Bu)$ |
| A-0181 | F | CH | CH | CH | CH | $CH_2Ph(3\text{-}s\text{-}Bu)$ |
| A-0182 | F | CH | CH | CH | CH | $CH_2Ph(4\text{-}s\text{-}Bu)$ |
| A-0183 | F | CH | CH | CH | CH | $CH_2Ph(2\text{-}i\text{-}Bu)$ |
| A-0184 | F | CH | CH | CH | CH | $CH_2Ph(3\text{-}i\text{-}Bu)$ |
| A-0185 | F | CH | CH | CH | CH | $CH_2Ph(4\text{-}i\text{-}Bu)$ |
| A-0186 | F | CH | CH | CH | CH | $CH_2Ph(2\text{-}t\text{-}Bu)$ |
| A-0187 | F | CH | CH | CH | CH | $CH_2Ph(3\text{-}t\text{-}Bu)$ |
| A-0188 | F | CH | CH | CH | CH | $CH_2Ph(4\text{-}t\text{-}Bu)$ |
| A-0189 | F | CH | CH | CH | CH | $CH_2Ph(2\text{-}CH{=}CH_2)$ |
| A-0190 | F | CH | CH | CH | CH | $CH_2Ph(3\text{-}CH{=}CH_2)$ |
| A-0191 | F | CH | CH | CH | CH | $CH_2Ph(4\text{-}CH{=}CH_2)$ |
| A-0192 | F | CH | CH | CH | CH | $CH_2Ph(2\text{-}CH_2CH{=}CH_2)$ |
| A-0193 | F | CH | CH | CH | CH | $CH_2Ph(3\text{-}CH_2CH{=}CH_2)$ |
| A-0194 | F | CH | CH | CH | CH | $CH_2Ph(4\text{-}CH_2CH{=}CH_2)$ |
| A-0195 | F | CH | CH | CH | CH | $CH_2Ph(2\text{-}CH{\equiv}CH)$ |
| A-0196 | F | CH | CH | CH | CH | $CH_2Ph(3\text{-}CH{\equiv}CH)$ |
| A-0197 | F | CH | CH | CH | CH | $CH_2Ph(4\text{-}CH{\equiv}CH)$ |
| A-0198 | F | CH | CH | CH | CH | $CH_2Ph(2\text{-}c\text{-}Pr)$ |
| A-0199 | F | CH | CH | CH | CH | $CH_2Ph(3\text{-}c\text{-}Pr)$ |
| A-0200 | F | CH | CH | CH | CH | $CH_2Ph(4\text{-}c\text{-}Pr)$ |
| A-0201 | F | CH | CH | CH | CH | $CH_2Ph(2\text{-}c\text{-}Bu)$ |
| A-0202 | F | CH | CH | CH | CH | $CH_2Ph(3\text{-}c\text{-}Bu)$ |
| A-0203 | F | CH | CH | CH | CH | $CH_2Ph(4\text{-}c\text{-}Bu)$ |
| A-0204 | F | CH | CH | CH | CH | $CH_2Ph(2\text{-}OMe)$ |

[Table 6]

| Compound Number | $R^1$ | $X^1$ | $X^2$ | $X^3$ | $X^4$ | $R^2$ |
|---|---|---|---|---|---|---|
| A-0205 | F | CH | CH | CH | CH | $CH_2Ph(3\text{-}OMe)$ |
| A-0206 | F | CH | CH | CH | CH | $CH_2Ph(4\text{-}OMe)$ |
| A-0207 | F | CH | CH | CH | CH | $CH_2Ph(2\text{-}OEt)$ |
| A-0208 | F | CH | CH | CH | CH | $CH_2Ph(3\text{-}OEt)$ |
| A-0209 | F | CH | CH | CH | CH | $CH_2Ph(4\text{-}OEt)$ |
| A-0210 | F | CH | CH | CH | CH | $CH_2Ph(2\text{-}On\text{-}Pr)$ |
| A-0211 | F | CH | CH | CH | CH | $CH_2Ph(3\text{-}On\text{-}Pr)$ |
| A-0212 | F | CH | CH | CH | CH | $CH_2Ph(4\text{-}On\text{-}Pr)$ |
| A-0213 | F | CH | CH | CH | CH | $CH_2Ph(2\text{-}Oi\text{-}Pr)$ |
| A-0214 | F | CH | CH | CH | CH | $CH_2Ph(3\text{-}Oi\text{-}Pr)$ |
| A-0215 | F | CH | CH | CH | CH | $CH_2Ph(4\text{-}Oi\text{-}Pr)$ |
| A-0216 | F | CH | CH | CH | CH | $CH_2Ph(2\text{-}OCF_3)$ |
| A-0217 | F | CH | CH | CH | CH | $CH_2Ph(3\text{-}OCF_3)$ |
| A-0218 | F | CH | CH | CH | CH | $CH_2Ph(4\text{-}OCF_3)$ |
| A-0219 | F | CH | CH | CH | CH | $CH_2Ph(2\text{-}OCHF_2)$ |
| A-0220 | F | CH | CH | CH | CH | $CH_2Ph(3\text{-}OCHF_2)$ |
| A-0221 | F | CH | CH | CH | CH | $CH_2Ph(4\text{-}OCHF_2)$ |
| A-0222 | F | CH | CH | CH | CH | $CH_2Ph(2\text{-}OCH_2CF_3)$ |
| A-0223 | F | CH | CH | CH | CH | $CH_2Ph(3\text{-}OCH_2CF_3)$ |
| A-0224 | F | CH | CH | CH | CH | $CH_2Ph(4\text{-}OCH_2CF_3)$ |
| A-0225 | F | CH | CH | CH | CH | $CH_2Ph(2\text{-}SMe)$ |
| A-0226 | F | CH | CH | CH | CH | $CH_2Ph(3\text{-}SMe)$ |
| A-0227 | F | CH | CH | CH | CH | $CH_2Ph(4\text{-}SMe)$ |
| A-0228 | F | CH | CH | CH | CH | $CH_2Ph(2\text{-}SEt)$ |
| A-0229 | F | CH | CH | CH | CH | $CH_2Ph(3\text{-}SEt)$ |
| A-0230 | F | CH | CH | CH | CH | $CH_2Ph(4\text{-}SEt)$ |
| A-0231 | F | CH | CH | CH | CH | $CH_2Ph(2\text{-}SCF_3)$ |
| A-0232 | F | CH | CH | CH | CH | $CH_2Ph(3\text{-}SCF_3)$ |
| A-0233 | F | CH | CH | CH | CH | $CH_2Ph(4\text{-}SCF_3)$ |
| A-0234 | F | CH | CH | CH | CH | $CH_2Ph(2\text{-}SOMe)$ |
| A-0235 | F | CH | CH | CH | CH | $CH_2Ph(3\text{-}SOMe)$ |
| A-0236 | F | CH | CH | CH | CH | $CH_2Ph(4\text{-}SOMe)$ |
| A-0237 | F | CH | CH | CH | CH | $CH_2Ph(2\text{-}SO_2Me)$ |
| A-0238 | F | CH | CH | CH | CH | $CH_2Ph(3\text{-}SO_2Me)$ |

(continued)

| Compound Number | $R^1$ | $X^1$ | $X^2$ | $X^3$ | $X^4$ | $R^2$ |
|---|---|---|---|---|---|---|
| A-0239 | F | CH | CH | CH | CH | $CH_2Ph(4-SO_2Me)$ |
| A-0240 | F | CH | CH | CH | CH | $CH_2Ph(2-CN)$ |
| A-0241 | F | CH | CH | CH | CH | $CH_2Ph(3-CN)$ |
| A-0242 | F | CH | CH | CH | CH | $CH_2Ph(4-CN)$ |
| A-0243 | F | CH | CH | CH | CH | $CH_2Ph(2-CH_2CN)$ |
| A-0244 | F | CH | CH | CH | CH | $CH_2Ph(3-CH_2CN)$ |
| A-0245 | F | CH | CH | CH | CH | $CH_2Ph(4-CH_2CN)$ |
| A-0246 | F | CH | CH | CH | CH | $CH_2Ph[2-(1-CN-c-Pr)]$ |

[Table 7]

| Compound Number | $R^1$ | $X^1$ | $X^2$ | $X^3$ | $X^4$ | $R^2$ |
|---|---|---|---|---|---|---|
| A-0247 | F | CH | CH | CH | CH | $CH_2Ph[3-(1-CN-c-Pr)]$ |
| A-0248 | F | CH | CH | CH | CH | $CH_2Ph[4-(1-CN-c-Pr)]$ |
| A-0249 | F | CH | CH | CH | CH | $CH_2Ph(2-CH_2OMe)$ |
| A-0250 | F | CH | CH | CH | CH | $CH_2Ph(3-CH_2OMe)$ |
| A-0251 | F | CH | CH | CH | CH | $CH_2Ph(4-CH_2OMe)$ |
| A-0252 | F | CH | CH | CH | CH | $CH_2Ph(2-CH_2OEt)$ |
| A-0253 | F | CH | CH | CH | CH | $CH_2Ph(3-CH_2OEt)$ |
| A-0254 | F | CH | CH | CH | CH | $CH_2Ph(4-CH_2OEt)$ |
| A-0255 | F | CH | CH | CH | CH | $CH_2Ph(2-CF_3)$ |
| A-0256 | F | CH | CH | CH | CH | $CH_2Ph(3-CF_3)$ |
| A-0257 | F | CH | CH | CH | CH | $CH_2Ph(4-CF_3)$ |
| A-0258 | F | CH | CH | CH | CH | $CH_2Ph(2-CHF_2)$ |
| A-0259 | F | CH | CH | CH | CH | $CH_2Ph(3-CHF_2)$ |
| A-0260 | F | CH | CH | CH | CH | $CH_2Ph(4-CHF_2)$ |
| A-0261 | F | CH | CH | CH | CH | $CH_2Ph(2-CH_2F)$ |
| A-0262 | F | CH | CH | CH | CH | $CH_2Ph(3-CH_2F)$ |
| A-0263 | F | CH | CH | CH | CH | $CH_2Ph(4-CH_2F)$ |
| A-0264 | F | CH | CH | CH | CH | $CH_2Ph(2-CF_2Cl)$ |
| A-0265 | F | CH | CH | CH | CH | $CH_2Ph(3-CF_2Cl)$ |
| A-0266 | F | CH | CH | CH | CH | $CH_2Ph(4-CF_2Cl)$ |
| A-0267 | F | CH | CH | CH | CH | $CH_2Ph[2-CF(CF_3)_2]$ |
| A-0268 | F | CH | CH | CH | CH | $CH_2Ph[3-CF(CF_3)_2]$ |
| A-0269 | F | CH | CH | CH | CH | $CH_2Ph[4-CF(CF_3)_2]$ |

(continued)

| Compound Number | $R^1$ | $X^1$ | $X^2$ | $X^3$ | $X^4$ | $R^2$ |
|---|---|---|---|---|---|---|
| A-0270 | F | CH | CH | CH | CH | $CH_2Ph(2\text{-}COMe)$ |
| A-0271 | F | CH | CH | CH | CH | $CH_2Ph(3\text{-}COMe)$ |
| A-0272 | F | CH | CH | CH | CH | $CH_2Ph(4\text{-}COMe)$ |
| A-0273 | F | CH | CH | CH | CH | $CH_2Ph(2\text{-}COEt)$ |
| A-0274 | F | CH | CH | CH | CH | $CH_2Ph(3\text{-}COEt)$ |
| A-0275 | F | CH | CH | CH | CH | $CH_2Ph(4\text{-}COEt)$ |
| A-0276 | F | CH | CH | CH | CH | $CH_2Ph(2\text{-}CO_2H)$ |
| A-0277 | F | CH | CH | CH | CH | $CH_2Ph(3\text{-}CO_2H)$ |
| A-0278 | F | CH | CH | CH | CH | $CH_2Ph(4\text{-}CO_2H)$ |
| A-0279 | F | CH | CH | CH | CH | $CH_2Ph(2\text{-}CO_2Me)$ |
| A-0280 | F | CH | CH | CH | CH | $CH_2Ph(3\text{-}CO_2Me)$ |
| A-0281 | F | CH | CH | CH | CH | $CH_2Ph(4\text{-}CO_2Me)$ |
| A-0282 | F | CH | CH | CH | CH | $CH_2Ph(2\text{-}CO_2Et)$ |
| A-0283 | F | CH | CH | CH | CH | $CH_2Ph(3\text{-}CO_2Et)$ |
| A-0284 | F | CH | CH | CH | CH | $CH_2Ph(4\text{-}CO_2Et)$ |
| A-0285 | F | CH | CH | CH | CH | $CH_2Ph(2\text{-}CONH_2)$ |
| A-0286 | F | CH | CH | CH | CH | $CH_2Ph(3\text{-}CONH_2)$ |
| A-0287 | F | CH | CH | CH | CH | $CH_2Ph(4\text{-}CONH_2)$ |
| A-0288 | F | CH | CH | CH | CH | $CH_2Ph(2\text{-}CONHMe)$ |

[Table 8]

| Compound Number | $R^1$ | $X^1$ | $X^2$ | $X^3$ | $X^4$ | $R^2$ |
|---|---|---|---|---|---|---|
| A-0289 | F | CH | CH | CH | CH | $CH_2Ph(3\text{-}CONHMe)$ |
| A-0290 | F | CH | CH | CH | CH | $CH_2Ph(4\text{-}CONHMe)$ |
| A-0291 | F | CH | CH | CH | CH | $CH_2Ph(2\text{-}CONHEt)$ |
| A-0292 | F | CH | CH | CH | CH | $CH_2Ph(3\text{-}CONHEt)$ |
| A-0293 | F | CH | CH | CH | CH | $CH_2Ph(4\text{-}CONHEt)$ |
| A-0294 | F | CH | CH | CH | CH | $CH_2Ph(2\text{-}NHCHO)$ |
| A-0295 | F | CH | CH | CH | CH | $CH_2Ph(3\text{-}NHCHO)$ |
| A-0296 | F | CH | CH | CH | CH | $CH_2Ph(4\text{-}NHCHO)$ |
| A-0297 | F | CH | CH | CH | CH | $CH_2Ph(2\text{-}NHCOMe)$ |
| A-0298 | F | CH | CH | CH | CH | $CH_2Ph(3\text{-}NHCOMe)$ |
| A-0299 | F | CH | CH | CH | CH | $CH_2Ph(4\text{-}NHCOMe)$ |
| A-0300 | F | CH | CH | CH | CH | $CH_2Ph(2\text{-}NHCOEt)$ |

(continued)

| Compound Number | $R^1$ | $X^1$ | $X^2$ | $X^3$ | $X^4$ | $R^2$ |
|---|---|---|---|---|---|---|
| A-0301 | F | CH | CH | CH | CH | $CH_2$Ph(3-NHCOEt) |
| A-0302 | F | CH | CH | CH | CH | $CH_2$Ph(4-NHCOEt) |
| A-0303 | F | CH | CH | CH | CH | $CH_2$Ph(2-CH=NOH) |
| A-0304 | F | CH | CH | CH | CH | $CH_2$Ph(3-CH=NOH) |
| A-0305 | F | CH | CH | CH | CH | $CH_2$Ph(4-CH=NOH) |
| A-0306 | F | CH | CH | CH | CH | $CH_2$Ph(2-CH=NOMe) |
| A-0307 | F | CH | CH | CH | CH | $CH_2$Ph(3-CH=NOMe) |
| A-0308 | F | CH | CH | CH | CH | $CH_2$Ph(4-CH=NOMe) |
| A-0309 | F | CH | CH | CH | CH | $CH_2$Ph(2-CMe=NOH) |
| A-0310 | F | CH | CH | CH | CH | $CH_2$Ph(3-CMe=NOH) |
| A-0311 | F | CH | CH | CH | CH | $CH_2$Ph(4-CMe=NOH) |
| A-0312 | F | CH | CH | CH | CH | $CH_2$Ph(2-CMe=NOMe) |
| A-0313 | F | CH | CH | CH | CH | $CH_2$Ph(3-CMe=NOMe) |
| A-0314 | F | CH | CH | CH | CH | $CH_2$Ph(4-CMe=NOMe) |
| A-0315 | F | CH | CH | CH | CH | $CH_2$Ph(2-$NO_2$) |
| A-0316 | F | CH | CH | CH | CH | $CH_2$Ph(3-$NO_2$) |
| A-0317 | F | CH | CH | CH | CH | $CH_2$Ph(4-$NO_2$) |
| A-0318 | F | CH | CH | CH | CH | $CH_2$Ph(2-$NH_2$) |
| A-0319 | F | CH | CH | CH | CH | $CH_2$Ph(3-$NH_2$) |
| A-0320 | F | CH | CH | CH | CH | $CH_2$Ph(4-$NH_2$) |
| A-0321 | F | CH | CH | CH | CH | $CH_2$Ph(2-OH) |
| A-0322 | F | CH | CH | CH | CH | $CH_2$Ph(3-OH) |
| A-0323 | F | CH | CH | CH | CH | $CH_2$Ph(4-OH) |
| A-0324 | F | CH | CH | CH | CH | $CH_2$Ph(2-SH) |
| A-0325 | F | CH | CH | CH | CH | $CH_2$Ph(3-SH) |
| A-0326 | F | CH | CH | CH | CH | $CH_2$Ph(4-SH) |
| A-0327 | F | CH | CH | CH | CH | $CH_2$Ph(2-$NMe_2$) |
| A-0328 | F | CH | CH | CH | CH | $CH_2$Ph(3-$NMe_2$) |
| A-0329 | F | CH | CH | CH | CH | $CH_2$Ph(4-$NMe_2$) |
| A-0330 | F | CH | CH | CH | CH | $CH_2$Ph(2-NHMe) |

[Table 9]

| Compound Number | $R^1$ | $X^1$ | $X^2$ | $X^3$ | $X^4$ | $R^2$ |
|---|---|---|---|---|---|---|
| A-0331 | F | CH | CH | CH | CH | $CH_2$Ph(3-NHMe) |

(continued)

| Compound Number | $R^1$ | $X^1$ | $X^2$ | $X^3$ | $X^4$ | $R^2$ |
|---|---|---|---|---|---|---|
| A-0332 | F | CH | CH | CH | CH | $CH_2Ph$(4-NHMe) |
| A-0333 | F | CH | CH | CH | CH | $CH_2Ph$(2-CHO) |
| A-0334 | F | CH | CH | CH | CH | $CH_2Ph$(3-CHO) |
| A-0335 | F | CH | CH | CH | CH | $CH_2Ph$(4-CHO) |
| A-0336 | F | CH | CH | CH | CH | $CH_2Ph$(2-Ph) |
| A-0337 | F | CH | CH | CH | CH | $CH_2Ph$(3-Ph) |
| A-0338 | F | CH | CH | CH | CH | $CH_2Ph$(4-Ph) |
| A-0339 | F | CH | CH | CH | CH | $CH_2Ph$(2-pyridin-2-yl) |
| A-0340 | F | CH | CH | CH | CH | $CH_2Ph$(3-pyridin-2-yl) |
| A-0341 | F | CH | CH | CH | CH | $CH_2Ph$(4-pyridin-2-yl) |
| A-0342 | F | CH | CH | CH | CH | $CH_2Ph$(2-pyridin-3-yl) |
| A-0343 | F | CH | CH | CH | CH | $CH_2Ph$(3-pyridin-3-yl) |
| A-0344 | F | CH | CH | CH | CH | $CH_2Ph$(4-pyridin-3-yl) |
| A-0345 | F | CH | CH | CH | CH | $CH_2Ph$(2-pyridin-4-yl) |
| A-0346 | F | CH | CH | CH | CH | $CH_2Ph$(3-pyridin-4-yl) |
| A-0347 | F | CH | CH | CH | CH | $CH_2Ph$(4-pyridin-4-yl) |
| A-0348 | F | CH | CH | CH | CH | $CH_2Ph(2-CH_2Ph)$ |
| A-0349 | F | CH | CH | CH | CH | $CH_2Ph(3-CH_2Ph)$ |
| A-0350 | F | CH | CH | CH | CH | $CH_2Ph(4-CH_2Ph)$ |
| A-0351 | F | CH | CH | CH | CH | $CH_2Ph(2,3-F_2)$ |
| A-0352 | F | CH | CH | CH | CH | $CH_2Ph(2,4-F_2)$ |
| A-0353 | F | CH | CH | CH | CH | $CH_2Ph(2,5-F_2)$ |
| A-0354 | F | CH | CH | CH | CH | $CH_2Ph(2,6-F_2)$ |
| A-0355 | F | CH | CH | CH | CH | $CH_2Ph(3,4-F_2)$ |
| A-0356 | F | CH | CH | CH | CH | $CH_2Ph(3,5-F_2)$ |
| A-0357 | F | CH | CH | CH | CH | $CH_2Ph$(2-Cl,3-F) |
| A-0358 | F | CH | CH | CH | CH | $CH_2Ph$(2-Cl,4-F) |
| A-0359 | F | CH | CH | CH | CH | $CH_2Ph$(2-Cl,5-F) |
| A-0360 | F | CH | CH | CH | CH | $CH_2Ph$(2-Cl,6-F) |
| A-0361 | F | CH | CH | CH | CH | $CH_2Ph$(3-Cl,2-F) |
| A-0362 | F | CH | CH | CH | CH | $CH_2Ph$(3-Cl,4-F) |
| A-0363 | F | CH | CH | CH | CH | $CH_2Ph$(3-Cl,5-F) |
| A-0364 | F | CH | CH | CH | CH | $CH_2Ph$(4-Cl,2-F) |
| A-0365 | F | CH | CH | CH | CH | $CH_2Ph$(4-Cl,3-F) |

(continued)

| Compound Number | $R^1$ | $X^1$ | $X^2$ | $X^3$ | $X^4$ | $R^2$ |
|---|---|---|---|---|---|---|
| A-0366 | F | CH | CH | CH | CH | $CH_2$Ph(5-Cl,2-F) |
| A-0367 | F | CH | CH | CH | CH | $CH_2$Ph(2-F,3-Me) |
| A-0368 | F | CH | CH | CH | CH | $CH_2$Ph(2-F,4-Me) |
| A-0369 | F | CH | CH | CH | CH | $CH_2$Ph(2-F,5-Me) |
| A-0370 | F | CH | CH | CH | CH | $CH_2$Ph(2-F,6-Me) |
| A-0371 | F | CH | CH | CH | CH | $CH_2$Ph(3-F,2-Me) |
| A-0372 | F | CH | CH | CH | CH | $CH_2$Ph(3-F,4-Me) |

[Table 10]

| Compound Number | $R^1$ | $X^1$ | $X^2$ | $X^3$ | $X^4$ | $R^2$ |
|---|---|---|---|---|---|---|
| A-0373 | F | CH | CH | CH | CH | $CH_2$Ph(3-F,5-Me) |
| A-0374 | F | CH | CH | CH | CH | $CH_2$Ph(4-F,2-Me) |
| A-0375 | F | CH | CH | CH | CH | $CH_2$Ph(4-F,3-Me) |
| A-0376 | F | CH | CH | CH | CH | $CH_2$Ph(5-F,2-Me) |
| A-0377 | F | CH | CH | CH | CH | $CH_2$Ph(2-CN,3-F) |
| A-0378 | F | CH | CH | CH | CH | $CH_2$Ph(2-CN,4-F) |
| A-0379 | F | CH | CH | CH | CH | $CH_2$Ph(2-CN,5-F) |
| A-0380 | F | CH | CH | CH | CH | $CH_2$Ph(2-CN,6-F) |
| A-0381 | F | CH | CH | CH | CH | $CH_2$Ph(3-CN,2-F) |
| A-0382 | F | CH | CH | CH | CH | $CH_2$Ph(3-CN,4-F) |
| A-0383 | F | CH | CH | CH | CH | $CH_2$Ph(3-CN,5-F) |
| A-0384 | F | CH | CH | CH | CH | $CH_2$Ph(4-CN,2-F) |
| A-0385 | F | CH | CH | CH | CH | $CH_2$Ph(4-CN,3-F) |
| A-0386 | F | CH | CH | CH | CH | $CH_2$Ph(5-CN,2-F) |
| A-0387 | F | CH | CH | CH | CH | $CH_2$Ph(2-F,3-OMe) |
| A-0388 | F | CH | CH | CH | CH | $CH_2$Ph(2-F,4-OMe) |
| A-0389 | F | CH | CH | CH | CH | $CH_2$Ph(2-F,5-OMe) |
| A-0390 | F | CH | CH | CH | CH | $CH_2$Ph(2-F,6-OMe) |
| A-0391 | F | CH | CH | CH | CH | $CH_2$Ph(3-F,2-OMe) |
| A-0392 | F | CH | CH | CH | CH | $CH_2$Ph(3-F,4-OMe) |
| A-0393 | F | CH | CH | CH | CH | $CH_2$Ph(3-F,5-OMe) |
| A-0394 | F | CH | CH | CH | CH | $CH_2$Ph(4-F,2-OMe) |
| A-0395 | F | CH | CH | CH | CH | $CH_2$Ph(4-F,3-OMe) |
| A-0396 | F | CH | CH | CH | CH | $CH_2$Ph(5-F,2-OMe) |

(continued)

| Compound Number | $R^1$ | $X^1$ | $X^2$ | $X^3$ | $X^4$ | $R^2$ |
|---|---|---|---|---|---|---|
| A-0397 | F | CH | CH | CH | CH | $CH_2Ph(2,3-Cl_2)$ |
| A-0398 | F | CH | CH | CH | CH | $CH_2Ph(2,4-Cl_2)$ |
| A-0399 | F | CH | CH | CH | CH | $CH_2Ph(2,5-Cl_2)$ |
| A-0400 | F | CH | CH | CH | CH | $CH_2Ph(2,6-Cl_2)$ |
| A-0401 | F | CH | CH | CH | CH | $CH_2Ph(3,4-Cl_2)$ |
| A-0402 | F | CH | CH | CH | CH | $CH_2Ph(3,5-Cl_2)$ |
| A-0403 | F | CH | CH | CH | CH | $CH_2Ph(2-Cl,3-Me)$ |
| A-0404 | F | CH | CH | CH | CH | $CH_2Ph(2-Cl,4-Me)$ |
| A-0405 | F | CH | CH | CH | CH | $CH_2Ph(2-Cl,5-Me)$ |
| A-0406 | F | CH | CH | CH | CH | $CH_2Ph(2-Cl,6-Me)$ |
| A-0407 | F | CH | CH | CH | CH | $CH_2Ph(3-Cl,2-Me)$ |
| A-0408 | F | CH | CH | CH | CH | $CH_2Ph(3-Cl,4-Me)$ |
| A-0409 | F | CH | CH | CH | CH | $CH_2Ph(3-Cl,5-Me)$ |
| A-0410 | F | CH | CH | CH | CH | $CH_2Ph(4-Cl,2-Me)$ |
| A-0411 | F | CH | CH | CH | CH | $CH_2Ph(4-Cl,3-Me)$ |
| A-0412 | F | CH | CH | CH | CH | $CH_2Ph(5-Cl,2-Me)$ |
| A-0413 | F | CH | CH | CH | CH | $CH_2Ph(2-Cl,3-CN)$ |
| A-0414 | F | CH | CH | CH | CH | $CH_2Ph(2-Cl,4-CN)$ |

[Table 11]

| Compound Number | $R^1$ | $X^1$ | $X^2$ | $X^3$ | $X^4$ | $R^2$ |
|---|---|---|---|---|---|---|
| A-0415 | F | CH | CH | CH | CH | $CH_2Ph(2-Cl,5-CN)$ |
| A-0416 | F | CH | CH | CH | CH | $CH_2Ph(2-Cl,6-CN)$ |
| A-0417 | F | CH | CH | CH | CH | $CH_2Ph(3-Cl,2-CN)$ |
| A-0418 | F | CH | CH | CH | CH | $CH_2Ph(3-Cl,4-CN)$ |
| A-0419 | F | CH | CH | CH | CH | $CH_2Ph(3-Cl,5-CN)$ |
| A-0420 | F | CH | CH | CH | CH | $CH_2Ph(4-Cl,2-CN)$ |
| A-0421 | F | CH | CH | CH | CH | $CH_2Ph(4-Cl,3-CN)$ |
| A-0422 | F | CH | CH | CH | CH | $CH_2Ph(5-Cl,2-CN)$ |
| A-0423 | F | CH | CH | CH | CH | $CH_2Ph(2-Cl,3-OMe)$ |
| A-0424 | F | CH | CH | CH | CH | $CH_2Ph(2-Cl,4-OMe)$ |
| A-0425 | F | CH | CH | CH | CH | $CH_2Ph(2-Cl,5-OMe)$ |
| A-0426 | F | CH | CH | CH | CH | $CH_2Ph(2-Cl,6-OMe)$ |
| A-0427 | F | CH | CH | CH | CH | $CH_2Ph(3-Cl,2-OMe)$ |

(continued)

| Compound Number | $R^1$ | $X^1$ | $X^2$ | $X^3$ | $X^4$ | $R^2$ |
|---|---|---|---|---|---|---|
| A-0428 | F | CH | CH | CH | CH | $CH_2Ph$(3-Cl,4-OMe) |
| A-0429 | F | CH | CH | CH | CH | $CH_2Ph$(3-Cl,5-OMe) |
| A-0430 | F | CH | CH | CH | CH | $CH_2Ph$(4-Cl,2-OMe) |
| A-0431 | F | CH | CH | CH | CH | $CH_2Ph$(4-Cl,3-OMe) |
| A-0432 | F | CH | CH | CH | CH | $CH_2Ph$(5-Cl,2-OMe) |
| A-0433 | F | CH | CH | CH | CH | $CH_2Ph(2,3\text{-}Me_2)$ |
| A-0434 | F | CH | CH | CH | CH | $CH_2Ph(2,4\text{-}Me_2)$ |
| A-0435 | F | CH | CH | CH | CH | $CH_2Ph(2,5\text{-}Me_2)$ |
| A-0436 | F | CH | CH | CH | CH | $CH_2Ph(2,6\text{-}Me_2)$ |
| A-0437 | F | CH | CH | CH | CH | $CH_2Ph(3,4\text{-}Me_2)$ |
| A-0438 | F | CH | CH | CH | CH | $CH_2Ph(3,5\text{-}Me_2)$ |
| A-0439 | F | CH | CH | CH | CH | $CH_2Ph$(2-CN,3-Me) |
| A-0440 | F | CH | CH | CH | CH | $CH_2Ph$(2-CN,4-Me) |
| A-0441 | F | CH | CH | CH | CH | $CH_2Ph$(2-CN,5-Me) |
| A-0442 | F | CH | CH | CH | CH | $CH_2Ph$(2-CN,6-Me) |
| A-0443 | F | CH | CH | CH | CH | $CH_2Ph$(3-CN,2-Me) |
| A-0444 | F | CH | CH | CH | CH | $CH_2Ph$(3-CN,4-Me) |
| A-0445 | F | CH | CH | CH | CH | $CH_2Ph$(3-CN,5-Me) |
| A-0446 | F | CH | CH | CH | CH | $CH_2Ph$(4-CN,2-Me) |
| A-0447 | F | CH | CH | CH | CH | $CH_2Ph$(4-CN,3-Me) |
| A-0448 | F | CH | CH | CH | CH | $CH_2Ph$(5-CN,2-Me) |
| A-0449 | F | CH | CH | CH | CH | $CH_2Ph$(2-OMe,3-Me) |
| A-0450 | F | CH | CH | CH | CH | $CH_2Ph$(2-OMe,4-Me) |
| A-0451 | F | CH | CH | CH | CH | $CH_2Ph$(2-OMe,5-Me) |
| A-0452 | F | CH | CH | CH | CH | $CH_2Ph$(2-OMe,6-Me) |
| A-0453 | F | CH | CH | CH | CH | $CH_2Ph$(3-OMe,2-Me) |
| A-0454 | F | CH | CH | CH | CH | $CH_2Ph$(3-OMe,4-Me) |
| A-0455 | F | CH | CH | CH | CH | $CH_2Ph$(3-OMe,5-Me) |
| A-0456 | F | CH | CH | CH | CH | $CH_2Ph$(4-OMe,2-Me) |

[Table 12]

| Compound Number | $R^1$ | $X^1$ | $X^2$ | $X^3$ | $X^4$ | $R^2$ |
|---|---|---|---|---|---|---|
| A-0457 | F | CH | CH | CH | CH | $CH_2Ph$(4-OMe,3-Me) |
| A-0458 | F | CH | CH | CH | CH | $CH_2Ph$(5-OMe,2-Me) |

(continued)

| Compound Number | $R^1$ | $X^1$ | $X^2$ | $X^3$ | $X^4$ | $R^2$ |
|---|---|---|---|---|---|---|
| A-0459 | F | CH | CH | CH | CH | $CH_2Ph[2,3-(OMe)_2]$ |
| A-0460 | F | CH | CH | CH | CH | $CH_2Ph[2,4-(OMe)_2]$ |
| A-0461 | F | CH | CH | CH | CH | $CH_2Ph[2,5-(OMe)_2]$ |
| A-0462 | F | CH | CH | CH | CH | $CH_2Ph[2,6-(OMe)_2]$ |
| A-0463 | F | CH | CH | CH | CH | $CH_2Ph[3,4-(OMe)_2]$ |
| A-0464 | F | CH | CH | CH | CH | $CH_2Ph[3,5-(OMe)_2]$ |
| A-0465 | F | CH | CH | CH | CH | $CH_2Ph(2-CN,3-OMe)$ |
| A-0466 | F | CH | CH | CH | CH | $CH_2Ph(2-CN,4-OMe)$ |
| A-0467 | F | CH | CH | CH | CH | $CH_2Ph(2-CN,5-OMe)$ |
| A-0468 | F | CH | CH | CH | CH | $CH_2Ph(2-CN,6-OMe)$ |
| A-0469 | F | CH | CH | CH | CH | $CH_2Ph(3-CN,2-OMe)$ |
| A-0470 | F | CH | CH | CH | CH | $CH_2Ph(3-CN,4-OMe)$ |
| A-0471 | F | CH | CH | CH | CH | $CH_2Ph(3-CN,5-OMe)$ |
| A-0472 | F | CH | CH | CH | CH | $CH_2Ph(4-CN,2-OMe)$ |
| A-0473 | F | CH | CH | CH | CH | $CH_2Ph(4-CN,3-OMe)$ |
| A-0474 | F | CH | CH | CH | CH | $CH_2Ph(5-CN,2-OMe)$ |
| A-0475 | F | CH | CH | CH | CH | $CH_2Ph[2,3-(CN)_2]$ |
| A-0476 | F | CH | CH | CH | CH | $CH_2Ph[2,4-(CN)_2]$ |
| A-0477 | F | CH | CH | CH | CH | $CH_2Ph[2,5-(CN)_2]$ |
| A-0478 | F | CH | CH | CH | CH | $CH_2Ph[2,6-(CN)_2]$ |
| A-0479 | F | CH | CH | CH | CH | $CH_2Ph[3,4-(CN)_2]$ |
| A-0480 | F | CH | CH | CH | CH | $CH_2Ph[3,5-(CN)_2]$ |
| A-0481 | F | CF | CH | CH | CH | H |
| A-0482 | F | CF | CH | CH | CH | $CH_2Ph$ |
| A-0483 | F | CH | CF | CH | CH | H |
| A-0484 | F | CH | CF | CH | CH | $CH_2Ph$ |
| A-0485 | F | CMe | CH | CH | CH | H |
| A-0486 | F | CMe | CH | CH | CH | $CH_2Ph$ |
| A-0487 | F | CH | CMe | CH | CH | H |
| A-0488 | F | CH | CMe | CH | CH | $CH_2Ph$ |
| A-0489 | Cl | CH | CH | CH | CH | H |
| A-0490 | Cl | CH | CH | CH | CH | $CH_2Ph$ |
| A-0491 | F | N | CH | CH | CH | H |
| A-0492 | F | N | CH | CH | CH | $CH_2Ph$ |
| A-0493 | F | CH | N | CH | CH | H |

(continued)

| Compound Number | $R^1$ | $X^1$ | $X^2$ | $X^3$ | $X^4$ | $R^2$ |
|---|---|---|---|---|---|---|
| A-0494 | F | CH | N | CH | CH | $CH_2Ph$ |
| A-0495 | F | N | N | CH | CH | H |
| A-0496 | F | N | N | CH | CH | $CH_2Ph$ |
| A-0497 | F | N | CH | N | CH | H |
| A-0498 | F | N | CH | N | CH | $CH_2Ph$ |

[Table 13]

| Compound Number | $R^1$ | $X^1$ | $X^2$ | $X^3$ | $X^4$ | $R^2$ |
|---|---|---|---|---|---|---|
| A-0499 | F | N | CH | CH | N | H |
| A-0500 | F | N | CH | CH | N | $CH_2Ph$ |
| A-0501 | F | CH | N | CH | N | H |
| A-0502 | F | CH | N | CH | N | $CH_2Ph$ |
| A-0503 | H | CH | CH | CH | CH | H |
| A-0504 | H | CH | CH | CH | CH | $CH_2Ph$ |
| A-0505 | F | CH | CH | CH | CH | $CH_2CH_2Ph(2-F)$ |
| A-0506 | F | CH | CH | CH | CH | $CH_2CH_2Ph(3-F)$ |
| A-0507 | F | CH | CH | CH | CH | $CH_2CH_2Ph(4-F)$ |
| A-0508 | F | CH | CH | CH | CH | $CH_2CH=CHPh$ |
| A-0509 | F | CH | CH | CH | CH | $CH_2CCPh$ |
| A-0510 | F | CH | CH | CH | CH | $CH_2CCTMS$ |
| A-0511 | F | CH | CH | CH | CH | $CH_2F$ |
| A-0512 | F | CH | CH | CH | CH | $CH_2CH_2F$ |
| A-0513 | F | CH | CH | CH | CH | $CH_2CH_2CH_2F$ |
| A-0514 | F | CH | CH | CH | CH | $CH_2CHO$ |
| A-0515 | F | CH | CH | CH | CH | $CH_2CH_2CHO$ |
| A-0516 | F | CH | CH | CH | CH | $CH_2COEt$ |
| A-0517 | F | CH | CH | CH | CH | $CH_2CO$n-Pr |
| A-0518 | F | CH | CH | CH | CH | $CH_2CO$n-Bu |
| A-0519 | F | CH | CH | CH | CH | $CH_2CO$i-Pr |
| A-0520 | F | CH | CH | CH | CH | $CH_2CO$t-Bu |
| A-0521 | F | CH | CH | CH | CH | $CH_2CO$i-Bu |
| A-0522 | F | CH | CH | CH | CH | $CH_2CO$sec-Bu |
| A-0523 | F | CH | CH | CH | CH | $CH_2COCH_2$t-Bu |
| A-0524 | F | CH | CH | CH | CH | $CH_2COCH(CH_2CH_3)Et$ |

(continued)

| Compound Number | $R^1$ | $X^1$ | $X^2$ | $X^3$ | $X^4$ | $R^2$ |
|---|---|---|---|---|---|---|
| A-0525 | F | CH | CH | CH | CH | $CH_2CO$c-Pr |
| A-0526 | F | CH | CH | CH | CH | $CH_2COCH_2$c-Pr |
| A-0527 | F | CH | CH | CH | CH | $CH_2CO$c-Bu |
| A-0528 | F | CH | CH | CH | CH | $CH_2CO$c-Pen |
| A-0529 | F | CH | CH | CH | CH | $CH_2CH_2COMe$ |
| A-0530 | F | CH | CH | CH | CH | $CH_2CH_2COEt$ |
| A-0531 | F | CH | CH | CH | CH | $CH_2COCF_3$ |
| A-0532 | F | CH | CH | CH | CH | $CH_2COCHF_2$ |
| A-0533 | F | CH | CH | CH | CH | $CH_2COCH_2CF_3$ |
| A-0534 | F | CH | CH | CH | CH | $CH_2COCH_2CHF_2$ |
| A-0535 | F | CH | CH | CH | CH | $CH_2COCH_2OCH_3$ |
| A-0536 | F | CH | CH | CH | CH | $CH_2COCH_2Ph$ |
| A-0537 | F | CH | CH | CH | CH | $CH_2CO$(pyrrolidin-1-yl) |
| A-0538 | F | CH | CH | CH | CH | $CH_2CO$(1H-pyrazol-1-yl) |
| A-0539 | F | CH | CH | CH | CH | $CH_2CO$(morpholino-1-yl) |
| A-0540 | F | CH | CH | CH | CH | $CH_2CO$(pyridin-2-yl) |

[Table 14]

| Compound Number | $R^1$ | $X^1$ | $X^2$ | $X^3$ | $X^4$ | $R^2$ |
|---|---|---|---|---|---|---|
| A-0541 | F | CH | CH | CH | CH | $CH_2CO$(pyridin-3-yl) |
| A-0542 | F | CH | CH | CH | CH | $CH_2CO$(pyridin-4-yl) |
| A-0543 | F | CH | CH | CH | CH | $CH_2COCH_2$(pyridin-2-yl) |
| A-0544 | F | CH | CH | CH | CH | $CH_2COCH_2$(pyridin-3-yl) |
| A-0545 | F | CH | CH | CH | CH | $CH_2COCH_2$(pyridin-4-yl) |
| A-0546 | F | CH | CH | CH | CH | $CH_2CO_2H$ |
| A-0547 | F | CH | CH | CH | CH | $CH_2CH_2CO_2Me$ |
| A-0548 | F | CH | CH | CH | CH | $CH(CH_2CH_3)CO_2Et$ |
| A-0549 | F | CH | CH | CH | CH | $CH(F)CO_2Et$ |
| A-0550 | F | CH | CH | CH | CH | $CH(CO_2Et)_2$ |
| A-0551 | F | CH | CH | CH | CH | $CH_2CH_2CO_2Et$ |
| A-0552 | F | CH | CH | CH | CH | $CH_2CH_2CH_2CO_2Et$ |
| A-0553 | F | CH | CH | CH | CH | $CH_2CH_2CH_2CH_2CH_2CO_2Et$ |
| A-0554 | F | CH | CH | CH | CH | $CH_2CO_2$n-Pr |
| A-0555 | F | CH | CH | CH | CH | $CH(CH_3)CO_2$n-Pr |

(continued)

| Compound Number | $R^1$ | $X^1$ | $X^2$ | $X^3$ | $X^4$ | $R^2$ |
|---|---|---|---|---|---|---|
| A-0556 | F | CH | CH | CH | CH | $C(CH_3)(CH_3)CO_2$n-Pr |
| A-0557 | F | CH | CH | CH | CH | $CH_2CO_2$i-Pr |
| A-0558 | F | CH | CH | CH | CH | $CH(CH_3)CO_2$i-Pr |
| A-0559 | F | CH | CH | CH | CH | $C(CH_3)(CH_3)CO_2$i-Pr |
| A-0560 | F | CH | CH | CH | CH | $CH_2CO_2$n-Bu |
| A-0561 | F | CH | CH | CH | CH | $CH_2CO_2$t-Bu |
| A-0562 | F | CH | CH | CH | CH | $CH_2CO_2$i-Bu |
| A-0563 | F | CH | CH | CH | CH | $CH_2CO_2$c-Pr |
| A-0564 | F | CH | CH | CH | CH | $CH_2CO_2CH_2$c-Pr |
| A-0565 | F | CH | CH | CH | CH | $CH_2CO_2$c-Bu |
| A-0566 | F | CH | CH | CH | CH | $CH_2CO_2$c-Pen |
| A-0567 | F | CH | CH | CH | CH | $CH_2CO_2CH{=}CH_2$ |
| A-0568 | F | CH | CH | CH | CH | $CH_2CO_2CH_2CH{=}CH_2$ |
| A-0569 | F | CH | CH | CH | CH | $CH_2CO_2CH_2CCH$ |
| A-0570 | F | CH | CH | CH | CH | $CH_2CO_2CF_3$ |
| A-0571 | F | CH | CH | CH | CH | $CH_2CO_2CHF_2$ |
| A-0572 | F | CH | CH | CH | CH | $CH_2CO_2CH_2CF_3$ |
| A-0573 | F | CH | CH | CH | CH | $CH_2CO_2CH_2CHF_2$ |
| A-0574 | F | CH | CH | CH | CH | $CH_2CO_2CH_2CN$ |
| A-0575 | F | CH | CH | CH | CH | $CH_2CO_2CH_2COMe$ |
| A-0576 | F | CH | CH | CH | CH | $CH_2CO_2CH_2CO_2Et$ |
| A-0577 | F | CH | CH | CH | CH | $CH_2CO_2CH_2CO_2CH_2CF_3$ |
| A-0578 | F | CH | CH | CH | CH | $CH_2CO_2CH_2Ph$ |
| A-0579 | F | CH | CH | CH | CH | $CH_2CO_2$(pyridin-2-yl) |
| A-0580 | F | CH | CH | CH | CH | $CH_2CO_2$(pyridin-3-yl) |
| A-0581 | F | CH | CH | CH | CH | $CH_2CO_2$(pyridin-4-yl) |
| A-0582 | F | CH | CH | CH | CH | $CH_2CO_2CH_2$(pyridin-2-yl) |

[Table 15]

| Compound Number | $R^1$ | $X^1$ | $X^2$ | $X^3$ | $X^4$ | $R^2$ |
|---|---|---|---|---|---|---|
| A-0583 | F | CH | CH | CH | CH | $CH_2CO_2CH_2$[pyridin-2-yl(6-F)] |
| A-0584 | F | CH | CH | CH | CH | $CH_2CO_2CH_2$[pyridin-2-yl(6-Cl)] |
| A-0585 | F | CH | CH | CH | CH | $CH_2CO_2CH_2$[pyridin-2-yl(6-Me)] |
| A-0586 | F | CH | CH | CH | CH | $CH_2CO_2CH_2$(pyridin-3-yl) |

(continued)

| Compound Number | $R^1$ | $X^1$ | $X^2$ | $X^3$ | $X^4$ | $R^2$ |
|---|---|---|---|---|---|---|
| A-0587 | F | CH | CH | CH | CH | $CH_2CO_2CH_2$[pyridin-3-yl(6-F)] |
| A-0588 | F | CH | CH | CH | CH | $CH_2CO_2CH_2$[pyridin-3-yl(6-Cl)] |
| A-0589 | F | CH | CH | CH | CH | $CH_2CO_2CH_2$[pyridin-3-yl(6-Me)] |
| A-0590 | F | CH | CH | CH | CH | $CH_2CO_2CH_2$(pyridin-4-yl) |
| A-0591 | F | CH | CH | CH | CH | $CH_2CO_2CH_2$(1H-pyrazol-1-yl) |
| A-0592 | F | CH | CH | CH | CH | $CH_2CO_2CH_2$[1H-pyrazol-3-yl(1-Me)] |
| A-0593 | F | CH | CH | CH | CH | $CH_2CO_2CH_2$[1H-pyrazol-4-yl(1-Me)] |
| A-0594 | F | CH | CH | CH | CH | $CH_2CO_2CH_2$[1H-pyrazol-5-yl(1-Me)] |
| A-0595 | F | CH | CH | CH | CH | $CH_2CONMe_2$ |
| A-0596 | F | CH | CH | CH | CH | $CH_2CON(CH_3)Et$ |
| A-0597 | F | CH | CH | CH | CH | $CH_2CON(CH_3)$n-Pr |
| A-0598 | F | CH | CH | CH | CH | $CH_2CONEt_2$ |
| A-0599 | F | CH | CH | CH | CH | $CH_2CONH$n-Pr |
| A-0600 | F | CH | CH | CH | CH | $CH_2CONH$i-Pr |
| A-0601 | F | CH | CH | CH | CH | $CH_2CONH$n-Bu |
| A-0602 | F | CH | CH | CH | CH | $CH_2CONH$t-Bu |
| A-0603 | F | CH | CH | CH | CH | $CH_2CONH$i-Bu |
| A-0604 | F | CH | CH | CH | CH | $CH_2CONH$c-Pr |
| A-0605 | F | CH | CH | CH | CH | $CH_2CONH$cPr(1-CN) |
| A-0606 | F | CH | CH | CH | CH | $CH_2CONHCH_2$c-Pr |
| A-0607 | F | CH | CH | CH | CH | $CH_2CONHCH=CH_2$ |
| A-0608 | F | CH | CH | CH | CH | $CH_2CONHCH_2CH=CH_2$ |
| A-0609 | F | CH | CH | CH | CH | $CH_2CONHCH_2CCH$ |
| A-0610 | F | CH | CH | CH | CH | $CH_2CONHCF_3$ |
| A-0611 | F | CH | CH | CH | CH | $CH_2CONHCHF_2$ |
| A-0612 | F | CH | CH | CH | CH | $CH_2CONHCH_2CF_3$ |
| A-0613 | F | CH | CH | CH | CH | $CH_2CONHCH_2CHF_2$ |
| A-0614 | F | CH | CH | CH | CH | $CH_2CH_2CONHMe$ |
| A-0615 | F | CH | CH | CH | CH | $CH_2CH_2CONHEt$ |
| A-0616 | F | CH | CH | CH | CH | $CH_2CH_2CONMe_2$ |
| A-0617 | F | CH | CH | CH | CH | $CH_2CH_2CON(CH_3)Et$ |
| A-0618 | F | CH | CH | CH | CH | $CH_2CONHCH_2Ph$ |
| A-0619 | F | CH | CH | CH | CH | $CH_2CONH$(pyridin-2-yl) |
| A-0620 | F | CH | CH | CH | CH | $CH_2CONH$(pyridin-3-yl) |

(continued)

| Compound Number | $R^1$ | $X^1$ | $X^2$ | $X^3$ | $X^4$ | $R^2$ |
|---|---|---|---|---|---|---|
| A-0621 | F | CH | CH | CH | CH | $CH_2$CONH(pyridin-4-yl) |
| A-0622 | F | CH | CH | CH | CH | $CH_2CONHCH_2$(pyridin-2-yl) |
| A-0623 | F | CH | CH | CH | CH | $CH_2CONHCH_2$(pyridin-3-yl) |
| A-0624 | F | CH | CH | CH | CH | $CH_2CONHCH_2$(pyridin-4-yl) |

[Table 16]

| Compound Number | $R^1$ | $X^1$ | $X^2$ | $X^3$ | $X^4$ | $R^2$ |
|---|---|---|---|---|---|---|
| A-0625 | F | CH | CH | CH | CH | $CH_2CH_2NHCHO$ |
| A-0626 | F | CH | CH | CH | CH | $CH_2CH_2NHCOMe$ |
| A-0627 | F | CH | CH | CH | CH | $CH_2CH_2NHCOEt$ |
| A-0628 | F | CH | CH | CH | CH | $CH_2CH_2N(CH_3)CHO$ |
| A-0629 | F | CH | CH | CH | CH | $CH_2CH_2N(CH_3)COMe$ |
| A-0630 | F | CH | CH | CH | CH | $CH_2CH_2N(CH_3)COEt$ |
| A-0631 | F | CH | CH | CH | CH | $CH_2CH_2N(CH_2CH_3)CHO$ |
| A-0632 | F | CH | CH | CH | CH | $CH_2CH_2N(CH_2CH_3)COMe$ |
| A-0633 | F | CH | CH | CH | CH | $CH_2CH_2N(CH_2CH_3)COEt$ |
| A-0634 | F | CH | CH | CH | CH | $CH_2$NHCOc-Pr |
| A-0635 | F | CH | CH | CH | CH | $CH_2NHCOCH_2$c-Pr |
| A-0636 | F | CH | CH | CH | CH | $CH_2CH_2$NHCOc-Pr |
| A-0637 | F | CH | CH | CH | CH | $CH_2CH_2NHCOCH_2$c-Pr |
| A-0638 | F | CH | CH | CH | CH | $CH_2NHCOCF_3$ |
| A-0639 | F | CH | CH | CH | CH | $CH_2NHCOCHF_2$ |
| A-0640 | F | CH | CH | CH | CH | $CH_2NHCOCH_2CF_3$ |
| A-0641 | F | CH | CH | CH | CH | $CH_2NHCOCH_2CHF_2$ |
| A-0642 | F | CH | CH | CH | CH | $CH_2NHCOCH_2Ph$ |
| A-0643 | F | CH | CH | CH | CH | $CH_2$NHCO(pyridin-2-yl) |
| A-0644 | F | CH | CH | CH | CH | $CH_2$NHCO(pyridin-3-yl) |
| A-0645 | F | CH | CH | CH | CH | $CH_2$NHCO(pyridin-4-yl) |
| A-0646 | F | CH | CH | CH | CH | $CH_2NHCOCH_2$(pyridin-2-yl) |
| A-0647 | F | CH | CH | CH | CH | $CH_2NHCOCH_2$(pyridin-3-yl) |
| A-0648 | F | CH | CH | CH | CH | $CH_2NHCOCH_2$(pyridin-4-yl) |
| A-0649 | F | CH | CH | CH | CH | $CH_2OCHO$ |
| A-0650 | F | CH | CH | CH | CH | $CH_2CH_2OCHO$ |
| A-0651 | F | CH | CH | CH | CH | $CH_2OCOMe$ |

(continued)

| Compound Number | $R^1$ | $X^1$ | $X^2$ | $X^3$ | $X^4$ | $R^2$ |
|---|---|---|---|---|---|---|
| A-0652 | F | CH | CH | CH | CH | $CH_2OCOEt$ |
| A-0653 | F | CH | CH | CH | CH | $CH_2OCOn$-Pr |
| A-0654 | F | CH | CH | CH | CH | $CH_2OCOi$-Pr |
| A-0655 | F | CH | CH | CH | CH | $CH_2OCOn$-Bu |
| A-0656 | F | CH | CH | CH | CH | $CH_2OCOi$-Bu |
| A-0657 | F | CH | CH | CH | CH | $CH_2OCOt$-Bu |
| A-0658 | F | CH | CH | CH | CH | $CH_2CH_2OCOMe$ |
| A-0659 | F | CH | CH | CH | CH | $CH_2CH_2OCOEt$ |
| A-0660 | F | CH | CH | CH | CH | $CH_2CH_2CH_2OCOMe$ |
| A-0661 | F | CH | CH | CH | CH | $CH_2OCOc$-Pr |
| A-0662 | F | CH | CH | CH | CH | $CH_2OCOCH_2c$-Pr |
| A-0663 | F | CH | CH | CH | CH | $CH_2OCOCF_3$ |
| A-0664 | F | CH | CH | CH | CH | $CH_2OCOCHF_2$ |
| A-0665 | F | CH | CH | CH | CH | $CH_2OCOCH_2CF_3$ |
| A-0666 | F | CH | CH | CH | CH | $CH_2OCOCH_2CHF_2$ |

[Table 17]

| Compound Number | $R^1$ | $X^1$ | $X^2$ | $X^3$ | $X^4$ | $R^2$ |
|---|---|---|---|---|---|---|
| A-0667 | F | CH | CH | CH | CH | $CH_2OCOPh$ |
| A-0668 | F | CH | CH | CH | CH | $CH_2OCOCH_2Ph$ |
| A-0669 | F | CH | CH | CH | CH | $CH_2OCO$(pyridin-2-yl) |
| A-0670 | F | CH | CH | CH | CH | $CH_2OCO$(pyridin-3-yl) |
| A-0671 | F | CH | CH | CH | CH | $CH_2OCO$(pyridin-4-yl) |
| A-0672 | F | CH | CH | CH | CH | $CH_2OCOCH_2$(pyridin-2-yl) |
| A-0673 | F | CH | CH | CH | CH | $CH_2OCOCH_2$(pyridin-3-yl) |
| A-0674 | F | CH | CH | CH | CH | $CH_2OCOCH_2$(pyridin-4-yl) |
| A-0675 | F | CH | CH | CH | CH | $CH_2SO_2NHMe$ |
| A-0676 | F | CH | CH | CH | CH | $CH_2SO_2NHEt$ |
| A-0677 | F | CH | CH | CH | CH | $CH_2SO_2NMe_2$ |
| A-0678 | F | CH | CH | CH | CH | $CH_2SO_2N(CH_3)Et$ |
| A-0679 | F | CH | CH | CH | CH | $CH_2CH_2SO_2NHMe$ |
| A-0680 | F | CH | CH | CH | CH | $CH_2CH_2SO_2NHEt$ |
| A-0681 | F | CH | CH | CH | CH | $CH_2CH_2SO_2NMe_2$ |
| A-0682 | F | CH | CH | CH | CH | $CH_2CH_2SO_2(CH_3)Et$ |

(continued)

| Compound Number | $R^1$ | $X^1$ | $X^2$ | $X^3$ | $X^4$ | $R^2$ |
|---|---|---|---|---|---|---|
| A-0683 | F | CH | CH | CH | CH | $CH_2SO_2NHc$-Pr |
| A-0684 | F | CH | CH | CH | CH | $CH_2SO_2NHCH_2c$-Pr |
| A-0685 | F | CH | CH | CH | CH | $CH_2SO_2NHCF_3$ |
| A-0686 | F | CH | CH | CH | CH | $CH_2SO_2NHCH_2CF_3$ |
| A-0687 | F | CH | CH | CH | CH | $CH_2SO_2NHCHF_2$ |
| A-0688 | F | CH | CH | CH | CH | $CH_2SO_2NHCH_2CHF_2$ |
| A-0689 | F | CH | CH | CH | CH | $CH_2SO_2NHPh$ |
| A-0690 | F | CH | CH | CH | CH | $CH_2SO_2NHCH_2Ph$ |
| A-0691 | F | CH | CH | CH | CH | $CH_2SO_2NH$(pyridin-2-yl) |
| A-0692 | F | CH | CH | CH | CH | $CH_2SO_2NH$(pyridin-3-yl) |
| A-0693 | F | CH | CH | CH | CH | $CH_2SO_2NH$(pyridin-4-yl) |
| A-0694 | F | CH | CH | CH | CH | $CH_2SO_2NHCH_2$(pyridin-2-yl) |
| A-0695 | F | CH | CH | CH | CH | $CH_2SO_2NHCH_2$(pyridin-3-yl) |
| A-0696 | F | CH | CH | CH | CH | $CH_2SO_2NHCH_2$(pyridin-4-yl) |
| A-0697 | F | CH | CH | CH | CH | $CH_2NHSO_2Me$ |
| A-0698 | F | CH | CH | CH | CH | $CH_2N(CH_3)SO_2Me$ |
| A-0699 | F | CH | CH | CH | CH | $CH_2NHSO_2Et$ |
| A-0700 | F | CH | CH | CH | CH | $CH_2NHSO_2c$-Pr |
| A-0701 | F | CH | CH | CH | CH | $CH_2NHSO_2CH_2c$-Pr |
| A-0702 | F | CH | CH | CH | CH | $CH_2NHSO_2CF_3$ |
| A-0703 | F | CH | CH | CH | CH | $CH_2NHSO_2CH_2CF_3$ |
| A-0704 | F | CH | CH | CH | CH | $CH_2NHSO_2CHF_2$ |
| A-0705 | F | CH | CH | CH | CH | $CH_2NHSO_2CH_2CHF_2$ |
| A-0706 | F | CH | CH | CH | CH | $CH_2CH_2NHSO_2Me$ |
| A-0707 | F | CH | CH | CH | CH | $CH_2CH_2N(CH_3)SO_2Me$ |
| A-0708 | F | CH | CH | CH | CH | $CH_2NHSO_2Ph$ |

[Table 18]

| Compound Number | $R^1$ | $X^1$ | $X^2$ | $X^3$ | $X^4$ | $R^2$ |
|---|---|---|---|---|---|---|
| A-0709 | F | CH | CH | CH | CH | $CH_2NHSO_2CH_2Ph$ |
| A-0710 | F | CH | CH | CH | CH | $CH_2NHSO_2$(pyridin-2-yl) |
| A-0711 | F | CH | CH | CH | CH | $CH_2NHSO_2$(pyridin-3-yl) |
| A-0712 | F | CH | CH | CH | CH | $CH_2NHSO_2$(pyridin-4-yl) |
| A-0713 | F | CH | CH | CH | CH | $CH_2NHSO_2CH_2$(pyridin-2-yl) |

(continued)

| Compound Number | $R^1$ | $X^1$ | $X^2$ | $X^3$ | $X^4$ | $R^2$ |
|---|---|---|---|---|---|---|
| A-0714 | F | CH | CH | CH | CH | $CH_2NHSO_2CH_2$(pyridin-3-yl) |
| A-0715 | F | CH | CH | CH | CH | $CH_2NHSO_2CH_2$(pyridin-4-yl) |
| A-0716 | F | CH | CH | CH | CH | $CH_2OH$ |
| A-0717 | F | CH | CH | CH | CH | $CH_2CH_2OH$ |
| A-0718 | F | CH | CH | CH | CH | $CH_2CH(OH)CH_2CH_2CH_3$ |
| A-0719 | F | CH | CH | CH | CH | $CH_2CH_2$On-Pr |
| A-0720 | F | CH | CH | CH | CH | $CH_2CH_2$Oi-Pr |
| A-0721 | F | CH | CH | CH | CH | $CH_2CH_2$Oc-Pr |
| A-0722 | F | CH | CH | CH | CH | $CH_2CH_2OCH_2$c-Pr |
| A-0723 | F | CH | CH | CH | CH | $CH_2CH_2$Ot-Bu |
| A-0724 | F | CH | CH | CH | CH | $CH_2CH_2$Oi-Bu |
| A-0725 | F | CH | CH | CH | CH | $CH_2CH_2$Osec-Bu |
| A-0726 | F | CH | CH | CH | CH | $CH(CH_3)OEt$ |
| A-0727 | F | CH | CH | CH | CH | $CH_2CH(CH_3)OEt$ |
| A-0728 | F | CH | CH | CH | CH | $CH_2CH(OMe)_2$ |
| A-0729 | F | CH | CH | CH | CH | $CH_2CH(OEt)_2$ |
| A-0730 | F | CH | CH | CH | CH | $CH(CH_3)CH(OMe)_2$ |
| A-0731 | F | CH | CH | CH | CH | $CH_2C(OMe)_2Me$ |
| A-0732 | F | CH | CH | CH | CH | $CH(CH_3)CH(OEt)_2$ |
| A-0733 | F | CH | CH | CH | CH | $CH_2C(OEt)_2Me$ |
| A-0734 | F | CH | CH | CH | CH | $CH_2CH_2CH(OMe)_2$ |
| A-0735 | F | CH | CH | CH | CH | $CH_2CH_2CH(OEt)_2$ |
| A-0736 | F | CH | CH | CH | CH | $CH_2CH_2CH_2OMe$ |
| A-0737 | F | CH | CH | CH | CH | $CH_2OCHF_2$ |
| A-0738 | F | CH | CH | CH | CH | $CH_2OCH_2CHF_2$ |
| A-0739 | F | CH | CH | CH | CH | $CH_2C(OH)_2CF_3$ |
| A-0740 | F | CH | CH | CH | CH | $CH_2CH_2OCHF_2$ |
| A-0741 | F | CH | CH | CH | CH | $CH_2CH_2OCH_2CHF_2$ |
| A-0742 | F | CH | CH | CH | CH | $CH_2OCH_2Ph$ |
| A-0743 | F | CH | CH | CH | CH | $CH_2OCH_2CH_2OMe$ |
| A-0744 | F | CH | CH | CH | CH | $CH_2CH_2OCH_2CH_2OMe$ |
| A-0745 | F | CH | CH | CH | CH | $CH_2O$(pyridin-2-yl) |
| A-0746 | F | CH | CH | CH | CH | $CH_2O$(pyridin-3-yl) |
| A-0747 | F | CH | CH | CH | CH | $CH_2O$(pyridin-4-yl) |

(continued)

| Compound Number | $R^1$ | $X^1$ | $X^2$ | $X^3$ | $X^4$ | $R^2$ |
|---|---|---|---|---|---|---|
| A-0748 | F | CH | CH | CH | CH | $CH_2OCH_2$(pyridin-2-yl) |
| A-0749 | F | CH | CH | CH | CH | $CH_2OCH_2$(pyridin-3-yl) |
| A-0750 | F | CH | CH | CH | CH | $CH_2OCH_2$(pyridin-4-yl) |

[Table 19]

| Compound Number | $R^1$ | $X^1$ | $X^2$ | $X^3$ | $X^4$ | $R^2$ |
|---|---|---|---|---|---|---|
| A-0751 | F | CH | CH | CH | CH | $CH_2SH$ |
| A-0752 | F | CH | CH | CH | CH | $CH_2CH_2SH$ |
| A-0753 | F | CH | CH | CH | CH | $CH_2SOMe$ |
| A-0754 | F | CH | CH | CH | CH | $CH_2SO_2Me$ |
| A-0755 | F | CH | CH | CH | CH | $CH_2SOEt$ |
| A-0756 | F | CH | CH | CH | CH | $CH_2SO_2Et$ |
| A-0757 | F | CH | CH | CH | CH | $CH_2CH_2SOMe$ |
| A-0758 | F | CH | CH | CH | CH | $CH_2CH_2SO_2Me$ |
| A-0759 | F | CH | CH | CH | CH | $CH_2CH_2SOEt$ |
| A-0760 | F | CH | CH | CH | CH | $CH_2CH_2SO_2Et$ |
| A-0761 | F | CH | CH | CH | CH | $CH_2$Sc-Pr |
| A-0762 | F | CH | CH | CH | CH | $CH_2$SOc-Pr |
| A-0763 | F | CH | CH | CH | CH | $CH_2SO_2$c-Pr |
| A-0764 | F | CH | CH | CH | CH | $CH_2SCH_2$c-Pr |
| A-0765 | F | CH | CH | CH | CH | $CH_2SOCH_2$c-Pr |
| A-0766 | F | CH | CH | CH | CH | $CH_2SO_2CH_2$c-Pr |
| A-0767 | F | CH | CH | CH | CH | $CH_2CH_2$Si-Pr |
| A-0768 | F | CH | CH | CH | CH | $CH_2CH_2$SOi-Pr |
| A-0769 | F | CH | CH | CH | CH | $CH_2CH_2SO_2$i-Pr |
| A-0770 | F | CH | CH | CH | CH | $CH_2SOCF_3$ |
| A-0771 | F | CH | CH | CH | CH | $CH_2SO_2CF_3$ |
| A-0772 | F | CH | CH | CH | CH | $CH_2SOCHF_2$ |
| A-0773 | F | CH | CH | CH | CH | $CH_2SO_2CHF_2$ |
| A-0774 | F | CH | CH | CH | CH | $CH_2SCH_2Ph$ |
| A-0775 | F | CH | CH | CH | CH | $CH_2SOCH_2Ph$ |
| A-0776 | F | CH | CH | CH | CH | $CH_2SO_2CH_2Ph$ |
| A-0777 | F | CH | CH | CH | CH | $CH_2S$(pyridin-2-yl) |
| A-0778 | F | CH | CH | CH | CH | $CH_2SO$(pyridin-2-yl) |

(continued)

| Compound Number | $R^1$ | $X^1$ | $X^2$ | $X^3$ | $X^4$ | $R^2$ |
|---|---|---|---|---|---|---|
| A-0779 | F | CH | CH | CH | CH | $CH_2SO_2$(pyridin-2-yl) |
| A-0780 | F | CH | CH | CH | CH | $CH_2SCH_2$(pyridin-2-yl) |
| A-0781 | F | CH | CH | CH | CH | $CH_2SOCH_2$(pyridin-2-yl) |
| A-0782 | F | CH | CH | CH | CH | $CH_2SO_2CH_2$(pyridin-2-yl) |
| A-0783 | F | CH | CH | CH | CH | $CH_2S$(pyridin-3-yl) |
| A-0784 | F | CH | CH | CH | CH | $CH_2SO$(pyridin-3-yl) |
| A-0785 | F | CH | CH | CH | CH | $CH_2SO_2$(pyridin-3-yl) |
| A-0786 | F | CH | CH | CH | CH | $CH_2SCH_2$(pyridin-3-yl) |
| A-0787 | F | CH | CH | CH | CH | $CH_2SOCH_2$(pyridin-3-yl) |
| A-0788 | F | CH | CH | CH | CH | $CH_2SO_2CH_2$(pyridin-3-yl) |
| A-0789 | F | CH | CH | CH | CH | $CH_2S$(pyridin-4-yl) |
| A-0790 | F | CH | CH | CH | CH | $CH_2SO$(pyridin-4-yl) |
| A-0791 | F | CH | CH | CH | CH | $CH_2SO_2$(pyridin-4-yl) |
| A-0792 | F | CH | CH | CH | CH | $CH_2SCH_2$(pyridin-4-yl) |

[Table 20]

| Compound Number | $R^1$ | $X^1$ | $X^2$ | $X^3$ | $X^4$ | $R^2$ |
|---|---|---|---|---|---|---|
| A-0793 | F | CH | CH | CH | CH | $CH_2SOCH_2$(pyridin-4-yl) |
| A-0794 | F | CH | CH | CH | CH | $CH_2SO_2CH_2$(pyridin-4-yl) |
| A-0795 | F | CH | CH | CH | CH | $CH_2CH$(=NOH) |
| A-0796 | F | CH | CH | CH | CH | $CH_2CH$(=NOMe) |
| A-0797 | F | CH | CH | CH | CH | $CH_2CMe$(=NOMe) |
| A-0798 | F | CH | CH | CH | CH | $CH_2CEt$(=NOMe) |
| A-0799 | F | CH | CH | CH | CH | $CH_2Cn$-Pr(=NOMe) |
| A-0800 | F | CH | CH | CH | CH | $CH_2CMe$(=NOEt) |
| A-0801 | F | CH | CH | CH | CH | $CH_2CMe(=NOCH_2CH=CH_2)$ |
| A-0802 | F | CH | CH | CH | CH | $CH_2CMe$(=NOPh) |
| A-0803 | F | CH | CH | CH | CH | $CH_2CMe(=NOCH_2Ph)$ |
| A-0804 | F | CH | CH | CH | CH | $CH_2CMe$[=NO(pyridin-2-yl)] |
| A-0805 | F | CH | CH | CH | CH | $CH_2CMe$[=NO(pyridin-3-yl)] |
| A-0806 | F | CH | CH | CH | CH | $CH_2CMe$[=NO(pyridin-4-yl)] |
| A-0807 | F | CH | CH | CH | CH | $CH_2CMe[=NOCH_2$(pyridin-2-yl)] |
| A-0808 | F | CH | CH | CH | CH | $CH_2CMe[=NOCH_2$(pyridin-3-yl)] |
| A-0809 | F | CH | CH | CH | CH | $CH_2CMe[=NOCH_2$(pyridin-4-yl)] |

(continued)

| Compound Number | $R^1$ | $X^1$ | $X^2$ | $X^3$ | $X^4$ | $R^2$ |
|---|---|---|---|---|---|---|
| A-0810 | F | CH | CH | CH | CH | dihydrofuran-2(3H)-one-3-yl |
| A-0811 | F | CH | CH | CH | CH | $CH_2OCO_2Me$ |
| A-0812 | F | CH | CH | CH | CH | $CH_2OCO_2Et$ |
| A-0813 | F | CH | CH | CH | CH | $CH_2OCO_2n\text{-}Pr$ |
| A-0814 | F | CH | CH | CH | CH | $CH_2OCO_2i\text{-}Pr$ |
| A-0815 | F | CH | CH | CH | CH | $CH_2OCO_2c\text{-}Pr$ |
| A-0816 | F | CH | CH | CH | CH | $CH_2OCO_2CH_2c\text{-}Pr$ |
| A-0817 | F | CH | CH | CH | CH | $CH_2OCO_2n\text{-}Bu$ |
| A-0818 | F | CH | CH | CH | CH | $CH_2OCO_2i\text{-}Bu$ |
| A-0819 | F | CH | CH | CH | CH | $CH_2OCO_2t\text{-}Bu$ |
| A-0820 | F | CH | CH | CH | CH | $CH_2OCO_2CF_3$ |
| A-0821 | F | CH | CH | CH | CH | $CH_2OCO_2CH_2CF_3$ |
| A-0822 | F | CH | CH | CH | CH | $CH_2OCO_2CHF_2$ |
| A-0823 | F | CH | CH | CH | CH | $CH_2OCO_2CH_2CHF_2$ |
| A-0824 | F | CH | CH | CH | CH | $CH_2CH_2OCO_2Me$ |
| A-0825 | F | CH | CH | CH | CH | $CH_2CH_2OCO_2Et$ |
| A-0826 | F | CH | CH | CH | CH | $CH_2OCO_2Ph$ |
| A-0827 | F | CH | CH | CH | CH | $CH_2OCO_2CH_2Ph$ |
| A-0828 | F | CH | CH | CH | CH | $CH_2OCO_2$(pyridin-2-yl) |
| A-0829 | F | CH | CH | CH | CH | $CH_2OCO_2$(pyridin-3-yl) |
| A-0830 | F | CH | CH | CH | CH | $CH_2OCO_2$(pyridin-4-yl) |
| A-0831 | F | CH | CH | CH | CH | $CH_2OCO_2CH_2$(pyridin-2-yl) |
| A-0832 | F | CH | CH | CH | CH | $CH_2OCO_2CH_2$(pyridin-3-yl) |
| A-0833 | F | CH | CH | CH | CH | $CH_2OCO_2CH_2$(pyridin-4-yl) |
| A-0834 | F | CH | CH | CH | CH | $CH_2NHCONHMe$ |

[Table 21]

| Compound Number | $R^1$ | $X^1$ | $X^2$ | $X^3$ | $X^4$ | $R^2$ |
|---|---|---|---|---|---|---|
| A-0835 | F | CH | CH | CH | CH | $CH_2NHCONHEt$ |
| A-0836 | F | CH | CH | CH | CH | $CH_2NHCONHn\text{-}Pr$ |
| A-0837 | F | CH | CH | CH | CH | $CH_2NHCONHi\text{-}Pr$ |
| A-0838 | F | CH | CH | CH | CH | $CH_2NHCONHc\text{-}Pr$ |
| A-0839 | F | CH | CH | CH | CH | $CH_2NHCONHCH_2c\text{-}Pr$ |
| A-0840 | F | CH | CH | CH | CH | $CH_2NHCONHn\text{-}Bu$ |

(continued)

| Compound Number | $R^1$ | $X^1$ | $X^2$ | $X^3$ | $X^4$ | $R^2$ |
|---|---|---|---|---|---|---|
| A-0841 | F | CH | CH | CH | CH | $CH_2$NHCONHi-Bu |
| A-0842 | F | CH | CH | CH | CH | $CH_2$NHCONHt-Bu |
| A-0843 | F | CH | CH | CH | CH | $CH_2NHCONHCF_3$ |
| A-0844 | F | CH | CH | CH | CH | $CH_2NHCONHCH_2CF_3$ |
| A-0845 | F | CH | CH | CH | CH | $CH_2NHCONHCHF_2$ |
| A-0846 | F | CH | CH | CH | CH | $CH_2NHCONHCH_2CHF_2$ |
| A-0847 | F | CH | CH | CH | CH | $CH_2CH_2NHCONHMe$ |
| A-0848 | F | CH | CH | CH | CH | $CH_2CH_2NHCONHEt$ |
| A-0849 | F | CH | CH | CH | CH | $CH_2NHCONHPh$ |
| A-0850 | F | CH | CH | CH | CH | $CH_2NHCONHCH_2Ph$ |
| A-0851 | F | CH | CH | CH | CH | $CH_2$NHCONH(pyridin-2-yl) |
| A-0852 | F | CH | CH | CH | CH | $CH_2$NHCONH(pyridin-3-yl) |
| A-0853 | F | CH | CH | CH | CH | $CH_2$NHCONH(pyridin-4-yl) |
| A-0854 | F | CH | CH | CH | CH | $CH_2NHCONH_2CH_2$(pyridin-2-yl) |
| A-0855 | F | CH | CH | CH | CH | $CH_2NHCONHCH_2$(pyridin-3-yl) |
| A-0856 | F | CH | CH | CH | CH | $CH_2NHCONHCH_2$(pyridin-4-yl) |
| A-0857 | F | CH | CH | CH | CH | $CH_2CONHNH_2$ |
| A-0858 | F | CH | CH | CH | CH | $CH_2CONHNMe_2$ |
| A-0859 | F | CH | CH | CH | CH | $CH_2CONHNHCO_2$t-Bu |
| A-0860 | F | CH | CH | CH | CH | $CH_2CHCCl_2$ |
| A-0861 | F | CH | CH | CH | CH | $CH_2CO_2CH_2CH_2$(morpholin-4-yl) |
| A-0862 | F | CH | CH | CH | CH | $CH_2CO_2CH_2CH_2OMe$ |
| A-0863 | F | CMe | CH | CH | CH | $CH_2CO_2H$ |
| A-0864 | F | CMe | CH | CH | CH | $CH_2CO_2Et$ |
| A-0865 | F | CMe | CH | CH | CH | $CH_2CO_2iPr$ |
| A-0866 | F | CMe | CH | CH | CH | $CH_2CO_2cPr$ |
| A-0867 | F | CMe | CH | CH | CH | $CH_2CO_2tBu$ |
| A-0868 | F | CF | CH | CH | CH | $CH_2CO_2Et$ |
| A-0869 | F | N | CH | CH | CH | $CH_2CO_2Et$ |
| A-0870 | F | CH | CH | CH | CH | $CH_2CH_2NHCO_2tBu$ |
| A-0871 | F | CH | CH | CH | CH | $CH_2CH_2N(COCF_3)CO_2tBu$ |
| A-0872 | F | CH | CH | CH | CH | $CH_2CH_2NMe_2$ |
| A-0873 | F | CH | CH | CH | CH | $CH_2$CONHn-Pen |
| A-0874 | F | CH | CH | CH | CH | $CH_2$CONHn-Hex |

(continued)

| Compound Number | $R^1$ | $X^1$ | $X^2$ | $X^3$ | $X^4$ | $R^2$ |
|---|---|---|---|---|---|---|
| A-0875 | F | CH | CH | CH | CH | $CH_2CONHCH_2CH_2OMe$ |
| A-0876 | F | CH | CH | CH | CH | $CH_2CH(Cl)CH_2SF_5$ |

[Table 22]

| Compound Number | $R^1$ | $X^1$ | $X^2$ | $X^3$ | $X^4$ | $R^2$ |
|---|---|---|---|---|---|---|
| A-0877 | F | CH | CH | CH | CH | $CH_2CH=CHSF_5$ |
| A-0878 | F | CH | CH | CH | CH | $CH_2CO_2$(tetrahydrofuran-3-yl) |
| A-0879 | F | CH | CH | CH | CH | 1-($CO_2H$)-c-Pr |
| A-0880 | F | CH | CH | CH | CH | 1-($CO_2H$)-c-Bu |
| A-0881 | F | CH | CH | CH | CH | 1-($CO_2Me$)-c-Pr |
| A-0882 | F | CH | CH | CH | CH | 1-($CO_2Me$)-c-Bu |
| A-0883 | F | CH | CH | CH | CH | 1-($CO_2Et$)-c-Pr |
| A-0884 | F | CH | CH | CH | CH | 1-($CO_2Et$)-c-Bu |
| A-0885 | F | CH | CH | CH | CH | 1-($CO_2nPr$)-c-Pr |
| A-0886 | F | CH | CH | CH | CH | 1-($CO_2nPr$)-c-Bu |
| A-0887 | F | CH | CH | CH | CH | 1-($CO_2iPr$)-c-Pr |
| A-0888 | F | CH | CH | CH | CH | 1-($CO_2iPr$)-c-Bu |
| A-0889 | F | CH | CH | CH | CH | 1-($CO_2cPr$)-c-Pr |
| A-0890 | F | CH | CH | CH | CH | 1-($CO_2cPr$)-c-Bu |
| A-0891 | F | CH | CH | CH | CH | 1-($CO_2cBu$)-c-Pr |
| A-0892 | F | CH | CH | CH | CH | 1-($CO_2cBu$)-c-Bu |
| A-0893 | F | CF | CH | CH | CH | $CH_2CO_2H$ |
| A-0894 | F | CH | CF | CH | CH | $CH_2CO_2H$ |
| A-0895 | F | CH | CMe | CH | CH | $CH_2CO_2H$ |
| A-0896 | Cl | CH | CH | CH | CH | $CH_2CO_2H$ |
| A-0897 | F | N | CH | CH | CH | $CH_2CO_2H$ |
| A-0898 | F | CH | N | CH | CH | $CH_2CO_2H$ |
| A-0899 | F | N | N | CH | CH | $CH_2CO_2H$ |
| A-0900 | F | N | CH | N | CH | $CH_2CO_2H$ |
| A-0901 | F | N | CH | CH | N | $CH_2CO_2H$ |
| A-0902 | F | CH | N | CH | N | $CH_2CO_2H$ |
| A-0903 | H | CH | CH | CH | CH | $CH_2CO_2H$ |
| A-0904 | F | CF | CH | CH | CH | $CH_2CO_2Me$ |
| A-0905 | F | CH | CF | CH | CH | $CH_2CO_2Me$ |

(continued)

| Compound Number | $R^1$ | $X^1$ | $X^2$ | $X^3$ | $X^4$ | $R^2$ |
|---|---|---|---|---|---|---|
| A-0906 | F | CMe | CH | CH | CH | $CH_2CO_2Me$ |
| A-0907 | F | CH | CMe | CH | CH | $CH_2CO_2Me$ |
| A-0908 | Cl | CH | CH | CH | CH | $CH_2CO_2Me$ |
| A-0909 | F | N | CH | CH | CH | $CH_2CO_2Me$ |
| A-0910 | F | CH | N | CH | CH | $CH_2CO_2Me$ |
| A-0911 | F | N | N | CH | CH | $CH_2CO_2Me$ |
| A-0912 | F | N | CH | N | CH | $CH_2CO_2Me$ |
| A-0913 | F | N | CH | CH | N | $CH_2CO_2Me$ |
| A-0914 | F | CH | N | CH | N | $CH_2CO_2Me$ |
| A-0915 | H | CH | CH | CH | CH | $CH_2CO_2Me$ |
| A-0916 | F | CH | CF | CH | CH | $CH_2CO_2Et$ |
| A-0917 | F | CH | CMe | CH | CH | $CH_2CO_2Et$ |
| A-0918 | Cl | CH | CH | CH | CH | $CH_2CO_2Et$ |

[Table 23]

| Compound Number | $R^1$ | $X^1$ | $X^2$ | $X^3$ | $X^4$ | $R^2$ |
|---|---|---|---|---|---|---|
| A-0919 | F | CH | N | CH | CH | $CH_2CO_2Et$ |
| A-0920 | F | N | N | CH | CH | $CH_2CO_2Et$ |
| A-0921 | F | N | CH | N | CH | $CH_2CO_2Et$ |
| A-0922 | F | N | CH | CH | N | $CH_2CO_2Et$ |
| A-0923 | F | CH | N | CH | N | $CH_2CO_2Et$ |
| A-0924 | H | CH | CH | CH | CH | $CH_2CO_2Et$ |
| A-0925 | F | CF | CH | CH | CH | $CH_2CO_2nPr$ |
| A-0926 | F | CH | CF | CH | CH | $CH_2CO_2nPr$ |
| A-0927 | F | CMe | CH | CH | CH | $CH_2CO_2nPr$ |
| A-0928 | F | CH | CMe | CH | CH | $CH_2CO_2nPr$ |
| A-0929 | Cl | CH | CH | CH | CH | $CH_2CO_2nPr$ |
| A-0930 | F | N | CH | CH | CH | $CH_2CO_2nPr$ |
| A-0931 | F | CH | N | CH | CH | $CH_2CO_2nPr$ |
| A-0932 | F | N | N | CH | CH | $CH_2CO_2nPr$ |
| A-0933 | F | N | CH | N | CH | $CH_2CO_2nPr$ |
| A-0934 | F | N | CH | CH | N | $CH_2CO_2nPr$ |
| A-0935 | F | CH | N | CH | N | $CH_2CO_2nPr$ |
| A-0936 | H | CH | CH | CH | CH | $CH_2CO_2nPr$ |

(continued)

| Compound Number | $R^1$ | $X^1$ | $X^2$ | $X^3$ | $X^4$ | $R^2$ |
|---|---|---|---|---|---|---|
| A-0937 | F | CF | CH | CH | CH | $CH_2CO_2$iPr |
| A-0938 | F | CH | CF | CH | CH | $CH_2CO_2$iPr |
| A-0939 | F | CH | CMe | CH | CH | $CH_2CO_2$iPr |
| A-0940 | Cl | CH | CH | CH | CH | $CH_2CO_2$iPr |
| A-0941 | F | N | CH | CH | CH | $CH_2CO_2$iPr |
| A-0942 | F | CH | N | CH | CH | $CH_2CO_2$iPr |
| A-0943 | F | N | N | CH | CH | $CH_2CO_2$iPr |
| A-0944 | F | N | CH | N | CH | $CH_2CO_2$iPr |
| A-0945 | F | N | CH | CH | N | $CH_2CO_2$iPr |
| A-0946 | F | CH | N | CH | N | $CH_2CO_2$iPr |
| A-0947 | H | CH | CH | CH | CH | $CH_2CO_2$iPr |
| A-0948 | F | CF | CH | CH | CH | $CH_2CO_2$cPr |
| A-0949 | F | CH | CF | CH | CH | $CH_2CO_2$cPr |
| A-0950 | F | CH | CMe | CH | CH | $CH_2CO_2$cPr |
| A-0951 | Cl | CH | CH | CH | CH | $CH_2CO_2$cPr |
| A-0952 | F | N | CH | CH | CH | $CH_2CO_2$cPr |
| A-0953 | F | CH | N | CH | CH | $CH_2CO_2$cPr |
| A-0954 | F | N | N | CH | CH | $CH_2CO_2$cPr |
| A-0955 | F | N | CH | N | CH | $CH_2CO_2$cPr |
| A-0956 | F | N | CH | CH | N | $CH_2CO_2$cPr |
| A-0957 | F | CH | N | CH | N | $CH_2CO_2$cPr |
| A-0958 | H | CH | CH | CH | CH | $CH_2CO_2$cPr |

[Table 24]

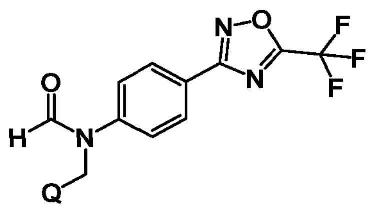

| Compound Number | Q |
|---|---|
| B-0001 | naphthalen-1-yl |
| B-0002 | naphthalen-2-yl |
| B-0003 | quinolin-2-yl |
| B-0004 | quinolin-3-yl |
| B-0005 | quinolin-6-yl |

(continued)

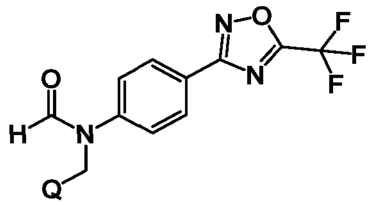

| Compound Number | Q |
|---|---|
| B-0006 | quinolin-7-yl |
| B-0007 | 1H-pyrrolo[2,3-b]pyridin-3-yl |
| B-0008 | benzo[d][1,3]dioxol-4-yl |
| B-0009 | benzo[d][1,3]dioxol-5-yl |
| B-0010 | benzo[d][1,3]dioxol-4-yl(2,2-$F_2$) |
| B-0011 | benzo[d][1,3]dioxol-5-yl(2,2-$F_2$) |
| B-0012 | pyridin-2-yl |
| B-0013 | $CH_2$pyridin-2-yl |
| B-0014 | pyridin-2-yl(3-F) |
| B-0015 | pyridin-2-yl(4-F) |
| B-0016 | pyridin-2-yl(5-F) |
| B-0017 | pyridin-2-yl(6-F) |
| B-0018 | pyridin-2-yl(3-Cl) |
| B-0019 | pyridin-2-yl(4-Cl) |
| B-0020 | pyridin-2-yl(5-Cl) |
| B-0021 | pyridin-2-yl(6-Cl) |
| B-0022 | pyridin-2-yl(3-Me) |
| B-0023 | pyridin-2-yl(4-Me) |
| B-0024 | pyridin-2-yl(5-Me) |
| B-0025 | pyridin-2-yl(6-Me) |
| B-0026 | pyridin-2-yl(3-OMe) |
| B-0027 | pyridin-2-yl(4-OMe) |
| B-0028 | pyridin-2-yl(5-OMe) |
| B-0029 | pyridin-2-yl(6-OMe) |
| B-0030 | pyridin-2-yl(3-CN) |
| B-0031 | pyridin-2-yl(4-CN) |
| B-0032 | pyridin-2-yl(5-CN) |
| B-0033 | pyridin-2-yl(6-CN) |
| B-0034 | pyridin-2-yl(3-$CF_3$) |
| B-0035 | pyridin-2-yl(4-$CF_3$) |
| B-0036 | pyridin-2-yl(5-$CF_3$) |

[Table 25]

| Compound Number | Q |
|---|---|
| B-0037 | pyridin-2-yl(6-$CF_3$) |
| B-0038 | pyridin-2-yl(3-$CO_2Et$) |
| B-0039 | pyridin-2-yl(4-$CO_2Et$) |
| B-0040 | pyridin-2-yl(5-$CO_2Et$) |
| B-0041 | pyridin-2-yl(6-$CO_2Et$) |
| B-0042 | pyridin-3-yl |
| B-0043 | $CH_2$pyridin-3-yl |
| B-0044 | pyridin-3-yl(2-F) |
| B-0045 | pyridin-3-yl(4-F) |
| B-0046 | pyridin-3-yl(5-F) |
| B-0047 | pyridin-3-yl(6-F) |
| B-0048 | pyridin-3-yl(2-Cl) |
| B-0049 | pyridin-3-yl(4-Cl) |
| B-0050 | pyridin-3-yl(5-Cl) |
| B-0051 | pyridin-3-yl(6-Cl) |
| B-0052 | pyridin-3-yl(2-Me) |
| B-0053 | pyridin-3-yl(4-Me) |
| B-0054 | pyridin-3-yl(5-Me) |
| B-0055 | pyridin-3-yl(6-Me) |
| B-0056 | pyridin-3-yl(2-OMe) |
| B-0057 | pyridin-3-yl(4-OMe) |
| B-0058 | pyridin-3-yl(5-OMe) |
| B-0059 | pyridin-3-yl(6-OMe) |
| B-0060 | pyridin-3-yl(2-CN) |
| B-0061 | pyridin-3-yl(4-CN) |
| B-0062 | pyridin-3-yl(5-CN) |
| B-0063 | pyridin-3-yl(6-CN) |
| B-0064 | pyridin-3-yl(2-$CF_3$) |
| B-0065 | pyridin-3-yl(4-$CF_3$) |
| B-0066 | pyridin-3-yl(5-$CF_3$) |
| B-0067 | pyridin-3-yl(6-$CF_3$) |
| B-0068 | pyridin-3-yl(2-$CO_2Et$) |
| B-0069 | pyridin-3-yl(4-$CO_2Et$) |
| B-0070 | pyridin-3-yl(5-$CO_2Et$) |
| B-0071 | pyridin-3-yl(6-$CO_2Et$) |
| B-0072 | pyridin-4-yl |

(continued)

| Compound Number | Q |
|---|---|
| B-0073 | $CH_2$pyridin-4-yl |
| B-0074 | pyridin-4-yl(2-F) |
| B-0075 | pyridin-4-yl(3-F) |
| B-0076 | pyridin-4-yl(2-Cl) |
| B-0077 | pyridin-4-yl(3-Cl) |
| B-0078 | pyridin-4-yl(2-Me) |

[Table 26]

| Compound Number | Q |
|---|---|
| B-0079 | pyridin-4-yl(3-Me) |
| B-0080 | pyridin-4-yl(2-OMe) |
| B-0081 | pyridin-4-yl(3-OMe) |
| B-0082 | pyridin-4-yl(2-CN) |
| B-0083 | pyridin-4-yl(3-CN) |
| B-0084 | pyridin-4-yl(2-$CF_3$) |
| B-0085 | pyridin-4-yl(3-$CF_3$) |
| B-0086 | pyridin-4-yl(2-$CO_2$Et) |
| B-0087 | pyridin-4-yl(3-$CO_2$Et) |
| B-0088 | pyrimidin-2-yl |
| B-0089 | $CH_2$pyrimidin-2-yl |
| B-0090 | pyrimidin-2-yl(4-F) |
| B-0091 | pyrimidin-2-yl(5-F) |
| B-0092 | pyrimidin-2-yl(4-Cl) |
| B-0093 | pyrimidin-2-yl(5-Cl) |
| B-0094 | pyrimidin-2-yl(4-Me) |
| B-0095 | pyrimidin-2-yl(5-Me) |
| B-0096 | pyrimidin-2-yl(4-OMe) |
| B-0097 | pyrimidin-2-yl(5-OMe) |
| B-0098 | pyrimidin-2-yl(4-CN) |
| B-0099 | pyrimidin-2-yl(5-CN) |
| B-0100 | pyrimidin-2-yl(4-$CF_3$) |
| B-0101 | pyrimidin-2-yl(5-$CF_3$) |
| B-0102 | pyrimidin-2-yl(4-$CO_2$Et) |
| B-0103 | pyrimidin-2-yl(5-$CO_2$Et) |
| B-0104 | pyrimidin-4-yl |
| B-0105 | $CH_2$pyrimidin-4-yl |

(continued)

| Compound Number | Q |
|---|---|
| B-0106 | pyrimidin-4-yl(2-F) |
| B-0107 | pyrimidin-4-yl(5-F) |
| B-0108 | pyrimidin-4-yl(6-F) |
| B-0109 | pyrimidin-4-yl(2-Cl) |
| B-0110 | pyrimidin-4-yl(5-Cl) |
| B-0111 | pyrimidin-4-yl(6-Cl) |
| B-0112 | pyrimidin-4-yl(2-Me) |
| B-0113 | pyrimidin-4-yl(5-Me) |
| B-0114 | pyrimidin-4-yl(6-Me) |
| B-0115 | pyrimidin-4-yl(2-OMe) |
| B-0116 | pyrimidin-4-yl(5-OMe) |
| B-0117 | pyrimidin-4-yl(6-OMe) |
| B-0118 | pyrimidin-4-yl(2-CN) |
| B-0119 | pyrimidin-4-yl(5-CN) |
| B-0120 | pyrimidin-4-yl(6-CN) |

[Table 27]

| Compound Number | Q |
|---|---|
| B-0121 | pyrimidin-4-yl(2-$CF_3$) |
| B-0122 | pyrimidin-4-yl(5-$CF_3$) |
| B-0123 | pyrimidin-4-yl(6-$CF_3$) |
| B-0124 | pyrimidin-4-yl(2-$CO_2$Et) |
| B-0125 | pyrimidin-4-yl(5-$CO_2$Et) |
| B-0126 | pyrimidin-4-yl(6-$CO_2$Et) |
| B-0127 | pyrimidin-5-yl |
| B-0128 | $CH_2$pyrimidin-5-yl |
| B-0129 | pyrimidin-5-yl(2-F) |
| B-0130 | pyrimidin-5-yl(4-F) |
| B-0131 | pyrimidin-5-yl(2-Cl) |
| B-0132 | pyrimidin-5-yl(4-Cl) |
| B-0133 | pyrimidin-5-yl(2-Me) |
| B-0134 | pyrimidin-5-yl(4-Me) |
| B-0135 | pyrimidin-5-yl(2-OMe) |
| B-0136 | pyrimidin-5-yl(4-OMe) |
| B-0137 | pyrimidin-5-yl(2-CN) |
| B-0138 | pyrimidin-5-yl(4-CN) |
| B-0139 | pyrimidin-5-yl(2-$CF_3$) |

(continued)

| Compound Number | Q |
|---|---|
| B-0140 | pyrimidin-5-yl(4-$CF_3$) |
| B-0141 | pyrimidin-5-yl(2-$CO_2$Et) |
| B-0142 | pyrimidin-5-yl(4-$CO_2$Et) |
| B-0143 | pyridazin-3-yl |
| B-0144 | $CH_2$pyridazin-3-yl |
| B-0145 | pyridazin-3-yl(4-F) |
| B-0146 | pyridazin-3-yl(5-F) |
| B-0147 | pyridazin-3-yl(6-F) |
| B-0148 | pyridazin-3-yl(4-Cl) |
| B-0149 | pyridazin-3-yl(5-Cl) |
| B-0150 | pyridazin-3-yl(6-Cl) |
| B-0151 | pyridazin-3-yl(4-Me) |
| B-0152 | pyridazin-3-yl(5-Me) |
| B-0153 | pyridazin-3-yl(6-Me) |
| B-0154 | pyridazin-3-yl(4-OMe) |
| B-0155 | pyridazin-3-yl(5-OMe) |
| B-0156 | pyridazin-3-yl(6-OMe) |
| B-0157 | pyridazin-3-yl(4-CN) |
| B-0158 | pyridazin-3-yl(5-CN) |
| B-0159 | pyridazin-3-yl(6-CN) |
| B-0160 | pyridazin-3-yl(4-$CF_3$) |
| B-0161 | pyridazin-3-yl(5-$CF_3$) |
| B-0162 | pyridazin-3-yl(6-$CF_3$) |

[Table 28]

| Compound Number | Q |
|---|---|
| B-0163 | pyridazin-3-yl(4-$CO_2$Et) |
| B-0164 | pyridazin-3-yl(5-$CO_2$Et) |
| B-0165 | pyridazin-3-yl(6-$CO_2$Et) |
| B-0166 | pyridazin-4-yl |
| B-0167 | $CH_2$pyridazin-4-yl |
| B-0168 | pyridazin-4-yl(3-F) |
| B-0169 | pyridazin-4-yl(5-F) |
| B-0170 | pyridazin-4-yl(6-F) |
| B-0171 | pyridazin-4-yl(3-Cl) |
| B-0172 | pyridazin-4-yl(5-Cl) |

(continued)

| Compound Number | Q |
|---|---|
| B-0173 | pyridazin-4-yl(6-Cl) |
| B-0174 | pyridazin-4-yl(3-Me) |
| B-0175 | pyridazin-4-yl(5-Me) |
| B-0176 | pyridazin-4-yl(6-Me) |
| B-0177 | pyridazin-4-yl(3-OMe) |
| B-0178 | pyridazin-4-yl(5-OMe) |
| B-0179 | pyridazin-4-yl(6-OMe) |
| B-0180 | pyridazin-4-yl(3-CN) |
| B-0181 | pyridazin-4-yl(5-CN) |
| B-0182 | pyridazin-4-yl(6-CN) |
| B-0183 | pyridazin-4-yl(3-$CF_3$) |
| B-0184 | pyridazin-4-yl(5-$CF_3$) |
| B-0185 | pyridazin-4-yl(6-$CF_3$) |
| B-0186 | pyridazin-4-yl(3-$CO_2$Et) |
| B-0187 | pyridazin-4-yl(5-$CO_2$Et) |
| B-0188 | pyridazin-4-yl(6-$CO_2$Et) |
| B-0189 | pyrazin-2-yl |
| B-0190 | $CH_2$pyrazin-2-yl |
| B-0191 | pyrazi n-2-yl(3-F) |
| B-0192 | pyrazi n-2-yl(5-F) |
| B-0193 | pyrazin-2-yl(6-F) |
| B-0194 | pyrazin-2-yl(3-Cl) |
| B-0195 | pyrazin-2-yl(5-Cl) |
| B-0196 | pyrazin-2-yl(6-Cl) |
| B-0197 | pyrazin-2-yl(3-Me) |
| B-0198 | pyrazin-2-yl(5-Me) |
| B-0199 | pyrazin-2-yl(6-Me) |
| B-0200 | pyrazi n-2-yl(3-O Me) |
| B-0201 | pyrazi n-2-yl(5-O Me) |
| B-0202 | pyrazi n-2-yl(6-O Me) |
| B-0203 | pyrazin-2-yl(3-CN) |
| B-0204 | pyrazin-2-yl(5-CN) |

[Table 29]

| Compound Number | Q |
|---|---|
| B-0205 | pyrazin-2-yl(6-CN) |
| B-0206 | pyrazin-2-yl(3-$CF_3$) |

(continued)

| Compound Number | Q |
|---|---|
| B-0207 | pyrazin-2-yl(5-$CF_3$) |
| B-0208 | pyrazin-2-yl(6-$CF_3$) |
| B-0209 | pyrazin-2-yl(3-$CO_2$Et) |
| B-0210 | pyrazi n-2-yl(5-$CO_2$Et) |
| B-0211 | pyrazi n-2-yl(6-$CO_2$Et) |
| B-0212 | 1,3,5-triazin-2-yl |
| B-0213 | $CH_2$1,3,5-triazin-2-yl |
| B-0214 | thiophen-2-yl |
| B-0215 | $CH_2$thiophen-2-yl |
| B-0216 | thiophen-2-yl(3-F) |
| B-0217 | thiophen-2-yl(4-F) |
| B-0218 | thiophen-2-yl(5-F) |
| B-0219 | thiophen-2-yl(3-Cl) |
| B-0220 | thiophen-2-yl(4-Cl) |
| B-0221 | thiophen-2-yl(5-Cl) |
| B-0222 | thiophen-2-yl(3-Me) |
| B-0223 | thiophen-2-yl(4-Me) |
| B-0224 | thiophen-2-yl(5-Me) |
| B-0225 | thiophen-2-yl(3-OMe) |
| B-0226 | thiophen-2-yl(4-OMe) |
| B-0227 | thiophen-2-yl(5-OMe) |
| B-0228 | thiophen-2-yl(3-CN) |
| B-0229 | thiophen-2-yl(4-CN) |
| B-0230 | thiophen-2-yl(5-CN) |
| B-0231 | thiophen-2-yl(3-$CF_3$) |
| B-0232 | thiophen-2-yl(4-$CF_3$) |
| B-0233 | thiophen-2-yl(5-$CF_3$) |
| B-0234 | thiophen-2-yl(3-$CO_2$Et) |
| B-0235 | thiophen-2-yl(4-$CO_2$Et) |
| B-0236 | thiophen-2-yl(5-$CO_2$Et) |
| B-0237 | thiophen-3-yl |
| B-0238 | $CH_2$thiophen-3-yl |
| B-0239 | thiophen-3-yl(2-F) |
| B-0240 | thiophen-3-yl(4-F) |
| B-0241 | thiophen-3-yl(5-F) |
| B-0242 | thiophen-3-yl(2-Cl) |

(continued)

| Compound Number | Q |
|---|---|
| B-0243 | thiophen-3-yl(4-Cl) |
| B-0244 | thiophen-3-yl(5-Cl) |
| B-0245 | thiophen-3-yl(2-Me) |
| B-0246 | thiophen-3-yl(4-Me) |

[Table 30]

| Compound Number | Q |
|---|---|
| B-0247 | thiophen-3-yl(5-Me) |
| B-0248 | thiophen-3-yl(2-OMe) |
| B-0249 | thiophen-3-yl(4-OMe) |
| B-0250 | thiophen-3-yl(5-OMe) |
| B-0251 | thiophen-3-yl(2-CN) |
| B-0252 | thiophen-3-yl(4-CN) |
| B-0253 | thiophen-3-yl(5-CN) |
| B-0254 | thiophen-3-yl(2-$CF_3$) |
| B-0255 | thiophen-3-yl(4-$CF_3$) |
| B-0256 | thiophen-3-yl(5-$CF_3$) |
| B-0257 | thiophen-3-yl(2-$CO_2Et$) |
| B-0258 | thiophen-3-yl(4-$CO_2Et$) |
| B-0259 | thiophen-3-yl(5-$CO_2Et$) |
| B-0260 | furan-2-yl |
| B-0261 | $CH_2$furan-2-yl |
| B-0262 | furan-2-yl(3-F) |
| B-0263 | furan-2-yl(4-F) |
| B-0264 | furan-2-yl(5-F) |
| B-0265 | furan-2-yl(3-Cl) |
| B-0266 | furan-2-yl(4-Cl) |
| B-0267 | furan-2-yl(5-Cl) |
| B-0268 | furan-2-yl(3-Me) |
| B-0269 | furan-2-yl(4-Me) |
| B-0270 | furan-2-yl(5-Me) |
| B-0271 | furan-2-yl(3-OMe) |
| B-0272 | furan-2-yl(4-OMe) |
| B-0273 | furan-2-yl(5-OMe) |
| B-0274 | furan-2-yl(3-CN) |
| B-0275 | furan-2-yl(4-CN) |
| B-0276 | furan-2-yl(5-CN) |

(continued)

| Compound Number | Q |
|---|---|
| B-0277 | furan-2-yl(3-$CF_3$) |
| B-0278 | furan-2-yl(4-$CF_3$) |
| B-0279 | furan-2-yl(5-$CF_3$) |
| B-0280 | furan-2-yl(3-$CO_2$Et) |
| B-0281 | furan-2-yl(4-$CO_2$Et) |
| B-0282 | furan-2-yl(5-$CO_2$Et) |
| B-0283 | furan-3-yl |
| B-0284 | $CH_2$furan-3-yl |
| B-0285 | furan-3-yl(2-F) |
| B-0286 | furan-3-yl(4-F) |
| B-0287 | furan-3-yl(5-F) |
| B-0288 | furan-3-yl(2-Cl) |

[Table 31]

| Compound Number | Q |
|---|---|
| B-0289 | furan-3-yl(4-Cl) |
| B-0290 | furan-3-yl(5-Cl) |
| B-0291 | furan-3-yl(2-Me) |
| B-0292 | furan-3-yl(4-Me) |
| B-0293 | furan-3-yl(5-Me) |
| B-0294 | furan-3-yl(2-OMe) |
| B-0295 | furan-3-yl(4-OMe) |
| B-0296 | furan-3-yl(5-OMe) |
| B-0297 | furan-3-yl(2-CN) |
| B-0298 | furan-3-yl(4-CN) |
| B-0299 | furan-3-yl(5-CN) |
| B-0300 | furan-3-yl(2-$CF_3$) |
| B-0301 | furan-3-yl(4-$CF_3$) |
| B-0302 | furan-3-yl(5-$CF_3$) |
| B-0303 | furan-3-yl(2-$CO_2$Et) |
| B-0304 | furan-3-yl(4-$CO_2$Et) |
| B-0305 | furan-3-yl(5-$CO_2$Et) |
| B-0306 | 1H-pyrrol-1-yl |
| B-0307 | $CH_2$1H-pyrrol-1-yl |
| B-0308 | 1 H-pyrrol-2-yl |
| B-0309 | $CH_2$1H-pyrrol-2-yl |

(continued)

| Compound Number | Q |
|---|---|
| B-0310 | 1H-pyrrol-2-yl(1-Me) |
| B-0311 | $CH_2$1H-pyrrol-2-yl(1-Me) |
| B-0312 | 1H-pyrrol-3-yl |
| B-0313 | $CH_2$1H-pyrrol-3-yl |
| B-0314 | 1H-pyrrol-3-yl(1-Me) |
| B-0315 | $CH_2$1H-pyrrol-3-yl(1-Me) |
| B-0316 | 1H-pyrazol-1-yl |
| B-0317 | $CH_2$1H-pyrazol-1-yl |
| B-0318 | 1H-pyrazol-1-yl(3-F) |
| B-0319 | 1H-pyrazol-1-yl(4-F) |
| B-0320 | 1H-pyrazol-1-yl(5-F) |
| B-0321 | 1H-pyrazol-1-yl(3-Cl) |
| B-0322 | 1H-pyrazol-1-yl(4-Cl) |
| B-0323 | 1H-pyrazol-1-yl(5-Cl) |
| B-0324 | 1H-pyrazol-1-yl(3-Me) |
| B-0325 | 1H-pyrazol-1-yl(4-Me) |
| B-0326 | 1H-pyrazol-1-yl(5-Me) |
| B-0327 | 1H-pyrazol-1-yl(3-OMe) |
| B-0328 | 1H-pyrazol-1-yl(4-OMe) |
| B-0329 | 1H-pyrazol-1-yl(5-OMe) |
| B-0330 | 1H-pyrazol-1-yl(3-CN) |

[Table 32]

| Compound Number | Q |
|---|---|
| B-0331 | 1H-pyrazol-1-yl(4-CN) |
| B-0332 | 1H-pyrazol-1-yl(5-CN) |
| B-0333 | 1H-pyrazol-1-yl(3-$CF_3$) |
| B-0334 | 1H-pyrazol-1-yl(4-$CF_3$) |
| B-0335 | 1H-pyrazol-1-yl(5-$CF_3$) |
| B-0336 | 1H-pyrazol-1-yl(3-$CO_2$Et) |
| B-0337 | 1H-pyrazol-1-yl(4-$CO_2$Et) |
| B-0338 | 1H-pyrazol-1-yl(5-$CO_2$Et) |
| B-0339 | 1H-pyrazol-3-yl |
| B-0340 | $CH_2$1H-pyrazol-3-yl |
| B-0341 | 1H-pyrazol-3-yl(1-Me) |
| B-0342 | $CH_2$1H-pyrazol-3-yl(1-Me) |

(continued)

| Compound Number | Q |
|---|---|
| B-0343 | 1H-pyrazol-3-yl(4-F,1-Me) |
| B-0344 | 1H-pyrazol-3-yl(5-F,1-Me) |
| B-0345 | 1H-pyrazol-3-yl(4-Cl,1-Me) |
| B-0346 | 1H-pyrazol-3-yl(5-Cl,1-Me) |
| B-0347 | 1H-pyrazol-3-yl(1,4-$Me_2$) |
| B-0348 | 1H-pyrazol-3-yl(1,5-$Me_2$) |
| B-0349 | 1H-pyrazol-3-yl(4-OMe,1-Me) |
| B-0350 | 1H-pyrazol-3-yl(5-OMe,1-Me) |
| B-0351 | 1H-pyrazol-3-yl(4-CN,1-Me) |
| B-0352 | 1H-pyrazol-3-yl(5-CN,1-Me) |
| B-0353 | 1H-pyrazol-3-yl(1-Me,4-$CF_3$) |
| B-0354 | 1H-pyrazol-3-yl(1-Me,5-$CF_3$) |
| B-0355 | 1H-pyrazol-3-yl(1-Me,4-$CO_2$Et) |
| B-0356 | 1H-pyrazol-3-yl(1-Me,5-$CO_2$Et) |
| B-0357 | 1H-pyrazol-4-yl |
| B-0358 | $CH_2$1H-pyrazol-4-yl |
| B-0359 | 1H-pyrazol-4-yl(1-Me) |
| B-0360 | $CH_2$1H-pyrazol-4-yl(1-Me) |
| B-0361 | 1H-pyrazol-4-yl(3-F,1-Me) |
| B-0362 | 1H-pyrazol-4-yl(5-F,1-Me) |
| B-0363 | 1H-pyrazol-4-yl(3-Cl,1-Me) |
| B-0364 | 1H-pyrazol-4-yl(5-Cl,1-Me) |
| B-0365 | 1H-pyrazol-4-yl(1,3-$Me_2$) |
| B-0366 | 1H-pyrazol-4-yl(1,5-$Me_2$) |
| B-0367 | 1H-pyrazol-4-yl(3-OMe,1-Me) |
| B-0368 | 1H-pyrazol-4-yl(5-OMe,1-Me) |
| B-0369 | 1H-pyrazol-4-yl(3-CN,1-Me) |
| B-0370 | 1H-pyrazol-4-yl(5-CN,1-Me) |
| B-0371 | 1H-pyrazol-4-yl(1-Me,3-$CF_3$) |
| B-0372 | 1H-pyrazol-4-yl(1-Me,5-$CF_3$) |

[Table 33]

| Compound Number | Q |
|---|---|
| B-0373 | 1H-pyrazol-4-yl(1-Me,3-$CO_2$Et) |
| B-0374 | 1H-pyrazol-4-yl(1-Me,5-$CO_2$Et) |
| B-0375 | 1H-pyrazol-5-yl |

(continued)

| Compound Number | Q |
|---|---|
| B-0376 | $CH_2$1H-pyrazol-5-yl |
| B-0377 | 1H-pyrazol-5-yl(1-Me) |
| B-0378 | $CH_2$1H-pyrazol-5-yl(1-Me) |
| B-0379 | 1H-pyrazol-5-yl(3-F,1-Me) |
| B-0380 | 1H-pyrazol-5-yl(4-F,1-Me) |
| B-0381 | 1H-pyrazol-5-yl(3-Cl,1-Me) |
| B-0382 | 1H-pyrazol-5-yl(4-Cl,1-Me) |
| B-0383 | 1H-pyrazol-5-yl(1,3-$Me_2$) |
| B-0384 | 1H-pyrazol-5-yl(1,4-$Me_2$) |
| B-0385 | 1H-pyrazol-5-yl(3-OMe,1-Me) |
| B-0386 | 1H-pyrazol-5-yl(4-OMe,1-Me) |
| B-0387 | 1H-pyrazol-5-yl(3-CN,1-Me) |
| B-0388 | 1H-pyrazol-5-yl(4-CN,1-Me) |
| B-0389 | 1H-pyrazol-5-yl(1-Me,3-$CF_3$) |
| B-0390 | 1H-pyrazol-5-yl(1-Me,4-$CF_3$) |
| B-0391 | 1H-pyrazol-5-yl(1-Me,3-$CO_2$Et) |
| B-0392 | 1H-pyrazol-5-yl(1-Me,4-$CO_2$Et) |
| B-0393 | 1H-imidazol-1-yl |
| B-0394 | $CH_2$1H-imidazol-1-yl |
| B-0395 | 1H-imidazol-1-yl(2-F) |
| B-0396 | 1H-imidazol-1-yl(4-F) |
| B-0397 | 1H-imidazol-1-yl(5-F) |
| B-0398 | 1H-imidazol-1-yl(2-Cl) |
| B-0399 | 1H-imidazol-1-yl(4-Cl) |
| B-0400 | 1H-imidazol-1-yl(5-Cl) |
| B-0401 | 1H-imidazol-1-yl(2-Me) |
| B-0402 | 1H-imidazol-1-yl(4-Me) |
| B-0403 | 1H-imidazol-1-yl(5-Me) |
| B-0404 | 1H-imidazol-1 -yl(2-OMe) |
| B-0405 | 1H-imidazol-1-yl(4-OMe) |
| B-0406 | 1H-imidazol-1-yl(5-OMe) |
| B-0407 | 1H-imidazol-1-yl(2-CN) |
| B-0408 | 1H-imidazol-1-yl(4-CN) |
| B-0409 | 1H-imidazol-1-yl(5-CN) |
| B-0410 | 1H-imidazol-1-yl(2-$CF_3$) |
| B-0411 | 1H-imidazol-1-yl(4-$CF_3$) |

(continued)

| Compound Number | Q |
|---|---|
| B-0412 | 1H-imidazol-1-yl(5-$CF_3$) |
| B-0413 | 1H-imidazol-1-yl(2-$CO_2$Et) |
| B-0414 | 1H-imidazol-1-yl(4-$CO_2$Et) |

[Table 34]

| Compound Number | Q |
|---|---|
| B-0415 | 1H-imidazol-1-yl(5-$CO_2$Et) |
| B-0416 | 1H-imidazol-2-yl |
| B-0417 | $CH_2$1H-imidazol-2-yl |
| B-0418 | 1H-imidazol-2-yl(1-Me) |
| B-0419 | $CH_2$1H-imidazol-2-yl(1-Me) |
| B-0420 | 1H-imidazol-2-yl(4-F,1-Me) |
| B-0421 | 1H-imidazol-2-yl(5-F,1-Me) |
| B-0422 | 1H-imidazol-2-yl(4-Cl,1-Me) |
| B-0423 | 1H-imidazol-2-yl(5-Cl,1-Me) |
| B-0424 | 1H-imidazol-2-yl(1,4-$Me_2$) |
| B-0425 | 1H-imidazol-2-yl(1,5-$Me_2$) |
| B-0426 | 1H-imidazol-2-yl(4-OMe,1-Me) |
| B-0427 | 1H-imidazol-2-yl(5-OMe,1-Me) |
| B-0428 | 1H-imidazol-2-yl(4-CN,1-Me) |
| B-0429 | 1H-imidazol-2-yl(5-CN,1-Me) |
| B-0430 | 1H-imidazol-2-yl(1-Me,4-$CF_3$) |
| B-0431 | 1H-imidazol-2-yl(1-Me,5-$CF_3$) |
| B-0432 | 1H-imidazol-2-yl(1-Me,4-$CO_2$Et) |
| B-0433 | 1H-imidazol-2-yl(1-Me,5-$CO_2$Et) |
| B-0434 | 1H-imidazol-4-yl |
| B-0435 | $CH_2$1H-imidazol-4-yl |
| B-0436 | 1H-imidazol-4-yl(1-Me) |
| B-0437 | $CH_2$1H-imidazol-4-yl(1-Me) |
| B-0438 | 1H-imidazol-4-yl(2-F,1-Me) |
| B-0439 | 1H-imidazol-4-yl(5-F,1-Me) |
| B-0440 | 1H-imidazol-4-yl(2-Cl,1-Me) |
| B-0441 | 1H-imidazol-4-yl(5-Cl,1-Me) |
| B-0442 | 1H-imidazol-4-yl(1,2-$Me_2$) |
| B-0443 | 1H-imidazol-4-yl(1,5-$Me_2$) |
| B-0444 | 1H-imidazol-4-yl(2-OMe,1-Me) |

(continued)

| Compound Number | Q |
|---|---|
| B-0445 | 1H-imidazol-4-yl(5-OMe,1-Me) |
| B-0446 | 1H-imidazol-4-yl(2-CN,1-Me) |
| B-0447 | 1H-imidazol-4-yl(5-CN,1-Me) |
| B-0448 | 1H-imidazol-4-yl(1-Me,2-$CF_3$) |
| B-0449 | 1H-imidazol-4-yl(1-Me,5-$CF_3$) |
| B-0450 | 1H-imidazol-4-yl(1-Me,2-$CO_2$Et) |
| B-0451 | 1H-imidazol-4-yl(1-Me,5-$CO_2$Et) |
| B-0452 | 1H-imidazol-5-yl |
| B-0453 | $CH_2$1H-imidazol-5-yl |
| B-0454 | 1H-imidazol-5-yl(1-Me) |
| B-0455 | $CH_2$1H-imidazol-5-yl(1-Me) |
| B-0456 | 1H-imidazol-5-yl(2-F,1-Me) |

[Table 35]

| Compound Number | Q |
|---|---|
| B-0457 | 1H-imidazol-5-yl(4-F,1-Me) |
| B-0458 | 1H-imidazol-5-yl(2-Cl,1-Me) |
| B-0459 | 1H-imidazol-5-yl(4-Cl,1-Me) |
| B-0460 | 1H-imidazol-5-yl(1,2-$Me_2$) |
| B-0461 | 1H-imidazol-5-yl(1,4-$Me_2$) |
| B-0462 | 1H-imidazol-5-yl(2-OMe,1-Me) |
| B-0463 | 1H-imidazol-5-yl(4-OMe,1-Me) |
| B-0464 | 1H-imidazol-5-yl(2-CN,1-Me) |
| B-0465 | 1H-imidazol-5-yl(4-CN,1-Me) |
| B-0466 | 1H-imidazol-5-yl(1-Me,2-$CF_3$) |
| B-0467 | 1H-imidazol-5-yl(1-Me,4-$CF_3$) |
| B-0468 | 1H-imidazol-5-yl(1-Me,2-$CO_2$Et) |
| B-0469 | 1H-imidazol-5-yl(1-Me,4-$CO_2$Et) |
| B-0470 | 1H-1,2,4-triazol-1-yl |
| B-0471 | $CH_2$1H-1,2,4-triazol-1-yl |
| B-0472 | 1H-1,2,4-triazol-1-yl(3-F) |
| B-0473 | 1H-1,2,4-triazol-1-yl(5-F) |
| B-0474 | 1H-1,2,4-triazol-1-yl(3-Cl) |
| B-0475 | 1H-1,2,4-triazol-1-yl(5-Cl) |
| B-0476 | 1H-1,2,4-triazol-1-yl(3-Me) |
| B-0477 | 1H-1,2,4-triazol-1-yl(5-Me) |

(continued)

| Compound Number | Q |
|---|---|
| B-0478 | 1H-1,2,4-triazol-1-yl(3-OMe) |
| B-0479 | 1H-1,2,4-triazol-1-yl(5-OMe) |
| B-0480 | 1H-1,2,4-triazol-1-yl(3-CN) |
| B-0481 | 1H-1,2,4-triazol-1-yl(5-CN) |
| B-0482 | 1H-1,2,4-triazol-1-yl(3-$CF_3$) |
| B-0483 | 1H-1,2,4-triazol-1-yl(5-$CF_3$) |
| B-0484 | 1H-1,2,4-triazol-1-yl(3-$CO_2$Et) |
| B-0485 | 1H-1,2,4-triazol-1-yl(5-$CO_2$Et) |
| B-0486 | 1H-1,2,4-triazol-3-yl |
| B-0487 | $CH_2$1H-1,2,4-triazol-3-yl |
| B-0488 | 1H-1,2,4-triazol-3-yl(1-Me) |
| B-0489 | $CH_2$1H-1,2,4-triazol-3-yl(1-Me) |
| B-0490 | 1H-1,2,4-triazol-3-yl(5-F,1-Me) |
| B-0491 | 1H-1,2,4-triazol-3-yl(5-Cl,1-Me) |
| B-0492 | 1H-1,2,4-triazol-3-yl(1,5-$Me_2$) |
| B-0493 | 1H-1,2,4-triazol-3-yl(5-OMe,1-Me) |
| B-0494 | 1H-1,2,4-triazol-3-yl(5-CN, 1-Me) |
| B-0495 | 1H-1,2,4-triazol-3-yl(1-Me,5-$CF_3$) |
| B-0496 | 1H-1,2,4-triazol-3-yl(1-Me,5-$CO_2$Et) |
| B-0497 | 4H-1,2,4-triazol-4-yl |
| B-0498 | $CH_2$4H-1,2,4-triazol-4-yl |

[Table 36]

| Compound Number | Q |
|---|---|
| B-0499 | 4H-1,2,4-triazol-4-yl(3-F) |
| B-0500 | 4H-1,2,4-triazol-4-yl(3-Cl) |
| B-0501 | 4H-1,2,4-triazol-4-yl(3-Me) |
| B-0502 | 4H-1,2,4-triazol-4-yl(3-OMe) |
| B-0503 | 4H-1,2,4-triazol-4-yl(3-CN) |
| B-0504 | 4H-1,2,4-triazol-4-yl(3-$CF_3$) |
| B-0505 | 4H-1,2,4-triazol-4-yl(3-$CO_2$Et) |
| B-0506 | 1H-1,2,4-triazol-5-yl |
| B-0507 | $CH_2$1H-1,2,4-triazol-5-yl |
| B-0508 | 1H-1,2,4-triazol-5-yl(1-Me) |
| B-0509 | $CH_2$1H-1,2,4-triazol-5-yl(1-Me) |
| B-0510 | 1H-1,2,4-triazol-5-yl(3-F,1-Me) |

(continued)

| Compound Number | Q |
|---|---|
| B-0511 | 1H-1,2,4-triazol-5-yl(3-Cl,1-Me) |
| B-0512 | 1H-1,2,4-triazol-5-yl(1,3-$Me_2$) |
| B-0513 | 1H-1,2,4-triazol-5-yl(3-OMe,1-Me) |
| B-0514 | 1H-1,2,4-triazol-5-yl(3-CN,1-Me) |
| B-0515 | 1H-1,2,4-tri azol-5-yl(1-Me,3-$CF_3$) |
| B-0516 | 1H-1,2,4-triazol-5-yl(1-Me,3-$CO_2$Et) |
| B-0517 | 1H-1,2,3-triazol-1-yl |
| B-0518 | $CH_2$1H-1,2,3-triazol-1-yl |
| B-0519 | 1H-1,2,3-triazol-1-yl(4-Me) |
| B-0520 | 1H-1,2,3-triazol-1-yl(5-Me) |
| B-0521 | 1H-1,2,a-triazol-1-yl(4-$CO_2$Et) |
| B-0522 | 1H-1,2,3-triazol-1-yl(5-$CO_2$Et) |
| B-0523 | 2H-1,2,3-triazol-2-yl |
| B-0524 | $CH_2$2H-1,2,3-triazol-2-yl |
| B-0525 | 1H-tetrazol-1-yl |
| B-0526 | $CH_2$1H-tetrazol-1-yl |
| B-0527 | 2H-tetrazol-2-yl |
| B-0528 | $CH_2$2H-tetrazol-2-yl |
| B-0529 | 1H-tetrazol-5-yl |
| B-0530 | $CH_2$1H-tetrazol-5-yl |
| B-0531 | 1H-tetrazol-5-yl(1-Me) |
| B-0532 | $CH_2$1H-tetrazol-5-yl(1-Me) |
| B-0533 | thiazol-2-yl |
| B-0534 | $CH_2$thiazol-2-yl |
| B-0535 | thiazol-2-yl(4-F) |
| B-0536 | thiazol-2-yl(5-F) |
| B-0537 | thiazol-2-yl(4-Cl) |
| B-0538 | thiazol-2-yl(5-Cl) |
| B-0539 | thiazol-2-yl(4-Me) |
| B-0540 | thiazol-2-yl(5-Me) |

[Table 37]

| Compound Number | Q |
|---|---|
| B-0541 | thiazol-2-yl(4-OMe) |
| B-0542 | thiazol-2-yl(5-OMe) |
| B-0543 | thiazol-2-yl(4-CN) |

(continued)

| Compound Number | Q |
|---|---|
| B-0544 | thiazol-2-yl(5-CN) |
| B-0545 | thiazol-2-yl(4-$CF_3$) |
| B-0546 | thiazol-2-yl(5-$CF_3$) |
| B-0547 | thiazol-2-yl(4-$CO_2$Et) |
| B-0548 | thiazol-2-yl(5-$CO_2$Et) |
| B-0549 | thiazol-4-yl |
| B-0550 | $CH_2$thiazol-4-yl |
| B-0551 | thiazol-4-yl(2-F) |
| B-0552 | thiazol-4-yl(5-F) |
| B-0553 | thiazol-4-yl(2-Cl) |
| B-0554 | thiazol-4-yl(5-Cl) |
| B-0555 | thiazol-4-yl(2-Me) |
| B-0556 | thiazol-4-yl(5-Me) |
| B-0557 | thiazol-4-yl(2-OMe) |
| B-0558 | thiazol-4-yl(5-OMe) |
| B-0559 | thiazol-4-yl(2-CN) |
| B-0560 | thiazol-4-yl(5-CN) |
| B-0561 | thiazol-4-yl(2-$CF_3$) |
| B-0562 | thiazol-4-yl(5-$CF_3$) |
| B-0563 | thiazol-4-yl(2-$CO_2$Et) |
| B-0564 | thiazol-4-yl(5-$CO_2$Et) |
| B-0565 | thiazol-5-yl |
| B-0566 | $CH_2$thiazol-5-yl |
| B-0567 | thiazol-5-yl(2-F) |
| B-0568 | thiazol-5-yl(4-F) |
| B-0569 | thiazol-5-yl(2-Cl) |
| B-0570 | thiazol-5-yl(4-Cl) |
| B-0571 | thiazol-5-yl(2-Me) |
| B-0572 | thiazol-5-yl(4-Me) |
| B-0573 | thiazol-5-yl(2-OMe) |
| B-0574 | thiazol-5-yl(4-OMe) |
| B-0575 | thiazol-5-yl(2-CN) |
| B-0576 | thiazol-5-yl(4-CN) |
| B-0577 | thiazol-5-yl(2-$CF_3$) |
| B-0578 | thiazol-5-yl(4-$CF_3$) |
| B-0579 | thiazol-5-yl(2-$CO_2$Et) |

(continued)

| Compound Number | Q |
|---|---|
| B-0580 | thiazol-5-yl(4-$CO_2Et$) |
| B-0581 | isothiazol-3-yl |
| B-0582 | $CH_2$isothiazol-3-yl |

[Table 38]

| Compound Number | Q |
|---|---|
| B-0583 | isothiazol-3-yl(4-F) |
| B-0584 | isothiazol-3-yl(5-F) |
| B-0585 | isothiazol-3-yl(4-Cl) |
| B-0586 | isothiazol-3-yl(5-Cl) |
| B-0587 | isothiazol-3-yl(4-Me) |
| B-0588 | isothiazol-3-yl(5-Me) |
| B-0589 | isothiazol-3-yl(4-OMe) |
| B-0590 | isothiazol-3-yl(5-OMe) |
| B-0591 | isothiazol-3-yl(4-CN) |
| B-0592 | isothiazol-3-yl(5-CN) |
| B-0593 | isothiazol-3-yl(4-$CF_3$) |
| B-0594 | isothiazol-3-yl(5-$CF_3$) |
| B-0595 | isothiazol-3-yl(4-$CO_2Et$) |
| B-0596 | isothiazol-3-yl(5-$CO_2Et$) |
| B-0597 | isothiazol-4-yl |
| B-0598 | $CH_2$isothiazol-4-yl |
| B-0599 | isothiazol-4-yl(3-F) |
| B-0600 | isothiazol-4-yl(5-F) |
| B-0601 | isothiazol-4-yl(3-Cl) |
| B-0602 | isothiazol-4-yl(5-Cl) |
| B-0603 | isothiazol-4-yl(3-Me) |
| B-0604 | isothiazol-4-yl(5-Me) |
| B-0605 | isothiazol-4-yl(3-OMe) |
| B-0606 | isothiazol-4-yl(5-OMe) |
| B-0607 | isothiazol-4-yl(3-CN) |
| B-0608 | isothiazol-4-yl(5-CN) |
| B-0609 | isothiazol-4-yl(3-$CF_3$) |
| B-0610 | isothiazol-4-yl(5-$CF_3$) |
| B-0611 | isothiazol-4-yl(3-$CO_2Et$) |
| B-0612 | isothiazol-4-yl(5-$CO_2Et$) |

(continued)

| Compound Number | Q |
|---|---|
| B-0613 | isothiazol-5-yl |
| B-0614 | $CH_2$isothiazol-5-yl |
| B-0615 | isothiazol-5-yl(3-F) |
| B-0616 | isothiazol-5-yl(4-F) |
| B-0617 | isothiazol-5-yl(3-Cl) |
| B-0618 | isothiazol-5-yl(4-Cl) |
| B-0619 | isothiazol-5-yl(3,4-$Cl_2$) |
| B-0620 | isothiazol-5-yl(3-Me) |
| B-0621 | isothiazol-5-yl(4-Me) |
| B-0622 | isothiazol-5-yl(3-OMe) |
| B-0623 | isothiazol-5-yl(4-OMe) |
| B-0624 | isothiazol-5-yl(3-CN) |

[Table 39]

| Compound Number | Q |
|---|---|
| B-0625 | isothiazol-5-yl(4-CN) |
| B-0626 | isothiazol-5-yl(3-$CF_3$) |
| B-0627 | isothiazol-5-yl(4-$CF_3$) |
| B-0628 | isothiazol-5-yl(3-$CO_2$Et) |
| B-0629 | isothiazol-5-yl(4-$CO_2$Et) |
| B-0630 | oxazol-2-yl |
| B-0631 | $CH_2$oxazol-2-yl |
| B-0632 | oxazol-2-yl(4-F) |
| B-0633 | oxazol-2-yl(5-F) |
| B-0634 | oxazol-2-yl(4-Cl) |
| B-0635 | oxazol-2-yl(5-Cl) |
| B-0636 | oxazol-2-yl(4-Me) |
| B-0637 | oxazol-2-yl(5-Me) |
| B-0638 | oxazol-2-yl(4-OMe) |
| B-0639 | oxazol-2-yl(5-OMe) |
| B-0640 | oxazol-2-yl(4-CN) |
| B-0641 | oxazol-2-yl(5-CN) |
| B-0642 | oxazol-2-yl(4-$CF_3$) |
| B-0643 | oxazol-2-yl(5-$CF_3$) |
| B-0644 | oxazol-2-yl(4-$CO_2$Et) |
| B-0645 | oxazol-2-yl(5-$CO_2$Et) |

(continued)

| Compound Number | Q |
|---|---|
| B-0646 | oxazol-4-yl |
| B-0647 | $CH_2$oxazol-4-yl |
| B-0648 | oxazol-4-yl(2-F) |
| B-0649 | oxazol-4-yl(5-F) |
| B-0650 | oxazol-4-yl(2-Cl) |
| B-0651 | oxazol-4-yl(5-Cl) |
| B-0652 | oxazol-4-yl(2-Me) |
| B-0653 | oxazol-4-yl(5-Me) |
| B-0654 | oxazol-4-yl(2-OMe) |
| B-0655 | oxazol-4-yl(5-OMe) |
| B-0656 | oxazol-4-yl(2-CN) |
| B-0657 | oxazol-4-yl(5-CN) |
| B-0658 | oxazol-4-yl(2-$CF_3$) |
| B-0659 | oxazol-4-yl(5-$CF_3$) |
| B-0660 | oxazol-4-yl(2-$CO_2$Et) |
| B-0661 | oxazol-4-yl(5-$CO_2$Et) |
| B-0662 | oxazol-5-yl |
| B-0663 | $CH_2$oxazol-5-yl |
| B-0664 | oxazol-5-yl(2-F) |
| B-0665 | oxazol-5-yl(4-F) |
| B-0666 | oxazol-5-yl(2-Cl) |

[Table 40]

| Compound Number | Q |
|---|---|
| B-0667 | oxazol-5-yl(4-Cl) |
| B-0668 | oxazol-5-yl(2-Me) |
| B-0669 | oxazol-5-yl(4-Me) |
| B-0670 | oxazol-5-yl(2-OMe) |
| B-0671 | oxazol-5-yl(4-OMe) |
| B-0672 | oxazol-5-yl(2-CN) |
| B-0673 | oxazol-5-yl(4-CN) |
| B-0674 | oxazol-5-yl(2-$CF_3$) |
| B-0675 | oxazol-5-yl(4-$CF_3$) |
| B-0676 | oxazol-5-yl(2-$CO_2$Et) |
| B-0677 | oxazol-5-yl(4-$CO_2$Et) |
| B-0678 | isoxazol-3-yl |

(continued)

| Compound Number | Q |
|---|---|
| B-0679 | $CH_2$isoxazol-3-yl |
| B-0680 | isoxazol-3-yl(4-F) |
| B-0681 | isoxazol-3-yl(5-F) |
| B-0682 | isoxazol-3-yl(4-Cl) |
| B-0683 | isoxazol-3-yl(5-Cl) |
| B-0684 | isoxazol-3-yl(4-Me) |
| B-0685 | isoxazol-3-yl(5-Me) |
| B-0686 | isoxazol-3-yl(4-OMe) |
| B-0687 | isoxazol-3-yl(5-OMe) |
| B-0688 | isoxazol-3-yl(4-CN) |
| B-0689 | isoxazol-3-yl(5-CN) |
| B-0690 | isoxazol-3-yl(4-$CF_3$) |
| B-0691 | isoxazol-3-yl(5-$CF_3$) |
| B-0692 | isoxazol-3-yl(4-$CO_2$Et) |
| B-0693 | isoxazol-3-yl(5-$CO_2$Et) |
| B-0694 | isoxazol-4-yl |
| B-0695 | $CH_2$isoxazol-4-yl |
| B-0696 | isoxazol-4-yl(3-F) |
| B-0697 | isoxazol-4-yl(5-F) |
| B-0698 | isoxazol-4-yl(3-Cl) |
| B-0699 | isoxazol-4-yl(5-Cl) |
| B-0700 | isoxazol-4-yl(3-Me) |
| B-0701 | isoxazol-4-yl(5-Me) |
| B-0702 | isoxazol-4-yl(3-OMe) |
| B-0703 | isoxazol-4-yl(5-OMe) |
| B-0704 | isoxazol-4-yl(3-CN) |
| B-0705 | isoxazol-4-yl(5-CN) |
| B-0706 | isoxazol-4-yl(3-$CF_3$) |
| B-0707 | isoxazol-4-yl(5-$CF_3$) |
| B-0708 | isoxazol-4-yl(3-$CO_2$Et) |

[Table 41]

| Compound Number | Q |
|---|---|
| B-0709 | isoxazol-4-yl(5-$CO_2$Et) |
| B-0710 | isoxazol-5-yl |
| B-0711 | $CH_2$isoxazol-5-yl |

(continued)

| Compound Number | Q |
|---|---|
| B-0712 | isoxazol-5-yl(3-F) |
| B-0713 | isoxazol-5-yl(4-F) |
| B-0714 | isoxazol-5-yl(3-Cl) |
| B-0715 | isoxazol-5-yl(4-Cl) |
| B-0716 | isoxazol-5-yl(3-Me) |
| B-0717 | isoxazol-5-yl(4-Me) |
| B-0718 | isoxazol-5-yl(3-OMe) |
| B-0719 | isoxazol-5-yl(4-OMe) |
| B-0720 | isoxazol-5-yl(3-CN) |
| B-0721 | isoxazol-5-yl(4-CN) |
| B-0722 | isoxazol-5-yl(3-$CF_3$) |
| B-0723 | isoxazol-5-yl(4-$CF_3$) |
| B-0724 | isoxazol-5-yl(3-$CO_2$Et) |
| B-0725 | isoxazol-5-yl(4-$CO_2$Et) |
| B-0726 | 1,2,3-oxadiazol-4-yl |
| B-0727 | $CH_2$1,2,3-oxadiazol-4-yl |
| B-0728 | 1,2,3-oxadiazol-4-yl(5-F) |
| B-0729 | 1,2,3-oxadiazol-4-yl(5-Cl) |
| B-0730 | 1,2,3-oxadiazol-4-yl(5-Me) |
| B-0731 | 1,2,3-oxadiazol-4-yl(5-OMe) |
| B-0732 | 1,2,3-oxadiazol-4-yl(5-CN) |
| B-0733 | 1,2,3-oxadiazol-4-yl(5-$CF_3$) |
| B-0734 | 1,2,3-oxadiazol-4-yl(5-$CO_2$Et) |
| B-0735 | 1,2,3-oxadiazol-5-yl |
| B-0736 | $CH_2$1,2,3-oxadiazol-5-yl |
| B-0737 | 1,2,3-oxadiazol-5-yl(4-F) |
| B-0738 | 1,2,3-oxadiazol-5-yl(4-Cl) |
| B-0739 | 1,2,3-oxadiazol-5-yl(4-Me) |
| B-0740 | 1,2,3-oxadiazol-5-yl(4-OMe) |
| B-0741 | 1,2,3-oxadiazol-5-yl(4-CN) |
| B-0742 | 1,2,3-oxadiazol-5-yl(4-$CF_3$) |
| B-0743 | 1,2,3-oxadiazol-5-yl(4-$CO_2$Et) |
| B-0744 | 1,2,4-oxadiazol-3-yl |
| B-0745 | $CH_2$1,2,4-oxadiazol-3-yl |
| B-0746 | 1,2,4-oxadiazol-3-yl(5-F) |
| B-0747 | 1,2,4-oxadiazol-3-yl(5-Cl) |

(continued)

| Compound Number | Q |
|---|---|
| B-0748 | 1,2,4-oxadiazol-3-yl(5-Me) |
| B-0749 | 1,2,4-oxad iazol-3-yl(5-OMe) |
| B-0750 | 1,2,4-oxadiazol-3-yl(5-CN) |

[Table 42]

| Compound Number | Q |
|---|---|
| B-0751 | 1,2,4-oxadiazol-3-yl(5-$CF_3$) |
| B-0752 | 1,2,4-oxadiazol-3-yl(5-$CO_2Et$) |
| B-0753 | 1,2,4-oxadiazol-5-yl |
| B-0754 | $CH_2$1,2,4-oxadiazol-5-yl |
| B-0755 | 1,2,4-oxadiazol-5-yl(3-F) |
| B-0756 | 1,2,4-oxadiazol-5-yl(3-Cl) |
| B-0757 | 1,2,4-oxadiazol-5-yl(3-Me) |
| B-0758 | 1,2,4-oxadiazol-5-yl(3-OMe) |
| B-0759 | 1,2,4-oxadiazol-5-yl(3-CN) |
| B-0760 | 1,2,4-oxadiazol-5-yl(3-$CF_3$) |
| B-0761 | 1,2,4-oxadiazol-5-yl(3-$CO_2Et$) |
| B-0762 | 1,3,4-oxadiazol-2-yl |
| B-0763 | $CH_2$1,3,4-oxadiazol-2-yl |
| B-0764 | 1,2,5-oxadiazol-3-yl |
| B-0765 | $CH_2$1,2,5-oxadiazol-3-yl |
| B-0766 | 1,2,3-thiadiazol-4-yl |
| B-0767 | $CH_2$1,2,3-thiadiazol-4-yl |
| B-0768 | 1,2,3-thiadiazol-5-yl |
| B-0769 | $CH_2$1,2,3-thiadiazol-5-yl |
| B-0770 | 1,2,4-thiadiazol-3-yl |
| B-0771 | $CH_2$1,2,4-thiadiazol-3-yl |
| B-0772 | 1,2,4-thiadiazol-5-yl |
| B-0773 | $CH_2$1,2,4-thiadiazol-5-yl |
| B-0774 | 1,3,4-thiadiazol-2-yl |
| B-0775 | $CH_2$1,3,4-thiadiazol-2-yl |
| B-0776 | 1,2,5-thiadiazol-3-yl |
| B-0777 | $CH_2$1,2,5-thiadiazol-3-yl |
| B-0778 | morpholin-4-yl |
| B-0779 | $CH_2$morpholin-4-yl |
| B-0780 | piperidin-4-yl |

(continued)

| Compound Number | Q |
|---|---|
| B-0781 | $CH_2$piperidin-4-yl |
| B-0782 | piperidin-4-yl(1-Me) |
| B-0783 | $CH_2$piperidin-4-yl(1-Me) |
| B-0784 | piperidin-1-yl |
| B-0785 | $CH_2$piperidin-1-yl |
| B-0786 | piperazin-1-yl |
| B-0787 | $CH_2$piperazin-1-yl |
| B-0788 | piperazin-1-yl(4-Me) |
| B-0789 | $CH_2$piperazin-1yl(4-Me) |
| B-0790 | tetrahydro-2H-pyran-4-yl |
| B-0791 | $CH_2$tetrahydro-2H-pyran-4-yl |
| B-0792 | 1,3-dioxolan-2-yl |

[Table 43]

| Compound Number | Q |
|---|---|
| B-0793 | $CH_2$1,3-dioxolan-2-yl |
| B-0794 | 1,3-dioxolan-2-yl(2-$CF_3$) |
| B-0795 | $CH_2$1,3-dioxolan-2-yl(2-$CF_3$) |
| B-0796 | 1,3-dioxan-2-yl |
| B-0797 | $CH_2$1,3-dioxan-2-yl |
| B-0798 | 1,3-dioxan-2-yl(2-$CF_3$) |
| B-0799 | $CH_2$1,3-dioxan-2-yl(2-$CF_3$) |
| B-0800 | pyrrolidin-1-yl |
| B-0801 | $CH_2$pyrrolidin-1-yl |
| B-0802 | pyrrolidin-2-yl |
| B-0803 | $CH_2$pyrrolidin-2-yl |
| B-0804 | pyrrolidin-2-yl(1-Me) |
| B-0805 | $CH_2$pyrrolidin-2-yl(1-Me) |
| B-0806 | pyrrolidin-3-yl |
| B-0807 | $CH_2$pyrrolidin-3-yl |
| B-0808 | pyrrolidin-3-yl(1-Me) |
| B-0809 | $CH_2$pyrrolidin-3-yl(1-Me) |
| B-0810 | tetrahydrofuran-2-yl |
| B-0811 | $CH_2$tetrahydrofuran-2-yl |
| B-0812 | tetrahydrofuran-3-yl |

(continued)

| Compound Number | Q |
|---|---|
| B-0813 | $CH_2$tetrahydrofuran-3-yl |
| B-0814 | 4,5-dihydroisoxazol-3-yl |
| B-0815 | $CH_2$4,5-dihydroisoxazol-3-yl |
| B-0816 | 4,5-dihydroisoxazol-4-yl |
| B-0817 | $CH_2$4,5-dihydroisoxazol-4-yl |
| B-0818 | 4,5-dihydroisoxazol-5-yl |
| B-0819 | $CH_2$4,5-dihydroisoxazol-5-yl |
| B-0820 | oxetan-2-yl |
| B-0821 | $CH_2$oxetan-2-yl |
| B-0822 | oxetan-3-yl |
| B-0823 | oxetan-3-yl(3-Me) |
| B-0824 | $CH_2$oxetan-3-yl |
| B-0825 | azetidin-1-yl |
| B-0826 | $CH_2$azetidin-1-yl |
| B-0827 | azetidin-2-yl |
| B-0828 | $CH_2$azetidin-2-yl |
| B-0829 | azetidin-2-yl(1-Me) |
| B-0830 | $CH_2$azetidin-2-yl(1-Me) |
| B-0831 | azetidin-3-yl |
| B-0832 | $CH_2$azetidin-3-yl |
| B-0833 | azetidin-3-yl(1-Me) |
| B-0834 | $CH_2$azetidin-3-yl(1-Me) |

[Table 44]

| Compound Number | Q |
|---|---|
| B-0835 | oxiran-2-yl |
| B-0836 | $CH_2$oxiran-2-yl |
| B-0837 | 1,3,2-dioxaborolan-2-yl(4,4,5,5-$Me_4$) |
| B-0838 | tetrahydro-2H-pyran-2-yl |
| B-0839 | $CH_2$tetrahydro-2H-pyran-2-yl |
| B-0840 | tetra hydro-2H-pyran-3-yl |
| B-0841 | $CH_2$tetrahydro-2H-pyran-3-yl |
| B-0842 | piperidin-2-yl |
| B-0843 | $CH_2$piperidin-2-yl |
| B-0844 | piperidin-2-yl(1-Me) |
| B-0845 | $CH_2$piperidin-2-yl(1-Me) |

(continued)

| Compound Number | Q |
|---|---|
| B-0846 | piperidin-3-yl |
| B-0847 | $CH_2$piperidin-3-yl |
| B-0848 | piperidin-3-yl(1-Me) |
| B-0849 | $CH_2$piperidin-3-yl(1-Me) |
| B-0850 | pyridin-2-yl(6-$CH_2OCH_3$) |
| B-0851 | 1H-pyrazol-5-yl(1-CHO) |
| B-0852 | (pyridin-1-oxide)-2-yl |
| B-0853 | (pyridin-1-oxide)-3-yl |
| B-0854 | (pyridin-1-oxide)-4-yl |

[Table 45]

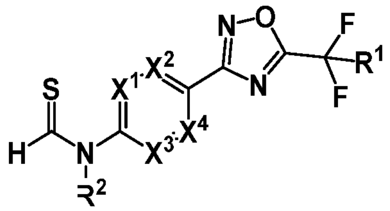

| Compound Number | $R^1$ | $X^1$ | $X^2$ | $X^3$ | $X^4$ | $R^2$ |
|---|---|---|---|---|---|---|
| C-0001 | F | CH | CH | CH | CH | H |
| C-0002 | F | CH | CH | CH | CH | Me |
| C-0003 | F | CH | CH | CH | CH | Et |
| C-0004 | F | CH | CH | CH | CH | n-Pr |
| C-0005 | F | CH | CH | CH | CH | i-Pr |
| C-0006 | F | CH | CH | CH | CH | n-Bu |
| C-0007 | F | CH | CH | CH | CH | i-Bu |
| C-0008 | F | CH | CH | CH | CH | s-Bu |
| C-0009 | F | CH | CH | CH | CH | t-Bu |
| C-0010 | F | CH | CH | CH | CH | $CH_2$t-Bu |
| C-0011 | F | CH | CH | CH | CH | n-Pen |
| C-0012 | F | CH | CH | CH | CH | n-Hex |
| C-0013 | F | CH | CH | CH | CH | $CH=CH_2$ |
| C-0014 | F | CH | CH | CH | CH | $CH_2CH=CH_2$ |
| C-0015 | F | CH | CH | CH | CH | $CH_2CH=CHCH_2Cl$ |
| C-0016 | F | CH | CH | CH | CH | $CH_2CH=CHCH_2NMe_2$ |
| C-0017 | F | CH | CH | CH | CH | C≡CH |
| C-0018 | F | CH | CH | CH | CH | $CH_2C$-CH |
| C-0019 | F | CH | CH | CH | CH | c-Pr |

(continued)

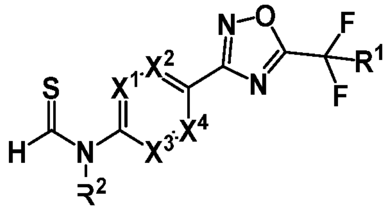

| Compound Number | $R^1$ | $X^1$ | $X^2$ | $X^3$ | $X^4$ | $R^2$ |
|---|---|---|---|---|---|---|
| C-0020 | F | CH | CH | CH | CH | c-Bu |
| C-0021 | F | CH | CH | CH | CH | c-Pen |
| C-0022 | F | CH | CH | CH | CH | c-Hex |
| C-0023 | F | CH | CH | CH | CH | $CH_2$c-Pr |
| C-0024 | F | CH | CH | CH | CH | $CH_2$c-Bu |
| C-0025 | F | CH | CH | CH | CH | $CH_2$c-Pen |
| C-0026 | F | CH | CH | CH | CH | $CH_2$c-Hex |
| C-0027 | F | CH | CH | CH | CH | $CH_2$c-Pr(2,2-$Cl_2$) |
| C-0028 | F | CH | CH | CH | CH | $CF_3$ |
| C-0029 | F | CH | CH | CH | CH | $CH_2CF_3$ |
| C-0030 | F | CH | CH | CH | CH | $CH_2CH_2CF_3$ |
| C-0031 | F | CH | CH | CH | CH | $CHF_2$ |
| C-0032 | F | CH | CH | CH | CH | $CH_2CHF_2$ |
| C-0033 | F | CH | CH | CH | CH | $CH_2CH_2CHF_2$ |
| C-0034 | F | CH | CH | CH | CH | $CH_2COMe$ |
| C-0035 | F | CH | CH | CH | CH | $CH_2CO_2Me$ |
| C-0036 | F | CH | CH | CH | CH | $CH(CH_3)CO_2Me$ |

[Table 46]

| Compound Number | $R^1$ | $X^1$ | $X^2$ | $X^3$ | $X^4$ | $R^2$ |
|---|---|---|---|---|---|---|
| C-0037 | F | CH | CH | CH | CH | $C(CH_3)(CH_3)CO_2Me$ |
| C-0038 | F | CH | CH | CH | CH | $CH_2CO_2Et$ |
| C-0039 | F | CH | CH | CH | CH | $CH(CH_3)CO_2Et$ |
| C-0040 | F | CH | CH | CH | CH | $C(CH_3)(CH_3)CO_2Et$ |
| C-0041 | F | CH | CH | CH | CH | $CH_2CONH_2$ |
| C-0042 | F | CH | CH | CH | CH | $CH_2CONHMe$ |
| C-0043 | F | CH | CH | CH | CH | $CH(CH_3)CONHMe$ |
| C-0044 | F | CH | CH | CH | CH | $C(CH_3)(CH_3)CONHMe$ |
| C-0045 | F | CH | CH | CH | CH | $CH_2CONHEt$ |

(continued)

| Compound Number | $R^1$ | $X^1$ | $X^2$ | $X^3$ | $X^4$ | $R^2$ |
|---|---|---|---|---|---|---|
| C-0046 | F | CH | CH | CH | CH | $CH(CH_3)CONHEt$ |
| C-0047 | F | CH | CH | CH | CH | $C(CH_3)(CH_3)CONHEt$ |
| C-0048 | F | CH | CH | CH | CH | $CH_2NHCHO$ |
| C-0049 | F | CH | CH | CH | CH | $CH(CH_3)NHCHO$ |
| C-0050 | F | CH | CH | CH | CH | $C(CH_3)(CH_3)NHCHO$ |
| C-0051 | F | CH | CH | CH | CH | $CH_2NHCOMe$ |
| C-0052 | F | CH | CH | CH | CH | $CH(CH_3)NHCOMe$ |
| C-0053 | F | CH | CH | CH | CH | $C(CH_3)(CH_3)NHCOMe$ |
| C-0054 | F | CH | CH | CH | CH | $CH_2NHCOEt$ |
| C-0055 | F | CH | CH | CH | CH | $CH(CH_3)NHCOEt$ |
| C-0056 | F | CH | CH | CH | CH | $C(CH_3)(CH_3)NHCOEt$ |
| C-0057 | F | CH | CH | CH | CH | $CH_2CN$ |
| C-0058 | F | CH | CH | CH | CH | $CH_2CH_2CN$ |
| C-0059 | F | CH | CH | CH | CH | $CH_2$c-Pr(1-CN) |
| C-0060 | F | CH | CH | CH | CH | $CH_2SCN$ |
| C-0061 | F | CH | CH | CH | CH | $CH_2CH_2SCN$ |
| C-0062 | F | CH | CH | CH | CH | $CH_2CH_2CH_2CH_2CH_2SCN$ |
| C-0063 | F | CH | CH | CH | CH | $CH_2CH_2CH_2CH_2CH_2CH_2SCN$ |
| C-0064 | F | CH | CH | CH | CH | $CH_2OCH_3$ |
| C-0065 | F | CH | CH | CH | CH | $CH_2OCH_2CH_3$ |
| C-0066 | F | CH | CH | CH | CH | $CH_2CH_2OCH_3$ |
| C-0067 | F | CH | CH | CH | CH | $CH_2CH_2OCH_2CH_3$ |
| C-0068 | F | CH | CH | CH | CH | $CH_2OCF_3$ |
| C-0069 | F | CH | CH | CH | CH | $CH_2OCH_2CF_3$ |
| C-0070 | F | CH | CH | CH | CH | $CH_2CH_2OCF_3$ |
| C-0071 | F | CH | CH | CH | CH | $CH_2CH_2OCH_2CF_3$ |
| C-0072 | F | CH | CH | CH | CH | $CH_2SCH_3$ |
| C-0073 | F | CH | CH | CH | CH | $CH_2SCH_2CH_3$ |
| C-0074 | F | CH | CH | CH | CH | $CH_2CH_2SCH_3$ |
| C-0075 | F | CH | CH | CH | CH | $CH_2CH_2SCH_2CH_3$ |
| C-0076 | F | CH | CH | CH | CH | $CH_2SCF_3$ |
| C-0077 | F | CH | CH | CH | CH | $CH_2SCH_2CF_3$ |
| C-0078 | F | CH | CH | CH | CH | $CH_2CH_2SCF_3$ |

[Table 47]

| Compound Number | $R^1$ | $X^1$ | $X^2$ | $X^3$ | $X^4$ | $R^2$ |
|---|---|---|---|---|---|---|
| C-0079 | F | CH | CH | CH | CH | $CH_2CH_2SCH_2CF_3$ |
| C-0080 | F | CH | CH | CH | CH | $CH_2CH_2CH_2CH_2CH_2SCF_3$ |
| C-0081 | F | CH | CH | CH | CH | $CH_2CH_2CH_2CH_2CH_2CH_2SCF_3$ |
| C-0082 | F | CH | CH | CH | CH | Ph |
| C-0083 | F | CH | CH | CH | CH | pyridin-2-yl |
| C-0084 | F | CH | CH | CH | CH | pyridin-3-yl |
| C-0085 | F | CH | CH | CH | CH | pyridin-4-yl |
| C-0086 | F | CH | CH | CH | CH | pyrimidin-2-yl |
| C-0087 | F | CH | CH | CH | CH | pyrimidin-4-yl |
| C-0088 | F | CH | CH | CH | CH | pyrimidin-5-yl |
| C-0089 | F | CH | CH | CH | CH | pyridazin-3-yl |
| C-0090 | F | CH | CH | CH | CH | pyridazin-4-yl |
| C-0091 | F | CH | CH | CH | CH | pyrazin-2-yl |
| C-0092 | F | CH | CH | CH | CH | azetidin-3-yl |
| C-0093 | F | CH | CH | CH | CH | azetidi n-3-yl(1-Me) |
| C-0094 | F | CH | CH | CH | CH | pyrrolidin-3-yl |
| C-0095 | F | CH | CH | CH | CH | pyrrolidin-3-yl(1-Me) |
| C-0096 | F | CH | CH | CH | CH | $CH_2Ph$ |
| C-0097 | F | CH | CH | CH | CH | $CH_2CH_2Ph$ |
| C-0098 | F | CH | CH | CH | CH | $CH_2CH_2CH_2Ph$ |
| C-0099 | F | CH | CH | CH | CH | $CH(CH_3)Ph$ |
| C-0100 | F | CH | CH | CH | CH | $C(CH_3)(CH_3)Ph$ |
| C-0101 | F | CH | CH | CH | CH | 1-Ph-c-Pr |
| C-0102 | F | CH | CH | CH | CH | 1-Ph-c-Bu |
| C-0103 | F | CH | CH | CH | CH | $CH_2COPh$ |
| C-0104 | F | CH | CH | CH | CH | $CH_2CO_2Ph$ |
| C-0105 | F | CH | CH | CH | CH | $CH_2CONHPh$ |
| C-0106 | F | CH | CH | CH | CH | $CH_2NHCOPh$ |
| C-0107 | F | CH | CH | CH | CH | $CH(CH_3)$pyridin-2-yl |
| C-0108 | F | CH | CH | CH | CH | $C(CH_3)(CH_3)$pyridin-2-yl |
| C-0109 | F | CH | CH | CH | CH | 1-(pyridin-2-yl)-c-Pr |
| C-0110 | F | CH | CH | CH | CH | 1-(pyridin-2-yl)-c-Bu |
| C-0111 | F | CH | CH | CH | CH | $CH(CH_3)$pyridin-3-yl |
| C-0112 | F | CH | CH | CH | CH | $C(CH_3)(CH_3)$pyridin-3-yl |
| C-0113 | F | CH | CH | CH | CH | 1-(pyridin-3-yl)-c-Pr |
| C-0114 | F | CH | CH | CH | CH | 1-(pyridin-3-yl)-c-Bu |

(continued)

| Compound Number | $R^1$ | $X^1$ | $X^2$ | $X^3$ | $X^4$ | $R^2$ |
|---|---|---|---|---|---|---|
| C-0115 | F | CH | CH | CH | CH | $CH(CH_3)$pyridin-4-yl |
| C-0116 | F | CH | CH | CH | CH | $C(CH_3)(CH_3)$pyridin-4-yl |
| C-0117 | F | CH | CH | CH | CH | 1-(pyridin-4-yl)-c-Pr |
| C-0118 | F | CH | CH | CH | CH | 1-(pyridin-4-yl)-c-Bu |
| C-0119 | F | CH | CH | CH | CH | $CH(CH_3)$pyrimidin-2-yl |
| C-0120 | F | CH | CH | CH | CH | $C(CH_3)(CH_3)$pyrimidin-2-yl |

[Table 48]

| Compound Number | $R^1$ | $X^1$ | $X^2$ | $X^3$ | $X^4$ | $R^2$ |
|---|---|---|---|---|---|---|
| C-0121 | F | CH | CH | CH | CH | 1-(pyrimidin-2-yl)-c-Pr |
| C-0122 | F | CH | CH | CH | CH | 1-(pyri m i di n-2-yl)-c-B u |
| C-0123 | F | CH | CH | CH | CH | $CH(CH_3)$pyrimidin-4-yl |
| C-0124 | F | CH | CH | CH | CH | $C(CH_3)(CH_3)$pyrimidin-4-yl |
| C-0125 | F | CH | CH | CH | CH | 1-(pyrimidin-4-yl)-c-Pr |
| C-0126 | F | CH | CH | CH | CH | 1-(pyrimidin-4-yl)-c-Bu |
| C-0127 | F | CH | CH | CH | CH | $CH(CH_3)$pyrimidin-5-yl |
| C-0128 | F | CH | CH | CH | CH | $C(CH_3)(CH_3)$pyrimidin-5-yl |
| C-0129 | F | CH | CH | CH | CH | 1-(pyrimidin-5-yl)-c-Pr |
| C-0130 | F | CH | CH | CH | CH | 1-(pyrimidin-5-yl)-c-Bu |
| C-0131 | F | CH | CH | CH | CH | $CH(CH_3)$pyridazin-3-yl |
| C-0132 | F | CH | CH | CH | CH | $C(CH_3)(CH_3)$pyridazin-3-yl |
| C-0133 | F | CH | CH | CH | CH | 1-(pyridazin-3-yl)-c-Pr |
| C-0134 | F | CH | CH | CH | CH | 1-(pyri d azi n-3-yl)-c-B u |
| C-0135 | F | CH | CH | CH | CH | $CH(CH_3)$pyridazin-4-yl |
| C-0136 | F | CH | CH | CH | CH | $C(CH_3)(CH_3)$pyridazin-4-yl |
| C-0137 | F | CH | CH | CH | CH | 1-(pyridazin-4-yl)-c-Pr |
| C-0138 | F | CH | CH | CH | CH | 1-(pyri d azi n-4-yl)-c-B u |
| C-0139 | F | CH | CH | CH | CH | $CH(CH_3)$pyrazin-2-yl |
| C-0140 | F | CH | CH | CH | CH | $C(CH_3)(CH_3)$pyrazin-2-yl |
| C-0141 | F | CH | CH | CH | CH | 1-(pyrazin-2-yl)-c-Pr |
| C-0142 | F | CH | CH | CH | CH | 1-(pyrazi n-2-yl)-c-B u |
| C-0143 | F | CH | CH | CH | CH | $CH_2OPh$ |
| C-0144 | F | CH | CH | CH | CH | $CH_2CH_2OPh$ |
| C-0145 | F | CH | CH | CH | CH | CH(OH)Ph |
| C-0146 | F | CH | CH | CH | CH | $CH(CH_2OH)$Ph |

(continued)

| Compound Number | $R^1$ | $X^1$ | $X^2$ | $X^3$ | $X^4$ | $R^2$ |
|---|---|---|---|---|---|---|
| C-0147 | F | CH | CH | CH | CH | $CH_2CH(OH)Ph$ |
| C-0148 | F | CH | CH | CH | CH | $CH_2SPh$ |
| C-0149 | F | CH | CH | CH | CH | $CH_2SOPh$ |
| C-0150 | F | CH | CH | CH | CH | $CH_2SO_2Ph$ |
| C-0151 | F | CH | CH | CH | CH | $CH_2CH_2SPh$ |
| C-0152 | F | CH | CH | CH | CH | $CH_2CH_2SOPh$ |
| C-0153 | F | CH | CH | CH | CH | $CH_2CH_2SO_2Ph$ |
| C-0154 | F | CH | CH | CH | CH | $CH_2NHPh$ |
| C-0155 | F | CH | CH | CH | CH | $CH_2CH_2NHPh$ |
| C-0156 | F | CH | CH | CH | CH | $CH_2Ph(2-F)$ |
| C-0157 | F | CH | CH | CH | CH | $CH_2Ph(3-F)$ |
| C-0158 | F | CH | CH | CH | CH | $CH_2Ph(4-F)$ |
| C-0159 | F | CH | CH | CH | CH | $CH_2Ph(2-Cl)$ |
| C-0160 | F | CH | CH | CH | CH | $CH_2Ph(3-Cl)$ |
| C-0161 | F | CH | CH | CH | CH | $CH_2Ph(4-Cl)$ |
| C-0162 | F | CH | CH | CH | CH | $CH_2Ph(2-Br)$ |

[Table 49]

| Compound Number | $R^1$ | $X^1$ | $X^2$ | $X^3$ | $X^4$ | $R^2$ |
|---|---|---|---|---|---|---|
| C-0163 | F | CH | CH | CH | CH | $CH_2Ph(3-Br)$ |
| C-0164 | F | CH | CH | CH | CH | $CH_2Ph(4-Br)$ |
| C-0165 | F | CH | CH | CH | CH | $CH_2Ph(2-I)$ |
| C-0166 | F | CH | CH | CH | CH | $CH_2Ph(3-I)$ |
| C-0167 | F | CH | CH | CH | CH | $CH_2Ph(4-I)$ |
| C-0168 | F | CH | CH | CH | CH | $CH_2Ph(2-Me)$ |
| C-0169 | F | CH | CH | CH | CH | $CH_2Ph(3-Me)$ |
| C-0170 | F | CH | CH | CH | CH | $CH_2Ph(4-Me)$ |
| C-0171 | F | CH | CH | CH | CH | $CH_2Ph(2-Et)$ |
| C-0172 | F | CH | CH | CH | CH | $CH_2Ph(3-Et)$ |
| C-0173 | F | CH | CH | CH | CH | $CH_2Ph(4-Et)$ |
| C-0174 | F | CH | CH | CH | CH | $CH_2Ph(2-n-Pr)$ |
| C-0175 | F | CH | CH | CH | CH | $CH_2Ph(3-n-Pr)$ |
| C-0176 | F | CH | CH | CH | CH | $CH_2Ph(4-n-Pr)$ |
| C-0177 | F | CH | CH | CH | CH | $CH_2Ph(2-i-Pr)$ |

(continued)

| Compound Number | $R^1$ | $X^1$ | $X^2$ | $X^3$ | $X^4$ | $R^2$ |
|---|---|---|---|---|---|---|
| C-0178 | F | CH | CH | CH | CH | $CH_2Ph(3\text{-}i\text{-}Pr)$ |
| C-0179 | F | CH | CH | CH | CH | $CH_2Ph(4\text{-}i\text{-}Pr)$ |
| C-0180 | F | CH | CH | CH | CH | $CH_2Ph(2\text{-}s\text{-}Bu)$ |
| C-0181 | F | CH | CH | CH | CH | $CH_2Ph(3\text{-}s\text{-}Bu)$ |
| C-0182 | F | CH | CH | CH | CH | $CH_2Ph(4\text{-}s\text{-}Bu)$ |
| C-0183 | F | CH | CH | CH | CH | $CH_2Ph(2\text{-}i\text{-}Bu)$ |
| C-0184 | F | CH | CH | CH | CH | $CH_2Ph(3\text{-}i\text{-}Bu)$ |
| C-0185 | F | CH | CH | CH | CH | $CH_2Ph(4\text{-}i\text{-}Bu)$ |
| C-0186 | F | CH | CH | CH | CH | $CH_2Ph(2\text{-}t\text{-}Bu)$ |
| C-0187 | F | CH | CH | CH | CH | $CH_2Ph(3\text{-}t\text{-}Bu)$ |
| C-0188 | F | CH | CH | CH | CH | $CH_2Ph(4\text{-}t\text{-}Bu)$ |
| C-0189 | F | CH | CH | CH | CH | $CH_2Ph(2\text{-}CH{=}CH_2)$ |
| C-0190 | F | CH | CH | CH | CH | $CH_2Ph(3\text{-}CH{=}CH_2)$ |
| C-0191 | F | CH | CH | CH | CH | $CH_2Ph(4\text{-}CH{=}CH_2)$ |
| C-0192 | F | CH | CH | CH | CH | $CH_2Ph(2\text{-}CH_2CH{=}CH_2)$ |
| C-0193 | F | CH | CH | CH | CH | $CH_2Ph(3\text{-}CH_2CH{=}CH_2)$ |
| C-0194 | F | CH | CH | CH | CH | $CH_2Ph(4\text{-}CH_2CH{=}CH_2)$ |
| C-0195 | F | CH | CH | CH | CH | $CH_2Ph(2\text{-}CH{=}CH)$ |
| C-0196 | F | CH | CH | CH | CH | $CH_2Ph(3\text{-}CH{\equiv}CH)$ |
| C-0197 | F | CH | CH | CH | CH | $CH_2Ph(4\text{-}CH{=}CH)$ |
| C-0198 | F | CH | CH | CH | CH | $CH_2Ph(2\text{-}c\text{-}Pr)$ |
| C-0199 | F | CH | CH | CH | CH | $CH_2Ph(3\text{-}c\text{-}Pr)$ |
| C-0200 | F | CH | CH | CH | CH | $CH_2Ph(4\text{-}c\text{-}Pr)$ |
| C-0201 | F | CH | CH | CH | CH | $CH_2Ph(2\text{-}c\text{-}Bu)$ |
| C-0202 | F | CH | CH | CH | CH | $CH_2Ph(3\text{-}c\text{-}Bu)$ |
| C-0203 | F | CH | CH | CH | CH | $CH_2Ph(4\text{-}c\text{-}Bu)$ |
| C-0204 | F | CH | CH | CH | CH | $CH_2Ph(2\text{-}OMe)$ |

[Table 50]

| Compound Number | $R^1$ | $X^1$ | $X^2$ | $X^3$ | $X^4$ | $R^2$ |
|---|---|---|---|---|---|---|
| C-0205 | F | CH | CH | CH | CH | $CH_2Ph(3\text{-}OMe)$ |
| C-0206 | F | CH | CH | CH | CH | $CH_2Ph(4\text{-}OMe)$ |
| C-0207 | F | CH | CH | CH | CH | $CH_2Ph(2\text{-}OEt)$ |
| C-0208 | F | CH | CH | CH | CH | $CH_2Ph(3\text{-}OEt)$ |

(continued)

| Compound Number | $R^1$ | $X^1$ | $X^2$ | $X^3$ | $X^4$ | $R^2$ |
|---|---|---|---|---|---|---|
| C-0209 | F | CH | CH | CH | CH | $CH_2Ph(4\text{-}OEt)$ |
| C-0210 | F | CH | CH | CH | CH | $CH_2Ph(2\text{-}On\text{-}Pr)$ |
| C-0211 | F | CH | CH | CH | CH | $CH_2Ph(3\text{-}On\text{-}Pr)$ |
| C-0212 | F | CH | CH | CH | CH | $CH_2Ph(4\text{-}On\text{-}Pr)$ |
| C-0213 | F | CH | CH | CH | CH | $CH_2Ph(2\text{-}Oi\text{-}Pr)$ |
| C-0214 | F | CH | CH | CH | CH | $CH_2Ph(3\text{-}Oi\text{-}Pr)$ |
| C-0215 | F | CH | CH | CH | CH | $CH_2Ph(4\text{-}Oi\text{-}Pr)$ |
| C-0216 | F | CH | CH | CH | CH | $CH_2Ph(2\text{-}OCF_3)$ |
| C-0217 | F | CH | CH | CH | CH | $CH_2Ph(3\text{-}OCF_3)$ |
| C-0218 | F | CH | CH | CH | CH | $CH_2Ph(4\text{-}OCF_3)$ |
| C-0219 | F | CH | CH | CH | CH | $CH_2Ph(2\text{-}OCHF_2)$ |
| C-0220 | F | CH | CH | CH | CH | $CH_2Ph(3\text{-}OCHF_2)$ |
| C-0221 | F | CH | CH | CH | CH | $CH_2Ph(4\text{-}OCHF_2)$ |
| C-0222 | F | CH | CH | CH | CH | $CH_2Ph(2\text{-}OCH_2CF_3)$ |
| C-0223 | F | CH | CH | CH | CH | $CH_2Ph(3\text{-}OCH_2CF_3)$ |
| C-0224 | F | CH | CH | CH | CH | $CH_2Ph(4\text{-}OCH_2CF_3)$ |
| C-0225 | F | CH | CH | CH | CH | $CH_2Ph(2\text{-}SMe)$ |
| C-0226 | F | CH | CH | CH | CH | $CH_2Ph(3\text{-}SMe)$ |
| C-0227 | F | CH | CH | CH | CH | $CH_2Ph(4\text{-}SMe)$ |
| C-0228 | F | CH | CH | CH | CH | $CH_2Ph(2\text{-}SEt)$ |
| C-0229 | F | CH | CH | CH | CH | $CH_2Ph(3\text{-}SEt)$ |
| C-0230 | F | CH | CH | CH | CH | $CH_2Ph(4\text{-}SEt)$ |
| C-0231 | F | CH | CH | CH | CH | $CH_2Ph(2\text{-}SCF_3)$ |
| C-0232 | F | CH | CH | CH | CH | $CH_2Ph(3\text{-}SCF_3)$ |
| C-0233 | F | CH | CH | CH | CH | $CH_2Ph(4\text{-}SCF_3)$ |
| C-0234 | F | CH | CH | CH | CH | $CH_2Ph(2\text{-}SOMe)$ |
| C-0235 | F | CH | CH | CH | CH | $CH_2Ph(3\text{-}SOMe)$ |
| C-0236 | F | CH | CH | CH | CH | $CH_2Ph(4\text{-}SOMe)$ |
| C-0237 | F | CH | CH | CH | CH | $CH_2Ph(2\text{-}SO_2Me)$ |
| C-0238 | F | CH | CH | CH | CH | $CH_2Ph(3\text{-}SO_2Me)$ |
| C-0239 | F | CH | CH | CH | CH | $CH_2Ph(4\text{-}SO_2Me)$ |
| C-0240 | F | CH | CH | CH | CH | $CH_2Ph(2\text{-}CN)$ |
| C-0241 | F | CH | CH | CH | CH | $CH_2Ph(3\text{-}CN)$ |
| C-0242 | F | CH | CH | CH | CH | $CH_2Ph(4\text{-}CN)$ |

(continued)

| Compound Number | $R^1$ | $X^1$ | $X^2$ | $X^3$ | $X^4$ | $R^2$ |
|---|---|---|---|---|---|---|
| C-0243 | F | CH | CH | CH | CH | $CH_2Ph(2-CH_2CN)$ |
| C-0244 | F | CH | CH | CH | CH | $CH_2Ph(3-CH_2CN)$ |
| C-0245 | F | CH | CH | CH | CH | $CH_2Ph(4-CH_2CN)$ |
| C-0246 | F | CH | CH | CH | CH | $CH_2Ph[2-(1-CN-c-Pr)]$ |

[Table 51]

| Compound Number | $R^1$ | $X^1$ | $X^2$ | $X^3$ | $X^4$ | $R^2$ |
|---|---|---|---|---|---|---|
| C-0247 | F | CH | CH | CH | CH | $CH_2Ph[3-(1-CN-c-Pr)]$ |
| C-0248 | F | CH | CH | CH | CH | $CH_2Ph[4-(1-CN-c-Pr)]$ |
| C-0249 | F | CH | CH | CH | CH | $CH_2Ph(2-CH_2OMe)$ |
| C-0250 | F | CH | CH | CH | CH | $CH_2Ph(3-CH_2OMe)$ |
| C-0251 | F | CH | CH | CH | CH | $CH_2Ph(4-CH_2OMe)$ |
| C-0252 | F | CH | CH | CH | CH | $CH_2Ph(2-CH_2OEt)$ |
| C-0253 | F | CH | CH | CH | CH | $CH_2Ph(3-CH_2OEt)$ |
| C-0254 | F | CH | CH | CH | CH | $CH_2Ph(4-CH_2OEt)$ |
| C-0255 | F | CH | CH | CH | CH | $CH_2Ph(2-CF_3)$ |
| C-0256 | F | CH | CH | CH | CH | $CH_2Ph(3-CF_3)$ |
| C-0257 | F | CH | CH | CH | CH | $CH_2Ph(4-CF_3)$ |
| C-0258 | F | CH | CH | CH | CH | $CH_2Ph(2-CHF_2)$ |
| C-0259 | F | CH | CH | CH | CH | $CH_2Ph(3-CHF_2)$ |
| C-0260 | F | CH | CH | CH | CH | $CH_2Ph(4-CHF_2)$ |
| C-0261 | F | CH | CH | CH | CH | $CH_2Ph(2-CH_2F)$ |
| C-0262 | F | CH | CH | CH | CH | $CH_2Ph(3-CH_2F)$ |
| C-0263 | F | CH | CH | CH | CH | $CH_2Ph(4-CH_2F)$ |
| C-0264 | F | CH | CH | CH | CH | $CH_2Ph(2-CF_2Cl)$ |
| C-0265 | F | CH | CH | CH | CH | $CH_2Ph(3-CF_2Cl)$ |
| C-0266 | F | CH | CH | CH | CH | $CH_2Ph(4-CF_2Cl)$ |
| C-0267 | F | CH | CH | CH | CH | $CH_2Ph[2-CF(CF_3)_2]$ |
| C-0268 | F | CH | CH | CH | CH | $CH_2Ph[3-CF(CF_3)_2]$ |
| C-0269 | F | CH | CH | CH | CH | $CH_2Ph[4-CF(CF_3)_2]$ |
| C-0270 | F | CH | CH | CH | CH | $CH_2Ph(2-COMe)$ |
| C-0271 | F | CH | CH | CH | CH | $CH_2Ph(3-COMe)$ |
| C-0272 | F | CH | CH | CH | CH | $CH_2Ph(4-COMe)$ |
| C-0273 | F | CH | CH | CH | CH | $CH_2Ph(2-COEt)$ |

(continued)

| Compound Number | $R^1$ | $X^1$ | $X^2$ | $X^3$ | $X^4$ | $R^2$ |
|---|---|---|---|---|---|---|
| C-0274 | F | CH | CH | CH | CH | $CH_2$Ph(3-COEt) |
| C-0275 | F | CH | CH | CH | CH | $CH_2$Ph(4-COEt) |
| C-0276 | F | CH | CH | CH | CH | $CH_2$Ph(2-$CO_2$H) |
| C-0277 | F | CH | CH | CH | CH | $CH_2$Ph(3-$CO_2$H) |
| C-0278 | F | CH | CH | CH | CH | $CH_2$Ph(4-$CO_2$H) |
| C-0279 | F | CH | CH | CH | CH | $CH_2$Ph(2-$CO_2$Me) |
| C-0280 | F | CH | CH | CH | CH | $CH_2$Ph(3-$CO_2$Me) |
| C-0281 | F | CH | CH | CH | CH | $CH_2$Ph(4-$CO_2$Me) |
| C-0282 | F | CH | CH | CH | CH | $CH_2$Ph(2-$CO_2$Et) |
| C-0283 | F | CH | CH | CH | CH | $CH_2$Ph(3-$CO_2$Et) |
| C-0284 | F | CH | CH | CH | CH | $CH_2$Ph(4-$CO_2$Et) |
| C-0285 | F | CH | CH | CH | CH | $CH_2$Ph(2-$CONH_2$) |
| C-0286 | F | CH | CH | CH | CH | $CH_2$Ph(3-$CONH_2$) |
| C-0287 | F | CH | CH | CH | CH | $CH_2$Ph(4-$CONH_2$) |
| C-0288 | F | CH | CH | CH | CH | $CH_2$Ph(2-CONHMe) |

[Table 52]

| Compound Number | $R^1$ | $X^1$ | $X^2$ | $X^3$ | $X^4$ | $R^2$ |
|---|---|---|---|---|---|---|
| C-0289 | F | CH | CH | CH | CH | $CH_2$Ph(3-CONHMe) |
| C-0290 | F | CH | CH | CH | CH | $CH_2$Ph(4-CONHMe) |
| C-0291 | F | CH | CH | CH | CH | $CH_2$Ph(2-CONHEt) |
| C-0292 | F | CH | CH | CH | CH | $CH_2$Ph(3-CONHEt) |
| C-0293 | F | CH | CH | CH | CH | $CH_2$Ph(4-CONHEt) |
| C-0294 | F | CH | CH | CH | CH | $CH_2$Ph(2-NHC HO) |
| C-0295 | F | CH | CH | CH | CH | $CH_2$Ph(3-NHC HO) |
| C-0296 | F | CH | CH | CH | CH | $CH_2$Ph(4-NHCHO) |
| C-0297 | F | CH | CH | CH | CH | $CH_2$Ph(2-NHCOMe) |
| C-0298 | F | CH | CH | CH | CH | $CH_2$Ph(3-NHCOMe) |
| C-0299 | F | CH | CH | CH | CH | $CH_2$Ph(4-NHCOMe) |
| C-0300 | F | CH | CH | CH | CH | $CH_2$Ph(2-NHCOEt) |
| C-0301 | F | CH | CH | CH | CH | $CH_2$Ph(3-NHCOEt) |
| C-0302 | F | CH | CH | CH | CH | $CH_2$Ph(4-NHCOEt) |
| C-0303 | F | CH | CH | CH | CH | $CH_2$Ph(2-CH=NOH) |
| C-0304 | F | CH | CH | CH | CH | $CH_2$Ph(3-CH=NOH) |

(continued)

| Compound Number | $R^1$ | $X^1$ | $X^2$ | $X^3$ | $X^4$ | $R^2$ |
|---|---|---|---|---|---|---|
| C-0305 | F | CH | CH | CH | CH | $CH_2Ph$(4-CH=NOH) |
| C-0306 | F | CH | CH | CH | CH | $CH_2Ph$(2-CH=NOMe) |
| C-0307 | F | CH | CH | CH | CH | $CH_2Ph$(3-CH=NOMe) |
| C-0308 | F | CH | CH | CH | CH | $CH_2Ph$(4-CH=NOMe) |
| C-0309 | F | CH | CH | CH | CH | $CH_2Ph$(2-CMe=NOH) |
| C-0310 | F | CH | CH | CH | CH | $CH_2Ph$(3-CMe=NOH) |
| C-0311 | F | CH | CH | CH | CH | $CH_2Ph$(4-CMe=NOH) |
| C-0312 | F | CH | CH | CH | CH | $CH_2Ph$(2-CMe=NOMe) |
| C-0313 | F | CH | CH | CH | CH | $CH_2Ph$(3-CMe=NOMe) |
| C-0314 | F | CH | CH | CH | CH | $CH_2Ph$(4-CMe=NOMe) |
| C-0315 | F | CH | CH | CH | CH | $CH_2Ph(2-NO_2)$ |
| C-0316 | F | CH | CH | CH | CH | $CH_2Ph(3-NO_2)$ |
| C-0317 | F | CH | CH | CH | CH | $CH_2Ph(4-NO_2)$ |
| C-0318 | F | CH | CH | CH | CH | $CH_2Ph(2-NH_2)$ |
| C-0319 | F | CH | CH | CH | CH | $CH_2Ph(3-NH_2)$ |
| C-0320 | F | CH | CH | CH | CH | $CH_2Ph(4-NH_2)$ |
| C-0321 | F | CH | CH | CH | CH | $CH_2Ph$(2-OH) |
| C-0322 | F | CH | CH | CH | CH | $CH_2Ph$(3-OH) |
| C-0323 | F | CH | CH | CH | CH | $CH_2Ph$(4-OH) |
| C-0324 | F | CH | CH | CH | CH | $CH_2Ph$(2-SH) |
| C-0325 | F | CH | CH | CH | CH | $CH_2Ph$(3-SH) |
| C-0326 | F | CH | CH | CH | CH | $CH_2Ph$(4-SH) |
| C-0327 | F | CH | CH | CH | CH | $CH_2Ph(2-NMe_2)$ |
| C-0328 | F | CH | CH | CH | CH | $CH_2Ph(3-NMe_2)$ |
| C-0329 | F | CH | CH | CH | CH | $CH_2Ph(4-NMe_2)$ |
| C-0330 | F | CH | CH | CH | CH | $CH_2Ph$(2-NHMe) |

[Table 53]

| Compound Number | $R^1$ | $X^1$ | $X^2$ | $X^3$ | $X^4$ | $R^2$ |
|---|---|---|---|---|---|---|
| C-0331 | F | CH | CH | CH | CH | $CH_2Ph$(3-NHMe) |
| C-0332 | F | CH | CH | CH | CH | $CH_2Ph$(4-NHMe) |
| C-0333 | F | CH | CH | CH | CH | $CH_2Ph$(2-CHO) |
| C-0334 | F | CH | CH | CH | CH | $CH_2Ph$(3-CHO) |
| C-0335 | F | CH | CH | CH | CH | $CH_2Ph$(4-CHO) |

(continued)

| Compound Number | $R^1$ | $X^1$ | $X^2$ | $X^3$ | $X^4$ | $R^2$ |
|---|---|---|---|---|---|---|
| C-0336 | F | CH | CH | CH | CH | $CH_2Ph$(2-Ph) |
| C-0337 | F | CH | CH | CH | CH | $CH_2Ph$(3-Ph) |
| C-0338 | F | CH | CH | CH | CH | $CH_2Ph$(4-Ph) |
| C-0339 | F | CH | CH | CH | CH | $CH_2Ph$(2-pyridin-2-yl) |
| C-0340 | F | CH | CH | CH | CH | $CH_2Ph$(3-pyridin-2-yl) |
| C-0341 | F | CH | CH | CH | CH | $CH_2Ph$(4-pyridin-2-yl) |
| C-0342 | F | CH | CH | CH | CH | $CH_2Ph$(2-pyridin-3-yl) |
| C-0343 | F | CH | CH | CH | CH | $CH_2Ph$(3-pyridin-3-yl) |
| C-0344 | F | CH | CH | CH | CH | $CH_2Ph$(4-pyridin-3-yl) |
| C-0345 | F | CH | CH | CH | CH | $CH_2Ph$(2-pyridin-4-yl) |
| C-0346 | F | CH | CH | CH | CH | $CH_2Ph$(3-pyridin-4-yl) |
| C-0347 | F | CH | CH | CH | CH | $CH_2Ph$(4-pyridin-4-yl) |
| C-0348 | F | CH | CH | CH | CH | $CH_2Ph(2-CH_2Ph)$ |
| C-0349 | F | CH | CH | CH | CH | $CH_2Ph(3-CH_2Ph)$ |
| C-0350 | F | CH | CH | CH | CH | $CH_2Ph(4-CH_2Ph)$ |
| C-0351 | F | CH | CH | CH | CH | $CH_2Ph(2,3-F_2)$ |
| C-0352 | F | CH | CH | CH | CH | $CH_2Ph(2,4-F_2)$ |
| C-0353 | F | CH | CH | CH | CH | $CH_2Ph(2,5-F_2)$ |
| C-0354 | F | CH | CH | CH | CH | $CH_2Ph(2,6-F_2)$ |
| C-0355 | F | CH | CH | CH | CH | $CH_2Ph(3,4-F_2)$ |
| C-0356 | F | CH | CH | CH | CH | $CH_2Ph(3,5-F_2)$ |
| C-0357 | F | CH | CH | CH | CH | $CH_2Ph$(2-Cl,3-F) |
| C-0358 | F | CH | CH | CH | CH | $CH_2Ph$(2-Cl,4-F) |
| C-0359 | F | CH | CH | CH | CH | $CH_2Ph$(2-Cl,5-F) |
| C-0360 | F | CH | CH | CH | CH | $CH_2Ph$(2-Cl,6-F) |
| C-0361 | F | CH | CH | CH | CH | $CH_2Ph$(3-Cl,2-F) |
| C-0362 | F | CH | CH | CH | CH | $CH_2Ph$(3-Cl,4-F) |
| C-0363 | F | CH | CH | CH | CH | $CH_2Ph$(3-Cl,5-F) |
| C-0364 | F | CH | CH | CH | CH | $CH_2Ph$(4-Cl,2-F) |
| C-0365 | F | CH | CH | CH | CH | $CH_2Ph$(4-Cl,3-F) |
| C-0366 | F | CH | CH | CH | CH | $CH_2Ph$(5-Cl,2-F) |
| C-0367 | F | CH | CH | CH | CH | $CH_2Ph$(2-F,3-Me) |
| C-0368 | F | CH | CH | CH | CH | $CH_2Ph$(2-F,4-Me) |
| C-0369 | F | CH | CH | CH | CH | $CH_2Ph$(2-F,5-Me) |

(continued)

| Compound Number | $R^1$ | $X^1$ | $X^2$ | $X^3$ | $X^4$ | $R^2$ |
|---|---|---|---|---|---|---|
| C-0370 | F | CH | CH | CH | CH | $CH_2Ph$(2-F,6-Me) |
| C-0371 | F | CH | CH | CH | CH | $CH_2Ph$(3-F,2-Me) |
| C-0372 | F | CH | CH | CH | CH | $CH_2Ph$(3-F,4-Me) |

[Table 54]

| Compound Number | $R^1$ | $X^1$ | $X^2$ | $X^3$ | $X^4$ | $R^2$ |
|---|---|---|---|---|---|---|
| C-0373 | F | CH | CH | CH | CH | $CH_2Ph$(3-F,5-Me) |
| C-0374 | F | CH | CH | CH | CH | $CH_2Ph$(4-F,2-Me) |
| C-0375 | F | CH | CH | CH | CH | $CH_2Ph$(4-F,3-Me) |
| C-0376 | F | CH | CH | CH | CH | $CH_2Ph$(5-F,2-Me) |
| C-0377 | F | CH | CH | CH | CH | $CH_2Ph$(2-CN,3-F) |
| C-0378 | F | CH | CH | CH | CH | $CH_2Ph$(2-CN,4-F) |
| C-0379 | F | CH | CH | CH | CH | $CH_2Ph$(2-CN,5-F) |
| C-0380 | F | CH | CH | CH | CH | $CH_2Ph$(2-CN,6-F) |
| C-0381 | F | CH | CH | CH | CH | $CH_2Ph$(3-CN,2-F) |
| C-0382 | F | CH | CH | CH | CH | $CH_2Ph$(3-CN,4-F) |
| C-0383 | F | CH | CH | CH | CH | $CH_2Ph$(3-CN,5-F) |
| C-0384 | F | CH | CH | CH | CH | $CH_2Ph$(4-CN,2-F) |
| C-0385 | F | CH | CH | CH | CH | $CH_2Ph$(4-CN,3-F) |
| C-0386 | F | CH | CH | CH | CH | $CH_2Ph$(5-CN,2-F) |
| C-0387 | F | CH | CH | CH | CH | $CH_2Ph$(2-F,3-OMe) |
| C-0388 | F | CH | CH | CH | CH | $CH_2Ph$(2-F,4-OMe) |
| C-0389 | F | CH | CH | CH | CH | $CH_2Ph$(2-F,5-OMe) |
| C-0390 | F | CH | CH | CH | CH | $CH_2Ph$(2-F,6-OMe) |
| C-0391 | F | CH | CH | CH | CH | $CH_2Ph$(3-F,2-OMe) |
| C-0392 | F | CH | CH | CH | CH | $CH_2Ph$(3-F,4-OMe) |
| C-0393 | F | CH | CH | CH | CH | $CH_2Ph$(3-F,5-OMe) |
| C-0394 | F | CH | CH | CH | CH | $CH_2Ph$(4-F,2-OMe) |
| C-0395 | F | CH | CH | CH | CH | $CH_2Ph$(4-F,3-OMe) |
| C-0396 | F | CH | CH | CH | CH | $CH_2Ph$(5-F,2-OMe) |
| C-0397 | F | CH | CH | CH | CH | $CH_2Ph(2,3-Cl_2)$ |
| C-0398 | F | CH | CH | CH | CH | $CH_2Ph(2,4-Cl_2)$ |
| C-0399 | F | CH | CH | CH | CH | $CH_2Ph(2,5-Cl_2)$ |
| C-0400 | F | CH | CH | CH | CH | $CH_2Ph(2,6-Cl_2)$ |

(continued)

| Compound Number | $R^1$ | $X^1$ | $X^2$ | $X^3$ | $X^4$ | $R^2$ |
|---|---|---|---|---|---|---|
| C-0401 | F | CH | CH | CH | CH | $CH_2Ph(3,4-Cl_2)$ |
| C-0402 | F | CH | CH | CH | CH | $CH_2Ph(3,5-Cl_2)$ |
| C-0403 | F | CH | CH | CH | CH | $CH_2$Ph(2-Cl,3-Me) |
| C-0404 | F | CH | CH | CH | CH | $CH_2$Ph(2-Cl,4-Me) |
| C-0405 | F | CH | CH | CH | CH | $CH_2$Ph(2-Cl,5-Me) |
| C-0406 | F | CH | CH | CH | CH | $CH_2$Ph(2-Cl,6-Me) |
| C-0407 | F | CH | CH | CH | CH | $CH_2$Ph(3-Cl,2-Me) |
| C-0408 | F | CH | CH | CH | CH | $CH_2$Ph(3-Cl,4-Me) |
| C-0409 | F | CH | CH | CH | CH | $CH_2$Ph(3-Cl,5-Me) |
| C-0410 | F | CH | CH | CH | CH | $CH_2$Ph(4-Cl,2-Me) |
| C-0411 | F | CH | CH | CH | CH | $CH_2$Ph(4-Cl,3-Me) |
| C-0412 | F | CH | CH | CH | CH | $CH_2$Ph(5-Cl,2-Me) |
| C-0413 | F | CH | CH | CH | CH | $CH_2$Ph(2-Cl,3-CN) |
| C-0414 | F | CH | CH | CH | CH | $CH_2$Ph(2-Cl,4-CN) |

[Table 55]

| Compound Number | $R^1$ | $X^1$ | $X^2$ | $X^3$ | $X^4$ | $R^2$ |
|---|---|---|---|---|---|---|
| C-0415 | F | CH | CH | CH | CH | $CH_2$Ph(2-Cl,5-CN) |
| C-0416 | F | CH | CH | CH | CH | $CH_2$Ph(2-Cl,6-CN) |
| C-0417 | F | CH | CH | CH | CH | $CH_2$Ph(3-Cl,2-CN) |
| C-0418 | F | CH | CH | CH | CH | $CH_2$Ph(3-Cl,4-CN) |
| C-0419 | F | CH | CH | CH | CH | $CH_2$Ph(3-Cl,5-CN) |
| C-0420 | F | CH | CH | CH | CH | $CH_2$Ph(4-Cl,2-CN) |
| C-0421 | F | CH | CH | CH | CH | $CH_2$Ph(4-Cl,3-CN) |
| C-0422 | F | CH | CH | CH | CH | $CH_2$Ph(5-Cl,2-CN) |
| C-0423 | F | CH | CH | CH | CH | $CH_2$Ph(2-Cl,3-OMe) |
| C-0424 | F | CH | CH | CH | CH | $CH_2$Ph(2-Cl,4-OMe) |
| C-0425 | F | CH | CH | CH | CH | $CH_2$Ph(2-Cl,5-OMe) |
| C-0426 | F | CH | CH | CH | CH | $CH_2$Ph(2-Cl,6-OMe) |
| C-0427 | F | CH | CH | CH | CH | $CH_2$Ph(3-Cl,2-OMe) |
| C-0428 | F | CH | CH | CH | CH | $CH_2$Ph(3-Cl,4-OMe) |
| C-0429 | F | CH | CH | CH | CH | $CH_2$Ph(3-Cl,5-OMe) |
| C-0430 | F | CH | CH | CH | CH | $CH_2$Ph(4-Cl,2-OMe) |
| C-0431 | F | CH | CH | CH | CH | $CH_2$Ph(4-Cl,3-OMe) |

(continued)

| Compound Number | $R^1$ | $X^1$ | $X^2$ | $X^3$ | $X^4$ | $R^2$ |
|---|---|---|---|---|---|---|
| C-0432 | F | CH | CH | CH | CH | $CH_2Ph$(5-Cl,2-OMe) |
| C-0433 | F | CH | CH | CH | CH | $CH_2Ph(2,3-Me_2)$ |
| C-0434 | F | CH | CH | CH | CH | $CH_2Ph(2,4-Me_2)$ |
| C-0435 | F | CH | CH | CH | CH | $CH_2Ph(2,5-Me_2)$ |
| C-0436 | F | CH | CH | CH | CH | $CH_2Ph(2,6-Me_2)$ |
| C-0437 | F | CH | CH | CH | CH | $CH_2Ph(3,4-Me_2)$ |
| C-0438 | F | CH | CH | CH | CH | $CH_2Ph(3,5-Me_2)$ |
| C-0439 | F | CH | CH | CH | CH | $CH_2Ph$(2-CN,3-Me) |
| C-0440 | F | CH | CH | CH | CH | $CH_2Ph$(2-CN,4-Me) |
| C-0441 | F | CH | CH | CH | CH | $CH_2Ph$(2-CN,5-Me) |
| C-0442 | F | CH | CH | CH | CH | $CH_2Ph$(2-CN,6-Me) |
| C-0443 | F | CH | CH | CH | CH | $CH_2Ph$(3-CN,2-Me) |
| C-0444 | F | CH | CH | CH | CH | $CH_2Ph$(3-CN,4-Me) |
| C-0445 | F | CH | CH | CH | CH | $CH_2Ph$(3-CN,5-Me) |
| C-0446 | F | CH | CH | CH | CH | $CH_2Ph$(4-CN,2-Me) |
| C-0447 | F | CH | CH | CH | CH | $CH_2Ph$(4-CN,3-Me) |
| C-0448 | F | CH | CH | CH | CH | $CH_2Ph$(5-CN,2-Me) |
| C-0449 | F | CH | CH | CH | CH | $CH_2Ph$(2-OMe,3-Me) |
| C-0450 | F | CH | CH | CH | CH | $CH_2Ph$(2-OMe,4-Me) |
| C-0451 | F | CH | CH | CH | CH | $CH_2Ph$(2-OMe,5-Me) |
| C-0452 | F | CH | CH | CH | CH | $CH_2Ph$(2-OMe,6-Me) |
| C-0453 | F | CH | CH | CH | CH | $CH_2Ph$(3-OMe,2-Me) |
| C-0454 | F | CH | CH | CH | CH | $CH_2Ph$(3-OMe,4-Me) |
| C-0455 | F | CH | CH | CH | CH | $CH_2Ph$(3-OMe,5-Me) |
| C-0456 | F | CH | CH | CH | CH | $CH_2Ph$(4-OMe,2-Me) |

[Table 56]

| Compound Number | $R^1$ | $X^1$ | $X^2$ | $X^3$ | $X^4$ | $R^2$ |
|---|---|---|---|---|---|---|
| C-0457 | F | CH | CH | CH | CH | $CH_2Ph$(4-OMe,3-Me) |
| C-0458 | F | CH | CH | CH | CH | $CH_2Ph$(5-OMe,2-Me) |
| C-0459 | F | CH | CH | CH | CH | $CH_2Ph[2,3-(OMe)_2]$ |
| C-0460 | F | CH | CH | CH | CH | $CH_2Ph[2,4-(OMe)_2]$ |
| C-0461 | F | CH | CH | CH | CH | $CH_2Ph[2,5-(OMe)_2]$ |
| C-0462 | F | CH | CH | CH | CH | $CH_2Ph[2,6-(OMe)_2]$ |

(continued)

| Compound Number | $R^1$ | $X^1$ | $X^2$ | $X^3$ | $X^4$ | $R^2$ |
|---|---|---|---|---|---|---|
| C-0463 | F | CH | CH | CH | CH | $CH_2Ph[3,4-(OMe)_2]$ |
| C-0464 | F | CH | CH | CH | CH | $CH_2Ph[3,5-(OMe)_2]$ |
| C-0465 | F | CH | CH | CH | CH | $CH_2Ph(2-CN,3-OMe)$ |
| C-0466 | F | CH | CH | CH | CH | $CH_2Ph(2-CN,4-OMe)$ |
| C-0467 | F | CH | CH | CH | CH | $CH_2Ph(2-CN,5-OMe)$ |
| C-0468 | F | CH | CH | CH | CH | $CH_2Ph(2-CN,6-OMe)$ |
| C-0469 | F | CH | CH | CH | CH | $CH_2Ph(3-CN,2-OMe)$ |
| C-0470 | F | CH | CH | CH | CH | $CH_2Ph(3-CN,4-OMe)$ |
| C-0471 | F | CH | CH | CH | CH | $CH_2Ph(3-CN,5-OMe)$ |
| C-0472 | F | CH | CH | CH | CH | $CH_2Ph(4-CN,2-OMe)$ |
| C-0473 | F | CH | CH | CH | CH | $CH_2Ph(4-CN,3-OMe)$ |
| C-0474 | F | CH | CH | CH | CH | $CH_2Ph(5-CN,2-OMe)$ |
| C-0475 | F | CH | CH | CH | CH | $CH_2Ph[2,3-(CN)_2]$ |
| C-0476 | F | CH | CH | CH | CH | $CH_2Ph[2,4-(CN)_2]$ |
| C-0477 | F | CH | CH | CH | CH | $CH_2Ph[2,5-(CN)_2]$ |
| C-0478 | F | CH | CH | CH | CH | $CH_2Ph[2,6-(CN)_2]$ |
| C-0479 | F | CH | CH | CH | CH | $CH_2Ph[3,4-(CN)_2]$ |
| C-0480 | F | CH | CH | CH | CH | $CH_2Ph[3,5-(CN)_2]$ |
| C-0481 | F | CF | CH | CH | CH | H |
| C-0482 | F | CF | CH | CH | CH | $CH_2Ph$ |
| C-0483 | F | CH | CF | CH | CH | H |
| C-0484 | F | CH | CF | CH | CH | $CH_2Ph$ |
| C-0485 | F | CMe | CH | CH | CH | H |
| C-0486 | F | CMe | CH | CH | CH | $CH_2Ph$ |
| C-0487 | F | CH | CMe | CH | CH | H |
| C-0488 | F | CH | CMe | CH | CH | $CH_2Ph$ |
| C-0489 | Cl | CH | CH | CH | CH | H |
| C-0490 | Cl | CH | CH | CH | CH | $CH_2Ph$ |
| C-0491 | F | N | CH | CH | CH | H |
| C-0492 | F | N | CH | CH | CH | $CH_2Ph$ |
| C-0493 | F | CH | N | CH | CH | H |
| C-0494 | F | CH | N | CH | CH | $CH_2Ph$ |
| C-0495 | F | N | N | CH | CH | H |
| C-0496 | F | N | N | CH | CH | $CH_2Ph$ |
| C-0497 | F | N | CH | N | CH | H |

(continued)

| Compound Number | $R^1$ | $X^1$ | $X^2$ | $X^3$ | $X^4$ | $R^2$ |
|---|---|---|---|---|---|---|
| C-0498 | F | N | CH | N | CH | $CH_2Ph$ |

[Table 57]

| Compound Number | $R^1$ | $X^1$ | $X^2$ | $X^3$ | $X^4$ | $R^2$ |
|---|---|---|---|---|---|---|
| C-0499 | F | N | CH | CH | N | H |
| C-0500 | F | N | CH | CH | N | $CH_2Ph$ |
| C-0501 | F | CH | N | CH | N | H |
| C-0502 | F | CH | N | CH | N | $CH_2Ph$ |
| C-0503 | H | CH | CH | CH | CH | H |
| C-0504 | H | CH | CH | CH | CH | $CH_2Ph$ |
| C-0505 | F | CH | CH | CH | CH | $CH_2CH_2Ph$(2-F) |
| C-0506 | F | CH | CH | CH | CH | $CH_2CH_2Ph$(3-F) |
| C-0507 | F | CH | CH | CH | CH | $CH_2CH_2Ph$(4-F) |
| C-0508 | F | CH | CH | CH | CH | $CH_2CH$=CHPh |
| C-0509 | F | CH | CH | CH | CH | $CH_2CCPh$ |
| C-0510 | F | CH | CH | CH | CH | $CH_2CCTMS$ |
| C-0511 | F | CH | CH | CH | CH | $CH_2F$ |
| C-0512 | F | CH | CH | CH | CH | $CH_2CH_2F$ |
| C-0513 | F | CH | CH | CH | CH | $CH_2CH_2CH_2F$ |
| C-0514 | F | CH | CH | CH | CH | $CH_2CHO$ |
| C-0515 | F | CH | CH | CH | CH | $CH_2CH_2CHO$ |
| C-0516 | F | CH | CH | CH | CH | $CH_2COEt$ |
| C-0517 | F | CH | CH | CH | CH | $CH_2CO$n-Pr |
| C-0518 | F | CH | CH | CH | CH | $CH_2CO$n-Bu |
| C-0519 | F | CH | CH | CH | CH | $CH_2CO$i-Pr |
| C-0520 | F | CH | CH | CH | CH | $CH_2CO$t-Bu |
| C-0521 | F | CH | CH | CH | CH | $CH_2CO$i-Bu |
| C-0522 | F | CH | CH | CH | CH | $CH_2CO$sec-Bu |
| C-0523 | F | CH | CH | CH | CH | $CH_2COCH_2$t-Bu |
| C-0524 | F | CH | CH | CH | CH | $CH_2COCH(CH_2CH_3)Et$ |
| C-0525 | F | CH | CH | CH | CH | $CH_2CO$c-Pr |
| C-0526 | F | CH | CH | CH | CH | $CH_2COCH_2$c-Pr |
| C-0527 | F | CH | CH | CH | CH | $CH_2CO$c-Bu |
| C-0528 | F | CH | CH | CH | CH | $CH_2CO$c-Pen |

(continued)

| Compound Number | $R^1$ | $X^1$ | $X^2$ | $X^3$ | $X^4$ | $R^2$ |
|---|---|---|---|---|---|---|
| C-0529 | F | CH | CH | CH | CH | $CH_2CH_2COMe$ |
| C-0530 | F | CH | CH | CH | CH | $CH_2CH_2COEt$ |
| C-0531 | F | CH | CH | CH | CH | $CH_2COCF_3$ |
| C-0532 | F | CH | CH | CH | CH | $CH_2COCHF_2$ |
| C-0533 | F | CH | CH | CH | CH | $CH_2COCH_2CF_3$ |
| C-0534 | F | CH | CH | CH | CH | $CH_2COCH_2CHF_2$ |
| C-0535 | F | CH | CH | CH | CH | $CH_2COCH_2OCH_3$ |
| C-0536 | F | CH | CH | CH | CH | $CH_2COCH_2Ph$ |
| C-0537 | F | CH | CH | CH | CH | $CH_2CO$(pyrrolidin-1-yl) |
| C-0538 | F | CH | CH | CH | CH | $CH_2CO$(1H-pyrazol-1-yl) |
| C-0539 | F | CH | CH | CH | CH | $CH_2CO$(morpholino-1-yl) |
| C-0540 | F | CH | CH | CH | CH | $CH_2CO$(pyridin-2-yl) |

[Table 58]

| Compound Number | $R^1$ | $X^1$ | $X^2$ | $X^3$ | $X^4$ | $R^2$ |
|---|---|---|---|---|---|---|
| C-0541 | F | CH | CH | CH | CH | $CH_2CO$(pyridin-3-yl) |
| C-0542 | F | CH | CH | CH | CH | $CH_2CO$(pyridin-4-yl) |
| C-0543 | F | CH | CH | CH | CH | $CH_2COCH_2$(pyridin-2-yl) |
| C-0544 | F | CH | CH | CH | CH | $CH_2COCH_2$(pyridin-3-yl) |
| C-0545 | F | CH | CH | CH | CH | $CH_2COCH_2$(pyridin-4-yl) |
| C-0546 | F | CH | CH | CH | CH | $CH_2CO_2H$ |
| C-0547 | F | CH | CH | CH | CH | $CH_2CH_2CO_2Me$ |
| C-0548 | F | CH | CH | CH | CH | $CH(CH_2CH_3)CO_2Et$ |
| C-0549 | F | CH | CH | CH | CH | $CH(F)CO_2Et$ |
| C-0550 | F | CH | CH | CH | CH | $CH(CO_2Et)_2$ |
| C-0551 | F | CH | CH | CH | CH | $CH_2CH_2CO_2Et$ |
| C-0552 | F | CH | CH | CH | CH | $CH_2CH_2CH_2CO_2Et$ |
| C-0553 | F | CH | CH | CH | CH | $CH_2CH_2CH_2CH_2CH_2CO_2Et$ |
| C-0554 | F | CH | CH | CH | CH | $CH_2CO_2$n-Pr |
| C-0555 | F | CH | CH | CH | CH | $CH(CH_3)CO_2$n-Pr |
| C-0556 | F | CH | CH | CH | CH | $C(CH_3)(CH_3)CO_2$n-Pr |
| C-0557 | F | CH | CH | CH | CH | $CH_2CO_2$i-Pr |
| C-0558 | F | CH | CH | CH | CH | $CH(CH_3)CO_2$i-Pr |
| C-0559 | F | CH | CH | CH | CH | $C(CH_3)(CH_3)CO_2$i-Pr |

(continued)

| Compound Number | $R^1$ | $X^1$ | $X^2$ | $X^3$ | $X^4$ | $R^2$ |
|---|---|---|---|---|---|---|
| C-0560 | F | CH | CH | CH | CH | $CH_2CO_2$n-Bu |
| C-0561 | F | CH | CH | CH | CH | $CH_2CO_2$t-Bu |
| C-0562 | F | CH | CH | CH | CH | $CH_2CO_2$i-Bu |
| C-0563 | F | CH | CH | CH | CH | $CH_2CO_2$c-Pr |
| C-0564 | F | CH | CH | CH | CH | $CH_2CO_2CH_2$c-Pr |
| C-0565 | F | CH | CH | CH | CH | $CH_2CO_2$c-Bu |
| C-0566 | F | CH | CH | CH | CH | $CH_2CO_2$c-Pen |
| C-0567 | F | CH | CH | CH | CH | $CH_2CO_2CH=CH_2$ |
| C-0568 | F | CH | CH | CH | CH | $CH_2CO_2CH_2CH=CH_2$ |
| C-0569 | F | CH | CH | CH | CH | $CH_2CO_2CH_2CCH$ |
| C-0570 | F | CH | CH | CH | CH | $CH_2CO_2CF_3$ |
| C-0571 | F | CH | CH | CH | CH | $CH_2CO_2CHF_2$ |
| C-0572 | F | CH | CH | CH | CH | $CH_2CO_2CH_2CF_3$ |
| C-0573 | F | CH | CH | CH | CH | $CH_2CO_2CH_2CHF_2$ |
| C-0574 | F | CH | CH | CH | CH | $CH_2CO_2CH_2CN$ |
| C-0575 | F | CH | CH | CH | CH | $CH_2CO_2CH_2COMe$ |
| C-0576 | F | CH | CH | CH | CH | $CH_2CO_2CH_2CO_2Et$ |
| C-0577 | F | CH | CH | CH | CH | $CH_2CO_2CH_2CO_2CH_2CF_3$ |
| C-0578 | F | CH | CH | CH | CH | $CH_2CO_2CH_2Ph$ |
| C-0579 | F | CH | CH | CH | CH | $CH_2CO_2$(pyridin-2-yl) |
| C-0580 | F | CH | CH | CH | CH | $CH_2CO_2$(pyridin-3-yl) |
| C-0581 | F | CH | CH | CH | CH | $CH_2CO_2$(pyridin-4-yl) |
| C-0582 | F | CH | CH | CH | CH | $CH_2CO_2CH_2$(pyridin-2-yl) |

[Table 59]

| Compound Number | $R^1$ | $X^1$ | $X^2$ | $X^3$ | $X^4$ | $R^2$ |
|---|---|---|---|---|---|---|
| C-0583 | F | CH | CH | CH | CH | $CH_2CO_2CH_2$[pyridin-2-yl(6-F)] |
| C-0584 | F | CH | CH | CH | CH | $CH_2CO_2CH_2$[pyridin-2-yl(6-Cl)] |
| C-0585 | F | CH | CH | CH | CH | $CH_2CO_2CH_{2[}$pyridin-2-yl(6-Me)] |
| C-0586 | F | CH | CH | CH | CH | $CH_2CO_2CH_2$(pyridin-3-yl) |
| C-0587 | F | CH | CH | CH | CH | $CH_2CO_2CH_{2[}$pyridin-3-yl(6-F)] |
| C-0588 | F | CH | CH | CH | CH | $CH_2CO_2CH_2$[pyridin-3-yl(6-Cl)] |
| C-0589 | F | CH | CH | CH | CH | $CH_2CO_2CH_{2[}$pyridin-3-yl(6-Me)] |
| C-0590 | F | CH | CH | CH | CH | $CH_2CO_2CH_2$(pyridin-4-yl) |

(continued)

| Compound Number | $R^1$ | $X^1$ | $X^2$ | $X^3$ | $X^4$ | $R^2$ |
|---|---|---|---|---|---|---|
| C-0591 | F | CH | CH | CH | CH | $CH_2CO_2CH_2$(1H-pyrazol-1-yl) |
| C-0592 | F | CH | CH | CH | CH | $CH_2CO_2CH_2$[1H-pyrazol-3-yl(1-Me)] |
| C-0593 | F | CH | CH | CH | CH | $CH_2CO_2CH_2$[1H-pyrazol-4-yl(1-Me)] |
| C-0594 | F | CH | CH | CH | CH | $CH_2CO_2CH_2$[1H-pyrazol-5-yl(1-Me)] |
| C-0595 | F | CH | CH | CH | CH | $CH_2CONMe_2$ |
| C-0596 | F | CH | CH | CH | CH | $CH_2CON(CH_3)Et$ |
| C-0597 | F | CH | CH | CH | CH | $CH_2CON(CH_3)$n-Pr |
| C-0598 | F | CH | CH | CH | CH | $CH_2CONEt_2$ |
| C-0599 | F | CH | CH | CH | CH | $CH_2CONH$n-Pr |
| C-0600 | F | CH | CH | CH | CH | $CH_2CONH$i-Pr |
| C-0601 | F | CH | CH | CH | CH | $CH_2CONH$n-Bu |
| C-0602 | F | CH | CH | CH | CH | $CH_2CONH$t-Bu |
| C-0603 | F | CH | CH | CH | CH | $CH_2CONH$i-Bu |
| C-0604 | F | CH | CH | CH | CH | $CH_2CONH$c-Pr |
| C-0605 | F | CH | CH | CH | CH | $CH_2CONH$cPr(1-CN) |
| C-0606 | F | CH | CH | CH | CH | $CH_2CONHCH_2$c-Pr |
| C-0607 | F | CH | CH | CH | CH | $CH_2CONHCH=CH_2$ |
| C-0608 | F | CH | CH | CH | CH | $CH_2CONHCH_2CH=CH_2$ |
| C-0609 | F | CH | CH | CH | CH | $CH_2CONHCH_2CCH$ |
| C-0610 | F | CH | CH | CH | CH | $CH_2CONHCF_3$ |
| C-0611 | F | CH | CH | CH | CH | $CH_2CONHCHF_2$ |
| C-0612 | F | CH | CH | CH | CH | $CH_2CONHCH_2CF_3$ |
| C-0613 | F | CH | CH | CH | CH | $CH_2CONHCH_2CHF_2$ |
| C-0614 | F | CH | CH | CH | CH | $CH_2CH_2CONHMe$ |
| C-0615 | F | CH | CH | CH | CH | $CH_2CH_2CONHEt$ |
| C-0616 | F | CH | CH | CH | CH | $CH_2CH_2CONMe_2$ |
| C-0617 | F | CH | CH | CH | CH | $CH_2CH_2CON(CH_3)Et$ |
| C-0618 | F | CH | CH | CH | CH | $CH_2CONHCH_2Ph$ |
| C-0619 | F | CH | CH | CH | CH | $CH_2CONH$(pyridin-2-yl) |
| C-0620 | F | CH | CH | CH | CH | $CH_2CONH$(pyridin-3-yl) |
| C-0621 | F | CH | CH | CH | CH | $CH_2CONH$(pyridin-4-yl) |
| C-0622 | F | CH | CH | CH | CH | $CH_2CONHCH_2$(pyridin-2-yl) |
| C-0623 | F | CH | CH | CH | CH | $CH_2CONHCH_2$(pyridin-3-yl) |
| C-0624 | F | CH | CH | CH | CH | $CH_2CONHCH_2$(pyridin-4-yl) |

[Table 60]

| Compound Number | $R^1$ | $X^1$ | $X^2$ | $X^3$ | $X^4$ | $R^2$ |
|---|---|---|---|---|---|---|
| C-0625 | F | CH | CH | CH | CH | $CH_2CH_2NHCHO$ |
| C-0626 | F | CH | CH | CH | CH | $CH_2CH_2NHCOMe$ |
| C-0627 | F | CH | CH | CH | CH | $CH_2CH_2NHCOEt$ |
| C-0628 | F | CH | CH | CH | CH | $CH_2CH_2N(CH_3)CHO$ |
| C-0629 | F | CH | CH | CH | CH | $CH_2CH_2N(CH_3)COMe$ |
| C-0630 | F | CH | CH | CH | CH | $CH_2CH_2N(CH_3)COEt$ |
| C-0631 | F | CH | CH | CH | CH | $CH_2CH_2N(CH_2CH_3)CHO$ |
| C-0632 | F | CH | CH | CH | CH | $CH_2CH_2N(CH_2CH_3)COMe$ |
| C-0633 | F | CH | CH | CH | CH | $CH_2CH_2N(CH_2CH_3)COEt$ |
| C-0634 | F | CH | CH | CH | CH | $CH_2NHCO$c-Pr |
| C-0635 | F | CH | CH | CH | CH | $CH_2NHCOCH_2$c-Pr |
| C-0636 | F | CH | CH | CH | CH | $CH_2CH_2NHCO$c-Pr |
| C-0637 | F | CH | CH | CH | CH | $CH_2CH_2NHCOCH_2$c-Pr |
| C-0638 | F | CH | CH | CH | CH | $CH_2NHCOCF_3$ |
| C-0639 | F | CH | CH | CH | CH | $CH_2NHCOCHF_2$ |
| C-0640 | F | CH | CH | CH | CH | $CH_2NHCOCH_2CF_3$ |
| C-0641 | F | CH | CH | CH | CH | $CH_2NHCOCH_2CHF_2$ |
| C-0642 | F | CH | CH | CH | CH | $CH_2NHCOCH_2Ph$ |
| C-0643 | F | CH | CH | CH | CH | $CH_2NHCO$(pyridin-2-yl) |
| C-0644 | F | CH | CH | CH | CH | $CH_2NHCO$(pyridin-3-yl) |
| C-0645 | F | CH | CH | CH | CH | $CH_2NHCO$(pyridin-4-yl) |
| C-0646 | F | CH | CH | CH | CH | $CH_2NHCOCH_2$(pyridin-2-yl) |
| C-0647 | F | CH | CH | CH | CH | $CH_2NHCOCH_2$(pyridin-3-yl) |
| C-0648 | F | CH | CH | CH | CH | $CH_2NHCOCH_2$(pyridin-4-yl) |
| C-0649 | F | CH | CH | CH | CH | $CH_2OCHO$ |
| C-0650 | F | CH | CH | CH | CH | $CH_2CH_2OCHO$ |
| C-0651 | F | CH | CH | CH | CH | $CH_2OCOMe$ |
| C-0652 | F | CH | CH | CH | CH | $CH_2OCOEt$ |
| C-0653 | F | CH | CH | CH | CH | $CH_2OCO$n-Pr |
| C-0654 | F | CH | CH | CH | CH | $CH_2OCO$i-Pr |
| C-0655 | F | CH | CH | CH | CH | $CH_2OCO$n-Bu |
| C-0656 | F | CH | CH | CH | CH | $CH_2OCO$i-Bu |
| C-0657 | F | CH | CH | CH | CH | $CH_2OCO$t-Bu |
| C-0658 | F | CH | CH | CH | CH | $CH_2CH_2OCOMe$ |

(continued)

| Compound Number | $R^1$ | $X^1$ | $X^2$ | $X^3$ | $X^4$ | $R^2$ |
|---|---|---|---|---|---|---|
| C-0659 | F | CH | CH | CH | CH | $CH_2CH_2OCOEt$ |
| C-0660 | F | CH | CH | CH | CH | $CH_2CH_2CH_2OCOMe$ |
| C-0661 | F | CH | CH | CH | CH | $CH_2OCOc$-Pr |
| C-0662 | F | CH | CH | CH | CH | $CH_2OCOCH_2c$-Pr |
| C-0663 | F | CH | CH | CH | CH | $CH_2OCOCF_3$ |
| C-0664 | F | CH | CH | CH | CH | $CH_2OCOCHF_2$ |
| C-0665 | F | CH | CH | CH | CH | $CH_2OCOCH_2CF_3$ |
| C-0666 | F | CH | CH | CH | CH | $CH_2OCOCH_2CHF_2$ |

[Table 61]

| Compound Number | $R^1$ | $X^1$ | $X^2$ | $X^3$ | $X^4$ | $R^2$ |
|---|---|---|---|---|---|---|
| C-0667 | F | CH | CH | CH | CH | $CH_2OCOPh$ |
| C-0668 | F | CH | CH | CH | CH | $CH_2OCOCH_2Ph$ |
| C-0669 | F | CH | CH | CH | CH | $CH_2OCO$(pyridin-2-yl) |
| C-0670 | F | CH | CH | CH | CH | $CH_2OCO$(pyridin-3-yl) |
| C-0671 | F | CH | CH | CH | CH | $CH_2OCO$(pyridin-4-yl) |
| C-0672 | F | CH | CH | CH | CH | $CH_2OCOCH_2$(pyridin-2-yl) |
| C-0673 | F | CH | CH | CH | CH | $CH_2OCOCH_2$(pyridin-3-yl) |
| C-0674 | F | CH | CH | CH | CH | $CH_2OCOCH_2$(pyridin-4-yl) |
| C-0675 | F | CH | CH | CH | CH | $CH_2SO_2NHMe$ |
| C-0676 | F | CH | CH | CH | CH | $CH_2SO_2NHEt$ |
| C-0677 | F | CH | CH | CH | CH | $CH_2SO_2NMe_2$ |
| C-0678 | F | CH | CH | CH | CH | $CH_2SO_2N(CH_3)Et$ |
| C-0679 | F | CH | CH | CH | CH | $CH_2CH_2SO_2NHMe$ |
| C-0680 | F | CH | CH | CH | CH | $CH_2CH_2SO_2NHEt$ |
| C-0681 | F | CH | CH | CH | CH | $CH_2CH_2SO_2NMe_2$ |
| C-0682 | F | CH | CH | CH | CH | $CH_2CH_2SO_2(CH_3)Et$ |
| C-0683 | F | CH | CH | CH | CH | $CH_2SO_2NHc$-Pr |
| C-0684 | F | CH | CH | CH | CH | $CH_2SO_2NHCH_2c$-Pr |
| C-0685 | F | CH | CH | CH | CH | $CH_2SO_2NHCF_3$ |
| C-0686 | F | CH | CH | CH | CH | $CH_2SO_2NHCH_2CF_3$ |
| C-0687 | F | CH | CH | CH | CH | $CH_2SO_2NHCHF_2$ |
| C-0688 | F | CH | CH | CH | CH | $CH_2SO_2NHCH_2CHF_2$ |
| C-0689 | F | CH | CH | CH | CH | $CH_2SO_2NHPh$ |

(continued)

| Compound Number | $R^1$ | $X^1$ | $X^2$ | $X^3$ | $X^4$ | $R^2$ |
|---|---|---|---|---|---|---|
| C-0690 | F | CH | CH | CH | CH | $CH_2SO_2NHCH_2Ph$ |
| C-0691 | F | CH | CH | CH | CH | $CH_2SO_2NH$(pyridin-2-yl) |
| C-0692 | F | CH | CH | CH | CH | $CH_2SO_2NH$(pyridin-3-yl) |
| C-0693 | F | CH | CH | CH | CH | $CH_2SO_2NH$(pyridin-4-yl) |
| C-0694 | F | CH | CH | CH | CH | $CH_2SO_2NHCH_2$(pyridin-2-yl) |
| C-0695 | F | CH | CH | CH | CH | $CH_2SO_2NHCH_2$(pyridin-3-yl) |
| C-0696 | F | CH | CH | CH | CH | $CH_2SO_2NHCH_2$(pyridin-4-yl) |
| C-0697 | F | CH | CH | CH | CH | $CH_2NHSO_2Me$ |
| C-0698 | F | CH | CH | CH | CH | $CH_2N(CH_3)SO_2Me$ |
| C-0699 | F | CH | CH | CH | CH | $CH_2NHSO_2Et$ |
| C-0700 | F | CH | CH | CH | CH | $CH_2NHSO_2$c-Pr |
| C-0701 | F | CH | CH | CH | CH | $CH_2NHSO_2CH_2$c-Pr |
| C-0702 | F | CH | CH | CH | CH | $CH_2NHSO_2CF_3$ |
| C-0703 | F | CH | CH | CH | CH | $CH_2NHSO_2CH_2CF_3$ |
| C-0704 | F | CH | CH | CH | CH | $CH_2NHSO_2CHF_2$ |
| C-0705 | F | CH | CH | CH | CH | $CH_2NHSO_2CH_2CHF_2$ |
| C-0706 | F | CH | CH | CH | CH | $CH_2CH_2NHSO_2Me$ |
| C-0707 | F | CH | CH | CH | CH | $CH_2CH_2N(CH_3)SO_2Me$ |
| C-0708 | F | CH | CH | CH | CH | $CH_2NHSO_2Ph$ |

[Table 62]

| Compound Number | $R^1$ | $X^1$ | $X^2$ | $X^3$ | $X^4$ | $R^2$ |
|---|---|---|---|---|---|---|
| C-0709 | F | CH | CH | CH | CH | $CH_2NHSO_2CH_2Ph$ |
| C-0710 | F | CH | CH | CH | CH | $CH_2NHSO_2$(pyridin-2-yl) |
| C-0711 | F | CH | CH | CH | CH | $CH_2NHSO_2$(pyridin-3-yl) |
| C-0712 | F | CH | CH | CH | CH | $CH_2NHSO_2$(pyridin-4-yl) |
| C-0713 | F | CH | CH | CH | CH | $CH_2NHSO_2CH_2$(pyridin-2-yl) |
| C-0714 | F | CH | CH | CH | CH | $CH_2NHSO_2CH_2$(pyridin-3-yl) |
| C-0715 | F | CH | CH | CH | CH | $CH_2NHSO_2CH_2$(pyridin-4-yl) |
| C-0716 | F | CH | CH | CH | CH | $CH_2OH$ |
| C-0717 | F | CH | CH | CH | CH | $CH_2CH_2OH$ |
| C-0718 | F | CH | CH | CH | CH | $CH_2CH(OH)CH_2CH_2CH_3$ |
| C-0719 | F | CH | CH | CH | CH | $CH_2CH_2O$n-Pr |
| C-0720 | F | CH | CH | CH | CH | $CH_2CH_2O$i-Pr |

(continued)

| Compound Number | $R^1$ | $X^1$ | $X^2$ | $X^3$ | $X^4$ | $R^2$ |
|---|---|---|---|---|---|---|
| C-0721 | F | CH | CH | CH | CH | $CH_2CH_2$Oc-Pr |
| C-0722 | F | CH | CH | CH | CH | $CH_2CH_2OCH_2$c-Pr |
| C-0723 | F | CH | CH | CH | CH | $CH_2CH_2$Ot-Bu |
| C-0724 | F | CH | CH | CH | CH | $CH_2CH_2$Oi-Bu |
| C-0725 | F | CH | CH | CH | CH | $CH_2CH_2$Osec-Bu |
| C-0726 | F | CH | CH | CH | CH | $CH(CH_3)OEt$ |
| C-0727 | F | CH | CH | CH | CH | $CH_2CH(CH_3)OEt$ |
| C-0728 | F | CH | CH | CH | CH | $CH_2CH(OMe)_2$ |
| C-0729 | F | CH | CH | CH | CH | $CH_2CH(OEt)_2$ |
| C-0730 | F | CH | CH | CH | CH | $CH(CH_3)CH(OMe)_2$ |
| C-0731 | F | CH | CH | CH | CH | $CH_2C(OMe)_2Me$ |
| C-0732 | F | CH | CH | CH | CH | $CH(CH_3)CH(OEt)_2$ |
| C-0733 | F | CH | CH | CH | CH | $CH_2C(OEt)_2Me$ |
| C-0734 | F | CH | CH | CH | CH | $CH_2CH_2CH(OMe)_2$ |
| C-0735 | F | CH | CH | CH | CH | $CH_2CH_2CH(OEt)_2$ |
| C-0736 | F | CH | CH | CH | CH | $CH_2CH_2CH_2OMe$ |
| C-0737 | F | CH | CH | CH | CH | $CH_2OCHF_2$ |
| C-0738 | F | CH | CH | CH | CH | $CH_2OCH_2CHF_2$ |
| C-0739 | F | CH | CH | CH | CH | $CH_2C(OH)_2CF_3$ |
| C-0740 | F | CH | CH | CH | CH | $CH_2CH_2OCHF_2$ |
| C-0741 | F | CH | CH | CH | CH | $CH_2CH_2OCH_2CHF_2$ |
| C-0742 | F | CH | CH | CH | CH | $CH_2OCH_2Ph$ |
| C-0743 | F | CH | CH | CH | CH | $CH_2OCH_2CH_2OMe$ |
| C-0744 | F | CH | CH | CH | CH | $CH_2CH_2OCH_2CH_2OMe$ |
| C-0745 | F | CH | CH | CH | CH | $CH_2O$(pyridin-2-yl) |
| C-0746 | F | CH | CH | CH | CH | $CH_2O$(pyridin-3-yl) |
| C-0747 | F | CH | CH | CH | CH | $CH_2O$(pyridin-4-yl) |
| C-0748 | F | CH | CH | CH | CH | $CH_2OCH_2$(pyridin-2-yl) |
| C-0749 | F | CH | CH | CH | CH | $CH_2OCH_2$(pyridin-3-yl) |
| C-0750 | F | CH | CH | CH | CH | $CH_2OCH_2$(pyridin-4-yl) |

[Table 63]

| Compound Number | $R^1$ | $X^1$ | $X^2$ | $X^3$ | $X^4$ | $R^2$ |
|---|---|---|---|---|---|---|
| C-0751 | F | CH | CH | CH | CH | $CH_2SH$ |

(continued)

| Compound Number | $R^1$ | $X^1$ | $X^2$ | $X^3$ | $X^4$ | $R^2$ |
|---|---|---|---|---|---|---|
| C-0752 | F | CH | CH | CH | CH | $CH_2CH_2SH$ |
| C-0753 | F | CH | CH | CH | CH | $CH_2SOMe$ |
| C-0754 | F | CH | CH | CH | CH | $CH_2SO_2Me$ |
| C-0755 | F | CH | CH | CH | CH | $CH_2SOEt$ |
| C-0756 | F | CH | CH | CH | CH | $CH_2SO_2Et$ |
| C-0757 | F | CH | CH | CH | CH | $CH_2CH_2SOMe$ |
| C-0758 | F | CH | CH | CH | CH | $CH_2CH_2SO_2Me$ |
| C-0759 | F | CH | CH | CH | CH | $CH_2CH_2SOEt$ |
| C-0760 | F | CH | CH | CH | CH | $CH_2CH_2SO_2Et$ |
| C-0761 | F | CH | CH | CH | CH | $CH_2Sc$-Pr |
| C-0762 | F | CH | CH | CH | CH | $CH_2SOc$-Pr |
| C-0763 | F | CH | CH | CH | CH | $CH_2SO_2c$-Pr |
| C-0764 | F | CH | CH | CH | CH | $CH_2SCH_2c$-Pr |
| C-0765 | F | CH | CH | CH | CH | $CH_2SOCH_2c$-Pr |
| C-0766 | F | CH | CH | CH | CH | $CH_2SO_2CH_2c$-Pr |
| C-0767 | F | CH | CH | CH | CH | $CH_2CH_2Si$-Pr |
| C-0768 | F | CH | CH | CH | CH | $CH_2CH_2SOi$-Pr |
| C-0769 | F | CH | CH | CH | CH | $CH_2CH_2SO_2i$-Pr |
| C-0770 | F | CH | CH | CH | CH | $CH_2SOCF_3$ |
| C-0771 | F | CH | CH | CH | CH | $CH_2SO_2CF_3$ |
| C-0772 | F | CH | CH | CH | CH | $CH_2SOCHF_2$ |
| C-0773 | F | CH | CH | CH | CH | $CH_2SO_2CHF_2$ |
| C-0774 | F | CH | CH | CH | CH | $CH_2SCH_2Ph$ |
| C-0775 | F | CH | CH | CH | CH | $CH_2SOCH_2Ph$ |
| C-0776 | F | CH | CH | CH | CH | $CH_2SO_2CH_2Ph$ |
| C-0777 | F | CH | CH | CH | CH | $CH_2S$(pyridin-2-yl) |
| C-0778 | F | CH | CH | CH | CH | $CH_2SO$(pyridin-2-yl) |
| C-0779 | F | CH | CH | CH | CH | $CH_2SO_2$(pyridin-2-yl) |
| C-0780 | F | CH | CH | CH | CH | $CH_2SCH_2$(pyridin-2-yl) |
| C-0781 | F | CH | CH | CH | CH | $CH_2SOCH_2$(pyridin-2-yl) |
| C-0782 | F | CH | CH | CH | CH | $CH_2SO_2CH_2$(pyridin-2-yl) |
| C-0783 | F | CH | CH | CH | CH | $CH_2S$(pyridin-3-yl) |
| C-0784 | F | CH | CH | CH | CH | $CH_2SO$(pyridin-3-yl) |
| C-0785 | F | CH | CH | CH | CH | $CH_2SO_2$(pyridin-3-yl) |

(continued)

| Compound Number | $R^1$ | $X^1$ | $X^2$ | $X^3$ | $X^4$ | $R^2$ |
|---|---|---|---|---|---|---|
| C-0786 | F | CH | CH | CH | CH | $CH_2SCH_2$(pyridin-3-yl) |
| C-0787 | F | CH | CH | CH | CH | $CH_2SOCH_2$(pyridin-3-yl) |
| C-0788 | F | CH | CH | CH | CH | $CH_2SO_2$CH2(pyridin-3-yl) |
| C-0789 | F | CH | CH | CH | CH | $CH_2S$(pyridin-4-yl) |
| C-0790 | F | CH | CH | CH | CH | $CH_2SO$(pyridin-4-yl) |
| C-0791 | F | CH | CH | CH | CH | $CH_2SO_2$(pyridin-4-yl) |
| C-0792 | F | CH | CH | CH | CH | $CH_2SCH_2$(pyridin-4-yl) |

[Table 64]

| Compound Number | $R^1$ | $X^1$ | $X^2$ | $X^1$ | $X^4$ | $R^2$ |
|---|---|---|---|---|---|---|
| C-0793 | F | CH | CH | CH | CH | $CH_2SOCH_2$(pyridin-4-yl) |
| C-0794 | F | CH | CH | CH | CH | $CH_2SO_2CH_2$(pyridin-4-yl) |
| C-0795 | F | CH | CH | CH | CH | $CH_2$CH(=NOH) |
| C-0796 | F | CH | CH | CH | CH | $CH_2$CH(=NOMe) |
| C-0797 | F | CH | CH | CH | CH | $CH_2$CMe(=NOMe) |
| C-0798 | F | CH | CH | CH | CH | $CH_2$CEt(=NOMe) |
| C-0799 | F | CH | CH | CH | CH | $CH_2$Cn-Pr(=NOMe) |
| C-0800 | F | CH | CH | CH | CH | $CH_2$CMe(=NOEt) |
| C-0801 | F | CH | CH | CH | CH | $CH_2$CMe(=NO$CH_2$CH=$CH_2$) |
| C-0802 | F | CH | CH | CH | CH | $CH_2$CMe(=NOPh) |
| C-0803 | F | CH | CH | CH | CH | $CH_2$CMe(=NO$CH_2$Ph) |
| C-0804 | F | CH | CH | CH | CH | $CH_2$CMe[=NO(pyridin-2-yl)] |
| C-0805 | F | CH | CH | CH | CH | $CH_2$CMe[=NO(pyridin-3-yl)] |
| C-0806 | F | CH | CH | CH | CH | $CH_2$CMe[=NO(pyridin-4-yl)] |
| C-0807 | F | CH | CH | CH | CH | $CH_2$CMe[=NO$CH_2$(pyridin-2-yl)] |
| C-0808 | F | CH | CH | CH | CH | $CH_2$CMe[=NO$CH_2$(pyridin-3-yl)] |
| C-0809 | F | CH | CH | CH | CH | $CH_2$CMe[=NO$CH_2$(pyridin-4-yl)] |
| C-0810 | F | CH | CH | CH | CH | dihydrofuran-2(3H)-one-3-yl |
| C-0811 | F | CH | CH | CH | CH | $CH_2OCO_2Me$ |
| C-0812 | F | CH | CH | CH | CH | $CH_2OCO_2Et$ |
| C-0813 | F | CH | CH | CH | CH | $CH_2OCO_2$n-Pr |
| C-0814 | F | CH | CH | CH | CH | $CH_2OCO_2$i-Pr |
| C-0815 | F | CH | CH | CH | CH | $CH_2OCO_2$c-Pr |
| C-0816 | F | CH | CH | CH | CH | $CH_2OCO_2CH_2$c-Pr |

(continued)

| Compound Number | $R^1$ | $X^1$ | $X^2$ | $X^1$ | $X^4$ | $R^2$ |
|---|---|---|---|---|---|---|
| C-0817 | F | CH | CH | CH | CH | $CH_2OCO_2$n-Bu |
| C-0818 | F | CH | CH | CH | CH | $CH_2OCO_2$i-Bu |
| C-0819 | F | CH | CH | CH | CH | $CH_2OCO_2$t-Bu |
| C-0820 | F | CH | CH | CH | CH | $CH_2OCO_2CF_3$ |
| C-0821 | F | CH | CH | CH | CH | $CH_2OCO_2CH_2CF_3$ |
| C-0822 | F | CH | CH | CH | CH | $CH_2OCO_2CHF_2$ |
| C-0823 | F | CH | CH | CH | CH | $CH_2OCO_2CH_2CHF_2$ |
| C-0824 | F | CH | CH | CH | CH | $CH_2CH_2OCO_2Me$ |
| C-0825 | F | CH | CH | CH | CH | $CH_2CH_2OCO_2Et$ |
| C-0826 | F | CH | CH | CH | CH | $CH_2OCO_2Ph$ |
| C-0827 | F | CH | CH | CH | CH | $CH_2OCO_2CH_2Ph$ |
| C-0828 | F | CH | CH | CH | CH | $CH_2OCO_2$(pyridin-2-yl) |
| C-0829 | F | CH | CH | CH | CH | $CH_2OC_2$(pyridin-3-yl) |
| C-0830 | F | CH | CH | CH | CH | $CH_2OCO_2$(pyridin-4-yl) |
| C-0831 | F | CH | CH | CH | CH | $CH_2OCO_2CH_2$(pyridin-2-yl) |
| C-0832 | F | CH | CH | CH | CH | $CH_2OCO_2CH_2$(pyridin-3-yl) |
| C-0833 | F | CH | CH | CH | CH | $CH_2OCO_2CH_2$(pyridin-4-yl) |
| C-0834 | F | CH | CH | CH | CH | $CH_2NHCONHMe$ |

[Table 65]

| Compound Number | $R^1$ | $X^1$ | $X^2$ | $X^3$ | $X^4$ | $R^2$ |
|---|---|---|---|---|---|---|
| C-0835 | F | CH | CH | CH | CH | $CH_2NHCONHEt$ |
| C-0836 | F | CH | CH | CH | CH | $CH_2NHCONH$n-Pr |
| C-0837 | F | CH | CH | CH | CH | $CH_2NHCONH$i-Pr |
| C-0838 | F | CH | CH | CH | CH | $CH_2NHCONH$c-Pr |
| C-0839 | F | CH | CH | CH | CH | $CH_2NHCONHCH_2$c-Pr |
| C-0840 | F | CH | CH | CH | CH | $CH_2NHCONH$n-Bu |
| C-0841 | F | CH | CH | CH | CH | $CH_2NHCONH$i-Bu |
| C-0842 | F | CH | CH | CH | CH | $CH_2NHCONH$t-Bu |
| C-0843 | F | CH | CH | CH | CH | $CH_2NHCONHCF_3$ |
| C-0844 | F | CH | CH | CH | CH | $CH_2NHCONHCH_2CF_3$ |
| C-0845 | F | CH | CH | CH | CH | $CH_2NHCONHCHF_2$ |
| C-0846 | F | CH | CH | CH | CH | $CH_2NHCONHCH_2CHF_2$ |
| C-0847 | F | CH | CH | CH | CH | $CH_2CH_2NHCONHMe$ |

(continued)

| Compound Number | $R^1$ | $X^1$ | $X^2$ | $X^3$ | $X^4$ | $R^2$ |
|---|---|---|---|---|---|---|
| C-0848 | F | CH | CH | CH | CH | $CH_2CH_2NHCONHEt$ |
| C-0849 | F | CH | CH | CH | CH | $CH_2NHCONHPh$ |
| C-0850 | F | CH | CH | CH | CH | $CH_2NHCONHCH_2Ph$ |
| C-0851 | F | CH | CH | CH | CH | $CH_2NHCONH$(pyridin-2-yl) |
| C-0852 | F | CH | CH | CH | CH | $CH_2NHCONH$(pyridin-3-yl) |
| C-0853 | F | CH | CH | CH | CH | $CH_2NHCONH$(pyridin-4-yl) |
| C-0854 | F | CH | CH | CH | CH | $CH_2NHCONH_2CH_2$(pyridin-2-yl) |
| C-0855 | F | CH | CH | CH | CH | $CH_2NHCONHCH_2$(pyridin-3-yl) |
| C-0856 | F | CH | CH | CH | CH | $CH_2NHCONHCH_2$(pyridin-4-yl) |
| C-0857 | F | CH | CH | CH | CH | $CH_2CONHNH_2$ |
| C-0858 | F | CH | CH | CH | CH | $CH_2CONHNMe_2$ |
| C-0859 | F | CH | CH | CH | CH | $CH_2CONHNHCO_2$t-Bu |
| C-0860 | F | CH | CH | CH | CH | $CH_2CHCCl_2$ |
| C-0861 | F | CH | CH | CH | CH | $CH_2CO_2CH_2CH_2$(morpholin-4-yl) |
| C-0862 | F | CH | CH | CH | CH | $CH_2CO_2CH_2CH_2OMe$ |
| C-0863 | F | CMe | CH | CH | CH | $CH_2CO_2H$ |
| C-0864 | F | CMe | CH | CH | CH | $CH_2CO_2Et$ |
| C-0865 | F | CMe | CH | CH | CH | $CH_2CO_2iPr$ |
| C-0866 | F | CMe | CH | CH | CH | $CH_2CO_2cPr$ |
| C-0867 | F | CMe | CH | CH | CH | $CH_2CO_2tBu$ |
| C-0868 | F | CF | CH | CH | CH | $CH_2CO_2Et$ |
| C-0869 | F | N | CH | CH | CH | $CH_2CO_2Et$ |
| C-0870 | F | CH | CH | CH | CH | $CH_2CH_2NHCO_2tBu$ |
| C-0871 | F | CH | CH | CH | CH | $CH_2CH_2N(COCF_3)CO_2tBu$ |
| C-0872 | F | CH | CH | CH | CH | $CH_2CH_2NMe_2$ |
| C-0873 | F | CH | CH | CH | CH | $CH_2CONHn$-Pen |
| C-0874 | F | CH | CH | CH | CH | $CH_2CONHn$-Hex |
| C-0875 | F | CH | CH | CH | CH | $CH_2CONHCH_2CH_2OMe$ |
| C-0876 | F | CH | CH | CH | CH | $CH_2CH(Cl)CH_2SF_5$ |

[Table 66]

| Compound Number | $R^1$ | $X^1$ | $X^2$ | $X^3$ | $X^4$ | $R^2$ |
|---|---|---|---|---|---|---|
| C-0877 | F | CH | CH | CH | CH | $CH_2CH=CHSF_5$ |
| C-0878 | F | CH | CH | CH | CH | $CH_2CO_2$(tetrahydrofuran-3-yl) |

(continued)

| Compound Number | $R^1$ | $X^1$ | $X^2$ | $X^3$ | $X^4$ | $R^2$ |
|---|---|---|---|---|---|---|
| C-0879 | F | CH | CH | CH | CH | 1-($CO_2H$)-c-Pr |
| C-0880 | F | CH | CH | CH | CH | 1-($CO_2H$)-c-Bu |
| C-0881 | F | CH | CH | CH | CH | 1-($CO_2Me$)-c-Pr |
| C-0882 | F | CH | CH | CH | CH | 1-($CO_2Me$)-c-Bu |
| C-0883 | F | CH | CH | CH | CH | 1-($CO_2Et$)-c-Pr |
| C-0884 | F | CH | CH | CH | CH | 1-($CO_2Et$)-c-Bu |
| C-0885 | F | CH | CH | CH | CH | 1-($CO_2nPr$)-c-Pr |
| C-0886 | F | CH | CH | CH | CH | 1-($CO_2nPr$)-c-Bu |
| C-0887 | F | CH | CH | CH | CH | 1-($CO_2iPr$)-c-Pr |
| C-0888 | F | CH | CH | CH | CH | 1-($CO_2iPr$)-c-Bu |
| C-0889 | F | CH | CH | CH | CH | 1-($CO_2cPr$)-c-Pr |
| C-0890 | F | CH | CH | CH | CH | 1-($CO_2cPr$)-c-Bu |
| C-0891 | F | CH | CH | CH | CH | 1-($CO_2cBu$)-c-Pr |
| C-0892 | F | CH | CH | CH | CH | 1-($CO_2cBu$)-c-Bu |
| C-0893 | F | CF | CH | CH | CH | $CH_2CO_2H$ |
| C-0894 | F | CH | CF | CH | CH | $CH_2CO_2H$ |
| C-0895 | F | CH | CMe | CH | CH | $CH_2CO_2H$ |
| C-0896 | Cl | CH | CH | CH | CH | $CH_2CO_2H$ |
| C-0897 | F | N | CH | CH | CH | $CH_2CO_2H$ |
| C-0898 | F | CH | N | CH | CH | $CH_2CO_2H$ |
| C-0899 | F | N | N | CH | CH | $CH_2CO_2H$ |
| C-0900 | F | N | CH | N | CH | $CH_2CO_2H$ |
| C-0901 | F | N | CH | CH | N | $CH_2CO_2H$ |
| C-0902 | F | CH | N | CH | N | $CH_2CO_2H$ |
| C-0903 | H | CH | CH | CH | CH | $CH_2CO_2H$ |
| C-0904 | F | CF | CH | CH | CH | $CH_2CO_2Me$ |
| C-0905 | F | CH | CF | CH | CH | $CH_2CO_2Me$ |
| C-0906 | F | CMe | CH | CH | CH | $CH_2CO_2Me$ |
| C-0907 | F | CH | CMe | CH | CH | $CH_2CO_2Me$ |
| C-0908 | Cl | CH | CH | CH | CH | $CH_2CO_2Me$ |
| C-0909 | F | N | CH | CH | CH | $CH_2CO_2Me$ |
| C-0910 | F | CH | N | CH | CH | $CH_2CO_2Me$ |
| C-0911 | F | N | N | CH | CH | $CH_2CO_2Me$ |
| C-0912 | F | N | CH | N | CH | $CH_2CO_2Me$ |

(continued)

| Compound Number | $R^1$ | $X^1$ | $X^2$ | $X^3$ | $X^4$ | $R^2$ |
|---|---|---|---|---|---|---|
| C-0913 | F | N | CH | CH | N | $CH_2CO_2Me$ |
| C-0914 | F | CH | N | CH | N | $CH_2CO_2Me$ |
| C-0915 | H | CH | CH | CH | CH | $CH_2CO_2Me$ |
| C-0916 | F | CH | CF | CH | CH | $CH_2CO_2Et$ |
| C-0917 | F | CH | CMe | CH | CH | $CH_2CO_2Et$ |
| C-0918 | Cl | CH | CH | CH | CH | $CH_2CO_2Et$ |

[Table 67]

| Compound Number | $R^1$ | $X^1$ | $X^2$ | $X^3$ | $X^4$ | $R^2$ |
|---|---|---|---|---|---|---|
| C-0919 | F | CH | N | CH | CH | $CH_2CO_2Et$ |
| C-0920 | F | N | N | CH | CH | $CH_2CO_2Et$ |
| C-0921 | F | N | CH | N | CH | $CH_2CO_2Et$ |
| C-0922 | F | N | CH | CH | N | $CH_2CO_2Et$ |
| C-0923 | F | CH | N | CH | N | $CH_2CO_2Et$ |
| C-0924 | H | CH | CH | CH | CH | $CH_2CO_2Et$ |
| C-0925 | F | CF | CH | CH | CH | $CH_2CO_2nPr$ |
| C-0926 | F | CH | CF | CH | CH | $CH_2CO_2nPr$ |
| C-0927 | F | CMe | CH | CH | CH | $CH_2CO_2nPr$ |
| C-0928 | F | CH | CMe | CH | CH | $CH_2CO_2nPr$ |
| C-0929 | Cl | CH | CH | CH | CH | $CH_2CO_2nPr$ |
| C-0930 | F | N | CH | CH | CH | $CH_2CO_2nPr$ |
| C-0931 | F | CH | N | CH | CH | $CH_2CO_2nPr$ |
| C-0932 | F | N | N | CH | CH | $CH_2CO_2nPr$ |
| C-0933 | F | N | CH | N | CH | $CH_2CO_2nPr$ |
| C-0934 | F | N | CH | CH | N | $CH_2CO_2nPr$ |
| C-0935 | F | CH | N | CH | N | $CH_2CO_2nPr$ |
| C-0936 | H | CH | CH | CH | CH | $CH_2CO_2nPr$ |
| C-0937 | F | CF | CH | CH | CH | $CH_2CO_2iPr$ |
| C-0938 | F | CH | CF | CH | CH | $CH_2CO_2iPr$ |
| C-0939 | F | CH | CMe | CH | CH | $CH_2CO_2iPr$ |
| C-0940 | Cl | CH | CH | CH | CH | $CH_2CO_2iPr$ |
| C-0941 | F | N | CH | CH | CH | $CH_2CO_2iPr$ |
| C-0942 | F | CH | N | CH | CH | $CH_2CO_2iPr$ |
| C-0943 | F | N | N | CH | CH | $CH_2CO_2iPr$ |

(continued)

| Compound Number | $R^1$ | $X^1$ | $X^2$ | $X^3$ | $X^4$ | $R^2$ |
|---|---|---|---|---|---|---|
| C-0944 | F | N | CH | N | CH | $CH_2CO_2iPr$ |
| C-0945 | F | N | CH | CH | N | $CH_2CO_2iPr$ |
| C-0946 | F | CH | N | CH | N | $CH_2CO_2iPr$ |
| C-0947 | H | CH | CH | CH | CH | $CH_2CO_2iPr$ |
| C-0948 | F | CF | CH | CH | CH | $CH_2CO_2cPr$ |
| C-0949 | F | CH | CF | CH | CH | $CH_2CO_2cPr$ |
| C-0950 | F | CH | CMe | CH | CH | $CH_2CO_2cPr$ |
| C-0951 | Cl | CH | CH | CH | CH | $CH_2CO_2cPr$ |
| C-0952 | F | N | CH | CH | CH | $CH_2CO_2cPr$ |
| C-0953 | F | CH | N | CH | CH | $CH_2CO_2cPr$ |
| C-0954 | F | N | N | CH | CH | $CH_2CO_2cPr$ |
| C-0955 | F | N | CH | N | CH | $CH_2CO_2cPr$ |
| C-0956 | F | N | CH | CH | N | $CH_2CO_2cPr$ |
| C-0957 | F | CH | N | CH | N | $CH_2CO_2cPr$ |
| C-0958 | H | CH | CH | CH | CH | $CH_2CO_2cPr$ |

[Table 68]

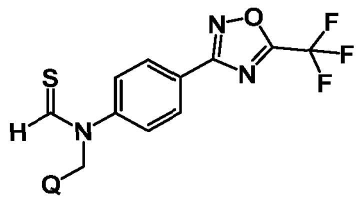

| Compound Number | Q |
|---|---|
| D-0001 | naphthalen-1-yl |
| D-0002 | naphthalen-2-yl |
| D-0003 | quinolin-2-yl |
| D-0004 | quinolin-3-yl |
| D-0005 | quinolin-6-yl |
| D-0006 | quinolin-7-yl |
| D-0007 | 1H-pyrrolo[2,3-b]pyridin-3-yl |
| D-0008 | benzo[d][1,3]dioxol-4-yl |
| D-0009 | benzo[d][1,3]dioxol-5-yl |
| D-0010 | benzo[d][1,3]dioxol-4-yl(2,2-$F_2$) |
| D-0011 | benzo[d][1,3]dioxol-5-yl(2,2-$F_2$) |
| D-0012 | pyridin-2-yl |

(continued)

| 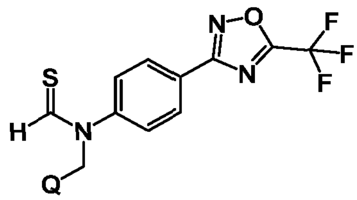 | |
|---|---|
| Compound Number | Q |
| D-0013 | $CH_2$pyridin-2-yl |
| D-0014 | pyridi n-2-yl(3-F) |
| D-0015 | pyridi n-2-yl(4-F) |
| D-0016 | pyridi n-2-yl(5-F) |
| D-0017 | pyridi n-2-yl(6-F) |
| D-0018 | pyridin-2-yl(3-Cl) |
| D-0019 | pyridin-2-yl(4-Cl) |
| D-0020 | pyridin-2-yl(5-Cl) |
| D-0021 | pyridin-2-yl(6-Cl) |
| D-0022 | pyridin-2-yl(3-Me) |
| D-0023 | pyridin-2-yl(4-Me) |
| D-0024 | pyridin-2-yl(5-Me) |
| D-0025 | pyridin-2-yl(6-Me) |
| D-0026 | pyridin-2-yl(3-OMe) |
| D-0027 | pyridin-2-yl(4-OMe) |
| D-0028 | pyridin-2-yl(5-OMe) |
| D-0029 | pyridin-2-yl(6-OMe) |
| D-0030 | pyridin-2-yl(3-CN) |
| D-0031 | pyridin-2-yl(4-CN) |
| D-0032 | pyridin-2-yl(5-CN) |
| D-0033 | pyridin-2-yl(6-CN) |
| D-0034 | pyridin-2-yl(3-$CF_3$) |
| D-0035 | pyridin-2-yl(4-$CF_3$) |
| D-0036 | pyridin-2-yl(5-$CF_3$) |

[Table 69]

| Compound Number | Q |
|---|---|
| D-0037 | pyridin-2-yl(6-C $F_3$) |
| D-0038 | pyridin-2-yl(3-$CO_2$Et) |
| D-0039 | pyridin-2-yl(4-$CO_2$Et) |
| D-0040 | pyridin-2-yl(5-$CO_2$Et) |

(continued)

| Compound Number | Q |
|---|---|
| D-0041 | pyridin-2-yl(6-$CO_2$Et) |
| D-0042 | pyridin-3-yl |
| D-0043 | $CH_2$pyridin-3-yl |
| D-0044 | pyridin-3-yl(2-F) |
| D-0045 | pyridin-3-yl(4-F) |
| D-0046 | pyridin-3-yl(5-F) |
| D-0047 | pyridin-3-yl(6-F) |
| D-0048 | pyridin-3-yl(2-Cl) |
| D-0049 | pyridin-3-yl(4-Cl) |
| D-0050 | pyridin-3-yl(5-Cl) |
| D-0051 | pyridin-3-yl(6-Cl) |
| D-0052 | pyridin-3-yl(2-Me) |
| D-0053 | pyridin-3-yl(4-Me) |
| D-0054 | pyridin-3-yl(5-Me) |
| D-0055 | pyridin-3-yl(6-Me) |
| D-0056 | pyridin-3-yl(2-OMe) |
| D-0057 | pyridin-3-yl(4-OMe) |
| D-0058 | pyridin-3-yl(5-OMe) |
| D-0059 | pyridin-3-yl(6-OMe) |
| D-0060 | pyridin-3-yl(2-CN) |
| D-0061 | pyridin-3-yl(4-CN) |
| D-0062 | pyridin-3-yl(5-CN) |
| D-0063 | pyridin-3-yl(6-CN) |
| D-0064 | pyridin-3-yl(2-$CF_3$) |
| D-0065 | pyridin-3-yl(4-$CF_3$) |
| D-0066 | pyridin-3-yl(5-$CF_3$) |
| D-0067 | pyridin-3-yl(6-$CF_3$) |
| D-0068 | pyridin-3-yl(2-$CO_2$Et) |
| D-0069 | pyridin-3-yl(4-$CO_2$Et) |
| D-0070 | pyridin-3-yl(5-$CO_2$Et) |
| D-0071 | pyridin-3-yl(6-$CO_2$Et) |
| D-0072 | pyridin-4-yl |
| D-0073 | $CH_2$pyridin-4-yl |
| D-0074 | pyridin-4-yl(2-F) |
| D-0075 | pyridin-4-yl(3-F) |
| D-0076 | pyridin-4-yl(2-Cl) |

(continued)

| Compound Number | Q |
|---|---|
| D-0077 | pyridin-4-yl(3-Cl) |
| D-0078 | pyridin-4-yl(2-Me) |

[Table 70]

| Compound Number | Q |
|---|---|
| D-0079 | pyridin-4-yl(3-Me) |
| D-0080 | pyridin-4-yl(2-OMe) |
| D-0081 | pyridin-4-yl(3-OMe) |
| D-0082 | pyridin-4-yl(2-CN) |
| D-0083 | pyridin-4-yl(3-CN) |
| D-0084 | pyridin-4-yl(2-C $F_3$) |
| D-0085 | pyridin-4-yl(3-C $F_3$) |
| D-0086 | pyridin-4-yl(2-$CO_2$Et) |
| D-0087 | pyridin-4-yl(3-$CO_2$Et) |
| D-0088 | pyrimidin-2-yl |
| D-0089 | $CH_2$pyrimidin-2-yl |
| D-0090 | pyrimidin-2-yl(4-F) |
| D-0091 | pyrimidin-2-yl(5-F) |
| D-0092 | pyrimidin-2-yl(4-Cl) |
| D-0093 | pyrimidin-2-yl(5-Cl) |
| D-0094 | pyrimidin-2-yl(4-Me) |
| D-0095 | pyrimidin-2-yl(5-Me) |
| D-0096 | pyrimidin-2-yl(4-OMe) |
| D-0097 | pyrimidin-2-yl(5-OMe) |
| D-0098 | pyrimidin-2-yl(4-CN) |
| D-0099 | pyrimidin-2-yl(5-CN) |
| D-0100 | pyrimidin-2-yl(4-$CF_3$) |
| D-0101 | pyrimidin-2-yl(5-$CF_3$) |
| 0-0102 | pyrimidin-2-yl(4-$CO_2$Et) |
| D-0103 | pyrimidin-2-yl(5-$CO_2$Et) |
| D-0104 | pyrimidin-4-yl |
| D-0105 | $CH_2$pyrimidin-4-yl |
| D-0106 | pyrimidin-4-yl(2-F) |
| D-0107 | pyrimidin-4-yl(5-F) |
| D-0108 | pyrimidin-4-yl(6-F) |
| D-0109 | pyrimidin-4-yl(2-Cl) |

(continued)

| Compound Number | Q |
|---|---|
| D-0110 | pyrimidin-4-yl(5-Cl) |
| D-0111 | pyrimidin-4-yl(6-Cl) |
| D-0112 | pyrimidin-4-yl(2-Me) |
| D-0113 | pyrimidin-4-yl(5-Me) |
| D-0114 | pyrimidin-4-yl(6-Me) |
| D-0115 | pyrimidin-4-yl(2-OMe) |
| D-0116 | pyrimidin-4-yl(5-OMe) |
| D-0117 | pyrimidin-4-yl(6-OMe) |
| D-0118 | pyrimidin-4-yl(2-CN) |
| D-0119 | pyrimidin-4-yl(5-CN) |
| D-0120 | pyrimidin-4-yl(6-CN) |

[Table 71]

| Compound Number | Q |
|---|---|
| D-0121 | pyrimidin-4-yl(2-$CF_3$) |
| D-0122 | pyrimidin-4-yl(5-$CF_3$) |
| D-0123 | pyrimidin-4-yl(6-$CF_3$) |
| D-0124 | pyrimidin-4-yl(2-$CO_2Et$) |
| D-0125 | pyrimidin-4-yl(5-$CO_2Et$) |
| D-0126 | pyrimidin-4-yl(6-$CO_2Et$) |
| D-0127 | pyrimidin-5-yl |
| 0-0128 | $CH_2$pyrimidin-5-yl |
| D-0129 | pyrimidin-5-yl(2-F) |
| D-0130 | pyrimidin-5-yl(4-F) |
| D-0131 | pyrimidin-5-yl(2-Cl) |
| D-0132 | pyrimidin-5-yl(4-Cl) |
| D-0133 | pyrimidin-5-yl(2-Me) |
| D-0134 | pyrimidin-5-yl(4-Me) |
| D-0135 | pyri midi n-5-yl(2-OMe) |
| D-0136 | pyrimidin-5-yl(4-OMe) |
| D-0137 | pyrimidin-5-yl(2-CN) |
| D-0138 | pyrimidin-5-yl(4-CN) |
| D-0139 | pyrimidin-5-yl(2-$CF_3$) |
| D-0140 | pyrimidin-5-yl(4-$CF_3$) |
| D-0141 | pyrimidin-5-yl(2-$CO_2Et$) |
| D-0142 | pyrimidin-5-yl(4-$CO_2Et$) |

(continued)

| Compound Number | Q |
|---|---|
| D-0143 | pyridazin-3-yl |
| D-0144 | $CH_2$pyridazin-3-yl |
| D-0145 | pyridazin-3-yl(4-F) |
| D-0146 | pyridazin-3-yl(5-F) |
| D-0147 | pyridazin-3-yl(6-F) |
| D-0148 | pyridazin-3-yl(4-Cl) |
| D-0149 | pyridazin-3-yl(5-Cl) |
| D-0150 | pyridazin-3-yl(6-Cl) |
| D-0151 | pyridazin-3-yl(4-Me) |
| D-0152 | pyridazin-3-yl(5-Me) |
| D-0153 | pyridazin-3-yl(6-Me) |
| D-0154 | pyridazin-3-yl(4-OMe) |
| D-0155 | pyridazin-3-yl(5-OMe) |
| D-0156 | pyridazin-3-yl(6-OMe) |
| D-0157 | pyridazin-3-yl(4-CN) |
| D-0158 | pyridazin-3-yl(5-CN) |
| D-0159 | pyridazin-3-yl(6-CN) |
| D-0160 | pyridazin-3-yl(4-$CF_3$) |
| D-0161 | pyridazin-3-yl(5-$CF_3$) |
| D-0162 | pyridazin-3-yl(6-$CF_3$) |

[Table 72]

| Compound Number | Q |
|---|---|
| D-0163 | pyridazin-3-yl(4-$CO_2$Et) |
| D-0164 | pyridazin-3-yl(5-$CO_2$Et) |
| D-0165 | pyridazin-3-yl(6-$CO_2$Et) |
| D-0166 | pyridazin-4-yl |
| D-0167 | $CH_2$pyridazin-4-yl |
| D-0168 | pyridazin-4-yl(3-F) |
| D-0169 | pyridazin-4-yl(5-F) |
| D-0170 | pyridazin-4-yl(6-F) |
| D-0171 | pyridazin-4-yl(3-Cl) |
| D-0172 | pyridazin-4-yl(5-Cl) |
| D-0173 | pyridazin-4-yl(6-Cl) |
| D-0174 | pyridazin-4-yl(3-Me) |
| D-0175 | pyridazin-4-yl(5-Me) |
| D-0176 | pyridazin-4-yl(6-Me) |

(continued)

| Compound Number | Q |
|---|---|
| D-0177 | pyridazin-4-yl(3-OMe) |
| D-0178 | pyridazin-4-yl(5-OMe) |
| D-0179 | pyridazin-4-yl(6-OMe) |
| D-0180 | pyridazin-4-yl(3-CN) |
| D-0181 | pyridazin-4-yl(5-CN) |
| D-0182 | pyridazin-4-yl(6-CN) |
| D-0183 | pyridazin-4-yl(3-$CF_3$) |
| D-0184 | pyridazin-4-yl(5-$CF_3$) |
| D-0185 | pyridazin-4-yl(6-$CF_3$) |
| D-0186 | pyridazin-4-yl(3-$CO_2$Et) |
| D-0187 | pyridazin-4-yl(5-$CO_2$Et) |
| D-0188 | pyridazin-4-yl(6-$CO_2$Et) |
| D-0189 | pyrazin-2-yl |
| D-0190 | $CH_2$pyrazin-2-yl |
| D-0191 | pyrazi n-2-yl(3-F) |
| D-0192 | pyrazi n-2-yl(5-F) |
| D-0193 | pyrazi n-2-yl(6-F) |
| D-0194 | pyrazin-2-yl(3-Cl) |
| D-0195 | pyrazin-2-yl(5-Cl) |
| D-0196 | pyrazin-2-yl(6-Cl) |
| D-0197 | pyrazin-2-yl(3-Me) |
| D-0198 | pyrazin-2-yl(5-Me) |
| D-0199 | pyrazin-2-yl(6-Me) |
| D-0200 | pyrazi n-2-yl(3-O Me) |
| D-0201 | pyrazi n-2-yl(5-O Me) |
| D-0202 | pyrazin-2-yl(6-O Me) |
| D-0203 | pyrazin-2-yl(3-CN) |
| D-0204 | pyrazin-2-yl(5-CN) |

[Table 73]

| Compound Number | Q |
|---|---|
| D-0205 | pyrazin-2-yl(6-CN) |
| D-0206 | pyrazin-2-yl(3-$CF_3$) |
| D-0207 | pyrazin-2-yl(5-$CF_3$) |
| D-0208 | pyrazin-2-yl(6-$CF_3$) |
| D-0209 | pyrazin-2-yl(3-$CO_2$Et) |

(continued)

| Compound Number | Q |
|---|---|
| D-0210 | pyrazin-2-yl(5-$CO_2$Et) |
| D-0211 | pyrazin-2-yl(6-$CO_2$Et) |
| D-0212 | 1,3,5-triazin-2-yl |
| D-0213 | $CH_2$1,3,5-triazin-2-yl |
| D-0214 | thiophen-2-yl |
| D-0215 | $CH_2$thiophen-2-yl |
| D-0216 | thiophen-2-yl(3-F) |
| D-0217 | thiophen-2-yl(4-F) |
| D-0218 | thiophen-2-yl(5-F) |
| D-0219 | thiophen-2-yl(3-Cl) |
| D-0220 | thiophen-2-yl(4-Cl) |
| D-0221 | thiophen-2-yl(5-Cl) |
| D-0222 | thiophen-2-yl(3-Me) |
| D-0223 | thiophen-2-yl(4-Me) |
| D-0224 | thiophen-2-yl(5-Me) |
| D-0225 | thiophen-2-yl(3-OMe) |
| D-0226 | thiophen-2-yl(4-OMe) |
| D-0227 | thiophen-2-yl(5-OMe) |
| D-0228 | thiophen-2-yl(3-CN) |
| D-0229 | thiophen-2-yl(4-CN) |
| D-0230 | thiophen-2-yl(5-CN) |
| D-0231 | thiophen-2-yl(3-$CF_3$) |
| D-0232 | thiophen-2-yl(4-$CF_3$) |
| D-0233 | thiophen-2-yl(5-$CF_3$) |
| D-0234 | thiophen-2-yl(3-$CO_2$Et) |
| D-0235 | thiophen-2-yl(4-$CO_2$Et) |
| D-0236 | thiophen-2-yl(5-$CO_2$Et) |
| D-0237 | thiophen-3-yl |
| D-0238 | $CH_2$thiophen-3-yl |
| D-0239 | thiophen-3-yl(2-F) |
| D-0240 | thiophen-3-yl(4-F) |
| D-0241 | thiophen-3-yl(5-F) |
| D-0242 | thiophen-3-yl(2-Cl) |
| D-0243 | thiophen-3-yl(4-Cl) |
| D-0244 | thiophen-3-yl(5-Cl) |
| D-0245 | thiophen-3-yl(2-Me) |

(continued)

| Compound Number | Q |
|---|---|
| D-0246 | thiophen-3-yl(4-Me) |

[Table 74]

| Compound Number | Q |
|---|---|
| D-0247 | thiophen-3-yl(5-Me) |
| D-0248 | thiophen-3-yl(2-OMe) |
| D-0249 | thiophen-3-yl(4-OMe) |
| D-0250 | thiophen-3-yl(5-OMe) |
| D-0251 | thiophen-3-yl(2-CN) |
| D-0252 | thiophen-3-yl(4-CN) |
| D-0253 | thiophen-3-yl(5-CN) |
| D-0254 | thiophen-3-yl(2-$CF_3$) |
| D-0255 | thiophen-3-yl(4-$CF_3$) |
| D-0256 | thiophen-3-yl(5-$CF_3$) |
| D-0257 | thiophen-3-yl(2-$CO_2Et$) |
| D-0258 | thiophen-3-yl(4-$CO_2Et$) |
| D-0259 | thiophen-3-yl(5-$CO_2Et$) |
| D-0260 | furan-2-yl |
| D-0261 | $CH_2$furan-2-yl |
| D-0262 | furan-2-yl(3-F) |
| D-0263 | furan-2-yl(4-F) |
| D-0264 | furan-2-yl(5-F) |
| D-0265 | furan-2-yl(3-Cl) |
| D-0266 | furan-2-yl(4-Cl) |
| D-0267 | furan-2-yl(5-Cl) |
| D-0268 | furan-2-yl(3-Me) |
| D-0269 | furan-2-yl(4-Me) |
| D-0270 | furan-2-yl(5-Me) |
| D-0271 | furan-2-yl(3-OMe) |
| D-0272 | furan-2-yl(4-OMe) |
| D-0273 | furan-2-yl(5-OMe) |
| D-0274 | furan-2-yl(3-C N) |
| D-0275 | furan-2-yl(4-C N) |
| D-0276 | furan-2-yl(5-C N) |
| D-0277 | furan-2-yl(3-$CF_3$) |
| D-0278 | furan-2-yl(4-$CF_3$) |

(continued)

| Compound Number | Q |
|---|---|
| D-0279 | furan-2-yl(5-$CF_3$) |
| D-0280 | furan-2-yl(3-$CO_2$Et) |
| D-0281 | furan-2-yl(4-$CO_2$Et) |
| D-0282 | furan-2-yl(5-$CO_2$Et) |
| D-0283 | furan-3-yl |
| D-0284 | $CH_2$furan-3-yl |
| D-0285 | furan-3-yl(2-F) |
| D-0286 | furan-3-yl(4-F) |
| D-0287 | furan-3-yl(5-F) |
| D-0288 | furan-3-yl(2-Cl) |

[Table 75]

| Compound Number | Q |
|---|---|
| D-0289 | furan-3-yl(4-Cl) |
| D-0290 | furan-3-yl(5-Cl) |
| D-0291 | furan-3-yl(2-Me) |
| D-0292 | furan-3-yl(4-Me) |
| D-0293 | furan-3-yl(5-Me) |
| D-0294 | furan-3-yl(2-OMe) |
| D-0295 | furan-3-yl(4-OMe) |
| D-0296 | furan-3-yl(5-OMe) |
| D-0297 | furan-3-yl(2-C N) |
| D-0298 | furan-3-yl(4-C N) |
| D-0299 | furan-3-yl(5-C N) |
| D-0300 | furan-3-yl(2-$CF_3$) |
| D-0301 | furan-3-yl(4-$CF_3$) |
| D-0302 | furan-3-yl(5-$CF_3$) |
| D-0303 | furan-3-yl(2-$CO_2$Et) |
| D-0304 | furan-3-yl(4-$CO_2$Et) |
| D-0305 | furan-3-yl(5-$CO_2$Et) |
| D-0306 | 1H-pyrrol-1-yl |
| D-0307 | $CH_2$1H-pyrrol-1-yl |
| D-0308 | 1H-pyrrol-2-yl |
| D-0309 | $CH_2$1H-pyrrol-2-yl |
| D-0310 | 1H-pyrrol-2-yl(1-Me) |
| D-0311 | $CH_2$1H-pyrrol-2-yl(1-Me) |

(continued)

| Compound Number | Q |
|---|---|
| D-0312 | 1H-pyrrol-3-yl |
| D-0313 | $CH_2$1H-pyrrol-3-yl |
| D-0314 | 1H-pyrrol-3-yl(1-Me) |
| D-0315 | $CH_2$1H-pyrrol-3-yl(1-Me) |
| D-0316 | 1H-pyrazol-1-yl |
| D-0317 | $CH_2$1H-pyrazol-1-yl |
| D-0318 | 1H-pyrazol-1-yl(3-F) |
| D-0319 | 1H-pyrazol-1-yl(4-F) |
| D-0320 | 1H-pyrazol-1-yl(5-F) |
| D-0321 | 1H-pyrazol-1-yl(3-Cl) |
| D-0322 | 1H-pyrazol-1-yl(4-Cl) |
| D-0323 | 1H-pyrazol-1-yl(5-Cl) |
| D-0324 | 1H-pyrazol-1-yl(3-Me) |
| D-0325 | 1H-pyrazol-1 -yl(4-Me) |
| D-0326 | 1H-pyrazol-1-yl(5-Me) |
| D-0327 | 1H-pyrazol-1-yl(3-OMe) |
| D-0328 | 1H-pyrazol-1-yl(4-OMe) |
| D-0329 | 1H-pyrazol-1-yl(5-OMe) |
| D-0330 | 1H-pyrazol-1-yl(3-CN) |

[Table 76]

| Compound Number | Q |
|---|---|
| D-0331 | 1H-pyrazol-1 -yl(4-C N) |
| D-0332 | 1H-pyrazol-1-yl(5-CN) |
| D-0333 | 1H-pyrazol-1-yl(3-$CF_3$) |
| D-0334 | 1H-pyrazol-1-yl(4-$CF_3$) |
| D-0335 | 1H-pyrazol-1-yl(5-$CF_3$) |
| D-0336 | 1H-pyrazol-1-yl(3-$CO_2$Et) |
| D-0337 | 1H-pyrazol-1-yl(4-$CO_2$Et) |
| D-0338 | 1H-pyrazol-1-yl(5-$CO_2$Et) |
| D-0339 | 1H-pyrazol-3-yl |
| D-0340 | $CH_2$1H-pyrazol-3-yl |
| D-0341 | 1H-pyrazol-3-yl(1-Me) |
| D-0342 | $CH_2$1H-pyrazol-3-yl(1-Me) |
| D-0343 | 1H-pyrazol-3-yl(4-F,1-Me) |
| D-0344 | 1H-pyrazol-3-yl(5-F,1-Me) |

(continued)

| Compound Number | Q |
|---|---|
| D-0345 | 1H-pyrazol-3-yl(4-Cl,1-Me) |
| D-0346 | 1H-pyrazol-3-yl(5-Cl,1-Me) |
| D-0347 | 1H-pyrazol-3-yl(1,4-$Me_2$) |
| D-0348 | 1H-pyrazol-3-yl(1,5-$Me_2$) |
| D-0349 | 1H-pyrazol-3-yl(4-OMe,1-Me) |
| D-0350 | 1H-pyrazol-3-yl(5-OMe,1-Me) |
| D-0351 | 1H-pyrazol-3-yl(4-CN,1-Me) |
| D-0352 | 1H-pyrazol-3-yl(5-CN,1-Me) |
| D-0353 | 1H-pyrazol-3-yl(1-Me,4-$CF_3$) |
| D-0354 | 1H-pyrazol-3-yl(1-Me,5-$CF_3$) |
| D-0355 | 1H-pyrazol-3-yl(1-Me,4-$CO_2$Et) |
| D-0356 | 1H-pyrazol-3-yl(1-Me,5-$CO_2$Et) |
| D-0357 | 1H-pyrazol-4-yl |
| D-0358 | $CH_2$1H-pyrazol-4-yl |
| D-0359 | 1H-pyrazol-4-yl(1-Me) |
| D-0360 | $CH_2$1H-pyrazol-4-yl(1-Me) |
| D-0361 | 1H-pyrazol-4-yl(3-F,1-Me) |
| D-0362 | 1H-pyrazol-4-yl(5-F,1-Me) |
| D-0363 | 1H-pyrazol-4-yl(3-Cl,1-Me) |
| D-0364 | 1H-pyrazol-4-yl(5-Cl,1-Me) |
| D-0365 | 1H-pyrazol-4-yl(1,3-$Me_2$) |
| D-0366 | 1H-pyrazol-4-yl(1,5-$Me_2$) |
| D-0367 | 1H-pyrazol-4-yl(3-OMe,1-Me) |
| D-0368 | 1H-pyrazol-4-yl(5-OMe,1-Me) |
| D-0369 | 1H-pyrazol-4-yl(3-CN,1-Me) |
| D-0370 | 1H-pyrazol-4-yl(5-CN,1-Me) |
| D-0371 | 1H-pyrazol-4-yl(1-Me,3-$CF_3$) |
| D-0372 | 1H-pyrazol-4-yl(1-Me,5-$CF_3$) |

[Table 77]

| Compound Number | Q |
|---|---|
| D-0373 | 1H-pyrazol-4-yl(1-Me,3-$CO_2$Et) |
| D-0374 | 1H-pyrazol-4-yl(1-Me,5-$CO_2$Et) |
| D-0375 | 1H-pyrazol-5-yl |
| D-0376 | $CH_2$1H-pyrazol-5-yl |
| D-0377 | 1H-pyrazol-5-yl(1-Me) |

(continued)

| Compound Number | Q |
|---|---|
| D-0378 | $CH_2$1H-pyrazol-5-yl(1-Me) |
| D-0379 | 1H-pyrazol-5-yl(3-F,1-Me) |
| D-0380 | 1H-pyrazol-5-yl(4-F,1-Me) |
| D-0381 | 1H-pyrazol-5-yl(3-Cl,1-Me) |
| D-0382 | 1H-pyrazol-5-yl(4-Cl,1-Me) |
| D-0383 | 1H-pyrazol-5-yl(1,3-$Me_2$) |
| D-0384 | 1H-pyrazol-5-yl(1,4-$Me_2$) |
| D-0385 | 1H-pyrazol-5-yl(3-OMe,1-Me) |
| D-0386 | 1H-pyrazol-5-yl(4-OMe,1-Me) |
| D-0387 | 1H-pyrazol-5-yl(3-CN,1-Me) |
| D-0388 | 1H-pyrazol-5-yl(4-CN,1-Me) |
| D-0389 | 1H-pyrazol-5-yl(1-Me,3-$CF_3$) |
| D-0390 | 1H-pyrazol-5-yl(1-Me,4-$CF_3$) |
| D-0391 | 1H-pyrazol-5-yl(1-Me,3-$CO_2$Et) |
| D-0392 | 1H-pyrazol-5-yl(1-Me,4-$CO_2$Et) |
| D-0393 | 1H-imidazol-1-yl |
| D-0394 | $CH_2$1H-imidazol-1-yl |
| D-0395 | 1H-imidazol-1-yl(2-F) |
| D-0396 | 1H-imidazol-1-yl(4-F) |
| D-0397 | 1H-imidazol-1-yl(5-F) |
| D-0398 | 1H-imidazol-1-yl(2-Cl) |
| D-0399 | 1H-imidazol-1-yl(4-Cl) |
| D-0400 | 1H-imidazol-1-yl(5-Cl) |
| D-0401 | 1H-imidazol-1-yl(2-Me) |
| D-0402 | 1H-imidazol-1-yl(4-Me) |
| D-0403 | 1H-imidazol-1-yl(5-Me) |
| D-0404 | 1H-imidazol-1-yl(2-OMe) |
| D-0405 | 1H-imidazol-1-yl(4-OMe) |
| D-0406 | 1H-imidazol-1-yl(5-OMe) |
| D-0407 | 1H-imidazol-1-yl(2-CN) |
| D-0408 | 1H-imidazol-1-yl(4-CN) |
| D-0409 | 1H-imidazol-1-yl(5-CN) |
| D-0410 | 1H-imidazol-1-yl(2-$CF_3$) |
| D-0411 | 1H-imidazol-1-yl(4-$CF_3$) |
| D-0412 | 1H-imidazol-1-yl(5-$CF_3$) |
| D-0413 | 1H-imidazol-1-yl(2-$CO_2$Et) |

(continued)

| Compound Number | Q |
|---|---|
| D-0414 | 1H-imidazol-1-yl(4-$CO_2$Et) |

[Table 78]

| Compound Number | Q |
|---|---|
| D-0415 | 1H-imidazol-1-yl(5-$CO_2$Et) |
| D-0416 | 1H-imidazol-2-yl |
| D-0417 | $CH_2$1H-imidazol-2-yl |
| D-0418 | 1H-imidazol-2-yl(1-Me) |
| D-0419 | $CH_2$1H-imidazol-2-yl(1-Me) |
| D-0420 | 1H-imidazol-2-yl(4-F,1-Me) |
| D-0421 | 1H-imidazol-2-yl(5-F,1-Me) |
| D-0422 | 1H-imidazol-2-yl(4-Cl,1-Me) |
| D-0423 | 1H-imidazol-2-yl(5-Cl,1-Me) |
| D-0424 | 1H-imidazol-2-yl(1,4-$Me_2$) |
| D-0425 | 1H-imidazol-2-yl(1,5-$Me_2$) |
| D-0426 | 1H-imidazol-2-yl(4-OMe,1-Me) |
| D-0427 | 1H-imidazol-2-yl(5-OMe,1-Me) |
| D-0428 | 1H-imidazol-2-yl(4-CN,1-Me) |
| D-0429 | 1H-imidazol-2-yl(5-CN,1-Me) |
| D-0430 | 1H-imidazol-2-yl(1-Me,4-$CF_3$) |
| D-0431 | 1H-imidazol-2-yl(1-Me,5-$CF_3$) |
| D-0432 | 1H-imidazol-2-yl(1-Me,4-$CO_2$Et) |
| D-0433 | 1H-imidazol-2-yl(1-Me,5-$CO_2$Et) |
| D-0434 | 1H-imidazol-4-yl |
| D-0435 | $CH_2$1H-imidazol-4-yl |
| D-0436 | 1H-imidazol-4-yl(1-Me) |
| D-0437 | $CH_2$1H-imidazol-4-yl(1-Me) |
| D-0438 | 1H-imidazol-4-yl(2-F,1-Me) |
| D-0439 | 1H-imidazol-4-yl(5-F,1-Me) |
| D-0440 | 1H-imidazol-4-yl(2-Cl,1-Me) |
| D-0441 | 1H-imidazol-4-yl(5-Cl,1-Me) |
| D-0442 | 1H-imidazol-4-yl(1,2-$Me_2$) |
| D-0443 | 1H-imidazol-4-yl(1,5-$Me_2$) |
| D-0444 | 1H-imidazol-4-yl(2-OMe,1-Me) |
| D-0445 | 1H-imidazol-4-yl(5-OMe,1-Me) |
| D-0446 | 1H-imidazol-4-yl(2-CN,1-Me) |

(continued)

| Compound Number | Q |
|---|---|
| D-0447 | 1H-imidazol-4-yl(5-CN,1-Me) |
| D-0448 | 1H-imidazol-4-yl(1-Me,2-$CF_3$) |
| D-0449 | 1H-imidazol-4-yl(1-Me,5-$CF_3$) |
| D-0450 | 1H-imidazol-4-yl(1-Me,2-$CO_2$Et) |
| D-0451 | 1H-imidazol-4-yl(1-Me,5-$CO_2$Et) |
| D-0452 | 1H-imidazol-5-yl |
| D-0453 | $CH_2$1H-imidazol-5-yl |
| D-0454 | 1H-imidazol-5-yl(1-Me) |
| D-0455 | $CH_2$1H-imidazol-5-yl(1-Me) |
| D-0456 | 1H-imidazol-5-yl(2-F,1-Me) |

[Table 79]

| Compound Number | Q |
|---|---|
| D-0457 | 1H-imidazol-5-yl(4-F,1-Me) |
| D-0458 | 1H-imidazol-5-yl(2-Cl,1-Me) |
| D-0459 | 1H-imidazol-5-yl(4-Cl,1-Me) |
| D-0460 | 1H-imidazol-5-yl(1,2-$Me_2$) |
| D-0461 | 1H-imidazol-5-yl(1,4-$Me_2$) |
| D-0462 | 1H-imidazol-5-yl(2-OMe,1-Me) |
| D-0463 | 1H-imidazol-5-yl(4-OMe,1-Me) |
| D-0464 | 1H-imidazol-5-yl(2-CN,1-Me) |
| D-0465 | 1H-imidazol-5-yl(4-CN,1-Me) |
| D-0466 | 1H-imidazol-5-yl(1-Me,2-$CF_3$) |
| D-0467 | 1H-imidazol-5-yl(1-Me,4-$CF_3$) |
| D-0468 | 1H-imidazol-5-yl(1-Me,2-$CO_2$Et) |
| D-0469 | 1H-imidazol-5-yl(1-Me,4-$CO_2$Et) |
| D-0470 | 1H-1,2,4-triazol-1-yl |
| D-0471 | $CH_2$1H-1,2,4-triazol-1-yl |
| D-0472 | 1H-1,2,4-triazol-1-yl(3-F) |
| D-0473 | 1H-1,2,4-triazol-1-yl(5-F) |
| D-0474 | 1H-1,2,4-triazol-1-yl(3-Cl) |
| D-0475 | 1H-1,2,4-triazol-1-yl(5-Cl) |
| D-0476 | 1H-1,2,4-triazol-1-yl(3-Me) |
| D-0477 | 1H-1,2,4-triazol-1-yl(5-Me) |
| D-0478 | 1H-1,2,4-triazol-1-yl(3-OMe) |
| D-0479 | 1H-1,2,4-triazol-1-yl(5-OMe) |

(continued)

| Compound Number | Q |
|---|---|
| D-0480 | 1H-1,2,4-triazol-1-yl(3-CN) |
| D-0481 | 1H-1,2,4-triazol-1-yl(5-CN) |
| D-0482 | 1H-1,2,4-triazol-1-yl(3-$CF_3$) |
| D-0483 | 1H-1,2,4-triazol-1-yl(5-$CF_3$) |
| D-0484 | 1H-1,2,4-triazol-1-yl(3-$CO_2$Et) |
| D-0485 | 1H-1,2,4-triazol-1-yl(5-$CO_2$Et) |
| D-0486 | 1H-1,2,4-triazol-3-yl |
| D-0487 | $CH_2$1H-1,2,4-triazol-3-yl |
| D-0488 | 1H-1,2,4-triazol-3-yl(1-Me) |
| D-0489 | $CH_2$1H-1,2,4-triazol-3-yl(1-Me) |
| D-0490 | 1H-1,2,4-triazol-3-yl(5-F,1-Me) |
| D-0491 | 1H-1,2,4-triazol-3-yl(5-Cl,1-Me) |
| D-0492 | 1H-1,2,4-triazol-3-yl(1,5-$Me_2$) |
| D-0493 | 1H-1,2,4-triazol-3-yl(5-OMe,1-Me) |
| D-0494 | 1H-1,2,4-triazol-3-yl(5-CN,1-Me) |
| D-0495 | 1H-1,2,4-triazol-3-yl(1-Me,5-$CF_3$) |
| D-0496 | 1H-1,2,4-triazol-3-yl(1-Me,5-$CO_2$Et) |
| D-0497 | 4H-1,2,4-triazol-4-yl |
| D-0498 | $CH_2$4H-1,2,4-triazol-4-yl |

[Table 80]

| Compound Number | Q |
|---|---|
| D-0499 | 4H-1,2,4-triazol-4-yl(3-F) |
| D-0500 | 4H-1,2,4-triazol-4-yl(3-Cl) |
| D-0501 | 4H-1,2,4-triazol-4-yl(3-Me) |
| D-0502 | 4H-1,2,4-triazol-4-yl(3-OMe) |
| D-0503 | 4H-1,2,4-triazol-4-yl(3-CN) |
| D-0504 | 4H-1,2,4-triazol-4-yl(3-$CF_3$) |
| D-0505 | 4H-1,2,4-triazol-4-yl(3-$CO_2$Et) |
| D-0506 | 1H-1,2,4-triazol-5-yl |
| D-0507 | $CH_2$1H-1,2,4-triazol-5-yl |
| D-0508 | 1H-1,2,4-triazol-5-yl(1-Me) |
| D-0509 | $CH_2$1H-1,2,4-triazol-5-yl(1-Me) |
| D-0510 | 1H-1,2,4-triazol-5-yl(3-F,1-Me) |
| D-0511 | 1H-1,2,4-triazol-5-yl(3-Cl,1-Me) |
| D-0512 | 1H-1,2,4-triazol-5-yl(1,3-$Me_2$) |

(continued)

| Compound Number | Q |
|---|---|
| D-0513 | 1H-1,2,4-triazol-5-yl(3-OMe,1-Me) |
| D-0514 | 1H-1,2,4-triazol-5-yl(3-CN,1-Me) |
| D-0515 | 1H-1,2,4-tri azol-5-yl(1-Me,3-$CF_3$) |
| D-0516 | 1H-1,2,4-triazol-5-yl(1-Me,3-$CO_2$Et) |
| D-0517 | 1H-1,2,3-triazol-1-yl |
| D-0518 | $CH_2$1H-1,2,3-triazol-1-yl |
| D-0519 | 1H-1,2,3-triazol-1-yl(4-Me) |
| D-0520 | 1H-1,2,3-triazol-1-yl(5-Me) |
| D-0521 | 1H-1,2,a-triazol-1-yi(4-$CO_2$Et) |
| D-0522 | 1H-1,2,3-triazol-1-yl(5-$CO_2$Et) |
| D-0523 | 2H-1,2,3-triazol-2-yl |
| D-0524 | $CH_2$2H-1,2,3-triazol-2-yl |
| D-0525 | 1H-tetrazol-1-yl |
| D-0526 | $CH_2$1H-tetrazol-1-yl |
| D-0527 | 2H-tetrazol-2-yl |
| D-0528 | $CH_2$2H-tetrazol-2-yl |
| D-0529 | 1H-tetrazol-5-yl |
| D-0530 | $CH_2$1H-tetrazol-5-yl |
| D-0531 | 1H-tetrazol-5-yl(1-Me) |
| D-0532 | $CH_2$1H-tetrazol-5-yl(1-Me) |
| D-0533 | thiazol-2-yl |
| D-0534 | $CH_2$thiazol-2-yl |
| D-0535 | thiazol-2-yl(4-F) |
| D-0536 | thiazol-2-yl(5-F) |
| D-0537 | thiazol-2-yl(4-Cl) |
| D-0538 | thiazol-2-yl(5-Cl) |
| D-0539 | thiazol-2-yl(4-Me) |
| D-0540 | thiazol-2-yl(5-Me) |

[Table 81]

| Compound Number | Q |
|---|---|
| D-0541 | thiazol-2-yl(4-OMe) |
| D-0542 | thiazol-2-yl(5-OMe) |
| D-0543 | thiazol-2-yl(4-CN) |
| D-0544 | thiazol-2-yl(5-CN) |
| D-0545 | thiazol-2-yl(4-$CF_3$) |

(continued)

| Compound Number | Q |
|---|---|
| D-0546 | thiazol-2-yl(5-$CF_3$) |
| D-0547 | thiazol-2-yl(4-$CO_2$Et) |
| D-0548 | thiazol-2-yl(5-$CO_2$Et) |
| D-0549 | thiazol-4-yl |
| D-0550 | $CH_2$thiazol-4-yl |
| D-0551 | thiazol-4-yl(2-F) |
| D-0552 | thiazol-4-yl(5-F) |
| D-0553 | thiazol-4-yl(2-Cl) |
| D-0554 | thiazol-4-yl(5-Cl) |
| D-0555 | thiazol-4-yl(2-Me) |
| D-0556 | thiazol-4-yl(5-Me) |
| D-0557 | thiazol-4-yl(2-OMe) |
| D-0558 | thiazol-4-yl(5-OMe) |
| D-0559 | thiazol-4-yl(2-CN) |
| D-0560 | thiazol-4-yl(5-CN) |
| D-0561 | thiazol-4-yl(2-$CF_3$) |
| D-0562 | thiazol-4-yl(5-$CF_3$) |
| D-0563 | thiazol-4-yl(2-$CO_2$Et) |
| D-0564 | thiazol-4-yl(5-$CO_2$Et) |
| D-0565 | thiazol-5-yl |
| D-0566 | $CH_2$thiazol-5-yl |
| D-0567 | thiazol-5-yl(2-F) |
| D-0568 | thiazol-5-yl(4-F) |
| D-0569 | thiazol-5-yl(2-Cl) |
| D-0570 | thiazol-5-yl(4-Cl) |
| D-0571 | thiazol-5-yl(2-Me) |
| D-0572 | thiazol-5-yl(4-Me) |
| D-0573 | thiazol-5-yl(2-OMe) |
| D-0574 | thiazol-5-yl(4-OMe) |
| D-0575 | thiazol-5-yl(2-CN) |
| D-0576 | thiazol-5-yl(4-CN) |
| D-0577 | thiazol-5-yl(2-$CF_3$) |
| D-0578 | thiazol-5-yl(4-$CF_3$) |
| D-0579 | thiazol-5-yl(2-$CO_2$Et) |
| D-0580 | thiazol-5-yl(4-$CO_2$Et) |
| D-0581 | isothiazol-3-yl |

(continued)

| Compound Number | Q |
|---|---|
| D-0582 | $CH_2$isothiazol-3-yl |

[Table 82]

| Compound Number | Q |
|---|---|
| D-0583 | isothiazol-3-yl(4-F) |
| D-0584 | isothiazol-3-yl(5-F) |
| D-0585 | isothiazol-3-yl(4-Cl) |
| D-0586 | isothiazol-3-yl(5-Cl) |
| D-0587 | isothiazol-3-yl(4-Me) |
| D-0588 | isothiazol-3-yl(5-Me) |
| D-0589 | isothiazol-3-yl(4-OMe) |
| D-0590 | isothiazol-3-yl(5-OMe) |
| D-0591 | isothiazol-3-yl(4-CN) |
| D-0592 | isothiazol-3-yl(5-CN) |
| D-0593 | isothiazol-3-yl(4-$CF_3$) |
| D-0594 | isothiazol-3-yl(5-$CF_3$) |
| D-0595 | isothiazol-3-yl(4-$CO_2$Et) |
| D-0596 | isothiazol-3-yl(5-$CO_2$Et) |
| D-0597 | isothiazol-4-yl |
| D-0598 | $CH_2$isothiazol-4-yl |
| D-0599 | isothiazol-4-yl(3-F) |
| D-0600 | isothiazol-4-yl(5-F) |
| D-0601 | isothiazol-4-yl(3-Cl) |
| D-0602 | isothiazol-4-yl(5-Cl) |
| D-0603 | isothiazol-4-yl(3-Me) |
| D-0604 | isothiazol-4-yl(5-Me) |
| D-0605 | isothiazol-4-yl(3-OMe) |
| D-0606 | isothiazol-4-yl(5-OMe) |
| D-0607 | isothiazol-4-yl(3-CN) |
| D-0608 | isothiazol-4-yl(5-CN) |
| D-0609 | isothiazol-4-yl(3-$CF_3$) |
| D-0610 | isothiazol-4-yl(5-$CF_3$) |
| D-0611 | isothiazol-4-yl(3-$CO_2$Et) |
| D-0612 | isothiazol-4-yl(5-$CO_2$Et) |
| D-0613 | isothiazol-5-yl |
| D-0614 | $CH_2$isothiazol-5-yl |

(continued)

| Compound Number | Q |
|---|---|
| D-0615 | isothiazol-5-yl(3-F) |
| D-0616 | isothiazol-5-yl(4-F) |
| D-0617 | isothiazol-5-yl(3-Cl) |
| D-0618 | isothiazol-5-yl(4-Cl) |
| D-0619 | isothiazol-5-yl(3,4-$Cl_2$) |
| D-0620 | isothiazol-5-yl(3-Me) |
| D-0621 | isothiazol-5-yl(4-Me) |
| D-0622 | isothiazol-5-yl(3-OMe) |
| D-0623 | isothiazol-5-yl(4-OMe) |
| D-0624 | isothiazol-5-yl(3-CN) |

[Table 83]

| Compound Number | Q |
|---|---|
| D-0625 | isothiazol-5-yl(4-CN) |
| D-0626 | isothiazol-5-yl(3-$CF_3$) |
| D-0627 | isothiazol-5-yl(4-$CF_3$) |
| D-0628 | isothiazol-5-yl(3-$CO_2$Et) |
| D-0629 | isothiazol-5-yl(4-$CO_2$Et) |
| D-0630 | oxazol-2-yl |
| D-0631 | $CH_2$oxazol-2-yl |
| D-0632 | oxazol-2-yl(4-F) |
| D-0633 | oxazol-2-yl(5-F) |
| D-0634 | oxazol-2-yl(4-Cl) |
| D-0635 | oxazol-2-yl(5-Cl) |
| D-0636 | oxazol-2-yl(4-Me) |
| D-0637 | oxazol-2-yl(5-Me) |
| D-0638 | oxazol-2-yl(4-OMe) |
| D-0639 | oxazol-2-yl(5-OMe) |
| D-0640 | oxazol-2-yl(4-CN) |
| D-0641 | oxazol-2-yl(5-CN) |
| D-0642 | oxazol-2-yl(4-$CF_3$) |
| D-0643 | oxazol-2-yl(5-$CF_3$) |
| D-0644 | oxazol-2-yl(4-$CO_2$Et) |
| D-0645 | oxazol-2-yl(5-$CO_2$Et) |
| D-0646 | oxazol-4-yl |
| D-0647 | $CH_2$oxazol-4-yl |

(continued)

| Compound Number | Q |
|---|---|
| D-0648 | oxazol-4-yl(2-F) |
| D-0649 | oxazol-4-yl(5-F) |
| D-0650 | oxazol-4-yl(2-Cl) |
| D-0651 | oxazol-4-yl(5-Cl) |
| D-0652 | oxazol-4-yl(2-Me) |
| D-0653 | oxazol-4-yl(5-Me) |
| D-0654 | oxazol-4-yl(2-OMe) |
| D-0655 | oxazol-4-yl(5-OMe) |
| D-0656 | oxazol-4-yl(2-CN) |
| D-0657 | oxazol-4-yl(5-CN) |
| D-0658 | oxazol-4-yl(2-$CF_3$) |
| D-0659 | oxazol-4-yl(5-$CF_3$) |
| D-0660 | oxazol-4-yl(2-$CO_2$Et) |
| D-0661 | oxazol-4-yl(5-$CO_2$Et) |
| D-0662 | oxazol-5-yl |
| D-0663 | $CH_2$oxazol-5-yl |
| D-0664 | oxazol-5-yl(2-F) |
| D-0665 | oxazol-5-yl(4-F) |
| D-0666 | oxazol-5-yl(2-Cl) |

[Table 84]

| Compound Number | Q |
|---|---|
| D-0667 | oxazol-5-yl(4-Cl) |
| D-0668 | oxazol-5-yl(2-Me) |
| D-0669 | oxazol-5-yl(4-Me) |
| D-0670 | oxazol-5-yl(2-OMe) |
| D-0671 | oxazol-5-yl(4-OMe) |
| D-0672 | oxazol-5-yl(2-CN) |
| D-0673 | oxazol-5-yl(4-CN) |
| D-0674 | oxazol-5-yl(2-$CF_3$) |
| D-0675 | oxazol-5-yl(4-$CF_3$) |
| D-0676 | oxazol-5-yl(2-$CO_2$Et) |
| D-0677 | oxazol-5-yl(4-$CO_2$Et) |
| D-0678 | isoxazol-3-yl |
| D-0679 | $CH_2$isoxazol-3-yl |
| D-0680 | isoxazol-3-yl(4-F) |

(continued)

| Compound Number | Q |
|---|---|
| D-0681 | isoxazol-3-yl(5-F) |
| D-0682 | isoxazol-3-yl(4-Cl) |
| D-0683 | isoxazol-3-yl(5-Cl) |
| D-0684 | isoxazol-3-yl(4-Me) |
| D-0685 | isoxazol-3-yl(5-Me) |
| D-0686 | isoxazol-3-yl(4-OMe) |
| D-0687 | isoxazol-3-yl(5-OMe) |
| D-0688 | isoxazol-3-yl(4-CN) |
| D-0689 | isoxazol-3-yl(5-CN) |
| D-0690 | isoxazol-3-yl(4-$CF_3$) |
| D-0691 | isoxazol-3-yl(5-$CF_3$) |
| D-0692 | isoxazol-3-yl(4-$CO_2$Et) |
| D-0693 | isoxazol-3-yl(5-$CO_2$Et) |
| D-0694 | isoxazol-4-yl |
| D-0695 | $CH_2$isoxazol-4-yl |
| D-0696 | isoxazol-4-yl(3-F) |
| D-0697 | isoxazol-4-yl(5-F) |
| D-0698 | isoxazol-4-yl(3-Cl) |
| D-0699 | isoxazol-4-yl(5-Cl) |
| D-0700 | isoxazol-4-yl(3-Me) |
| D-0701 | isoxazol-4-yl(5-Me) |
| D-0702 | isoxazol-4-yl(3-OMe) |
| D-0703 | isoxazol-4-yl(5-OMe) |
| D-0704 | isoxazol-4-yl(3-CN) |
| D-0705 | isoxazol-4-yl(5-CN) |
| D-0706 | isoxazol-4-yl(3-$CF_3$) |
| D-0707 | isoxazol-4-yl(5-$CF_3$) |
| D-0708 | isoxazol-4-yl(3-$CO_2$Et) |

[Table 85]

| Compound Number | Q |
|---|---|
| D-0709 | isoxazol-4-yl(5-$CO_2$Et) |
| D-0710 | isoxazol-5-yl |
| D-0711 | $CH_2$isoxazol-5-yl |
| D-0712 | isoxazol-5-yl(3-F) |
| D-0713 | isoxazol-5-yl(4-F) |

(continued)

| Compound Number | Q |
|---|---|
| D-0714 | isoxazol-5-yl(3-Cl) |
| D-0715 | isoxazol-5-yl(4-Cl) |
| D-0716 | isoxazol-5-yl(3-Me) |
| D-0717 | isoxazol-5-yl(4-Me) |
| D-0718 | isoxazol-5-yl(3-OMe) |
| D-0719 | isoxazol-5-yl(4-OMe) |
| D-0720 | isoxazol-5-yl(3-CN) |
| D-0721 | isoxazol-5-yl(4-CN) |
| D-0722 | isoxazol-5-yl(3-$CF_3$) |
| D-0723 | isoxazol-5-yl(4-$CF_3$) |
| D-0724 | isoxazol-5-yl(3-$CO_2$Et) |
| D-0725 | isoxazol-5-yl(4-$CO_2$Et) |
| D-0726 | 1,2,3-oxadiazol-4-yl |
| D-0727 | $CH_2$1,2,3-oxadiazol-4-yl |
| D-0728 | 1,2,3-oxadiazol-4-yl(5-F) |
| D-0729 | 1,2,3-oxadiazol-4-yl(5-Cl) |
| D-0730 | 1,2,3-oxadiazol-4-yl(5-Me) |
| D-0731 | 1,2,3-oxadiazol-4-yl(5-OMe) |
| D-0732 | 1,2,3-oxadiazol-4-yl(5-CN) |
| D-0733 | 1,2,3-oxadiazol-4-yl(5-$CF_3$) |
| D-0734 | 1,2,3-oxadiazol-4-yl(5-$CO_2$Et) |
| D-0735 | 1,2,3-oxadiazol-5-yl |
| D-0736 | $CH_2$1,2,3-oxadiazol-5-yl |
| D-0737 | 1,2,3-oxadiazol-5-yl(4-F) |
| D-0738 | 1,2,3-oxadiazol-5-yl(4-Cl) |
| D-0739 | 1,2,3-oxadiazol-5-yl(4-Me) |
| D-0740 | 1,2,3-oxadiazol-5-yl(4-OMe) |
| D-0741 | 1,2,3-oxadiazol-5-yl(4-CN) |
| D-0742 | 1,2,3-oxadiazol-5-yl(4-$CF_3$) |
| D-0743 | 1,2,3-oxadiazol-5-yl(4-$CO_2$Et) |
| D-0744 | 1,2,4-oxadiazol-3-yl |
| D-0745 | $CH_2$1,2,4-oxadiazol-3-yl |
| D-0746 | 1,2,4-oxadiazol-3-yl(5-F) |
| D-0747 | 1,2,4-oxadiazol-3-yl(5-Cl) |
| D-0748 | 1,2,4-oxadiazol-3-yl(5-Me) |
| D-0749 | 1,2,4-oxadiazol-3-yl(5-OMe) |

(continued)

| Compound Number | Q |
|---|---|
| D-0750 | 1,2,4-oxadiazol-3-yl(5-CN) |

[Table 86]

| Compound Number | Q |
|---|---|
| D-0751 | 1,2,4-oxadiazol-3-yl(5-$CF_3$) |
| D-0752 | 1,2,4-oxadiazol-3-yl(5-$CO_2$Et) |
| D-0753 | 1,2,4-oxadiazol-5-yl |
| D-0754 | $CH_2$1,2,4-oxadiazol-5-yl |
| D-0755 | 1,2,4-oxadiazol-5-yl(3-F) |
| D-0756 | 1,2,4-oxadiazol-5-yl(3-Cl) |
| D-0757 | 1,2,4-oxadiazol-5-yl(3-Me) |
| D-0758 | 1,2,4-oxadiazol-5-yl(3-OMe) |
| D-0759 | 1,2,4-oxadiazol-5-yl(3-CN) |
| D-0760 | 1,2,4-oxadiazol-5-yl(3-$CF_3$) |
| D-0761 | 1,2,4-oxadiazol-5-yl(3-$CO_2$Et) |
| D-0762 | 1,3,4-oxadiazol-2-yl |
| D-0763 | $CH_2$1,3,4-oxadiazol-2-yl |
| D-0764 | 1,2,5-oxadiazol-3-yl |
| D-0765 | $CH_2$1,2,5-oxadiazol-3-yl |
| D-0766 | 1,2,3-thiadiazol-4-yl |
| D-0767 | $CH_2$1,2,3-thiadiazol-4-yl |
| D-0768 | 1,2,3-thiadiazol-5-yl |
| D-0769 | $CH_2$1,2,3-thiadiazol-5-yl |
| D-0770 | 1,2,4-thiadiazol-3-yl |
| D-0771 | $CH_2$1,2,4-thiadiazol-3-yl |
| D-0772 | 1,2,4-thiadiazol-5-yl |
| D-0773 | $CH_2$1,2,4-thiadiazol-5-yl |
| D-0774 | 1,3,4-thiadiazol-2-yl |
| D-0775 | $CH_2$1,3,4-thiadiazol-2-yl |
| D-0776 | 1,2,5-thiadiazol-3-yl |
| D-0777 | $CH_2$1,2,5-thiadiazol-3-yl |
| D-0778 | morpholin-4-yl |
| D-0779 | $CH_2$morpholin-4-yl |
| D-0780 | piperidin-4-yl |
| D-0781 | $CH_2$piperidin-4-yl |
| D-0782 | piperidin-4-yl(1-Me) |

(continued)

| Compound Number | Q |
|---|---|
| D-0783 | $CH_2$piperidin-4-yl(1-Me) |
| D-0784 | piperidin-1-yl |
| D-0785 | $CH_2$piperidin-1-yl |
| D-0786 | piperazin-1-yl |
| D-0787 | $CH_2$piperazin-1-yl |
| D-0788 | piperazin-1-yl(4-Me) |
| D-0789 | $CH_2$piperazin-1yl(4-Me) |
| D-0790 | tetrahydro-2H-pyran-4-yl |
| D-0791 | $CH_2$tetra hydro-2H-pyran-4-yl |
| D-0792 | 1,3-dioxolan-2-yl |

[Table 87]

| Compound Number | Q |
|---|---|
| D-0793 | $CH_2$1,3-dioxolan-2-yl |
| D-0794 | 1,3-dioxolan-2-yl(2-$CF_3$) |
| D-0795 | $CH_2$1,3-dioxolan-2-yl(2-$CF_3$) |
| D-0796 | 1,3-dioxan-2-yl |
| D-0797 | $CH_2$1,3-dioxan-2-yl |
| D-0798 | 1,3-dioxan-2-yl(2-$CF_3$) |
| D-0799 | $CH_2$1,3-dioxan-2-yl(2-$CF_3$) |
| D-0800 | pyrrolidin-1-yl |
| D-0801 | $CH_2$pyrrolidin-1-yl |
| D-0802 | pyrrolidin-2-yl |
| D-0803 | $CH_2$pyrrolidin-2-yl |
| D-0804 | pyrrolidin-2-yl(1-Me) |
| D-0805 | $CH_2$pyrrolidin-2-yl(1-Me) |
| D-0806 | pyrrolidin-3-yl |
| D-0807 | $CH_2$pyrrolidin-3-yl |
| D-0808 | pyrrolidin-3-yl(1-Me) |
| D-0809 | $CH_2$pyrrolidin-3-yl(1-Me) |
| D-0810 | tetrahydrofuran-2-yl |
| D-0811 | C $H_2$tetra hyd rofura n-2-yl |
| D-0812 | tetrahydrofuran-3-yl |
| D-0813 | C $H_2$tetra hyd rofura n-3-yl |
| D-0814 | 4,5-dihydroisoxazol-3-yl |

(continued)

| Compound Number | Q |
|---|---|
| D-0815 | $CH_2$4,5-dihydroisoxazol-3-yl |
| D-0816 | 4,5-dihydroisoxazol-4-yl |
| D-0817 | $CH_2$4,5-dihydroisoxazol-4-yl |
| D-0818 | 4,5-dihydroisoxazol-5-yl |
| D-0819 | $CH_2$4,5-dihydroisoxazol-5-yl |
| D-0820 | oxetan-2-yl |
| D-0821 | $CH_2$oxetan-2-yl |
| D-0822 | oxetan-3-yl |
| D-0823 | oxetan-3-yl(3-Me) |
| D-0824 | $CH_2$oxetan-3-yl |
| D-0825 | azetidin-1-yl |
| D-0826 | $CH_2$azetidin-1-yl |
| D-0827 | azetidin-2-yl |
| D-0828 | $CH_2$azetidin-2-yl |
| D-0829 | azetidin-2-yl(1-Me) |
| D-0830 | $CH_2$azetidin-2-yl(1-Me) |
| D-0831 | azetidin-3-yl |
| D-0832 | $CH_2$azetidin-3-yl |
| D-0833 | azetidin-3-yl(1-Me) |
| D-0834 | $CH_2$azetidin-3-yl(1-Me) |

[Table 88]

| Compound Number | Q |
|---|---|
| D-0835 | oxiran-2-yl |
| D-0836 | $CH_2$oxiran-2-yl |
| D-0837 | 1,3,2-dioxaborolan-2-yl(4,4,5,5-$Me_4$) |
| D-0838 | tetrahydro-2H-pyran-2-yl |
| D-0839 | $CH_2$tetrahydro-2H-pyran-2-yl |
| D-0840 | tetrahydro-2H-pyran-3-yl |
| D-0841 | C $H_2$tetra hyd ro-2H-pyra n-3-yl |
| D-0842 | piperidin-2-yl |
| D-0843 | $CH_2$piperidin-2-yl |
| D-0844 | piperidin-2-yl(1-Me) |
| D-0845 | $CH_2$piperidin-2-yl(1-Me) |
| D-0846 | piperidin-3-yl |
| D-0847 | $CH_2$piperidin-3-yl |

(continued)

| Compound Number | Q |
|---|---|
| D-0848 | piperidin-3-yl(1-Me) |
| D-0849 | $CH_2$piperidin-3-yl(1-Me) |
| D-0850 | pyridin-2-yl(6-$CH_2OCH_3$) |
| D-0851 | 1H-pyrazol-5-yl(1-CHO) |
| D-0852 | (pyridin-1-oxide)-2-yl |
| D-0853 | (pyridin-1-oxide)-3-yl |
| D-0854 | (pyridin-1-oxide)-4-yl |

[Table 89]

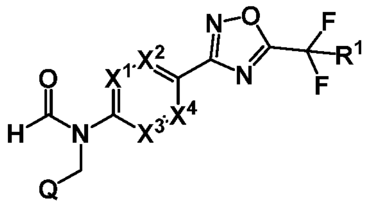

| Compound Number | $R^1$ | $X^1$ | $X^2$ | $X^3$ | $X^4$ | Q |
|---|---|---|---|---|---|---|
| E-0001 | F | CF | CH | CH | CH | pyridin-2-yl |
| E-0002 | F | CF | CH | CH | CH | pyridin-3-yl |
| E-0003 | F | CF | CH | CH | CH | pyridin-4-yl |
| E-0004 | F | CF | CH | CH | CH | pyrimidin-2-yl |
| E-0005 | F | CF | CH | CH | CH | pyrimidin-4-yl |
| E-0006 | F | CF | CH | CH | CH | pyrimidin-5-yl |
| E-0007 | F | CF | CH | CH | CH | pyridazin-3-yl |
| E-0008 | F | CF | CH | CH | CH | pyridazin-4-yl |
| E-0009 | F | CF | CH | CH | CH | pyrazin-2-yl |
| E-0010 | F | CF | CH | CH | CH | 1,3,5-triazin-2-yl |
| E-0011 | F | CF | CH | CH | CH | thiophen-2-yl |
| E-0012 | F | CF | CH | CH | CH | thiophen-3-yl |
| E-0013 | F | CF | CH | CH | CH | furan-2-yl |
| E-0014 | F | CF | CH | CH | CH | furan-3-yl |
| E-0015 | F | CF | CH | CH | CH | 1H-pyrrol-1-yl |
| E-0016 | F | CF | CH | CH | CH | 1H-pyrrol-2-yl |
| E-0017 | F | CF | CH | CH | CH | 1H-pyrrol-2-yl(1-Me) |
| E-0018 | F | CF | CH | CH | CH | 1H-pyrrol-3-yl |
| E-0019 | F | CF | CH | CH | CH | 1H-pyrrol-3-yl(1-Me) |
| E-0020 | F | CF | CH | CH | CH | 1H-pyrazol-1-yl |
| E-0021 | F | CF | CH | CH | CH | 1H-pyrazol-3-yl |
| E-0022 | F | CF | CH | CH | CH | 1H-pyrazol-3-yl(1 -Me) |

(continued)

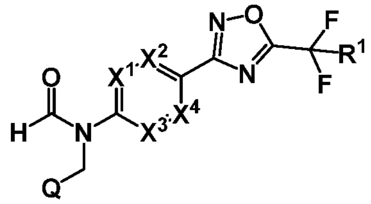

| Compound Number | $R^1$ | $X^1$ | $X^2$ | $X^3$ | $X^4$ | Q |
|---|---|---|---|---|---|---|
| E-0023 | F | CF | CH | CH | CH | 1H-pyrazol-4-yl |
| E-0024 | F | CF | CH | CH | CH | 1H-pyrazol-4-yl(1 -Me) |
| E-0025 | F | CF | CH | CH | CH | 1H-pyrazol-5-yl |
| E-0026 | F | CF | CH | CH | CH | 1H-pyrazol-5-yl(1 -Me) |
| E-0027 | F | CF | CH | CH | CH | 1H-imidazol-1-yl |
| E-0028 | F | CF | CH | CH | CH | 1H-imidazol-2-yl |
| E-0029 | F | CF | CH | CH | CH | 1H-imidazol-2-yl(1-Me) |
| E-0030 | F | CF | CH | CH | CH | 1H-imidazol-4-yl |
| E-0031 | F | CF | CH | CH | CH | 1H-imidazol-4-yl(1-Me) |
| E-0032 | F | CF | CH | CH | CH | 1H-imidazol-5-yl |
| E-0033 | F | CF | CH | CH | CH | 1H-imidazol-5-yl(1-Me) |
| E-0034 | F | CF | CH | CH | CH | 1H-1 ,2,4-triazol-1-yl |
| E-0035 | F | CF | CH | CH | CH | 1H-1 ,2,4-triazol-3-yl |
| E-0036 | F | CF | CH | CH | CH | 1H-1 ,2,4-triazol-3-yl(1-Me) |

[Table 90]

| Compound Number | $R^1$ | $X^1$ | $X^2$ | $X^3$ | $X^4$ | Q |
|---|---|---|---|---|---|---|
| E-0037 | F | CF | CH | CH | CH | 4H-1,2,4-triazol-4-yl |
| E-0038 | F | CF | CH | CH | CH | 1H-1,2,4-triazol-5-yl |
| E-0039 | F | CF | CH | CH | CH | 1H-1,2,4-triazol-5-yl(1-Me) |
| E-0040 | F | CF | CH | CH | CH | 1H-1,2,3-triazol-1-yl |
| E-0041 | F | CF | CH | CH | CH | 2H-1,2,3-triazol-2-yl |
| E-0042 | F | CF | CH | CH | CH | 1H-tetrazol-1-yl |
| E-0043 | F | CF | CH | CH | CH | 2H-tetrazol-2-yl |
| E-0044 | F | CF | CH | CH | CH | 1H-tetrazol-5-yl |
| E-0045 | F | CF | CH | CH | CH | 1H-tetrazol-5-yl(1 -Me) |
| E-0046 | F | CF | CH | CH | CH | thiazol-2-yl |
| E-0047 | F | CF | CH | CH | CH | thiazol-4-yl |
| E-0048 | F | CF | CH | CH | CH | thiazol-5-yl |
| E-0049 | F | CF | CH | CH | CH | isothiazol-3-yl |
| E-0050 | F | CF | CH | CH | CH | isothiazol-4-yl |
| E-0051 | F | CF | CH | CH | CH | isothiazol-5-yl |

(continued)

| Compound Number | $R^1$ | $X^1$ | $X^2$ | $X^3$ | $X^4$ | Q |
|---|---|---|---|---|---|---|
| E-0052 | F | CF | CH | CH | CH | oxazol-2-yl |
| E-0053 | F | CF | CH | CH | CH | oxazol-4-yl |
| E-0054 | F | CF | CH | CH | CH | oxazol-5-yl |
| E-0055 | F | CF | CH | CH | CH | isoxazol-3-yl |
| E-0056 | F | CF | CH | CH | CH | isoxazol-4-yl |
| E-0057 | F | CF | CH | CH | CH | isoxazol-5-yl |
| E-0058 | F | CF | CH | CH | CH | 1,2,3-oxadiazol-4-yl |
| E-0059 | F | CF | CH | CH | CH | 1,2,3-oxadiazol-5-yl |
| E-0060 | F | CF | CH | CH | CH | 1,2,4-oxadiazol-3-yl |
| E-0061 | F | CF | CH | CH | CH | 1,2,4-oxadiazol-5-yl |
| E-0062 | F | CF | CH | CH | CH | 1,3,4-oxadiazol-2-yl |
| E-0063 | F | CF | CH | CH | CH | 1,2,5-oxadiazol-3-yl |
| E-0064 | F | CF | CH | CH | CH | 1,2,3-thiadiazol-4-yl |
| E-0065 | F | CF | CH | CH | CH | 1,2,3-thiadiazol-5-yl |
| E-0066 | F | CF | CH | CH | CH | 1,2,4-thiadiazol-3-yl |
| E-0067 | F | CF | CH | CH | CH | 1,2,4-thiadiazol-5-yl |
| E-0068 | F | CF | CH | CH | CH | 1,3,4-thiadiazol-2-yl |
| E-0069 | F | CF | CH | CH | CH | 1,2,5-thiadiazol-3-yl |
| E-0070 | F | CF | CH | CH | CH | morpholin-4-yl |
| E-0071 | F | CF | CH | CH | CH | piperidin-4-yl |
| E-0072 | F | CF | CH | CH | CH | piperidin-4-yl(1-Me) |
| E-0073 | F | CF | CH | CH | CH | piperidin-1-yl |
| E-0074 | F | CF | CH | CH | CH | piperazin-1-yl |
| E-0075 | F | CF | CH | CH | CH | piperazin-1-yl(4-Me) |
| E-0076 | F | CF | CH | CH | CH | tetrahydro-2H-pyran-4-yl |
| E-0077 | F | CF | CH | CH | CH | 1,3-dioxolan-2-yl |
| E-0078 | F | CF | CH | CH | CH | 1,3-dioxolan-2-yl(2-$CF_3$) |

[Table 91]

| Compound Number | $R^1$ | $X^1$ | $X^2$ | $X^3$ | $X^4$ | Q |
|---|---|---|---|---|---|---|
| E-0079 | F | CF | CH | CH | CH | 1,3-dioxan-2-yl |
| E-0080 | F | CF | CH | CH | CH | 1,3-dioxan-2-yl(2-$CF_3$ |
| E-0081 | F | CF | CH | CH | CH | pyrrolidin-1-yl |
| E-0082 | F | CF | CH | CH | CH | pyrrolidin-2-yl |
| E-0083 | F | CF | CH | CH | CH | pyrrolidin-2-yl(1-Me) |
| E-0084 | F | CF | CH | CH | CH | pyrrolidin-3-yl |
| E-0085 | F | CF | CH | CH | CH | pyrrolidin-3-yl(1-Me) |

(continued)

| Compound Number | $R^1$ | $X^1$ | $X^2$ | $X^3$ | $X^4$ | Q |
|---|---|---|---|---|---|---|
| E-0086 | F | CF | CH | CH | CH | tetrahydrofuran-2-yl |
| E-0087 | F | CF | CH | CH | CH | tetrahydrofuran-3-yl |
| E-0088 | F | CF | CH | CH | CH | 4,5-dihydroisoxazol-3-yl |
| E-0089 | F | CF | CH | CH | CH | 4,5-dihydroisoxazol-4-yl |
| E-0090 | F | CF | CH | CH | CH | 4,5-dihydroisoxazol-5-yl |
| E-0091 | F | CF | CH | CH | CH | oxetan-2-yl |
| E-0092 | F | CF | CH | CH | CH | oxetan-3-yl |
| E-0093 | F | CF | CH | CH | CH | azetidin-1-yl |
| E-0094 | F | CF | CH | CH | CH | azetidin-2-yl |
| E-0095 | F | CF | CH | CH | CH | azetidi n-2-yl(1-Me) |
| E-0096 | F | CF | CH | CH | CH | azetidin-3-yl |
| E-0097 | F | CF | CH | CH | CH | azetidi n-3-yl(1-Me) |
| E-0098 | F | CF | CH | CH | CH | oxiran-2-yl |
| E-0099 | F | CF | CH | CH | CH | 1,3,2-dioxaborolan-2-yl(4,4,5,5-$Me_4$) |
| E-0100 | F | CH | CF | CH | CH | pyridin-2-yl |
| E-0101 | F | CH | CF | CH | CH | pyridin-3-yl |
| E-0102 | F | CH | CF | CH | CH | pyridin-4-yl |
| E-0103 | F | CH | CF | CH | CH | pyrimidin-2-yl |
| E-0104 | F | CH | CF | CH | CH | pyrimidin-4-yl |
| E-0105 | F | CH | CF | CH | CH | pyrimidin-5-yl |
| E-0106 | F | CH | CF | CH | CH | pyridazin-3-yl |
| E-0107 | F | CH | CF | CH | CH | pyridazin-4-yl |
| E-0108 | F | CH | CF | CH | CH | pyrazin-2-yl |
| E-0109 | F | CH | CF | CH | CH | 1,3,5-triazin-2-yl |
| E-0110 | F | CH | CF | CH | CH | thiophen-2-yl |
| E-0111 | F | CH | CF | CH | CH | thiophen-3-yl |
| E-0112 | F | CH | CF | CH | CH | furan-2-yl |
| E-0113 | F | CH | CF | CH | CH | furan-3-yl |
| E-0114 | F | CH | CF | CH | CH | 1H-pyrrol-1-yl |
| E-0115 | F | CH | CF | CH | CH | 1H-pyrrol-2-yl |
| E-0116 | F | CH | CF | CH | CH | 1H-pyrrol-2-yl(1-Me) |
| E-0117 | F | CH | CF | CH | CH | 1H-pyrrol-3-yl |
| E-0118 | F | CH | CF | CH | CH | 1H-pyrrol-3-yl(1-Me) |
| E-0119 | F | CH | CF | CH | CH | 1H-pyrazol-1-yl |
| E-0120 | F | CH | CF | CH | CH | 1H-pyrazol-3-yl |

[Table 92]

| Compound Number | $R^1$ | $X^1$ | $X^2$ | $X^3$ | $X^4$ | Q |
|---|---|---|---|---|---|---|
| E-0121 | F | CH | CF | CH | CH | 1H-pyrazol-3-yl(1 -Me) |
| E-0122 | F | CH | CF | CH | CH | 1H-pyrazol-4-yl |
| E-0123 | F | CH | CF | CH | CH | 1H-pyrazol-4-yl(1 -Me) |
| E-0124 | F | CH | CF | CH | CH | 1H-pyrazol-5-yl |
| E-0125 | F | CH | CF | CH | CH | 1H-pyrazol-5-yl(1 -Me) |
| E-0126 | F | CH | CF | CH | CH | 1H-imidazol-1-yl |
| E-0127 | F | CH | CF | CH | CH | 1H-imidazol-2-yl |
| E-0128 | F | CH | CF | CH | CH | 1H-imidazol-2-yl(1-Me) |
| E-0129 | F | CH | CF | CH | CH | 1H-imidazol-4-yl |
| E-0130 | F | CH | CF | CH | CH | 1H-imidazol-4-yl(1-Me) |
| E-0131 | F | CH | CF | CH | CH | 1H-imidazol-5-yl |
| E-0132 | F | CH | CF | CH | CH | 1H-imidazol-5-yl(1-Me) |
| E-0133 | F | CH | CF | CH | CH | 1H-1,2,4-triazol-1-yl |
| E-0134 | F | CH | CF | CH | CH | 1H-1 ,2,4-triazol-3-yl |
| E-0135 | F | CH | CF | CH | CH | 1H-1 ,2,4-triazol-3-yl(1-Me) |
| E-0136 | F | CH | CF | CH | CH | 4H-1,2,4-triazol-4-yl |
| E-0137 | F | CH | CF | CH | CH | 1H-1,2,4-triazol-5-yl |
| E-0138 | F | CH | CF | CH | CH | 1H-1,2,4-triazol-5-yl(1-Me) |
| E-0139 | F | CH | CF | CH | CH | 1H-1,2,3-triazol-1-yl |
| E-0140 | F | CH | CF | CH | CH | 2H-1,2,3-triazol-2-yl |
| E-0141 | F | CH | CF | CH | CH | 1H-tetrazol-1-yl |
| E-0142 | F | CH | CF | CH | CH | 2H-tetrazol-2-yl |
| E-0143 | F | CH | CF | CH | CH | 1H-tetrazol-5-yl |
| E-0144 | F | CH | CF | CH | CH | 1H-tetrazol-5-yl(1 -Me) |
| E-0145 | F | CH | CF | CH | CH | thiazol-2-yl |
| E-0146 | F | CH | CF | CH | CH | thiazol-4-yl |
| E-0147 | F | CH | CF | CH | CH | thiazol-5-yl |
| E-0148 | F | CH | CF | CH | CH | isothiazol-3-yl |
| E-0149 | F | CH | CF | CH | CH | isothiazol-4-yl |
| E-0150 | F | CH | CF | CH | CH | isothiazol-5-yl |
| E-0151 | F | CH | CF | CH | CH | oxazol-2-yl |
| E-0152 | F | CH | CF | CH | CH | oxazol-4-yl |
| E-0153 | F | CH | CF | CH | CH | oxazol-5-yl |
| E-0154 | F | CH | CF | CH | CH | isoxazol-3-yl |
| E-0155 | F | CH | CF | CH | CH | isoxazol-4-yl |
| E-0156 | F | CH | CF | CH | CH | isoxazol-5-yl |
| E-0157 | F | CH | CF | CH | CH | 1,2,3-oxadiazol-4-yl |
| E-0158 | F | CH | CF | CH | CH | 1,2,3-oxadiazol-5-yl |

(continued)

| Compound Number | $R^1$ | $X^1$ | $X^2$ | $X^3$ | $X^4$ | Q |
|---|---|---|---|---|---|---|
| E-0159 | F | CH | CF | CH | CH | 1,2,4-oxadiazol-3-yl |
| E-0160 | F | CH | CF | CH | CH | 1,2,4-oxadiazol-5-yl |
| E-0161 | F | CH | CF | CH | CH | 1,3,4-oxadiazol-2-yl |
| E-0162 | F | CH | CF | CH | CH | 1,2,5-oxadiazol-3-yl |

[Table 93]

| Compound Number | $R^1$ | $X^1$ | $X^2$ | $X^3$ | $X^4$ | Q |
|---|---|---|---|---|---|---|
| E-0163 | F | CH | CF | CH | CH | 1,2,3-thiadiazol-4-yl |
| E-0164 | F | CH | CF | CH | CH | 1,2,3-thiadiazol-5-yl |
| E-0165 | F | CH | CF | CH | CH | 1,2,4-thiadiazol-3-yl |
| E-0166 | F | CH | CF | CH | CH | 1,2,4-thiadiazol-5-yl |
| E-0167 | F | CH | CF | CH | CH | 1,3,4-thiadiazol-2-yl |
| E-0168 | F | CH | CF | CH | CH | 1,2,5-thiadiazol-3-yl |
| E-0169 | F | CH | CF | CH | CH | morpholin-4-yl |
| E-0170 | F | CH | CF | CH | CH | piperidin-4-yl |
| E-0171 | F | CH | CF | CH | CH | piperidin-4-yl(1-Me) |
| E-0172 | F | CH | CF | CH | CH | piperidin-1-yl |
| E-0173 | F | CH | CF | CH | CH | piperazin-1-yl |
| E-0174 | F | CH | CF | CH | CH | piperazin-1-yl(4-Me) |
| E-0175 | F | CH | CF | CH | CH | tetrahydro-2H-pyran-4-yl |
| E-0176 | F | CH | CF | CH | CH | 1,3-dioxolan-2-yl |
| E-0177 | F | CH | CF | CH | CH | 1,3-dioxolan-2-yl(2-$CF_3$) |
| E-0178 | F | CH | CF | CH | CH | 1,3-dioxan-2-yl |
| E-0179 | F | CH | CF | CH | CH | 1,3-dioxan-2-yl(2-$CF_3$) |
| E-0180 | F | CH | CF | CH | CH | pyrrolidin-1-yl |
| E-0181 | F | CH | CF | CH | CH | pyrrolidin-2-yl |
| E-0182 | F | CH | CF | CH | CH | pyrrolidin-2-yl(1-Me) |
| E-0183 | F | CH | CF | CH | CH | pyrrolidin-3-yl |
| E-0184 | F | CH | CF | CH | CH | pyrrolidin-3-yl(1-Me) |
| E-0185 | F | CH | CF | CH | CH | tetrahydrofuran-2-yl |
| E-0186 | F | CH | CF | CH | CH | tetrahydrofuran-3-yl |
| E-0187 | F | CH | CF | CH | CH | 4,5-dihydroisoxazol-3-yl |
| E-0188 | F | CH | CF | CH | CH | 4,5-dihydroisoxazol-4-yl |
| E-0189 | F | CH | CF | CH | CH | 4,5-dihydroisoxazol-5-yl |
| E-0190 | F | CH | CF | CH | CH | oxetan-2-yl |
| E-0191 | F | CH | CF | CH | CH | oxetan-3-yl |
| E-0192 | F | CH | CF | CH | CH | azetidin-1-yl |

(continued)

| Compound Number | $R^1$ | $X^1$ | $X^2$ | $X^3$ | $X^4$ | Q |
|---|---|---|---|---|---|---|
| E-0193 | F | CH | CF | CH | CH | azetidin-2-yl |
| E-0194 | F | CH | CF | CH | CH | azetidi n-2-yl(1-Me) |
| E-0195 | F | CH | CF | CH | CH | azetidin-3-yl |
| E-0196 | F | CH | CF | CH | CH | azetidi n-3-yl(1-Me) |
| E-0197 | F | CH | CF | CH | CH | oxiran-2-yl |
| E-0198 | F | CH | CF | CH | CH | 1,3,2-dioxaborolan-2-yl(4,4,5,5-$Me_4$) |
| E-0199 | F | CMe | CH | CH | CH | pyridin-2-yl |
| E-0200 | F | CMe | CH | CH | CH | pyridin-3-yl |
| E-0201 | F | CMe | CH | CH | CH | pyridin-4-yl |
| E-0202 | F | CMe | CH | CH | CH | pyrimidin-2-yl |
| E-0203 | F | CMe | CH | CH | CH | pyrimidin-4-yl |
| E-0204 | F | CMe | CH | CH | CH | pyrimidin-5-yl |

[Table 94]

| Compound Number | $R^1$ | $X^1$ | $X^2$ | $X^3$ | $X^1$ | Q |
|---|---|---|---|---|---|---|
| E-0205 | F | CMe | CH | CH | CH | pyridazin-3-yl |
| E-0206 | F | CMe | CH | CH | CH | pyridazin-4-yl |
| E-0207 | F | CMe | CH | CH | CH | pyrazin-2-yl |
| E-0208 | F | CMe | CH | CH | CH | 1,3,5-triazin-2-yl |
| E-0209 | F | CMe | CH | CH | CH | thiophen-2-yl |
| E-0210 | F | CMe | CH | CH | CH | thiophen-3-yl |
| E-0211 | F | CMe | CH | CH | CH | furan-2-yl |
| E-0212 | F | CMe | CH | CH | CH | furan-3-yl |
| E-0213 | F | CMe | CH | CH | CH | 1H-pyrrol-1-yl |
| E-0214 | F | CMe | CH | CH | CH | 1H-pyrrol-2-yl |
| E-0215 | F | CMe | CH | CH | CH | 1H-pyrrol-2-yl(1-Me) |
| E-0216 | F | CMe | CH | CH | CH | 1H-pyrrol-3-yl |
| E-0217 | F | CMe | CH | CH | CH | 1H-pyrrol-3-yl(1-Me) |
| E-0218 | F | CMe | CH | CH | CH | 1H-pyrazol-1-yl |
| E-0219 | F | CMe | CH | CH | CH | 1H-pyrazol-3-yl |
| E-0220 | F | CMe | CH | CH | CH | 1H-pyrazol-3-yl(1-Me) |
| E-0221 | F | CMe | CH | CH | CH | 1H-pyrazol-4-yl |
| E-0222 | F | CMe | CH | CH | CH | 1H-pyrazol-4-yl(1 -Me) |
| E-0223 | F | CMe | CH | CH | CH | 1H-pyrazol-5-yl |
| E-0224 | F | CMe | CH | CH | CH | 1H-pyrazol-5-yl(1 -Me) |
| E-0225 | F | CMe | CH | CH | CH | 1H-imidazol-1-yl |
| E-0226 | F | CMe | CH | CH | CH | 1H-imidazol-2-yl |

(continued)

| Compound Number | $R^1$ | $X^1$ | $X^2$ | $X^3$ | $X^1$ | Q |
|---|---|---|---|---|---|---|
| E-0227 | F | CMe | CH | CH | CH | 1H-imidazol-2-yl(1-Me) |
| E-0228 | F | CMe | CH | CH | CH | 1H-imidazol-4-yl |
| E-0229 | F | CMe | CH | CH | CH | 1H-imidazol-4-yl(1-Me) |
| E-0230 | F | CMe | CH | CH | CH | 1H-imidazol-5-yl |
| E-0231 | F | CMe | CH | CH | CH | 1H-imidazol-5-yl(1-Me) |
| E-0232 | F | CMe | CH | CH | CH | 1H-1,2,4-triazol-1-yl |
| E-0233 | F | CMe | CH | CH | CH | 1H-1,2,4-triazol-3-yl |
| E-0234 | F | CMe | CH | CH | CH | 1H-1,2,4-triazol-3-yl(1-Me) |
| E-0235 | F | CMe | CH | CH | CH | 4H-1,2,4-triazol-4-yl |
| E-0236 | F | CMe | CH | CH | CH | 1H-1,2,4-triazol-5-yl |
| E-0237 | F | CMe | CH | CH | CH | 1H-1,2,4-triazol-5-yl(1-Me) |
| E-0238 | F | CMe | CH | CH | CH | 1H-1,2,3-triazol-1-yl |
| E-0239 | F | CMe | CH | CH | CH | 2H-1,2,3-triazol-2-yl |
| E-0240 | F | CMe | CH | CH | CH | 1H-tetrazol-1-yl |
| E-0241 | F | CMe | CH | CH | CH | 2H-tetrazol-2-yl |
| E-0242 | F | CMe | CH | CH | CH | 1H-tetrazol-5-yl |
| E-0243 | F | CMe | CH | CH | CH | 1H-tetrazol-5-yl(1 -Me) |
| E-0244 | F | CMe | CH | CH | CH | thiazol-2-yl |
| E-0245 | F | CMe | CH | CH | CH | thiazol-4-yl |
| E-0246 | F | CMe | CH | CH | CH | thiazol-5-yl |

[Table 95]

| Compound Number | $R^1$ | $X^1$ | $X^2$ | $X^3$ | $X^1$ | Q |
|---|---|---|---|---|---|---|
| E-0247 | F | CMe | CH | CH | CH | isothiazol-3-yl |
| E-0248 | F | CMe | CH | CH | CH | isothiazol-4-yl |
| E-0249 | F | CMe | CH | CH | CH | isothiazol-5-yl |
| E-0250 | F | CMe | CH | CH | CH | oxazol-2-yl |
| E-0251 | F | CMe | CH | CH | CH | oxazol-4-yl |
| E-0252 | F | CMe | CH | CH | CH | oxazol-5-yl |
| E-0253 | F | CMe | CH | CH | CH | isoxazol-3-yl |
| E-0254 | F | CMe | CH | CH | CH | isoxazol-4-yl |
| E-0255 | F | CMe | CH | CH | CH | isoxazol-5-yl |
| E-0256 | F | CMe | CH | CH | CH | 1,2,3-oxadiazol-4-yl |
| E-0257 | F | CMe | CH | CH | CH | 1,2,3-oxadiazol-5-yl |
| E-0258 | F | CMe | CH | CH | CH | 1,2,4-oxadiazol-3-yl |
| E-0259 | F | CMe | CH | CH | CH | 1,2,4-oxadiazol-5-yl |
| E-0260 | F | CMe | CH | CH | CH | 1,3,4-oxadiazol-2-yl |

(continued)

| Compound Number | $R^1$ | $X^1$ | $X^2$ | $X^3$ | $X^1$ | Q |
|---|---|---|---|---|---|---|
| E-0261 | F | CMe | CH | CH | CH | 1,2,5-oxadiazol-3-yl |
| E-0262 | F | CMe | CH | CH | CH | 1,2,3-thiadiazol-4-yl |
| E-0263 | F | CMe | CH | CH | CH | 1,2,3-thiadiazol-5-yl |
| E-0264 | F | CMe | CH | CH | CH | 1,2,4-thiadiazol-3-yl |
| E-0265 | F | CMe | CH | CH | CH | 1,2,4-thiadiazol-5-yl |
| E-0266 | F | CMe | CH | CH | CH | 1,3,4-thiadiazol-2-yl |
| E-0267 | F | CMe | CH | CH | CH | 1,2,5-thiadiazol-3-yl |
| E-0268 | F | CMe | CH | CH | CH | morpholin-4-yl |
| E-0269 | F | CMe | CH | CH | CH | piperidin-4-yl |
| E-0270 | F | CMe | CH | CH | CH | piperidin-4-yl(1-Me) |
| E-0271 | F | CMe | CH | CH | CH | piperidin-1-yl |
| E-0272 | F | CMe | CH | CH | CH | piperazin-1-yl |
| E-0273 | F | CMe | CH | CH | CH | piperazin-1-yl(4-Me) |
| E-0274 | F | CMe | CH | CH | CH | tetrahydro-2H-pyran-4-yl |
| E-0275 | F | CMe | CH | CH | CH | 1,3-dioxolan-2-yl |
| E-0276 | F | CMe | CH | CH | CH | 1,3-dioxolan-2-yl(2-$CF_3$) |
| E-0277 | F | CMe | CH | CH | CH | 1,3-dioxan-2-yl |
| E-0278 | F | CMe | CH | CH | CH | 1,3-dioxan-2-yl(2-$CF_3$) |
| E-0279 | F | CMe | CH | CH | CH | pyrrolidin-1-yl |
| E-0280 | F | CMe | CH | CH | CH | pyrrolidin-2-yl |
| E-0281 | F | CMe | CH | CH | CH | pyrrolidin-2-yl(1-Me) |
| E-0282 | F | CMe | CH | CH | CH | pyrrolidin-3-yl |
| E-0283 | F | CMe | CH | CH | CH | pyrrolidin-3-yl(1-Me) |
| E-0284 | F | CMe | CH | CH | CH | tetrahydrofuran-2-yl |
| E-0285 | F | CMe | CH | CH | CH | tetrahydrofuran-3-yl |
| E-0286 | F | CMe | CH | CH | CH | 4,5-dihydroisoxazol-3-yl |
| E-0287 | F | CMe | CH | CH | CH | 4,5-dihydroisoxazol-4-yl |
| E-0288 | F | CMe | CH | CH | CH | 4,5-dihydroisoxazol-5-yl |

[Table 96]

| Compound Number | $R^1$ | $X^1$ | $X^2$ | $X^3$ | $X^1$ | Q |
|---|---|---|---|---|---|---|
| E-0289 | F | CMe | CH | CH | CH | oxetan-2-yl |
| E-0290 | F | CMe | CH | CH | CH | oxetan-3-yl |
| E-0291 | F | CMe | CH | CH | CH | azetidin-1-yl |
| E-0292 | F | CMe | CH | CH | CH | azetidin-2-yl |
| E-0293 | F | CMe | CH | CH | CH | azetidi n-2-yl(1-Me) |
| E-0294 | F | CMe | CH | CH | CH | azetidin-3-yl |

(continued)

| Compound Number | $R^1$ | $X^1$ | $X^2$ | $X^3$ | $X^1$ | Q |
|---|---|---|---|---|---|---|
| E-0295 | F | CMe | CH | CH | CH | azetidi n-3-yl(1-Me) |
| E-0296 | F | CMe | CH | CH | CH | oxiran-2-yl |
| E-0297 | F | CMe | CH | CH | CH | 1,3,2-dioxaborolan-2-yl(4,4,5,5-$Me_4$) |
| E-0298 | F | CH | CMe | CH | CH | pyridin-2-yl |
| E-0299 | F | CH | CMe | CH | CH | pyridin-3-yl |
| E-0300 | F | CH | CMe | CH | CH | pyridin-4-yl |
| E-0301 | F | CH | CMe | CH | CH | pyrimidin-2-yl |
| E-0302 | F | CH | CMe | CH | CH | pyrimidin-4-yl |
| E-0303 | F | CH | CMe | CH | CH | pyrimidin-5-yl |
| E-0304 | F | CH | CMe | CH | CH | pyridazin-3-yl |
| E-0305 | F | CH | CMe | CH | CH | pyridazin-4-yl |
| E-0306 | F | CH | CMe | CH | CH | pyrazin-2-yl |
| E-0307 | F | CH | CMe | CH | CH | 1,3,5-triazin-2-yl |
| E-0308 | F | CH | CMe | CH | CH | thiophen-2-yl |
| E-0309 | F | CH | CMe | CH | CH | thiophen-3-yl |
| E-0310 | F | CH | CMe | CH | CH | furan-2-yl |
| E-0311 | F | CH | CMe | CH | CH | furan-3-yl |
| E-0312 | F | CH | CMe | CH | CH | 1H-pyrrol-1-yl |
| E-0313 | F | CH | CMe | CH | CH | 1H-pyrrol-2-yl |
| E-0314 | F | CH | CMe | CH | CH | 1H-pyrrol-2-yl(1-Me) |
| E-0315 | F | CH | CMe | CH | CH | 1H-pyrrol-3-yl |
| E-0316 | F | CH | CMe | CH | CH | 1H-pyrrol-3-yl(1-Me) |
| E-0317 | F | CH | CMe | CH | CH | 1H-pyrazol-1-yl |
| E-0318 | F | CH | CMe | CH | CH | 1H-pyrazol-3-yl |
| E-0319 | F | CH | CMe | CH | CH | 1H-pyrazol-3-yl(1-Me) |
| E-0320 | F | CH | CMe | CH | CH | 1H-pyrazol-4-yl |
| E-0321 | F | CH | CMe | CH | CH | 1H-pyrazol-4-yl(1-Me) |
| E-0322 | F | CH | CMe | CH | CH | 1H-pyrazol-5-yl |
| E-0323 | F | CH | CMe | CH | CH | 1H-pyrazol-5-yl(1-Me) |
| E-0324 | F | CH | CMe | CH | CH | 1H-imidazol-1-yl |
| E-0325 | F | CH | CMe | CH | CH | 1H-imidazol-2-yl |
| E-0326 | F | CH | CMe | CH | CH | 1H-imidazol-2-yl(1-Me) |
| E-0327 | F | CH | CMe | CH | CH | 1H-imidazol-4-yl |
| E-0328 | F | CH | CMe | CH | CH | 1H-imidazol-4-yl(1-Me) |
| E-0329 | F | CH | CMe | CH | CH | 1H-imidazol-5-yl |
| E-0330 | F | CH | CMe | CH | CH | 1H-imidazol-5-yl(1-Me) |

[Table 97]

| Compound Number | $R^1$ | $X^1$ | $X^2$ | $X^3$ | $X^4$ | Q |
|---|---|---|---|---|---|---|
| E-0331 | F | CH | CMe | CH | CH | 1H-1,2,4-triazol-1-yl |
| E-0332 | F | CH | CMe | CH | CH | 1H-1,2,4-triazol-3-yl |
| E-0333 | F | CH | CMe | CH | CH | 1H-1,2,4-triazol-3-yl(1-Me) |
| E-0334 | F | CH | CMe | CH | CH | 4H-1,2,4-triazol-4-yl |
| E-0335 | F | CH | CMe | CH | CH | 1H-1,2,4-triazol-5-yl |
| E-0336 | F | CH | CMe | CH | CH | 1H-1,2,4-triazol-5-yl(1-Me) |
| E-0337 | F | CH | CMe | CH | CH | 1H-1,2,3-triazol-1-yl |
| E-0338 | F | CH | CMe | CH | CH | 2H-1,2,3-triazol-2-yl |
| E-0339 | F | CH | CMe | CH | CH | 1H-tetrazol-1-yl |
| E-0340 | F | CH | CMe | CH | CH | 2H-tetrazol-2-yl |
| E-0341 | F | CH | CMe | CH | CH | 1H-tetrazol-5-yl |
| E-0342 | F | CH | CMe | CH | CH | 1H-tetrazol-5-yl(1-Me) |
| E-0343 | F | CH | CMe | CH | CH | thiazol-2-yl |
| E-0344 | F | CH | CMe | CH | CH | thiazol-4-yl |
| E-0345 | F | CH | CMe | CH | CH | thiazol-5-yl |
| E-0346 | F | CH | CMe | CH | CH | isothiazol-3-yl |
| E-0347 | F | CH | CMe | CH | CH | isothiazol-4-yl |
| E-0348 | F | CH | CMe | CH | CH | isothiazol-5-yl |
| E-0349 | F | CH | CMe | CH | CH | oxazol-2-yl |
| E-0350 | F | CH | CMe | CH | CH | oxazol-4-yl |
| E-0351 | F | CH | CMe | CH | CH | oxazol-5-yl |
| E-0352 | F | CH | CMe | CH | CH | isoxazol-3-yl |
| E-0353 | F | CH | CMe | CH | CH | isoxazol-4-yl |
| E-0354 | F | CH | CMe | CH | CH | isoxazol-5-yl |
| E-0355 | F | CH | CMe | CH | CH | 1,2,3-oxadiazol-4-yl |
| E-0356 | F | CH | CMe | CH | CH | 1,2,3-oxadiazol-5-yl |
| E-0357 | F | CH | CMe | CH | CH | 1,2,4-oxadiazol-3-yl |
| E-0358 | F | CH | CMe | CH | CH | 1,2,4-oxadiazol-5-yl |
| E-0359 | F | CH | CMe | CH | CH | 1,3,4-oxadiazol-2-yl |
| E-0360 | F | CH | CMe | CH | CH | 1,2,5-oxadiazol-3-yl |
| E-0361 | F | CH | CMe | CH | CH | 1,2,3-thiadiazol-4-yl |
| E-0362 | F | CH | CMe | CH | CH | 1,2,3-thiadiazol-5-yl |
| E-0363 | F | CH | CMe | CH | CH | 1,2,4-thiadiazol-3-yl |
| E-0364 | F | CH | CMe | CH | CH | 1,2,4-thiadiazol-5-yl |
| E-0365 | F | CH | CMe | CH | CH | 1,3,4-thiadiazol-2-yl |
| E-0366 | F | CH | CMe | CH | CH | 1,2,5-thiadiazol-3-yl |
| E-0367 | F | CH | CMe | CH | CH | morpholin-4-yl |

(continued)

| Compound Number | $R^1$ | $X^1$ | $X^2$ | $X^3$ | $X^4$ | Q |
|---|---|---|---|---|---|---|
| E-0368 | F | CH | CMe | CH | CH | piperidin-4-yl |
| E-0369 | F | CH | CMe | CH | CH | piperidin-4-yl(1-Me) |
| E-0370 | F | CH | CMe | CH | CH | piperidin-1-yl |
| E-0371 | F | CH | CMe | CH | CH | piperazin-1-yl |
| E-0372 | F | CH | CMe | CH | CH | piperazin-1-yl(4-Me) |

[Table 98]

| Compound Number | $R^1$ | $X^1$ | $X^2$ | $X^3$ | $X^1$ | Q |
|---|---|---|---|---|---|---|
| E-0373 | F | CH | CMe | CH | CH | tetrahydro-2H-pyran-4-yl |
| E-0374 | F | CH | CMe | CH | CH | 1,3-dioxolan-2-yl |
| E-0375 | F | CH | CMe | CH | CH | 1,3-dioxolan-2-yl(2-$CF_3$) |
| E-0376 | F | CH | CMe | CH | CH | 1,3-dioxan-2-yl |
| E-0377 | F | CH | CMe | CH | CH | 1,3-dioxan-2-yl(2-$CF_3$) |
| E-0378 | F | CH | CMe | CH | CH | pyrrolidin-1-yl |
| E-0379 | F | CH | CMe | CH | CH | pyrrolidin-2-yl |
| E-0380 | F | CH | CMe | CH | CH | pyrrolidin-2-yl(1-Me) |
| E-0381 | F | CH | CMe | CH | CH | pyrrolidin-3-yl |
| E-0382 | F | CH | CMe | CH | CH | pyrrolidin-3-yl(1-Me) |
| E-0383 | F | CH | CMe | CH | CH | tetrahydrofuran-2-yl |
| E-0384 | F | CH | CMe | CH | CH | tetrahydrofuran-3-yl |
| E-0385 | F | CH | CMe | CH | CH | 4,5-dihydroisoxazol-3-yl |
| E-0386 | F | CH | CMe | CH | CH | 4,5-dihydroisoxazol-4-yl |
| E-0387 | F | CH | CMe | CH | CH | 4,5-dihydroisoxazol-5-yl |
| E-0388 | F | CH | CMe | CH | CH | oxetan-2-yl |
| E-0389 | F | CH | CMe | CH | CH | oxetan-3-yl |
| E-0390 | F | CH | CMe | CH | CH | azetidin-1-yl |
| E-0391 | F | CH | CMe | CH | CH | azetidin-2-yl |
| E-0392 | F | CH | CMe | CH | CH | azetidi n-2-yl(1-Me) |
| E-0393 | F | CH | CMe | CH | CH | azetidin-3-yl |
| E-0394 | F | CH | CMe | CH | CH | azetidi n-3-yl(1-Me) |
| E-0395 | F | CH | CMe | CH | CH | oxiran-2-yl |
| E-0396 | F | CH | CMe | CH | CH | 1,3,2-dioxaborolan-2-yl(4,4,5,5-$Me_4$) |
| E-0397 | Cl | CH | CH | CH | CH | pyridin-2-yl |
| E-0398 | Cl | CH | CH | CH | CH | pyridin-3-yl |
| E-0399 | Cl | CH | CH | CH | CH | pyridin-4-yl |
| E-0400 | Cl | CH | CH | CH | CH | pyrimidin-2-yl |
| E-0401 | Cl | CH | CH | CH | CH | pyrimidin-4-yl |

(continued)

| Compound Number | $R^1$ | $X^1$ | $X^2$ | $X^3$ | $X^1$ | Q |
|---|---|---|---|---|---|---|
| E-0402 | Cl | CH | CH | CH | CH | pyrimidin-5-yl |
| E-0403 | Cl | CH | CH | CH | CH | pyridazin-3-yl |
| E-0404 | Cl | CH | CH | CH | CH | pyridazin-4-yl |
| E-0405 | Cl | CH | CH | CH | CH | pyrazin-2-yl |
| E-0406 | Cl | CH | CH | CH | CH | 1,3,5-triazin-2-yl |
| E-0407 | Cl | CH | CH | CH | CH | thiophen-2-yl |
| E-0408 | Cl | CH | CH | CH | CH | thiophen-3-yl |
| E-0409 | Cl | CH | CH | CH | CH | furan-2-yl |
| E-0410 | Cl | CH | CH | CH | CH | furan-3-yl |
| E-0411 | Cl | CH | CH | CH | CH | 1H-pyrrol-1-yl |
| E-0412 | Cl | CH | CH | CH | CH | 1H-pyrrol-2-yl |
| E-0413 | Cl | CH | CH | CH | CH | 1H-pyrrol-2-yl(1-Me) |
| E-0414 | Cl | CH | CH | CH | CH | 1H-pyrrol-3-yl |

[Table 99]

| Compound Number | $R^1$ | $X^1$ | $X^2$ | $X^3$ | $X^4$ | Q |
|---|---|---|---|---|---|---|
| E-0415 | Cl | CH | CH | CH | CH | 1H-pyrrol-3-yl(1-Me) |
| E-0416 | Cl | CH | CH | CH | CH | 1H-pyrazol-1-yl |
| E-0417 | Cl | CH | CH | CH | CH | 1H-pyrazol-3-yl |
| E-0418 | Cl | CH | CH | CH | CH | 1H-pyrazol-3-yl(1-Me) |
| E-0419 | Cl | CH | CH | CH | CH | 1H-pyrazol-4-yl |
| E-0420 | Cl | CH | CH | CH | CH | 1H-pyrazol-4-yl(1-Me) |
| E-0421 | Cl | CH | CH | CH | CH | 1H-pyrazol-5-yl |
| E-0422 | Cl | CH | CH | CH | CH | 1H-pyrazol-5-yl(1-Me) |
| E-0423 | Cl | CH | CH | CH | CH | 1H-imidazol-1-yl |
| E-0424 | Cl | CH | CH | CH | CH | 1H-imidazol-2-yl |
| E-0425 | Cl | CH | CH | CH | CH | 1H-imidazol-2-yl(1-Me) |
| E-0426 | Cl | CH | CH | CH | CH | 1H-imidazol-4-yl |
| E-0427 | Cl | CH | CH | CH | CH | 1H-imidazol-4-yl(1-Me) |
| E-0428 | Cl | CH | CH | CH | CH | 1H-imidazol-5-yl |
| E-0429 | Cl | CH | CH | CH | CH | 1H-imidazol-5-yl(1-Me) |
| E-0430 | Cl | CH | CH | CH | CH | 1H-1,2,4-triazol-1-yl |
| E-0431 | Cl | CH | CH | CH | CH | 1H-1,2,4-triazol-3-yl |
| E-0432 | Cl | CH | CH | CH | CH | 1H-1,2,4-triazol-3-yl(1-Me) |
| E-0433 | Cl | CH | CH | CH | CH | 4H-1,2,4-triazol-4-yl |
| E-0434 | Cl | CH | CH | CH | CH | 1H-1,2,4-triazol-5-yl |
| E-0435 | Cl | CH | CH | CH | CH | 1H-1,2,4-triazol-5-yl(1-Me) |

(continued)

| Compound Number | $R^1$ | $X^1$ | $X^2$ | $X^3$ | $X^4$ | Q |
|---|---|---|---|---|---|---|
| E-0436 | Cl | CH | CH | CH | CH | 1H-1,2,3-triazol-1-yl |
| E-0437 | Cl | CH | CH | CH | CH | 2H-1,2,3-triazol-2-yl |
| E-0438 | Cl | CH | CH | CH | CH | 1H-tetrazol-1-yl |
| E-0439 | Cl | CH | CH | CH | CH | 2H-tetrazol-2-yl |
| E-0440 | Cl | CH | CH | CH | CH | 1H-tetrazol-5-yl |
| E-0441 | Cl | CH | CH | CH | CH | 1H-tetrazol-5-yl(1-Me) |
| E-0442 | Cl | CH | CH | CH | CH | thiazol-2-yl |
| E-0443 | Cl | CH | CH | CH | CH | thiazol-4-yl |
| E-0444 | Cl | CH | CH | CH | CH | thiazol-5-yl |
| E-0445 | Cl | CH | CH | CH | CH | isothiazol-3-yl |
| E-0446 | Cl | CH | CH | CH | CH | isothiazol-4-yl |
| E-0447 | Cl | CH | CH | CH | CH | isothiazol-5-yl |
| E-0448 | Cl | CH | CH | CH | CH | oxazol-2-yl |
| E-0449 | Cl | CH | CH | CH | CH | oxazol-4-yl |
| E-0450 | Cl | CH | CH | CH | CH | oxazol-5-yl |
| E-0451 | Cl | CH | CH | CH | CH | isoxazol-3-yl |
| E-0452 | Cl | CH | CH | CH | CH | isoxazol-4-yl |
| E-0453 | Cl | CH | CH | CH | CH | isoxazol-5-yl |
| E-0454 | Cl | CH | CH | CH | CH | 1,2,3-oxadiazol-4-yl |
| E-0455 | Cl | CH | CH | CH | CH | 1,2,3-oxadiazol-5-yl |
| E-0456 | Cl | CH | CH | CH | CH | 1,2,4-oxadiazol-3-yl |

[Table 100]

| Compound Number | $R^1$ | $X^1$ | $X^2$ | $X^3$ | $X^4$ | Q |
|---|---|---|---|---|---|---|
| E-0457 | Cl | CH | CH | CH | CH | 1,2,4-oxadiazol-5-yl |
| E-0458 | Cl | CH | CH | CH | CH | 1,3,4-oxadiazol-2-yl |
| E-0459 | Cl | CH | CH | CH | CH | 1,2,5-oxadiazol-3-yl |
| E-0460 | Cl | CH | CH | CH | CH | 1,2,3-thiadiazol-4-yl |
| E-0461 | Cl | CH | CH | CH | CH | 1,2,3-thiadiazol-5-yl |
| E-0462 | Cl | CH | CH | CH | CH | 1,2,4-thiadiazol-3-yl |
| E-0463 | Cl | CH | CH | CH | CH | 1,2,4-thiadiazol-5-yl |
| E-0464 | Cl | CH | CH | CH | CH | 1,3,4-thiadiazol-2-yl |
| E-0465 | Cl | CH | CH | CH | CH | 1,2,5-thiadiazol-3-yl |
| E-0466 | Cl | CH | CH | CH | CH | morpholin-4-yl |
| E-0467 | Cl | CH | CH | CH | CH | piperidin-4-yl |
| E-0468 | Cl | CH | CH | CH | CH | piperidin-4-yl(1-Me) |
| E-0469 | Cl | CH | CH | CH | CH | piperidin-1-yl |

(continued)

| Compound Number | $R^1$ | $X^1$ | $X^2$ | $X^3$ | $X^4$ | Q |
|---|---|---|---|---|---|---|
| E-0470 | Cl | CH | CH | CH | CH | piperazin-1-yl |
| E-0471 | Cl | CH | CH | CH | CH | piperazin-1-yl(4-Me) |
| E-0472 | Cl | CH | CH | CH | CH | tetrahydro-2H-pyran-4-yl |
| E-0473 | Cl | CH | CH | CH | CH | 1,3-dioxolan-2-yl |
| E-0474 | Cl | CH | CH | CH | CH | 1,3-dioxolan-2-yl(2-$CF_3$) |
| E-0475 | Cl | CH | CH | CH | CH | 1,3-dioxan-2-yl |
| E-0476 | Cl | CH | CH | CH | CH | 1,3-dioxan-2-yl(2-$CF_3$) |
| E-0477 | Cl | CH | CH | CH | CH | pyrrolidin-1-yl |
| E-0478 | Cl | CH | CH | CH | CH | pyrrolidin-2-yl |
| E-0479 | Cl | CH | CH | CH | CH | pyrrolidin-2-yl(1-Me) |
| E-0480 | Cl | CH | CH | CH | CH | pyrrolidin-3-yl |
| E-0481 | Cl | CH | CH | CH | CH | pyrrolidin-3-yl(1-Me) |
| E-0482 | Cl | CH | CH | CH | CH | tetrahydrofuran-2-yl |
| E-0483 | Cl | CH | CH | CH | CH | tetrahydrofuran-3-yl |
| E-0484 | Cl | CH | CH | CH | CH | 4,5-dihydroisoxazol-3-yl |
| E-0485 | Cl | CH | CH | CH | CH | 4,5-dihydroisoxazol-4-yl |
| E-0486 | Cl | CH | CH | CH | CH | 4,5-dihydroisoxazol-5-yl |
| E-0487 | Cl | CH | CH | CH | CH | oxetan-2-yl |
| E-0488 | Cl | CH | CH | CH | CH | oxetan-3-yl |
| E-0489 | Cl | CH | CH | CH | CH | azetidin-1-yl |
| E-0490 | Cl | CH | CH | CH | CH | azetidin-2-yl |
| E-0491 | Cl | CH | CH | CH | CH | azetidin-2-yl(1-Me) |
| E-0492 | Cl | CH | CH | CH | CH | azetidin-3-yl |
| E-0493 | Cl | CH | CH | CH | CH | azetidin-3-yl(1-Me) |
| E-0494 | Cl | CH | CH | CH | CH | oxiran-2-yl |
| E-0495 | Cl | CH | CH | CH | CH | 1,3,2-dioxaborolan-2-yl(4,4,5,5-$Me_4$) |
| E-0496 | F | N | CH | CH | CH | pyridin-2-yl |
| E-0497 | F | N | CH | CH | CH | pyridin-3-yl |
| E-0498 | F | N | CH | CH | CH | pyridin-4-yl |

[Table 101]

| Compound Number | $R^1$ | $X^1$ | $X^2$ | $X^3$ | $X^4$ | Q |
|---|---|---|---|---|---|---|
| E-0499 | F | N | CH | CH | CH | pyrimidin-2-yl |
| E-0500 | F | N | CH | CH | CH | pyrimidin-4-yl |
| E-0501 | F | N | CH | CH | CH | pyrimidin-5-yl |
| E-0502 | F | N | CH | CH | CH | pyridazin-3-yl |
| E-0503 | F | N | CH | CH | CH | pyridazin-4-yl |

(continued)

| Compound Number | $R^1$ | $X^1$ | $X^2$ | $X^3$ | $X^4$ | Q |
|---|---|---|---|---|---|---|
| E-0504 | F | N | CH | CH | CH | pyrazin-2-yl |
| E-0505 | F | N | CH | CH | CH | 1,3,5-triazin-2-yl |
| E-0506 | F | N | CH | CH | CH | thiophen-2-yl |
| E-0507 | F | N | CH | CH | CH | thiophen-3-yl |
| E-0508 | F | N | CH | CH | CH | furan-2-yl |
| E-0509 | F | N | CH | CH | CH | furan-3-yl |
| E-0510 | F | N | CH | CH | CH | 1H-pyrrol-1-yl |
| E-0511 | F | N | CH | CH | CH | 1H-pyrrol-2-yl |
| E-0512 | F | N | CH | CH | CH | 1H-pyrrol-2-yl(1-Me) |
| E-0513 | F | N | CH | CH | CH | 1H-pyrrol-3-yl |
| E-0514 | F | N | CH | CH | CH | 1H-pyrrol-3-yl(1-Me) |
| E-0515 | F | N | CH | CH | CH | 1H-pyrazol-1-yl |
| E-0516 | F | N | CH | CH | CH | 1H-pyrazol-3-yl |
| E-0517 | F | N | CH | CH | CH | 1H-pyrazol-3-yl(1-Me) |
| E-0518 | F | N | CH | CH | CH | 1H-pyrazol-4-yl |
| E-0519 | F | N | CH | CH | CH | 1H-pyrazol-4-yl(1-Me) |
| E-0520 | F | N | CH | CH | CH | 1H-pyrazol-5-yl |
| E-0521 | F | N | CH | CH | CH | 1H-pyrazol-5-yl(1-Me) |
| E-0522 | F | N | CH | CH | CH | 1H-imidazol-1-yl |
| E-0523 | F | N | CH | CH | CH | 1H-imidazol-2-yl |
| E-0524 | F | N | CH | CH | CH | 1H-imidazol-2-yl(1-Me) |
| E-0525 | F | N | CH | CH | CH | 1H-imidazol-4-yl |
| E-0526 | F | N | CH | CH | CH | 1H-imidazol-4-yl(1-Me) |
| E-0527 | F | N | CH | CH | CH | 1H-imidazol-5-yl |
| E-0528 | F | N | CH | CH | CH | 1H-imidazol-5-yl(1-Me) |
| E-0529 | F | N | CH | CH | CH | 1H-1,2,4-triazol-1-yl |
| E-0530 | F | N | CH | CH | CH | 1H-1,2,4-triazol-3-yl |
| E-0531 | F | N | CH | CH | CH | 1H-1,2,4-triazol-3-yl(1-Me) |
| E-0532 | F | N | CH | CH | CH | 4H-1,2,4-triazol-4-yl |
| E-0533 | F | N | CH | CH | CH | 1H-1,2,4-triazol-5-yl |
| E-0534 | F | N | CH | CH | CH | 1H-1,2,4-triazol-5-yl(1-Me) |
| E-0535 | F | N | CH | CH | CH | 1H-1,2,3-triazol-1-yl |
| E-0536 | F | N | CH | CH | CH | 2H-1,2,3-triazol-2-yl |
| E-0537 | F | N | CH | CH | CH | 1H-tetrazol-1-yl |
| E-0538 | F | N | CH | CH | CH | 2H-tetrazol-2-yl |
| E-0539 | F | N | CH | CH | CH | 1H-tetrazol-5-yl |
| E-0540 | F | N | CH | CH | CH | 1H-tetrazol-5-yl(1-Me) |

[Table 102]

| Compound Number | $R^1$ | $X^1$ | $X^2$ | $X^3$ | $X^4$ | Q |
|---|---|---|---|---|---|---|
| E-0541 | F | N | CH | CH | CH | thiazol-2-yl |
| E-0542 | F | N | CH | CH | CH | thiazol-4-yl |
| E-0543 | F | N | CH | CH | CH | thiazol-5-yl |
| E-0544 | F | N | CH | CH | CH | isothiazol-3-yl |
| E-0545 | F | N | CH | CH | CH | isothiazol-4-yl |
| E-0546 | F | N | CH | CH | CH | isothiazol-5-yl |
| E-0547 | F | N | CH | CH | CH | oxazol-2-yl |
| E-0548 | F | N | CH | CH | CH | oxazol-4-yl |
| E-0549 | F | N | CH | CH | CH | oxazol-5-yl |
| E-0550 | F | N | CH | CH | CH | isoxazol-3-yl |
| E-0551 | F | N | CH | CH | CH | isoxazol-4-yl |
| E-0552 | F | N | CH | CH | CH | isoxazol-5-yl |
| E-0553 | F | N | CH | CH | CH | 1,2,3-oxadiazol-4-yl |
| E-0554 | F | N | CH | CH | CH | 1,2,3-oxadiazol-5-yl |
| E-0555 | F | N | CH | CH | CH | 1,2,4-oxadiazol-3-yl |
| E-0556 | F | N | CH | CH | CH | 1,2,4-oxadiazol-5-yl |
| E-0557 | F | N | CH | CH | CH | 1,3,4-oxadiazol-2-yl |
| E-0558 | F | N | CH | CH | CH | 1,2,5-oxadiazol-3-yl |
| E-0559 | F | N | CH | CH | CH | 1,2,3-thiadiazol-4-yl |
| E-0560 | F | N | CH | CH | CH | 1,2,3-thiadiazol-5-yl |
| E-0561 | F | N | CH | CH | CH | 1,2,4-thiadiazol-3-yl |
| E-0562 | F | N | CH | CH | CH | 1,2,4-thiadiazol-5-yl |
| E-0563 | F | N | CH | CH | CH | 1,3,4-thiadiazol-2-yl |
| E-0564 | F | N | CH | CH | CH | 1,2,5-thiadiazol-3-yl |
| E-0565 | F | N | CH | CH | CH | morpholin-4-yl |
| E-0566 | F | N | CH | CH | CH | piperidin-4-yl |
| E-0567 | F | N | CH | CH | CH | piperidin-4-yl(1-Me) |
| E-0568 | F | N | CH | CH | CH | piperidin-1-yl |
| E-0569 | F | N | CH | CH | CH | piperazin-1-yl |
| E-0570 | F | N | CH | CH | CH | piperazin-1-yl(4-Me) |
| E-0571 | F | N | CH | CH | CH | tetrahydro-2H-pyran-4-yl |
| E-0572 | F | N | CH | CH | CH | 1,3-dioxolan-2-yl |
| E-0573 | F | N | CH | CH | CH | 1,3-dioxolan-2-yl(2-$CF_3$) |
| E-0574 | F | N | CH | CH | CH | 1,3-dioxan-2-yl |
| E-0575 | F | N | CH | CH | CH | 1,3-dioxan-2-yl(2-$CF_3$) |
| E-0576 | F | N | CH | CH | CH | pyrrolidin-1-yl |
| E-0577 | F | N | CH | CH | CH | pyrrolidin-2-yl |

(continued)

| Compound Number | $R^1$ | $X^1$ | $X^2$ | $X^3$ | $X^4$ | Q |
|---|---|---|---|---|---|---|
| E-0578 | F | N | CH | CH | CH | pyrrolidin-2-yl(1-Me) |
| E-0579 | F | N | CH | CH | CH | pyrrolidin-3-yl |
| E-0580 | F | N | CH | CH | CH | pyrrolidin-3-yl(1-Me) |
| E-0581 | F | N | CH | CH | CH | tetrahydrofuran-2-yl |
| E-0582 | F | N | CH | CH | CH | tetrahydrofuran-3-yl |

[Table 103]

| Compound Number | $R^1$ | $X^1$ | $X^2$ | $X^3$ | $X^4$ | Q |
|---|---|---|---|---|---|---|
| E-0583 | F | N | CH | CH | CH | 4,5-dihydroisoxazol-3-yl |
| E-0584 | F | N | CH | CH | CH | 4,5-dihydroisoxazol-4-yl |
| E-0585 | F | N | CH | CH | CH | 4,5-dihydroisoxazol-5-yl |
| E-0586 | F | N | CH | CH | CH | oxetan-2-yl |
| E-0587 | F | N | CH | CH | CH | oxetan-3-yl |
| E-0588 | F | N | CH | CH | CH | azetidin-1-yl |
| E-0589 | F | N | CH | CH | CH | azetidin-2-yl |
| E-0590 | F | N | CH | CH | CH | azetidi n-2-yl(1-Me) |
| E-0591 | F | N | CH | CH | CH | azetidin-3-yl |
| E-0592 | F | N | CH | CH | CH | azetidi n-3-yl(1-Me) |
| E-0593 | F | N | CH | CH | CH | oxiran-2-yl |
| E-0594 | F | N | CH | CH | CH | 1,3,2-dioxaborolan-2-yl(4,4,5,5-$Me_4$) |
| E-0595 | F | CH | N | CH | CH | pyridin-2-yl |
| E-0596 | F | CH | N | CH | CH | pyridin-3-yl |
| E-0597 | F | CH | N | CH | CH | pyridin-4-yl |
| E-0598 | F | CH | N | CH | CH | pyrimidin-2-yl |
| E-0599 | F | CH | N | CH | CH | pyrimidin-4-yl |
| E-0600 | F | CH | N | CH | CH | pyrimidin-5-yl |
| E-0601 | F | CH | N | CH | CH | pyridazin-3-yl |
| E-0602 | F | CH | N | CH | CH | pyridazin-4-yl |
| E-0603 | F | CH | N | CH | CH | pyrazin-2-yl |
| E-0604 | F | CH | N | CH | CH | 1,3,5-triazin-2-yl |
| E-0605 | F | CH | N | CH | CH | thiophen-2-yl |
| E-0606 | F | CH | N | CH | CH | thiophen-3-yl |
| E-0607 | F | CH | N | CH | CH | furan-2-yl |
| E-0608 | F | CH | N | CH | CH | furan-3-yl |
| E-0609 | F | CH | N | CH | CH | 1H-pyrrol-1-yl |
| E-0610 | F | CH | N | CH | CH | 1H-pyrrol-2-yl |
| E-0611 | F | CH | N | CH | CH | 1H-pyrrol-2-yl(1-Me) |

(continued)

| Compound Number | $R^1$ | $X^1$ | $X^2$ | $X^3$ | $X^4$ | Q |
|---|---|---|---|---|---|---|
| E-0612 | F | CH | N | CH | CH | 1H-pyrrol-3-yl |
| E-0613 | F | CH | N | CH | CH | 1H-pyrrol-3-yl(1-Me) |
| E-0614 | F | CH | N | CH | CH | 1H-pyrazol-1-yl |
| E-0615 | F | CH | N | CH | CH | 1H-pyrazol-3-yl |
| E-0616 | F | CH | N | CH | CH | 1H-pyrazol-3-yl(1-Me) |
| E-0617 | F | CH | N | CH | CH | 1H-pyrazol-4-yl |
| E-0618 | F | CH | N | CH | CH | 1H-pyrazol-4-yl(1-Me) |
| E-0619 | F | CH | N | CH | CH | 1H-pyrazol-5-yl |
| E-0620 | F | CH | N | CH | CH | 1H-pyrazol-5-yl(1-Me) |
| E-0621 | F | CH | N | CH | CH | 1H-imidazol-1-yl |
| E-0622 | F | CH | N | CH | CH | 1H-imidazol-2-yl |
| E-0623 | F | CH | N | CH | CH | 1H-imidazol-2-yl(1-Me) |
| E-0624 | F | CH | N | CH | CH | 1H-imidazol-4-yl |

[Table 104]

| Compound Number | $R^1$ | $X^1$ | $X^2$ | $X^3$ | $X^4$ | Q |
|---|---|---|---|---|---|---|
| E-0625 | F | CH | N | CH | CH | 1H-imidazol-4-yl(1-Me) |
| E-0626 | F | CH | N | CH | CH | 1H-imidazol-5-yl |
| E-0627 | F | CH | N | CH | CH | 1H-imidazol-5-yl(1-Me) |
| E-0628 | F | CH | N | CH | CH | 1H-1,2,4-triazol-1-yl |
| E-0629 | F | CH | N | CH | CH | 1H-1,2,4-triazol-3-yl |
| E-0630 | F | CH | N | CH | CH | 1H-1,2,4-triazol-3-yl(1-Me) |
| E-0631 | F | CH | N | CH | CH | 4H-1,2,4-triazol-4-yl |
| E-0632 | F | CH | N | CH | CH | 1H-1,2,4-triazol-5-yl |
| E-0633 | F | CH | N | CH | CH | 1H-1,2,4-triazol-5-yl(1-Me) |
| E-0634 | F | CH | N | CH | CH | 1H-1,2,3-triazol-1-yl |
| E-0635 | F | CH | N | CH | CH | 2H-1,2,3-triazol-2-yl |
| E-0636 | F | CH | N | CH | CH | 1H-tetrazol-1-yl |
| E-0637 | F | CH | N | CH | CH | 2H-tetrazol-2-yl |
| E-0638 | F | CH | N | CH | CH | 1H-tetrazol-5-yl |
| E-0639 | F | CH | N | CH | CH | 1H-tetrazol-5-yl(1-Me) |
| E-0640 | F | CH | N | CH | CH | thiazol-2-yl |
| E-0641 | F | CH | N | CH | CH | thiazol-4-yl |
| E-0642 | F | CH | N | CH | CH | thiazol-5-yl |
| E-0643 | F | CH | N | CH | CH | isothiazol-3-yl |
| E-0644 | F | CH | N | CH | CH | isothiazol-4-yl |
| E-0645 | F | CH | N | CH | CH | isothiazol-5-yl |

(continued)

| Compound Number | $R^1$ | $X^1$ | $X^2$ | $X^3$ | $X^4$ | Q |
|---|---|---|---|---|---|---|
| E-0646 | F | CH | N | CH | CH | oxazol-2-yl |
| E-0647 | F | CH | N | CH | CH | oxazol-4-yl |
| E-0648 | F | CH | N | CH | CH | oxazol-5-yl |
| E-0649 | F | CH | N | CH | CH | isoxazol-3-yl |
| E-0650 | F | CH | N | CH | CH | isoxazol-4-yl |
| E-0651 | F | CH | N | CH | CH | isoxazol-5-yl |
| E-0652 | F | CH | N | CH | CH | 1,2,3-oxadiazol-4-yl |
| E-0653 | F | CH | N | CH | CH | 1,2,3-oxadiazol-5-yl |
| E-0654 | F | CH | N | CH | CH | 1,2,4-oxadiazol-3-yl |
| E-0655 | F | CH | N | CH | CH | 1,2,4-oxadiazol-5-yl |
| E-0656 | F | CH | N | CH | CH | 1,3,4-oxadiazol-2-yl |
| E-0657 | F | CH | N | CH | CH | 1,2,5-oxadiazol-3-yl |
| E-0658 | F | CH | N | CH | CH | 1,2,3-thiadiazol-4-yl |
| E-0659 | F | CH | N | CH | CH | 1,2,3-thiadiazol-5-yl |
| E-0660 | F | CH | N | CH | CH | 1,2,4-thiadiazol-3-yl |
| E-0661 | F | CH | N | CH | CH | 1,2,4-thiadiazol-5-yl |
| E-0662 | F | CH | N | CH | CH | 1,3,4-thiadiazol-2-yl |
| E-0663 | F | CH | N | CH | CH | 1,2,5-thiadiazol-3-yl |
| E-0664 | F | CH | N | CH | CH | morpholin-4-yl |
| E-0665 | F | CH | N | CH | CH | piperidin-4-yl |
| E-0666 | F | CH | N | CH | CH | piperidin-4-yl(1-Me) |

[Table 105]

| Compound Number | $R^1$ | $X^1$ | $X^2$ | $X^3$ | $X^4$ | Q |
|---|---|---|---|---|---|---|
| E-0667 | F | CH | N | CH | CH | piperidin-1-yl |
| E-0668 | F | CH | N | CH | CH | piperazin-1-yl |
| E-0669 | F | CH | N | CH | CH | piperazin-1-yl(4-Me) |
| E-0670 | F | CH | N | CH | CH | tetrahydro-2H-pyran-4-yl |
| E-0671 | F | CH | N | CH | CH | 1,3-dioxolan-2-yl |
| E-0672 | F | CH | N | CH | CH | 1,3-dioxolan-2-yl(2-$CF_3$) |
| E-0673 | F | CH | N | CH | CH | 1,3-dioxan-2-yl |
| E-0674 | F | CH | N | CH | CH | 1,3-dioxan-2-yl(2-$CF_3$) |
| E-0675 | F | CH | N | CH | CH | pyrrolidin-1-yl |
| E-0676 | F | CH | N | CH | CH | pyrrolidin-2-yl |
| E-0677 | F | CH | N | CH | CH | pyrrolidin-2-yl(1-Me) |
| E-0678 | F | CH | N | CH | CH | pyrrolidin-3-yl |
| E-0679 | F | CH | N | CH | CH | pyrrolidin-3-yl(1-Me) |

(continued)

| Compound Number | $R^1$ | $X^1$ | $X^2$ | $X^3$ | $X^4$ | Q |
|---|---|---|---|---|---|---|
| E-0680 | F | CH | N | CH | CH | tetrahydrofuran-2-yl |
| E-0681 | F | CH | N | CH | CH | tetrahydrofuran-3-yl |
| E-0682 | F | CH | N | CH | CH | 4,5-dihydroisoxazol-3-yl |
| E-0683 | F | CH | N | CH | CH | 4,5-dihydroisoxazol-4-yl |
| E-0684 | F | CH | N | CH | CH | 4,5-dihydroisoxazol-5-yl |
| E-0685 | F | CH | N | CH | CH | oxetan-2-yl |
| E-0686 | F | CH | N | CH | CH | oxetan-3-yl |
| E-0687 | F | CH | N | CH | CH | azetidin-1-yl |
| E-0688 | F | CH | N | CH | CH | azetidin-2-yl |
| E-0689 | F | CH | N | CH | CH | azetidin-2-yl(1-Me) |
| E-0690 | F | CH | N | CH | CH | azetidin-3-yl |
| E-0691 | F | CH | N | CH | CH | azetidin-3-yl(1-Me) |
| E-0692 | F | CH | N | CH | CH | oxiran-2-yl |
| E-0693 | F | CH | N | CH | CH | 1,3,2-dioxaborolan-2-yl(4,4,5,5-$Me_4$) |
| E-0694 | F | N | N | CH | CH | pyridin-2-yl |
| E-0695 | F | N | N | CH | CH | pyridin-3-yl |
| E-0696 | F | N | N | CH | CH | pyridin-4-yl |
| E-0697 | F | N | N | CH | CH | pyrimidin-2-yl |
| E-0698 | F | N | N | CH | CH | pyrimidin-4-yl |
| E-0699 | F | N | N | CH | CH | pyrimidin-5-yl |
| E-0700 | F | N | N | CH | CH | pyridazin-3-yl |
| E-0701 | F | N | N | CH | CH | pyridazin-4-yl |
| E-0702 | F | N | N | CH | CH | pyrazin-2-yl |
| E-0703 | F | N | N | CH | CH | 1,3,5-triazin-2-yl |
| E-0704 | F | N | N | CH | CH | thiophen-2-yl |
| E-0705 | F | N | N | CH | CH | thiophen-3-yl |
| E-0706 | F | N | N | CH | CH | furan-2-yl |
| E-0707 | F | N | N | CH | CH | furan-3-yl |
| E-0708 | F | N | N | CH | CH | 1H-pyrrol-1-yl |

[Table 106]

| Compound Number | $R^1$ | $X^1$ | $X^2$ | $X^3$ | $X^4$ | Q |
|---|---|---|---|---|---|---|
| E-0709 | F | N | N | CH | CH | 1H-pyrrol-2-yl |
| E-0710 | F | N | N | CH | CH | 1H-pyrrol-2-yl(1-Me) |
| E-0711 | F | N | N | CH | CH | 1H-pyrrol-3-yl |
| E-0712 | F | N | N | CH | CH | 1H-pyrrol-3-yl(1-Me) |
| E-0713 | F | N | N | CH | CH | 1H-pyrazol-1-yl |

(continued)

| Compound Number | $R^1$ | $X^1$ | $X^2$ | $X^3$ | $X^4$ | Q |
|---|---|---|---|---|---|---|
| E-0714 | F | N | N | CH | CH | 1H-pyrazol-3-yl |
| E-0715 | F | N | N | CH | CH | 1H-pyrazol-3-yl(1-Me) |
| E-0716 | F | N | N | CH | CH | 1H-pyrazol-4-yl |
| E-0717 | F | N | N | CH | CH | 1H-pyrazol-4-yl(1-Me) |
| E-0718 | F | N | N | CH | CH | 1H-pyrazol-5-yl |
| E-0719 | F | N | N | CH | CH | 1H-pyrazol-5-yl(1-Me) |
| E-0720 | F | N | N | CH | CH | 1H-imidazol-1-yl |
| E-0721 | F | N | N | CH | CH | 1H-imidazol-2-yl |
| E-0722 | F | N | N | CH | CH | 1H-imidazol-2-yl(1-Me) |
| E-0723 | F | N | N | CH | CH | 1H-imidazol-4-yl |
| E-0724 | F | N | N | CH | CH | 1H-imidazol-4-yl(1-Me) |
| E-0725 | F | N | N | CH | CH | 1H-imidazol-5-yl |
| E-0726 | F | N | N | CH | CH | 1H-imidazol-5-yl(1-Me) |
| E-0727 | F | N | N | CH | CH | 1H-1,2,4-triazol-1-yl |
| E-0728 | F | N | N | CH | CH | 1H-1,2,4-triazol-3-yl |
| E-0729 | F | N | N | CH | CH | 1H-1,2,4-triazol-3-yl(1-Me) |
| E-0730 | F | N | N | CH | CH | 4H-1,2,4-triazol-4-yl |
| E-0731 | F | N | N | CH | CH | 1H-1,2,4-triazol-5-yl |
| E-0732 | F | N | N | CH | CH | 1H-1,2,4-triazol-5-yl(1-Me) |
| E-0733 | F | N | N | CH | CH | 1H-1,2,3-triazol-1-yl |
| E-0734 | F | N | N | CH | CH | 2H-1,2,3-triazol-2-yl |
| E-0735 | F | N | N | CH | CH | 1H-tetrazol-1-yl |
| E-0736 | F | N | N | CH | CH | 2H-tetrazol-2-yl |
| E-0737 | F | N | N | CH | CH | 1H-tetrazol-5-yl |
| E-0738 | F | N | N | CH | CH | 1H-tetrazol-5-yl(1-Me) |
| E-0739 | F | N | N | CH | CH | thiazol-2-yl |
| E-0740 | F | N | N | CH | CH | thiazol-4-yl |
| E-0741 | F | N | N | CH | CH | thiazol-5-yl |
| E-0742 | F | N | N | CH | CH | isothiazol-3-yl |
| E-0743 | F | N | N | CH | CH | isothiazol-4-yl |
| E-0744 | F | N | N | CH | CH | isothiazol-5-yl |
| E-0745 | F | N | N | CH | CH | oxazol-2-yl |
| E-0746 | F | N | N | CH | CH | oxazol-4-yl |
| E-0747 | F | N | N | CH | CH | oxazol-5-yl |
| E-0748 | F | N | N | CH | CH | isoxazol-3-yl |
| E-0749 | F | N | N | CH | CH | isoxazol-4-yl |
| E-0750 | F | N | N | CH | CH | isoxazol-5-yl |

[Table 107]

| Compound Number | $R^1$ | $X^1$ | $X^2$ | $X^3$ | $X^4$ | Q |
|---|---|---|---|---|---|---|
| E-0751 | F | N | N | CH | CH | 1,2,3-oxadiazol-4-yl |
| E-0752 | F | N | N | CH | CH | 1,2,3-oxadiazol-5-yl |
| E-0753 | F | N | N | CH | CH | 1,2,4-oxadiazol-3-yl |
| E-0754 | F | N | N | CH | CH | 1,2,4-oxadiazol-5-yl |
| E-0755 | F | N | N | CH | CH | 1,3,4-oxadiazol-2-yl |
| E-0756 | F | N | N | CH | CH | 1,2,5-oxadiazol-3-yl |
| E-0757 | F | N | N | CH | CH | 1,2,3-thiadiazol-4-yl |
| E-0758 | F | N | N | CH | CH | 1,2,3-thiadiazol-5-yl |
| E-0759 | F | N | N | CH | CH | 1,2,4-thiadiazol-3-yl |
| E-0760 | F | N | N | CH | CH | 1,2,4-thiadiazol-5-yl |
| E-0761 | F | N | N | CH | CH | 1,3,4-thiadiazol-2-yl |
| E-0762 | F | N | N | CH | CH | 1,2,5-thiadiazol-3-yl |
| E-0763 | F | N | N | CH | CH | morpholin-4-yl |
| E-0764 | F | N | N | CH | CH | piperidin-4-yl |
| E-0765 | F | N | N | CH | CH | piperidin-4-yl(1-Me) |
| E-0766 | F | N | N | CH | CH | piperidin-1-yl |
| E-0767 | F | N | N | CH | CH | piperazin-1-yl |
| E-0768 | F | N | N | CH | CH | piperazin-1-yl(4-Me) |
| E-0769 | F | N | N | CH | CH | tetrahydro-2H-pyran-4-yl |
| E-0770 | F | N | N | CH | CH | 1,3-dioxolan-2-yl |
| E-0771 | F | N | N | CH | CH | 1,3-dioxolan-2-yl(2-$CF_3$) |
| E-0772 | F | N | N | CH | CH | 1,3-dioxan-2-yl |
| E-0773 | F | N | N | CH | CH | 1,3-dioxan-2-yl(2-$CF_3$) |
| E-0774 | F | N | N | CH | CH | pyrrolidin-1-yl |
| E-0775 | F | N | N | CH | CH | pyrrolidin-2-yl |
| E-0776 | F | N | N | CH | CH | pyrrolidin-2-yl(1-Me) |
| E-0777 | F | N | N | CH | CH | pyrrolidin-3-yl |
| E-0778 | F | N | N | CH | CH | pyrrolidin-3-yl(1-Me) |
| E-0779 | F | N | N | CH | CH | tetrahydrofuran-2-yl |
| E-0780 | F | N | N | CH | CH | tetrahydrofuran-3-yl |
| E-0781 | F | N | N | CH | CH | 4,5-dihydroisoxazol-3-yl |
| E-0782 | F | N | N | CH | CH | 4,5-dihydroisoxazol-4-yl |
| E-0783 | F | N | N | CH | CH | 4,5-dihydroisoxazol-5-yl |
| E-0784 | F | N | N | CH | CH | oxetan-2-yl |
| E-0785 | F | N | N | CH | CH | oxetan-3-yl |
| E-0786 | F | N | N | CH | CH | azetidin-1-yl |
| E-0787 | F | N | N | CH | CH | azetidin-2-yl |

(continued)

| Compound Number | $R^1$ | $X^1$ | $X^2$ | $X^3$ | $X^4$ | Q |
|---|---|---|---|---|---|---|
| E-0788 | F | N | N | CH | CH | azetidin-2-yl(1-Me) |
| E-0789 | F | N | N | CH | CH | azetidin-3-yl |
| E-0790 | F | N | N | CH | CH | azetidin-3-yl(1-Me) |
| E-0791 | F | N | N | CH | CH | oxiran-2-yl |
| E-0792 | F | N | N | CH | CH | 1,3,2-dioxaborolan-2-yl(4,4,5,5-$Me_4$) |

[Table 108]

| Compound Number | $R^1$ | $X^1$ | $X^2$ | $X^3$ | $X^4$ | Q |
|---|---|---|---|---|---|---|
| E-0793 | F | N | CH | N | CH | pyridin-2-yl |
| E-0794 | F | N | CH | N | CH | pyridin-3-yl |
| E-0795 | F | N | CH | N | CH | pyridin-4-yl |
| E-0796 | F | N | CH | N | CH | pyrimidin-2-yl |
| E-0797 | F | N | CH | N | CH | pyrimidin-4-yl |
| E-0798 | F | N | CH | N | CH | pyrimidin-5-yl |
| E-0799 | F | N | CH | N | CH | pyridazin-3-yl |
| E-0800 | F | N | CH | N | CH | pyridazin-4-yl |
| E-0801 | F | N | CH | N | CH | pyrazin-2-yl |
| E-0802 | F | N | CH | N | CH | 1,3,5-triazin-2-yl |
| E-0803 | F | N | CH | N | CH | thiophen-2-yl |
| E-0804 | F | N | CH | N | CH | thiophen-3-yl |
| E-0805 | F | N | CH | N | CH | furan-2-yl |
| E-0806 | F | N | CH | N | CH | furan-3-yl |
| E-0807 | F | N | CH | N | CH | 1H-pyrrol-1-yl |
| E-0808 | F | N | CH | N | CH | 1H-pyrrol-2-yl |
| E-0809 | F | N | CH | N | CH | 1H-pyrrol-2-yl(1-Me) |
| E-0810 | F | N | CH | N | CH | 1H-pyrrol-3-yl |
| E-0811 | F | N | CH | N | CH | 1H-pyrrol-3-yl(1-Me) |
| E-0812 | F | N | CH | N | CH | 1H-pyrazol-1-yl |
| E-0813 | F | N | CH | N | CH | 1H-pyrazol-3-yl |
| E-0814 | F | N | CH | N | CH | 1H-pyrazol-3-yl(1-Me) |
| E-0815 | F | N | CH | N | CH | 1H-pyrazol-4-yl |
| E-0816 | F | N | CH | N | CH | 1H-pyrazol-4-yl(1-Me) |
| E-0817 | F | N | CH | N | CH | 1H-pyrazol-5-yl |
| E-0818 | F | N | CH | N | CH | 1H-pyrazol-5-yl(1-Me) |
| E-0819 | F | N | CH | N | CH | 1H-imidazol-1-yl |
| E-0820 | F | N | CH | N | CH | 1H-imidazol-2-yl |
| E-0821 | F | N | CH | N | CH | 1H-imidazol-2-yl(1-Me) |

(continued)

| Compound Number | $R^1$ | $X^1$ | $X^2$ | $X^3$ | $X^4$ | Q |
|---|---|---|---|---|---|---|
| E-0822 | F | N | CH | N | CH | 1H-imidazol-4-yl |
| E-0823 | F | N | CH | N | CH | 1H-imidazol-4-yl(1-Me) |
| E-0824 | F | N | CH | N | CH | 1H-imidazol-5-yl |
| E-0825 | F | N | CH | N | CH | 1H-imidazol-5-yl(1-Me) |
| E-0826 | F | N | CH | N | CH | 1H-1,2,4-triazol-1-yl |
| E-0827 | F | N | CH | N | CH | 1H-1,2,4-triazol-3-yl |
| E-0828 | F | N | CH | N | CH | 1H-1,2,4-triazol-3-yl(1-Me) |
| E-0829 | F | N | CH | N | CH | 4H-1,2,4-triazol-4-yl |
| E-0830 | F | N | CH | N | CH | 1H-1,2,4-triazol-5-yl |
| E-0831 | F | N | CH | N | CH | 1H-1,2,4-triazol-5-yl(1-Me) |
| E-0832 | F | N | CH | N | CH | 1H-1,2,3-triazol-1-yl |
| E-0833 | F | N | CH | N | CH | 2H-1,2,3-triazol-2-yl |
| E-0834 | F | N | CH | N | CH | 1H-tetrazol-1-yl |

[Table 109]

| Compound Number | $R^1$ | $X^1$ | $X^2$ | $X^3$ | $X^4$ | Q |
|---|---|---|---|---|---|---|
| E-0835 | F | N | CH | N | CH | 2H-tetrazol-2-yl |
| E-0836 | F | N | CH | N | CH | 1H-tetrazol-5-yl |
| E-0837 | F | N | CH | N | CH | 1H-tetrazol-5-yl(1-Me) |
| E-0838 | F | N | CH | N | CH | thiazol-2-yl |
| E-0839 | F | N | CH | N | CH | thiazol-4-yl |
| E-0840 | F | N | CH | N | CH | thiazol-5-yl |
| E-0841 | F | N | CH | N | CH | isothiazol-3-yl |
| E-0842 | F | N | CH | N | CH | isothiazol-4-yl |
| E-0843 | F | N | CH | N | CH | isothiazol-5-yl |
| E-0844 | F | N | CH | N | CH | oxazol-2-yl |
| E-0845 | F | N | CH | N | CH | oxazol-4-yl |
| E-0846 | F | N | CH | N | CH | oxazol-5-yl |
| E-0847 | F | N | CH | N | CH | isoxazol-3-yl |
| E-0848 | F | N | CH | N | CH | isoxazol-4-yl |
| E-0849 | F | N | CH | N | CH | isoxazol-5-yl |
| E-0850 | F | N | CH | N | CH | 1,2,3-oxadiazol-4-yl |
| E-0851 | F | N | CH | N | CH | 1,2,3-oxadiazol-5-yl |
| E-0852 | F | N | CH | N | CH | 1,2,4-oxadiazol-3-yl |
| E-0853 | F | N | CH | N | CH | 1,2,4-oxadiazol-5-yl |
| E-0854 | F | N | CH | N | CH | 1,3,4-oxadiazol-2-yl |
| E-0855 | F | N | CH | N | CH | 1,2,5-oxadiazol-3-yl |

(continued)

| Compound Number | $R^1$ | $X^1$ | $X^2$ | $X^3$ | $X^4$ | Q |
|---|---|---|---|---|---|---|
| E-0856 | F | N | CH | N | CH | 1,2,3-thiadiazol-4-yl |
| E-0857 | F | N | CH | N | CH | 1,2,3-thiadiazol-5-yl |
| E-0858 | F | N | CH | N | CH | 1,2,4-thiadiazol-3-yl |
| E-0859 | F | N | CH | N | CH | 1,2,4-thiadiazol-5-yl |
| E-0860 | F | N | CH | N | CH | 1,3,4-thiadiazol-2-yl |
| E-0861 | F | N | CH | N | CH | 1,2,5-thiadiazol-3-yl |
| E-0862 | F | N | CH | N | CH | morpholin-4-yl |
| E-0863 | F | N | CH | N | CH | piperidin-4-yl |
| E-0864 | F | N | CH | N | CH | piperidin-4-yl(1-Me) |
| E-0865 | F | N | CH | N | CH | piperidin-1-yl |
| E-0866 | F | N | CH | N | CH | piperazin-1-yl |
| E-0867 | F | N | CH | N | CH | piperazin-1-yl(4-Me) |
| E-0868 | F | N | CH | N | CH | tetrahydro-2H-pyran-4-yl |
| E-0869 | F | N | CH | N | CH | 1,3-dioxolan-2-yl |
| E-0870 | F | N | CH | N | CH | 1,3-dioxolan-2-yl(2-$CF_3$) |
| E-0871 | F | N | CH | N | CH | 1,3-dioxan-2-yl |
| E-0872 | F | N | CH | N | CH | 1,3-dioxan-2-yl(2-$CF_3$) |
| E-0873 | F | N | CH | N | CH | pyrrolidin-1-yl |
| E-0874 | F | N | CH | N | CH | pyrrolidin-2-yl |
| E-0875 | F | N | CH | N | CH | pyrrolidin-2-yl(1-Me) |
| E-0876 | F | N | CH | N | CH | pyrrolidin-3-yl |

[Table 110]

| Compound Number | $R^1$ | $X^1$ | $X^2$ | $X^3$ | $X^4$ | Q |
|---|---|---|---|---|---|---|
| E-0877 | F | N | CH | N | CH | pyrrolidin-3-yl(1-Me) |
| E-0878 | F | N | CH | N | CH | tetrahydrofuran-2-yl |
| E-0879 | F | N | CH | N | CH | tetrahydrofuran-3-yl |
| E-0880 | F | N | CH | N | CH | 4,5-dihydroisoxazol-3-yl |
| E-0881 | F | N | CH | N | CH | 4,5-dihydroisoxazol-4-yl |
| E-0882 | F | N | CH | N | CH | 4,5-dihydroisoxazol-5-yl |
| E-0883 | F | N | CH | N | CH | oxetan-2-yl |
| E-0884 | F | N | CH | N | CH | oxetan-3-yl |
| E-0885 | F | N | CH | N | CH | azetidin-1-yl |
| E-0886 | F | N | CH | N | CH | azetidin-2-yl |
| E-0887 | F | N | CH | N | CH | azetidin-2-yl(1-Me) |
| E-0888 | F | N | CH | N | CH | azetidin-3-yl |
| E-0889 | F | N | CH | N | CH | azetidin-3-yl(1-Me) |

(continued)

| Compound Number | $R^1$ | $X^1$ | $X^2$ | $X^3$ | $X^4$ | Q |
|---|---|---|---|---|---|---|
| E-0890 | F | N | CH | N | CH | oxiran-2-yl |
| E-0891 | F | N | CH | N | CH | 1,3,2-dioxaborolan-2-yl(4,4,5,5-$Me_4$) |
| E-0892 | F | N | CH | CH | N | pyridin-2-yl |
| E-0893 | F | N | CH | CH | N | pyridin-3-yl |
| E-0894 | F | N | CH | CH | N | pyridin-4-yl |
| E-0895 | F | N | CH | CH | N | pyrimidin-2-yl |
| E-0896 | F | N | CH | CH | N | pyrimidin-4-yl |
| E-0897 | F | N | CH | CH | N | pyrimidin-5-yl |
| E-0898 | F | N | CH | CH | N | pyridazin-3-yl |
| E-0899 | F | N | CH | CH | N | pyridazin-4-yl |
| E-0900 | F | N | CH | CH | N | pyrazin-2-yl |
| E-0901 | F | N | CH | CH | N | 1,3,5-triazin-2-yl |
| E-0902 | F | N | CH | CH | N | thiophen-2-yl |
| E-0903 | F | N | CH | CH | N | thiophen-3-yl |
| E-0904 | F | N | CH | CH | N | furan-2-yl |
| E-0905 | F | N | CH | CH | N | furan-3-yl |
| E-0906 | F | N | CH | CH | N | 1H-pyrrol-1-yl |
| E-0907 | F | N | CH | CH | N | 1H-pyrrol-2-yl |
| E-0908 | F | N | CH | CH | N | 1H-pyrrol-2-yl(1-Me) |
| E-0909 | F | N | CH | CH | N | 1H-pyrrol-3-yl |
| E-0910 | F | N | CH | CH | N | 1H-pyrrol-3-yl(1-Me) |
| E-0911 | F | N | CH | CH | N | 1H-pyrazol-1-yl |
| E-0912 | F | N | CH | CH | N | 1H-pyrazol-3-yl |
| E-0913 | F | N | CH | CH | N | 1H-pyrazol-3-yl(1-Me) |
| E-0914 | F | N | CH | CH | N | 1H-pyrazol-4-yl |
| E-0915 | F | N | CH | CH | N | 1H-pyrazol-4-yl(1-Me) |
| E-0916 | F | N | CH | CH | N | 1H-pyrazol-5-yl |
| E-0917 | F | N | CH | CH | N | 1H-pyrazol-5-yl(1-Me) |
| E-0918 | F | N | CH | CH | N | 1H-imidazol-1-yl |

[Table 111]

| Compound Number | $R^1$ | $X^1$ | $X^2$ | $X^3$ | $X^4$ | Q |
|---|---|---|---|---|---|---|
| E-0919 | F | N | CH | CH | N | 1H-imidazol-2-yl |
| E-0920 | F | N | CH | CH | N | 1H-imidazol-2-yl(1-Me) |
| E-0921 | F | N | CH | CH | N | 1H-imidazol-4-yl |
| E-0922 | F | N | CH | CH | N | 1H-imidazol-4-yl(1-Me) |
| E-0923 | F | N | CH | CH | N | 1H-imidazol-5-yl |

(continued)

| Compound Number | $R^1$ | $X^1$ | $X^2$ | $X^3$ | $X^4$ | Q |
|---|---|---|---|---|---|---|
| E-0924 | F | N | CH | CH | N | 1H-imidazol-5-yl(1-Me) |
| E-0925 | F | N | CH | CH | N | 1H-1,2,4-triazol-1-yl |
| E-0926 | F | N | CH | CH | N | 1H-1,2,4-triazol-3-yl |
| E-0927 | F | N | CH | CH | N | 1H-1,2,4-triazol-3-yl(1-Me) |
| E-0928 | F | N | CH | CH | N | 4H-1,2,4-triazol-4-yl |
| E-0929 | F | N | CH | CH | N | 1H-1,2,4-triazol-5-yl |
| E-0930 | F | N | CH | CH | N | 1H-1,2,4-triazol-5-yl(1-Me) |
| E-0931 | F | N | CH | CH | N | 1H-1,2,3-triazol-1-yl |
| E-0932 | F | N | CH | CH | N | 2H-1,2,3-triazol-2-yl |
| E-0933 | F | N | CH | CH | N | 1H-tetrazol-1-yl |
| E-0934 | F | N | CH | CH | N | 2H-tetrazol-2-yl |
| E-0935 | F | N | CH | CH | N | 1H-tetrazol-5-yl |
| E-0936 | F | N | CH | CH | N | 1H-tetrazol-5-yl(1-Me) |
| E-0937 | F | N | CH | CH | N | thiazol-2-yl |
| E-0938 | F | N | CH | CH | N | thiazol-4-yl |
| E-0939 | F | N | CH | CH | N | thiazol-5-yl |
| E-0940 | F | N | CH | CH | N | isothiazol-3-yl |
| E-0941 | F | N | CH | CH | N | isothiazol-4-yl |
| E-0942 | F | N | CH | CH | N | isothiazol-5-yl |
| E-0943 | F | N | CH | CH | N | oxazol-2-yl |
| E-0944 | F | N | CH | CH | N | oxazol-4-yl |
| E-0945 | F | N | CH | CH | N | oxazol-5-yl |
| E-0946 | F | N | CH | CH | N | isoxazol-3-yl |
| E-0947 | F | N | CH | CH | N | isoxazol-4-yl |
| E-0948 | F | N | CH | CH | N | isoxazol-5-yl |
| E-0949 | F | N | CH | CH | N | 1,2,3-oxadiazol-4-yl |
| E-0950 | F | N | CH | CH | N | 1,2,3-oxadiazol-5-yl |
| E-0951 | F | N | CH | CH | N | 1,2,4-oxadiazol-3-yl |
| E-0952 | F | N | CH | CH | N | 1,2,4-oxadiazol-5-yl |
| E-0953 | F | N | CH | CH | N | 1,3,4-oxadiazol-2-yl |
| E-0954 | F | N | CH | CH | N | 1,2,5-oxadiazol-3-yl |
| E-0955 | F | N | CH | CH | N | 1,2,3-thiadiazol-4-yl |
| E-0956 | F | N | CH | CH | N | 1,2,3-thiadiazol-5-yl |
| E-0957 | F | N | CH | CH | N | 1,2,4-thiadiazol-3-yl |
| E-0958 | F | N | CH | CH | N | 1,2,4-thiadiazol-5-yl |
| E-0959 | F | N | CH | CH | N | 1,3,4-thiadiazol-2-yl |
| E-0960 | F | N | CH | CH | N | 1,2,5-thiadiazol-3-yl |

[Table 112]

| Compound Number | $R^1$ | $X^1$ | $X^2$ | $X^3$ | $X^4$ | Q |
|---|---|---|---|---|---|---|
| E-0961 | F | N | CH | CH | N | morpholin-4-yl |
| E-0962 | F | N | CH | CH | N | piperidin-4-yl |
| E-0963 | F | N | CH | CH | N | piperidin-4-yl(1-Me) |
| E-0964 | F | N | CH | CH | N | piperidin-1-yl |
| E-0965 | F | N | CH | CH | N | piperazin-1-yl |
| E-0966 | F | N | CH | CH | N | piperazin-1-yl(4-Me) |
| E-0967 | F | N | CH | CH | N | tetrahydro-2H-pyran-4-yl |
| E-0968 | F | N | CH | CH | N | 1,3-dioxolan-2-yl |
| E-0969 | F | N | CH | CH | N | 1,3-dioxolan-2-yl(2-$CF_3$) |
| E-0970 | F | N | CH | CH | N | 1,3-dioxan-2-yl |
| E-0971 | F | N | CH | CH | N | 1,3-dioxan-2-yl(2-$CF_3$) |
| E-0972 | F | N | CH | CH | N | pyrrolidin-1-yl |
| E-0973 | F | N | CH | CH | N | pyrrolidin-2-yl |
| E-0974 | F | N | CH | CH | N | pyrrolidin-2-yl(1-Me) |
| E-0975 | F | N | CH | CH | N | pyrrolidin-3-yl |
| E-0976 | F | N | CH | CH | N | pyrrolidin-3-yl(1-Me) |
| E-0977 | F | N | CH | CH | N | tetrahydrofuran-2-yl |
| E-0978 | F | N | CH | CH | N | tetrahydrofuran-3-yl |
| E-0979 | F | N | CH | CH | N | 4,5-dihydroisoxazol-3-yl |
| E-0980 | F | N | CH | CH | N | 4,5-dihydroisoxazol-4-yl |
| E-0981 | F | N | CH | CH | N | 4,5-dihydroisoxazol-5-yl |
| E-0982 | F | N | CH | CH | N | oxetan-2-yl |
| E-0983 | F | N | CH | CH | N | oxetan-3-yl |
| E-0984 | F | N | CH | CH | N | azetidin-1-yl |
| E-0985 | F | N | CH | CH | N | azetidin-2-yl |
| E-0986 | F | N | CH | CH | N | azetidin-2-yl(1-Me) |
| E-0987 | F | N | CH | CH | N | azetidin-3-yl |
| E-0988 | F | N | CH | CH | N | azetidin-3-yl(1-Me) |
| E-0989 | F | N | CH | CH | N | oxiran-2-yl |
| E-0990 | F | N | CH | CH | N | 1,3,2-dioxaborolan-2-yl(4,4,5,5-$Me_4$) |
| E-0991 | F | CH | N | CH | N | pyridin-2-yl |
| E-0992 | F | CH | N | CH | N | pyridin-3-yl |
| E-0993 | F | CH | N | CH | N | pyridin-4-yl |
| E-0994 | F | CH | N | CH | N | pyrimidin-2-yl |
| E-0995 | F | CH | N | CH | N | pyrimidin-4-yl |
| E-0996 | F | CH | N | CH | N | pyrimidin-5-yl |
| E-0997 | F | CH | N | CH | N | pyridazin-3-yl |

(continued)

| Compound Number | $R^1$ | $X^1$ | $X^2$ | $X^3$ | $X^4$ | Q |
|---|---|---|---|---|---|---|
| E-0998 | F | CH | N | CH | N | pyridazin-4-yl |
| E-0999 | F | CH | N | CH | N | pyrazin-2-yl |
| E-1000 | F | CH | N | CH | N | 1,3,5-triazin-2-yl |
| E-1001 | F | CH | N | CH | N | thiophen-2-yl |
| E-1002 | F | CH | N | CH | N | thiophen-3-yl |

[Table 113]

| Compound Number | $R^1$ | $X^1$ | $X^2$ | $X^3$ | $X^4$ | Q |
|---|---|---|---|---|---|---|
| E-1003 | F | CH | N | CH | N | furan-2-yl |
| E-1004 | F | CH | N | CH | N | furan-3-yl |
| E-1005 | F | CH | N | CH | N | 1H-pyrrol-1-yl |
| E-1006 | F | CH | N | CH | N | 1H-pyrrol-2-yl |
| E-1007 | F | CH | N | CH | N | 1H-pyrrol-2-yl(1-Me) |
| E-1008 | F | CH | N | CH | N | 1H-pyrrol-3-yl |
| E-1009 | F | CH | N | CH | N | 1H-pyrrol-3-yl(1-Me) |
| E-1010 | F | CH | N | CH | N | 1H-pyrazol-1-yl |
| E-1011 | F | CH | N | CH | N | 1H-pyrazol-3-yl |
| E-1012 | F | CH | N | CH | N | 1H-pyrazol-3-yl(1 -Me) |
| E-1013 | F | CH | N | CH | N | 1H-pyrazol-4-yl |
| E-1014 | F | CH | N | CH | N | 1H-pyrazol-4-yl(1-Me) |
| E-1015 | F | CH | N | CH | N | 1H-pyrazol-5-yl |
| E-1016 | F | CH | N | CH | N | 1H-pyrazol-5-yl(1-Me) |
| E-1017 | F | CH | N | CH | N | 1H-imidazol-1-yl |
| E-1018 | F | CH | N | CH | N | 1H-imidazol-2-yl |
| E-1019 | F | CH | N | CH | N | 1H-imidazol-2-yl(1-Me) |
| E-1020 | F | CH | N | CH | N | 1H-imidazol-4-yl |
| E-1021 | F | CH | N | CH | N | 1H-imidazol-4-yl(1-Me) |
| E-1022 | F | CH | N | CH | N | 1H-imidazol-5-yl |
| E-1023 | F | CH | N | CH | N | 1H-imidazol-5-yl(1-Me) |
| E-1024 | F | CH | N | CH | N | 1H-1,2,4-triazol-1-yl |
| E-1025 | F | CH | N | CH | N | 1H-1,2,4-triazol-3-yl |
| E-1026 | F | CH | N | CH | N | 1H-1,2,4-triazol-3-yl(1-Me) |
| E-1027 | F | CH | N | CH | N | 4H-1,2,4-triazol-4-yl |
| E-1028 | F | CH | N | CH | N | 1H-1,2,4-triazol-5-yl |
| E-1029 | F | CH | N | CH | N | 1H-1,2,4-triazol-5-yl(1-Me) |
| E-1030 | F | CH | N | CH | N | 1H-1,2,3-triazol-1-yl |
| E-1031 | F | CH | N | CH | N | 2H-1,2,3-triazol-2-yl |

(continued)

| Compound Number | $R^1$ | $X^1$ | $X^2$ | $X^3$ | $X^4$ | Q |
|---|---|---|---|---|---|---|
| E-1032 | F | CH | N | CH | N | 1H-tetrazol-1-yl |
| E-1033 | F | CH | N | CH | N | 2H-tetrazol-2-yl |
| E-1034 | F | CH | N | CH | N | 1H-tetrazol-5-yl |
| E-1035 | F | CH | N | CH | N | 1H-tetrazol-5-yl(1-Me) |
| E-1036 | F | CH | N | CH | N | thiazol-2-yl |
| E-1037 | F | CH | N | CH | N | thiazol-4-yl |
| E-1038 | F | CH | N | CH | N | thiazol-5-yl |
| E-1039 | F | CH | N | CH | N | isothiazol-3-yl |
| E-1040 | F | CH | N | CH | N | isothiazol-4-yl |
| E-1041 | F | CH | N | CH | N | isothiazol-5-yl |
| E-1042 | F | CH | N | CH | N | oxazol-2-yl |
| E-1043 | F | CH | N | CH | N | oxazol-4-yl |
| E-1044 | F | CH | N | CH | N | oxazol-5-yl |

[Table 114]

| Compound Number | $R^1$ | $X^1$ | $X^2$ | $X^3$ | $X^4$ | Q |
|---|---|---|---|---|---|---|
| E-1045 | F | CH | N | CH | N | isoxazol-3-yl |
| E-1046 | F | CH | N | CH | N | isoxazol-4-yl |
| E-1047 | F | CH | N | CH | N | isoxazol-5-yl |
| E-1048 | F | CH | N | CH | N | 1,2,3-oxadiazol-4-yl |
| E-1049 | F | CH | N | CH | N | 1,2,3-oxadiazol-5-yl |
| E-1050 | F | CH | N | CH | N | 1,2,4-oxadiazol-3-yl |
| E-1051 | F | CH | N | CH | N | 1,2,4-oxadiazol-5-yl |
| E-1052 | F | CH | N | CH | N | 1,3,4-oxadiazol-2-yl |
| E-1053 | F | CH | N | CH | N | 1,2,5-oxadiazol-3-yl |
| E-1054 | F | CH | N | CH | N | 1,2,3-thiadiazol-4-yl |
| E-1055 | F | CH | N | CH | N | 1,2,3-thiadiazol-5-yl |
| E-1056 | F | CH | N | CH | N | 1,2,4-thiadiazol-3-yl |
| E-1057 | F | CH | N | CH | N | 1,2,4-thiadiazol-5-yl |
| E-1058 | F | CH | N | CH | N | 1,3,4-thiadiazol-2-yl |
| E-1059 | F | CH | N | CH | N | 1,2,5-thiadiazol-3-yl |
| E-1060 | F | CH | N | CH | N | morpholin-4-yl |
| E-1061 | F | CH | N | CH | N | piperidin-4-yl |
| E-1062 | F | CH | N | CH | N | piperidin-4-yl(1-Me) |
| E-1063 | F | CH | N | CH | N | piperidin-1-yl |
| E-1064 | F | CH | N | CH | N | piperazin-1-yl |
| E-1065 | F | CH | N | CH | N | piperazin-1-yl(4-Me) |

(continued)

| Compound Number | R1 | X1 | X2 | X3 | X4 | Q |
|---|---|---|---|---|---|---|
| E-1066 | F | CH | N | CH | N | tetrahydro-2H-pyran-4-yl |
| E-1067 | F | CH | N | CH | N | 1,3-dioxolan-2-yl |
| E-1068 | F | CH | N | CH | N | 1,3-dioxolan-2-yl(2-$CF_3$) |
| E-1069 | F | CH | N | CH | N | 1,3-dioxan-2-yl |
| E-1070 | F | CH | N | CH | N | 1,3-dioxan-2-yl(2-$CF_3$) |
| E-1071 | F | CH | N | CH | N | pyrrolidin-1-yl |
| E-1072 | F | CH | N | CH | N | pyrrolidin-2-yl |
| E-1073 | F | CH | N | CH | N | pyrrolidin-2-yl(1-Me) |
| E-1074 | F | CH | N | CH | N | pyrrolidin-3-yl |
| E-1075 | F | CH | N | CH | N | pyrrolidin-3-yl(1-Me) |
| E-1076 | F | CH | N | CH | N | tetrahydrofuran-2-yl |
| E-1077 | F | CH | N | CH | N | tetrahydrofuran-3-yl |
| E-1078 | F | CH | N | CH | N | 4,5-dihydroisoxazol-3-yl |
| E-1079 | F | CH | N | CH | N | 4,5-dihydroisoxazol-4-yl |
| E-1080 | F | CH | N | CH | N | 4,5-dihydroisoxazol-5-yl |
| E-1081 | F | CH | N | CH | N | oxetan-2-yl |
| E-1082 | F | CH | N | CH | N | oxetan-3-yl |
| E-1083 | F | CH | N | CH | N | azetidin-1-yl |
| E-1084 | F | CH | N | CH | N | azetidin-2-yl |
| E-1085 | F | CH | N | CH | N | azetidin-2-yl(1-Me) |
| E-1086 | F | CH | N | CH | N | azetidin-3-yl |

[Table 115]

| Compound Number | R1 | X1 | X2 | X3 | X4 | Q |
|---|---|---|---|---|---|---|
| E-1087 | F | CH | N | CH | N | azetidin-3-yl(1-Me) |
| E-1088 | F | CH | N | CH | N | oxiran-2-yl |
| E-1089 | F | CH | N | CH | N | 1,3,2-dioxaborolan-2-yl(4,4,5,5-$Me_4$) |
| E-1090 | F | CF | CH | CH | CH | tetrahydro-2H-pyran-2-yl |
| E-1091 | F | CF | CH | CH | CH | tetrahydro-2H-pyran-3-yl |
| E-1092 | F | CF | CH | CH | CH | piperidin-2-yl |
| E-1093 | F | CF | CH | CH | CH | piperidin-2-yl(1-Me) |
| E-1094 | F | CF | CH | CH | CH | piperidin-3-yl |
| E-1095 | F | CF | CH | CH | CH | piperidin-3-yl(1-Me) |
| E-1096 | F | CH | CF | CH | CH | tetrahydro-2H-pyran-2-yl |
| E-1097 | F | CH | CF | CH | CH | tetrahydro-2H-pyran-3-yl |
| E-1098 | F | CH | CF | CH | CH | piperidin-2-yl |
| E-1099 | F | CH | CF | CH | CH | piperidin-2-yl(1-Me) |

(continued)

| Compound Number | $R^1$ | $X^1$ | $X^2$ | $X^3$ | $X^4$ | Q |
|---|---|---|---|---|---|---|
| E-1100 | F | CH | CF | CH | CH | piperidin-3-yl |
| E-1101 | F | CH | CF | CH | CH | piperidin-3-yl(1-Me) |
| E-1102 | F | CMe | CH | CH | CH | tetrahydro-2H-pyran-2-yl |
| E-1103 | F | CMe | CH | CH | CH | tetrahydro-2H-pyran-3-yl |
| E-1104 | F | CMe | CH | CH | CH | piperidin-2-yl |
| E-1105 | F | CMe | CH | CH | CH | piperidin-2-yl(1-Me) |
| E-1106 | F | CMe | CH | CH | CH | piperidin-3-yl |
| E-1107 | F | CMe | CH | CH | CH | piperidin-3-yl(1-Me) |
| E-1108 | F | CH | CMe | CH | CH | tetrahydro-2H-pyran-2-yl |
| E-1109 | F | CH | CMe | CH | CH | tetrahydro-2H-pyran-3-yl |
| E-1110 | F | CH | CMe | CH | CH | piperidin-2-yl |
| E-1111 | F | CH | CMe | CH | CH | piperidin-2-yl(1-Me) |
| E-1112 | F | CH | CMe | CH | CH | piperidin-3-yl |
| E-1113 | F | CH | CMe | CH | CH | piperidin-3-yl(1-Me) |
| E-1114 | Cl | CH | CH | CH | CH | tetrahydro-2H-pyran-2-yl |
| E-1115 | Cl | CH | CH | CH | CH | tetrahydro-2H-pyran-3-yl |
| E-1116 | Cl | CH | CH | CH | CH | piperidin-2-yl |
| E-1117 | Cl | CH | CH | CH | CH | piperidin-2-yl(1-Me) |
| E-1118 | Cl | CH | CH | CH | CH | piperidin-3-yl |
| E-1119 | Cl | CH | CH | CH | CH | piperidin-3-yl(1-Me) |
| E-1120 | F | N | CH | CH | CH | tetrahydro-2H-pyran-2-yl |
| E-1121 | F | N | CH | CH | CH | tetrahydro-2H-pyran-3-yl |
| E-1122 | F | N | CH | CH | CH | piperidin-2-yl |
| E-1123 | F | N | CH | CH | CH | piperidin-2-yl(1-Me) |
| E-1124 | F | N | CH | CH | CH | piperidin-3-yl |
| E-1125 | F | N CH | | CH | CH | piperidin-3-yl(1-Me) |
| E-1126 | F | CH N | | CH | CH | tetrahydro-2H-pyran-2-yl |
| E-1127 | F | CH N | | CH | CH | tetrahydro-2H-pyran-3-yl |
| E-1128 | F | CH N | | CH | CH | piperidin-2-yl |

[Table 116]

| Compound Number | $R^1$ | $X^1$ | $X^2$ | $X^3$ | $X^4$ | Q |
|---|---|---|---|---|---|---|
| E-1129 | F | CH | N | CH | CH | piperidin-2-yl(1-Me) |
| E-1130 | F | CH | N | CH | CH | piperidin-3-yl |
| E-1131 | F | CH | N | CH | CH | piperidin-3-yl(1-Me) |
| E-1132 | F | N | N | CH | CH | tetrahydro-2H-pyran-2-yl |
| E-1133 | F | N | N | CH | CH | tetrahydro-2H-pyran-3-yl |

(continued)

| Compound Number | $R^1$ | $X^1$ | $X^2$ | $X^3$ | $X^4$ | Q |
|---|---|---|---|---|---|---|
| E-1134 | F | N | N | CH | CH | piperidin-2-yl |
| E-1135 | F | N | N | CH | CH | piperidin-2-yl(1-Me) |
| E-1136 | F | N | N | CH | CH | piperidin-3-yl |
| E-1137 | F | N | N | CH | CH | piperidin-3-yl(1-Me) |
| E-1138 | F | N | CH | N | CH | tetrahydro-2H-pyran-2-yl |
| E-1139 | F | N | CH | N | CH | tetrahydro-2H-pyran-3-yl |
| E-1140 | F | N | CH | N | CH | piperidin-2-yl |
| E-1141 | F | N | CH | N | CH | piperidin-2-yl(1-Me) |
| E-1142 | F | N | CH | N | CH | piperidin-3-yl |
| E-1143 | F | N | CH | N | CH | piperidin-3-yl(1-Me) |
| E-1144 | F | N | CH | CH | N | tetrahydro-2H-pyran-2-yl |
| E-1145 | F | N | CH | CH | N | tetrahydro-2H-pyran-3-yl |
| E-1146 | F | N | CH | CH | N | piperidin-2-yl |
| E-1147 | F | N | CH | CH | N | piperidin-2-yl(1-Me) |
| E-1148 | F | N | CH | CH | N | piperidin-3-yl |
| E-1149 | F | N | CH | CH | N | piperidin-3-yl(1-Me) |
| E-1150 | F | CH | N | CH | N | tetrahydro-2H-pyran-2-yl |
| E-1151 | F | CH | N | CH | N | tetrahydro-2H-pyran-3-yl |
| E-1152 | F | CH | N | CH | N | piperidin-2-yl |
| E-1153 | F | CH | N | CH | N | piperidin-2-yl(1-Me) |
| E-1154 | F | CH | N | CH | N | piperidin-3-yl |
| E-1155 | F | CH | N | CH | N | piperidin-3-yl(1-Me) |
| E-1156 | H | CH | CH | CH | CH | pyridin-2-yl |
| E-1157 | H | CH | CH | CH | CH | pyridin-3-yl |
| E-1158 | H | CH | CH | CH | CH | pyridin-4-yl |
| E-1159 | H | CH | CH | CH | CH | pyrimidin-2-yl |
| E-1160 | H | CH | CH | CH | CH | pyrimidin-4-yl |
| E-1161 | H | CH | CH | CH | CH | pyrimidin-5-yl |
| E-1162 | H | CH | CH | CH | CH | pyridazin-3-yl |
| E-1163 | H | CH | CH | CH | CH | pyridazin-4-yl |
| E-1164 | H | CH | CH | CH | CH | pyrazin-2-yl |
| E-1165 | H | CH | CH | CH | CH | 1,3,5-triazin-2-yl |
| E-1166 | H | CH | CH | CH | CH | thiophen-2-yl |
| E-1167 | H | CH | CH | CH | CH | thiophen-3-yl |
| E-1168 | H | CH | CH | CH | CH | furan-2-yl |
| E-1169 | H | CH | CH | CH | CH | furan-3-yl |
| E-1170 | H | CH | CH | CH | CH | 1H-pyrrol-1-yl |

[Table 117]

| Compound Number | $R^1$ | $X^1$ | $X^2$ | $X^3$ | $X^4$ | Q |
|---|---|---|---|---|---|---|
| E-1171 | H | CH | CH | CH | CH | 1H-pyrrol-2-yl |
| E-1172 | H | CH | CH | CH | CH | 1H-pyrrol-2-yl(1-Me) |
| E-1173 | H | CH | CH | CH | CH | 1H-pyrrol-3-yl |
| E-1174 | H | CH | CH | CH | CH | 1H-pyrrol-3-yl(1-Me) |
| E-1175 | H | CH | CH | CH | CH | 1H-pyrazol-1-yl |
| E-1176 | H | CH | CH | CH | CH | 1H-pyrazol-3-yl |
| E-1177 | H | CH | CH | CH | CH | 1H-pyrazol-3-yl(1-Me) |
| E-1178 | H | CH | CH | CH | CH | 1H-pyrazol-4-yl |
| E-1179 | H | CH | CH | CH | CH | 1H-pyrazol-4-yl(1-Me) |
| E-1180 | H | CH | CH | CH | CH | 1H-pyrazol-5-yl |
| E-1181 | H | CH | CH | CH | CH | 1H-pyrazol-5-yl(1-Me) |
| E-1182 | H | CH | CH | CH | CH | 1H-imidazol-1-yl |
| E-1183 | H | CH | CH | CH | CH | 1H-imidazol-2-yl |
| E-1184 | H | CH | CH | CH | CH | 1H-imidazol-2-yl(1-Me) |
| E-1185 | H | CH | CH | CH | CH | 1H-imidazol-4-yl |
| E-1186 | H | CH | CH | CH | CH | 1H-imidazol-4-yl(1-Me) |
| E-1187 | H | CH | CH | CH | CH | 1H-imidazol-5-yl |
| E-1188 | H | CH | CH | CH | CH | 1H-imidazol-5-yl(1-Me) |
| E-1189 | H | CH | CH | CH | CH | 1H-1,2,4-triazol-1-yl |
| E-1190 | H | CH | CH | CH | CH | 1H-1,2,4-triazol-3-yl |
| E-1191 | H | CH | CH | CH | CH | 1H-1,2,4-triazol-3-yl(1-Me) |
| E-1192 | H | CH | CH | CH | CH | 4H-1,2,4-triazol-4-yl |
| E-1193 | H | CH | CH | CH | CH | 1H-1,2,4-triazol-5-yl |
| E-1194 | H | CH | CH | CH | CH | 1H-1,2,4-triazol-5-yl(1-Me) |
| E-1195 | H | CH | CH | CH | CH | 1H-1,2,3-triazol-1-yl |
| E-1196 | H | CH | CH | CH | CH | 2H-1,2,3-triazol-2-yl |
| E-1197 | H | CH | CH | CH | CH | 1H-tetrazol-1-yl |
| E-1198 | H | CH | CH | CH | CH | 2H-tetrazol-2-yl |
| E-1199 | H | CH | CH | CH | CH | 1H-tetrazol-5-yl |
| E-1200 | H | CH | CH | CH | CH | 1H-tetrazol-5-yl(1-Me) |
| E-1201 | H | CH | CH | CH | CH | thiazol-2-yl |
| E-1202 | H | CH | CH | CH | CH | thiazol-4-yl |
| E-1203 | H | CH | CH | CH | CH | thiazol-5-yl |
| E-1204 | H | CH | CH | CH | CH | isothiazol-3-yl |
| E-1205 | H | CH | CH | CH | CH | isothiazol-4-yl |
| E-1206 | H | CH | CH | CH | CH | isothiazol-5-yl |
| E-1207 | H | CH | CH | CH | CH | oxazol-2-yl |

(continued)

| Compound Number | $R^1$ | $X^1$ | $X^2$ | $X^3$ | $X^4$ | Q |
|---|---|---|---|---|---|---|
| E-1208 | H | CH | CH | CH | CH | oxazol-4-yl |
| E-1209 | H | CH | CH | CH | CH | oxazol-5-yl |
| E-1210 | H | CH | CH | CH | CH | isoxazol-3-yl |
| E-1211 | H | CH | CH | CH | CH | isoxazol-4-yl |
| E-1212 | H | CH | CH | CH | CH | isoxazol-5-yl |

[Table 118]

| Compound Number | $R^1$ | $X^1$ | $X^2$ | $X^3$ | $X^4$ | Q |
|---|---|---|---|---|---|---|
| E-1213 | H | CH | CH | CH | CH | 1,2,3-oxadiazol-4-yl |
| E-1214 | H | CH | CH | CH | CH | 1,2,3-oxadiazol-5-yl |
| E-1215 | H | CH | CH | CH | CH | 1,2,4-oxadiazol-3-yl |
| E-1216 | H | CH | CH | CH | CH | 1,2,4-oxadiazol-5-yl |
| E-1217 | H | CH | CH | CH | CH | 1,3,4-oxadiazol-2-yl |
| E-1218 | H | CH | CH | CH | CH | 1,2,5-oxadiazol-3-yl |
| E-1219 | H | CH | CH | CH | CH | 1,2,3-thiadiazol-4-yl |
| E-1220 | H | CH | CH | CH | CH | 1,2,3-thiadiazol-5-yl |
| E-1221 | H | CH | CH | CH | CH | 1,2,4-thiadiazol-3-yl |
| E-1222 | H | CH | CH | CH | CH | 1,2,4-thiadiazol-5-yl |
| E-1223 | H | CH | CH | CH | CH | 1,3,4-thiadiazol-2-yl |
| E-1224 | H | CH | CH | CH | CH | 1,2,5-thiadiazol-3-yl |
| E-1225 | H | CH | CH | CH | CH | morpholin-4-yl |
| E-1226 | H | CH | CH | CH | CH | piperidin-4-yl |
| E-1227 | H | CH | CH | CH | CH | piperidin-4-yl(1-Me) |
| E-1228 | H | CH | CH | CH | CH | piperidin-1-yl |
| E-1229 | H | CH | CH | CH | CH | piperazin-1-yl |
| E-1230 | H | CH | CH | CH | CH | piperazin-1-yl(4-Me) |
| E-1231 | H | CH | CH | CH | CH | tetrahydro-2H-pyran-4-yl |
| E-1232 | H | CH | CH | CH | CH | 1,3-dioxolan-2-yl |
| E-1233 | H | CH | CH | CH | CH | 1,3-dioxolan-2-yl(2-$CF_3$) |
| E-1234 | H | CH | CH | CH | CH | 1,3-dioxan-2-yl |
| E-1235 | H | CH | CH | CH | CH | 1,3-dioxan-2-yl(2-$CF_3$) |
| E-1236 | H | CH | CH | CH | CH | pyrrolidin-1-yl |
| E-1237 | H | CH | CH | CH | CH | pyrrolidin-2-yl |
| E-1238 | H | CH | CH | CH | CH | pyrrolidin-2-yl(1-Me) |
| E-1239 | H | CH | CH | CH | CH | pyrrolidin-3-yl |
| E-1240 | H | CH | CH | CH | CH | pyrrolidin-3-yl(1-Me) |
| E-1241 | H | CH | CH | CH | CH | tetrahydrofuran-2-yl |

(continued)

| Compound Number | $R^1$ | $X^1$ | $X^2$ | $X^3$ | $X^4$ | Q |
|---|---|---|---|---|---|---|
| E-1242 | H | CH | CH | CH | CH | tetrahydrofuran-3-yl |
| E-1243 | H | CH | CH | CH | CH | 4,5-dihydroisoxazol-3-yl |
| E-1244 | H | CH | CH | CH | CH | 4,5-dihydroisoxazol-4-yl |
| E-1245 | H | CH | CH | CH | CH | 4,5-dihydroisoxazol-5-yl |
| E-1246 | H | CH | CH | CH | CH | oxetan-2-yl |
| E-1247 | H | CH | CH | CH | CH | oxetan-3-yl |
| E-1248 | H | CH | CH | CH | CH | azetidin-1-yl |
| E-1249 | H | CH | CH | CH | CH | azetidin-2-yl |
| E-1250 | H | CH | CH | CH | CH | azetidi n-2-yl(1-Me) |
| E-1251 | H | CH | CH | CH | CH | azetidin-3-yl |
| E-1252 | H | CH | CH | CH | CH | azetidi n-3-yl(1-Me) |
| E-1253 | H | CH | CH | CH | CH | oxiran-2-yl |
| E-1254 | H | CH | CH | CH | CH | 1,3,2-dioxaborolan-2-yl(4,4,5,5-$Me_4$) |

[Table 119]

| Compound Number | $R^1$ | $X^1$ | $X^2$ | $X^3$ | $X^4$ | Q |
|---|---|---|---|---|---|---|
| E-1255 | H | CH | CH | CH | CH | tetrahydro-2H-pyran-2-yl |
| E-1256 | H | CH | CH | CH | CH | tetrahydro-2H-pyran-3-yl |
| E-1257 | H | CH | CH | CH | CH | piperidin-2-yl |
| E-1258 | H | CH | CH | CH | CH | piperidin-2-yl(1-Me) |
| E-1259 | H | CH | CH | CH | CH | piperidin-3-yl |
| E-1260 | H | CH | CH | CH | CH | piperidin-3-yl(1-Me) |

[Table 120]

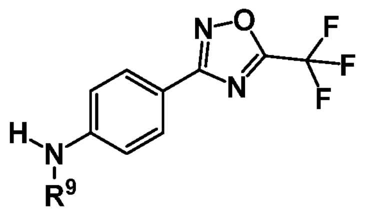

| Compound Number | $R^9$ |
|---|---|
| F-0001 | n-Pr |
| F-0002 | i-Pr |
| F-0003 | n-Bu |
| F-0004 | i-Bu |
| F-0005 | s-Bu |
| F-0006 | t-Bu |

(continued)

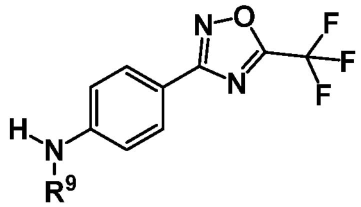

| Compound Number | $R^9$ |
|---|---|
| F-0007 | $CH_2$t-Bu |
| F-0008 | n-Pen |
| F-0009 | n-Hex |
| F-0010 | $CH=CH_2$ |
| F-0011 | $CH_2CH=CH_2$ |
| F-0012 | $CH_2CH=CHCH_2Cl$ |
| F-0013 | $CH_2CH=CHCH_2NMe_2$ |
| F-0014 | $C\equiv CH$ |
| F-0015 | $CH_2C\equiv CH$ |
| F-0016 | $CH_2$c-Pr |
| F-0017 | $CH_2$c-Bu |
| F-0018 | $CH_2$c-Pen |
| F-0019 | $CH_2$c-Hex |
| F-0020 | $CH_2$c-Pr(2,2-$Cl_2$) |
| F-0021 | $CF_3$ |
| F-0022 | $CH_2CF_3$ |
| F-0023 | $CH_2CH_2CF_3$ |
| F-0024 | $CHF_2$ |
| F-0025 | $CH_2CHF_2$ |
| F-0026 | $CH_2CH_2CHF_2$ |
| F-0027 | $CH_2COMe$ |
| F-0028 | $CH_2CO_2Me$ |
| F-0029 | $CH(CH_3)CO_2Me$ |
| F-0030 | $C(CH_3)(CH_3)CO_2Me$ |
| F-0031 | $CH_2CO_2Et$ |
| F-0032 | $CH(CH_3)CO_2Et$ |
| F-0033 | $C(CH_3)(CH_3)CO_2Et$ |
| F-0034 | $CH_2CONH_2$ |
| F-0035 | $CH_2CONHMe$ |

(continued)

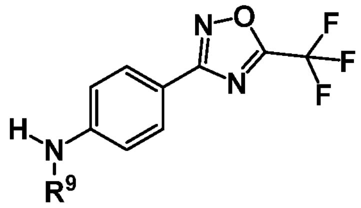

| Compound Number | $R^9$ |
|---|---|
| F-0036 | $CH(CH_3)CONHMe$ |

[Table 121]

| Compound Number | $R^9$ |
|---|---|
| F-0037 | $C(CH_3)(CH_3)CONHMe$ |
| F-0038 | $CH_2CONHEt$ |
| F-0039 | $CH(CH_3)CONHEt$ |
| F-0040 | $C(CH_3)(CH_3)CONHEt$ |
| F-0041 | $CH_2NHCHO$ |
| F-0042 | $CH(CH_3)NHCHO$ |
| F-0043 | $C(CH_3)(CH_3)NHCHO$ |
| F-0044 | $CH_2NHCOMe$ |
| F-0045 | $CH(CH_3)NHCOMe$ |
| F-0046 | $C(CH_3)(CH_3)NHCOMe$ |
| F-0047 | $CH_2NHCOEt$ |
| F-0048 | $CH(CH_3)NHCOEt$ |
| F-0049 | $C(CH_3)(CH_3)NHCOEt$ |
| F-0050 | $CH_2CN$ |
| F-0051 | $CH_2CH_2CN$ |
| F-0052 | $CH_2SCN$ |
| F-0053 | $CH_2CH_2SCN$ |
| F-0054 | $CH_2CH_2CH_2CH_2CH_2SCN$ |
| F-0055 | $CH_2CH_2CH_2CH_2CH_2CH_2SCN$ |
| F-0056 | $CH_2OCH_3$ |
| F-0057 | $CH_2OCH_2CH_3$ |
| F-0058 | $CH_2CH_2OCH_3$ |
| F-0059 | $CH_2CH_2OCH_2CH_3$ |
| F-0060 | $CH_2OCF_3$ |
| F-0061 | $CH_2OCH_2CF_3$ |

(continued)

| Compound Number | $R^9$ |
|---|---|
| F-0062 | $CH_2CH_2OCF_3$ |
| F-0063 | $CH_2CH_2OCH_2CF_3$ |
| F-0064 | $CH_2SCH_3$ |
| F-0065 | $CH_2SCH_2CH_3$ |
| F-0066 | $CH_2CH_2SCH_3$ |
| F-0067 | $CH_2CH_2SCH_2CH_3$ |
| F-0068 | $CH_2SCF_3$ |
| F-0069 | $CH_2SCH_2CF_3$ |
| F-0070 | $CH_2CH_2SCF_3$ |
| F-0071 | $CH_2CH_2SCH_2CF_3$ |
| F-0072 | $CH_2CH_2CH_2CH_2CH_2SCF_3$ |
| F-0073 | $CH_2CH_2CH_2CH_2CH_2CH_2SCF_3$ |
| F-0074 | $CH_2Ph$ |
| F-0075 | $CH_2CH_2Ph$ |
| F-0076 | $CH_2CH_2CH_2Ph$ |
| F-0077 | $CH(CH_3)Ph$ |
| F-0078 | $C(CH_3)(CH_3)Ph$ |

[Table 122]

| Compound Number | $R^9$ |
|---|---|
| F-0079 | $CH_2COPh$ |
| F-0080 | $CH_2CO_2Ph$ |
| F-0081 | $CH_2CONHPh$ |
| F-0082 | $CH_2NHCOPh$ |
| F-0083 | $CH(CH_3)$pyridin-2-yl |
| F-0084 | $C(CH_3)(CH_3)$pyridin-2-yl |
| F-0085 | $CH(CH_3)$pyridin-3-yl |
| F-0086 | $C(CH_3)(CH_3)$pyridin-3-yl |
| F-0087 | $CH(CH_3)$pyridin-4-yl |
| F-0088 | $C(CH_3)(CH_3)$pyridin-4-yl |
| F-0089 | $CH(CH_3)$pyrimidin-2-yl |
| F-0090 | $C(CH_3)(CH_3)$pyrimidin-2-yl |
| F-0091 | $CH(CH_3)$pyrimidin-4-yl |
| F-0092 | $C(CH_3)(CH_3)$pyrimidin-4-yl |

(continued)

| Compound Number | $R^9$ |
|---|---|
| F-0093 | $CH(CH_3)$pyrimidin-5-yl |
| F-0094 | $C(CH_3)(CH_3)$pyrimidin-5-yl |
| F-0095 | $CH(CH_3)$pyridazin-3-yl |
| F-0096 | $C(CH_3)(CH_3)$pyridazin-3-yl |
| F-0097 | $CH(CH_3)$pyridazin-4-yl |
| F-0098 | $C(CH_3)(CH_3)$pyridazin-4-yl |
| F-0099 | $CH(CH_3)$pyrazin-2-yl |
| F-0100 | $C(CH_3)(CH_3)$pyrazin-2-yl |
| F-0101 | $CH_2OPh$ |
| F-0102 | $CH_2CH_2OPh$ |
| F-0103 | CH(OH)Ph |
| F-0104 | $CH(CH_2OH)Ph$ |
| F-0105 | $CH_2CH(OH)Ph$ |
| F-0106 | $CH_2SPh$ |
| F-0107 | $CH_2SOPh$ |
| F-0108 | $CH_2SO_2Ph$ |
| F-0109 | $CH_2CH_2SPh$ |
| F-0110 | $CH_2CH_2SOPh$ |
| F-0111 | $CH_2CH_2SO_2Ph$ |
| F-0112 | $CH_2NHPh$ |
| F-0113 | $CH_2CH_2NHPh$ |
| F-0114 | $CH_2Ph$(2-F) |
| F-0115 | $CH_2Ph$(3-F) |
| F-0116 | $CH_2Ph$(4-F) |
| F-0117 | $CH_2Ph$(2-Cl) |
| F-0118 | $CH_2Ph$(3-Cl) |
| F-0119 | $CH_2Ph$(4-Cl) |
| F-0120 | $CH_2Ph$(2-Br) |

[Table 123]

| Compound Number | $R^9$ |
|---|---|
| F-0121 | $CH_2Ph$(3-Br) |
| F-0122 | $CH_2Ph$(4-Br) |
| F-0123 | $CH_2Ph$(2-I) |

(continued)

| Compound Number | $R^9$ |
|---|---|
| F-0124 | $CH_2Ph(3-I)$ |
| F-0125 | $CH_2Ph(4-I)$ |
| F-0126 | $CH_2Ph(2-Me)$ |
| F-0127 | $CH_2Ph(3-Me)$ |
| F-0128 | $CH_2Ph(4-Me)$ |
| F-0129 | $CH_2Ph(2-Et)$ |
| F-0130 | $CH_2Ph(3-Et)$ |
| F-0131 | $CH_2Ph(4-Et)$ |
| F-0132 | $CH_2Ph(2-n-Pr)$ |
| F-0133 | $CH_2Ph(3-n-Pr)$ |
| F-0134 | $CH_2Ph(4-n-Pr)$ |
| F-0135 | $CH_2Ph(2-i-Pr)$ |
| F-0136 | $CH_2Ph(3-i-Pr)$ |
| F-0137 | $CH_2Ph(4-i-Pr)$ |
| F-0138 | $CH_2Ph(2-s-Bu)$ |
| F-0139 | $CH_2Ph(3-s-Bu)$ |
| F-0140 | $CH_2Ph(4-s-Bu)$ |
| F-0141 | $CH_2Ph(2-i-Bu)$ |
| F-0142 | $CH_2Ph(3-i-Bu)$ |
| F-0143 | $CH_2Ph(4-i-Bu)$ |
| F-0144 | $CH_2Ph(2-t-Bu)$ |
| F-0145 | $CH_2Ph(3-t-Bu)$ |
| F-0146 | $CH_2Ph(4-t-Bu)$ |
| F-0147 | $CH_2Ph(2-CH=CH_2)$ |
| F-0148 | $CH_2Ph(3-CH=CH_2)$ |
| F-0149 | $CH_2Ph(4-CH=CH_2)$ |
| F-0150 | $CH_2Ph(2-CH_2CH=CH_2)$ |
| F-0151 | $CH_2Ph(3-CH_2CH=CH_2)$ |
| F-0152 | $CH_2Ph(4-CH_2CH=CH_2)$ |
| F-0153 | $CH_2Ph(2-CH\equiv CH)$ |
| F-0154 | $CH_2Ph(3-CH\equiv CH)$ |
| F-0155 | $CH_2Ph(4-CH\equiv CH)$ |
| F-0156 | $CH_2Ph(2-c-Pr)$ |
| F-0157 | $CH_2Ph(3-c-Pr)$ |

(continued)

| Compound Number | $R^9$ |
|---|---|
| F-0158 | $CH_2Ph(4\text{-}c\text{-}Pr)$ |
| F-0159 | $CH_2Ph(2\text{-}c\text{-}Bu)$ |
| F-0160 | $CH_2Ph(3\text{-}c\text{-}Bu)$ |
| F-0161 | $CH_2Ph(4\text{-}c\text{-}Bu)$ |
| F-0162 | $CH_2Ph(2\text{-}OMe)$ |

[Table 124]

| Compound Number | $R^9$ |
|---|---|
| F-0163 | $CH_2Ph(3\text{-}OMe)$ |
| F-0164 | $CH_2Ph(4\text{-}OMe)$ |
| F-0165 | $CH_2Ph(2\text{-}OEt)$ |
| F-0166 | $CH_2Ph(3\text{-}OEt)$ |
| F-0167 | $CH_2Ph(4\text{-}OEt)$ |
| F-0168 | $CH_2Ph(2\text{-}On\text{-}Pr)$ |
| F-0169 | $CH_2Ph(3\text{-}On\text{-}Pr)$ |
| F-0170 | $CH_2Ph(4\text{-}On\text{-}Pr)$ |
| F-0171 | $CH_2Ph(2\text{-}Oi\text{-}Pr)$ |
| F-0172 | $CH_2Ph(3\text{-}Oi\text{-}Pr)$ |
| F-0173 | $CH_2Ph(4\text{-}Oi\text{-}Pr)$ |
| F-0174 | $CH_2Ph(2\text{-}OCF_3)$ |
| F-0175 | $CH_2Ph(3\text{-}OCF_3)$ |
| F-0176 | $CH_2Ph(4\text{-}OCF_3)$ |
| F-0177 | $CH_2Ph(2\text{-}OCHF_2)$ |
| F-0178 | $CH_2Ph(3\text{-}OCHF_2)$ |
| F-0179 | $CH_2Ph(4\text{-}OCHF_2)$ |
| F-0180 | $CH_2Ph(2\text{-}OCH_2CF_3)$ |
| F-0181 | $CH_2Ph(3\text{-}OCH_2CF_3)$ |
| F-0182 | $CH_2Ph(4\text{-}OCH_2CF_3)$ |
| F-0183 | $CH_2Ph(2\text{-}SMe)$ |
| F-0184 | $CH_2Ph(3\text{-}SMe)$ |
| F-0185 | $CH_2Ph(4\text{-}SMe)$ |
| F-0186 | $CH_2Ph(2\text{-}SEt)$ |
| F-0187 | $CH_2Ph(3\text{-}SEt)$ |
| F-0188 | $CH_2Ph(4\text{-}SEt)$ |

(continued)

| Compound Number | $R^9$ |
|---|---|
| F-0189 | $CH_2Ph(2-SCF_3)$ |
| F-0190 | $CH_2Ph(3-SCF_3)$ |
| F-0191 | $CH_2Ph(4-SCF_3)$ |
| F-0192 | $CH_2Ph(2-SOMe)$ |
| F-0193 | $CH_2Ph(3-SOMe)$ |
| F-0194 | $CH_2Ph(4-SOMe)$ |
| F-0195 | $CH_2Ph(2-SO_2Me)$ |
| F-0196 | $CH_2Ph(3-SO_2Me)$ |
| F-0197 | $CH_2Ph(4-SO_2Me)$ |
| F-0198 | $CH_2Ph(2-CN)$ |
| F-0199 | $CH_2Ph(3-CN)$ |
| F-0200 | $CH_2Ph(4-CN)$ |
| F-0201 | $CH_2Ph(2-CH_2CN)$ |
| F-0202 | $CH_2Ph(3-CH_2CN)$ |
| F-0203 | $CH_2Ph(4-CH_2CN)$ |
| F-0204 | $CH_2Ph[2-(1-CN-c-Pr)]$ |

[Table 125]

| Compound Number | $R^9$ |
|---|---|
| F-0205 | $CH_2Ph[3-(1-CN-c-Pr)]$ |
| F-0206 | $CH_2Ph[4-(1-CN-c-Pr)]$ |
| F-0207 | $CH_2Ph(2-CH_2OMe)$ |
| F-0208 | $CH_2Ph(3-CH_2OMe)$ |
| F-0209 | $CH_2Ph(4-CH_2OMe)$ |
| F-0210 | $CH_2Ph(2-CH_2OEt)$ |
| F-0211 | $CH_2Ph(3-CH_2OEt)$ |
| F-0212 | $CH_2Ph(4-CH_2OEt)$ |
| F-0213 | $CH_2Ph(2-CF_3)$ |
| F-0214 | $CH_2Ph(3-CF_3)$ |
| F-0215 | $CH_2Ph(4-CF_3)$ |
| F-0216 | $CH_2Ph(2-CHF_2)$ |
| F-0217 | $CH_2Ph(3-CHF_2)$ |
| F-0218 | $CH_2Ph(4-CHF_2)$ |
| F-0219 | $CH_2Ph(2-CH_2F)$ |

(continued)

| Compound Number | $R^9$ |
|---|---|
| F-0220 | $CH_2Ph(3-CH_2F)$ |
| F-0221 | $CH_2Ph(4-CH_2F)$ |
| F-0222 | $CH_2Ph(2-CF_2Cl)$ |
| F-0223 | $CH_2Ph(3-CF_2Cl)$ |
| F-0224 | $CH_2Ph(4-CF_2Cl)$ |
| F-0225 | $CH_2Ph[2-CF(CF_3)_2]$ |
| F-0226 | $CH_2Ph[3-CF(CF_3)_2]$ |
| F-0227 | $CH_2Ph[4-CF(CF_3)_2]$ |
| F-0228 | $CH_2Ph(2-COMe)$ |
| F-0229 | $CH_2Ph(3-COMe)$ |
| F-0230 | $CH_2Ph(4-COMe)$ |
| F-0231 | $CH_2Ph(2-COEt)$ |
| F-0232 | $CH_2Ph(3-COEt)$ |
| F-0233 | $CH_2Ph(4-COEt)$ |
| F-0234 | $CH_2Ph(2-CO_2H)$ |
| F-0235 | $CH_2Ph(3-CO_2H)$ |
| F-0236 | $CH_2Ph(4-CO_2H)$ |
| F-0237 | $CH_2Ph(2-CO_2Me)$ |
| F-0238 | $CH_2Ph(3-CO_2Me)$ |
| F-0239 | $CH_2Ph(4-CO_2Me)$ |
| F-0240 | $CH_2Ph(2-CO_2Et)$ |
| F-0241 | $CH_2Ph(3-CO_2Et)$ |
| F-0242 | $CH_2Ph(4-CO_2Et)$ |
| F-0243 | $CH_2Ph(2-CONH_2)$ |
| F-0244 | $CH_2Ph(3-CONH_2)$ |
| F-0245 | $CH_2Ph(4-CONH_2)$ |
| F-0246 | $CH_2Ph(2-CONHMe)$ |

[Table 126]

| Compound Number | $R^9$ |
|---|---|
| F-0247 | $CH_2Ph(3-CONHMe)$ |
| F-0248 | $CH_2Ph(4-CONHMe)$ |
| F-0249 | $CH_2Ph(2-CONHEt)$ |
| F-0250 | $CH_2Ph(3-CONHEt)$ |

(continued)

| Compound Number | $R^9$ |
|---|---|
| F-0251 | $CH_2Ph$(4-CONHEt) |
| F-0252 | $CH_2Ph$(2-NHCHO) |
| F-0253 | $CH_2Ph$(3-NHCHO) |
| F-0254 | $CH_2Ph$(4-NHCHO) |
| F-0255 | $CH_2Ph$(2-NHCOMe) |
| F-0256 | $CH_2Ph$(3-NHCOMe) |
| F-0257 | $CH_2Ph$(4-NHCOMe) |
| F-0258 | $CH_2Ph$(2-NHCOEt) |
| F-0259 | $CH_2Ph$(3-NHCOEt) |
| F-0260 | $CH_2Ph$(4-NHCOEt) |
| F-0261 | $CH_2Ph$(2-CH=NOH) |
| F-0262 | $CH_2Ph$(3-CH=NOH) |
| F-0263 | $CH_2Ph$(4-CH=NOH) |
| F-0264 | $CH_2Ph$(2-CH=NOMe) |
| F-0265 | $CH_2Ph$(3-CH=NOMe) |
| F-0266 | $CH_2Ph$(4-CH=NOMe) |
| F-0267 | $CH_2Ph$(2-CMe=NOH) |
| F-0268 | $CH_2Ph$(3-CMe=NOH) |
| F-0269 | $CH_2Ph$(4-CMe=NOH) |
| F-0270 | $CH_2Ph$(2-CMe=NOMe) |
| F-0271 | $CH_2Ph$(3-CMe=NOMe) |
| F-0272 | $CH_2Ph$(4-CMe=NOMe) |
| F-0273 | $CH_2Ph(2-NO_2)$ |
| F-0274 | $CH_2Ph(3-NO_2)$ |
| F-0275 | $CH_2Ph(4-NO_2)$ |
| F-0276 | $CH_2Ph(2-NH_2)$ |
| F-0277 | $CH_2Ph(3-NH_2)$ |
| F-0278 | $CH_2Ph(4-NH_2)$ |
| F-0279 | $CH_2Ph$(2-OH) |
| F-0280 | $CH_2Ph$(3-OH) |
| F-0281 | $CH_2Ph$(4-OH) |
| F-0282 | $CH_2Ph$(2-SH) |
| F-0283 | $CH_2Ph$(3-SH) |
| F-0284 | $CH_2Ph$(4-SH) |

(continued)

| Compound Number | $R^9$ |
|---|---|
| F-0285 | $CH_2Ph(2-NMe_2)$ |
| F-0286 | $CH_2Ph(3-NMe_2)$ |
| F-0287 | $CH_2Ph(4-NMe_2)$ |
| F-0288 | $CH_2Ph$(2-NHMe) |

[Table 127]

| Compound Number | $R^9$ |
|---|---|
| F-0289 | $CH_2Ph$(3-NHMe) |
| F-0290 | $CH_2Ph$(4-NHMe) |
| F-0291 | $CH_2Ph$(2-CHO) |
| F-0292 | $CH_2Ph$(3-CHO) |
| F-0293 | $CH_2Ph$(4-CHO) |
| F-0294 | $CH_2Ph$(2-Ph) |
| F-0295 | $CH_2Ph$(3-Ph) |
| F-0296 | $CH_2Ph$(4-Ph) |
| F-0297 | $CH_2Ph$(2-pyridin-2-yl) |
| F-0298 | $CH_2Ph$(3-pyridin-2-yl) |
| F-0299 | $CH_2Ph$(4-pyridin-2-yl) |
| F-0300 | $CH_2Ph$(2-pyridin-3-yl) |
| F-0301 | $CH_2Ph$(3-pyridin-3-yl) |
| F-0302 | $CH_2Ph$(4-pyridin-3-yl) |
| F-0303 | $CH_2Ph$(2-pyridin-4-yl) |
| F-0304 | $CH_2Ph$(3-pyridin-4-yl) |
| F-0305 | $CH_2Ph$(4-pyridin-4-yl) |
| F-0306 | $CH_2Ph(2-CH_2Ph)$ |
| F-0307 | $CH_2Ph(3-CH_2Ph)$ |
| F-0308 | $CH_2Ph(4-CH_2Ph)$ |
| F-0309 | $CH_2Ph(2,3-F_2)$ |
| F-0310 | $CH_2Ph(2,4-F_2)$ |
| F-0311 | $CH_2Ph(2,5-F_2)$ |
| F-0312 | $CH_2Ph(2,6-F_2)$ |
| F-0313 | $CH_2Ph(3,4-F_2)$ |
| F-0314 | $CH_2Ph(3,5-F_2)$ |
| F-0315 | $CH_2Ph$(2-Cl,3-F) |

(continued)

| Compound Number | $R^9$ |
|---|---|
| F-0316 | $CH_2$Ph(2-Cl,4-F) |
| F-0317 | $CH_2$Ph(2-Cl,5-F) |
| F-0318 | $CH_2$Ph(2-Cl,6-F) |
| F-0319 | $CH_2$Ph(3-Cl,2-F) |
| F-0320 | $CH_2$Ph(3-Cl,4-F) |
| F-0321 | $CH_2$Ph(3-Cl,5-F) |
| F-0322 | $CH_2$Ph(4-Cl,2-F) |
| F-0323 | $CH_2$Ph(4-Cl,3-F) |
| F-0324 | $CH_2$Ph(5-Cl,2-F) |
| F-0325 | $CH_2$Ph(2-F,3-Me) |
| F-0326 | $CH_2$Ph(2-F,4-Me) |
| F-0327 | $CH_2$Ph(2-F,5-Me) |
| F-0328 | $CH_2$Ph(2-F,6-Me) |
| F-0329 | $CH_2$Ph(3-F,2-Me) |
| F-0330 | $CH_2$Ph(3-F,4-Me) |

[Table 128]

| Compound Number | $R^9$ |
|---|---|
| F-0331 | $CH_2$Ph(3-F,5-Me) |
| F-0332 | $CH_2$Ph(4-F,2-Me) |
| F-0333 | $CH_2$Ph(4-F,3-Me) |
| F-0334 | $CH_2$Ph(5-F,2-Me) |
| F-0335 | $CH_2$Ph(2-CN,3-F) |
| F-0336 | $CH_2$Ph(2-CN,4-F) |
| F-0337 | $CH_2$Ph(2-CN,5-F) |
| F-0338 | $CH_2$Ph(2-CN,6-F) |
| F-0339 | $CH_2$Ph(3-CN,2-F) |
| F-0340 | $CH_2$Ph(3-CN,4-F) |
| F-0341 | $CH_2$Ph(3-CN,5-F) |
| F-0342 | $CH_2$Ph(4-CN,2-F) |
| F-0343 | $CH_2$Ph(4-CN,3-F) |
| F-0344 | $CH_2$Ph(5-CN,2-F) |
| F-0345 | $CH_2$Ph(2-F,3-OMe) |
| F-0346 | $CH_2$Ph(2-F,4-OMe) |

(continued)

| Compound Number | $R^9$ |
|---|---|
| F-0347 | $CH_2Ph(2\text{-}F,5\text{-}OMe)$ |
| F-0348 | $CH_2Ph(2\text{-}F,6\text{-}OMe)$ |
| F-0349 | $CH_2Ph(3\text{-}F,2\text{-}OMe)$ |
| F-0350 | $CH_2Ph(3\text{-}F,4\text{-}OMe)$ |
| F-0351 | $CH_2Ph(3\text{-}F,5\text{-}OMe)$ |
| F-0352 | $CH_2Ph(4\text{-}F,2\text{-}OMe)$ |
| F-0353 | $CH_2Ph(4\text{-}F,3\text{-}OMe)$ |
| F-0354 | $CH_2Ph(5\text{-}F,2\text{-}OMe)$ |
| F-0355 | $CH_2Ph(2,3\text{-}Cl_2)$ |
| F-0356 | $CH_2Ph(2,4\text{-}Cl_2)$ |
| F-0357 | $CH_2Ph(2,5\text{-}Cl_2)$ |
| F-0358 | $CH_2Ph(2,6\text{-}Cl_2)$ |
| F-0359 | $CH_2ph(3,4\text{-}Cl_2)$ |
| F-0360 | $CH_2Ph(3,5\text{-}Cl_2)$ |
| F-0361 | $CH_2Ph(2\text{-}Cl,3\text{-}Me)$ |
| F-0362 | $CH_2Ph(2\text{-}Cl,4\text{-}Me)$ |
| F-0363 | $CH_2Ph(2\text{-}Cl,5\text{-}Me)$ |
| F-0364 | $CH_2Ph(2\text{-}Cl,6\text{-}Me)$ |
| F-0365 | $CH_2Ph(3\text{-}Cl,2\text{-}Me)$ |
| F-0366 | $CH_2Ph(3\text{-}Cl,4\text{-}Me)$ |
| F-0367 | $CH_2Ph(3\text{-}Cl,5\text{-}Me)$ |
| F-0368 | $CH_2Ph(4\text{-}Cl,2\text{-}Me)$ |
| F-0369 | $CH_2Ph(4\text{-}Cl,3\text{-}Me)$ |
| F-0370 | $CH_2Ph(5\text{-}Cl,2\text{-}Me)$ |
| F-0371 | $CH_2Ph(2\text{-}Cl,3\text{-}CN)$ |
| F-0372 | $CH_2Ph(2\text{-}Cl,4\text{-}CN)$ |

[Table 129]

| Compound Number | $R^9$ |
|---|---|
| F-0373 | $CH_2Ph(2\text{-}Cl,5\text{-}CN)$ |
| F-0374 | $CH_2Ph(2\text{-}Cl,6\text{-}CN)$ |
| F-0375 | $CH_2Ph(3\text{-}Cl,2\text{-}CN)$ |
| F-0376 | $CH_2Ph(3\text{-}Cl,4\text{-}CN)$ |
| F-0377 | $CH_2Ph(3\text{-}Cl,5\text{-}CN)$ |

(continued)

| Compound Number | $R^9$ |
|---|---|
| F-0378 | $CH_2$Ph(4-Cl,2-CN) |
| F-0379 | $CH_2$Ph(4-Cl,3-CN) |
| F-0380 | $CH_2$Ph(5-Cl,2-CN) |
| F-0381 | $CH_2$Ph(2-Cl,3-OMe) |
| F-0382 | $CH_2$Ph(2-Cl,4-OMe) |
| F-0383 | $CH_2$Ph(2-Cl,5-OMe) |
| F-0384 | $CH_2$Ph(2-Cl,6-OMe) |
| F-0385 | $CH_2$Ph(3-Cl,2-OMe) |
| F-0386 | $CH_2$Ph(3-Cl,4-OMe) |
| F-0387 | $CH_2$Ph(3-Cl,5-OMe) |
| F-0388 | $CH_2$Ph(4-Cl,2-OMe) |
| F-0389 | $CH_2$Ph(4-Cl,3-OMe) |
| F-0390 | $CH_2$Ph(5-Cl,2-OMe) |
| F-0391 | $CH_2$Ph(2,3-$Me_2$) |
| F-0392 | $CH_2$Ph(2,4-$Me_2$) |
| F-0393 | $CH_2$Ph(2,5-$Me_2$) |
| F-0394 | $CH_2$Ph(2,6-$Me_2$) |
| F-0395 | $CH_2$Ph(3,4-$Me_2$) |
| F-0396 | $CH_2$Ph(3,5-$Me_2$) |
| F-0397 | $CH_2$Ph(2-CN,3-Me) |
| F-0398 | $CH_2$Ph(2-CN,4-Me) |
| F-0399 | $CH_2$Ph(2-CN,5-Me) |
| F-0400 | $CH_2$Ph(2-CN,6-Me) |
| F-0401 | $CH_2$Ph(3-CN,2-Me) |
| F-0402 | $CH_2$Ph(3-CN,4-Me) |
| F-0403 | $CH_2$Ph(3-CN,5-Me) |
| F-0404 | $CH_2$Ph(4-CN,2-Me) |
| F-0405 | $CH_2$Ph(4-CN,3-Me) |
| F-0406 | $CH_2$Ph(5-CN,2-Me) |
| F-0407 | $CH_2$Ph(2-OMe,3-Me) |
| F-0408 | $CH_2$Ph(2-OMe,4-Me) |
| F-0409 | $CH_2$Ph(2-OMe,5-Me) |
| F-0410 | $CH_2$Ph(2-OMe,6-Me) |
| F-0411 | $CH_2$Ph(3-OMe,2-Me) |

(continued)

| Compound Number | $R^9$ |
|---|---|
| F-0412 | $CH_2Ph(3\text{-}OMe,4\text{-}Me)$ |
| F-0413 | $CH_2Ph(3\text{-}OMe,5\text{-}Me)$ |
| F-0414 | $CH_2Ph(4\text{-}OMe,2\text{-}Me)$ |

[Table 130]

| Compound Number | $R^9$ |
|---|---|
| F-0415 | $CH_2Ph(4\text{-}OMe,3\text{-}Me)$ |
| F-0416 | $CH_2Ph(5\text{-}OMe,2\text{-}Me)$ |
| F-0417 | $CH_2Ph[2,3\text{-}(OMe)_2]$ |
| F-0418 | $CH_2Ph[2,4\text{-}(OMe)_2]$ |
| F-0419 | $CH_2Ph[2,5\text{-}(OMe)_2]$ |
| F-0420 | $CH_2Ph[2,6\text{-}(OMe)_2]$ |
| F-0421 | $CH_2Ph[3,4\text{-}(OMe)_2]$ |
| F-0422 | $CH_2Ph[3,5\text{-}(OMe)_2]$ |
| F-0423 | $CH_2Ph(2\text{-}CN,3\text{-}OMe)$ |
| F-0424 | $CH_2Ph(2\text{-}CN,4\text{-}OMe)$ |
| F-0425 | $CH_2Ph(2\text{-}CN,5\text{-}OMe)$ |
| F-0426 | $CH_2Ph(2\text{-}CN,6\text{-}OMe)$ |
| F-0427 | $CH_2Ph(3\text{-}CN,2\text{-}OMe)$ |
| F-0428 | $CH_2Ph(3\text{-}CN,4\text{-}OMe)$ |
| F-0429 | $CH_2Ph(3\text{-}CN,5\text{-}OMe)$ |
| F-0430 | $CH_2Ph(4\text{-}CN,2\text{-}OMe)$ |
| F-0431 | $CH_2Ph(4\text{-}CN,3\text{-}OMe)$ |
| F-0432 | $CH_2Ph(5\text{-}CN,2\text{-}OMe)$ |
| F-0433 | $CH_2Ph[2,3\text{-}(CN)_2]$ |
| F-0434 | $CH_2Ph[2,4\text{-}(CN)_2]$ |
| F-0435 | $CH_2Ph[2,5\text{-}(CN)_2]$ |
| F-0436 | $CH_2Ph[2,6\text{-}(CN)_2]$ |
| F-0437 | $CH_2Ph[3,4\text{-}(CN)_2]$ |
| F-0438 | $CH_2Ph[3,5\text{-}(CN)_2]$ |
| F-0439 | $CH_2CH_2Ph(2\text{-}F)$ |
| F-0440 | $CH_2CH_2Ph(3\text{-}F)$ |
| F-0441 | $CH_2CH_2Ph(4\text{-}F)$ |
| F-0442 | $CH_2CH{=}CHPh$ |

(continued)

| Compound Number | $R^9$ |
|---|---|
| F-0443 | $CH_2CCPh$ |
| F-0444 | $CH_2CCTMS$ |
| F-0445 | $CH_2F$ |
| F-0446 | $CH_2CH_2F$ |
| F-0447 | $CH_2CH_2CH_2F$ |
| F-0448 | $CH_2CHO$ |
| F-0449 | $CH_2CH_2CHO$ |
| F-0450 | $CH_2COEt$ |
| F-0451 | $CH_2CO$n-Pr |
| F-0452 | $CH_2CO$n-Bu |
| F-0453 | $CH_2CO$i-Pr |
| F-0454 | $CH_2CO$t-Bu |
| F-0455 | $CH_2CO$i-Bu |
| F-0456 | $CH_2CO$sec-Bu |

[Table 131]

| Compound Number | $R^9$ |
|---|---|
| F-0457 | $CH_2COCH_2$t-Bu |
| F-0458 | $CH_2COCH(CH_2CH_3)Et$ |
| F-0459 | $CH_2CO$c-Pr |
| F-0460 | $CH_2COCH_2$c-Pr |
| F-0461 | $CH_2CO$c-Bu |
| F-0462 | $CH_2CO$c-Pen |
| F-0463 | $CH_2CH_2COMe$ |
| F-0464 | $CH_2CH_2COEt$ |
| F-0465 | $CH_2COCF_3$ |
| F-0466 | $CH_2COCHF_2$ |
| F-0467 | $CH_2COCH_2CF_3$ |
| F-0468 | $CH_2COCH_2CHF_2$ |
| F-0469 | $CH_2COCH_2OCH_3$ |
| F-0470 | $CH_2COCH_2Ph$ |
| F-0471 | $CH_2CO$(pyrrolidin-1-yl) |
| F-0472 | $CH_2CO$(1H-pyrazol-1-yl) |
| F-0473 | $CH_2CO$(morpholino-1-yl) |

(continued)

| Compound Number | $R^9$ |
|---|---|
| F-0474 | $CH_2CO$(pyridin-2-yl) |
| F-0475 | $CH_2CO$(pyridin-3-yl) |
| F-0476 | $CH_2CO$(pyridin-4-yl) |
| F-0477 | $CH_2COCH_2$(pyridin-2-yl) |
| F-0478 | $CH_2COCH_2$(pyridin-3-yl) |
| F-0479 | $CH_2COCH_2$(pyridin-4-yl) |
| F-0480 | $CH_2CO_2H$ |
| F-0481 | $CH_2CH_2CO_2Me$ |
| F-0482 | $CH(CH_2CH_3)CO_2Et$ |
| F-0483 | $CH(F)CO_2Et$ |
| F-0484 | $CH(CO_2Et)_2$ |
| F-0485 | $CH_2CH_2CO_2Et$ |
| F-0486 | $CH_2CH_2CH_2CO_2Et$ |
| F-0487 | $CH_2CH_2CH_2CH_2CH_2CO_2Et$ |
| F-0488 | $CH_2CO_2$n-Pr |
| F-0489 | $CH(CH_3)CO_2$n-Pr |
| F-0490 | $C(CH_3)(CH_3)CO_2$n-Pr |
| F-0491 | $CH_2CO_2$i-Pr |
| F-0492 | $CH(CH_3)CO_2$i-Pr |
| F-0493 | $C(CH_3)(CH_3)CO_2$i-Pr |
| F-0494 | $CH_2CO_2$n-Bu |
| F-0495 | $CH_2CO_2$t-Bu |
| F-0496 | $CH_2CO_2$i-Bu |
| F-0497 | $CH_2CO_2$c-Pr |
| F-0498 | $CH_2CO_2CH_2$c-Pr |

[Table 132]

| Compound Number | $R^9$ |
|---|---|
| F-0499 | $CH_2CO_2$c-Bu |
| F-0500 | $CH_2CO_2$c-Pen |
| F-0501 | $CH_2CO_2CH=CH_2$ |
| F-0502 | $CH_2CO_2CH_2CH=CH_2$ |
| F-0503 | $CH_2CO_2CH_2CCH$ |
| F-0504 | $CH_2CO_2CF_3$ |

(continued)

| Compound Number | $R^9$ |
|---|---|
| F-0505 | $CH_2CO_2CHF_2$ |
| F-0506 | $CH_2CO_2CH_2CF_3$ |
| F-0507 | $CH_2CO_2CH_2CHF_2$ |
| F-0508 | $CH_2CO_2CH_2CN$ |
| F-0509 | $CH_2CO_2CH_2COMe$ |
| F-0510 | $CH_2CO_2CH_2CO_2Et$ |
| F-0511 | $CH_2CO_2CH_2CO_2CH_2CF_3$ |
| F-0512 | $CH_2CO_2CH_2Ph$ |
| F-0513 | $CH_2CO_2$(pyridin-2-yl) |
| F-0514 | $CH_2CO_2$(pyridin-3-yl) |
| F-0515 | $CH_2CO_2$(pyridin-4-yl) |
| F-0516 | $CH_2CO_2CH_2$(pyridin-2-yl) |
| F-0517 | $CH_2CO_2CH_2$[pyridin-2-yl(6-F)] |
| F-0518 | $CH_2CO_2CH_2$[pyridin-2-yl(6-Cl)] |
| F-0519 | $CH_2CO_2CH_2$[pyridin-2-yl(6-Me)] |
| F-0520 | $CH_2CO_2CH_2$(pyridin-3-yl) |
| F-0521 | $CH_2CO_2CH_2$[pyridin-3-yl(6-F)] |
| F-0522 | $CH_2CO_2CH_2$[pyridin-3-yl(6-Cl)] |
| F-0523 | $CH_2CO_2CH_2$[pyridin-3-yl(6-Me)] |
| F-0524 | $CH_2CO_2CH_2$(pyridin-4-yl) |
| F-0525 | $CH_2CO_2CH_2$(1H-pyrazol-1-yl) |
| F-0526 | $CH_2CO_2CH_2$[1H-pyrazol-3-yl(1-Me)] |
| F-0527 | $CH_2CO_2CH_2$[1H-pyrazol-4-yl(1-Me)] |
| F-0528 | $CH_2CO_2CH_2$[1H-pyrazol-5-yl(1-Me)] |
| F-0529 | $CH_2CONMe_2$ |
| F-0530 | $CH_2CON(CH_3)Et$ |
| F-0531 | $CH_2CON(CH_3)$n-Pr |
| F-0532 | $CH_2CONEt_2$ |
| F-0533 | $CH_2CONH$n-Pr |
| F-0534 | $CH_2CONH$i-Pr |
| F-0535 | $CH_2CONH$n-Bu |
| F-0536 | $CH_2CONH$i-Bu |
| F-0537 | $CH_2CONH$c-Pr |
| F-0538 | $CH_2CONH$cPr(1-CN) |

(continued)

| Compound Number | $R^9$ |
|---|---|
| F-0539 | $CH_2CONHCH_2$c-Pr |
| F-0540 | $CH_2CONHCH{=}CH_2$ |

[Table 133]

| Compound Number | $R^9$ |
|---|---|
| F-0541 | $CH_2CONHCH_2CH{=}CH_2$ |
| F-0542 | $CH_2CONHCH_2CCH$ |
| F-0543 | $CH_2CONHCF_3$ |
| F-0544 | $CH_2CONHCHF_2$ |
| F-0545 | $CH_2CONHCH_2CF_3$ |
| F-0546 | $CH_2CONHCH_2CHF_2$ |
| F-0547 | $CH_2CH_2CONHMe$ |
| F-0548 | $CH_2CH_2CONHEt$ |
| F-0549 | $CH_2CH_2CONMe_2$ |
| F-0550 | $CH_2CH_2CON(CH_3)Et$ |
| F-0551 | $CH_2CONHCH_2Ph$ |
| F-0552 | $CH_2CONH$(pyridin-2-yl) |
| F-0553 | $CH_2CONH$(pyridin-3-yl) |
| F-0554 | $CH_2CONH$(pyridin-4-yl) |
| F-0555 | $CH_2CONHCH_2$(pyridin-2-yl) |
| F-0556 | $CH_2CONHCH_2$(pyridin-3-yl) |
| F-0557 | $CH_2CONHCH_2$(pyridin-4-yl) |
| F-0558 | $CH_2CH_2NHCHO$ |
| F-0559 | $CH_2CH_2NHCOMe$ |
| F-0560 | $CH_2CH_2NHCOEt$ |
| F-0561 | $CH_2CH_2N(CH_3)CHO$ |
| F-0562 | $CH_2CH_2N(CH_3)COMe$ |
| F-0563 | $CH_2CH_2N(CH_3)COEt$ |
| F-0564 | $CH_2CH_2N(CH_2CH_3)CHO$ |
| F-0565 | $CH_2CH_2N(CH_2CH_3)COMe$ |
| F-0566 | $CH_2CH_2N(CH_2CH_3)COEt$ |
| F-0567 | $CH_2NHCO$c-Pr |
| F-0568 | $CH_2NHCOCH_2$c-Pr |
| F-0569 | $CH_2CH_2NHCO$c-Pr |

(continued)

| Compound Number | $R^9$ |
|---|---|
| F-0570 | $CH_2CH_2NHCOCH_2$c-Pr |
| F-0571 | $CH_2NHCOCF_3$ |
| F-0572 | $CH_2NHCOCHF_2$ |
| F-0573 | $CH_2NHCOCH_2CF_3$ |
| F-0574 | $CH_2NHCOCH_2CHF_2$ |
| F-0575 | $CH_2NHCOCH_2Ph$ |
| F-0576 | $CH_2NHCO$(pyridin-2-yl) |
| F-0577 | $CH_2NHCO$(pyridin-3-yl) |
| F-0578 | $CH_2NHCO$(pyridin-4-yl) |
| F-0579 | $CH_2NHCOCH_2$(pyridin-2-yl) |
| F-0580 | $CH_2NHCOCH_2$(pyridin-3-yl) |
| F-0581 | $CH_2NHCOCH_2$(pyridin-4-yl) |
| F-0582 | $CH_2OCHO$ |

[Table 134]

| Compound Number | $R^9$ |
|---|---|
| F-0583 | $CH_2CH_2OCHO$ |
| F-0584 | $CH_2OCOMe$ |
| F-0585 | $CH_2OCOEt$ |
| F-0586 | $CH_2OCO$n-Pr |
| F-0587 | $CH_2OCO$i-Pr |
| F-0588 | $CH_2OCO$n-Bu |
| F-0589 | $CH_2OCO$i-Bu |
| F-0590 | $CH_2OCO$t-Bu |
| F-0591 | $CH_2CH_2OCOMe$ |
| F-0592 | $CH_2CH_2OCOEt$ |
| F-0593 | $CH_2CH_2CH_2OCOMe$ |
| F-0594 | $CH_2OCO$c-Pr |
| F-0595 | $CH_2OCOCH_2$c-Pr |
| F-0596 | $CH_2OCOCF_3$ |
| F-0597 | $CH_2OCOCHF_2$ |
| F-0598 | $CH_2OCOCH_2CF_3$ |
| F-0599 | $CH_2OCOCH_2CHF_2$ |
| F-0600 | $CH_2OCOPh$ |

(continued)

| Compound Number | $R^9$ |
|---|---|
| F-0601 | $CH_2OCOCH_2Ph$ |
| F-0602 | $CH_2OCO$(pyridin-2-yl) |
| F-0603 | $CH_2OCO$(pyridin-3-yl) |
| F-0604 | $CH_2OCO$(pyridin-4-yl) |
| F-0605 | $CH_2OCOCH_2$(pyridin-2-yl) |
| F-0606 | $CH_2OCOCH_2$(pyridin-3-yl) |
| F-0607 | $CH_2OCOCH_2$(pyridin-4-yl) |
| F-0608 | $CH_2SO_2NHMe$ |
| F-0609 | $CH_2SO_2NHEt$ |
| F-0610 | $CH_2SO_2NMe_2$ |
| F-0611 | $CH_2SO_2N(CH_3)Et$ |
| F-0612 | $CH_2CH_2SO_2NHMe$ |
| F-0613 | $CH_2CH_2SO_2NHEt$ |
| F-0614 | $CH_2CH_2SO_2NMe_2$ |
| F-0615 | $CH_2CH_2SO_2(CH_3)Et$ |
| F-0616 | $CH_2SO_2NHc$-Pr |
| F-0617 | $CH_2SO_2NHCH_2c$-Pr |
| F-0618 | $CH_2SO_2NHCF_3$ |
| F-0619 | $CH_2SO_2NHCH_2CF_3$ |
| F-0620 | $CH_2SO_2NHCHF_2$ |
| F-0621 | $CH_2SO_2NHCH_2CHF_2$ |
| F-0622 | $CH_2SO_2NHPh$ |
| F-0623 | $CH_2SO_2NHCH_2Ph$ |
| F-0624 | $CH_2SO_2NH$(pyridin-2-yl) |

[Table 135]

| Compound Number | $R^9$ |
|---|---|
| F-0625 | $CH_2SO_2NH$(pyridin-3-yl) |
| F-0626 | $CH_2SO_2NH$(pyridin-4-yl) |
| F-0627 | $CH_2SO_2NHCH_2$(pyridin-2-yl) |
| F-0628 | $CH_2SO_2NHCH_2$(pyridin-3-yl) |
| F-0629 | $CH_2SO_2NHCH_2$(pyridin-4-yl) |
| F-0630 | $CH_2NHSO_2Me$ |
| F-0631 | $CH_2N(CH_3)SO_2Me$ |

(continued)

| Compound Number | $R^9$ |
|---|---|
| F-0632 | $CH_2NHSO_2Et$ |
| F-0633 | $CH_2NHSO_2$c-Pr |
| F-0634 | $CH_2NHSO_2CH_2$c-Pr |
| F-0635 | $CH_2NHSO_2CF_3$ |
| F-0636 | $CH_2NHSO_2CH_2CF_3$ |
| F-0637 | $CH_2NHSO_2CHF_2$ |
| F-0638 | $CH_2NHSO_2CH_2CHF_2$ |
| F-0639 | $CH_2CH_2NHSO_2Me$ |
| F-0640 | $CH_2CH_2N(CH_3)SO_2Me$ |
| F-0641 | $CH_2NHSO_2Ph$ |
| F-0642 | $CH_2NHSO_2CH_2Ph$ |
| F-0643 | $CH_2NHSO_2$(pyridin-2-yl) |
| F-0644 | $CH_2NHSO_2$(pyridin-3-yl) |
| F-0645 | $CH_2NHSO_2$(pyridin-4-yl) |
| F-0646 | $CH_2NHSO_2CH_2$(pyridin-2-yl) |
| F-0647 | $CH_2NHSO_2CH_2$(pyridin-3-yl) |
| F-0648 | $CH_2NHSO_2CH_2$(pyridin-4-yl) |
| F-0649 | $CH_2OH$ |
| F-0650 | $CH_2CH_2OH$ |
| F-0651 | $CH_2CH(OH)CH_2CH_2CH_3$ |
| F-0652 | $CH_2CH_2O$n-Pr |
| F-0653 | $CH_2CH_2O$i-Pr |
| F-0654 | $CH_2CH_2O$c-Pr |
| F-0655 | $CH_2CH_2OCH_2$c-Pr |
| F-0656 | $CH_2CH_2O$t-Bu |
| F-0657 | $CH_2CH_2O$i-Bu |
| F-0658 | $CH_2CH_2O$sec-Bu |
| F-0659 | $CH(CH_3)OEt$ |
| F-0660 | $CH_2CH(CH_3)OEt$ |
| F-0661 | $CH_2CH(OMe)_2$ |
| F-0662 | $CH_2CH(OEt)_2$ |
| F-0663 | $CH(CH_3)CH(OMe)_2$ |
| F-0664 | $CH_2C(OMe)_2Me$ |
| F-0665 | $CH(CH_3)CH(OEt)_2$ |

(continued)

| Compound Number | $R^9$ |
|---|---|
| F-0666 | $CH_2C(OEt)_2Me$ |

[Table 136]

| Compound Number | $R^9$ |
|---|---|
| F-0667 | $CH_2CH_2CH(OMe)_2$ |
| F-0668 | $CH_2CH_2CH(OEt)_2$ |
| F-0669 | $CH_2CH_2CH_2OMe$ |
| F-0670 | $CH_2OCHF_2$ |
| F-0671 | $CH_2OCH_2CHF_2$ |
| F-0672 | $CH_2C(OH)_2CF_3$ |
| F-0673 | $CH_2CH_2OCHF_2$ |
| F-0674 | $CH_2CH_2OCH_2CHF_2$ |
| F-0675 | $CH_2OCH_2Ph$ |
| F-0676 | $CH_2OCH_2CH_2OMe$ |
| F-0677 | $CH_2CH_2OCH_2CH_2OMe$ |
| F-0678 | $CH_2O$(pyridin-2-yl) |
| F-0679 | $CH_2O$(pyridin-3-yl) |
| F-0680 | $CH_2O$(pyridin-4-yl) |
| F-0681 | $CH_2OCH_2$(pyridin-2-yl) |
| F-0682 | $CH_2OCH_2$(pyridin-3-yl) |
| F-0683 | $CH_2OCH_2$(pyridin-4-yl) |
| F-0684 | $CH_2SH$ |
| F-0685 | $CH_2CH_2SH$ |
| F-0686 | $CH_2SOMe$ |
| F-0687 | $CH_2SO_2Me$ |
| F-0688 | $CH_2SOEt$ |
| F-0689 | $CH_2SO_2Et$ |
| F-0690 | $CH_2CH_2SOMe$ |
| F-0691 | $CH_2CH_2SO_2Me$ |
| F-0692 | $CH_2CH_2SOEt$ |
| F-0693 | $CH_2CH_2SO_2Et$ |
| F-0694 | $CH_2Sc$-Pr |
| F-0695 | $CH_2SOc$-Pr |
| F-0696 | $CH_2SO_2c$-Pr |

(continued)

| Compound Number | $R^9$ |
|---|---|
| F-0697 | $CH_2SCH_2$c-Pr |
| F-0698 | $CH_2SOCH_2$c-Pr |
| F-0699 | $CH_2SO_2CH_2$c-Pr |
| F-0700 | $CH_2CH_2$Si-Pr |
| F-0701 | $CH_2CH_2$SOi-Pr |
| F-0702 | $CH_2CH_2SO_2$i-Pr |
| F-0703 | $CH_2SOCF_3$ |
| F-0704 | $CH_2SO_2CF_3$ |
| F-0705 | $CH_2SOCHF_2$ |
| F-0706 | $CH_2SO_2CHF_2$ |
| F-0707 | $CH_2SCH_2$Ph |
| F-0708 | $CH_2SOCH_2$Ph |

[Table 137]

| Compound Number | $R^9$ |
|---|---|
| F-0709 | $CH_2SO_2CH_2$Ph |
| F-071 0 | $CH_2$S(pyridin-2-yl) |
| F-0711 | $CH_2$SO(pyridin-2-yl) |
| F-0712 | $CH_2SO_2$(pyridin-2-yl) |
| F-0713 | $CH_2SCH_2$(pyridin-2-yl) |
| F-0714 | $CH_2SOCH_2$(pyridin-2-yl) |
| F-0715 | $CH_2SO_2CH_2$(pyridin-2-yl) |
| F-0716 | $CH_2$S(pyridin-3-yl) |
| F-0717 | $CH_2$SO(pyridin-3-yl) |
| F-0718 | $CH_2SO_2$(pyridin-3-yl) |
| F-0719 | $CH_2SCH_2$(pyridin-3-yl) |
| F-0720 | $CH_2SOCH_2$(pyridin-3-yl) |
| F-0721 | $CH_2SO_2CH_2$(pyridin-3-yl) |
| F-0722 | $CH_2$S(pyridin-4-yl) |
| F-0723 | $CH_2$SO(pyridin-4-yl) |
| F-0724 | $CH_2SO_2$(pyridin-4-yl) |
| F-0725 | $CH_2SCH_2$(pyridin-4-yl) |
| F-0726 | $CH_2SOCH_2$(pyridin-4-yl) |
| F-0727 | $CH_2SO_2CH_2$(pyridin-4-yl) |

(continued)

| Compound Number | $R^9$ |
|---|---|
| F-0728 | $CH_2CH(=NOH)$ |
| F-0729 | $CH_2CH(=NOMe)$ |
| F-0730 | $CH_2CMe(=NOMe)$ |
| F-0731 | $CH_2CEt(=NOMe)$ |
| F-0732 | $CH_2$Cn-Pr(=NOMe) |
| F-0733 | $CH_2CMe(=NOEt)$ |
| F-0734 | $CH_2CMe(=NOCH_2CH=CH_2)$ |
| F-0735 | $CH_2CMe(=NOPh)$ |
| F-0736 | $CH_2CMe(=NOCH_2Ph)$ |
| F-0737 | $CH_2$CMe[=NO(pyridin-2-yl)] |
| F-0738 | $CH_2$CMe[=NO(pyridin-3-yl)] |
| F-0739 | $CH_2$CMe[=NO(pyridin-4-yl)] |
| F-0740 | $CH_2$CMe[=NO$CH_2$(pyridin-2-yl)] |
| F-0741 | $CH_2$CMe[=NO$CH_2$(pyridin-3-yl)] |
| F-0742 | $CH_2$CMe[=NO$CH_2$(pyridin-4-yl)] |
| F-0743 | dihydrofuran-2(3H)-one-3-yl |
| F-0744 | $CH_2OCO_2Me$ |
| F-0745 | $CH_2OCO_2Et$ |
| F-0746 | $CH_2OCO_2$n-Pr |
| F-0747 | $CH_2OCO_2$i-Pr |
| F-0748 | $CH_2OCO_2$c-Pr |
| F-0749 | $CH_2OCO_2CH_2$c-Pr |
| F-0750 | $CH_2OCO_2$n-Bu |

[Table 138]

| Compound Number | $R^9$ |
|---|---|
| F-0751 | $CH_2OCO_2$i-Bu |
| F-0752 | $CH_2OCO_2$t-Bu |
| F-0753 | $CH_2OCO_2CF_3$ |
| F-0754 | $CH_2OCO_2CH_2CF_3$ |
| F-0755 | $CH_2OCO_2CHF_2$ |
| F-0756 | $CH_2OCO_2CH_2CHF_2$ |
| F-0757 | $CH_2CH_2OCO_2Me$ |
| F-0758 | $CH_2CH_2OCO_2Et$ |

(continued)

| Compound Number | $R^9$ |
|---|---|
| F-0759 | $CH_2OCO_2Ph$ |
| F-0760 | $CH_2OCO_2CH_2Ph$ |
| F-0761 | $CH_2OCO_2$(pyridin-2-yl) |
| F-0762 | $CH_2OCO_2$(pyridin-3-yl) |
| F-0763 | $CH_2OCO_2$(pyridin-4-yl) |
| F-0764 | $CH_2OCO_2CH_2$(pyridin-2-yl) |
| F-0765 | $CH_2OCO_2CH_2$(pyridin-3-yl) |
| F-0766 | $CH_2OCO_2CH_2$(pyridin-4-yl) |
| F-0767 | $CH_2NHCONHMe$ |
| F-0768 | $CH_2NHCONHEt$ |
| F-0769 | $CH_2NHCONHn$-Pr |
| F-0770 | $CH_2NHCONHi$-Pr |
| F-0771 | $CH_2NHCONHc$-Pr |
| F-0772 | $CH_2NHCONHCH_2c$-Pr |
| F-0773 | $CH_2NHCONHn$-Bu |
| F-0774 | $CH_2NHCONHi$-Bu |
| F-0775 | $CH_2NHCONHt$-Bu |
| F-0776 | $CH_2NHCONHCF_3$ |
| F-0777 | $CH_2NHCONHCH_2CF_3$ |
| F-0778 | $CH_2NHCONHCHF_2$ |
| F-0779 | $CH_2NHCONHCH_2CHF_2$ |
| F-0780 | $CH_2CH_2NHCONHMe$ |
| F-0781 | $CH_2CH_2NHCONHEt$ |
| F-0782 | $CH_2NHCONHPh$ |
| F-0783 | $CH_2NHCONHCH_2Ph$ |
| F-0784 | $CH_2NHCONH$(pyridin-2-yl) |
| F-0785 | $CH_2NHCONH$(pyridin-3-yl) |
| F-0786 | $CH_2NHCONH$(pyridin-4-yl) |
| F-0787 | $CH_2NHCONH_2CH_2$(pyridin-2-yl) |
| F-0788 | $CH_2NHCONHCH_2$(pyridin-3-yl) |
| F-0789 | $CH_2NHCONHCH_2$(pyridin-4-yl) |
| F-0790 | $CH_2CONHNH_2$ |
| F-0791 | $CH_2CONHNMe_2$ |
| F-0792 | $CH_2CONHNHCO_2t$-Bu |

(continued)

| Compound Number | $R^9$ |
|---|---|
| F-0793 | $CH_2CHCCl_2$ |
| F-0794 | $CH_2CO_2CH_2CH_2$(morpholin-4-yl) |

[Table 139]

| Compound Number | $R^9$ |
|---|---|
| F-0795 | $CH_2CO_2CH_2CH_2OMe$ |
| F-0796 | $CH_2CH_2NHCO_2tBu$ |
| F-0797 | $CH_2CH_2N(COCF_3)CO_2tBu$ |
| F-0798 | $CH_2CH_2NMe_2$ |
| F-0799 | $CH_2CONHn$-Pen |
| F-0800 | $CH_2CONHn$-Hex |
| F-0801 | $CH_2CONHCH_2CH_2OMe$ |
| F-0802 | $CH_2CH(Cl)CH_2SF_5$ |
| F-0803 | $CH_2CH{=}CHSF_5$ |
| F-0804 | $CH_2CO_2$(tetrahydrofuran-3-yl) |

[Table 140]

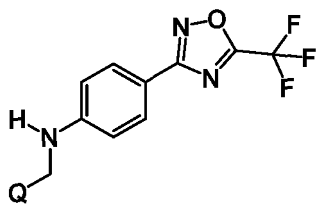

| Compound Number | Q |
|---|---|
| G-0001 | naphthalen-1-yl |
| G-0002 | naphthalen-2-yl |
| G-0003 | quinolin-2-yl |
| G-0004 | quinolin-3-yl |
| G-0005 | quinolin-6-yl |
| G-0006 | quinolin-7-yl |
| G-0007 | 1H-pyrrolo[2,3-b]pyridin-3-yl |
| G-0008 | benzo[d][1,3]dioxol-4-yl |
| G-0009 | benzo[d][1,3]dioxol-5-yl |
| G-0010 | benzo[d][1,3]dioxol-4-yl(2,2-$F_2$) |
| G-0011 | benzo[d][1,3]dioxol-5-yl(2,2-$F_2$) |
| G-0012 | pyridin-2-yl |

(continued)

| 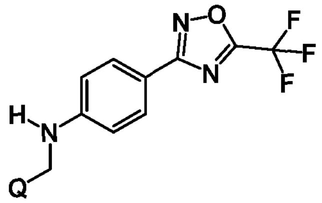 | |
|---|---|
| Compound Number | Q |
| G-0013 | $CH_2$pyridin-2-yl |
| G-0014 | pyridi n-2-yl(3-F) |
| G-0015 | pyridi n-2-yl(4-F) |
| G-0016 | pyridi n-2-yl(5-F) |
| G-0017 | pyridin-2-yl(6-F) |
| G-0018 | pyridin-2-yl(3-Cl) |
| G-0019 | pyridin-2-yl(4-Cl) |
| G-0020 | pyridin-2-yl(5-Cl) |
| G-0021 | pyridin-2-yl(6-Cl) |
| G-0022 | pyridin-2-yl(3-Me) |
| G-0023 | pyridin-2-yl(4-Me) |
| G-0024 | pyridin-2-yl(5-Me) |
| G-0025 | pyridin-2-yl(6-Me) |
| G-0026 | pyridin-2-yl(3-OMe) |
| G-0027 | pyridin-2-yl(4-OMe) |
| G-0028 | pyridin-2-yl(5-0Me) |
| G-0029 | pyridin-2-yl(6-OMe) |
| G-0030 | pyridin-2-yl(3-CN) |
| G-0031 | pyridin-2-yl(4-CN) |
| G-0032 | pyridin-2-yl(5-CN) |
| G-0033 | pyridin-2-yl(6-CN) |
| G-0034 | pyridin-2-yl(3-$CF_3$) |
| G-0035 | pyridin-2-yl(4-$CF_3$) |
| G-0036 | pyridin-2-yl(5-$CF_3$) |

[Table 141]

| Compound Number | Q |
|---|---|
| G-0037 | pyridin-2-yl(6-$CF_3$) |
| G-0038 | pyridin-2-yl(3-$CO_2$Et) |
| G-0039 | pyridin-2-yl(4-$CO_2$Et) |
| G-0040 | pyridin-2-yl(5-$CO_2$Et) |

(continued)

| Compound Number | Q |
|---|---|
| G-0041 | pyridin-2-yl(6-$CO_2$Et) |
| G-0042 | pyridin-3-yl |
| G-0043 | $CH_2$pyridin-3-yl |
| G-0044 | pyridin-3-yl(2-F) |
| G-0045 | pyridin-3-yl(4-F) |
| G-0046 | pyridin-3-yl(5-F) |
| G-0047 | pyridin-3-yl(6-F) |
| G-0048 | pyridin-3-yl(2-Cl) |
| G-0049 | pyridin-3-yl(4-Cl) |
| G-0050 | pyridin-3-yl(5-Cl) |
| G-0051 | pyridin-3-yl(6-Cl) |
| G-0052 | pyridin-3-yl(2-Me) |
| G-0053 | pyridin-3-yl(4-Me) |
| G-0054 | pyridin-3-yl(5-Me) |
| G-0055 | pyridin-3-yl(6-Me) |
| G-0056 | pyridin-3-yl(2-OMe) |
| G-0057 | pyridin-3-yl(4-OMe) |
| G-0058 | pyridin-3-yl(5-OMe) |
| G-0059 | pyridin-3-yl(6-OMe) |
| G-0060 | pyridin-3-yl(2-CN) |
| G-0061 | pyridin-3-yl(4-CN) |
| G-0062 | pyridin-3-yl(5-CN) |
| G-0063 | pyridin-3-yl(6-CN) |
| G-0064 | pyridin-3-yl(2-$CF_3$) |
| G-0065 | pyridin-3-yl(4-$CF_3$) |
| G-0066 | pyridin-3-yl(5-$CF_3$) |
| G-0067 | pyridin-3-yl(6-$CF_3$) |
| G-0068 | pyridin-3-yl(2-$CO_2$Et) |
| G-0069 | pyridin-3-yl(4-$CO_2$Et) |
| G-0070 | pyridin-3-yl(5-$CO_2$Et) |
| G-0071 | pyridin-3-yl(6-$CO_2$Et) |
| G-0072 | pyridin-4-yl |
| G-0073 | $CH_2$pyridin-4-yl |
| G-0074 | pyridin-4-yl(2-F) |
| G-0075 | pyridin-4-yl(3-F) |
| G-0076 | pyridin-4-yl(2-Cl) |

(continued)

| Compound Number | Q |
|---|---|
| G-0077 | pyridin-4-yl(3-Cl) |
| G-0078 | pyridin-4-yl(2-Me) |

[Table 142]

| Compound Number | Q |
|---|---|
| G-0079 | pyridin-4-yl(3-Me) |
| G-0080 | pyridin-4-yl(2-OMe) |
| G-0081 | pyridin-4-yl(3-OMe) |
| G-0082 | pyridin-4-yl(2-CN) |
| G-0083 | pyridin-4-yl(3-CN) |
| G-0084 | pyridin-4-yl(2-$CF_3$) |
| G-0085 | pyridin-4-yl(3-$CF_3$) |
| G-0086 | pyridin-4-yl(2-$CO_2$Et) |
| G-0087 | pyridin-4-yl(3-$CO_2$Et) |
| G-0088 | pyrimidin-2-yl |
| G-0089 | $CH_2$pyrimidin-2-yl |
| G-0090 | pyrimidin-2-yl(4-F) |
| G-0091 | pyrimidin-2-yl(5-F) |
| G-0092 | pyrimidin-2-yl(4-Cl) |
| G-0093 | pyrimidin-2-yl(5-Cl) |
| G-0094 | pyrimidin-2-yl(4-Me) |
| G-0095 | pyrimidin-2-yl(5-Me) |
| G-0096 | pyrimidin-2-yl(4-OMe) |
| G-0097 | pyrimidin-2-yl(5-OMe) |
| G-0098 | pyrimidin-2-yl(4-CN) |
| G-0099 | pyrimidin-2-yl(5-CN) |
| G-0100 | pyrimidin-2-yl(4-$CF_3$) |
| G-0101 | pyrimidin-2-yl(5-$CF_3$) |
| G-0102 | pyrimidin-2-yl(4-$CO_2$Et) |
| G-0103 | pyrimidin-2-yl(5-$CO_2$Et) |
| G-0104 | pyrimidin-4-yl |
| G-0105 | $CH_2$pyrimidin-4-yl |
| G-0106 | pyrimidin-4-yl(2-F) |
| G-0107 | pyrimidin-4-yl(5-F) |
| G-0108 | pyrimidin-4-yl(6-F) |
| G-0109 | pyrimidin-4-yl(2-Cl) |

(continued)

| Compound Number | Q |
|---|---|
| G-0110 | pyrimidin-4-yl(5-Cl) |
| G-0111 | pyrimidin-4-yl(6-Cl) |
| G-0112 | pyrimidin-4-yl(2-Me) |
| G-0113 | pyrimidin-4-yl(5-Me) |
| G-0114 | pyrimidin-4-yl(6-Me) |
| G-0115 | pyrimidin-4-yl(2-OMe) |
| G-0116 | pyrimidin-4-yl(5-OMe) |
| G-0117 | pyrimidin-4-yl(6-OMe) |
| G-0118 | pyrimidin-4-yl(2-CN) |
| G-0119 | pyrimidin-4-yl(5-CN) |
| G-0120 | pyrimidin-4-yl(6-CN) |

[Table 143]

| Compound Number | Q |
|---|---|
| G-0121 | pyrimidin-4-yl(2-$CF_3$) |
| G-0122 | pyrimidin-4-yl(5-$CF_3$) |
| G-0123 | pyrimidin-4-yl(6-$CF_3$) |
| G-0124 | pyrimidin-4-yl(2-$CO_2Et$) |
| G-0125 | pyrimidin-4-yl(5-$CO_2Et$) |
| G-0126 | pyrimidin-4-yl(6-$CO_2Et$) |
| G-0127 | pyrimidin-5-yl |
| G-0128 | $CH_2$pyrimidin-5-yl |
| G-0129 | pyrimidin-5-yl(2-F) |
| G-0130 | pyrimidin-5-yl(4-F) |
| G-0131 | pyrimidin-5-yl(2-Cl) |
| G-0132 | pyrimidin-5-yl(4-Cl) |
| G-0133 | pyrimidin-5-yl(2-Me) |
| G-0134 | pyrimidin-5-yl(4-Me) |
| G-0135 | pyrimidin-5-yl(2-OMe) |
| G-0136 | pyrimidin-5-yl(4-OMe) |
| G-0137 | pyrimidin-5-yl(2-CN) |
| G-0138 | pyrimidin-5-yl(4-CN) |
| G-0139 | pyrimidin-5-yl(2-$CF_3$) |
| G-0140 | pyrimidin-5-yl(4-$CF_3$) |
| G-0141 | pyrimidin-5-yl(2-$CO_2Et$) |
| G-0142 | pyrimidin-5-yl(4-$CO_2Et$) |

(continued)

| Compound Number | Q |
|---|---|
| G-0143 | pyridazin-3-yl |
| G-0144 | $CH_2$pyridazin-3-yl |
| G-0145 | pyridazin-3-yl(4-F) |
| G-0146 | pyridazin-3-yl(5-F) |
| G-0147 | pyridazin-3-yl(6-F) |
| G-0148 | pyridazin-3-yl(4-Cl) |
| G-0149 | pyridazin-3-yl(5-Cl) |
| G-0150 | pyridazin-3-yl(6-Cl) |
| G-0151 | pyridazin-3-yl(4-Me) |
| G-0152 | pyridazin-3-yl(5-Me) |
| G-0153 | pyridazin-3-yl(6-Me) |
| G-0154 | pyridazin-3-yl(4-OMe) |
| G-0155 | pyridazin-3-yl(5-OMe) |
| G-0156 | pyridazin-3-yl(6-OMe) |
| G-0157 | pyridazin-3-yl(4-CN) |
| G-0158 | pyridazin-3-yl(5-CN) |
| G-0159 | pyridazin-3-yl(6-CN) |
| G-0160 | pyridazin-3-yl(4-$CF_3$) |
| G-0161 | pyridazin-3-yl(5-$CF_3$) |
| G-0162 | pyridazin-3-yl(6-$CF_3$) |

[Table 144]

| Compound Number | Q |
|---|---|
| G-0163 | pyridazin-3-yl(4-$CO_2$Et) |
| G-0164 | pyridazin-3-yl(5-$CO_2$Et) |
| G-0165 | pyridazin-3-yl(6-$CO_2$Et) |
| G-0166 | pyridazin-4-yl |
| G-0167 | $CH_2$pyridazin-4-yl |
| G-0168 | pyridazin-4-yl(3-F) |
| G-0169 | pyridazin-4-yl(5-F) |
| G-0170 | pyridazin-4-yl(6-F) |
| G-0171 | pyridazin-4-yl(3-Cl) |
| G-0172 | pyridazin-4-yl(5-Cl) |
| G-0173 | pyridazin-4-yl(6-Cl) |
| G-0174 | pyridazin-4-yl(3-Me) |
| G-0175 | pyridazin-4-yl(5-Me) |
| G-0176 | pyridazin-4-yl(6-Me) |

(continued)

| Compound Number | Q |
|---|---|
| G-0177 | pyridazin-4-yl(3-OMe) |
| G-0178 | pyridazin-4-yl(5-OMe) |
| G-0179 | pyridazin-4-yl(6-OMe) |
| G-0180 | pyridazin-4-yl(3-CN) |
| G-0181 | pyridazin-4-yl(5-CN) |
| G-0182 | pyridazin-4-yl(6-CN) |
| G-0183 | pyridazin-4-yl(3-$CF_3$) |
| G-0184 | pyridazin-4-yl(5-$CF_3$) |
| G-0185 | pyridazin-4-yl(6-$CF_3$) |
| G-0186 | pyridazin-4-yl(3-$CO_2$Et) |
| G-0187 | pyridazin-4-yl(5-$CO_2$Et) |
| G-0188 | pyridazin-4-yl(6-$CO_2$Et) |
| G-0189 | pyrazin-2-yl |
| G-0190 | $CH_2$pyrazin-2-yl |
| G-0191 | pyrazin-2-yl(3-F) |
| G-0192 | pyrazi n-2-yl(5-F) |
| G-0193 | pyrazi n-2-yl(6-F) |
| G-0194 | pyrazin-2-yl(3-Cl) |
| G-0195 | pyrazin-2-yl(5-Cl) |
| G-0196 | pyrazin-2-yl(6-Cl) |
| G-0197 | pyrazin-2-yl(3-Me) |
| G-0198 | pyrazin-2-yl(5-Me) |
| G-0199 | pyrazin-2-yl(6-Me) |
| G-0200 | pyrazi n-2-yl(3-O Me) |
| G-0201 | pyrazi n-2-yl(5-O Me) |
| G-0202 | pyrazi n-2-yl(6-O Me) |
| G-0203 | pyrazin-2-yl(3-CN) |
| G-0204 | pyrazin-2-yl(5-CN) |

[Table 145]

| Compound Number | Q |
|---|---|
| G-0205 | pyrazin-2-yl(6-CN) |
| G-0206 | pyrazin-2-yl(3-$CF_3$) |
| G-0207 | pyrazin-2-yl(5-$CF_3$) |
| G-0208 | pyrazin-2-yl(6-$CF_3$) |
| G-0209 | pyrazin-2-yl(3-$CO_2$Et) |

(continued)

| Compound Number | Q |
|---|---|
| G-0210 | pyrazin-2-yl(5-$CO_2$Et) |
| G-0211 | pyrazin-2-yl(6-$CO_2$Et) |
| G-0212 | 1,3,5-triazin-2-yl |
| G-0213 | $CH_2$1,3,5-triazin-2-yl |
| G-0214 | thiophen-2-yl |
| G-0215 | $CH_2$thiophen-2-yl |
| G-0216 | thiophen-2-yl(3-F) |
| G-0217 | thiophen-2-yl(4-F) |
| G-0218 | thiophen-2-yl(5-F) |
| G-0219 | thiophen-2-yl(3-Cl) |
| G-0220 | thiophen-2-yl(4-Cl) |
| G-0221 | thiophen-2-yl(5-Cl) |
| G-0222 | thiophen-2-yl(3-Me) |
| G-0223 | thiophen-2-yl(4-Me) |
| G-0224 | thiophen-2-yl(5-Me) |
| G-0225 | thiophen-2-yl(3-OMe) |
| G-0226 | thiophen-2-yl(4-OMe) |
| G-0227 | thiophen-2-yl(5-OMe) |
| G-0228 | thiophen-2-yl(3-CN) |
| G-0229 | thiophen-2-yl(4-CN) |
| G-0230 | thiophen-2-yl(5-CN) |
| G-0231 | thiophen-2-yl(3-$CF_3$) |
| G-0232 | thiophen-2-yl(4-$CF_3$) |
| G-0233 | thiophen-2-yl(5-$CF_3$) |
| G-0234 | thiophen-2-yl(3-$CO_2$Et) |
| G-0235 | thiophen-2-yl(4-$CO_2$Et) |
| G-0236 | thiophen-2-yl(5-$CO_2$Et) |
| G-0237 | thiophen-3-yl |
| G-0238 | $CH_2$thiophen-3-yl |
| G-0239 | thiophen-3-yl(2-F) |
| G-0240 | thiophen-3-yl(4-F) |
| G-0241 | thiophen-3-yl(5-F) |
| G-0242 | thiophen-3-yl(2-Cl) |
| G-0243 | thiophen-3-yl(4-Cl) |
| G-0244 | thiophen-3-yl(5-Cl) |
| G-0245 | thiophen-3-yl(2-Me) |

(continued)

| Compound Number | Q |
|---|---|
| G-0246 | thiophen-3-yl(4-Me) |

[Table 146]

| Compound Number | Q |
|---|---|
| G-0247 | thiophen-3-yl(5-Me) |
| G-0248 | thiophen-3-yl(2-OMe) |
| G-0249 | thiophen-3-yl(4-OMe) |
| G-0250 | thiophen-3-yl(5-OMe) |
| G-0251 | thiophen-3-yl(2-CN) |
| G-0252 | thiophen-3-yl(4-CN) |
| G-0253 | thiophen-3-yl(5-CN) |
| G-0254 | thiophen-3-yl(2-$CF_3$) |
| G-0255 | thiophen-3-yl(4-$CF_3$) |
| G-0256 | thiophen-3-yl(5-$CF_3$) |
| G-0257 | thiophen-3-yl(2-$CO_2Et$) |
| G-0258 | thiophen-3-yl(4-$CO_2Et$) |
| G-0259 | thiophen-3-yl(5-$CO_2Et$) |
| G-0260 | furan-2-yl |
| G-0261 | $CH_2$furan-2-yl |
| G-0262 | furan-2-yl(3-F) |
| G-0263 | furan-2-yl(4-F) |
| G-0264 | furan-2-yl(5-F) |
| G-0265 | furan-2-yl(3-Cl) |
| G-0266 | furan-2-yl(4-Cl) |
| G-0267 | furan-2-yl(5-Cl) |
| G-0268 | furan-2-yl(3-Me) |
| G-0269 | furan-2-yl(4-Me) |
| G-0270 | furan-2-yl(5-Me) |
| G-0271 | furan-2-yl(3-OMe) |
| G-0272 | furan-2-yl(4-OMe) |
| G-0273 | furan-2-yl(5-OMe) |
| G-0274 | furan-2-yl(3-C N) |
| G-0275 | furan-2-yl(4-C N) |
| G-0276 | furan-2-yl(5-C N) |
| G-0277 | furan-2-yl(3-$CF_3$) |
| G-0278 | furan-2-yl(4-$CF_3$) |

(continued)

| Compound Number | Q |
|---|---|
| G-0279 | furan-2-yl(5-$CF_3$) |
| G-0280 | furan-2-yl(3-$CO_2$Et) |
| G-0281 | furan-2-yl(4-$CO_2$Et) |
| G-0282 | furan-2-yl(5-$CO_2$Et) |
| G-0283 | furan-3-yl |
| G-0284 | $CH_2$furan-3-yl |
| G-0285 | furan-3-yl(2-F) |
| G-0286 | furan-3-yl(4-F) |
| G-0287 | furan-3-yl(5-F) |
| G-0288 | furan-3-yl(2-Cl) |

[Table 147]

| Compound Number | Q |
|---|---|
| G-0289 | furan-3-yl(4-Cl) |
| G-0290 | furan-3-yl(5-Cl) |
| G-0291 | furan-3-yl(2-Me) |
| G-0292 | furan-3-yl(4-Me) |
| G-0293 | furan-3-yl(5-Me) |
| G-0294 | furan-3-yl(2-OMe) |
| G-0295 | furan-3-yl(4-OMe) |
| G-0296 | furan-3-yl(5-OMe) |
| G-0297 | furan-3-yl(2-C N) |
| G-0298 | furan-3-yl(4-C N) |
| G-0299 | furan-3-yl(5-C N) |
| G-0300 | furan-3-yl(2-$CF_3$) |
| G-0301 | furan-3-yl(4-$CF_3$) |
| G-0302 | furan-3-yl(5-$CF_3$) |
| G-0303 | furan-3-yl(2-$CO_2$Et) |
| G-0304 | furan-3-yl(4-$CO_2$Et) |
| G-0305 | furan-3-yl(5-$CO_2$Et) |
| G-0306 | 1H-pyrrol-1-yl |
| G-0307 | $CH_2$1H-pyrrol-1-yl |
| G-0308 | 1H-pyrrol-2-yl |
| G-0309 | $CH_2$1H-pyrrol-2-yl |
| G-0310 | 1H-pyrrol-2-yl(1-Me) |
| G-0311 | $CH_2$1H-pyrrol-2-yl(1-Me) |

(continued)

| Compound Number | Q |
|---|---|
| G-0312 | 1H-pyrrol-3-yl |
| G-0313 | $CH_2$1H-pyrrol-3-yl |
| G-0314 | 1H-pyrrol-3-yl(1-Me) |
| G-0315 | $CH_2$1H-pyrrol-3-yl(1-Me) |
| G-0316 | 1H-pyrazol-1-yl |
| G-0317 | $CH_2$1H-pyrazol-1-yl |
| G-0318 | 1H-pyrazol-1-yl(3-F) |
| G-0319 | 1H-pyrazol-1-yl(4-F) |
| G-0320 | 1H-pyrazol-1-yl(5-F) |
| G-0321 | 1H-pyrazol-1-yl(3-Cl) |
| G-0322 | 1H-pyrazol-1-yl(4-Cl) |
| G-0323 | 1H-pyrazol-1-yl(5-Cl) |
| G-0324 | 1H-pyrazol-1 -yl(3-Me) |
| G-0325 | 1H-pyrazol-1 -yl(4-Me) |
| G-0326 | 1H-pyrazol-1-yl(5-Me) |
| G-0327 | 1H-pyrazol-1-yl(3-OMe) |
| G-0328 | 1H-pyrazol-1-yl(4-OMe) |
| G-0329 | 1H-pyrazol-1-yl(5-OMe) |
| G-0330 | 1H-pyrazol-1-yl(3-CN) |

[Table 148]

| Compound Number | Q |
|---|---|
| G-0331 | 1H-pyrazol-1-yl(4-CN) |
| G-0332 | 1H-pyrazol-1-yl(5-CN) |
| G-0333 | 1H-pyrazol-1-yl(3-$CF_3$) |
| G-0334 | 1H-pyrazol-1-yl(4-$CF_3$) |
| G-0335 | 1H-pyrazol-1-yl(5-$CF_3$) |
| G-0336 | 1H-pyrazol-1-yl(3-$CO_2$Et) |
| G-0337 | 1H-pyrazol-1-yl(4-$CO_2$Et) |
| G-0338 | 1H-pyrazol-1-yl(5-$CO_2$Et) |
| G-0339 | 1H-pyrazol-3-yl |
| G-0340 | $CH_2$1H-pyrazol-3-yl |
| G-0341 | 1H-pyrazol-3-yl(1-Me) |
| G-0342 | $CH_2$1H-pyrazol-3-yl(1-Me) |
| G-0343 | 1H-pyrazol-3-yl(4-F,1-Me) |
| G-0344 | 1H-pyrazol-3-yl(5-F,1-Me) |

(continued)

| Compound Number | Q |
|---|---|
| G-0345 | 1H-pyrazol-3-yl(4-Cl,1-Me) |
| G-0346 | 1H-pyrazol-3-yl(5-Cl,1-Me) |
| G-0347 | 1H-pyrazol-3-yl(1,4-$Me_2$) |
| G-0348 | 1H-pyrazol-3-yl(1,5-$Me_2$) |
| G-0349 | 1H-pyrazol-3-yl(4-OMe,1-Me) |
| G-0350 | 1H-pyrazol-3-yl(5-OMe,1-Me) |
| G-0351 | 1H-pyrazol-3-yl(4-CN,1-Me) |
| G-0352 | 1H-pyrazol-3-yl(5-CN,1-Me) |
| G-0353 | 1H-pyrazol-3-yl(1-Me,4-$CF_3$) |
| G-0354 | 1H-pyrazol-3-yl(1-Me,5-$CF_3$) |
| G-0355 | 1H-pyrazol-3-yl(1-Me,4-$CO_2$Et) |
| G-0356 | 1H-pyrazol-3-yl(1-Me,5-$CO_2$Et) |
| G-0357 | 1H-pyrazol-4-yl |
| G-0358 | $CH_2$1H-pyrazol-4-yl |
| G-0359 | 1H-pyrazol-4-yl(1-Me) |
| G-0360 | $CH_2$1H-pyrazol-4-yl(1-Me) |
| G-0361 | 1H-pyrazol-4-yl(3-F,1-Me) |
| G-0362 | 1H-pyrazol-4-yl(5-F,1-Me) |
| G-0363 | 1H-pyrazol-4-yl(3-Cl,1-Me) |
| G-0364 | 1H-pyrazol-4-yl(5-Cl,1-Me) |
| G-0365 | 1H-pyrazol-4-yl(1,3-$Me_2$) |
| G-0366 | 1H-pyrazol-4-yl(1,5-$Me_2$) |
| G-0367 | 1H-pyrazol-4-yl(3-OMe,1-Me) |
| G-0368 | 1H-pyrazol-4-yl(5-OMe,1-Me) |
| G-0369 | 1H-pyrazol-4-yl(3-CN,1-Me) |
| G-0370 | 1H-pyrazol-4-yl(5-CN,1-Me) |
| G-0371 | 1H-pyrazol-4-yl(1-Me,3-$CF_3$) |
| G-0372 | 1H-pyrazol-4-yl(1-Me,5-$CF_3$) |

[Table 149]

| Compound Number | Q |
|---|---|
| G-0373 | 1H-pyrazol-4-yl(1-Me,3-$CO_2$Et) |
| G-0374 | 1H-pyrazol-4-yl(1-Me,5-$CO_2$Et) |
| G-0375 | 1H-pyrazol-5-yl |
| G-0376 | $CH_2$1H-pyrazol-5-yl |
| G-0377 | 1H-pyrazol-5-yl(1-Me) |

(continued)

| Compound Number | Q |
|---|---|
| G-0378 | $CH_2$1H-pyrazol-5-yl(1-Me) |
| G-0379 | 1H-pyrazol-5-yl(3-F,1-Me) |
| G-0380 | 1H-pyrazol-5-yl(4-F,1-Me) |
| G-0381 | 1H-pyrazol-5-yl(3-Cl,1-Me) |
| G-0382 | 1H-pyrazol-5-yl(4-Cl,1-Me) |
| G-0383 | 1H-pyrazol-5-yl(1,3-$Me_2$) |
| G-0384 | 1H-pyrazol-5-yl(1,4-$Me_2$) |
| G-0385 | 1H-pyrazol-5-yl(3-OMe,1-Me) |
| G-0386 | 1H-pyrazol-5-yl(4-OMe, 1-Me) |
| G-0387 | 1H-pyrazol-5-yl(3-CN,1-Me) |
| G-0388 | 1H-pyrazol-5-yl(4-CN,1-Me) |
| G-0389 | 1H-pyrazol-5-yl(1-Me,3-$CF_3$) |
| G-0390 | 1H-pyrazol-5-yl(1-Me,4-$CF_3$) |
| G-0391 | 1H-pyrazol-5-yl(1-Me,3-$CO_2$Et) |
| G-0392 | 1H-pyrazol-5-yl(1-Me,4-$CO_2$Et) |
| G-0393 | 1H-imidazol-1-yl |
| G-0394 | $CH_2$1H-imidazol-1-yl |
| G-0395 | 1H-imidazol-1-yl(2-F) |
| G-0396 | 1H-imidazol-1-yl(4-F) |
| G-0397 | 1H-imidazol-1-yl(5-F) |
| G-0398 | 1H-imidazol-1-yl(2-Cl) |
| G-0399 | 1H-imidazol-1-yl(4-Cl) |
| G-0400 | 1H-imidazol-1-yl(5-Cl) |
| G-0401 | 1H-imidazol-1-yl(2-Me) |
| G-0402 | 1H-imidazol-1-yl(4-Me) |
| G-0403 | 1H-imidazol-1-yl(5-Me) |
| G-0404 | 1H-imidazol-1-yl(2-OMe) |
| G-0405 | 1H-imidazol-1-yl(4-OMe) |
| G-0406 | 1H-imidazol-1-yl(5-OMe) |
| G-0407 | 1H-imidazol-1-yl(2-CN) |
| G-0408 | 1H-imidazol-1-yl(4-CN) |
| G-0409 | 1H-imidazol-1-yl(5-CN) |
| G-0410 | 1H-imidazol-1-yl(2-$CF_3$) |
| G-0411 | 1H-imidazol-1-yl(4-$CF_3$) |
| G-0412 | 1H-imidazol-1-yl(5-$CF_3$) |
| G-0413 | 1H-imidazol-1-yl(2-$CO_2$Et) |

(continued)

| Compound Number | Q |
|---|---|
| G-0414 | 1H-imidazol-1-yl(4-$CO_2$Et) |

[Table 150]

| Compound Number | Q |
|---|---|
| G-0415 | 1H-imidazol-1-yl(5-$CO_2$Et) |
| G-0416 | 1H-imidazol-2-yl |
| G-0417 | $CH_2$1H-imidazol-2-yl |
| G-0418 | 1H-imidazol-2-yl(1-Me) |
| G-0419 | $CH_2$1H-imidazol-2-yl(1-Me) |
| G-0420 | 1H-imidazol-2-yl(4-F,1-Me) |
| G-0421 | 1H-imidazol-2-yl(5-F,1-Me) |
| G-0422 | 1H-imidazol-2-yl(4-Cl,1-Me) |
| G-0423 | 1H-imidazol-2-yl(5-Cl,1-Me) |
| G-0424 | 1H-imidazol-2-yl(1,4-$Me_2$) |
| G-0425 | 1H-imidazol-2-yl(1,5-$Me_2$) |
| G-0426 | 1H-imidazol-2-yl(4-OMe,1-Me) |
| G-0427 | 1H-imidazol-2-yl(5-OMe,1-Me) |
| G-0428 | 1H-imidazol-2-yl(4-CN,1-Me) |
| G-0429 | 1H-imidazol-2-yl(5-CN,1-Me) |
| G-0430 | 1H-imidazol-2-yl(1-Me,4-$CF_3$) |
| G-0431 | 1H-imidazol-2-yl(1-Me,5-$CF_3$) |
| G-0432 | 1H-imidazol-2-yl(1-Me,4-$CO_2$Et) |
| G-0433 | 1H-imidazol-2-yl(1-Me,5-$CO_2$Et) |
| G-0434 | 1H-imidazol-4-yl |
| G-0435 | $CH_2$1H-imidazol-4-yl |
| G-0436 | 1H-imidazol-4-yl(1-Me) |
| G-0437 | $CH_2$1H-imidazol-4-yl(1-Me) |
| G-0438 | 1H-imidazol-4-yl(2-F,1-Me) |
| G-0439 | 1H-imidazol-4-yl(5-F,1-Me) |
| G-0440 | 1H-imidazol-4-yl(2-Cl,1-Me) |
| G-0441 | 1H-imidazol-4-yl(5-Cl,1-Me) |
| G-0442 | 1H-imidazol-4-yl(1,2-$Me_2$) |
| G-0443 | 1H-imidazol-4-yl(1,5-$Me_2$) |
| G-0444 | 1H-imidazol-4-yl(2-OMe,1-Me) |
| G-0445 | 1H-imidazol-4-yl(5-OMe,1-Me) |
| G-0446 | 1H-imidazol-4-yl(2-CN,1-Me) |

(continued)

| Compound Number | Q |
|---|---|
| G-0447 | 1H-imidazol-4-yl(5-CN,1-Me) |
| G-0448 | 1H-imidazol-4-yl(1-Me,2-$CF_3$) |
| G-0449 | 1H-imidazol-4-yl(1-Me,5-$CF_3$) |
| G-0450 | 1H-imidazol-4-yl(1-Me,2-$CO_2$Et) |
| G-0451 | 1H-imidazol-4-yl(1-Me,5-$CO_2$Et) |
| G-0452 | 1H-imidazol-5-yl |
| G-0453 | $CH_2$1H-imidazol-5-yl |
| G-0454 | 1H-imidazol-5-yl(1-Me) |
| G-0455 | $CH_2$1H-imidazol-5-yl(1-Me) |
| G-0456 | 1H-imidazol-5-yl(2-F,1-Me) |

[Table 151]

| Compound Number | Q |
|---|---|
| G-0457 | 1H-imidazol-5-yl(4-F,1-Me) |
| G-0458 | 1H-imidazol-5-yl(2-Cl,1-Me) |
| G-0459 | 1H-imidazol-5-yl(4-Cl,1-Me) |
| G-0460 | 1H-imidazol-5-yl(1,2-$Me_2$) |
| G-0461 | 1H-imidazol-5-yl(1,4-$Me_2$) |
| G-0462 | 1H-imidazol-5-yl(2-OMe,1-Me) |
| G-0463 | 1H-imidazol-5-yl(4-OMe,1-Me) |
| G-0464 | 1H-imidazol-5-yl(2-CN,1-Me) |
| G-0465 | 1H-imidazol-5-yl(4-CN,1-Me) |
| G-0466 | 1H-imidazol-5-yl(1-Me,2-$CF_3$) |
| G-0467 | 1H-imidazol-5-yl(1-Me,4-$CF_3$) |
| G-0468 | 1H-imidazol-5-yl(1-Me,2-$CO_2$Et) |
| G-0469 | 1H-imidazol-5-yl(1-Me,4-$CO_2$Et) |
| G-0470 | 1H-1,2,4-triazol-1-yl |
| G-0471 | $CH_2$1H-1,2,4-triazol-1-yl |
| G-0472 | 1H-1,2,4-triazol-1-yl(3-F) |
| G-0473 | 1H-1,2,4-triazol-1-yl(5-F) |
| G-0474 | 1H-1,2,4-triazol-1-yl(3-Cl) |
| G-0475 | 1H-1,2,4-triazol-1-yl(5-Cl) |
| G-0476 | 1H-1,2,4-triazol-1-yl(3-Me) |
| G-0477 | 1H-1,2,4-triazol-1-yl(5-Me) |
| G-0478 | 1H-1,2,4-triazol-1-yl(3-OMe) |
| G-0479 | 1H-1,2,4-triazol-1-yl(5-OMe) |

(continued)

| Compound Number | Q |
|---|---|
| G-0480 | 1H-1,2,4-triazol-1-yl(3-CN) |
| G-0481 | 1H-1,2,4-triazol-1-yl(5-CN) |
| G-0482 | 1H-1,2,4-triazol-1-yl(3-$CF_3$) |
| G-0483 | 1H-1,2,4-triazol-1-yl(5-$CF_3$) |
| G-0484 | 1H-1,2,4-triazol-1-yl(3-$CO_2$Et) |
| G-0485 | 1H-1,2,4-triazol-1-yl(5-$CO_2$Et) |
| G-0486 | 1H-1,2,4-triazol-3-yl |
| G-0487 | $CH_2$1H-1,2,4-triazol-3-yl |
| G-0488 | 1H-1,2,4-triazol-3-yl(1-Me) |
| G-0489 | $CH_2$1H-1,2,4-triazol-3-yl(1-Me) |
| G-0490 | 1H-1,2,4-triazol-3-yl(5-F,1-Me) |
| G-0491 | 1H-1,2,4-triazol-3-yl(5-Cl,1-Me) |
| G-0492 | 1H-1,2,4-triazol-3-yl(1,5-$Me_2$) |
| G-0493 | 1H-1,2,4-triazol-3-yl(5-OMe,1-Me) |
| G-0494 | 1H-1,2,4-triazol-3-yl(5-CN,1-Me) |
| G-0495 | 1H-1,2,4-triazol-3-yl(1-Me,5-$CF_3$) |
| G-0496 | 1H-1,2,4-triazol-3-yl(1-Me,5-$CO_2$Et) |
| G-0497 | 4H-1,2,4-triazol-4-yl |
| G-0498 | $CH_2$4H-1,2,4-triazol-4-yl |

[Table 152]

| Compound Number | Q |
|---|---|
| G-0499 | 4H-1,2,4-triazol-4-yl(3-F) |
| G-0500 | 4H-1,2,4-triazol-4-yl(3-Cl) |
| G-0501 | 4H-1,2,4-triazol-4-yl(3-Me) |
| G-0502 | 4H-1,2,4-triazol-4-yl(3-OMe) |
| G-0503 | 4H-1,2,4-triazol-4-yl(3-CN) |
| G-0504 | 4H-1,2,4-triazol-4-yl(3-$CF_3$) |
| G-0505 | 4H-1,2,4-triazol-4-yl(3-$CO_2$Et) |
| G-0506 | 1H-1,2,4-triazol-5-yl |
| G-0507 | $CH_2$1H-1,2,4-triazol-5-yl |
| G-0508 | 1H-1,2,4-triazol-5-yl(1-Me) |
| G-0509 | $CH_2$1H-1,2,4-triazol-5-yl(1-Me) |
| G-0510 | 1H-1,2,4-triazol-5-yl(3-F,1-Me) |
| G-0511 | 1H-1,2,4-triazol-5-yl(3-Cl,1-Me) |
| G-0512 | 1H-1 ,2,4-triazol-5-yl(1,3-$Me_2$) |

(continued)

| Compound Number | Q |
|---|---|
| G-0513 | 1H-1,2,4-triazol-5-yl(3-OMe,1-Me) |
| G-0514 | 1H-1,2,4-triazol-5-yl(3-CN,1-Me) |
| G-0515 | 1H-1,2,4-triazol-5-yl(1-Me,3-$CF_3$) |
| G-0516 | 1H-1,2,4-triazol-5-yl(1-Me,3-$CO_2$Et) |
| G-0517 | 1H-1,2,3-triazol-1-yl |
| G-0518 | $CH_2$1H-1,2,3-triazol-1-yl |
| G-0519 | 1H-1,2,3-triazol-1-yl(4-Me) |
| G-0520 | 1H-1,2,3-triazol-1-yl(5-Me) |
| G-0521 | 1H-1,2,3-triazol-1-yl(4-$CO_2$Et) |
| G-0522 | 1H-1,2,3-triazol-1-yl(5-$CO_2$Et) |
| G-0523 | 2H-1,2,3-triazol-2-yl |
| G-0524 | $CH_2$2H-1,2,3-triazol-2-yl |
| G-0525 | 1H-tetrazol-1-yl |
| G-0526 | $CH_2$1H-tetrazol-1-yl |
| G-0527 | 2H-tetrazol-2-yl |
| G-0528 | $CH_2$2H-tetrazol-2-yl |
| G-0529 | 1H-tetrazol-5-yl |
| G-0530 | $CH_2$1H-tetrazol-5-yl |
| G-0531 | 1H-tetrazol-5-yl(1-Me) |
| G-0532 | $CH_2$1H-tetrazol-5-yl(1-Me) |
| G-0533 | thiazol-2-yl |
| G-0534 | $CH_2$thiazol-2-yl |
| G-0535 | thiazol-2-yl(4-F) |
| G-0536 | thiazol-2-yl(5-F) |
| G-0537 | thiazol-2-yl(4-Cl) |
| G-0538 | thiazol-2-yl(5-Cl) |
| G-0539 | thiazol-2-yl(4-Me) |
| G-0540 | thiazol-2-yl(5-Me) |

[Table 153]

| Compound Number | Q |
|---|---|
| G-0541 | thiazol-2-yl(4-OMe) |
| G-0542 | thiazol-2-yl(5-OMe) |
| G-0543 | thiazol-2-yl(4-C N) |
| G-0544 | thiazol-2-yl(5-C N) |
| G-0545 | thiazol-2-yl(4-$CF_3$) |

(continued)

| Compound Number | Q |
|---|---|
| G-0546 | thiazol-2-yl(5-$CF_3$) |
| G-0547 | thiazol-2-yl(4-$CO_2$Et) |
| G-0548 | thiazol-2-yl(5-$CO_2$Et) |
| G-0549 | thiazol-4-yl |
| G-0550 | $CH_2$thiazol-4-yl |
| G-0551 | thiazol-4-yl(2-F) |
| G-0552 | thiazol-4-yl(5-F) |
| G-0553 | thiazol-4-yl(2-Cl) |
| G-0554 | thiazol-4-yl(5-Cl) |
| G-0555 | thiazol-4-yl(2-Me) |
| G-0556 | thiazol-4-yl(5-Me) |
| G-0557 | thiazol-4-yl(2-OMe) |
| G-0558 | thiazol-4-yl(5-OMe) |
| G-0559 | thiazol-4-yl(2-C N) |
| G-0560 | thiazol-4-yl(5-C N) |
| G-0561 | thiazol-4-yl(2-$CF_3$) |
| G-0562 | thiazol-4-yl(5-$CF_3$) |
| G-0563 | thiazol-4-yl(2-$CO_2$Et) |
| G-0564 | thiazol-4-yl(5-$CO_2$Et) |
| G-0565 | thiazol-5-yl |
| G-0566 | $CH_2$thiazol-5-yl |
| G-0567 | thiazol-5-yl(2-F) |
| G-0568 | thiazol-5-yl(4-F) |
| G-0569 | thiazol-5-yl(2-Cl) |
| G-0570 | thiazol-5-yl(4-Cl) |
| G-0571 | thiazol-5-yl(2-Me) |
| G-0572 | thiazol-5-yl(4-Me) |
| G-0573 | thiazol-5-yl(2-OMe) |
| G-0574 | thiazol-5-yl(4-OMe) |
| G-0575 | thiazol-5-yl(2-C N) |
| G-0576 | thiazol-5-yl(4-C N) |
| G-0577 | thiazol-5-yl(2-$CF_3$) |
| G-0578 | thiazol-5-yl(4-$CF_3$) |
| G-0579 | thiazol-5-yl(2-$CO_2$Et) |
| G-0580 | thiazol-5-yl(4-$CO_2$Et) |
| G-0581 | isothiazol-3-yl |

(continued)

| Compound Number | Q |
|---|---|
| G-0582 | $CH_2$isothiazol-3-yl |

[Table 154]

| Compound Number | Q |
|---|---|
| G-0583 | isothiazol-3-yl(4-F) |
| G-0584 | isothiazol-3-yl(5-F) |
| G-0585 | isothiazol-3-yl(4-Cl) |
| G-0586 | isothiazol-3-yl(5-Cl) |
| G-0587 | isothiazol-3-yl(4-Me) |
| G-0588 | isothiazol-3-yl(5-Me) |
| G-0589 | isothiazol-3-yl(4-OMe) |
| G-0590 | isothiazol-3-yl(5-OMe) |
| G-0591 | isothiazol-3-yl(4-CN) |
| G-0592 | isothiazol-3-yl(5-CN) |
| G-0593 | isothiazol-3-yl(4-$CF_3$) |
| G-0594 | isothiazol-3-yl(5-$CF_3$) |
| G-0595 | isothiazol-3-yl(4-$CO_2$Et) |
| G-0596 | isothiazol-3-yl(5-$CO_2$Et) |
| G-0597 | isothiazol-4-yl |
| G-0598 | $CH_2$isothiazol-4-yl |
| G-0599 | isothiazol-4-yl(3-F) |
| G-0600 | isothiazol-4-yl(5-F) |
| G-0601 | isothiazol-4-yl(3-Cl) |
| G-0602 | isothiazol-4-yl(5-Cl) |
| G-0603 | isothiazol-4-yl(3-Me) |
| G-0604 | isothiazol-4-yl(5-Me) |
| G-0605 | isothiazol-4-yl(3-OMe) |
| G-0606 | isothiazol-4-yl(5-OMe) |
| G-0607 | isothiazol-4-yl(3-CN) |
| G-0608 | isothiazol-4-yl(5-CN) |
| G-0609 | isothiazol-4-yl(3-$CF_3$) |
| G-0610 | isothiazol-4-yl(5-$CF_3$) |
| G-0611 | isothiazol-4-yl(3-$CO_2$Et) |
| G-0612 | isothiazol-4-yl(5-$CO_2$Et) |
| G-0613 | isothiazol-5-yl |
| G-0614 | $CH_2$isothiazol-5-yl |

(continued)

| Compound Number | Q |
|---|---|
| G-0615 | isothiazol-5-yl(3-F) |
| G-0616 | isothiazol-5-yl(4-F) |
| G-0617 | isothiazol-5-yl(3-Cl) |
| G-0618 | isothiazol-5-yl(4-Cl) |
| G-0619 | isothiazol-5-yl(3,4-$Cl_2$) |
| G-0620 | isothiazol-5-yl(3-Me) |
| G-0621 | isothiazol-5-yl(4-Me) |
| G-0622 | isothiazol-5-yl(3-OMe) |
| G-0623 | isothiazol-5-yl(4-OMe) |
| G-0624 | isothiazol-5-yl(3-CN) |

[Table 155]

| Compound Number | Q |
|---|---|
| G-0625 | isothiazol-5-yl(4-CN) |
| G-0626 | isothiazol-5-yl(3-$CF_3$) |
| G-0627 | isothiazol-5-yl(4-$CF_3$) |
| G-0628 | isothiazol-5-yl(3-$CO_2$Et) |
| G-0629 | isothiazol-5-yl(4-$CO_2$Et) |
| G-0630 | oxazol-2-yl |
| G-0631 | $CH_2$oxazol-2-yl |
| G-0632 | oxazol-2-yl(4-F) |
| G-0633 | oxazol-2-yl(5-F) |
| G-0634 | oxazol-2-yl(4-Cl) |
| G-0635 | oxazol-2-yl(5-Cl) |
| G-0636 | oxazol-2-yl(4-Me) |
| G-0637 | oxazol-2-yl(5-Me) |
| G-0638 | oxazol-2-yl(4-OMe) |
| G-0639 | oxazol-2-yl(5-OMe) |
| G-0640 | oxazol-2-yl(4-C N) |
| G-0641 | oxazol-2-yl(5-C N) |
| G-0642 | oxazol-2-yl(4-$CF_3$) |
| G-0643 | oxazol-2-yl(5-$CF_3$) |
| G-0644 | oxazol-2-yl(4-$CO_2$Et) |
| G-0645 | oxazol-2-yl(5-$CO_2$Et) |
| G-0646 | oxazol-4-yl |
| G-0647 | $CH_2$oxazol-4-yl |

(continued)

| Compound Number | Q |
|---|---|
| G-0648 | oxazol-4-yl(2-F) |
| G-0649 | oxazol-4-yl(5-F) |
| G-0650 | oxazol-4-yl(2-Cl) |
| G-0651 | oxazol-4-yl(5-Cl) |
| G-0652 | oxazol-4-yl(2-Me) |
| G-0653 | oxazol-4-yl(5-Me) |
| G-0654 | oxazol-4-yl(2-OMe) |
| G-0655 | oxazol-4-yl(5-OMe) |
| G-0656 | oxazol-4-yl(2-C N) |
| G-0657 | oxazol-4-yl(5-C N) |
| G-0658 | oxazol-4-yl(2-$CF_3$) |
| G-0659 | oxazol-4-yl(5-$CF_3$) |
| G-0660 | oxazol-4-yl(2-$CO_2Et$) |
| G-0661 | oxazol-4-yl(5-$CO_2Et$) |
| G-0662 | oxazol-5-yl |
| G-0663 | $CH_2$oxazol-5-yl |
| G-0664 | oxazol-5-yl(2-F) |
| G-0665 | oxazol-5-yl(4-F) |
| G-0666 | oxazol-5-yl(2-Cl) |

[Table 156]

| Compound Number | Q |
|---|---|
| G-0667 | oxazol-5-yl(4-Cl) |
| G-0668 | oxazol-5-yl(2-Me) |
| G-0669 | oxazol-5-yl(4-Me) |
| G-0670 | oxazol-5-yl(2-OMe) |
| G-0671 | oxazol-5-yl(4-OMe) |
| G-0672 | oxazol-5-yl(2-C N) |
| G-0673 | oxazol-5-yl(4-C N) |
| G-0674 | oxazol-5-yl(2-$CF_3$) |
| G-0675 | oxazol-5-yl(4-$CF_3$) |
| G-0676 | oxazol-5-yl(2-$CO_2Et$) |
| G-0677 | oxazol-5-yl(4-$CO_2Et$) |
| G-0678 | isoxazol-3-yl |
| G-0679 | $CH_2$isoxazol-3-yl |
| G-0680 | isoxazol-3-yl(4-F) |

(continued)

| Compound Number | Q |
|---|---|
| G-0681 | isoxazol-3-yl(5-F) |
| G-0682 | isoxazol-3-yl(4-Cl) |
| G-0683 | isoxazol-3-yl(5-Cl) |
| G-0684 | isoxazol-3-yl(4-Me) |
| G-0685 | isoxazol-3-yl(5-Me) |
| G-0686 | isoxazol-3-yl(4-OMe) |
| G-0687 | isoxazol-3-yl(5-OMe) |
| G-0688 | isoxazol-3-yl(4-C N) |
| G-0689 | isoxazol-3-yl(5-C N) |
| G-0690 | isoxazol-3-yl(4-$CF_3$) |
| G-0691 | isoxazol-3-yl(5-$CF_3$) |
| G-0692 | isoxazol-3-yl(4-$CO_2$Et) |
| G-0693 | isoxazol-3-yl(5-$CO_2$Et) |
| G-0694 | isoxazol-4-yl |
| G-0695 | $CH_2$isoxazol-4-yl |
| G-0696 | isoxazol-4-yl(3-F) |
| G-0697 | isoxazol-4-yl(5-F) |
| G-0698 | isoxazol-4-yl(3-Cl) |
| G-0699 | isoxazol-4-yl(5-Cl) |
| G-0700 | isoxazol-4-yl(3-Me) |
| G-0701 | isoxazol-4-yl(5-Me) |
| G-0702 | isoxazol-4-yl(3-OMe) |
| G-0703 | isoxazol-4-yl(5-OMe) |
| G-0704 | isoxazol-4-yl(3-C N) |
| G-0705 | isoxazol-4-yl(5-C N) |
| G-0706 | isoxazol-4-yl(3-$CF_3$) |
| G-0707 | isoxazol-4-yl(5-$CF_3$) |
| G-0708 | isoxazol-4-yl(3-$CO_2$Et) |

[Table 157]

| Compound Number | Q |
|---|---|
| G-0709 | isoxazol-4-yl(5-$CO_2$Et) |
| G-0710 | isoxazol-5-yl |
| G-0711 | $CH_2$isoxazol-5-yl |
| G-0712 | isoxazol-5-yl(3-F) |
| G-0713 | isoxazol-5-yl(4-F) |

(continued)

| Compound Number | Q |
|---|---|
| G-0714 | isoxazol-5-yl(3-Cl) |
| G-0715 | isoxazol-5-yl(4-Cl) |
| G-0716 | isoxazol-5-yl(3-Me) |
| G-0717 | isoxazol-5-yl(4-Me) |
| G-0718 | isoxazol-5-yl(3-OMe) |
| G-0719 | isoxazol-5-yl(4-OMe) |
| G-0720 | isoxazol-5-yl(3-C N) |
| G-0721 | isoxazol-5-yl(4-CN) |
| G-0722 | isoxazol-5-yl(3-$CF_3$) |
| G-0723 | isoxazol-5-yl(4-$CF_3$) |
| G-0724 | isoxazol-5-yl(3-$CO_2$Et) |
| G-0725 | isoxazol-5-yl(4-$CO_2$Et) |
| G-0726 | 1,2,3-oxadiazol-4-yl |
| G-0727 | $CH_2$1,2,3-oxadiazol-4-yl |
| G-0728 | 1,2,3-oxadiazol-4-yl(5-F) |
| G-0729 | 1,2,3-oxadiazol-4-yl(5-Cl) |
| G-0730 | 1,2,3-oxadiazol-4-yl(5-Me) |
| G-0731 | 1,2,3-oxadiazol-4-yl(5-OMe) |
| G-0732 | 1,2,3-oxadiazol-4-yl(5-CN) |
| G-0733 | 1,2,3-oxadiazol-4-yl(5-$CF_3$) |
| G-0734 | 1,2,3-oxadiazol-4-yl(5-$CO_2$Et) |
| G-0735 | 1,2,3-oxadiazol-5-yl |
| G-0736 | $CH_2$1,2,3-oxadiazol-5-yl |
| G-0737 | 1,2,3-oxadiazol-5-yl(4-F) |
| G-0738 | 1,2,3-oxadiazol-5-yl(4-Cl) |
| G-0739 | 1,2,3-oxadiazol-5-yl(4-Me) |
| G-0740 | 1,2,3-oxadiazol-5-yl(4-OMe) |
| G-0741 | 1,2,3-oxadiazol-5-yl(4-CN) |
| G-0742 | 1,2,3-oxadiazol-5-yl(4-$CF_3$) |
| G-0743 | 1,2,3-oxadiazol-5-yl(4-$CO_2$Et) |
| G-0744 | 1,2,4-oxadiazol-3-yl |
| G-0745 | $CH_2$1,2,4-oxadiazol-3-yl |
| G-0746 | 1,2,4-oxadiazol-3-yl(5-F) |
| G-0747 | 1,2,4-oxadiazol-3-yl(5-Cl) |
| G-0748 | 1,2,4-oxadiazol-3-yl(5-Me) |
| G-0749 | 1,2,4-oxadiazol-3-yl(5-OMe) |

(continued)

| Compound Number | Q |
|---|---|
| G-0750 | 1,2,4-oxadiazol-3-yl(5-CN) |

[Table 158]

| Compound Number | Q |
|---|---|
| G-0751 | 1,2,4-oxadiazol-3-yl(5-$CF_3$) |
| G-0752 | 1,2,4-oxadiazol-3-yl(5-$CO_2Et$) |
| G-0753 | 1,2,4-oxadiazol-5-yl |
| G-0754 | $CH_2$1,2,4-oxadiazol-5-yl |
| G-0755 | 1,2,4-oxadiazol-5-yl(3-F) |
| G-0756 | 1,2,4-oxadiazol-5-yl(3-Cl) |
| G-0757 | 1,2,4-oxadiazol-5-yl(3-Me) |
| G-0758 | 1,2,4-oxadiazol-5-yl(3-OMe) |
| G-0759 | 1,2,4-oxadiazol-5-yl(3-CN) |
| G-0760 | 1,2,4-oxadiazol-5-yl(3-$CF_3$) |
| G-0761 | 1,2,4-oxadiazol-5-yl(3-$CO_2Et$) |
| G-0762 | 1,3,4-oxadiazol-2-yl |
| G-0763 | $CH_2$1,3,4-oxadiazol-2-yl |
| G-0764 | 1,2,5-oxadiazol-3-yl |
| G-0765 | $CH_2$1,2,5-oxadiazol-3-yl |
| G-0766 | 1,2,3-thiadiazol-4-yl |
| G-0767 | $CH_2$1,2,3-thiadiazol-4-yl |
| G-0768 | 1,2,3-thiadiazol-5-yl |
| G-0769 | $CH_2$1,2,3-thiadiazol-5-yl |
| G-0770 | 1,2,4-thiadiazol-3-yl |
| G-0771 | $CH_2$1,2,4-thiadiazol-3-yl |
| G-0772 | 1,2,4-thiadiazol-5-yl |
| G-0773 | $CH_2$1,2,4-thiadiazol-5-yl |
| G-0774 | 1,3,4-thiadiazol-2-yl |
| G-0775 | $CH_2$1,3,4-thiadiazol-2-yl |
| G-0776 | 1,2,5-thiadiazol-3-yl |
| G-0777 | $CH_2$1,2,5-thiadiazol-3-yl |
| G-0778 | morpholin-4-yl |
| G-0779 | $CH_2$morpholin-4-yl |
| G-0780 | piperidin-4-yl |
| G-0781 | $CH_2$piperidin-4-yl |
| G-0782 | piperidin-4-yl(1-Me) |

(continued)

| Compound Number | Q |
|---|---|
| G-0783 | $CH_2$piperidin-4-yl(1-Me) |
| G-0784 | piperidin-1-yl |
| G-0785 | $CH_2$piperidin-1-yl |
| G-0786 | piperazin-1-yl |
| G-0787 | $CH_2$piperazin-1-yl |
| G-0788 | piperazin-1-yl(4-Me) |
| G-0789 | $CH_2$piperazin-1yl(4-Me) |
| G-0790 | tetrahydro-2H-pyran-4-yl |
| G-0791 | $CH_2$tetrahydro-2H-pyran-4-yl |
| G-0792 | 1,3-dioxolan-2-yl |

[Table 159]

| Compound Number | Q |
|---|---|
| G-0793 | $CH_2$1,3-dioxolan-2-yl |
| G-0794 | 1,3-dioxolan-2-yl(2-$CF_3$) |
| G-0795 | $CH_2$1,3-dioxolan-2-yl(2-$CF_3$) |
| G-0796 | 1,3-dioxan-2-yl |
| G-0797 | $CH_2$1,3-dioxan-2-yl |
| G-0798 | 1,3-dioxan-2-yl(2-$CF_3$) |
| G-0799 | $CH_2$1,3-dioxan-2-yl(2-$CF_3$) |
| G-0800 | pyrrolidin-1-yl |
| G-0801 | $CH_2$pyrrolidin-1-yl |
| G-0802 | pyrrolidin-2-yl |
| G-0803 | $CH_2$pyrrolidin-2-yl |
| G-0804 | pyrrolidin-2-yl(1-Me) |
| G-0805 | $CH_2$pyrrolidin-2-yl(1-Me) |
| G-0806 | pyrrolidin-3-yl |
| G-0807 | $CH_2$pyrrolidin-3-yl |
| G-0808 | pyrrolidin-3-yl(1-Me) |
| G-0809 | $CH_2$pyrrolidin-3-yl(1-Me) |
| G-0810 | tetrahydrofuran-2-yl |
| G-0811 | $CH_2$tetrahydrofuran-2-yl |
| G-0812 | tetrahydrofuran-3-yl |
| G-0813 | $CH_2$tetrahydrofuran-3-yl |
| G-0814 | 4,5-dihydroisoxazol-3-yl |

(continued)

| Compound Number | Q |
|---|---|
| G-0815 | $CH_2$4,5-dihydroisoxazol-3-yl |
| G-0816 | 4,5-dihydroisoxazol-4-yl |
| G-0817 | $CH_2$4,5-dihydroisoxazol-4-yl |
| G-0818 | 4,5-dihydroisoxazol-5-yl |
| G-0819 | $CH_2$4,5-dihydroisoxazol-5-yl |
| G-0820 | oxetan-2-yl |
| G-0821 | $CH_2$oxetan-2-yl |
| G-0822 | oxetan-3-yl |
| G-0823 | oxetan-3-yl(3-Me) |
| G-0824 | $CH_2$oxetan-3-yl |
| G-0825 | azetidin-1-yl |
| G-0826 | $CH_2$azetidin-1-yl |
| G-0827 | azetidin-2-yl |
| G-0828 | $CH_2$azetidin-2-yl |
| G-0829 | azetidin-2-yl(1-Me) |
| G-0830 | $CH_2$azetidin-2-yl(1-Me) |
| G-0831 | azetidin-3-yl |
| G-0832 | $CH_2$azetidin-3-yl |
| G-0833 | azetidin-3-yl(1-Me) |
| G-0834 | $CH_2$azetidin-3-yl(1-Me) |

[Table 160]

| Compound Number | Q |
|---|---|
| G-0835 | oxiran-2-yl |
| G-0836 | $CH_2$oxiran-2-yl |
| G-0837 | 1,3,2-dioxaborolan-2-yl(4,4,5,5-$Me_4$) |
| G-0838 | tetrahydro-2H-pyran-2-yl |
| G-0839 | $CH_2$tetrahydro-2H-pyran-2-yl |
| G-0840 | tetrahydro-2H-pyran-3-yl |
| G-0841 | $CH_2$tetrahydro-2H-pyran-3-yl |
| G-0842 | piperidin-2-yl |
| G-0843 | $CH_2$piperidin-2-yl |
| G-0844 | piperidin-2-yl(1-Me) |
| G-0845 | $CH_2$piperidin-2-yl(1-Me) |
| G-0846 | piperidin-3-yl |
| G-0847 | $CH_2$piperidin-3-yl |

(continued)

| Compound Number | Q |
|---|---|
| G-0848 | piperidin-3-yl(1-Me) |
| G-0849 | $CH_2$piperidin-3-yl(1-Me) |
| G-0850 | pyridin-2-yl(6-$CH_2OCH_3$) |
| G-0851 | 1H-pyrazol-5-yl(1-CHO) |
| G-0852 | (pyridin-1-oxide)-2-yl |
| G-0853 | (pyridin-1-oxide)-3-yl |
| G-0854 | (pyridin-1 -oxide)-4-yl |

[Table 161]

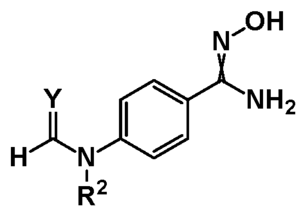

| Compound Number | Y | $R^2$ |
|---|---|---|
| H-0001 | O | H |
| H-0002 | O | Me |
| H-0003 | O | Et |
| H-0004 | O | n-Pr |
| H-0005 | O | i-Pr |
| H-0006 | O | n-Bu |
| H-0007 | O | i-Bu |
| H-0008 | O | s-Bu |
| H-0009 | O | t-Bu |
| H-0010 | O | $CH_2$t-Bu |
| H-0011 | O | n-Pen |
| H-0012 | O | n-Hex |
| H-0013 | O | $CH=CH_2$ |
| H-0014 | O | $CH_2CH=CH_2$ |
| H-0015 | O | $CH_2CH=CHCH_2Cl$ |
| H-0016 | O | $CH_2CH=CHCH_2NMe_2$ |
| H-0017 | O | C≡CH |
| H-0018 | O | $CH_2C≡CH$ |
| H-0019 | O | c-Pr |
| H-0020 | O | c-Bu |
| H-0021 | O | c-Pen |

(continued)

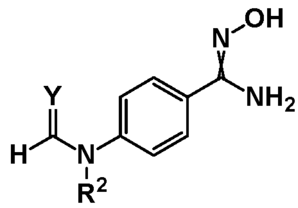

| Compound Number | Y | $R^2$ |
|---|---|---|
| H-0022 | O | c-Hex |
| H-0023 | O | $CH_2$c-Pr |
| H-0024 | O | $CH_2$c-Bu |
| H-0025 | O | $CH_2$c-Pen |
| H-0026 | O | C $H_2$c-Hex |
| H-0027 | O | $CH_2$c-Pr(2,2-$Cl_2$) |
| H-0028 | O | $CF_3$ |
| H-0029 | O | $CH_2CF_3$ |
| H-0030 | O | $CH_2CH_2CF_3$ |
| H-0031 | O | $CHF_2$ |
| H-0032 | O | $CH_2CHF_2$ |
| H-0033 | O | $CH_2CH_2CHF_2$ |
| H-0034 | O | $CH_2COMe$ |
| H-0035 | O | $CH_2CO_2Me$ |
| H-0036 | O | $CH(CH_3)CO_2Me$ |

[Table 162]

| Compound Number | Y | $R^2$ |
|---|---|---|
| H-0037 | O | $C(CH_3)(CH_3)CO_2Me$ |
| H-0038 | O | $CH_2CO_2Et$ |
| H-0039 | O | $CH(CH_3)CO_2Et$ |
| H-0040 | O | $C(CH_3)(CH_3)CO_2Et$ |
| H-0041 | O | $CH_2CONH_2$ |
| H-0042 | O | $CH_2CONHMe$ |
| H-0043 | O | $CH(CH_3)CONHMe$ |
| H-0044 | O | $C(CH_3)(CH_3)CONHMe$ |
| H-0045 | O | $CH_2CONHEt$ |
| H-0046 | O | $CH(CH_3)CONHEt$ |
| H-0047 | O | $C(CH_3)(CH_3)CONHEt$ |

(continued)

| Compound Number | Y | $R^2$ |
|---|---|---|
| H-0048 | O | $CH_2NHCHO$ |
| H-0049 | O | $CH(CH_3)NHCHO$ |
| H-0050 | O | $C(CH_3)(CH_3)NHCHO$ |
| H-0051 | O | $CH_2NHCOMe$ |
| H-0052 | O | $CH(CH_3)NHCOMe$ |
| H-0053 | O | $C(CH_3)(CH_3)NHCOMe$ |
| H-0054 | O | $CH_2NHCOEt$ |
| H-0055 | O | $CH(CH_3)NHCOEt$ |
| H-0056 | O | $C(CH_3)(CH_3)NHCOEt$ |
| H-0057 | O | $CH_2CN$ |
| H-0058 | O | $CH_2CH_2CN$ |
| H-0059 | O | $CH_2$c-Pr(1-CN) |
| H-0060 | O | $CH_2SCN$ |
| H-0061 | O | $CH_2CH_2SCN$ |
| H-0062 | O | $CH_2CH_2CH_2CH_2CH_2SCN$ |
| H-0063 | O | $CH_2CH_2CH_2CH_2CH_2CH_2SCN$ |
| H-0064 | O | $CH_2OCH_3$ |
| H-0065 | O | $CH_2OCH_2CH_3$ |
| H-0066 | O | $CH_2CH_2OCH_3$ |
| H-0067 | O | $CH_2CH_2OCH_2CH_3$ |
| H-0068 | O | $CH_2OCF_3$ |
| H-0069 | O | $CH_2OCH_2CF_3$ |
| H-0070 | O | $CH_2CH_2OCF_3$ |
| H-0071 | O | $CH_2CH_2OCH_2CF_3$ |
| H-0072 | O | $CH_2SCH_3$ |
| H-0073 | O | $CH_2SCH_2CH_3$ |
| H-0074 | O | $CH_2CH_2SCH_3$ |
| H-0075 | O | $CH_2CH_2SCH_2CH_3$ |
| H-0076 | O | $CH_2SCF_3$ |
| H-0077 | O | $CH_2SCH_2CF_3$ |
| H-0078 | O | $CH_2CH_2SCF_3$ |

[Table 163]

| Compound Number | Y | $R^2$ |
|---|---|---|
| H-0079 | O | $CH_2CH_2SCH_2CF_3$ |
| H-0080 | O | $CH_2CH_2CH_2CH_2CH_2SCF_3$ |
| H-0081 | O | $CH_2CH_2CH_2CH_2CH_2CH_2SCF_3$ |
| H-0082 | O | Ph |
| H-0083 | O | pyridin-2-yl |
| H-0084 | O | pyridin-3-yl |
| H-0085 | O | pyridin-4-yl |
| H-0086 | O | pyrimidin-2-yl |
| H-0087 | O | pyrimidin-4-yl |
| H-0088 | O | pyrimidin-5-yl |
| H-0089 | O | pyridazin-3-yl |
| H-0090 | O | pyridazin-4-yl |
| H-0091 | O | pyrazi n-2-yl |
| H-0092 | O | azetidin-3-yl |
| H-0093 | O | azetidin-3-yl(1-Me) |
| H-0094 | O | pyrrolidin-3-yl |
| H-0095 | O | pyrrolidin-3-yl(1-Me) |
| H-0096 | O | $CH_2Ph$ |
| H-0097 | O | $CH_2CH_2Ph$ |
| H-0098 | O | $CH_2CH_2CH_2Ph$ |
| H-0099 | O | $CH(CH_3)Ph$ |
| H-0100 | O | $C(CH_3)(CH_3)Ph$ |
| H-0101 | O | 1-Ph-c-Pr |
| H-0102 | O | 1-Ph-c-Bu |
| H-0103 | O | $CH_2COPh$ |
| H-0104 | O | $CH_2CO_2Ph$ |
| H-0105 | O | $CH_2CONHPh$ |
| H-0106 | O | $CH_2NHCOPh$ |
| H-0107 | O | $CH(CH_3)$pyridin-2-yl |
| H-0108 | O | $C(CH_3)(CH_3)$pyridin-2-yl |
| H-0109 | O | 1-(pyridin-2-yl)-c-Pr |
| H-0110 | O | 1-(pyridin-2-yl)-c-Bu |
| H-0111 | O | $CH(CH_3)$pyridin-3-yl |
| H-0112 | O | $C(CH_3)(CH_3)$pyridin-3-yl |
| H-0113 | O | 1-(pyridin-3-yl)-c-Pr |
| H-0114 | O | 1-(pyridin-3-yl)-c-Bu |

(continued)

| Compound Number | Y | $R^2$ |
|---|---|---|
| H-0115 | O | $CH(CH_3)$pyridin-4-yl |
| H-0116 | O | $C(CH_3)(CH_3)$pyridin-4-yl |
| H-0117 | O | 1-(pyridin-4-yl)-c-Pr |
| H-0118 | O | 1-(pyridin-4-yl)-c-Bu |
| H-0119 | O | $CH(CH_3)$pyrimidin-2-yl |
| H-0120 | O | $C(CH_3)(CH_3)$pyrimidin-2-yl |

[Table 164]

| Compound Number | Y | R2 |
|---|---|---|
| H-0121 | O | 1-(pyrimidin-2-yl)-c-Pr |
| H-0122 | O | 1-(pyrimidin-2-yl)-c-Bu |
| H-0123 | O | $CH(CH_3)$pyrimidin-4-yl |
| H-0124 | O | $C(CH_3)(CH_3)$pyrimidin-4-yl |
| H-0125 | O | 1-(pyrimidin-4-yl)-c-Pr |
| H-0126 | O | 1-(pyrimidin-4-yl)-c-Bu |
| H-0127 | O | $CH(CH_3)$pyrimidin-5-yl |
| H-0128 | O | $C(CH_3)(CH_3)$pyrimidin-5-yl |
| H-0129 | O | 1-(pyrimidin-5-yl)-c-Pr |
| H-0130 | O | 1-(pyrimidin-5-yl)-c-Bu |
| H-0131 | O | $CH(CH_3)$pyridazin-3-yl |
| H-0132 | O | $C(CH_3)(CH_3)$pyridazin-3-yl |
| H-0133 | O | 1-(pyridazin-3-yl)-c-Pr |
| H-0134 | O | 1-(pyridazin-3-yl)-c-Bu |
| H-0135 | O | $CH(CH_3)$pyridazin-4-yl |
| H-0136 | O | $C(CH_3)(CH_3)$pyridazin-4-yl |
| H-0137 | O | 1-(pyridazin-4-yl)-c-Pr |
| H-0138 | O | 1-(pyridazin-4-yl)-c-Bu |
| H-0139 | O | $CH(CH_3)$pyrazin-2-yl |
| H-0140 | O | $C(CH_3)(CH_3)$pyrazin-2-yl |
| H-0141 | O | 1-(pyrazin-2-yl)-c-Pr |
| H-0142 | O | 1-(pyrazin-2-yl)-c-Bu |
| H-0143 | O | $CH_2OPh$ |
| H-0144 | O | $CH_2CH_2OPh$ |
| H-0145 | O | CH(OH)Ph |
| H-0146 | O | $CH(CH_2OH)Ph$ |

(continued)

| Compound Number | Y | R2 |
| --- | --- | --- |
| H-0147 | O | $CH_2CH(OH)Ph$ |
| H-0148 | O | $CH_2SPh$ |
| H-0149 | O | $CH_2SOPh$ |
| H-0150 | O | $CH_2SO_2Ph$ |
| H-0151 | O | $CH_2CH_2SPh$ |
| H-0152 | O | $CH_2CH_2SOPh$ |
| H-0153 | O | $CH_2CH_2SO_2Ph$ |
| H-0154 | O | $CH_2NHPh$ |
| H-0155 | O | $CH_2CH_2NHPh$ |
| H-0156 | O | $CH_2Ph$(2-F) |
| H-0157 | O | $CH_2Ph$(3-F) |
| H-0158 | O | $CH_2Ph$(4-F) |
| H-0159 | O | $CH_2Ph$(2-Cl) |
| H-0160 | O | $CH_2Ph$(3-Cl) |
| H-0161 | O | $CH_2Ph$(4-Cl) |
| H-0162 | O | $CH_2Ph$(2-Br) |

[Table 165]

| Compound Number | Y | R2 |
| --- | --- | --- |
| H-0163 | O | $CH_2Ph$(3-Br) |
| H-0164 | O | $CH_2Ph$(4-Br) |
| H-0165 | O | $CH_2Ph$(2-I) |
| H-0166 | O | $CH_2Ph$(3-I) |
| H-0167 | O | $CH_2Ph$(4-I) |
| H-0168 | O | $CH_2Ph$(2-Me) |
| H-0169 | O | $CH_2Ph$(3-Me) |
| H-0170 | O | $CH_2Ph$(4-Me) |
| H-0171 | O | $CH_2Ph$(2-Et) |
| H-0172 | O | $CH_2Ph$(3-Et) |
| H-0173 | O | $CH_2Ph$(4-Et) |
| H-0174 | O | $CH_2Ph$(2-n-Pr) |
| H-0175 | O | $CH_2Ph$(3-n-Pr) |
| H-0176 | O | $CH_2Ph$(4-n-Pr) |
| H-0177 | O | $CH_2Ph$(2-i-Pr) |

(continued)

| Compound Number | Y | R2 |
|---|---|---|
| H-0178 | O | $CH_2Ph(3-i-Pr)$ |
| H-0179 | O | $CH_2Ph(4-i-Pr)$ |
| H-0180 | O | $CH_2Ph(2-s-Bu)$ |
| H-0181 | O | $CH_2Ph(3-s-Bu)$ |
| H-0182 | O | $CH_2Ph(4-s-Bu)$ |
| H-0183 | O | $CH_2Ph(2-i-Bu)$ |
| H-0184 | O | $CH_2Ph(3-i-Bu)$ |
| H-0185 | O | $CH_2Ph(4-i-Bu)$ |
| H-0186 | O | $CH_2Ph(2-t-Bu)$ |
| H-0187 | O | $CH_2Ph(3-t-Bu)$ |
| H-0188 | O | $CH_2Ph(4-t-Bu)$ |
| H-0189 | O | $CH_2Ph(2-CH=CH_2)$ |
| H-0190 | O | $CH_2Ph(3-CH=CH_2)$ |
| H-0191 | O | $CH_2Ph(4-CH=CH_2)$ |
| H-0192 | O | $CH_2Ph(2-CH_2CH=CH_2)$ |
| H-0193 | O | $CH_2Ph(3-CH_2CH=CH_2)$ |
| H-0194 | O | $CH_2Ph(4-CH_2CH=CH_2)$ |
| H-0195 | O | $CH_2Ph(2-CH=CH)$ |
| H-0196 | O | $CH_2Ph(3-CH=CH)$ |
| H-0197 | O | $CH_2Ph(4-CH=CH)$ |
| H-0198 | O | $CH_2Ph(2-c-Pr)$ |
| H-0199 | O | $CH_2Ph(3-c-Pr)$ |
| H-0200 | O | $CH_2Ph(4-c-Pr)$ |
| H-0201 | O | $CH_2Ph(2-c-Bu)$ |
| H-0202 | O | $CH_2Ph(3-c-Bu)$ |
| H-0203 | O | $CH_2Ph(4-c-Bu)$ |
| H-0204 | O | $CH_2Ph(2-OMe)$ |

[Table 166]

| Compound Number | Y | R2 |
|---|---|---|
| H-0205 | O | $CH_2Ph(3-OMe)$ |
| H-0206 | O | $CH_2Ph(4-OMe)$ |
| H-0207 | O | $CH_2Ph(2-OEt)$ |
| H-0208 | O | $CH_2Ph(3-OEt)$ |

(continued)

| Compound Number | Y | R2 |
|---|---|---|
| H-0209 | O | $CH_2Ph(4\text{-}OEt)$ |
| H-0210 | O | $CH_2Ph(2\text{-}On\text{-}Pr)$ |
| H-0211 | O | $CH_2Ph(3\text{-}On\text{-}Pr)$ |
| H-0212 | O | $CH_2Ph(4\text{-}On\text{-}Pr)$ |
| H-0213 | O | $CH_2Ph(2\text{-}Oi\text{-}Pr)$ |
| H-0214 | O | $CH_2Ph(3\text{-}Oi\text{-}Pr)$ |
| H-0215 | O | $CH_2Ph(4\text{-}Oi\text{-}Pr)$ |
| H-0216 | O | $CH_2Ph(2\text{-}OCF_3)$ |
| H-0217 | O | $CH_2Ph(3\text{-}OCF_3)$ |
| H-0218 | O | $CH_2Ph(4\text{-}OCF_3)$ |
| H-0219 | O | $CH_2Ph(2\text{-}OCHF_2)$ |
| H-0220 | O | $CH_2Ph(3\text{-}OCHF_2)$ |
| H-0221 | O | $CH_2Ph(4\text{-}OCHF_2)$ |
| H-0222 | O | $CH_2Ph(2\text{-}OCH_2CF_3)$ |
| H-0223 | O | $CH_2Ph(3\text{-}OCH_2CF_3)$ |
| H-0224 | O | $CH_2Ph(4\text{-}OCH_2CF_3)$ |
| H-0225 | O | $CH_2Ph(2\text{-}SMe)$ |
| H-0226 | O | $CH_2Ph(3\text{-}SMe)$ |
| H-0227 | O | $CH_2Ph(4\text{-}SMe)$ |
| H-0228 | O | $CH_2Ph(2\text{-}SEt)$ |
| H-0229 | O | $CH_2Ph(3\text{-}SEt)$ |
| H-0230 | O | $CH_2Ph(4\text{-}SEt)$ |
| H-0231 | O | $CH_2Ph(2\text{-}SCF_3)$ |
| H-0232 | O | $CH_2Ph(3\text{-}SCF_3)$ |
| H-0233 | O | $CH_2Ph(4\text{-}SCF_3)$ |
| H-0234 | O | $CH_2Ph(2\text{-}SOMe)$ |
| H-0235 | O | $CH_2Ph(3\text{-}SOMe)$ |
| H-0236 | O | $CH_2Ph(4\text{-}SOMe)$ |
| H-0237 | O | $CH_2Ph(2\text{-}SO_2Me)$ |
| H-0238 | O | $CH_2Ph(3\text{-}SO_2Me)$ |
| H-0239 | O | $CH_2Ph(4\text{-}SO_2Me)$ |
| H-0240 | O | $CH_2Ph(2\text{-}CN)$ |
| H-0241 | O | $CH_2Ph(3\text{-}CN)$ |
| H-0242 | O | $CH_2Ph(4\text{-}CN)$ |

(continued)

| Compound Number | Y | R2 |
|---|---|---|
| H-0243 | O | $CH_2Ph(2-CH_2CN)$ |
| H-0244 | O | $CH_2Ph(3-CH_2CN)$ |
| H-0245 | O | $CH_2Ph(4-CH_2CN)$ |
| H-0246 | O | $CH_2Ph[2-(1-CN-c-Pr)]$ |

[Table 167]

| Compound Number | Y | R2 |
|---|---|---|
| H-0247 | O | $CH_2Ph[3-(1-CN-c-Pr)]$ |
| H-0248 | O | $CH_2Ph[4-(1-CN-c-Pr)]$ |
| H-0249 | O | $CH_2Ph(2-CH_2OMe)$ |
| H-0250 | O | $CH_2Ph(3-CH_2OMe)$ |
| H-0251 | O | $CH_2Ph(4-CH_2OMe)$ |
| H-0252 | O | $CH_2Ph(2-CH_2OEt)$ |
| H-0253 | O | $CH_2Ph(3-CH_2OEt)$ |
| H-0254 | O | $CH_2Ph(4-CH_2OEt)$ |
| H-0255 | O | $CH_2Ph(2-CF_3)$ |
| H-0256 | O | $CH_2Ph(3-CF_3)$ |
| H-0257 | O | $CH_2Ph(4-CF_3)$ |
| H-0258 | O | $CH_2Ph(2-CHF_2)$ |
| H-0259 | O | $CH_2Ph(3-CHF_2)$ |
| H-0260 | O | $CH_2Ph(4-CHF_2)$ |
| H-0261 | O | $CH_2Ph(2-CH_2F)$ |
| H-0262 | O | $CH_2Ph(3-CH_2F)$ |
| H-0263 | O | $CH_2Ph(4-CH_2F)$ |
| H-0264 | O | $CH_2Ph(2-CF_2Cl)$ |
| H-0265 | O | $CH_2Ph(3-CF_2Cl)$ |
| H-0266 | O | $CH_2Ph(4-CF_2Cl)$ |
| H-0267 | O | $CH_2Ph[2-CF(CF_3)_2]$ |
| H-0268 | O | $CH_2Ph[3-CF(CF_3)_2]$ |
| H-0269 | O | $CH_2Ph[4-CF(CF_3)_2]$ |
| H-0270 | O | $CH_2Ph(2-COMe)$ |
| H-0271 | O | $CH_2Ph(3-COMe)$ |
| H-0272 | O | $CH_2Ph(4-COMe)$ |
| H-0273 | O | $CH_2Ph(2-COEt)$ |

(continued)

| Compound Number | Y | R2 |
|---|---|---|
| H-0274 | O | $CH_2Ph(3\text{-}COEt)$ |
| H-0275 | O | $CH_2Ph(4\text{-}COEt)$ |
| H-0276 | O | $CH_2Ph(2\text{-}CO_2H)$ |
| H-0277 | O | $CH_2Ph(3\text{-}CO_2H)$ |
| H-0278 | O | $CH_2Ph(4\text{-}CO_2H)$ |
| H-0279 | O | $CH_2Ph(2\text{-}CO_2Me)$ |
| H-0280 | O | $CH_2Ph(3\text{-}CO_2Me)$ |
| H-0281 | O | $CH_2Ph(4\text{-}CO_2Me)$ |
| H-0282 | O | $CH_2Ph(2\text{-}CO_2Et)$ |
| H-0283 | O | $CH_2Ph(3\text{-}CO_2Et)$ |
| H-0284 | O | $CH_2Ph(4\text{-}CO_2Et)$ |
| H-0285 | O | $CH_2Ph(2\text{-}CONH_2)$ |
| H-0286 | O | $CH_2Ph(3\text{-}CONH_2)$ |
| H-0287 | O | $CH_2Ph(4\text{-}CONH_2)$ |
| H-0288 | O | $CH_2Ph(2\text{-}CONHMe)$ |

[Table 168]

| Compound Number | Y | R2 |
|---|---|---|
| H-0289 | O | $CH_2Ph(3\text{-}CONHMe)$ |
| H-0290 | O | $CH_2Ph(4\text{-}CONHMe)$ |
| H-0291 | O | $CH_2Ph(2\text{-}CONHEt)$ |
| H-0292 | O | $CH_2Ph(3\text{-}CONHEt)$ |
| H-0293 | O | $CH_2Ph(4\text{-}CONHEt)$ |
| H-0294 | O | $CH_2Ph(2\text{-}NHCHO)$ |
| H-0295 | O | $CH_2Ph(3\text{-}NHCHO)$ |
| H-0296 | O | $CH_2Ph(4\text{-}NHCHO)$ |
| H-0297 | O | $CH_2Ph(2\text{-}NHCOMe)$ |
| H-0298 | O | $CH_2Ph(3\text{-}NHCOMe)$ |
| H-0299 | O | $CH_2Ph(4\text{-}NHCOMe)$ |
| H-0300 | O | $CH_2Ph(2\text{-}NHCOEt)$ |
| H-0301 | O | $CH_2Ph(3\text{-}NHCOEt)$ |
| H-0302 | O | $CH_2Ph(4\text{-}NHCOEt)$ |
| H-0303 | O | $CH_2Ph(2\text{-}CH=NOH)$ |
| H-0304 | O | $CH_2Ph(3\text{-}CH=NOH)$ |

(continued)

| Compound Number | Y | R2 |
|---|---|---|
| H-0305 | O | $CH_2Ph(4\text{-}CH{=}NOH)$ |
| H-0306 | O | $CH_2Ph(2\text{-}CH{=}NOMe)$ |
| H-0307 | O | $CH_2Ph(3\text{-}CH{=}NOMe)$ |
| H-0308 | O | $CH_2Ph(4\text{-}CH{=}NOMe)$ |
| H-0309 | O | $CH_2Ph(2\text{-}CMe{=}NOH)$ |
| H-031 0 | O | $CH_2Ph(3\text{-}CMe{=}NOH)$ |
| H-0311 | O | $CH_2Ph(4\text{-}CMe{=}NOH)$ |
| H-0312 | O | $CH_2Ph(2\text{-}CMe{=}NOMe)$ |
| H-0313 | O | $CH_2Ph(3\text{-}CMe{=}NOMe)$ |
| H-0314 | O | $CH_2Ph(4\text{-}CMe{=}NOMe)$ |
| H-0315 | O | $CH_2Ph(2\text{-}NO_2)$ |
| H-0316 | O | $CH_2Ph(3\text{-}NO_2)$ |
| H-0317 | O | $CH_2Ph(4\text{-}NO_2)$ |
| H-0318 | O | $CH_2Ph(2\text{-}NH_2)$ |
| H-0319 | O | $CH_2Ph(3\text{-}NH_2)$ |
| H-0320 | O | $CH_2Ph(4\text{-}NH_2)$ |
| H-0321 | O | $CH_2Ph(2\text{-}OH)$ |
| H-0322 | O | $CH_2Ph(3\text{-}OH)$ |
| H-0323 | O | $CH_2Ph(4\text{-}OH)$ |
| H-0324 | O | $CH_2Ph(2\text{-}SH)$ |
| H-0325 | O | $CH_2Ph(3\text{-}SH)$ |
| H-0326 | O | $CH_2Ph(4\text{-}SH)$ |
| H-0327 | O | $CH_2Ph(2\text{-}NMe_2)$ |
| H-0328 | O | $CH_2Ph(3\text{-}NMe_2)$ |
| H-0329 | O | $CH_2Ph(4\text{-}NMe_2)$ |
| H-0330 | O | $CH_2Ph(2\text{-}NHMe)$ |

[Table 169]

| Compound Number | Y | $R^2$ |
|---|---|---|
| H-0331 | O | $CH_2Ph(3\text{-}NHMe)$ |
| H-0332 | O | $CH_2Ph(4\text{-}NHMe)$ |
| H-0333 | O | $CH_2Ph(2\text{-}CHO)$ |
| H-0334 | O | $CH_2Ph(3\text{-}CHO)$ |
| H-0335 | O | $CH_2Ph(4\text{-}CHO)$ |

(continued)

| Compound Number | Y | $R^2$ |
|---|---|---|
| H-0336 | O | $CH_2Ph$(2-Ph) |
| H-0337 | O | $CH_2Ph$(3-Ph) |
| H-0338 | O | $CH_2Ph$(4-Ph) |
| H-0339 | O | $CH_2Ph$(2-pyridin-2-yl) |
| H-0340 | O | $CH_2Ph$(3-pyridin-2-yl) |
| H-0341 | O | $CH_2Ph$(4-pyridin-2-yl) |
| H-0342 | O | $CH_2Ph$(2-pyridin-3-yl) |
| H-0343 | O | $CH_2Ph$(3-pyridin-3-yl) |
| H-0344 | O | $CH_2Ph$(4-pyridin-3-yl) |
| H-0345 | O | $CH_2Ph$(2-pyridin-4-yl) |
| H-0346 | O | $CH_2Ph$(3-pyridin-4-yl) |
| H-0347 | O | $CH_2Ph$(4-pyridin-4-yl) |
| H-0348 | O | $CH_2Ph(2\text{-}CH_2Ph)$ |
| H-0349 | O | $CH_2Ph(3\text{-}CH_2Ph)$ |
| H-0350 | O | $CH_2Ph(4\text{-}CH_2Ph)$ |
| H-0351 | O | $CH_2Ph(2,3\text{-}F_2)$ |
| H-0352 | O | $CH_2Ph(2,4\text{-}F_2)$ |
| H-0353 | O | $CH_2Ph(2,5\text{-}F_2)$ |
| H-0354 | O | $CH_2Ph(2,6\text{-}F_2)$ |
| H-0355 | O | $CH_2Ph(3,4\text{-}F_2)$ |
| H-0356 | O | $CH_2Ph(3,5\text{-}F_2)$ |
| H-0357 | O | $CH_2Ph$(2-Cl,3-F) |
| H-0358 | O | $CH_2Ph$(2-Cl,4-F) |
| H-0359 | O | $CH_2Ph$(2-Cl,5-F) |
| H-0360 | O | $CH_2Ph$(2-Cl,6-F) |
| H-0361 | O | $CH_2Ph$(3-Cl,2-F) |
| H-0362 | O | $CH_2Ph$(3-Cl,4-F) |
| H-0363 | O | $CH_2Ph$(3-Cl,5-F) |
| H-0364 | O | $CH_2Ph$(4-Cl,2-F) |
| H-0365 | O | $CH_2Ph$(4-Cl,3-F) |
| H-0366 | O | $CH_2Ph$(5-Cl,2-F) |
| H-0367 | O | $CH_2Ph$(2-F,3-Me) |
| H-0368 | O | $CH_2Ph$(2-F,4-Me) |
| H-0369 | O | $CH_2Ph$(2-F,5-Me) |

(continued)

| Compound Number | Y | $R^2$ |
|---|---|---|
| H-0370 | O | $CH_2Ph$(2-F,6-Me) |
| H-0371 | O | $CH_2Ph$(3-F,2-Me) |
| H-0372 | O | $CH_2Ph$(3-F,4-Me) |

[Table 170]

| Compound Number | Y | $R^2$ |
|---|---|---|
| H-0373 | O | $CH_2Ph$(3-F,5-Me) |
| H-0374 | O | $CH_2Ph$(4-F,2-Me) |
| H-0375 | O | $CH_2Ph$(4-F,3-Me) |
| H-0376 | O | $CH_2Ph$(5-F,2-Me) |
| H-0377 | O | $CH_2Ph$(2-CN,3-F) |
| H-0378 | O | $CH_2Ph$(2-CN,4-F) |
| H-0379 | O | $CH_2Ph$(2-CN,5-F) |
| H-0380 | O | $CH_2Ph$(2-CN,6-F) |
| H-0381 | O | $CH_2Ph$(3-CN,2-F) |
| H-0382 | O | $CH_2Ph$(3-CN,4-F) |
| H-0383 | O | $CH_2Ph$(3-CN,5-F) |
| H-0384 | O | $CH_2Ph$(4-CN,2-F) |
| H-0385 | O | $CH_2Ph$(4-CN,3-F) |
| H-0386 | O | $CH_2Ph$(5-CN,2-F) |
| H-0387 | O | $CH_2Ph$(2-F,3-OMe) |
| H-0388 | O | $CH_2Ph$(2-F,4-OMe) |
| H-0389 | O | $CH_2Ph$(2-F,5-OMe) |
| H-0390 | O | $CH_2Ph$(2-F,6-OMe) |
| H-0391 | O | $CH_2Ph$(3-F,2-OMe) |
| H-0392 | O | $CH_2Ph$(3-F,4-OMe) |
| H-0393 | O | $CH_2Ph$(3-F,5-OMe) |
| H-0394 | O | $CH_2Ph$(4-F,2-OMe) |
| H-0395 | O | $CH_2Ph$(4-F,3-OMe) |
| H-0396 | O | $CH_2Ph$(5-F,2-OMe) |
| H-0397 | O | $CH_2Ph(2,3-Cl_2)$ |
| H-0398 | O | $CH_2Ph(2,4-Cl_2)$ |
| H-0399 | O | $CH_2Ph(2,5-Cl_2)$ |
| H-0400 | O | $CH_2Ph(2,6-Cl_2)$ |

(continued)

| Compound Number | Y | $R^2$ |
|---|---|---|
| H-0401 | O | $CH_2Ph(3,4-Cl_2)$ |
| H-0402 | O | $CH_2Ph(3,5-Cl_2)$ |
| H-0403 | O | $CH_2$Ph(2-Cl,3-Me) |
| H-0404 | O | $CH_2$Ph(2-Cl,4-Me) |
| H-0405 | O | $CH_2$Ph(2-Cl,5-Me) |
| H-0406 | O | $CH_2$Ph(2-Cl,6-Me) |
| H-0407 | O | $CH_2$Ph(3-Cl,2-Me) |
| H-0408 | O | $CH_2$Ph(3-Cl,4-Me) |
| H-0409 | O | $CH_2$Ph(3-Cl,5-Me) |
| H-0410 | O | $CH_2$Ph(4-Cl,2-Me) |
| H-0411 | O | $CH_2$Ph(4-Cl,3-Me) |
| H-0412 | O | $CH_2$Ph(5-Cl,2-Me) |
| H-0413 | O | $CH_2$Ph(2-Cl,3-CN) |
| H-0414 | O | $CH_2$Ph(2-Cl,4-CN) |

[Table 171]

| Compound Number | Y | R2 |
|---|---|---|
| H-0415 | O | $CH_2$Ph(2-Cl,5-CN) |
| H-0416 | O | $CH_2$Ph(2-Cl,6-CN) |
| H-0417 | O | $CH_2$Ph(3-Cl,2-CN) |
| H-0418 | O | $CH_2$Ph(3-Cl,4-CN) |
| H-0419 | O | $CH_2$Ph(3-Cl,5-CN) |
| H-0420 | O | $CH_2$Ph(4-Cl,2-CN) |
| H-0421 | O | $CH_2$Ph(4-Cl,3-CN) |
| H-0422 | O | $CH_2$Ph(5-Cl,2-CN) |
| H-0423 | O | $CH_2$Ph(2-Cl,3-OMe) |
| H-0424 | O | $CH_2$Ph(2-Cl,4-OMe) |
| H-0425 | O | $CH_2$Ph(2-Cl,5-OMe) |
| H-0426 | O | $CH_2$Ph(2-Cl,6-OMe) |
| H-0427 | O | $CH_2$Ph(3-Cl,2-OMe) |
| H-0428 | O | $CH_2$Ph(3-Cl,4-OMe) |
| H-0429 | O | $CH_2$Ph(3-Cl,5-OMe) |
| H-0430 | O | $CH_2$Ph(4-Cl,2-OMe) |
| H-0431 | O | $CH_2$Ph(4-Cl,3-OMe) |

(continued)

| Compound Number | Y | R2 |
|---|---|---|
| H-0432 | O | $CH_2Ph(5-Cl,2-OMe)$ |
| H-0433 | O | $CH_2Ph(2,3-Me_2)$ |
| H-0434 | O | $CH_2Ph(2,4-Me_2)$ |
| H-0435 | O | $CH_2Ph(2,5-Me_2)$ |
| H-0436 | O | $CH_2Ph(2,6-Me_2)$ |
| H-0437 | O | $CH_2Ph(3,4-Me_2)$ |
| H-0438 | O | $CH_2Ph(3,5-Me_2)$ |
| H-0439 | O | $CH_2Ph(2-CN,3-Me)$ |
| H-0440 | O | $CH_2Ph(2-CN,4-Me)$ |
| H-0441 | O | $CH_2Ph(2-CN,5-Me)$ |
| H-0442 | O | $CH_2Ph(2-CN,6-Me)$ |
| H-0443 | O | $CH_2Ph(3-CN,2-Me)$ |
| H-0444 | O | $CH_2Ph(3-CN,4-Me)$ |
| H-0445 | O | $CH_2Ph(3-CN,5-Me)$ |
| H-0446 | O | $CH_2Ph(4-CN,2-Me)$ |
| H-0447 | O | $CH_2Ph(4-CN,3-Me)$ |
| H-0448 | O | $CH_2Ph(5-CN,2-Me)$ |
| H-0449 | O | $CH_2Ph(2-OMe,3-Me)$ |
| H-0450 | O | $CH_2Ph(2-OMe,4-Me)$ |
| H-0451 | O | $CH_2Ph(2-OMe,5-Me)$ |
| H-0452 | O | $CH_2Ph(2-OMe,6-Me)$ |
| H-0453 | O | $CH_2Ph(3-OMe,2-Me)$ |
| H-0454 | O | $CH_2Ph(3-OMe,4-Me)$ |
| H-0455 | O | $CH_2Ph(3-OMe,5-Me)$ |
| H-0456 | O | $CH_2Ph(4-OMe,2-Me)$ |

[Table 172]

| Compound Number | Y | R2 |
|---|---|---|
| H-0457 | O | $CH_2Ph(4-OMe,3-Me)$ |
| H-0458 | O | $CH_2Ph(5-OMe,2-Me)$ |
| H-0459 | O | $CH_2Ph[2,3-(OMe)_2]$ |
| H-0460 | O | $CH_2Ph[2,4-(OMe)_2]$ |
| H-0461 | O | $CH_2Ph[2,5-(OMe)_2]$ |
| H-0462 | O | $CH_2Ph[2,6-(OMe)_2]$ |

(continued)

| Compound Number | Y | R2 |
|---|---|---|
| H-0463 | O | $CH_2Ph[3,4-(OMe)_2]$ |
| H-0464 | O | $CH_2Ph[3,5-(OMe)_2]$ |
| H-0465 | O | $CH_2Ph$(2-CN,3-OMe) |
| H-0466 | O | $CH_2Ph$(2-CN,4-OMe) |
| H-0467 | O | $CH_2Ph$(2-CN,5-OMe) |
| H-0468 | O | $CH_2Ph$(2-CN,6-OMe) |
| H-0469 | O | $CH_2Ph$(3-CN,2-OMe) |
| H-0470 | O | $CH_2Ph$(3-CN,4-OMe) |
| H-0471 | O | $CH_2Ph$(3-CN,5-OMe) |
| H-0472 | O | $CH_2Ph$(4-CN,2-OMe) |
| H-0473 | O | $CH_2Ph$(4-CN,3-OMe) |
| H-0474 | O | $CH_2Ph$(5-CN,2-OMe) |
| H-0475 | O | $CH_2Ph[2,3-(CN)_2]$ |
| H-0476 | O | $CH_2Ph[2,4-(CN)_2]$ |
| H-0477 | O | $CH_2Ph[2,5-(CN)_2]$ |
| H-0478 | O | $CH_2Ph[2,6-(CN)_2]$ |
| H-0479 | O | $CH_2Ph[3,4-(CN)_2]$ |
| H-0480 | O | $CH_2Ph[3,5-(CN)_2]$ |
| H-0481 | O | $CH_2CH_2Ph$(2-F) |
| H-0482 | O | $CH_2CH_2Ph$(3-F) |
| H-0483 | O | $CH_2CH_2Ph$(4-F) |
| H-0484 | O | $CH_2CH$=CHPh |
| H-0485 | O | $CH_2CCPh$ |
| H-0486 | O | $CH_2CCTMS$ |
| H-0487 | O | $CH_2F$ |
| H-0488 | O | $CH_2CH_2F$ |
| H-0489 | O | $CH_2CH_2CH_2F$ |
| H-0490 | O | $CH_2CHO$ |
| H-0491 | O | $CH_2CH_2CHO$ |
| H-0492 | O | $CH_2COEt$ |
| H-0493 | O | $CH_2CO$n-Pr |
| H-0494 | O | $CH_2CO$n-Bu |
| H-0495 | O | $CH_2CO$i-Pr |
| H-0496 | O | $CH_2CO$t-Bu |

(continued)

| Compound Number | Y | R2 |
|---|---|---|
| H-0497 | O | $CH_2CO$i-Bu |
| H-0498 | O | $CH_2CO$sec-Bu |

[Table 173]

| Compound Number | Y | $R^2$ |
|---|---|---|
| H-0499 | O | $CH_2COCH_2$t-Bu |
| H-0500 | O | $CH_2COCH(CH_2CH_3)Et$ |
| H-0501 | O | $CH_2CO$c-Pr |
| H-0502 | O | $CH_2COCH_2$c-Pr |
| H-0503 | O | $CH_2CO$c-Bu |
| H-0504 | O | $CH_2CO$c-Pen |
| H-0505 | O | $CH_2CH_2COMe$ |
| H-0506 | O | $CH_2CH_2COEt$ |
| H-0507 | O | $CH_2COCF_3$ |
| H-0508 | O | $CH_2COCHF_2$ |
| H-0509 | O | $CH_2COCH_2CF_3$ |
| H-051 0 | O | $CH_2COCH_2CHF_2$ |
| H-0511 | O | $CH_2COCH_2OCH_3$ |
| H-0512 | O | $CH_2COCH_2Ph$ |
| H-0513 | O | $CH_2CO$(pyrrolidin-1-yl) |
| H-0514 | O | $CH_2CO$(1H-pyrazol-1-yl) |
| H-0515 | O | $CH_2CO$(morpholino-1-yl) |
| H-0516 | O | $CH_2CO$(pyridin-2-yl) |
| H-0517 | O | $CH_2CO$(pyridin-3-yl) |
| H-0518 | O | $CH_2CO$(pyridin-4-yl) |
| H-0519 | O | $CH_2COCH_2$(pyridin-2-yl) |
| H-0520 | O | $CH_2COCH_2$(pyridin-3-yl) |
| H-0521 | O | $CH_2COCH_2$(pyridin-4-yl) |
| H-0522 | O | $CH_2CO_2H$ |
| H-0523 | O | $CH_2CH_2CO_2Me$ |
| H-0524 | O | $CH(CH_2CH_3)CO_2Et$ |
| H-0525 | O | $CH(F)CO_2Et$ |
| H-0526 | O | $CH(CO_2Et)_2$ |
| H-0527 | O | $CH_2CH_2CO_2Et$ |

(continued)

| Compound Number | Y | $R^2$ |
|---|---|---|
| H-0528 | O | $CH_2CH_2CH_2CO_2Et$ |
| H-0529 | O | $CH_2CH_2CH_2CH_2CH_2CO_2Et$ |
| H-0530 | O | $CH_2CO_2$n-Pr |
| H-0531 | O | $CH(CH_3)CO_2$n-Pr |
| H-0532 | O | $C(CH_3)(CH_3)CO_2$n-Pr |
| H-0533 | O | $CH_2CO_2$i-Pr |
| H-0534 | O | $CH(CH_3)CO_2$i-Pr |
| H-0535 | O | $C(CH_3)(CH_3)CO_2$i-Pr |
| H-0536 | O | $CH_2CO_2$n-Bu |
| H-0537 | O | $CH_2CO_2$t-Bu |
| H-0538 | O | $CH_2CO_2$i-Bu |
| H-0539 | O | $CH_2CO_2$c-Pr |
| H-0540 | O | $CH_2CO_2CH_2$c-Pr |

[Table 174]

| Compound Number | Y | $R^2$ |
|---|---|---|
| H-0541 | O | $CH_2CO_2$c-Bu |
| H-0542 | O | $CH_2CO_2$c-Pen |
| H-0543 | O | $CH_2CO_2CH{=}CH_2$ |
| H-0544 | O | $CH_2CO_2CH_2CH{=}CH_2$ |
| H-0545 | O | $CH_2CO_2CH_2CCH$ |
| H-0546 | O | $CH_2CO_2CF_3$ |
| H-0547 | O | $CH_2CO_2CHF_2$ |
| H-0548 | O | $CH_2CO_2CH_2CF_3$ |
| H-0549 | O | $CH_2CO_2CH_2CHF_2$ |
| H-0550 | O | $CH_2CO_2CH_2CN$ |
| H-0551 | O | $CH_2CO_2CH_2COMe$ |
| H-0552 | O | $CH_2CO_2CH_2CO_2Et$ |
| H-0553 | O | $CH_2CO_2CH_2CO_2CH_2CF_3$ |
| H-0554 | O | $CH_2CO_2CH_2Ph$ |
| H-0555 | O | $CH_2CO_2$(pyridin-2-yl) |
| H-0556 | O | $CH_2CO_2$(pyridin-3-yl) |
| H-0557 | O | $CH_2CO_2$(pyridin-4-yl) |
| H-0558 | O | $CH_2CO_2CH_2$(pyridin-2-yl) |

(continued)

| Compound Number | Y | $R^2$ |
|---|---|---|
| H-0559 | O | $CH_2CO_2CH_2$[pyridin-2-yl(6-F)] |
| H-0560 | O | $CH_2CO_2CH_2$[pyridin-2-yl(6-Cl)] |
| H-0561 | O | $CH_2CO_2CH_2$[pyridin-2-yl(6-Me)] |
| H-0562 | O | $CH_2CO_2CH_2$(pyridin-3-yl) |
| H-0563 | O | $CH_2CO_2CH_2$[pyridin-3-yl(6-F)] |
| H-0564 | O | $CH_2CO_2CH_2$[pyridin-3-yl(6-Cl)] |
| H-0565 | O | $CH_2CO_2CH_2$[pyridin-3-yl(6-Me)] |
| H-0566 | O | $CH_2CO_2CH_2$(pyridin-4-yl) |
| H-0567 | O | $CH_2CO_2CH_2$(1H-pyrazol-1-yl) |
| H-0568 | O | $CH_2CO_2CH_2$[1H-pyrazol-3-yl(1-Me)] |
| H-0569 | O | $CH_2CO_2CH_2$[1H-pyrazol-4-yl(1-Me)] |
| H-0570 | O | $CH_2CO_2CH_2$[1H-pyrazol-5-yl(1-Me)] |
| H-0571 | O | $CH_2CONMe_2$ |
| H-0572 | O | $CH_2CON(CH_3)Et$ |
| H-0573 | O | $CH_2CON(CH_3)$n-Pr |
| H-0574 | O | $CH_2CONEt_2$ |
| H-0575 | O | $CH_2CONH$n-Pr |
| H-0576 | O | $CH_2CONH$i-Pr |
| H-0577 | O | $CH_2CONH$n-Bu |
| H-0578 | O | $CH_2CONH$t-Bu |
| H-0579 | O | $CH_2CONH$i-Bu |
| H-0580 | O | $CH_2CONH$c-Pr |
| H-0581 | O | $CH_2CONH$cPr(1-CN) |
| H-0582 | O | $CH_2CONHCH_2$c-Pr |

[Table 175]

| Compound Number | Y | $R^2$ |
|---|---|---|
| H-0583 | O | $CH_2CONHCH=CH_2$ |
| H-0584 | O | $CH_2CONHCH_2CH=CH_2$ |
| H-0585 | O | $CH_2CONHCH_2CCH$ |
| H-0586 | O | $CH_2CONHCF_3$ |
| H-0587 | O | $CH_2CONHCHF_2$ |
| H-0588 | O | $CH_2CONHCH_2CF_3$ |
| H-0589 | O | $CH_2CONHCH_2CHF_2$ |

(continued)

| Compound Number | Y | $R^2$ |
|---|---|---|
| H-0590 | O | $CH_2CH_2CONHMe$ |
| H-0591 | O | $CH_2CH_2CONHEt$ |
| H-0592 | O | $CH_2CH_2CONMe_2$ |
| H-0593 | O | $CH_2CH_2CON(CH_3)Et$ |
| H-0594 | O | $CH_2CONHCH_2Ph$ |
| H-0595 | O | $CH_2CONH$(pyridin-2-yl) |
| H-0596 | O | $CH_2CONH$(pyridin-3-yl) |
| H-0597 | O | $CH_2CONH$(pyridin-4-yl) |
| H-0598 | O | $CH_2CONHCH_2$(pyridin-2-yl) |
| H-0599 | O | $CH_2CONHCH_2$(pyridin-3-yl) |
| H-0600 | O | $CH_2CONHCH_2$(pyridin-4-yl) |
| H-0601 | O | $CH_2CH_2NHCHO$ |
| H-0602 | O | $CH_2CH_2NHCOMe$ |
| H-0603 | O | $CH_2CH_2NHCOEt$ |
| H-0604 | O | $CH_2CH_2N(CH_3)CHO$ |
| H-0605 | O | $CH_2CH_2N(CH_3)COMe$ |
| H-0606 | O | $CH_2CH_2N(CH_3)COEt$ |
| H-0607 | O | $CH_2CH_2N(CH_2CH_3)CHO$ |
| H-0608 | O | $CH_2CH_2N(CH_2CH_3)COMe$ |
| H-0609 | O | $CH_2CH_2N(CH_2CH_3)COEt$ |
| H-061 0 | O | $CH_2NHCO$c-Pr |
| H-0611 | O | $CH_2NHCOCH_2$c-Pr |
| H-0612 | O | $CH_2CH_2NHCO$c-Pr |
| H-0613 | O | $CH_2CH_2NHCOCH_2$c-Pr |
| H-0614 | O | $CH_2NHCOCF_3$ |
| H-0615 | O | $CH_2NHCOCHF_2$ |
| H-0616 | O | $CH_2NHCOCH_2CF_3$ |
| H-0617 | O | $CH_2NHCOCH_2CHF_2$ |
| H-0618 | O | $CH_2NHCOCH_2Ph$ |
| H-0619 | O | $CH_2NHCO$(pyridin-2-yl) |
| H-0620 | O | $CH_2NHCO$(pyridin-3-yl) |
| H-0621 | O | $CH_2NHCO$(pyridin-4-yl) |
| H-0622 | O | $CH_2NHCOCH_2$(pyridin-2-yl) |
| H-0623 | O | $CH_2NHCOCH_2$(pyridin-3-yl) |

(continued)

| Compound Number | Y | $R^2$ |
|---|---|---|
| H-0624 | O | $CH_2NHCOCH_2$(pyridin-4-yl) |

[Table 176]

| Compound Number | Y | R2 |
|---|---|---|
| H-0625 | O | $CH_2OCHO$ |
| H-0626 | O | $CH_2CH_2OCHO$ |
| H-0627 | O | $CH_2OCOMe$ |
| H-0628 | O | $CH_2OCOEt$ |
| H-0629 | O | $CH_2OCOn$-Pr |
| H-0630 | O | $CH_2OCOi$-Pr |
| H-0631 | O | $CH_2OCOn$-Bu |
| H-0632 | O | $CH_2OCOi$-Bu |
| H-0633 | O | $CH_2OCOt$-Bu |
| H-0634 | O | $CH_2CH_2OCOMe$ |
| H-0635 | O | $CH_2CH_2OCOEt$ |
| H-0636 | O | $CH_2CH_2CH_2OCOMe$ |
| H-0637 | O | $CH_2OCOc$-Pr |
| H-0638 | O | $CH_20COCH_2c$-Pr |
| H-0639 | O | $CH_2OCOCF_3$ |
| H-0640 | O | $CH_2OCOCHF_2$ |
| H-0641 | O | $CH_2OCOCH_2CF_3$ |
| H-0642 | O | $CH_2OCOCH_2CHF_2$ |
| H-0643 | O | $CH_2OCOPh$ |
| H-0644 | O | $CH_2OCOCH_2Ph$ |
| H-0645 | O | $CH_2OCO$(pyridin-2-yl) |
| H-0646 | O | $CH_2OCO$(pyridin-3-yl) |
| H-0647 | O | $CH_2OCO$(pyridin-4-yl) |
| H-0648 | O | $CH_2OCOCH_2$(pyridin-2-yl) |
| H-0649 | O | $CH_2OCOCH_2$(pyridin-3-yl) |
| H-0650 | O | $CH_2OCOCH_2$(pyridin-4-yl) |
| H-0651 | O | $CH_2SO_2NHMe$ |
| H-0652 | O | $CH_2SO_2NHEt$ |
| H-0653 | O | $CH_2SO_2NMe_2$ |
| H-0654 | O | $CH_2SO_2N(CH_3)Et$ |

(continued)

| Compound Number | Y | R2 |
|---|---|---|
| H-0655 | O | $CH_2CH_2SO_2NHMe$ |
| H-0656 | O | $CH_2CH_2SO_2NHEt$ |
| H-0657 | O | $CH_2CH_2SO_2NMe_2$ |
| H-0658 | O | $CH_2CH_2SO_2(CH_3)Et$ |
| H-0659 | O | $CH_2SO_2NHc$-Pr |
| H-0660 | O | $CH_2SO_2NHCH_2c$-Pr |
| H-0661 | O | $CH_2SO_2NHCF_3$ |
| H-0662 | O | $CH_2SO_2NHCH_2CF_3$ |
| H-0663 | O | $CH_2SO_2NHCHF_2$ |
| H-0664 | O | $CH_2SO_2NHCH_2CHF_2$ |
| H-0665 | O | $CH_2SO_2NHPh$ |
| H-0666 | O | $CH_2SO_2NHCH_2Ph$ |

[Table 177]

| Compound Number | Y | R2 |
|---|---|---|
| H-0667 | O | $CH_2SO_2NH$(pyridin-2-yl) |
| H-0668 | O | $CH_2SO_2NH$(pyridin-3-yl) |
| H-0669 | O | $CH_2SO_2NH$(pyridin-4-yl) |
| H-0670 | O | $CH_2SO_2NHCH_2$(pyridin-2-yl) |
| H-0671 | O | $CH_2SO_2NHCH_2$(pyridin-3-yl) |
| H-0672 | O | $CH_2SO_2NHCH_2$(pyridin-4-yl) |
| H-0673 | O | $CH_2NHSO_2Me$ |
| H-0674 | O | $CH_2N(CH_3)SO_2Me$ |
| H-0675 | O | $CH_2NHSO_2Et$ |
| H-0676 | O | $CH_2NHSO_2c$-Pr |
| H-0677 | O | $CH_2NHSO_2CH_2c$-Pr |
| H-0678 | O | $CH_2NHSO_2CF_3$ |
| H-0679 | O | $CH_2NHSO_2CH_2CF_3$ |
| H-0680 | O | $CH_2NHSO_2CHF_2$ |
| H-0681 | O | $CH_2NHSO_2CH_2CHF_2$ |
| H-0682 | O | $CH_2CH_2NHSO_2Me$ |
| H-0683 | O | $CH_2CH_2N(CH_3)SO_2Me$ |
| H-0684 | O | $CH_2NHSO_2Ph$ |
| H-0685 | O | $CH_2NHSO_2CH_2Ph$ |

(continued)

| Compound Number | Y | R2 |
|---|---|---|
| H-0686 | O | $CH_2NHSO_2$(pyridin-2-yl) |
| H-0687 | O | $CH_2NHSO_2$(pyridin-3-yl) |
| H-0688 | O | $CH_2NHSO_2$(pyridin-4-yl) |
| H-0689 | O | $CH_2NHSO_2CH_2$(pyridin-2-yl) |
| H-0690 | O | $CH_2NHSO_2CH_2$(pyridin-3-yl) |
| H-0691 | O | $CH_2NHSO_2CH_2$(pyridin-4-yl) |
| H-0692 | O | $CH_2OH$ |
| H-0693 | O | $CH_2CH_2OH$ |
| H-0694 | O | $CH_2CH(OH)CH_2CH_2CH_3$ |
| H-0695 | O | $CH_2CH_2$On-Pr |
| H-0696 | O | $CH_2CH_2$Oi-Pr |
| H-0697 | O | $CH_2CH_2$Oc-Pr |
| H-0698 | O | $CH_2CH_2OCH_2$c-Pr |
| H-0699 | O | $CH_2CH_2$Ot-Bu |
| H-0700 | O | $CH_2CH_2$Oi-Bu |
| H-0701 | O | $CH_2CH_2$Osec-Bu |
| H-0702 | O | $CH(CH_3)OEt$ |
| H-0703 | O | $CH_2CH(CH_3)OEt$ |
| H-0704 | O | $CH_2CH(OMe)_2$ |
| H-0705 | O | $CH_2CH(OEt)_2$ |
| H-0706 | O | $CH(CH_3)CH(OMe)_2$ |
| H-0707 | O | $CH_2C(OMe)_2Me$ |
| H-0708 | O | $CH(CH_3)CH(OEt)_2$ |

[Table 178]

| Compound Number | Y | R2 |
|---|---|---|
| H-0709 | O | $CH_2C(OEt)_2Me$ |
| H-071 0 | O | $CH_2CH_2CH(OMe)_2$ |
| H-0711 | O | $CH_2CH_2CH(OEt)_2$ |
| H-0712 | O | $CH_2CH_2CH_2OMe$ |
| H-0713 | O | $CH_2OCHF_2$ |
| H-0714 | O | $CH_2OCH_2CHF_2$ |
| H-0715 | O | $CH_2C(OH)_2CF_3$ |
| H-0716 | O | $CH_2CH_2OCHF_2$ |

(continued)

| Compound Number | Y | R2 |
|---|---|---|
| H-0717 | O | $CH_2CH_2OCH_2CHF_2$ |
| H-0718 | O | $CH_2OCH_2Ph$ |
| H-0719 | O | $CH_2OCH_2CH_2OMe$ |
| H-0720 | O | $CH_2CH_2OCH_2CH_2OMe$ |
| H-0721 | O | $CH_2O$(pyridin-2-yl) |
| H-0722 | O | $CH_2O$(pyridin-3-yl) |
| H-0723 | O | $CH_2O$(pyridin-4-yl) |
| H-0724 | O | $CH_2OCH_2$(pyridin-2-yl) |
| H-0725 | O | $CH_2OCH_2$(pyridin-3-yl) |
| H-0726 | O | $CH_2OCH_2$(pyridin-4-yl) |
| H-0727 | O | $CH_2SH$ |
| H-0728 | O | $CH_2CH_2SH$ |
| H-0729 | O | $CH_2SOMe$ |
| H-0730 | O | $CH_2SO_2Me$ |
| H-0731 | O | $CH_2SOEt$ |
| H-0732 | O | $CH_2SO_2Et$ |
| H-0733 | O | $CH_2CH_2SOMe$ |
| H-0734 | O | $CH_2CH_2SO_2Me$ |
| H-0735 | O | $CH_2CH_2SOEt$ |
| H-0736 | O | $CH_2CH_2SO_2Et$ |
| H-0737 | O | $CH_2Sc$-Pr |
| H-0738 | O | $CH_2SOc$-Pr |
| H-0739 | O | $CH_2SO_2c$-Pr |
| H-0740 | O | $CH_2SCH_2c$-Pr |
| H-0741 | O | $CH_2SOCH_2c$-Pr |
| H-0742 | O | $CH_2SO_2CH_2c$-Pr |
| H-0743 | O | $CH_2CH_2Si$-Pr |
| H-0744 | O | $CH_2CH_2SOi$-Pr |
| H-0745 | O | $CH_2CH_2SO_2i$-Pr |
| H-0746 | O | $CH_2SOCF_3$ |
| H-0747 | O | $CH_2SO_2CF_3$ |
| H-0748 | O | $CH_2SOCHF_2$ |
| H-0749 | O | $CH_2SO_2CHF_2$ |
| H-0750 | O | $CH_2SCH_2Ph$ |

[Table 179]

| Compound Number | Y | R2 |
|---|---|---|
| H-0751 | O | $CH_2SOCH_2Ph$ |
| H-0752 | O | $CH_2SO_2CH_2Ph$ |
| H-0753 | O | $CH_2S$(pyridin-2-yl) |
| H-0754 | O | $CH_2SO$(pyridin-2-yl) |
| H-0755 | O | $CH_2SO_2$(pyridin-2-yl) |
| H-0756 | O | $CH_2SCH_2$(pyridin-2-yl) |
| H-0757 | O | $CH_2SOCH_2$(pyridin-2-yl) |
| H-0758 | O | $CH_2SO_2CH_2$(pyridin-2-yl) |
| H-0759 | O | $CH_2S$(pyridin-3-yl) |
| H-0760 | O | $CH_2SO$(pyridin-3-yl) |
| H-0761 | O | $CH_2SO_2$(pyridin-3-yl) |
| H-0762 | O | $CH_2SCH_2$(pyridin-3-yl) |
| H-0763 | O | $CH_2SOCH_2$(pyridin-3-yl) |
| H-0764 | O | $CH_2SO_2CH_2$(pyridin-3-yl) |
| H-0765 | O | $CH_2S$(pyridin-4-yl) |
| H-0766 | O | $CH_2SO$(pyridin-4-yl) |
| H-0767 | O | $CH_2SO_2$(pyridin-4-yl) |
| H-0768 | O | $CH_2SCH_2$(pyridin-4-yl) |
| H-0769 | O | $CH_2SOCH_2$(pyridin-4-yl) |
| H-0770 | O | $CH_2SO_2CH_2$(pyridin-4-yl) |
| H-0771 | O | $CH_2CH$(=NOH) |
| H-0772 | O | $CH_2CH$(=NOMe) |
| H-0773 | O | $CH_2CMe$(=NOMe) |
| H-0774 | O | $CH_2CEt$(=NOMe) |
| H-0775 | O | $CH_2Cn$-Pr(=NOMe) |
| H-0776 | O | $CH_2CMe$(=NOEt) |
| H-0777 | O | $CH_2CMe(=NOCH_2CH=CH_2)$ |
| H-0778 | O | $CH_2CMe$(=NOPh) |
| H-0779 | O | $CH_2CMe(=NOCH_2Ph)$ |
| H-0780 | O | $CH_2CMe$[=NO(pyridin-2-yl)] |
| H-0781 | O | $CH_2CMe$[=NO(pyridin-3-yl)] |
| H-0782 | O | $CH_2CMe$[=NO(pyridin-4-yl)] |
| H-0783 | O | $CH_2CMe[=NOCH_2$(pyridin-2-yl)] |
| H-0784 | O | $CH_2CMe[=NOCH_2$(pyridin-3-yl)] |

(continued)

| Compound Number | Y | R2 |
|---|---|---|
| H-0785 | O | $CH_2CMe[=NOCH_2(pyridin-4-yl)]$ |
| H-0786 | O | dihydrofuran-2(3H)-one-3-yl |
| H-0787 | O | $CH_2OCO_2Me$ |
| H-0788 | O | $CH_2OCO_2Et$ |
| H-0789 | O | $CH_2OCO_2n$-Pr |
| H-0790 | O | $CH_2OCO_2i$-Pr |
| H-0791 | O | $CH_2OCO_2c$-Pr |
| H-0792 | O | $CH_2OCO_2CH_2c$-Pr |

[Table 180]

| Compound Number | Y | $R^2$ |
|---|---|---|
| H-0793 | O | $CH_2OCO_2n$-Bu |
| H-0794 | O | $CH_2OCO_2i$-Bu |
| H-0795 | O | $CH_2OCO_2t$-Bu |
| H-0796 | O | $CH_2OCO_2CF_3$ |
| H-0797 | O | $CH_20CO_2CH_2CF_3$ |
| H-0798 | O | $CH_2OCO_2CHF_2$ |
| H-0799 | O | $CH_2OCO_2CH_2CHF_2$ |
| H-0800 | O | $CH_2CH_2OCO_2Me$ |
| H-0801 | O | $CH_2CH_2OCO_2Et$ |
| H-0802 | O | $CH_2OCO_2Ph$ |
| H-0803 | O | $CH_2OCO_2CH_2Ph$ |
| H-0804 | O | $CH_2OCO_2$(pyridin-2-yl) |
| H-0805 | O | $CH_2OCO_2$(pyridin-3-yl) |
| H-0806 | O | $CH_2OCO_2$(pyridin-4-yl) |
| H-0807 | O | $CH_2OCO_2CH_2$(pyridin-2-yl) |
| H-0808 | O | $CH_2OCO_2CH_2$(pyridin-3-yl) |
| H-0809 | O | $CH_2OCO_2CH_2$(pyridin-4-yl) |
| H-081 0 | O | $CH_2NHCONHMe$ |
| H-0811 | O | $CH_2NHCONHEt$ |
| H-0812 | O | $CH_2NHCONHn$-Pr |
| H-0813 | O | $CH_2NHCONHi$-Pr |
| H-0814 | O | $CH_2NHCONHc$-Pr |
| H-0815 | O | $CH_2NHCONHCH_2c$-Pr |

(continued)

| Compound Number | Y | $R^2$ |
|---|---|---|
| H-0816 | O | $CH_2NHCONHn\text{-}Bu$ |
| H-0817 | O | $CH_2NHCONHi\text{-}Bu$ |
| H-0818 | O | $CH_2NHCONHt\text{-}Bu$ |
| H-0819 | O | $CH_2NHCONHCF_3$ |
| H-0820 | O | $CH_2NHCONHCH_2CF_3$ |
| H-0821 | O | $CH_2NHCONHCHF_2$ |
| H-0822 | O | $CH_2NHCONHCH_2CHF_2$ |
| H-0823 | O | $CH_2CH_2NHCONHMe$ |
| H-0824 | O | $CH_2CH_2NHCONHEt$ |
| H-0825 | O | $CH_2NHCONHPh$ |
| H-0826 | O | $CH_2NHCONHCH_2Ph$ |
| H-0827 | O | $CH_2NHCONH$(pyridin-2-yl) |
| H-0828 | O | $CH_2NHCONH$(pyridin-3-yl) |
| H-0829 | O | $CH_2NHCONH$(pyridin-4-yl) |
| H-0830 | O | $CH_2NHCONH_2CH_2$(pyridin-2-yl) |
| H-0831 | O | $CH_2NHCONHCH_2$(pyridin-3-yl) |
| H-0832 | O | $CH_2NHCONHCH_2$(pyridin-4-yl) |
| H-0833 | O | $CH_2CONHNH_2$ |
| H-0834 | O | $CH_2CONHNMe_2$ |
| H-0835 | O | $CH_2CONHNHCO_2t\text{-}Bu$ |
| H-0836 | O | $CH_2CHCC1_2$ |
| H-0837 | O | $CH_2CO_2CH_2CH_2$(morpholin-4-yl) |

[Table 181]

| Compound Number | Y | R2 |
|---|---|---|
| H-0838 | O | $CH_2CO_2CH_2CH_2OMe$ |
| H-0839 | O | $CH_2CH_2NHCO_2tBu$ |
| H-0840 | O | $CH_2CH_2N(COCF_3)CO_2tBu$ |
| H-0841 | O | $CH_2CH_2NMe_2$ |
| H-0842 | O | $CH_2CONHn\text{-}Pen$ |
| H-0843 | O | $CH_2CONHn\text{-}Hex$ |
| H-0844 | O | $CH_2CONHCH_2CH_2OMe$ |
| H-0845 | O | $CH_2CH(Cl)CH_2SF_5$ |
| H-0846 | O | $CH_2CH{=}CHSF_5$ |

(continued)

| Compound Number | Y | R2 |
|---|---|---|
| H-0847 | O | $CH_2CO_2$(tetrahydrofuran-3-yl) |
| H-0848 | O | 1-($CO_2H$)-c-Pr |
| H-0849 | O | 1-($CO_2H$)-c-Bu |
| H-0850 | O | 1-($CO_2$Me)-c-Pr |
| H-0851 | O | 1-($CO_2$Me)-c-Bu |
| H-0852 | O | 1-($CO_2$Et)-c-Pr |
| H-0853 | O | 1-($CO_2$Et)-c-Bu |
| H-0854 | O | 1-($CO_2$nPr)-c-Pr |
| H-0855 | O | 1-($CO_2$nPr)-c-Bu |
| H-0856 | O | 1-($CO_2$iPr)-c-Pr |
| H-0857 | O | 1-($CO_2$iPr)-c-Bu |
| H-0858 | O | 1-($CO_2$cPr)-c-Pr |
| H-0859 | O | 1-($CO_2$cPr)-c-Bu |
| H-0860 | O | 1-($CO_2$cBu)-c-Pr |
| H-0861 | O | 1-($CO_2$cBu)-c-Bu |

[Table 182]

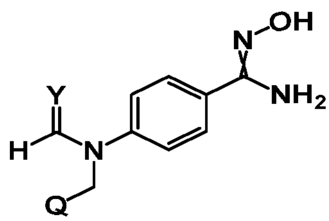

| Compound Number | Y | Q |
|---|---|---|
| I-0001 | O | naphthalen-1-yl |
| I-0002 | O | naphthalen-2-yl |
| I-0003 | O | quinolin-2-yl |
| I-0004 | O | quinolin-3-yl |
| I-0005 | O | quinolin-6-yl |
| I-0006 | O | quinolin-7-yl |
| I-0007 | O | 1 H-pyrrolo[2,3-b]pyridin-3-yl |
| I-0008 | O | benzo[d][1,3]dioxol-4-yl |
| I-0009 | O | benzo[d][1,3]dioxol-5-yl |
| I-0010 | O | benzo[d][1,3]dioxol-4-yl(2,2-$F_2$) |
| I-0011 | O | benzo[d][1,3]dioxol-5-yl(2,2-$F_2$) |
| I-0012 | O | pyridin-2-yl |

(continued)

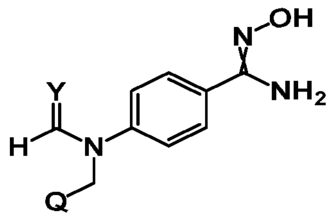

| Compound Number | Y | Q |
|---|---|---|
| I-0013 | O | $CH_2$pyridin-2-yl |
| I-0014 | O | pyridin-2-yl(3-F) |
| I-0015 | O | pyridin-2-yl(4-F) |
| I-0016 | O | pyridin-2-yl(5-F) |
| I-0017 | O | pyridin-2-yl(6-F) |
| I-0018 | O | pyridin-2-yl(3-Cl) |
| I-0019 | O | pyridin-2-yl(4-Cl) |
| I-0020 | O | pyridin-2-yl(5-Cl) |
| I-0021 | O | pyridin-2-yl(6-Cl) |
| I-0022 | O | pyridin-2-yl(3-Me) |
| I-0023 | O | pyridin-2-yl(4-Me) |
| I-0024 | O | pyridin-2-yl(5-Me) |
| I-0025 | O | pyridin-2-yl(6-Me) |
| I-0026 | O | pyridin-2-yl(3-OMe) |
| I-0027 | O | pyridin-2-yl(4-OMe) |
| I-0028 | O | pyridin-2-yl(5-OMe) |
| 1-0029 | O | pyridin-2-yl(6-OMe) |
| I-0030 | O | pyridin-2-yl(3-CN) |
| I-0031 | O | pyridin-2-yl(4-CN) |
| I-0032 | O | pyridin-2-yl(5-CN) |
| I-0033 | O | pyridin-2-yl(6-CN) |
| I-0034 | O | pyridin-2-yl(3-$CF_3$) |
| I-0035 | O | pyridin-2-yl(4-$CF_3$) |
| I-0036 | O | pyridin-2-yl(5-$CF_3$) |

[Table 183]

| Compound Number | Y | Q |
|---|---|---|
| I-0037 | O | pyridin-2-yl(6-$CF_3$) |
| I-0038 | O | pyridin-2-yl(3-$CO_2$Et) |
| I-0039 | O | pyridin-2-yl(4-$CO_2$Et) |
| I-0040 | O | pyridin-2-yl(5-$CO_2$Et) |

(continued)

| Compound Number | Y | Q |
|---|---|---|
| I-0041 | O | pyridin-2-yl(6-$CO_2$Et) |
| I-0042 | O | pyridin-3-yl |
| I-0043 | O | $CH_2$pyridin-3-yl |
| I-0044 | O | pyridin-3-yl(2-F) |
| I-0045 | O | pyridin-3-yl(4-F) |
| I-0046 | O | pyridin-3-yl(5-F) |
| I-0047 | O | pyridin-3-yl(6-F) |
| I-0048 | O | pyridin-3-yl(2-Cl) |
| I-0049 | O | pyridin-3-yl(4-Cl) |
| I-0050 | O | pyridin-3-yl(5-Cl) |
| I-0051 | O | pyridin-3-yl(6-Cl) |
| I-0052 | O | pyridin-3-yl(2-Me) |
| I-0053 | O | pyridin-3-yl(4-Me) |
| I-0054 | O | pyridin-3-yl(5-Me) |
| I-0055 | O | pyridin-3-yl(6-Me) |
| I-0056 | O | pyridin-3-yl(2-OMe) |
| I-0057 | O | pyridin-3-yl(4-OMe) |
| I-0058 | O | pyridin-3-yl(5-OMe) |
| I-0059 | O | pyridin-3-yl(6-OMe) |
| I-0060 | O | pyridin-3-yl(2-CN) |
| I-0061 | O | pyridin-3-yl(4-CN) |
| I-0062 | O | pyridin-3-yl(5-CN) |
| I-0063 | O | pyridin-3-yl(6-CN) |
| I-0064 | O | pyridin-3-yl(2-$CF_3$) |
| I-0065 | O | pyridin-3-yl(4-$CF_3$) |
| I-0066 | O | pyridin-3-yl(5-$CF_3$) |
| I-0067 | O | pyridin-3-yl(6-$CF_3$) |
| I-0068 | O | pyridin-3-yl(2-$CO_2$Et) |
| I-0069 | O | pyridin-3-yl(4-$CO_2$Et) |
| I-0070 | O | pyridin-3-yl(5-$CO_2$Et) |
| I-0071 | O | pyridin-3-yl(6-$CO_2$Et) |
| I-0072 | O | pyridin-4-yl |
| I-0073 | O | $CH_2$pyridin-4-yl |
| I-0074 | O | pyridin-4-yl(2-F) |
| I-0075 | O | pyridin-4-yl(3-F) |
| I-0076 | O | pyridin-4-yl(2-Cl) |

(continued)

| Compound Number | Y | Q |
|---|---|---|
| I-0077 | O | pyridin-4-yl(3-Cl) |
| I-0078 | O | pyridin-4-yl(2-Me) |

[Table 184]

| Compound Number | Y | Q |
|---|---|---|
| I-0079 | O | pyridin-4-yl(3-Me) |
| I-0080 | O | pyridin-4-yl(2-OMe) |
| I-0081 | O | pyridin-4-yl(3-OMe) |
| I-0082 | O | pyridin-4-yl(2-CN) |
| I-0083 | O | pyridin-4-yl(3-CN) |
| I-0084 | O | pyridin-4-yl(2-$CF_3$) |
| I-0085 | O | pyridin-4-yl(3-$CF_3$) |
| I-0086 | O | pyridin-4-yl(2-$CO_2$Et) |
| I-0087 | O | pyridin-4-yl(3-$CO_2$Et) |
| I-0088 | O | pyrimidin-2-yl |
| I-0089 | O | $CH_2$pyrimidin-2-yl |
| I-0090 | O | pyrimidin-2-yl(4-F) |
| I-0091 | O | pyrimidin-2-yl(5-F) |
| I-0092 | O | pyrimidin-2-yl(4-Cl) |
| I-0093 | O | pyrimidin-2-yl(5-Cl) |
| I-0094 | O | pyrimidin-2-yl(4-Me) |
| I-0095 | O | pyrimidin-2-yl(5-Me) |
| I-0096 | O | pyrimidin-2-yl(4-OMe) |
| I-0097 | O | pyrimidin-2-yl(5-OMe) |
| I-0098 | O | pyrimidin-2-yl(4-CN) |
| I-0099 | O | pyrimidin-2-yl(5-CN) |
| I-0100 | O | pyrimidin-2-yl(4-$CF_3$) |
| I-0101 | O | pyrimidin-2-yl(5-$CF_3$) |
| I-0102 | O | pyrimidin-2-yl(4-$CO_2$Et) |
| I-0103 | O | pyrimidin-2-yl(5-$CO_2$Et) |
| I-0104 | O | pyrimidin-4-yl |
| !-0105 | O | $CH_2$pyrimidin-4-yl |
| I-0106 | O | pyrimidin-4-yl(2-F) |
| I-0107 | O | pyrimidin-4-yl(5-F) |
| I-0108 | O | pyrimidin-4-yl(6-F) |
| I-0109 | O | pyrimidin-4-yl(2-Cl) |

(continued)

| Compound Number | Y | Q |
|---|---|---|
| I-0110 | O | pyrimidin-4-yl(5-Cl) |
| I-0111 | O | pyrimidin-4-yl(6-Cl) |
| I-0112 | O | pyrimidin-4-yl(2-Me) |
| I-0113 | O | pyrimidin-4-yl(5-Me) |
| I-0114 | O | pyrimidin-4-yl(6-Me) |
| I-0115 | O | pyrimidin-4-yl(2-OMe) |
| I-0116 | O | pyrimidin-4-yl(5-OMe) |
| I-0117 | O | pyrimidin-4-yl(6-OMe) |
| I-0118 | O | pyrimidin-4-yl(2-CN) |
| I-0119 | O | pyrimidin-4-yl(5-CN) |
| I-0120 | O | pyrimidin-4-yl(6-CN) |

[Table 185]

| Compound Number | Y | Q |
|---|---|---|
| I-0121 | O | pyrimidin-4-yl(2-$CF_3$) |
| I-0122 | O | pyrimidin-4-yl(5-$CF_3$) |
| I-0123 | O | pyrimidin-4-yl(6-$CF_3$) |
| I-0124 | O | pyrimidin-4-yl(2-$CO_2$Et) |
| I-0125 | O | pyrimidin-4-yl(5-$CO_2$Et) |
| I-0126 | O | pyrimidin-4-yl(6-$CO_2$Et) |
| I-0127 | O | pyrimidin-5-yl |
| I-0128 | O | $CH_2$pyrimidin-5-yl |
| I-0129 | O | pyrimidin-5-yl(2-F) |
| I-0130 | O | pyrimidin-5-yl(4-F) |
| I-0131 | O | pyrimidin-5-yl(2-Cl) |
| I-0132 | O | pyrimidin-5-yl(4-Cl) |
| I-0133 | O | pyrimidin-5-yl(2-Me) |
| I-0134 | O | pyrimidin-5-yl(4-Me) |
| I-0135 | O | pyrimidin-5-yl(2-OMe) |
| I-0136 | O | pyrimidin-5-yl(4-OMe) |
| I-0137 | O | pyrimidin-5-yl(2-CN) |
| I-0138 | O | pyrimidin-5-yl(4-CN) |
| I-0139 | O | pyrimidin-5-yl(2-$CF_3$) |
| I-0140 | O | pyrimidin-5-yl(4-$CF_3$) |
| I-0141 | O | pyrimidin-5-yl(2-$CO_2$Et) |
| I-0142 | O | pyrimidin-5-yl(4-$CO_2$Et) |

(continued)

| Compound Number | Y | Q |
|---|---|---|
| I-0143 | O | pyridazin-3-yl |
| I-0144 | O | $CH_2$pyridazin-3-yl |
| I-0145 | O | pyridazin-3-yl(4-F) |
| I-0146 | O | pyridazin-3-yl(5-F) |
| I-0147 | O | pyridazin-3-yl(6-F) |
| I-0148 | O | pyridazin-3-yl(4-Cl) |
| I-0149 | O | pyridazin-3-yl(5-Cl) |
| I-0150 | O | pyridazin-3-yl(6-Cl) |
| I-0151 | O | pyridazin-3-yl(4-Me) |
| I-0152 | O | pyridazin-3-yl(5-Me) |
| I-0153 | O | pyridazin-3-yl(6-Me) |
| I-0154 | O | pyridazin-3-yl(4-OMe) |
| I-0155 | O | pyridazin-3-yl(5-OMe) |
| I-0156 | O | pyridazin-3-yl(6-OMe) |
| I-0157 | O | pyridazin-3-yl(4-CN) |
| I-0158 | O | pyridazin-3-yl(5-CN) |
| I-0159 | O | pyridazin-3-yl(6-CN) |
| I-0160 | O | pyridazin-3-yl(4-$CF_3$) |
| I-0161 | O | pyridazin-3-yl(5-$CF_3$) |
| I-0162 | O | pyridazin-3-yl(6-$CF_3$) |

[Table 186]

| Compound Number | Y | Q |
|---|---|---|
| I-0163 | O | pyridazin-3-yl(4-$CO_2$Et) |
| I-0164 | O | pyridazin-3-yl(5-$CO_2$Et) |
| I-0165 | O | pyridazin-3-yl(6-$CO_2$Et) |
| I-0166 | O | pyridazin-4-yl |
| I-0167 | O | $CH_2$pyridazin-4-yl |
| I-0168 | O | pyridazin-4-yl(3-F) |
| I-0169 | O | pyridazin-4-yl(5-F) |
| I-0170 | O | pyridazin-4-yl(6-F) |
| I-0171 | O | pyridazin-4-yl(3-Cl) |
| I-0172 | O | pyridazin-4-yl(5-Cl) |
| I-0173 | O | pyridazin-4-yl(6-Cl) |
| I-0174 | O | pyridazin-4-yl(3-Me) |
| I-0175 | O | pyridazin-4-yl(5-Me) |
| I-0176 | O | pyridazin-4-yl(6-Me) |

(continued)

| Compound Number | Y | Q |
|---|---|---|
| I-0177 | O | pyridazin-4-yl(3-OMe) |
| I-0178 | O | pyridazin-4-yl(5-OMe) |
| I-0179 | O | pyridazin-4-yl(6-OMe) |
| I-0180 | O | pyridazin-4-yl(3-CN) |
| I-0181 | O | pyridazin-4-yl(5-CN) |
| I-0182 | O | pyridazin-4-yl(6-CN) |
| I-0183 | O | pyridazin-4-yl(3-$CF_3$) |
| I-0184 | O | pyridazin-4-yl(5-$CF_3$) |
| I-0185 | O | pyridazin-4-yl(6-$CF_3$) |
| I-0186 | O | pyridazin-4-yl(3-$CO_2$Et) |
| I-0187 | O | pyridazin-4-yl(5-$CO_2$Et) |
| I-0188 | O | pyridazin-4-yl(6-$CO_2$Et) |
| I-0189 | O | pyrazin-2-yl |
| I-0190 | O | $CH_2$pyrazin-2-yl |
| I-0191 | O | pyrazin-2-yl(3-F) |
| I-0192 | O | pyrazin-2-yl(5-F) |
| I-0193 | O | pyrazin-2-yl(6-F) |
| I-0194 | O | pyrazin-2-yl(3-Cl) |
| I-0195 | O | pyrazin-2-yl(5-Cl) |
| I-0196 | O | pyrazin-2-yl(6-Cl) |
| I-0197 | O | pyrazin-2-yl(3-Me) |
| I-0198 | O | pyrazin-2-yl(5-Me) |
| I-0199 | O | pyrazin-2-yl(6-Me) |
| I-0200 | O | pyrazin-2-yl(3-OMe) |
| I-0201 | O | pyrazin-2-yl(5-OMe) |
| I-0202 | O | pyrazin-2-yl(6-OMe) |
| I-0203 | O | pyrazin-2-yl(3-CN) |
| I-0204 | O | pyrazin-2-yl(5-CN) |

[Table 187]

| Compound Number | Y | Q |
|---|---|---|
| I-0205 | O | pyrazin-2-yl(6-CN) |
| I-0206 | O | pyrazin-2-yl(3-$CF_3$) |
| I-0207 | O | pyrazin-2-yl(5-$CF_3$) |
| I-0208 | O | pyrazin-2-yl(6-$CF_3$) |
| I-0209 | O | pyrazin-2-yl(3-$CO_2$Et) |

(continued)

| Compound Number | Y | Q |
|---|---|---|
| I-0210 | O | pyrazin-2-yl(5-$CO_2$Et) |
| I-0211 | O | pyrazin-2-yl(6-$CO_2$Et) |
| I-0212 | O | 1,3,5-triazin-2-yl |
| I-0213 | O | $CH_2$1,3,5-triazin-2-yl |
| I-0214 | O | thiophen-2-yl |
| I-0215 | O | $CH_2$thiophen-2-yl |
| I-0216 | O | thiophen-2-yl(3-F) |
| I-0217 | O | thiophen-2-yl(4-F) |
| I-0218 | O | thiophen-2-yl(5-F) |
| I-0219 | O | thiophen-2-yl(3-Cl) |
| I-0220 | O | thiophen-2-yl(4-Cl) |
| I-0221 | O | thiophen-2-yl(5-Cl) |
| I-0222 | O | thiophen-2-yl(3-Me) |
| I-0223 | O | thiophen-2-yl(4-Me) |
| I-0224 | O | thiophen-2-yl(5-Me) |
| I-0225 | O | thiophen-2-yl(3-OMe) |
| I-0226 | O | thiophen-2-yl(4-OMe) |
| I-0227 | O | thiophen-2-yl(5-OMe) |
| I-0228 | O | thiophen-2-yl(3-CN) |
| 1-0229 | O | thiophen-2-yl(4-CN) |
| I-0230 | O | thiophen-2-yl(5-CN) |
| I-0231 | O | thiophen-2-yl(3-$CF_3$) |
| I-0232 | O | thiophen-2-yl(4-$CF_3$) |
| I-0233 | O | thiophen-2-yl(5-$CF_3$) |
| I-0234 | O | thiophen-2-yl(3-$CO_2$Et) |
| I-0235 | O | thiophen-2-yl(4-$CO_2$Et) |
| I-0236 | O | thiophen-2-yl(5-$CO_2$Et) |
| I-0237 | O | thiophen-3-yl |
| I-0238 | O | $CH_2$thiophen-3-yl |
| I-0239 | O | thiophen-3-yl(2-F) |
| I-0240 | O | thiophen-3-yl(4-F) |
| I-0241 | O | thiophen-3-yl(5-F) |
| I-0242 | O | thiophen-3-yl(2-Cl) |
| I-0243 | O | thiophen-3-yl(4-Cl) |
| I-0244 | O | thiophen-3-yl(5-Cl) |
| I-0245 | O | thiophen-3-yl(2-Me) |

(continued)

| Compound Number | Y | Q |
|---|---|---|
| I-0246 | O | thiophen-3-yl(4-Me) |

[Table 188]

| Compound Number | Y | Q |
|---|---|---|
| I-0247 | O | thiophen-3-yl(5-Me) |
| I-0248 | O | thiophen-3-yl(2-OMe) |
| I-0249 | O | thiophen-3-yl(4-OMe) |
| I-0250 | O | thiophen-3-yl(5-OMe) |
| I-0251 | O | thiophen-3-yl(2-CN) |
| I-0252 | O | thiophen-3-yl(4-CN) |
| I-0253 | O | thiophen-3-yl(5-CN) |
| I-0254 | O | thiophen-3-yl(2-$CF_3$) |
| I-0255 | O | thiophen-3-yl(4-$CF_3$) |
| I-0256 | O | thiophen-3-yl(5-$CF_3$) |
| I-0257 | O | thiophen-3-yl(2-$CO_2$Et) |
| I-0258 | O | thiophen-3-yl(4-$CO_2$Et) |
| I-0259 | O | thiophen-3-yl(5-$CO_2$Et) |
| I-0260 | O | furan-2-yl |
| I-0261 | O | $CH_2$furan-2-yl |
| I-0262 | O | furan-2-yl(3-F) |
| I-0263 | O | furan-2-yl(4-F) |
| I-0264 | O | furan-2-yl(5-F) |
| I-0265 | O | furan-2-yl(3-Cl) |
| I-0266 | O | furan-2-yl(4-Cl) |
| I-0267 | O | furan-2-yl(5-Cl) |
| I-0268 | O | furan-2-yl(3-Me) |
| I-0269 | O | furan-2-yl(4-Me) |
| I-0270 | O | furan-2-yl(5-Me) |
| I-0271 | O | furan-2-yl(3-OMe) |
| I-0272 | O | furan-2-yl(4-OMe) |
| I-0273 | O | furan-2-yl(5-OMe) |
| I-0274 | O | furan-2-yl(3-CN) |
| I-0275 | O | furan-2-yl(4-CN) |
| I-0276 | O | furan-2-yl(5-CN) |
| I-0277 | O | furan-2-yl(3-$CF_3$) |
| I-0278 | O | furan-2-yl(4-$CF_3$) |

(continued)

| Compound Number | Y | Q |
|---|---|---|
| I-0279 | O | furan-2-yl(5-$CF_3$) |
| I-0280 | O | furan-2-yl(3-$CO_2$Et) |
| I-0281 | O | furan-2-yl(4-$CO_2$Et) |
| I-0282 | O | furan-2-yl(5-$CO_2$Et) |
| I-0283 | O | furan-3-yl |
| I-0284 | O | $CH_2$furan-3-yl |
| I-0285 | O | furan-3-yl(2-F) |
| I-0286 | O | furan-3-yl(4-F) |
| I-0287 | O | furan-3-yl(5-F) |
| I-0288 | O | furan-3-yl(2-Cl) |

[Table 189]

| Compound Number | Y | Q |
|---|---|---|
| I-0289 | O | furan-3-yl(4-Cl) |
| I-0290 | O | furan-3-yl(5-Cl) |
| I-0291 | O | furan-3-yl(2-Me) |
| I-0292 | O | furan-3-yl(4-Me) |
| I-0293 | O | furan-3-yl(5-Me) |
| I-0294 | O | furan-3-yl(2-OMe) |
| I-0295 | O | furan-3-yl(4-OMe) |
| I-0296 | O | furan-3-yl(5-OMe) |
| I-0297 | O | furan-3-yl(2-CN) |
| I-0298 | O | furan-3-yl(4-CN) |
| I-0299 | O | furan-3-yl(5-CN) |
| I-0300 | O | furan-3-yl(2-$CF_3$) |
| I-0301 | O | furan-3-yl(4-$CF_3$) |
| I-0302 | O | furan-3-yl(5-$CF_3$) |
| I-0303 | O | furan-3-yl(2-$CO_2$Et) |
| I-0304 | O | furan-3-yl(4-$CO_2$Et) |
| I-0305 | O | furan-3-yl(5-$CO_2$Et) |
| I-0306 | O | 1H-pyrrol-1-yl |
| I-0307 | O | $CH_2$1H-pyrrol-1-yl |
| I-0308 | O | 1H-pyrrol-2-yl |
| I-0309 | O | $CH_2$1H-pyrrol-2-yl |
| I-0310 | O | 1H-pyrrol-2-yl(1-Me) |
| I-0311 | O | $CH_2$1H-pyrrol-2-yl(1-Me) |

(continued)

| Compound Number | Y | Q |
|---|---|---|
| I-0312 | O | 1H-pyrrol-3-yl |
| I-0313 | O | $CH_2$1H-pyrrol-3-yl |
| I-0314 | O | 1H-pyrrol-3-yl(1-Me) |
| I-0315 | O | $CH_2$1H-pyrrol-3-yl(1-Me) |
| I-0316 | O | 1H-pyrazol-1-yl |
| I-0317 | O | $CH_2$1H-pyrazol-1-yl |
| I-0318 | O | 1H-pyrazol-1-yl(3-F) |
| I-0319 | O | 1H-pyrazol-1-yl(4-F) |
| I-0320 | O | 1H-pyrazol-1-yl(5-F) |
| I-0321 | O | 1H-pyrazol-1-yl(3-Cl) |
| I-0322 | O | 1H-pyrazol-1-yl(4-Cl) |
| I-0323 | O | 1H-pyrazol-1-yl(5-Cl) |
| I-0324 | O | 1H-pyrazol-1-yl(3-Me) |
| I-0325 | O | 1H-pyrazol-1-yl(4-Me) |
| I-0326 | O | 1H-pyrazol-1-yl(5-Me) |
| I-0327 | O | 1H-pyrazol-1-yl(3-OMe) |
| I-0328 | O | 1H-pyrazol-1-yl(4-OMe) |
| I-0329 | O | 1H-pyrazol-1-yl(5-OMe) |
| I-0330 | O | 1H-pyrazol-1-yl(3-CN) |

[Table 190]

| Compound Number | Y | Q |
|---|---|---|
| I-0331 | O | 1H-pyrazol-1-yl(4-CN) |
| I-0332 | O | 1H-pyrazol-1-yl(5-CN) |
| I-0333 | O | 1H-pyrazol-1-yl(3-$CF_3$) |
| I-0334 | O | 1H-pyrazol-1-yl(4-$CF_3$) |
| I-0335 | O | 1H-pyrazol-1-yl(5-$CF_3$) |
| I-0336 | O | 1H-pyrazol-1-yl(3-$CO_2$Et) |
| I-0337 | O | 1H-pyrazol-1-yl(4-$CO_2$Et) |
| I-0338 | O | 1H-pyrazol-1-yl(5-$CO_2$Et) |
| I-0339 | O | 1H-pyrazol-3-yl |
| I-0340 | O | $CH_2$1H-pyrazol-3-yl |
| I-0341 | O | 1H-pyrazol-3-yl(1-Me) |
| I-0342 | O | $CH_2$1H-pyrazol-3-yl(1-Me) |
| I-0343 | O | 1H-pyrazol-3-yl(4-F,1-Me) |
| I-0344 | O | 1H-pyrazol-3-yl(5-F,1-Me) |

(continued)

| Compound Number | Y | Q |
|---|---|---|
| I-0345 | O | 1H-pyrazol-3-yl(4-Cl,1-Me) |
| I-0346 | O | 1H-pyrazol-3-yl(5-Cl,1-Me) |
| I-0347 | O | 1H-pyrazol-3-yl(1,4-$Me_2$) |
| I-0348 | O | 1H-pyrazol-3-yl(1,5-$Me_2$) |
| I-0349 | O | 1H-pyrazol-3-yl(4-OMe,1-Me) |
| I-0350 | O | 1H-pyrazol-3-yl(5-OMe,1-Me) |
| I-0351 | O | 1H-pyrazol-3-yl(4-CN,1-Me) |
| I-0352 | O | 1H-pyrazol-3-yl(5-CN,1-Me) |
| I-0353 | O | 1H-pyrazol-3-yl(1-Me,4-$CF_3$) |
| I-0354 | O | 1H-pyrazol-3-yl(1-Me,5-$CF_3$) |
| 1-0355 | O | 1H-pyrazol-3-yl(1-Me,4-$CO_2$Et) |
| I-0356 | O | 1H-pyrazol-3-yl(1-Me,5-$CO_2$Et) |
| I-0357 | O | 1H-pyrazol-4-yl |
| I-0358 | O | $CH_2$1H-pyrazol-4-yl |
| I-0359 | O | 1H-pyrazol-4-yl(1-Me) |
| I-0360 | O | $CH_2$1H-pyrazol-4-yl(1-Me) |
| I-0361 | O | 1H-pyrazol-4-yl(3-F,1-Me) |
| I-0362 | O | 1H-pyrazol-4-yl(5-F,1-Me) |
| I-0363 | O | 1H-pyrazol-4-yl(3-Cl,1-Me) |
| I-0364 | O | 1H-pyrazol-4-yl(5-Cl,1-Me) |
| I-0365 | O | 1H-pyrazol-4-yl(1,3-$Me_2$) |
| I-0366 | O | 1H-pyrazol-4-yl(1,5-$Me_2$) |
| I-0367 | O | 1H-pyrazol-4-yl(3-OMe,1-Me) |
| I-0368 | O | 1H-pyrazol-4-yl(5-OMe,1-Me) |
| I-0369 | O | 1H-pyrazol-4-yl(3-CN,1-Me) |
| I-0370 | O | 1H-pyrazol-4-yl(5-CN,1-Me) |
| I-0371 | O | 1H-pyrazol-4-yl(1-Me,3-$CF_3$) |
| I-0372 | O | 1H-pyrazol-4-yl(1-Me,5-$CF_3$) |

[Table 191]

| Compound Number | Y | Q |
|---|---|---|
| I-0373 | O | 1H-pyrazol-4-yl(1-Me,3-$CO_2$Et) |
| I-0374 | O | 1H-pyrazol-4-yl(1-Me,5-$CO_2$Et) |
| I-0375 | O | 1H-pyrazol-5-yl |
| I-0376 | O | $CH_2$1H-pyrazol-5-yl |
| I-0377 | O | 1H-pyrazol-5-yl(1-Me) |

(continued)

| Compound Number | Y | Q |
|---|---|---|
| I-0378 | O | $CH_2$1H-pyrazol-5-yl(1-Me) |
| I-0379 | O | 1H-pyrazol-5-yl(3-F,1-Me) |
| I-0380 | O | 1H-pyrazol-5-yl(4-F,1-Me) |
| I-0381 | O | 1H-pyrazol-5-yl(3-Cl,1-Me) |
| I-0382 | O | 1H-pyrazol-5-yl(4-Cl,1-Me) |
| I-0383 | O | 1H-pyrazol-5-yl(1,3-$Me_2$) |
| I-0384 | O | 1H-pyrazol-5-yl(1,4-$Me_2$) |
| I-0385 | O | 1H-pyrazol-5-yl(3-OMe,1-Me) |
| I-0386 | O | 1H-pyrazol-5-yl(4-OMe,1-Me) |
| I-0387 | O | 1H-pyrazol-5-yl(3-CN,1-Me) |
| I-0388 | O | 1H-pyrazol-5-yl(4-CN,1-Me) |
| I-0389 | O | 1H-pyrazol-5-yl(1-Me,3-$CF_3$) |
| I-0390 | O | 1H-pyrazol-5-yl(1-Me,4-$CF_3$) |
| I-0391 | O | 1H-pyrazol-5-yl(1-Me,3-$CO_2$Et) |
| I-0392 | O | 1H-pyrazol-5-yl(1-Me,4-$CO_2$Et) |
| I-0393 | O | 1H-imidazol-1-yl |
| I-0394 | O | $CH_2$1H-imidazol-1-yl |
| I-0395 | O | 1H-imidazol-1-yl(2-F) |
| I-0396 | O | 1H-imidazol-1-yl(4-F) |
| I-0397 | O | 1H-imidazol-1-yl(5-F) |
| I-0398 | O | 1H-imidazol-1-yl(2-Cl) |
| I-0399 | O | 1H-imidazol-1-yl(4-Cl) |
| I-0400 | O | 1H-imidazol-1-yl(5-Cl) |
| I-0401 | O | 1H-imidazol-1-yl(2-Me) |
| I-0402 | O | 1H-imidazol-1-yl(4-Me) |
| I-0403 | O | 1H-imidazol-1-yl(5-Me) |
| I-0404 | O | 1H-imidazol-1-yl(2-OMe) |
| I-0405 | O | 1H-imidazol-1-yl(4-OMe) |
| I-0406 | O | 1H-imidazol-1-yl(5-OMe) |
| I-0407 | O | 1H-imidazol-1-yl(2-CN) |
| I-0408 | O | 1H-imidazol-1-yl(4-CN) |
| I-0409 | O | 1H-imidazol-1-yl(5-CN) |
| I-0410 | O | 1H-imidazol-1-yl(2-$CF_3$) |
| I-0411 | O | 1H-imidazol-1-yl(4-$CF_3$) |
| I-0412 | O | 1H-imidazol-1-yl(5-$CF_3$) |
| I-0413 | O | 1H-imidazol-1-yl(2-$CO_2$Et) |

(continued)

| Compound Number | Y | Q |
|---|---|---|
| I-0414 | O | 1H-imidazol-1-yl(4-$CO_2$Et) |

[Table 192]

| Compound Number | Y | Q |
|---|---|---|
| I-0415 | O | 1H-imidazol-1-yl(5-$CO_2$Et) |
| I-0416 | O | 1H-imidazol-2-yl |
| I-0417 | O | $CH_2$1H-imidazol-2-yl |
| I-0418 | O | 1H-imidazol-2-yl(1-Me) |
| I-0419 | O | $CH_2$1H-imidazol-2-yl(1-Me) |
| I-0420 | O | 1H-imidazol-2-yl(4-F,1-Me) |
| I-0421 | O | 1H-imidazol-2-yl(5-F,1-Me) |
| I-0422 | O | 1H-imidazol-2-yl(4-Cl,1-Me) |
| I-0423 | O | 1H-imidazol-2-yl(5-Cl,1-Me) |
| I-0424 | O | 1H-imidazol-2-yl(1,4-$Me_2$) |
| I-0425 | O | 1H-imidazol-2-yl(1,5-$Me_2$) |
| I-0426 | O | 1H-imidazol-2-yl(4-OMe,1-Me) |
| I-0427 | O | 1H-imidazol-2-yl(5-OMe,1-Me) |
| I-0428 | O | 1H-imidazol-2-yl(4-CN,1-Me) |
| I-0429 | O | 1H-imidazol-2-yl(5-CN,1-Me) |
| I-0430 | O | 1H-imidazol-2-yl(1-Me,4-$CF_3$) |
| I-0431 | O | 1H-imidazol-2-yl(1-Me,5-$CF_3$) |
| I-0432 | O | 1H-imidazol-2-yl(1-Me,4-$CO_2$Et) |
| I-0433 | O | 1H-imidazol-2-yl(1-Me,5-$CO_2$Et) |
| I-0434 | O | 1H-imidazol-4-yl |
| I-0435 | O | $CH_2$1H-imidazol-4-yl |
| I-0436 | O | 1H-imidazol-4-yl(1-Me) |
| I-0437 | O | $CH_2$1H-imidazol-4-yl(1-Me) |
| I-0438 | O | 1H-imidazol-4-yl(2-F,1-Me) |
| I-0439 | O | 1H-imidazol-4-yl(5-F,1-Me) |
| 1-0440 | O | 1H-imidazol-4-yl(2-Cl,1-Me) |
| I-0441 | O | 1H-imidazol-4-yl(5-Cl,1-Me) |
| I-0442 | O | 1H-imidazol-4-yl(1,2-$Me_2$) |
| I-0443 | O | 1H-imidazol-4-yl(1,5-$Me_2$) |
| I-0444 | O | 1H-imidazol-4-yl(2-OMe,1-Me) |
| I-0445 | O | 1H-imidazol-4-yl(5-OMe,1-Me) |
| I-0446 | O | 1H-imidazol-4-yl(2-CN,1-Me) |

(continued)

| Compound Number | Y | Q |
|---|---|---|
| I-0447 | O | 1H-imidazol-4-yl(5-CN,1-Me) |
| I-0448 | O | 1H-imidazol-4-yl(1-Me,2-$CF_3$) |
| I-0449 | O | 1H-imidazol-4-yl(1-Me,5-$CF_3$) |
| I-0450 | O | 1H-imidazol-4-yl(1-Me,2-$CO_2$Et) |
| I-0451 | O | 1H-imidazol-4-yl(1-Me,5-$CO_2$Et) |
| I-0452 | O | 1H-imidazol-5-yl |
| !-0453 | O | $CH_2$1H-imidazol-5-yl |
| I-0454 | O | 1H-imidazol-5-yl(1-Me) |
| I-0455 | O | $CH_2$1H-imidazol-5-yl(1-Me) |
| 1-0456 | O | 1H-imidazol-5-yl(2-F,1-Me) |

[Table 193]

| Compound Number | Y | Q |
|---|---|---|
| I-0457 | O | 1H-imidazol-5-yl(4-F,1-Me) |
| I-0458 | O | 1H-imidazol-5-yl(2-Cl,1-Me) |
| I-0459 | O | 1H-imidazol-5-yl(4-Cl,1-Me) |
| I-0460 | O | 1H-imidazol-5-yl(1,2-$Me_2$) |
| I-0461 | O | 1H-imidazol-5-yl(1,4-$Me_2$) |
| I-0462 | O | 1H-imidazol-5-yl(2-OMe,1-Me) |
| I-0463 | O | 1H-imidazol-5-yl(4-OMe,1-Me) |
| I-0464 | O | 1H-imidazol-5-yl(2-CN,1-Me) |
| I-0465 | O | 1H-imidazol-5-yl(4-CN,1-Me) |
| I-0466 | O | 1H-imidazol-5-yl(1-Me,2-$CF_3$) |
| I-0467 | O | 1H-imidazol-5-yl(1-Me,4-$CF_3$) |
| I-0468 | O | 1H-imidazol-5-yl(1-Me,2-$CO_2$Et) |
| I-0469 | O | 1H-imidazol-5-yl(1-Me,4-$CO_2$Et) |
| I-0470 | O | 1H-1,2,4-triazol-1-yl |
| I-0471 | O | $CH_2$1H-1,2,4-triazol-1-yl |
| I-0472 | O | 1H-1,2,4-triazol-1-yl(3-F) |
| I-0473 | O | 1H-1,2,4-triazol-1-yl(5-F) |
| I-0474 | O | 1H-1,2,4-triazol-1-yl(3-Cl) |
| I-0475 | O | 1H-1,2,4-triazol-1-yl(5-Cl) |
| I-0476 | O | 1H-1,2,4-triazol-1-yl(3-Me) |
| I-0477 | O | 1H-1,2,4-triazol-1-yl(5-Me) |
| I-0478 | O | 1H-1,2,4-triazol-1-yl(3-OMe) |
| I-0479 | O | 1H-1,2,4-triazol-1-yl(5-OMe) |

(continued)

| Compound Number | Y | Q |
|---|---|---|
| I-0480 | O | 1H-1,2,4-triazol-1-yl(3-CN) |
| I-0481 | O | 1H-1,2,4-triazol-1-yl(5-CN) |
| I-0482 | O | 1H-1,2,4-triazol-1-yl(3-$CF_3$) |
| I-0483 | O | 1H-1,2,4-triazol-1-yl(5-$CF_3$) |
| I-0484 | O | 1H-1,2,4-triazol-1-yl(3-$CO_2$Et) |
| I-0485 | O | 1H-1,2,4-triazol-1-yl(5-$CO_2$Et) |
| I-0486 | O | 1H-1,2,4-triazol-3-yl |
| I-0487 | O | $CH_2$1H-1,2,4-triazol-3-yl |
| I-0488 | O | 1H-1,2,4-triazol-3-yl(1-Me) |
| I-0489 | O | $CH_2$1H-1,2,4-triazol-3-yl(1-Me) |
| I-0490 | O | 1H-1,2,4-triazol-3-yl(5-F,1-Me) |
| I-0491 | O | 1H-1,2,4-triazol-3-yl(5-Cl,1-Me) |
| I-0492 | O | 1H-1,2,4-triazol-3-yl(1,5-$Me_2$) |
| I-0493 | O | 1H-1,2,4-triazol-3-yl(5-OMe,1-Me) |
| I-0494 | O | 1H-1,2,4-triazol-3-yl(5-CN,1-Me) |
| I-0495 | O | 1H-1,2,4-triazol-3-yl(1-Me,5-$CF_3$) |
| I-0496 | O | 1H-1,2,4-triazol-3-yl(1-Me,5-$CO_2$Et) |
| I-0497 | O | 4H-1,2,4-triazol-4-yl |
| I-0498 | O | $CH_2$4H-1,2,4-triazol-4-yl |

[Table 194]

| Compound Number | Y | Q |
|---|---|---|
| I-0499 | O | 4H-1,2,4-triazol-4-yl(3-F) |
| I-0500 | O | 4H-1,2,4-triazol-4-yl(3-Cl) |
| I-0501 | O | 4H-1,2,4-triazol-4-yl(3-Me) |
| I-0502 | O | 4H-1,2,4-triazol-4-yl(3-OMe) |
| I-0503 | O | 4H-1,2,4-triazol-4-yl(3-CN) |
| I-0504 | O | 4H-1,2,4-triazol-4-yl(3-$CF_3$) |
| I-0505 | O | 4H-1,2,4-triazol-4-yl(3-$CO_2$Et) |
| I-0506 | O | 1H-1,2,4-triazol-5-yl |
| I-0507 | O | $CH_2$1H-1,2,4-triazol-5-yl |
| 1-0508 | O | 1H-1,2,4-triazol-5-yl(1-Me) |
| I-0509 | O | $CH_2$1H-1,2,4-triazol-5-yl(1-Me) |
| I-0510 | O | 1H-1,2,4-triazol-5-yl(3-F,1-Me) |
| I-0511 | O | 1H-1,2,4-triazol-5-yl(3-Cl,1-Me) |
| I-0512 | O | 1H-1,2,4-triazol-5-yl(1,3-$Me_2$) |

(continued)

| Compound Number | Y | Q |
|---|---|---|
| I-0513 | O | 1H-1,2,4-triazol-5-yl(3-OMe,1-Me) |
| I-0514 | O | 1H-1,2,4-triazol-5-yl(3-CN,1-Me) |
| I-0515 | O | 1H-1,2,4-triazol-5-yl(1-Me,3-$CF_3$) |
| I-0516 | O | 1H-1,2,4-triazol-5-yl(1-Me,3-$CO_2$Et) |
| I-0517 | O | 1H-1,2,3-triazol-1-yl |
| I-0518 | O | $CH_2$1H-1,2,3-triazol-1-yl |
| I-0519 | O | 1H-1,2,3-triazol-1-yl(4-Me) |
| I-0520 | O | 1H-1,2,3-triazol-1-yl(5-Me) |
| 1-0521 | O | 1H-1,2,3-triazol-1-yl(4-$CO_2$Et) |
| I-0522 | O | 1H-1,2,3-triazol-1-yl(5-$CO_2$Et) |
| I-0523 | O | 2H-1,2,3-triazol-2-yl |
| I-0524 | O | $CH_2$2H-1,2,3-triazol-2-yl |
| I-0525 | O | 1H-tetrazol-1-yl |
| I-0526 | O | $CH_2$1H-tetrazol-1-yl |
| I-0527 | O | 2H-tetrazol-2-yl |
| I-0528 | O | $CH_2$2H-tetrazol-2-yl |
| I-0529 | O | 1H-tetrazol-5-yl |
| I-0530 | O | $CH_2$1H-tetrazol-5-yl |
| I-0531 | O | 1H-tetrazol-5-yl(1-Me) |
| I-0532 | O | $CH_2$1H-tetrazol-5-yl(1-Me) |
| I-0533 | O | thiazol-2-yl |
| I-0534 | O | $CH_2$thiazol-2-yl |
| I-0535 | O | thiazol-2-yl(4-F) |
| I-0536 | O | thiazol-2-yl(5-F) |
| I-0537 | O | thiazol-2-yl(4-Cl) |
| I-0538 | O | thiazol-2-yl(5-Cl) |
| I-0539 | O | thiazol-2-yl(4-Me) |
| I-0540 | O | thiazol-2-yl(5-Me) |

[Table 195]

| Compound Number | Y | Q |
|---|---|---|
| I-0541 | O | thiazol-2-yl(4-OMe) |
| I-0542 | O | thiazol-2-yl(5-OMe) |
| I-0543 | O | thiazol-2-yl(4-CN) |
| I-0544 | O | thiazol-2-yl(5-CN) |
| I-0545 | O | thiazol-2-yl(4-$CF_3$) |

(continued)

| Compound Number | Y | Q |
|---|---|---|
| I-0546 | O | thiazol-2-yl(5-$CF_3$) |
| I-0547 | O | thiazol-2-yl(4-$CO_2$Et) |
| I-0548 | O | thiazol-2-yl(5-$CO_2$Et) |
| I-0549 | O | thiazol-4-yl |
| I-0550 | O | $CH_2$thiazol-4-yl |
| I-0551 | O | thiazol-4-yl(2-F) |
| I-0552 | O | thiazol-4-yl(5-F) |
| I-0553 | O | thiazol-4-yl(2-Cl) |
| I-0554 | O | thiazol-4-yl(5-Cl) |
| I-0555 | O | thiazol-4-yl(2-Me) |
| I-0556 | O | thiazol-4-yl(5-Me) |
| I-0557 | O | thiazol-4-yl(2-OMe) |
| I-0558 | O | thiazol-4-yl(5-OMe) |
| I-0559 | O | thiazol-4-yl(2-CN) |
| I-0560 | O | thiazol-4-yl(5-CN) |
| I-0561 | O | thiazol-4-yl(2-$CF_3$) |
| I-0562 | O | thiazol-4-yl(5-$CF_3$) |
| I-0563 | O | thiazol-4-yl(2-$CO_2$Et) |
| I-0564 | O | thiazol-4-yl(5-$CO_2$Et) |
| I-0565 | O | thiazol-5-yl |
| I-0566 | O | $CH_2$thiazol-5-yl |
| I-0567 | O | thiazol-5-yl(2-F) |
| I-0568 | O | thiazol-5-yl(4-F) |
| I-0569 | O | thiazol-5-yl(2-Cl) |
| I-0570 | O | thiazol-5-yl(4-Cl) |
| I-0571 | O | thiazol-5-yl(2-Me) |
| I-0572 | O | thiazol-5-yl(4-Me) |
| I-0573 | O | thiazol-5-yl(2-OMe) |
| I-0574 | O | thiazol-5-yl(4-OMe) |
| I-0575 | O | thiazol-5-yl(2-CN) |
| I-0576 | O | thiazol-5-yl(4-CN) |
| I-0577 | O | thiazol-5-yl(2-$CF_3$) |
| I-0578 | O | thiazol-5-yl(4-$CF_3$) |
| I-0579 | O | thiazol-5-yl(2-$CO_2$Et) |
| I-0580 | O | thiazol-5-yl(4-$CO_2$Et) |
| I-0581 | O | isothiazol-3-yl |

(continued)

| Compound Number | Y | Q |
|---|---|---|
| I-0582 | O | $CH_2$isothiazol-3-yl |

[Table 196]

| Compound Number | Y | Q |
|---|---|---|
| I-0583 | O | isothiazol-3-yl(4-F) |
| I-0584 | O | isothiazol-3-yl(5-F) |
| I-0585 | O | isothiazol-3-yl(4-Cl) |
| I-0586 | O | isothiazol-3-yl(5-Cl) |
| I-0587 | O | isothiazol-3-yl(4-Me) |
| I-0588 | O | isothiazol-3-yl(5-Me) |
| I-0589 | O | isothiazol-3-yl(4-OMe) |
| I-0590 | O | isothiazol-3-yl(5-OMe) |
| I-0591 | O | isothiazol-3-yl(4-CN) |
| I-0592 | O | isothiazol-3-yl(5-CN) |
| I-0593 | O | isothiazol-3-yl(4-$CF_3$) |
| I-0594 | O | isothiazol-3-yl(5-$CF_3$) |
| I-0595 | O | isothiazol-3-yl(4-$CO_2$Et) |
| I-0596 | O | isothiazol-3-yl(5-$CO_2$Et) |
| I-0597 | O | isothiazol-4-yl |
| I-0598 | O | $CH_2$isothiazol-4-yl |
| I-0599 | O | isothiazol-4-yl(3-F) |
| I-0600 | O | isothiazol-4-yl(5-F) |
| I-0601 | O | isothiazol-4-yl(3-Cl) |
| I-0602 | O | isothiazol-4-yl(5-Cl) |
| I-0603 | O | isothiazol-4-yl(3-Me) |
| I-0604 | O | isothiazol-4-yl(5-Me) |
| I-0605 | O | isothiazol-4-yl(3-OMe) |
| I-0606 | O | isothiazol-4-yl(5-OMe) |
| I-0607 | O | isothiazol-4-yl(3-CN) |
| I-0608 | O | isothiazol-4-yl(5-CN) |
| I-0609 | O | isothiazol-4-yl(3-$CF_3$) |
| I-0610 | O | isothiazol-4-yl(5-$CF_3$) |
| I-0611 | O | isothiazol-4-yl(3-$CO_2$Et) |
| I-0612 | O | isothiazol-4-yl(5-$CO_2$Et) |
| I-0613 | O | isothiazol-5-yl |
| I-0614 | O | $CH_2$isothiazol-5-yl |

(continued)

| Compound Number | Y | Q |
|---|---|---|
| I-0615 | O | isothiazol-5-yl(3-F) |
| I-0616 | O | isothiazol-5-yl(4-F) |
| I-0617 | O | isothiazol-5-yl(3-Cl) |
| I-0618 | O | isothiazol-5-yl(4-Cl) |
| I-0619 | O | isothiazol-5-yl(3,4-$Cl_2$) |
| I-0620 | O | isothiazol-5-yl(3-Me) |
| I-0621 | O | isothiazol-5-yl(4-Me) |
| I-0622 | O | isothiazol-5-yl(3-OMe) |
| I-0623 | O | isothiazol-5-yl(4-OMe) |
| I-0624 | O | isothiazol-5-yl(3-CN) |

[Table 197]

| Compound Number | Y | Q |
|---|---|---|
| I-0625 | O | isothiazol-5-yl(4-CN) |
| I-0626 | O | isothiazol-5-yl(3-$CF_3$) |
| I-0627 | O | isothiazol-5-yl(4-$CF_3$) |
| I-0628 | O | isothiazol-5-yl(3-$CO_2$Et) |
| I-0629 | O | isothiazol-5-yl(4-$CO_2$Et) |
| I-0630 | O | oxazol-2-yl |
| I-0631 | O | $CH_2$oxazol-2-yl |
| I-0632 | O | oxazol-2-yl(4-F) |
| I-0633 | O | oxazol-2-yl(5-F) |
| I-0634 | O | oxazol-2-yl(4-Cl) |
| I-0635 | O | oxazol-2-yl(5-Cl) |
| I-0636 | O | oxazol-2-yl(4-Me) |
| I-0637 | O | oxazol-2-yl(5-Me) |
| I-0638 | O | oxazol-2-yl(4-OMe) |
| I-0639 | O | oxazol-2-yl(5-OMe) |
| I-0640 | O | oxazol-2-yl(4-CN) |
| I-0641 | O | oxazol-2-yl(5-CN) |
| I-0642 | O | oxazol-2-yl(4-$CF_3$) |
| I-0643 | O | oxazol-2-yl(5-$CF_3$) |
| I-0644 | O | oxazol-2-yl(4-$CO_2$Et) |
| I-0645 | O | oxazol-2-yl(5-$CO_2$Et) |
| I-0646 | O | oxazol-4-yl |
| I-0647 | O | $CH_2$oxazol-4-yl |

(continued)

| Compound Number | Y | Q |
|---|---|---|
| I-0648 | O | oxazol-4-yl(2-F) |
| I-0649 | O | oxazol-4-yl(5-F) |
| I-0650 | O | oxazol-4-yl(2-Cl) |
| I-0651 | O | oxazol-4-yl(5-Cl) |
| I-0652 | O | oxazol-4-yl(2-Me) |
| I-0653 | O | oxazol-4-yl(5-Me) |
| I-0654 | O | oxazol-4-yl(2-OMe) |
| I-0655 | O | oxazol-4-yl(5-OMe) |
| I-0656 | O | oxazol-4-yl(2-CN) |
| I-0657 | O | oxazol-4-yl(5-CN) |
| I-0658 | O | oxazol-4-yl(2-$CF_3$) |
| I-0659 | O | oxazol-4-yl(5-$CF_3$) |
| I-0660 | O | oxazol-4-yl(2-$CO_2$Et) |
| I-0661 | O | oxazol-4-yl(5-$CO_2$Et) |
| I-0662 | O | oxazol-5-yl |
| I-0663 | O | $CH_2$oxazol-5-yl |
| I-0664 | O | oxazol-5-yl(2-F) |
| I-0665 | O | oxazol-5-yl(4-F) |
| I-0666 | O | oxazol-5-yl(2-Cl) |

[Table 198]

| Compound Number | Y | Q |
|---|---|---|
| I-0667 | O | oxazol-5-yl(4-Cl) |
| I-0668 | O | oxazol-5-yl(2-Me) |
| I-0669 | O | oxazol-5-yl(4-Me) |
| I-0670 | O | oxazol-5-yl(2-OMe) |
| I-0671 | O | oxazol-5-yl(4-OMe) |
| I-0672 | O | oxazol-5-yl(2-CN) |
| I-0673 | O | oxazol-5-yl(4-CN) |
| I-0674 | O | oxazol-5-yl(2-$CF_3$) |
| I-0675 | O | oxazol-5-yl(4-$CF_3$) |
| I-0676 | O | oxazol-5-yl(2-$CO_2$Et) |
| I-0677 | O | oxazol-5-yl(4-$CO_2$Et) |
| I-0678 | O | isoxazol-3-yl |
| I-0679 | O | $CH_2$isoxazol-3-yl |
| I-0680 | O | isoxazol-3-yl(4-F) |

(continued)

| Compound Number | Y | Q |
|---|---|---|
| I-0681 | O | isoxazol-3-yl(5-F) |
| I-0682 | O | isoxazol-3-yl(4-Cl) |
| I-0683 | O | isoxazol-3-yl(5-Cl) |
| I-0684 | O | isoxazol-3-yl(4-Me) |
| I-0685 | O | isoxazol-3-yl(5-Me) |
| I-0686 | O | isoxazol-3-yl(4-OMe) |
| I-0687 | O | isoxazol-3-yl(5-OMe) |
| I-0688 | O | isoxazol-3-yl(4-CN) |
| I-0689 | O | isoxazol-3-yl(5-CN) |
| I-0690 | O | isoxazol-3-yl(4-$CF_3$) |
| I-0691 | O | isoxazol-3-yl(5-$CF_3$) |
| I-0692 | O | isoxazol-3-yl(4-$CO_2$Et) |
| I-0693 | O | isoxazol-3-yl(5-$CO_2$Et) |
| I-0694 | O | isoxazol-4-yl |
| I-0695 | O | $CH_2$isoxazol-4-yl |
| I-0696 | O | isoxazol-4-yl(3-F) |
| I-0697 | O | isoxazol-4-yl(5-F) |
| I-0698 | O | isoxazol-4-yl(3-Cl) |
| I-0699 | O | isoxazol-4-yl(5-Cl) |
| I-0700 | O | isoxazol-4-yl(3-Me) |
| I-0701 | O | isoxazol-4-yl(5-Me) |
| I-0702 | O | isoxazol-4-yl(3-OMe) |
| I-0703 | O | isoxazol-4-yl(5-OMe) |
| I-0704 | O | isoxazol-4-yl(3-CN) |
| I-0705 | O | isoxazol-4-yl(5-CN) |
| I-0706 | O | isoxazol-4-yl(3-$CF_3$) |
| I-0707 | O | isoxazol-4-yl(5-$CF_3$) |
| I-0708 | O | isoxazol-4-yl(3-$CO_2$Et) |

[Table 199]

| Compound Number | Y | Q |
|---|---|---|
| I-0709 | O | isoxazol-4-yl(5-$CO_2$Et) |
| I-0710 | O | isoxazol-5-yl |
| I-0711 | O | $CH_2$isoxazol-5-yl |
| I-0712 | O | isoxazol-5-yl(3-F) |
| I-0713 | O | isoxazol-5-yl(4-F) |

(continued)

| Compound Number | Y | Q |
|---|---|---|
| I-0714 | O | isoxazol-5-yl(3-Cl) |
| I-0715 | O | isoxazol-5-yl(4-Cl) |
| I-0716 | O | isoxazol-5-yl(3-Me) |
| I-0717 | O | isoxazol-5-yl(4-Me) |
| I-0718 | O | isoxazol-5-yl(3-OMe) |
| I-0719 | O | isoxazol-5-yl(4-OMe) |
| I-0720 | O | isoxazol-5-yl(3-CN) |
| I-0721 | O | isoxazol-5-yl(4-CN) |
| I-0722 | O | isoxazol-5-yl(3-$CF_3$) |
| I-0723 | O | isoxazol-5-yl(4-$CF_3$) |
| I-0724 | O | isoxazol-5-yl(3-$CO_2$Et) |
| I-0725 | O | isoxazol-5-yl(4-$CO_2$Et) |
| I-0726 | O | 1,2,3-oxadiazol-4-yl |
| I-0727 | O | $CH_2$1,2,3-oxadiazol-4-yl |
| I-0728 | O | 1,2,3-oxadiazol-4-yl(5-F) |
| I-0729 | O | 1,2,3-oxadiazol-4-yl(5-Cl) |
| I-0730 | O | 1,2,3-oxadiazol-4-yl(5-Me) |
| I-0731 | O | 1,2,3-oxadiazol-4-yl(5-OMe) |
| I-0732 | O | 1,2,3-oxadiazol-4-yl(5-CN) |
| I-0733 | O | 1,2,3-oxadiazol-4-yl(5-$CF_3$) |
| I-0734 | O | 1,2,3-oxadiazol-4-yl(5-$CO_2$Et) |
| I-0735 | O | 1,2,3-oxadiazol-5-yl |
| I-0736 | O | $CH_2$1,2,3-oxadiazol-5-yl |
| I-0737 | O | 1,2,3-oxadiazol-5-yl(4-F) |
| I-0738 | O | 1,2,3-oxadiazol-5-yl(4-Cl) |
| I-0739 | O | 1,2,3-oxad iazol-5-yl(4-Me) |
| I-0740 | O | 1,2,3-oxadiazol-5-yl(4-OMe) |
| I-0741 | O | 1,2,3-oxadiazol-5-yl(4-CN) |
| I-0742 | O | 1,2,3-oxadiazol-5-yl(4-$CF_3$) |
| I-0743 | O | 1,2,3-oxadiazol-5-yl(4-$CO_2$Et) |
| I-0744 | O | 1,2,4-oxadiazol-3-yl |
| I-0745 | O | $CH_2$1,2,4-oxadiazol-3-yl |
| I-0746 | O | 1,2,4-oxadiazol-3-yl(5-F) |
| I-0747 | O | 1,2,4-oxadiazol-3-yl(5-Cl) |
| I-0748 | O | 1,2,4-oxadiazol-3-yl(5-Me) |
| I-0749 | O | 1,2,4-oxadiazol-3-yl(5-OMe) |

(continued)

| Compound Number | Y | Q |
|---|---|---|
| t-0750 | O | 1,2,4-oxadiazol-3-yl(5-CN) |

[Table 200]

| Compound Number | Y | Q |
|---|---|---|
| I-0751 | O | 1,2,4-oxadiazol-3-yl(5-$CF_3$) |
| I-0752 | O | 1,2,4-oxadiazol-3-yl(5-$CO_2$Et) |
| I-0753 | O | 1,2,4-oxadiazol-5-yl |
| I-0754 | O | $CH_2$1,2,4-oxadiazol-5-yl |
| I-0755 | O | 1 ,2,4-oxad iazol-5-yl(3-F) |
| I-0756 | O | 1,2,4-oxadiazol-5-yl(3-Cl) |
| I-0757 | O | 1,2,4-oxadiazol-5-yl(3-Me) |
| I-0758 | O | 1,2,4-oxadiazol-5-yl(3-OMe) |
| I-0759 | O | 1,2,4-oxadiazol-5-yl(3-CN) |
| I-0760 | O | 1,2,4-oxadiazol-5-yl(3-$CF_3$) |
| I-0761 | O | 1,2,4-oxadiazol-5-yl(3-$CO_2$Et) |
| I-0762 | O | 1,3,4-oxadiazol-2-yl |
| I-0763 | O | $CH_2$1,3,4-oxadiazol-2-yl |
| I-0764 | O | 1,2,5-oxadiazol-3-yl |
| I-0765 | O | $CH_2$1,2,5-oxadiazol-3-yl |
| I-0766 | O | 1,2,3-thiadiazol-4-yl |
| I-0767 | O | $CH_2$1,2,3-thiadiazol-4-yl |
| I-0768 | O | 1,2,3-thiadiazol-5-yl |
| I-0769 | O | $CH_2$1,2,3-thiadiazol-5-yl |
| I-0770 | O | 1,2,4-thiadiazol-3-yl |
| I-0771 | O | $CH_2$1,2,4-thiadiazol-3-yl |
| I-0772 | O | 1,2,4-thiadiazol-5-yl |
| I-0773 | O | $CH_2$1,2,4-thiadiazol-5-yl |
| I-0774 | O | 1,3,4-thiadiazol-2-yl |
| I-0775 | O | $CH_2$1,3,4-thiadiazol-2-yl |
| I-0776 | O | 1,2,5-thiadiazol-3-yl |
| I-0777 | O | $CH_2$1,2,5-thiadiazol-3-yl |
| I-0778 | O | morpholin-4-yl |
| I-0779 | O | $CH_2$morpholin-4-yl |
| I-0780 | O | piperidin-4-yl |
| I-0781 | O | $CH_2$piperidin-4-yl |
| I-0782 | O | piperidin-4-yl(1-Me) |

(continued)

| Compound Number | Y | Q |
|---|---|---|
| I-0783 | O | $CH_2$piperidin-4-yl(1-Me) |
| I-0784 | O | piperidin-1-yl |
| I-0785 | O | $CH_2$piperidin-1-yl |
| I-0786 | O | piperazin-1-yt |
| I-0787 | O | $CH_2$piperazin-1-yl |
| I-0788 | O | piperazin-1-yl(4-Me) |
| I-0789 | O | $CH_2$piperazin-1yl(4-Me) |
| I-0790 | O | tetrahydro-2H-pyran-4-yl |
| I-0791 | O | $CH_2$tetrahydro-2H-pyran-4-yl |
| I-0792 | O | 1,3-dioxolan-2-yl |

[Table 201]

| Compound Number | Y | Q |
|---|---|---|
| I-0793 | O | $CH_2$1,3-dioxolan-2-yl |
| I-0794 | O | 1,3-dioxolan-2-yl(2-$CF_3$) |
| I-0795 | O | $CH_2$1,3-dioxolan-2-yl(2-$CF_3$) |
| I-0796 | O | 1,3-dioxan-2-yl |
| I-0797 | O | $CH_2$1,3-dioxan-2-yl |
| I-0798 | O | 1,3-dioxan-2-yl(2-$CF_3$) |
| I-0799 | O | $CH_2$1,3-dioxan-2-yl(2-$CF_3$) |
| I-0800 | O | pyrrolidin-1-yl |
| I-0801 | O | $CH_2$pyrrolidin-1-yl |
| I-0802 | O | pyrrolidin-2-yl |
| I-0803 | O | $CH_2$pyrrolidin-2-yl |
| I-0804 | O | pyrrolidin-2-yl(1-Me) |
| I-0805 | O | $CH_2$pyrrolidin-2-yl(1-Me) |
| I-0806 | O | pyrrolidin-3-yl |
| I-0807 | O | $CH_2$pyrrolidin-3-yl |
| I-0808 | O | pyrrolidin-3-yl(1-Me) |
| I-0809 | O | $CH_2$pyrrolidin-3-yl(1-Me) |
| I-0810 | O | tetrahydrofuran-2-yl |
| I-0811 | O | $CH_2$tetrahydrofuran-2-yl |
| I-0812 | O | tetrahydrofuran-3-yl |
| I-0813 | O | $CH_2$tetrahydrofuran-3-yl |
| I-0814 | O | 4,5-dihydroisoxazol-3-yl |

(continued)

| Compound Number | Y | Q |
|---|---|---|
| I-0815 | O | $CH_2$4,5-dihydroisoxazol-3-yl |
| I-0816 | O | 4,5-dihydroisoxazol-4-yl |
| I-0817 | O | $CH_2$4,5-dihydroisoxazol-4-yl |
| I-0818 | O | 4,5-dihydroisoxazol-5-yl |
| I-0819 | O | $CH_2$4,5-dihydroisoxazol-5-yl |
| I-0820 | O | oxetan-2-yl |
| I-0821 | O | $CH_2$oxetan-2-yl |
| I-0822 | O | oxetan-3-yl |
| I-0823 | O | oxetan-3-yl(3-Me) |
| I-0824 | O | $CH_2$oxetan-3-yl |
| I-0825 | O | azetidin-1-yl |
| I-0826 | O | $CH_2$azetidin-1-yl |
| I-0827 | O | azetidin-2-yl |
| I-0828 | O | $CH_2$azetidin-2-yl |
| I-0829 | O | azetidin-2-yl(1-Me) |
| I-0830 | O | $CH_2$azetidin-2-yl(1-Me) |
| I-0831 | O | azetidin-3-yl |
| I-0832 | O | $CH_2$azetidin-3-yl |
| I-0833 | O | azetidin-3-yl(1-Me) |
| I-0834 | O | $CH_2$azetidin-3-yl(1-Me) |

[Table 202]

| Compound Number | Y | Q |
|---|---|---|
| I-0835 | O | oxiran-2-yl |
| I-0836 | O | $CH_2$oxiran-2-yl |
| I-0837 | O | 1,3,2-dioxaborolan-2-yl(4,4,5,5-$Me_4$) |
| I-0838 | O | tetrahydro-2H-pyran-2-yl |
| I-0839 | O | $CH_2$tetrahydro-2H-pyran-2-yl |
| I-0840 | O | tetrahydro-2H-pyran-3-yl |
| I-0841 | O | $CH_2$tetrahydro-2H-pyran-3-yl |
| I-0842 | O | piperidin-2-yl |
| I-0843 | O | $CH_2$piperidin-2-yl |
| I-0844 | O | piperidin-2-yl(1-Me) |
| I-0845 | O | $CH_2$piperidin-2-yl(1-Me) |
| I-0846 | O | piperidin-3-yl |
| I-0847 | O | $CH_2$piperidin-3-yl |

(continued)

| Compound Number | Y | Q |
|---|---|---|
| I-0848 | O | piperidin-3-yl(1-Me) |
| I-0849 | O | $CH_2$piperidin-3-yl(1-Me) |
| I-0850 | O | pyridin-2-yl(6-$CH_2OCH_3$) |
| I-0851 | O | 1H-pyrazol-5-yl(1-CHO) |
| I-0852 | O | (pyridin-1-oxide)-2-yl |
| I-0853 | O | (pyridin-1-oxide)-3-yl |
| I-0854 | O | (pyridin-1-oxide)-4-yl |
| I-0855 | O | piperidin-4-yl(1-Me) ▪ HCl |

Meanwhile, the compound of the present invention of formula [I], [II], or [III] can be produced according to the following production method, but the production method is not limited to these methods. Hereinafter, for example, a "compound of formula [I-1]" is synonymous with a "compound [I-1]".

<Production Method 1>

A compound of formula [I-1] among the compounds of the present invention can be produced, for example, according to a method based on the reaction scheme exemplified below.

[Chem. 4]

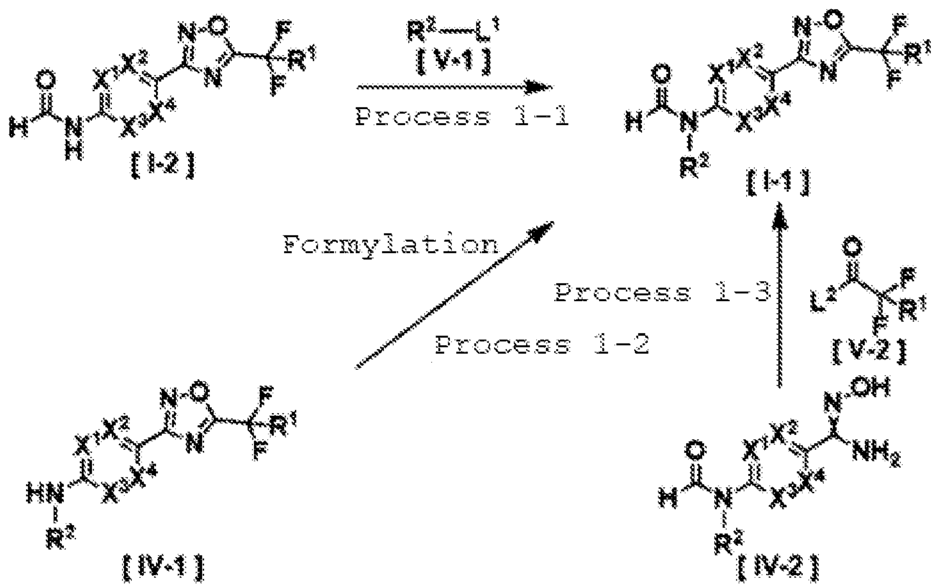


[I-2] [I-1] [V-2] [IV-1] [IV-2]

(in the formula, $L^1$ represents a halogen atom, a (Cl-C6)alkylsulfonyloxy, a (C1-C6)haloalkylsulfonyloxy, a phenylsulfonyloxy, a 4-toluenesulfonyloxy, or a 1-imidazolyl, $L^2$ represents a halogen atom, a (C1-C6)alkoxy, a (Cl-C6)haloalkoxy, a phenoxy, a (C1-C6)alkylsulfonyloxy, a (Cl-C6)haloalkylsulfonyloxy, a phenylsulfonyloxy, a 4-toluenesulfonyloxy, a 1-imidazolyl, a (Cl-C6)alkoxycarbonyloxy, a (C1-C6)alkylcarbonyloxy, a (Cl-C6)haloalkylcarbonyloxy, or a (C1-C6)haloalkoxycarbonyloxy, and $X^1$, $X^2$, $X^3$, $X^4$, $R^1$, and $R^2$ are as defined above.)

(Process 1-1)

That is, the compound of formula [1-1] can be produced by reacting a compound [1-2] and a compound [V-1]

in the presence or absence of an appropriate solvent, in the presence or absence of an appropriate base, and in the presence or absence of an appropriate catalyst.

The amount of the compound [V-1] used in the present reaction may be appropriately selected from a range of usually 1.0 to 20 mol, and is preferably 1.0 to 3.0 mol, based on 1 mol of the compound [1-2].

Examples of the base that can be used in the present reaction include hydroxides of alkali metal, such as lithium hydroxide, sodium hydroxide, and potassium hydroxide; hydroxides of alkali earth metal, such as calcium hydroxide and magnesium hydroxide; carbonates of alkali metal, such as lithium carbonate, sodium carbonate, potassium carbonate, and cesium carbonate; bicarbonates of alkali metal, such as sodium bicarbonate and potassium bicarbonate; metal hydrides of alkali metal, such as lithium hydride, sodium hydride, and potassium hydride; metal salts of alcohol, such as sodium methoxide, sodium ethoxide, and potassium tert-butoxide; and organic bases such as triethylamine, tributylamine, diisopropylethylamine, N,N-dimethylaniline, pyridine, 2,6-lutidine, 4-N,N-dimethylaminopyridine, and 1,8-diazacyclo[5.4.0]-7-undecene. The amount of the base used may be appropriately selected from a range of 0.5 to 10 mol, and is preferably 1.0 to 5.0 mol, based on 1 mol of the compound [1-2] .

Examples of the catalyst that can be used in the present reaction include iodides of alkali metal, such as sodium iodide and potassium iodide. The amount of the catalyst used may be appropriately selected from a range of 0.01 to 0.5 mol, and is preferably 0.01 to 0.1 mol, based on 1 mol of the compound [1-2].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; nitriles such as acetonitrile and propionitrile; aliphatic hydrocarbons such as pentane, hexane, cyclohexane, and heptane; pyridines such as pyridine, picoline, and lutidine; tertiary amines such as triethylamine and tributylamine; water; and a mixed solvent thereof. The amount of the solvent used is 0.1 to 100 liters, and preferably 0.1 to 20 liters, based on 1 mol of the compound [1-2].

The reaction temperature of the present reaction may be selected from a range of any temperature usually from -78°C to a reflux temperature in a reaction system, and may be preferably in a range of 0°C to 150°C. The reaction time of the present reaction is usually 10 minutes to 72 hours although it varies depending on the reaction temperature, the reactant, the reaction amount, and the like.

After the completion of the reaction, the compound [I-1] can be isolated by subjecting the reaction mixture to operations such as pouring into water, extraction with an organic solvent, and concentration. The isolated compound [I-1] can be further purified by column chromatography, recrystallization, or the like, as necessary.

(Process 1-2)

That is, the compound of formula [1-1] can be produced by reacting a compound [IV-1] and an appropriate formylating agent in the presence or absence of an appropriate solvent and in the presence or absence of an appropriate acid catalyst.

The compound [IV-1] can be produced in accordance with the method described in of the description of EP 3,187,497 A, page 68, or the method thereof.

Examples of the formylating agent that can be used in the present reaction include formic acid, acetic anhydride, sodium formate, methyl formate, ethyl formate, cyanomethyl formate, N-formyl saccharin, and 1-formyl-1H-benzotriazole. The amount of the formylating agent used may be appropriately selected from a range of usually 1.0 to 20 mol, and is preferably 1.0 to 10 mol, based on 1 mol of the compound [IV-1].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; halogenated hydrocarbons such as dichloromethane, chloroform, and 1,2-dichloroethane; aliphatic hydrocarbons such as pentane, hexane, cyclohexane, and heptane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, 2-propanol, tert-butyl alcohol, and methyl cellosolve; carboxylic acids such as formic acid, acetic acid, propionic acid, and trifluoroacetic acid; water; and a mixed solvent thereof. The amount of the solvent used is 0.1 to 100 liters, and preferably 0.1 to 20 liters, based on 1 mol of the compound [IV-1].

Examples of the acid catalyst that can be used in the present reaction include mineral acids such as hydrochloric acid, hydrobromic acid, and sulfuric acid, carboxylic acids such as acetic acid, propionic acid, dichloroacetic acid, and trifluoroacetic acid; and sulfonic acids such as methanesulfonic acid, benzenesulfonic acid, 4-toluenesulfonic acid, and trifluoromethanesulfonic acid. The amount of the acid catalyst used may be appropriately selected from a range of usually 0.01 to 10 mol, and is preferably 0.01 to 5.0 mol, based on 1 mol of the compound [IV-1].

The reaction temperature of the present reaction may be selected from a range of any temperature usually from 0°C to a reflux temperature in a reaction system, and may be preferably in a range of a reflux temperature in a reaction system from room temperature. The reaction time of the present reaction is usually 10 minutes to 72 hours although it varies depending on the reaction temperature, the reactant, the reaction amount, and the like.
After the completion of the reaction, the compound [1-1] can be isolated by subjecting the reaction mixture to operations such as pouring into water, extraction with an organic solvent, and concentration. The isolated compound [I-1] can be further purified by column chromatography, recrystallization, or the like, as necessary.

(Process 1-3)

That is, the compound of formula [1-1] can be produced by reacting a compound [IV-2] and a compound [V-2] in the presence or absence of an appropriate solvent and in the presence or absence of an appropriate base.
The amount of the compound [V-2] used in the present reaction may be appropriately selected from a range of usually 1.0 to 20 mol, and is preferably 1.0 to 3.0 mol, based on 1 mol of the compound [IV-2].
Examples of the base that can be used in the present reaction include hydroxides of alkali metal, such as lithium hydroxide, sodium hydroxide, and potassium hydroxide; hydroxides of alkali earth metal, such as calcium hydroxide and magnesium hydroxide; carbonates of alkali metal, such as lithium carbonate, sodium carbonate, potassium carbonate, and cesium carbonate; bicarbonates of alkali metal, such as sodium bicarbonate and potassium bicarbonate; metal hydrides of alkali metal, such as lithium hydride, sodium hydride, and potassium hydride; metal salts of alcohol, such as sodium methoxide, sodium ethoxide, and potassium tert-butoxide; and organic bases such as triethylamine, tributylamine, diisopropylethylamine, N,N-dimethylaniline, pyridine, 2,6-lutidine, 4-N,N-dimethylaminopyridine, and 1,8-diazacyclo[5.4.0]-7-undecene. The amount of the base used may be appropriately selected from a range of 0.5 to 10 mol, and is preferably 1.0 to 5.0 mol, based on 1 mol of the compound [IV-2].
Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, 2-propanol, tert-butyl alcohol, and methyl cellosolve; nitriles such as acetonitrile and propionitrile; aliphatic hydrocarbons such as pentane, hexane, cyclohexane, and heptane; pyridines such as pyridine, picoline, and lutidine; tertiary amines such as triethylamine and tributylamine; water; and a mixed solvent thereof. The amount of the solvent used is 0.1 to 100 liters, and preferably 0.1 to 20 liters, based on 1 mol of the compound [IV-2].
The reaction temperature of the present reaction may be selected from a range of any temperature usually from -78°C to a reflux temperature in a reaction system, and may be preferably in a range of a reflux temperature in a reaction system from 0°C. The reaction time of the present reaction is usually 10 minutes to 72 hours although it varies depending on the reaction temperature, the reactant, the reaction amount, and the like.
After the completion of the reaction, the compound [1-1] can be isolated by subjecting the reaction mixture to operations such as pouring into water, extraction with an organic solvent, and concentration. The isolated compound [I-1] can be further purified by column chromatography, recrystallization, or the like, as necessary.

<Production Method 2>

A compound of formula [1-2] among the compounds of the present invention can be produced, for example, according to a method based on the reaction scheme exemplified below.

[Chem. 5]

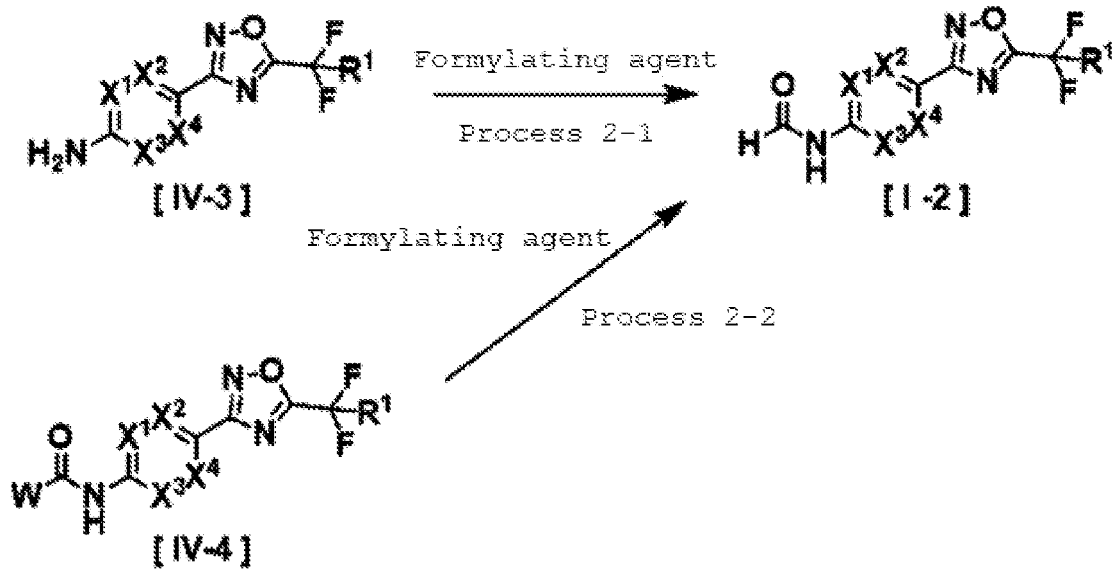


[IV-3]

[I-2]

[IV-4]

(in the formula, W represents (C1-C6)alkoxy or (C1-C6)haloalkyl, and $X^1$, $X^2$, $X^3$, $X^4$, and $R^1$ are as defined above.)

(Process 2-1)

That is, the compound of formula [1-2] can be produced by reacting a compound [IV-3] and an appropriate formylating agent in the presence or absence of an appropriate solvent and in the presence or absence of an appropriate acid catalyst. The compound [IV-3] can be produced in accordance with the method described in the description of WO 2007/111212 A, page 323, or the method thereof.

The formylating agent, the solvent, the acid catalyst, the reaction temperature, and the reaction time that can be used in the present reaction are the same as those in Process 1-2 of Production Method 1.

After the completion of the reaction, the compound [1-2] can be isolated by subjecting the reaction mixture to operations such as pouring into water, extraction with an organic solvent, and concentration. The isolated compound [I-2] can be further purified by column chromatography, recrystallization, or the like, as necessary.

(Process 2-2)

That is, the compound of formula [1-2] can be produced by reacting a compound [IV-4] and an appropriate formylating agent in the presence or absence of an appropriate solvent and in the presence or absence of an appropriate acid catalyst. The compound [IV-4] can be produced in accordance with the method described in the description of WO 2007/111212 A, page 323, or the method thereof.

The formylating agent, the solvent, the acid catalyst, the reaction temperature, and the reaction time that can be used in the present reaction are the same as those in Process 1-2 of Production Method 1.

After the completion of the reaction, the compound [1-2] can be isolated by subjecting the reaction mixture to operations such as pouring into water, extraction with an organic solvent, and concentration. The isolated compound [I-2] can be further purified by column chromatography, recrystallization, or the like, as necessary.

<Production Method 3>

A compound of formula [IV-2] can be produced, for example, according to a method based on the reaction scheme exemplified below.

[Chem. 6]

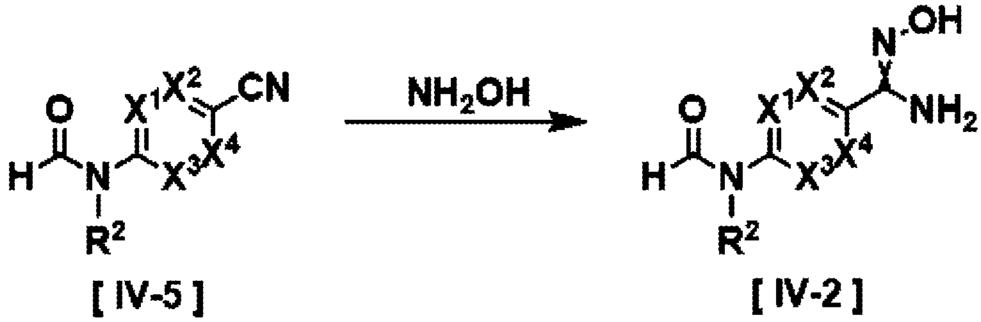


(in the formula, $X^1$, $X^2$, $X^3$, $X^4$, and $R^2$ are as defined above.)

That is, the compound of formula [IV-2] can be produced by reacting the compound [IV-5] and appropriate hydroxylamine in the presence of an appropriate solvent and in the presence or absence of an appropriate base.

Examples of the hydroxylamine that can be used in the present reaction include a hydroxylamine aqueous solution, hydroxylamine hydrochloride, and hydroxylamine sulfate. The amount of the hydroxylamine used may be appropriately selected from a range of 1.0 to 20 mol, and is preferably 1.0 to 10 mol, based on 1 mol of the compound [IV-5].

Examples of the base that can be used in the present reaction include hydroxides of alkali metal, such as lithium hydroxide, sodium hydroxide, and potassium hydroxide; hydroxides of alkali earth metal, such as calcium hydroxide and magnesium hydroxide; carbonates of alkali metal, such as lithium carbonate, sodium carbonate, potassium carbonate, and cesium carbonate; bicarbonates of alkali metal, such as sodium bicarbonate and potassium bicarbonate; metal hydrides of alkali metal, such as lithium hydride, sodium hydride, and potassium hydride; metal salts of alcohol, such as sodium methoxide, sodium ethoxide, and potassium tert-butoxide; and organic bases such as triethylamine, tributylamine, diisopropylethylamine, N,N-dimethylaniline, pyridine, 2,6-lutidine, 4-N,N-dimethylaminopyridine, and 1,8-diazacyclo[5.4.0]-7-undecene. The amount of the base used may be appropriately selected from a range of 0.5 to 10 mol, and is preferably 1.0 to 10 mol, based on 1 mol of the compound [IV-5].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, 2-propanol, tert-butyl alcohol, and methyl cellosolve; nitriles such as acetonitrile and propionitrile; aliphatic hydrocarbons such as pentane, hexane, cyclohexane, and heptane; pyridines such as pyridine, picoline, and lutidine; tertiary amines such as triethylamine and tributylamine; water; and a mixed solvent thereof. The amount of the solvent used is 0.1 to 100 liters, and preferably 0.1 to 20 liters, based on 1 mol of the compound [IV-5].

The reaction temperature of the present reaction may be selected from a range of any temperature usually from 0°C to a reflux temperature in a reaction system, and may be preferably in a range of a reflux temperature in a reaction system from room temperature. The reaction time of the present reaction is usually 10 minutes to 72 hours although it varies depending on the reaction temperature, the reactant, the reaction amount, and the like.

After the completion of the reaction, the compound [IV-2] can be isolated by subjecting the reaction mixture to operations such as pouring into water, extraction with an organic solvent, and concentration. The isolated compound [IV-2] can be further purified by column chromatography, recrystallization, or the like, as necessary.

<Production Method 4>

A compound of formula [IV-5] can be produced, for example, according to a method based on the reaction scheme exemplified below.

[Chem. 7]

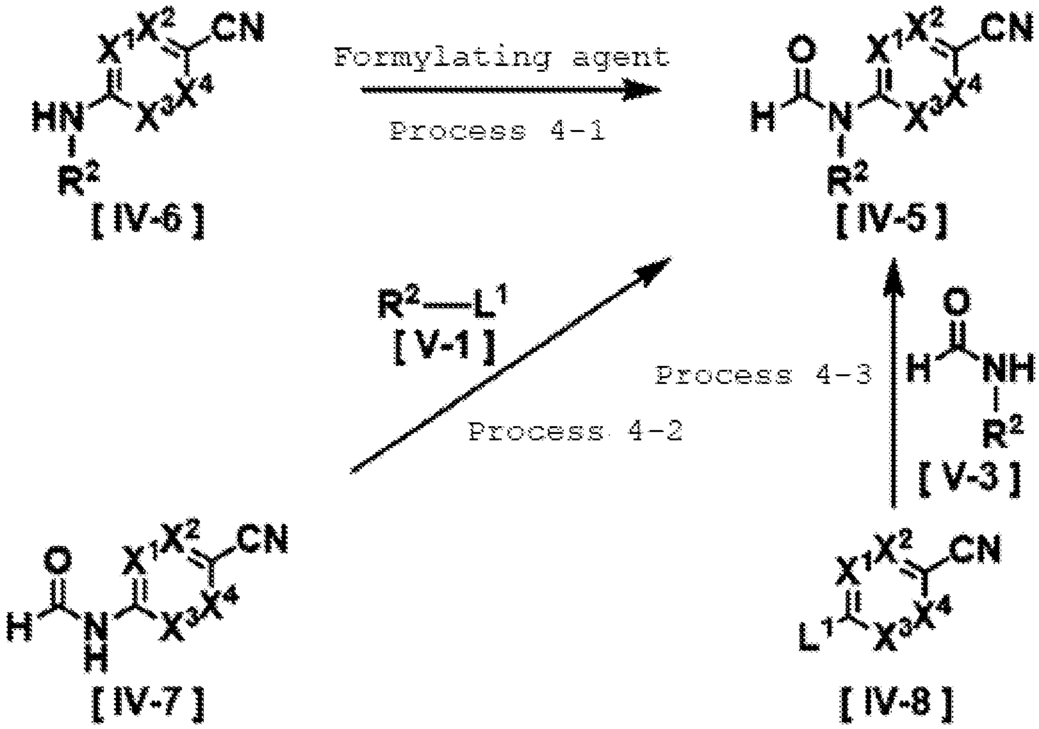


(in the formula, $L^1$, $X^1$, $X^2$, $X^3$, $X^4$, and $R^2$ are as defined above.)

(Process 4-1)

That is, the compound of formula [IV-5] can be produced by reacting a compound [IV-6] and an appropriate formylating agent in the presence or absence of an appropriate solvent and in the presence or absence of an appropriate acid catalyst.
The formylating agent, the solvent, the acid catalyst, the reaction temperature, and the reaction time that can be used in the present reaction are the same as those in Process 1-2 of Production Method 1.
After the completion of the reaction, the compound [IV-5] can be isolated by subjecting the reaction mixture to operations such as pouring into water, extraction with an organic solvent, and concentration. The isolated compound [IV-5] can be further purified by column chromatography, recrystallization, or the like, as necessary.

(Process 4-2)

That is, the compound of formula [IV-5] can be produced by reacting a compound [IV-7] and a compound [V-1] in the presence or absence of an appropriate solvent, in the presence or absence of an appropriate base, and in the presence or absence of an appropriate catalyst. The compound [IV-7] can be produced in accordance with the method described in European Journal of Organic Chemistry, vol. 28, page 4,539 page, 2019, or the method thereof.
The amount of the compound [V-1] used in the present reaction may be appropriately selected from a range of usually 1.0 to 20 mol, and is preferably 1.0 to 3.0 mol, based on 1 mol of the compound [IV-7].
The base, the catalyst, and the solvent that can be used in the present reaction are the same as those in Process 1-1 of Production Method 1.
The reaction temperature of the present reaction may be selected from a range of any temperature usually from 0°C to a reflux temperature in a reaction system, and may be preferably in a range of 0°C to 100°C. The reaction time of the present reaction is usually 10 minutes to 72 hours although it varies depending on the reaction temperature, the reactant, the reaction amount, and the like.
After the completion of the reaction, the compound [IV-5] can be isolated by subjecting the reaction mixture to operations such as pouring into water, extraction with an organic solvent, and concentration. The isolated compound [IV-5] can be further purified by column chromatography, recrystallization, or the like, as necessary.

(Process 4-3)

That is, the compound of formula [IV-5] can be produced by reacting a compound [IV-8] and a compound [V-3] in the presence or absence of an appropriate solvent, in the presence or absence of an appropriate base, in the presence or absence of an appropriate catalyst, and in the presence or absence of a copper salt.

The amount of the compound [V-3] used in the present reaction may be appropriately selected from a range of usually 1.0 to 20 mol, and is preferably 1.0 to 3.0 mol, based on 1 mol of the compound [IV-8].

Examples of the base that can be used in the present reaction include hydroxides of alkali metal, such as lithium hydroxide, sodium hydroxide, and potassium hydroxide; hydroxides of alkali earth metal, such as calcium hydroxide and magnesium hydroxide; carbonates of alkali metal, such as lithium carbonate, sodium carbonate, potassium carbonate, and cesium carbonate; bicarbonates of alkali metal, such as sodium bicarbonate and potassium bicarbonate; metal hydrides of alkali metal, such as lithium hydride, sodium hydride, and potassium hydride; inorganic bases such as fluoride of alkali metal, such as sodium fluoride and potassium fluoride, and tripotassium phosphate; metal salts of alcohol, such as sodium methoxide, sodium ethoxide, and potassium tert-butoxide; and organic bases such as triethylamine, tributylamine, diisopropylethylamine, N,N-dimethylaniline, pyridine, 2,6-lutidine, 4-N,N-dimethylaminopyridine, and 1,8-diazacyclo[5.4.0]-7-undecene. The amount of the base used may be appropriately selected from a range of 0.5 to 10 mol, and is preferably 1.0 to 5.0 mol, based on 1 mol of the compound [IV-8].

As the catalyst that can be used in the present reaction, a transition metal complex or a combination of a transition metal and a ligand may be used. Examples of the transition metal complex include tetrakis(triphenylphosphine)palladium and a dichloro[1,1'-bis(diphenylphosphino)ferrocene]palladium-dichloromethane adduct. Examples of the transition metal include palladium chloride, palladium bromide, and palladium acetate. Examples of the ligand include trimethylphosphine, triethylphosphine, tributylphosphine, triphenylphosphine, 1,3-bisdimethylphosphinopropane, 1,2-bisdiphenylphosphinoetane, and 1,3-bisdiphenylphosphinopropane. The amount of the catalyst used is 0.001 to 0.5 mol, and preferably 0.01 to 0.2 mol, based on 1 mol of the compound [IV-8].

Examples of the copper salt that can be used in the present reaction include copper(I) chloride, copper(I) bromide, and copper(I) iodide. The amount of the copper salt used is 0.1 to 10 mol, and preferably 0.1 to 4.0 mol, based on 1 mol of the compound [IV-8].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; nitriles such as acetonitrile and propionitrile; aliphatic hydrocarbons such as pentane, hexane, cyclohexane, and heptane; pyridines such as pyridine, picoline, and lutidine; tertiary amines such as triethylamine and tributylamine; water; and a mixed solvent thereof. The amount of the solvent used is 0.1 to 100 liters, and preferably 0.1 to 20 liters, based on 1 mol of the compound [IV-8].

The reaction temperature of the present reaction may be selected from a range of any temperature usually from 0°C to a reflux temperature in a reaction system, and may be preferably in a range of 0°C to 150°C. The reaction time of the present reaction is usually 10 minutes to 72 hours although it varies depending on the reaction temperature, the reactant, the reaction amount, and the like.

After the completion of the reaction, the compound [IV-5] can be isolated by subjecting the reaction mixture to operations such as pouring into water, extraction with an organic solvent, and concentration. The isolated compound [IV-5] can be further purified by column chromatography, recrystallization, or the like, as necessary.

<Production Method 5>

A compound of formula [IV-6] can be produced, for example, according to the method shown by the following reaction formula using a compound of formula [IV-8].

[Chem. 8]

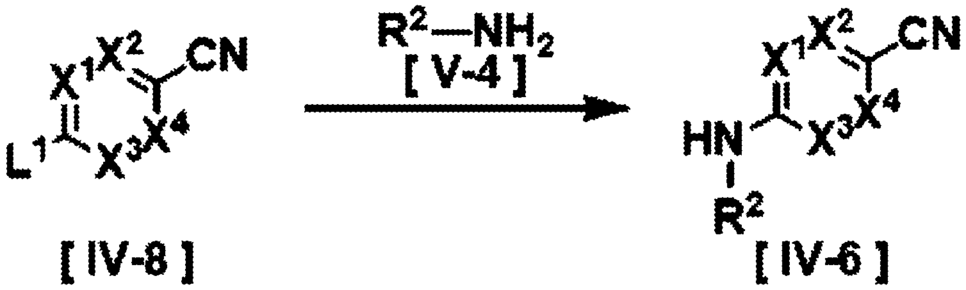


(in the formula, $L^1$, $X^1$, $X^2$, $X^3$, $X^4$, and $R^2$ are as defined above.)

That is, the compound of formula [IV-6] can be produced by reacting a compound [IV-8] and a compound [V-4] in the presence or absence of an appropriate solvent, in the presence or absence of an appropriate base, in the presence or absence of an appropriate catalyst, and in the presence or absence of a copper salt.

The amount of the compound [V-4] used in the present reaction may be appropriately selected from a range of usually 1.0 to 20 mol, and is preferably 1.0 to 3.0 mol, based on 1 mol of the compound [IV-8].

The base, the catalyst, the copper salt, the solvent, the reaction temperature, and the reaction time that can be used in the present reaction are the same as those in Process 4-3 of Production Method 4.

After the completion of the reaction, the compound [IV-6] can be isolated by subjecting the reaction mixture to operations such as pouring into water, extraction with an organic solvent, and concentration. The isolated compound [IV-6] can be further purified by column chromatography, recrystallization, or the like, as necessary.

<Production Method 6>

A compound of formula [IV-9] can be produced, for example, according to a method based on the reaction scheme exemplified below.

[Chem. 9]

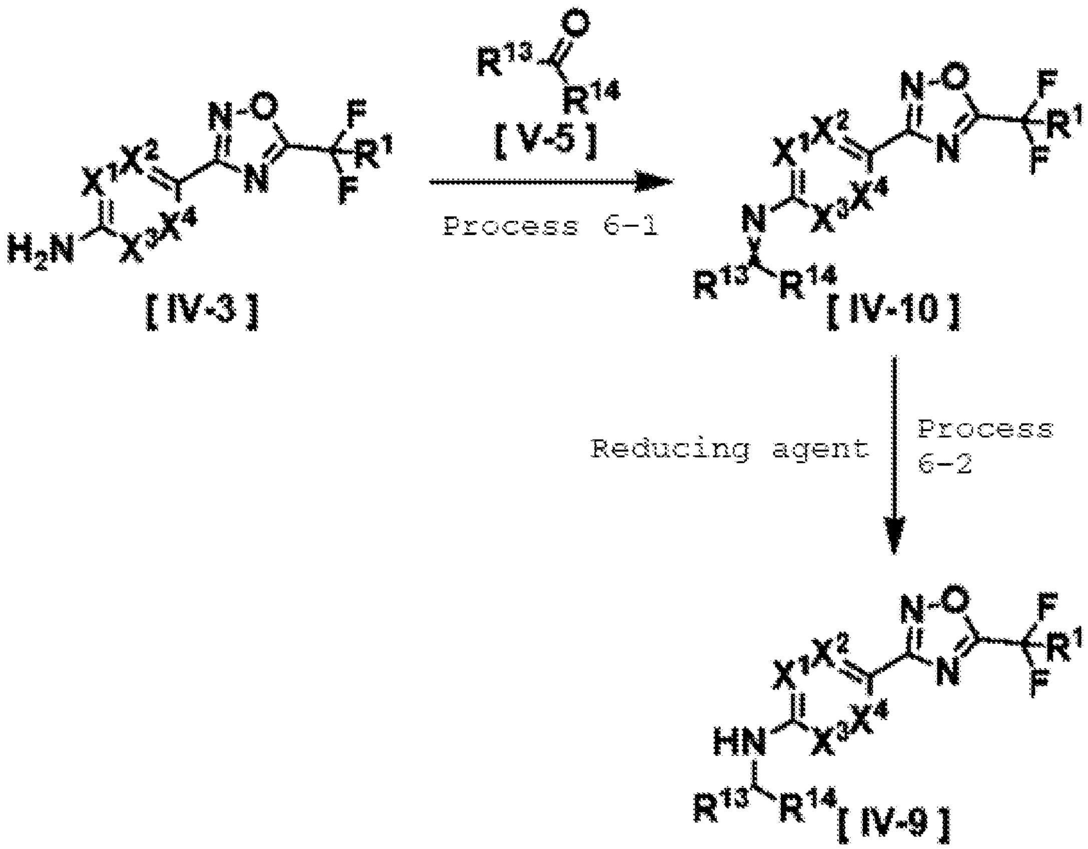


(in the formula, $R^{13}$ and $R^{14}$ are each independently selected and each represent a hydrogen atom, a (C1-C5)alkyl (the (C1-C5)alkyl is optionally mono-substituted or poly-substituted with $R^4$), a (C2-C5)alkenyl (the (C2-C5)alkenyl is optionally mono-substituted or poly-substituted with $R^4$), a (C2-C5)alkynyl group (the (C2-C5)alkynyl is optionally mono-substituted or poly-substituted with $R^4$), a (C3-C6)cycloalkyl, a (C6-C12)aryl (the (C6-C12)aryl is optionally mono-substituted or poly-substituted with $R^5$), a 3- to 6-membered heterocyclyl (the 3-to 6-membered heterocyclyl is optionally mono-substituted or poly-substituted with $R^5$), or a 5- to 12-membered heteroaryl (the 5- to 12-membered heteroaryl is optionally mono-substituted or poly-substituted with $R^5$), and $X^1$, $X^2$, $X^3$, $X^4$, and $R^1$ are as defined above.)

(Process 6-1)

That is, the compound of formula [IV-10] can be produced by reacting a compound of formula [IV-3] and a compound [V-5] in the presence or absence of an appropriate solvent and in the presence or absence of an appropriate acid catalyst.

The amount of the compound [V-5] used in the present reaction may be appropriately selected from a range of usually 1.0 to 20 mol, and is preferably 1.0 to 5.0 mol, based on 1 mol of the compound [IV-3].

Examples of the acid catalyst that can be used in the present reaction include sulfonic acids such as methanesulfonic acid, trifluoromethanesulfonic acid, benzenesulfonic acid, and 4-toluenesulfonic acid; and Lewis acids such as titanium tetrachloride. The amount of the acid catalyst used may be appropriately selected from a range of 0.01 to 5.0 mol, and is preferably 0.01 to 2.0 mol, based on 1 mol of the compound [IV-3].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; nitriles such as acetonitrile and propionitrile; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, 2-propanol, tert-butyl alcohol, and methyl cellosolve; aliphatic hydrocarbons such as pentane, hexane, cyclohexane, and heptane; pyridines such as pyridine and picoline; and a mixed solvent thereof. The amount of the solvent used is 0.1 to 100 liters, and preferably 0.1 to 20 liters, based on 1 mol of the compound [IV-3].

The reaction temperature of the present reaction may be selected from a range of any temperature usually

from 0°C to a reflux temperature in a reaction system, and may be preferably in a range of a reflux temperature in a reaction system from room temperature. The reaction time of the present reaction is usually 10 minutes to 72 hours although it varies depending on the reaction temperature, the reactant, the reaction amount, and the like.

After the completion of the reaction, the compound [IV-10] can be isolated by subjecting the reaction mixture to operations such as pouring into water, extraction with an organic solvent, and concentration. The isolated compound [IV-10] can be further purified by column chromatography, recrystallization, or the like, as necessary.

(Process 6-2)

That is, the compound of formula [IV-9] can be produced by reacting a compound of formula [IV-10] and an appropriate reducing agent in the presence or absence of an appropriate solvent and in the presence or absence of an appropriate acid catalyst.

Examples of the reducing agent that can be used in the present reaction include alkali metal salts of borohydride compounds, such as sodium borohydride, sodium triacetoxyborohydride, lithium borohydride, lithium triethylborohydride, and sodium borohydride; borane complexes of organic Lewis bases, such as a borane-tert-butylamine complex, a borane-dimethylsulfide complex, and a borane-2-picoline complex; alkali metal salts of aluminum hydride compounds, such as lithium aluminum hydride and bis(2-methoxyethoxy)aluminum sodium hydride; and organic aluminum hydride compounds such as diisobutylaluminum hydride. The amount of the reducing agent used may be appropriately selected from a range of usually 0.25 to 5.0 mol, and is preferably 0.25 to 2.0 mol, based on 1 mol of the compound [IV-10].

Examples of the acid catalyst that can be used in the present reaction include sulfonic acids such as methanesulfonic acid, trifluoromethanesulfonic acid, benzenesulfonic acid, and 4-toluenesulfonic acid; and carboxylic acids such as formic acid, acetic acid, propionic acid, dichloroacetic acid, and trifluoroacetic acid. The amount of the acid catalyst used may be appropriately selected from a range of 0.01 to 10 mol, and is preferably 0.01 to 3.0 mol, based on 1 mol of the compound [IV-10].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; halogenated hydrocarbons such as dichloromethane, chloroform, and 1,2-dichloroethane; alcohols such as methanol, ethanol, 2-propanol, tert-butyl alcohol, and methyl cellosolve; aliphatic hydrocarbons such as pentane, hexane, cyclohexane, and heptane; water; and a mixed solvent thereof. The amount of the solvent used is 0.1 to 100 liters, and preferably 0.1 to 20 liters, based on 1 mol of the compound [IV-10].

The reaction temperature of the present reaction may be selected from a range of any temperature usually from -78°C to a reflux temperature in a reaction system, and may be preferably in a range of a reflux temperature in a reaction system from 0°C. The reaction time of the present reaction is usually 10 minutes to 72 hours although it varies depending on the reaction temperature, the reactant, the reaction amount, and the like.

After the completion of the reaction, the compound [IV-9] can be isolated by subjecting the reaction mixture to operations such as pouring into water, extraction with an organic solvent, and concentration. The isolated compound [IV-9] can be further purified by column chromatography, recrystallization, or the like, as necessary.

<Production Method 7>

A compound of formula [IV-3] can be produced, for example, according to a method based on the reaction scheme exemplified below.

[Chem. 10]

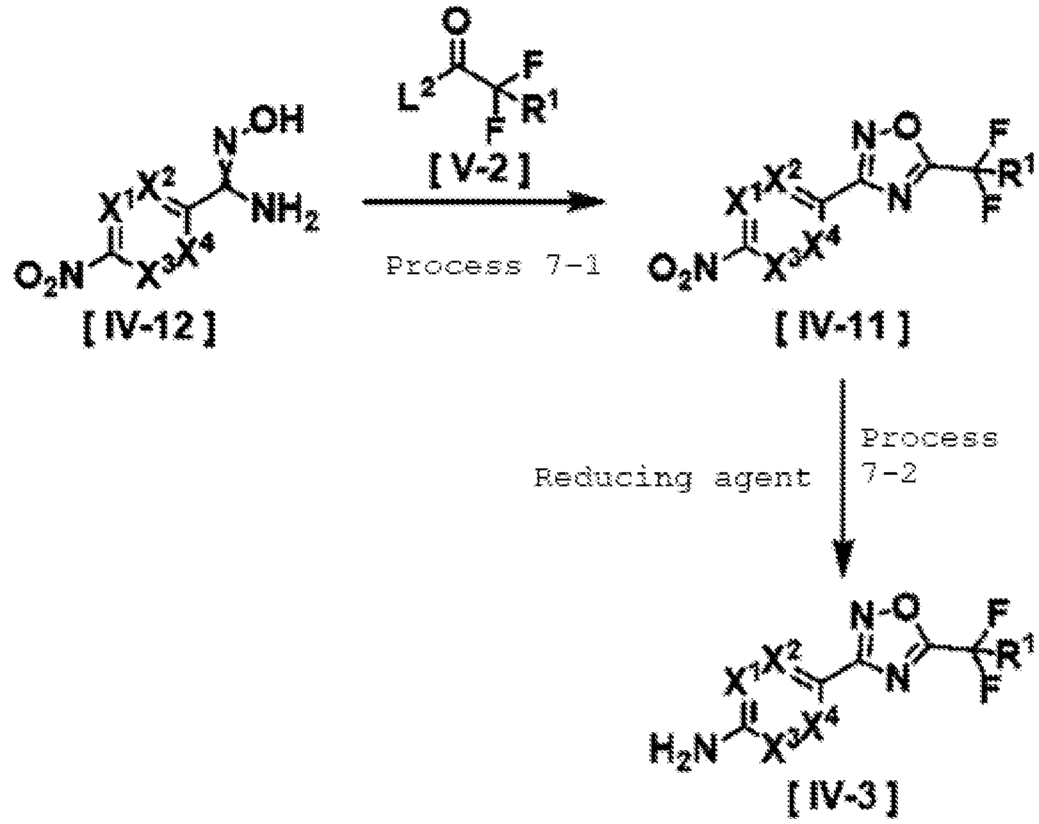


(in the formula, $L^2$, $X^1$, $X^2$, $X^3$, $X^4$, and $R^1$ are as defined above.)

(Process 7-1)

That is, the compound of formula [IV-11] can be produced by reacting a compound [IV-12] and a compound [V-2] in the presence or absence of an appropriate solvent and in the presence or absence of an appropriate base. The compound [IV-12] can be produced in accordance with the method described in European Journal of Organic Chemistry, vol. 34, page 7,472 page, 2015, or the method thereof.

The amount of the compound [V-2] used in the present reaction may be appropriately selected from a range of usually 1.0 to 20 mol, and is preferably 1.0 to 5.0 mol, based on 1 mol of the compound [IV-12].

The base, the solvent, the reaction temperature, and the reaction time that can be used in the present reaction are the same as those in Process 1-3 of Production Method 1.

After the completion of the reaction, the compound [IV-11] can be isolated by subjecting the reaction mixture to operations such as pouring into water, extraction with an organic solvent, and concentration. The isolated compound [IV-11] can be further purified by column chromatography, recrystallization, or the like, as necessary.

(Process 7-2)

That is, the compound of formula [IV-3] can be produced by reacting a compound of formula [IV-11] and an appropriate reducing agent in the presence or absence of an appropriate acid catalyst and in the presence or absence of an appropriate phase transfer catalyst.

Examples of the reducing agent that can be used in the present reaction include transition metals such as iron and zinc; typical metals such as tin; alkali metal salts of borohydride compounds, such as sodium borohydride, lithium borohydride, and lithium triethylborohydride; alkali metal salts of dithionous acid such as sodium dithionite; borane complexes of organic Lewis bases, such as a borane-tert-butylamine complex and a borane-dimethylsulfide complex; alkali metal salts of aluminum hydride compounds, such as lithium aluminum hydride and bis(2-methoxyethoxy)aluminum sodium hydride; and organic aluminum hydride compounds such as diisobutylaluminum hydride. The amount of the reducing agent used may be appropriately selected from a range of usually 0.25 to 50 mol, and is preferably 0.25 to 50 mol, based on 1 mol of the compound [IV-11].

Examples of the acid catalyst that can be used in the present reaction include sulfonic acids such as methanesulfonic acid, trifluoromethanesulfonic acid, benzenesulfonic acid, and 4-toluenesulfonic acid; and carboxylic acids such as formic acid, acetic acid, propionic acid, dichloroacetic acid, and trifluoroacetic acid. The amount of the acid catalyst used may be appropriately selected from a range of 0.01 to 10 mol, and is preferably 0.01 to 2.0 mol, based on 1 mol of the compound [IV-11].

Examples the phase transfer catalyst that can be used in the present reaction include tetrabutylammonium salts such as tetrabutylammonium bromide and tetrabutylammonium chloride. The amount of the phase transfer catalyst used may be appropriately selected from a range of 0.01 to 10 mol, and is preferably 0.01 to 2.0 mol, based on 1 mol of the compound [IV-11].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; halogenated hydrocarbons such as dichloromethane, chloroform, and 1,2-dichloroethane; alcohols such as methanol, ethanol, 2-propanol, tert-butyl alcohol, and methyl cellosolve; aliphatic hydrocarbons such as pentane, hexane, cyclohexane, and heptane; water; and a mixed solvent thereof. The amount of the solvent used is 0.1 to 100 liters, and preferably 0.1 to 20 liters, based on 1 mol of the compound [IV-11].

The reaction temperature of the present reaction may be selected from a range of any temperature usually from -78°C to a reflux temperature in a reaction system, and may be preferably in a range of a reflux temperature in a reaction system from 0°C. The reaction time of the present reaction is usually 10 minutes to 72 hours although it varies depending on the reaction temperature, the reactant, the reaction amount, and the like.

After the completion of the reaction, the compound [IV-3] can be isolated by subjecting the reaction mixture to operations such as pouring into water, extraction with an organic solvent, and concentration. The isolated compound [IV-3] can be further purified by column chromatography, recrystallization, or the like, as necessary.

<Production Method 8>

A compound of formula [IV-12] can be produced, for example, according to the following methods.

[Chem. 11]

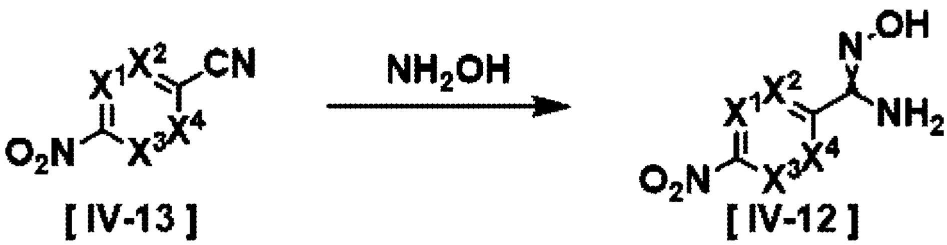


[IV-13] [IV-12]

(in the formula, $X^1$, $X^2$, $X^3$, and $X^4$ are as defined above.)

That is, the compound of formula [IV-12] can be produced by reacting the compound [IV-13] and appropriate hydroxylamine in the presence of an appropriate solvent and in the presence or absence of an appropriate base.

The hydroxylamine, the base, the solvent, the reaction temperature, and the reaction time that can be used in the present reaction are the same as those in Production Method 3.

After the completion of the reaction, the compound [IV-12] can be isolated by subjecting the reaction mixture to operations such as pouring into water, extraction with an organic solvent, and concentration. The isolated compound [IV-12] can be further purified by column chromatography, recrystallization, or the like, as necessary.

<Production Method 9>

A compound of formula [I-3] among the compounds of the present invention can be produced, for example, according to the following methods.

[Chem. 12]

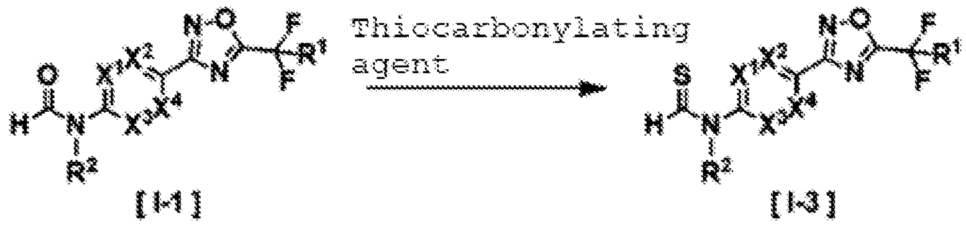


(in the formula, $X^1$, $X^2$, $X^3$, $X^4$, $R^1$, and $R^2$ are as defined above.)

That is, the compound of formula [I-3] can be produced by reacting a compound of formula [I-1] and an appropriate thiocarbonylating agent in the presence or absence of an appropriate base and in the presence or absence of an appropriate solvent.

Examples of the thiocarbonylating agent used in the present reaction include phosphorus pentasulfide and Lawesson's Reagent (2,4-bis(4-methoxyphenyl)-1,3,2,4-dithiadiphosphetan-2,4-disulfide). The amount of the thiocarbonylating agent used may be appropriately selected from a range of 0.25 to 100 mol, and is preferably 0.5 to 3.0 mol, based on 1 mol of the compound [1-1].

Examples of the base that can be used in the present reaction include inorganic bases such as carbonates of alkali metals such as sodium carbonate and potassium carbonate; and organic bases such as triethylamine, N,N-dimethylaniline, pyridine, 4-N,N-dimethylaminopyridine, and 1,8-diazabicyclo[5.4.0]-7-undecene. The amount of the base used may be appropriately selected from a range of 0.01 to 10 mol, and is preferably 0.1 to 2.0 mol, based on 1 mol of the compound [I-1].

Examples of the solvent that can be used in the present reaction include ethers such as diethyl ether, diisopropyl ether, methyl tert-butyl ether, cyclopentyl methyl ether, tetrahydrofuran, 2-methyl tetrahydrofuran, 4-methyltetrahydropyran, 1,4-dioxane, and monoglyme; aromatic hydrocarbons such as benzene, toluene, xylene, mesitylene, and chlorobenzene; aprotic polar solvents such as dimethyl sulfoxide and sulfolane; halogenated hydrocarbons such as dichloromethane, chloroform, and 1,2-dichloroethane; aliphatic hydrocarbons such as pentane, hexane, cyclohexane, and heptane; and a mixed solvent thereof. The amount of the solvent used is 0.1 to 100 liters, and preferably 0.1 to 50 liters, based on 1 mol of the compound [I-1].

The reaction temperature of the present reaction may be selected from a range of any temperature from 0°C to a reflux temperature in a reaction system, and may be preferably in a range of 0°C to 150°C.

The reaction time of the present reaction is usually 10 minutes to 72 hours although it varies depending on the reaction temperature, the reactant, the reaction amount, and the like.

After the completion of the reaction, the compound [I-3] can be isolated by concentrating the reaction mixture, or subjecting the reaction mixture to operations such as concentration, pouring into water, extraction with an organic solvent, and concentration. The isolated compound [I-3] can be further purified by column chromatography, recrystallization, or the like, as necessary.

<Production Method 10>

A compound of formula [IV-1] can be produced, for example, according to a method based on the reaction scheme exemplified below.

[Chem. 13]

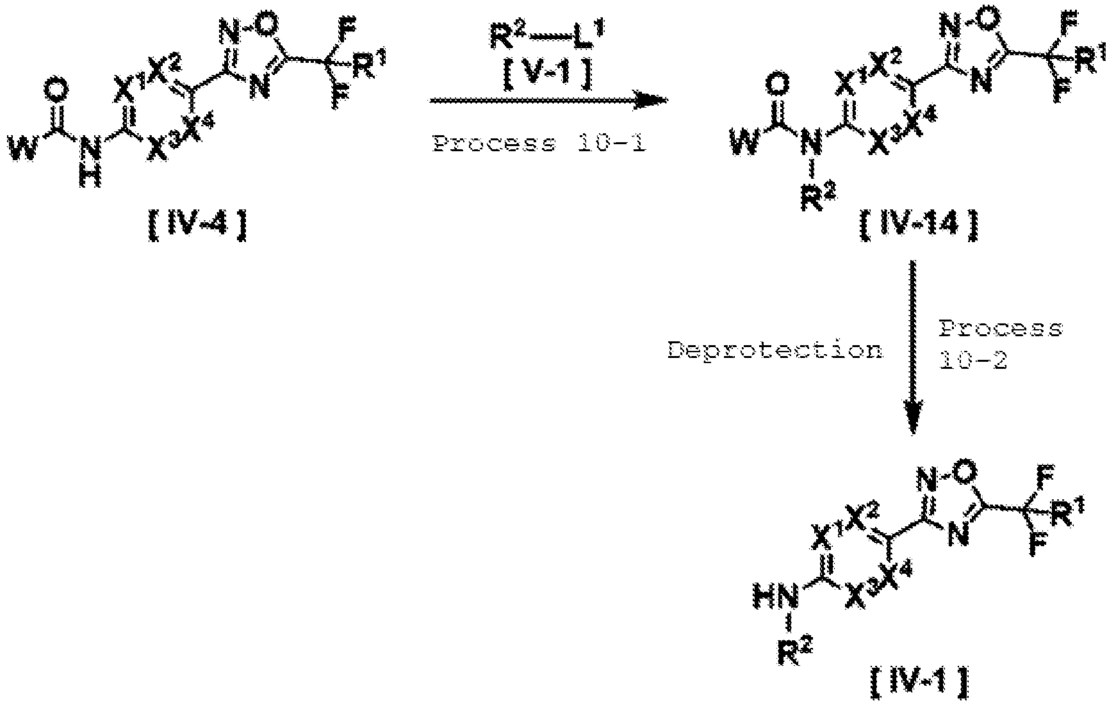


(in the formula, $L^1$, W, $X^1$, $X^2$, $X^3$, $X^4$, $R^1$, and $R^2$ are as defined above.)

(Process 10-1)

That is, the compound of formula [IV-14] can be produced by reacting a compound [IV-4] and a compound [V-1] in the presence or absence of an appropriate solvent, in the presence or absence of an appropriate base, and in the presence or absence of an appropriate catalyst.

The amount of the compound [V-1] used in the present reaction may be appropriately selected from a range of usually 1.0 to 20 mol, and is preferably 1.0 to 3.0 mol, based on 1 mol of the compound [IV-4].

The base, the catalyst, the solvent, the reaction temperature, and the reaction time that can be used in the present reaction are the same as those in Process 1-1 of Production Method 1.

After the completion of the reaction, the compound [IV-14] can be isolated by subjecting the reaction mixture to operations such as pouring into water, extraction with an organic solvent, and concentration. The isolated compound [IV-14] can be further purified by column chromatography, recrystallization, or the like, as necessary.

(Process 10-2)

That is, the compound of formula [IV-1] can be produced from the compound [IV-14] in accordance with the method described in GREEN'S PROTECTIVE GROUPS in Organic Synthesis; 5th Edition (John Wiley and Sons, Peter G.M. Wuts, 2014).

The compound [IV-1] can be further purified by column chromatography, recrystallization, or the like, as necessary.

<Production Method 1 of Intermediate>

A compound of formula [II-1] among the compounds of the present invention can be produced, for example, according to the following methods.

[Chem. 14]

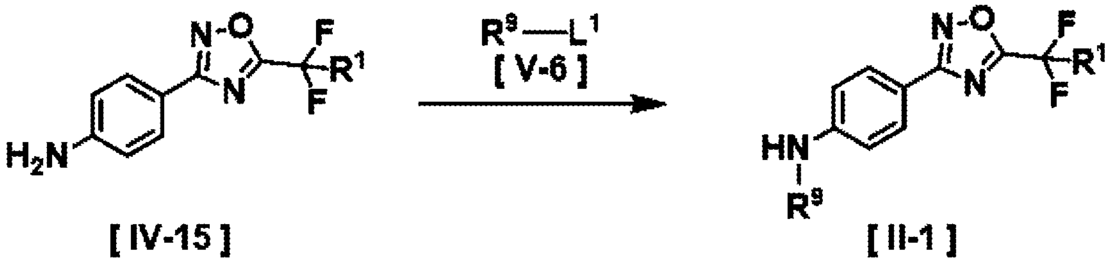


[IV-15] [II-1]

(in the formula, $L^1$, $R^1$, and $R^9$ are as defined above.)

That is, the compound of formula [II-1] can be produced by reacting a compound [IV-15] and a compound [V-6] in the presence or absence of an appropriate solvent, in the presence or absence of an appropriate base, and in the presence or absence of an appropriate catalyst.

The amount of the compound [V-6] used in the present reaction may be appropriately selected from a range of usually 1.0 to 20 mol, and is preferably 1.0 to 3.0 mol, based on 1 mol of the compound [IV-15].

The base, the catalyst, and the solvent that can be used in the present reaction are the same as those in Process 1-1 of Production Method 1.

The reaction temperature of the present reaction may be selected from a range of any temperature usually from -78°C to a reflux temperature in a reaction system, and may be preferably in a range of 0°C to 150°C.

The reaction time of the present reaction is usually 10 minutes to 72 hours although it varies depending on the reaction temperature, the reactant, the reaction amount, and the like.

After the completion of the reaction, the compound [II-1] can be isolated by subjecting the reaction mixture to operations such as pouring into water, extraction with an organic solvent, and concentration. The isolated compound [II-1] can be further purified by column chromatography, recrystallization, or the like, as necessary.

<Production Method 2 of Intermediate>

A compound of formula [II-2] among the compounds of the present invention can be produced, for example, according to a method based on the reaction scheme exemplified below.

[Chem. 15]

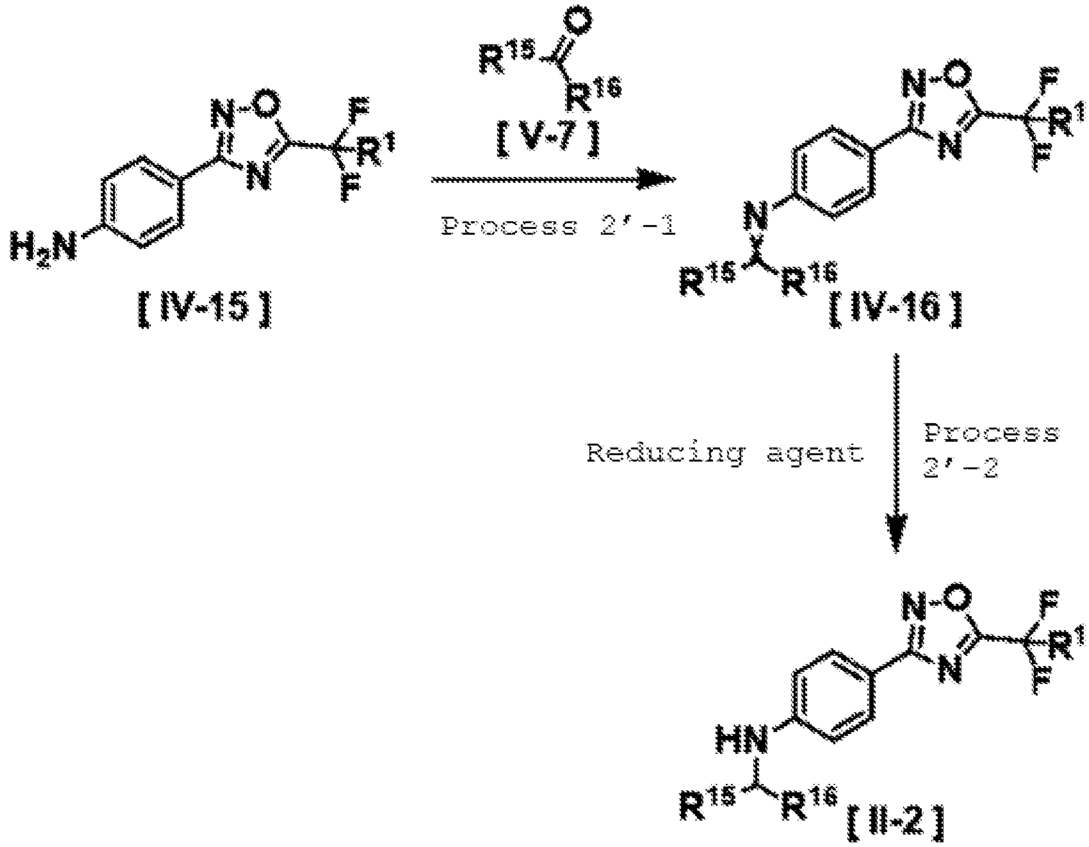


(in the formula, $R^{15}$ represents a hydrogen atom, $R^{16}$ represents a (C1-C5)alkyl mono-substituted or poly-substituted with substituent(s) selected from a substituent group $R^{10}$, a (C2-C5)alkenyl mono-substituted or poly-substituted with substituent(s) selected from a substituent group $R^{11}$, a (C2-C5)alkynyl mono-substituted or poly-substituted with substituent(s) selected from a substituent group $R^{12}$, a (C3-C6)cycloalkyl, a (C6-C12)aryl (the (C6-C12)aryl is optionally mono-substituted or poly-substituted with $R^5$), a 3- to 6-membered heterocyclyl (the 3- to 6-membered heterocyclyl is optionally mono-substituted or poly-substituted with $R^5$), or a 5- to 12-membered heteroaryl (the 5- to 12-membered heteroaryl is optionally mono-substituted or poly-substituted with $R^5$), and $R^1$ is as defined above.)

(Process 2'-1)

That is, the compound of formula [IV-16] can be produced by reacting a compound of formula [IV-15] and a compound [V-7] in the presence or absence of an appropriate solvent and in the presence or absence of an appropriate acid catalyst.

The amount of the compound [V-7] used in the present reaction may be appropriately selected from a range of usually 1.0 to 20 mol, and is preferably 1.0 to 5.0 mol, based on 1 mol of the compound [IV-15].

The acid catalyst, the solvent, the reaction temperature, and the reaction time that can be used in the present reaction are the same as those in Process 6-1 of Production Method 6.

After the completion of the reaction, the compound [IV-16] can be isolated by subjecting the reaction mixture to operations such as pouring into water, extraction with an organic solvent, and concentration. The isolated compound [IV-16] can be further purified by column chromatography, recrystallization, or the like, as necessary.

(Process 2'-2)

That is, the compound of formula [II-2] can be produced by reacting a compound of formula [IV-16] and an appropriate reducing agent in the presence or absence of an appropriate solvent and in the presence or absence of an appropriate acid catalyst.

The reducing agent, the acid catalyst, the solvent, the reaction temperature, and the reaction time that can be

used in the present reaction are the same as those in Process 6-2 of Production Method 6.

After the completion of the reaction, the compound [II-2] can be isolated by subjecting the reaction mixture to operations such as pouring into water, extraction with an organic solvent, and concentration. The isolated compound [II-2] can be further purified by column chromatography, recrystallization, or the like, as necessary.

<Production Method 3 of Intermediate>

A compound of formula [II-1] among the compounds of the present invention can be produced, for example, according to a method based on the reaction scheme exemplified below.

[Chem. 16]

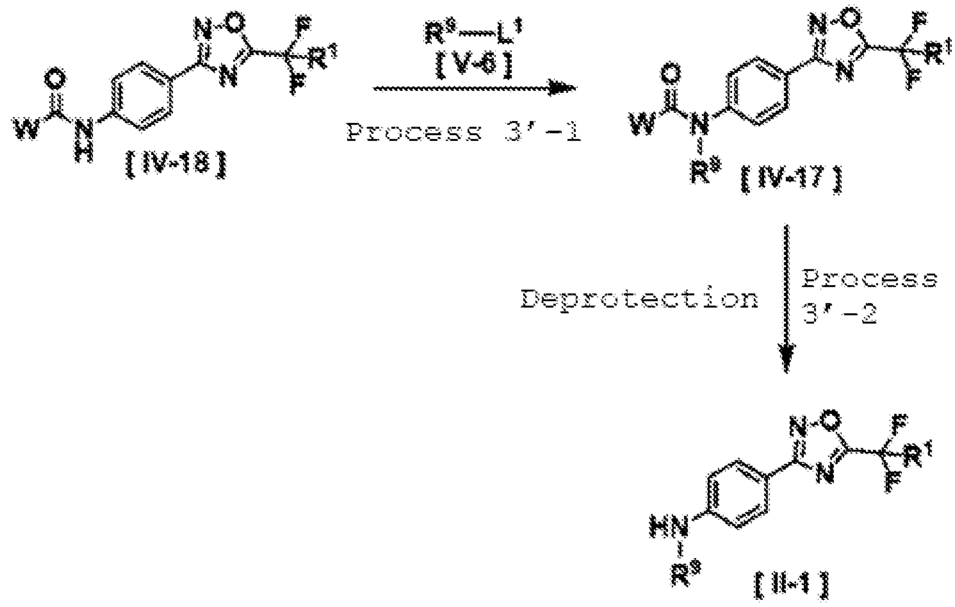


(in the formula, $L^1$, W, $R^1$, and $R^9$ are as defined above.)

(Process 3'-1)

That is, the compound of formula [IV-17] can be produced by reacting a compound [IV-18] and a compound [V-6] in the presence or absence of an appropriate solvent, in the presence or absence of an appropriate base, and in the presence or absence of an appropriate catalyst.

The amount of the compound [V-6] used in the present reaction may be appropriately selected from a range of usually 1.0 to 20 mol, and is preferably 1.0 to 3.0 mol, based on 1 mol of the compound [IV-18].

The base, the catalyst, the solvent, the reaction temperature, and the reaction time that can be used in the present reaction are the same as those in Process 1-1 of Production Method 1.

After the completion of the reaction, the compound [IV-17] can be isolated by subjecting the reaction mixture to operations such as pouring into water, extraction with an organic solvent, and concentration. The isolated compound [IV-17] can be further purified by column chromatography, recrystallization, or the like, as necessary.

(Process 3'-2)

That is, the compound of formula [II-1] can be produced from the compound [IV-17] in accordance with the method described in GREEN'S PROTECTIVE GROUPS in Organic Synthesis; 5th Edition (John Wiley and Sons, Peter G.M. Wuts, 2014).

The compound [II-1] can be further purified by column chromatography, recrystallization, or the like, as necessary.

<Production Method 4 of Intermediate>

A compound of formula [III-1] among the compounds of the present invention can be produced, for example, according to the following methods.

[Chem. 17]

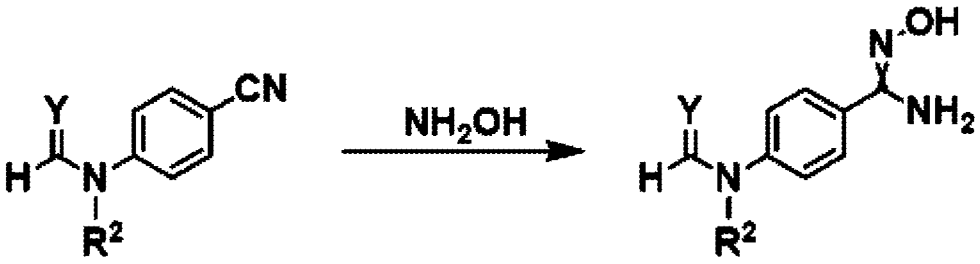


[IV-19]

[III-1]

(in the formula, Y and $R^2$ are as defined above.)

That is, the compound of formula [III-1] can be produced by reacting [IV-19] and appropriate hydroxylamine in the presence of an appropriate solvent and in the presence or absence of an appropriate base.

The hydroxylamine, the base, the solvent, the reaction temperature, and the reaction time that can be used in the present reaction are the same as those in Production Method 3.

After the completion of the reaction, the compound [III-1] can be isolated by subjecting the reaction mixture to operations such as pouring into water, extraction with an organic solvent, and concentration. The isolated compound [III-1] can be further purified by column chromatography, recrystallization, or the like, as necessary.

The agricultural and horticultural plant disease control agent of the present invention contains the formamide derivative of formula [I] of the present invention or the agriculturally acceptable salt thereof as an active ingredient.

The agricultural and horticultural plant disease control agent of the present invention can contain an additive component (carrier) that is usually used in an agrochemical formulation, as necessary.

Examples of the additive component include a carrier such as a solid carrier or a liquid carrier, a surfactant, a binder or a tackifier, a thickener, a coloring agent, a spreader, a sticker, an antifreezing agent, an anticaking agent, a disintegrating agent, and a decomposition inhibitor, and in addition, an antiseptic, a plant piece, or the like may be added to the additive component, as necessary. These additive components may be used alone or in combination of two or more thereof.

Hereinafter, the additive components described above are described.

Examples of the solid carrier include mineral carriers such as pyrophyllite clay, kaolin clay, silica clay, talc, diatomite, zeolite, bentonite, Japanese acid clay, activated clay, attapulgus clay, vermiculite, pearlite, pumice, white carbon (synthetic silicic acid, synthetic silicate, or the like), and titanium dioxide; vegetal carriers such as woody powder, corn haulm, walnut shell, fruit stone, chaff, sawdust, bran, soybean meal, powdered cellulose, starch, dextrin, and saccgarudes; inorganic salt carriers such as calcium carbonate, ammonium sulfate, sodium sulfate, and potassium chloride; and polymeric carriers such as polyethylene, polypropylene, polyvinyl chloride, polyvinyl acetate, an ethylene-vinyl acetate copolymer, and a urea-aldehyde resin.

Examples of the liquid carrier include monohydric alcohols such as methanol, ethanol, propanol, 2-propanol, butanol, and cyclohexanol; polyhydric alcohols such as ethylene glycol, diethylene glycol, propylene glycol, hexylene glycol, polyethylene glycol, polypropylene glycol, and glycerin; polyhydric alcohol derivatives such as polypropylene glycol ether; ketones such as acetone, methyl ethyl ketone, methyl isobutyl ketone, diisobutyl ketone, cyclohexanone, and isophoron; ethers such as ethyl ether, 1,4-dioxane, cellosolve, dipropyl ether, and tetrahydrofuran; aliphatic hydrocarbons such as normal paraffin, naphthene, isoparaffin, kerosene, and mineral oil; aromatic hydrocarbons such as toluene, $C_9$-$C_{10}$ alkylbenzene, xylene, solvent naphtha, alkyl naphthalene, and high boiling point aromatic hydrocarbons; halogenated hydrocarbons such as 1,2-dichloroethane, chloroform, and carbon tetrachloride; esters such as ethyl acetate, diisopropyl phthalate, dibutyl phthalate, dioctyl phthalate, and dimethyl adipate; lactones such as $\gamma$-butyrolactone; amides such as N,N-dimethylformamide, N,N-diethylformamide, N,N-dimethylacetamide, and N-methyl-2-pyrrolidone; nitriles such as acetonitrile; sulfur compounds such as dimethyl sulfoxide; vegetable oils such as soybean oil, rapeseed oil, cottonseed oil, coconut oil, and castor oil; lower alkyl ester of fatty acid derived from the vegetable oils; and water.

The surfactant is not particularly limited, and is preferably a gel in water or exhibits swellability. Examples of the surfactant include noionic surfactants such as sorbitan fatty acid ester, polyoxyethylene sorbitan fatty acid ester,

sucrose fatty acid ester, polyoxyethylene fatty acid ester, polyoxyethylene resin acid ester, polyoxyethylene fatty acid diester, polyoxyethylene alkyl ether, polyoxyethylene alkylphenyl ether, polyoxyethylene dialkylphenyl ether, polyoxyethylene alkylphenyl ether formalin condensate, polyoxyethylene polyoxypropylene block polymer, alkylpolyoxyethylene polypropylene block polymer ether, polyoxyethylene alkyl amine, polyoxyethylene fatty acid amide, polyoxyethylene fatty acid bisphenyl ether, polyalkylene benzyl phenyl ether, polyoxyalkylene styryl phenyl ether, acetylene diol, polyoxyalkylene-added acetylene diol, polyoxyethylene ether silicone, ester silicone, fluorine surfactant, polyoxyethylene castor oil, and polyoxyethylene hardened castor oil; anionic surfactants such as alkyl sulfates, polyoxyethylene alkyl ether sulfates, polyoxyethylene alkyl phenyl ether sulfates, polyoxyethylene styryl phenyl ether sulfate, alkyl benzenesulfonates, lignin sulfonate, alkyl sulfosuccinates, naphthalene sulfonate, alkylnaphthalene sulfonates, salts of formalin condensate of naphthalene sulfonate, salts of formalin condensate of alkylnaphthalene sulfonate, fatty acid salts, polycarboxylates, N-methyl-fatty acid sarcosinates, resinates, polyoxyethylene alkyl ether phosphates, and polyoxyethylene alkyl phenyl ether phosphates; cationic surfactants such as alkyl amine salts such as lauryl amine hydrochloride, stearyl amine hydrochloride, oleyl amine hydrochloride, stearyl amine acetate, stearyl aminopropylamine acetate, alkyl trimethyl ammonium chlorides, and alkyl dimethylbenzarconium chloride; and amphoteric surfactants including a betaine type such as dialkyl diaminoethyl betaine and alkyl dimethylbenzyl betaine, and an amino acid type such as dialkylaminoethyl glycine and alkyl dimethyl benzyl glycine.

Examples of the binder or the tackifier include carboxymethyl cellulose or a salt thereof, dextrin, soluble starch, xanthan gum, guar gum, sucrose, polyvinylpyrrolidone, gum arabic, polyvinyl alcohol, polyvinyl acetate, sodium polyacrylate, polyethylene glycol having an average molecular weight of 6,000 to 20,000, polyethylene oxide having an average molecular weight of 100,000 to 5,000,000, and naturally-occurring phospholipid (for example, cephalin acid, lecithin, or the like).

Examples of the thickener include water-soluble polymers such as xanthan gum, guar gum, carboxymethylcellulose, polyvinylpyrrolidone, carboxyvinyl polymers, acrylic polymers, starch derivatives, and polysaccharides; and inorganic fine powders such as high purity bentonite and white carbon.

Examples of the coloring agent include inorganic pigments such as iron oxide, titanium oxide, and Prussian blue; and organic dyes such as an alizarin dye, an azo dye, and a metal phthalocyanine dye.

Examples of the spreader include a silicone-based surfactant, cellulose powder, dextrin, processed starch, a polyaminocarboxylic acid chelate compound, crosslinked polyvinylpyrrolidone, a maleic acid/styrene copolymer, a methacrylic acid copolymer, a half ester of a polyhydric alcohol polymer and a dicarboxylic acid anhydride, a water-soluble salt of polystyrenesulfonic acid, polyoxyethylene alkanediols, polyoxyethylene alkynediols, and alkynediols.

Examples of the sticker include various surfactants such as sodium dialkyl sulfosuccinate, polyoxyethylene alkyl ether, polyoxyethylene alkylphenyl ether, and polyoxyethylene fatty acid ester; paraffin, terpene, a polyamide resin, polyacrylate, polyoxyethylene, wax, polyvinylalkyl ether, an alkylphenol formalin condensate, and a synthetic resin emulsion.

Examples of the antifreezing agent include polyhydric alcohols such as ethylene glycol, diethylene glycol, propylene glycol, and glycerin.

Examples of the anticaking agent include polysaccharides such as starch, alginic acid, mannose, and galactose; polyvinylpyrrolidone, white carbon, ester gum, and a petroleum resin.

Examples of the disintegrating agent include sodium tripolyphosphate, sodium hexametaphosphate, a stearic metal salt, cellulose powder, dextrin, a copolymer of methacrylic ester, polyvinylpyrrolidone, a polyaminocarboxylic chelate compound, a styrene sulfonate-isobutylene-maleic anhydride copolymer, and a starch-polyacrylonitrile graft copolymer.

Examples of the decomposition inhibitor include a desiccant such as zeolite, calcined lime, or magnesium oxide; a phenolic, amine-based, sulfur-based, or phosphoric acid-based antioxidant; and a salicylic acid-based or benzophenone-based ultraviolet absorber.

Examples of the antiseptic include potassium sorbate and 1,2-benzothiazol-3(2H)-one.

Examples of the plant piece include sawdust, coconut shellflower, corncob, and tobacco stem.

Meanwhile, in the agricultural and horticultural plant disease control agent of the present invention, when the additive component is contained, a content thereof is selected usually in a range of 5 to 95%, and preferably in a range of 20 to 90% in a case of a carrier such as a solid carrier or a liquid carrier, is selected usually in a range of 0.1 to 30%, and preferably in a range of 0.5 to 10% in a case of a surfactant, and is selected usually in a range of 0.1 to 30%, and preferably in a range of 0.5 to 10% in a case of other additives, on a mass basis.

The agricultural and horticultural plant disease control agent of the present invention is used in any formulation such as dusts, dusts and granules, granules, wettable powders, water-soluble powders, wettable granules, tablets, Jumbo formulations, emulsifiable concentrates, oils, solutions, flowable concentrates, emulsions, microemulsions, suspoemulsions, ultra-low volume formulations, microcapsules, smoking formulations, aerosols, baiting formulations, and pastes.

In actual use of the formulation, the formulation can be used as it is or after dilution with a diluent such as water

in a predetermined concentration. Application of various formulations containing the compound of the present invention or dilution products thereof can be conducted by a method ordinarily used, that is, dispersion (for example, spraying, misting, atomizing, powder dispersion, granule dispersion, on-water-surface dispersion, or inbox dispersion), in-soil application (for example, mixing or drenching), on-surface application (for example, coating, dust coating, or covering), seed treatment (for example, smearing or dressing treatment), immersion, poison bait, or smoking. It is also possible to mix the active ingredient with a livestock feed in order to control harmful insects in excreta of livestock, particularly outbreak and growth of harmful pests.

A method for controlling an agricultural and horticultural plant disease of the present invention can be performed by using the amount of the active ingredient of an alkoxyalkoxybenzene derivative having a sulfur-containing substituent of the present invention or an agriculturally acceptable salt thereof by the application method described above.

The mixing proportion (% by mass) of the active ingredient in the agricultural and horticultural plant disease control agent of the present invention is appropriately selected, as necessary. For example, the mixing proportion may be appropriately selected in the following ranges: 0.01 to 20% and preferably 0.05 to 10% for dusts, dusts and granules, microgranules, or the like; 0.1 to 30% and preferably 0.5 to 20% for granules or the like; 1 to 70% and preferably 5 to 50% for wettable powders, wettable granules, or the like; 1 to 95% and preferably 10 to 80% for water-soluble powders, solutions, or the like; 5 to 90% and preferably 10 to 80% for emulsifiable concentrates or the like; 1 to 50% and preferably 5 to 30% for oils or the like; 5 to 60% and preferably 10 to 50% for flowable concentrates or the like; 5 to 70% and preferably 10 to 60% for emulsions, microemulsions, suspoemulsions, or the like; 1 to 80% and preferably 5 to 50% for tablets, baiting formulations, pastes, or the like; 0.1 to 50% and preferably 1 to 30% for smoking formulations or the like; and 0.05 to 20% and preferably 0.1 to 10% for aerosols or the like.

The formulation is sprayed after dilution in an appropriate concentration, or is applied directly.

When the agricultural and horticultural plant disease control agent of the present invention is used after dilution with a diluent, the concentration of the active ingredient is generally 0.1 to 5,000 ppm. When the formulation is used as it is, the application amount thereof per unit area is 0.1 to 5,000 g per 1 ha in terms of the active ingredient compound; however, the application amount is not limited thereto.

It goes without saying that the agricultural and horticultural plant disease control agent of the present invention is sufficiently effective even when the compound of the present invention is used alone as an active ingredient, and the agricultural and horticultural plant disease control agent of the present invention may be mixed or used in combination, as necessary, with other fertilizers and pesticides such as a bactericide, an antiviral agent, an insecticide, a miticide, a nematicide, a synergist, an attractant, a herbicide, and a plant growth regulator. In this case, a more excellent effect can be exhibited.

Next, examples of known bactericides (bactericidal active ingredients) or disease control agent compounds that can be mixed or used in combination are shown below.

Bactericidal active ingredients or disease control agent compounds:

Agrobacterium radiobacter, azaconazole, acibenzolar-S-methyl, azoxystrobin, anilazine, amisulbrom, aminopyrifen, ametoctradin, aldimorph, isotianil, isopyrazam, isofetamid, isoflucypram, isoprothiolane, ipconazole, ipflufenoquin, ipfentrifluconazole, iprodione, iprovalicarb, iprobenfos, imazalil, iminoctadine-albesilate, iminoctadine-triacetate, imibenconazole, inpyrfluxam, imprimatin A, imprimatin B, edifenphos, etaconazole, ethaboxam, ethirimol, ethoxyquin, etridiazole, enestroburin, enoxastrobin, epoxiconazole, organic oils, oxadixyl, oxazinylazole, oxathiapiprolin, oxycarboxin, oxine-copper, oxytetracycline, oxpoconazole-fumarate, oxolinic acid, copper dioctanoate, octhilinone, ofurace, orysastrobin, o-phenylphenol, kasugamycin, captafol, carpropamid, carbendazim, carboxin, carvone, Candida oleophila, Candida saitoana, quinoxyfen, quinofumelin, chinomethionat, captan, quinconazole, quintozene, guazatine, cufraneb, coumethoxystrobin, coumoxystrobin, Gliocradium catenulatum, Cryptococcus albidus, kresoxim-methyl, clozylacon, Clonostachys rosea, chlozolinate, chloroinconazide, chlorothalonil, chloroneb, Chaetomium cupreum, Coniothyrium minitans, cyazofamid, diethofencarb, diclocymet, dichlofluanid, dichlobentiazox, diclomezine, dicloran, dichlorophen, dithianon, diniconazole, diniconazole-M, zineb, dinocap, dipymetitrone, diphenylamine, difenoconazole, cyflufenamid, diflumetorim, cyproconazole, cyprodinil, simeconazole, dimethirimol, dimethyl disulfide, dimethomorph, cymoxanil, dimoxystrobin, Pseudozyma flocculosa, Pseudomonas aureofaciens, Pseudomonas chlororaphis, Pseudomonas syringae, Pseudomonas flurorescens, Pseudomonas rhodesiae, ziram, silthiofam, Zucchini yellow mosaic virus, streptomycin, Streptomyces griseoviridis, Streptomyces lygicus, spiroxamine, sedaxane, seboctylamine, zoxamide, solatenol, dazomet, Talaromyces flavus, tiadinil, thiabendazole, thiram, thiophanate, thiophanate-methyl, thifluzamide, thiram, tecnazene, tecloftalam, tetraconazole, debacarb, tebuconazole, tebufloquin, terbinafine, dodine, dodemorph, triadimenol, triadimefon, triazoxide, trichlamide, triclopyricarb, Trichoderma asperellum, Trichoderma atroviride, Trichoderma gamsii, Trichoderma stromaticum, Trichoderma paecilomyces, Trichoderma harzianum, Trichoderma viride, Trichoderma virens, Trichoderma polysporum, Trichoderma harzianum rifai, Trichoderma lignorum, tricyclazole, triticonazole, tridemorph, triflumizole, trifloxystrobin, triforine, tolylfluanid, tolclofos-methyl, tolnifanide, tolprocarb, nabam, natamycin, naftifine, nitrapyrin, nitrothal-isopropyl, nuarimol, copper nonyl phenol sulphonate, Paenibacillus polymyxa, Barkholderia cepacia, Bacillus amyloliquefaciens, Bacillus simplex, Bacillus subtilis, Bacillus pumilus, Bacillus mycoides, Bacillus licheniformis,

harpin protein, Variovorax paradoxus, validamycin, valifenalate, Pantoea agglomerans, picarbutrazox, bixafen, picoxystrobin, Pythium oligandrum, pydiflumetofen, bitertanol, binapacryl, hinokitiol, Erwinia carotovora, Rhizobium vitis, biphenyl, piperalin, hymexazol, pyraoxystrobin, pyraclostrobin, pyraziflumid, pyrazophos, pyrapropoyne, pyrametostrobin, pyriofenone, pyrisoxazole, pyridachlometyl, pyrifenox, pyributicarb, pyribencarb, pyrimethanil, pyroquilon, vinclozolin, ferbam, famoxadone, phenazine oxide, fenamidone, fenaminstrobin, fenarimol, fenoxanil, ferimzone, fenpiclonil, fenpicoxamid, fenpyrazamine, fenbuconazole, fenfuram, fenpropidin, fenpropimorph, fenhexamid, folpet, phthalide, Fusarium oxysporum, bupirimate, fuberidazole, blasticidin-S, furametpyr, furalaxyl, furancarboxylic acid, fluazinam, fluindapyr, fluoxastrobin, fluoxapiprolin, fluoxytioconazole, fluopicolide, fluopimomide, fluopyram, fluoroimide, fluxapyroxad, fluquinconazole, furconazole, furconazole -cis, fludioxonil, flusilazole, flusulfamide, flutianil, flutolanil, flutriafol, flufenoxadiazam, flufenoxystrobin, flubeneteram, flumetylsulforim, flumetover, flumorph, Phlebiopsis gigantea, proquinazid, prochloraz, procymidone, prothiocarb, prothioconazole, bronopol, propamocarb-hydrochloride, propiconazole, propineb, probenazole, bromuconazole, flometoquin, florylpicoxamid, hexaconazole, benalaxyl, benalaxyl-M, benodanil, benomyl, pefurazoate, penconazole, pencycuron, benzovindiflupyr, benthiazole, benthiavalicarb-isopropyl, penthiopyrad, penflufen, boscalid, fosetyl (including a salt of aluminum, calcium, sodium, or the like), polyoxin, polycarbamate, Bordeaux mixture, mancopper, mancozeb, mandipropamid, mandestrobin, maneb, myclobutanil, mineral oils, mildiomycin, methasulfocarb, metam, metalaxyl, metalaxyl-M, metarylpicoxamid, metiram, metyltetraprole, metconazole, metominostrobin, metrafenone, mepanipyrim, mefentrifluconazole, meptyldinocap, mepronil, iodocarb, laminarin, phosphorous acid and salts, copper oxychloride, silver, copper(II) sulfate, cuprous oxide, copper hydroxide, potassium bicarbonate, sodium bicarbonate, sulfur, oxyquinoline sulfate, copper sulfate, UK-2A (code number), dodecylbenzenesulfonic acid bisethylenediamine copper[II] salt (DBEDC), triphenyltin acetate (TPTA), triphenyltin chloride (TPTC), triphenyltin hydroxide (TPTH), 6-chloro-N-[2-(2,4-dimethylphenyl)-2,2-difluoro-ethyl]-3-[3-(trifluoromethyl)phenoxy]pyridazine-4-carboxamide (Chemical name, CAS registry number: 2435594-93-5) (WO 2020/109391 A), rac-3-[6-chloro-3-[3-(trifluoromethyl)phenoxy]pyridazin-4-yl]-5-[(2,4-dimethylphenyl)methyl]-5,6-dihydro-4H-1,2,4-oxadiazine) (Chemical name, CAS registry number: 2446124-68-9) (WO 2020/127780 A), 6-chloro-N-[2-(2,4-dimethylphenyl)-2,2-difluoroethyl]-5-methyl-3-[3-(trifluoromethyl)phenoxy]pyridazine-4-carboxamide) (Chemical name, CAS registry number: 2435603-06-6) (WO 2020/109391 A), 6-chloro-N-[2-(2,4-dimethylphenyl)ethyl]-3-[3-(trifluoromethyl)phenoxy]pyridazine-4-carboxamide) (Chemical name, CAS registry number: 2435600-09-0) (WO 2020/109391 A), 6-chloro-N-[2-(2,4-dimethylphenyl)ethyl]-5-methyl-3-[3-(trifluoromethyl)phenoxy]pyridazine-4-carboxamide) (Chemical name, CAS registry number: 2435603-15-7) (WO 2020/109391 A), and rac-3-[6-chloro-5-methyl-3-[3-(trifluoromethyl)phenoxy]pyridazin-4-yl]-5-[(2,4-dimethylphenyl)methyl]-5,6-dihydro-4H-1,2,4-oxadiazine) (Chemical name, CAS registry number: 2446123-98-2) (WO 2020/127780 A).

Next, examples of known insecticides (insecticidal active ingredients), miticides (miticidal active ingredients), nematicides (nematicidal active ingredients), and synergistic compounds (synergistic active ingredients) that may be mixed or used in combination are shown below.

Insecticidal active ingredients, miticidal active ingredients, nematicidal active ingredient, and synergistic active ingredients:

acrinathrin, azadirachtin, azamethiphos, acynonapyr, azinphos-ethyl, azinphos-methyl, acequinocyl, acetamiprid, acetoprole, acephate, azocyclotin, abamectin, afidopyropen, afoxolaner, amidoflumet, amitraz, alanycarb, aldicarb, aldoxycarb, allethrin) [including d-cis-trans-body and d-trans-body], isazophos, isamidofos, isocarbophos, isoxathion, isocycloseram, isofenphos-methyl, isoprocarb, epsilon-metofluthrin, epsilon-momfluorothrin, ivermectin, imicyafos, imidacloprid, imiprothrin, indazapyroxamet, indoxacarb, esfenvalerate, ethiofencarb, ethion, ethiprole, ethylene dibromide, etoxazole, etofenprox, ethoprophos, etrimfos, emamectin, emamectin benzoate, endosulfan, empenthrin, oxazosulfyl, oxamyl, oxydemeton-methyl, oxydeprofos, omethoate, Nuclear polyhedrosis virus, cadusafos, kappa-tefluthrin, kappa-bifenthrin, karanjin, cartap, Granulosis virus, carbaryl, carbosulfan, carbofuran, gamma-BHC, xylylcarb, quinalphos, kinoprene, chinomethionat, Entero virus, coumaphos, cryolite, clothianidin, clofentezine, chromafenozide, chlorantraniliprole, chlorethoxyfos, chlordane, chloropicrin, chlorpyrifos, chlorpyrifos-methyl, chlorfenapyr, chlorfenvinphos, chlorfluazuron, chlormephos, chloroprallethrin, Entomopoxi virus, Irido virus, cyazypyr, cyanophos, diafenthiuron, diamidafos, cyantraniliprole, cyetpyrafen, dienochlor, cyenopyrafen, dioxabenzofos, diofenolan, Sigma virus, cyclaniliprole, cycloxaprid, dicrotophos, dichlofenthion, cyclobutrifluram, cycloprothrin, dichlorvos, dicloromezotiaz, dicofol, dicyclanil, disulfoton, dinotefuran, dinobuton, cyhalodiamide, cyhalothrin [including gamma-body and lambda-body], cyphenothrin [including (1R)-trans-body], cyfluthrin [including beta-body], diflubenzuron, cyflumetofen, diflovidazin, cyproflanilide, cyhexatin, cypermethrin [including alpha-body, beta-body, theta-body, and zeta-body], dimpropyridaz, dimethyl-2,2,2-trichloro-1-hydroxyethyl phosphate (DEP), dimethylvinphos, dimethoate, dimefluthrin, jasmone, cis-jasmone, jasmonic acid, methyl jasmonate, silafluofen, cyromazine, Steinernema carpocapsae, Steinernema kushidai, Steinernema glaseri, spidoxamat, spinetoram, spinosad, spirodiclofen, spirotetramat, spiropidion, spiromesifen, sulcofuron-sodium, sulfluramid, sulfoxaflor, sulfotep, diazinon, thiacloprid, thiamethoxam, tioxazafen, thiodicarb, thiocyclam, thiosultap, thionazin, thiofanox, thiometon, tyclopyrazoflor, tetrachlorantraniliprole, tetrachlorvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, tralomethrin, transfluthrin, triazamate, triazophos, trichlorfon, Trichoderma asperellum, Trichoderma paecilomyces, Trichoderma harzianum, triflumuron, trifluenfuronate, triflumezopyrim, trimethacarb, tolfenpyrad, naled, nicotine, nicofluprole, nitenpyram, nemadectin, Denso virus, novaluron, noviflumuron, paecilomyces lilacinus, Barkholderia cepacia, Barkholderia rinojensis, Verticillium lecanii, hydroprene, Pasteuria nishizawae, Pasteuria penetrans, Bacillus amyloliquefaciens, Bacillus firmus, Bacillus sphaericus, Bacillus subtillis, Bacillus thuringiensis, insect toxin produced by Bacillus thuringiensis, Bacillus thuringiensis subsp. Aizawai, Bacillus thuringiensis subsp. Israelensis, Bacillus thuringiensis subsp. Kurstaki, Bacillus thuringiensis subsp. Tenebrionis, Bacillus popilliae, Bacillus licheniformis, vamidothion, parathion, parathion-methyl, halfenprox, halofenozide, bioallethrin, bioallethrin S-cyclopentenyl, bioresmethrin, bis-(2-chloro-1-methylethyl)ether (DCIP), bistrifluron, hydramethylnon, bifenazate, bifenthrin, pyflubumide, piperonyl butoxide, pymetrozine, pyraclofos, pyrafluprole, pyridaphenthion, pyridaben, pyridalyl, pyrifluquinazon, pyriprole, pyriproxyfen, pirimicarb, pyrimidifen, pyriminostrobin, pirimiphos-methyl, pyrethrine, famphur, fipronil, fenazaquin, fenamiphos, fenitrothion, fenoxycarb, fenothiocarb, phenothrin [including (1R)-trans-body], fenobucarb, fenthion, phenthoate, fenvalerate, fenpyroximate, fenbutatin oxide, fenpropathrin, fenmezoditiaz, fonofos, sulfuryl fluoride, butocarboxim, butoxycarboxim, buprofezin, furathiocarb, prallethrin, fluacrypyrim, fluazaindolizine, fluazuron, fluensulfone, fluopyram, sodium fluoroacetate, fluxametamide, fluchlordiniliprole, flucycloxuron, flucythrinate, flusulfamide, fluthrin, fluvalinate [including tau-body], flupyradifurone, flupyrazofos, flupyrimin, flufiprole, flufenerim, flufenoxystrobin, flufenoxuron, fluhexafon, flubendiamide, flupentiofenox, flumethrin, fluralaner, flurimfen, prothiofos, protrifenbute, flonicamid, propaphos, propargite, prohydrojasmon, profenofos, broflanilide, profluthrin, propetamphos, propoxur, flometoquin, bromopropylate, hexythiazox, hexaflumuron, Pacilimyces tenuipes, Paecilomyces fumosoroceus, Paecilomyces lilacinus, heptafluthrin, heptenophos, permethrin, benclothiaz, benzpyrimoxan, bensultap, benzoximate, bendiocarb, benfuracarb, Pochonia chlamydosporia, Beauveria tenella, Beauveria bassiana, Beauveria brongniartii, phoxim, phosalone, fosthiazate, fosthietan, phosphamidon, phosmet, polynactins, formetanate, phorate, machine oil, malathion, milbemectin, mecarbam, mesulfenfos, methomyl, metaldehyde, metaflumizone, methamidophos, metham, methiocarb, methidathion, methyl isothiocyanate, methyl bromide, methoxychlor, methoxyfenozide, methothrin, metofluthrin, methoprene, metolcarb, mevinphos, meperfluthrin, Monacrosporium phymatophagum, Monacrosporium phymatophagum, monocrotophos, momfluorothrin, Trichoderma harzianum, litlure-A, litlure-B, aluminium phosphide, zinc phosphide, phosphine, lufenuron, rescalure, resmethrin, lepimectin, rotenone, Cytoplasmic polyhedrosis virus, fenbutatin oxide, calcium cyanide, organotins, nicotine-sulfate, (Z)-11-tetradecenyl=acetate, (Z)-11-hexadecenal, (Z)-11-hexadecenyl=acetate, (Z)-9,12-tetradecadienyl=acetate, (Z)-9-tetradecen-1-ol, (Z,E)-9,11-tetradecadienyl=acetate, (Z,E)-9,12-tetradecadienyl=acetate, 1,1,1-trichloro-2,2-bis(4-chlorophenyl)ethane (DDT), 1,3-dichloropropene, 4,6-dinitro-O-cresol (DNOC), Bt proteins (Cry1Ab, Cry1Ac, Cry1Fa, Cry2Ab, mCry3A, Cry3Ab, Cry3Bb, and Cry34/35Ab1), methyl eugenol, 4-(p-acetoxyphenyl)-2-butanone, (Z)-10-tetradecenyl=acetate, (E,Z)-4,10-tetradecadienyl=acetate, (Z)-8-dodecenyl=acetate, (Z)-11-tetradecenyl=acetate, (Z)-13-icosen-10-one, 14-methyl-1-octadecene, AKD-1193 (code number), BCS-AA10147 (code number), CL900167 (code number), O,O-diethyl-O-[4-(dimethylsulfamoyl)phenyl]-phosphorothioate (DSP), O-ethyl-O-4-(nitrophenyl)phenylphosphonothioate (EPN), RU15525 (code number), XMC, Z-13-icosen-10-one, ZXI8901 (code number), and F4260 (code number).

Next, examples of known herbicide compounds, herbicidal active ingredients, and plant growth regulator compounds that may be mixed or used in combination are shown below, but the present invention is not limited thereto.

Herbicide compounds and herbicidal active ingredients:

ioxynil (including a salt with a lithium salt, a sodium salt, octanoic acid, or the like), aclonifen, acrolein, azafenidin, acifluorfen (including a salt with sodium or the like), azimsulfuron, asulam, acetochlor, atrazine, anilofos, amicarbazone, amidosulfuron, amitrole, aminocyclopyrachlor, aminopyralid, amiprofos-methyl, ametryn, Araujia Mosaic Virus, alachlor, Alternaria destruens, alloxydim (including a salt with sodium or the like), ancymidol, isouron, isoxachlortole, isoxaflutole, isoxaben, isodecylalkoholethoxylate, isoproturon, ipfencarbazone, imazaquin, imazapic (including a salt with amine or the like), imazapyr (including a salt of isopropylamine or the like), imazamethabenz, imazamethabenz-methyl, imazamox, imazethapyr, imazosulfuron, indaziflam, indanofan, eglinazine-ethyl, esprocarb, ethametsulfuron-methyl, ethalfluralin, ethidimuron, ethoxysulfuron, ethoxyfen, ethoxyfen-ethyl, ethofumesate, etobenzanid, epyrifenacil, endothal-disodium, oxadiazon, oxadiargyl, oxaziclomefone, oxasulfuron, oxyfluorfen, oryzalin, Obuda Pepper Virus, orthosulfamuron, orbencarb, oleic acid, cafenstrole, caprylic acid, capric acid, carfentrazone-ethyl, karbutilate, carbetamide, quizalofop, quizalofop-ethyl, quizalofop-P-ethyl, quizalofop-P-tefuryl, Xanthomonas campestris, quinoclamine, quinclorac, quinmerac, citric acid, cumyluron, clacyfos, glyphosate (including a salt of sodium, potassium, amine, propylamine, isopropylamine, ammonium, isopropylammonium, guanidine, monoethanolamine, choline, N,N-bis(aminopropyl)methylamine (BAPMA), dimethylamine, trimesium, or the like), glufosinate (including a salt of amine, sodium, or the like), glufosinate-P, glufosinate-P-sodium, clethodim, clodinafop, clodinafop-propargyl, clopyralid (including a monoethanolamine salt), clomazone, chlomethoxyfen, clomeprop, cloransulam-methyl, chloramben, chloridazon, chlorimuron, chlorimuron-ethyl, chlorsulfuron, chlorthal-dimethyl, chlorthiamid, chlorphthalim, chlorflurenol-methyl, chlorpropham, chlorbromuron, chloroxuron, chlorotoluron, ketospiradox (including a salt of sodium, calcium, ammonia, or the like), Colletotrichum orbiculare, Colletotrichum gloeosporioides, Colletotrichum truncatum, Chondrostercum purpureum, saflufenacil, sarmentine, cyanazine, cyanamide, diuron, diethatyl-ethyl, dioxopyritrione, dicamba (including a salt of amine, diethylamine, isopropylamine, diglycolamine, dimethylammonium, diolamine, isopropylammonium, auramine, potassium, trolamine, N,N-bis(aminopropyl)methylamine (BAPMA), choline, sodium, lithium, or the like, or ester of methyl ester or the like), cycloate, cycloxydim, diclosulam, cyclosulfamuron, cyclopyranil, cyclopyrimorate, dichlobenil, diclofop, diclofop-P-methyl, diclofop-methyl, dichlorprop, dichlorprop-P (including a salt of dimethylammonium, potassium, sodium, choline, or the like, or ester of butotyl ester, 2-ethylhexyl ester, isoctyl ester, methyl ester, or the like), diquat, diquat dibromide, dithiopyr, siduron, dinitramine, cinidon-ethyl, cinosulfuron, dinoseb (including acetate), dinoterb, cyhalofop, cyhalofop-butyl, cypyrafluone, diphenamid, difenzoquat, diflufenican, diflufenzopyr, simazine, dimesulfazet, dimethachlor, dimethametryn, dimethenamid, dimethenamid-P, simetryn, dimepiperate, dimefuron, Pseudomonas fluorescens, cinmethylin, swep, sulcotrione, sulfentrazone, sulfosate, sulfosulfuron, sulfometuron-methyl, sethoxydim, Sclerotinia minor, terbacil, daimuron, thaxtomin A, Tobacco Mild Green Mosaic Tobamovirus, Tobacco Rattle Virus, dalapon, thiazopyr, tiafenacil, thiencarbazone (including a sodium salt, methyl ester, or the like), tiocarbazil, thiobencarb, thidiazimin, thidiazuron, thifensulfuron, thifensulfuron-methyl, desmedipham, desmetryne, tetflupyrolimet, thenylchlor, tebutam, tebuthiuron, epraloxydim, tefuryltrione, terbuthylazine, terbutryn, terbumeton, tembotrione, topramezone, tralkoxydim, triaziflam, triasulfuron, triafamone, tri-allate, trietazine, triclopyr, triclopyr-butotyl, triclopyr-triethylammonium, tritosulfuron, tripyrasulfone, trifludimoxazin, triflusulfuron-methyl, trifluralin, trifloxysulfuron (including a salt of sodium), tribenuron-methyl, tolpyralate, naptalam (including a salt of sodium), naproanilide, napropamide, napropamide-M, nicosulfuron, lactic acid, neburon, norflurazon, Barkholderia rinojensis, vernolate, paraquat, paraquat dichloride, halauxifen, halauxifen-benzyl, halauxifen-methyl, Burkholderia rinojensis, haloxyfop, haloxyfop-P, haloxyfop-etotyl, haloxyfop-P-methyl, halosafen, halosulfuron-methyl, bixlozone, picloram (including a salt of dichloroammonium, trolamine, or the like), picolinafen, bicyclopyrone, bispyribac-sodium, pinoxaden, bipyrazone, bifenox, piperophos, pyraclonil, pyrasulfotole, pyrazoxyfen, pyrazosulfuron-ethyl, pyrazolynate, bilanafos, pyraflufen, pyraflufen-ethyl, pyridafol, pyrithiobac-sodium, pyridate, pyriftalid, pyributicarb, pyribenzoxim, pyrimisulfan, pyriminobac-methyl, pyroxasulfone, pyroxsulam, Phytophthora palmivora, phenisopham, fenuron, fenoxasulfone, fenoxaprop (including methyl, ethyl, or isopropyl ester), fenoxaprop-P (including methyl, ethyl, or isopropyl ester), fenquinotrione, fenthiaprop-ethyl, fentrazamide, fenpyrazone, phenmedipham, Phoma chenopodicola, Phoma herbarum, Phoma macrostoma, butachlor, butafenacil, butamifos, butylate, Puccinia canaliculata, Puccinia thlaspeos, butenachlor, butralin, butroxydim, flazasulfuron, flamprop (including methyl, ethyl, or isopropyl ester), flamprop-M (including methyl, ethyl, or isopropyl ester), primisulfuron, primisulfuron-methyl, fluazifop-butyl, fluazifop-P, fluazifop-P-butyl, fluazolate, fluometuron, fluoroglycofen-ethyl, flucarbazone-sodium, fluchloralin, flucetosulfuron, fluthiacet-methyl, flupyrsulfuron-methyl (including a salt of sodium, calcium, ammonia, or the like), flufenacet, flufenpyr-ethyl, flupropanate (including a sodium salt), flupoxame, flumioxazin, flumiclorac-pentyl, flumetsulam, fluridone, flurtamone, fluroxypyr (including ester of butomethyl, meptyl, or the like, or a salt of sodium, calcium, ammonia, or the like), flurochloridone, pretilachlor, procarbazone (including a salt of sodium or the like), prodiamine, prosulfuron, prosulfocarb, propaquizafop, propachlor, propazine, propanil, propyzamide, propisochlor, propyrisulfuron, propham, profluazol, prohexadione-calcium, propoxycarbazone, propoxycarbazone-sodium, profoxydim, bromacil, brompyrazon, prometryn, prometon, bromoxynil (including ester of butyric acid, octanoic acid, enanthic acid, or the like), bromofenoxim, bromobutide, florasulam, florpyrauxifen, florpyrauxifen-benzyl, hexazinone, pethoxamid, benazolin, benazolin-ethyl, penoxsulam, Pepino Mosaic Virus, heptamaloxyloglucan, beflubutamid, beflubutamid-M, pebulate, pelargonic acid, bencarbazone, benquitrione, benzfendizone, bensulide, bensulfuron, bensulfuron-methyl, benzobicyclon, benzofenap, bentazone, pentanochlor, pendimethalin, pentoxazone, benfluralin, benfuresate, fosamine, fomesafen, foramsulfuron, forchlorfenuron, mecoprop (including a salt of sodium, potassium, isopropylamine, triethanolamine, dimethylamine, diolamine, trolamine, choline, or the like, or ester of ethadyl ester, 2-ethylhexyl ester, isoctyl ester, methyl ester, or the like), mecoprop-P-potassium, mesosulfuron (including ester of methyl or the like), mesotrione, metazachlor, metazosulfuron, methabenzthiazuron, metamitron, metamifop, metam (including a salt of sodium or the like), disodium methanearsonate (DSMA), methiozolin, methyldymuron, metoxuron, metosulam, metsulfuron-methyl, metobromuron, metobenzuron, metolachlor, metribuzin, mepiquat chloride, mefenacet, monosulfuron (including methyl, ethyl, or isopropyl ester), monolinuron, molinate, iodosulfuron, iodosulfulon-methyl-sodium, iofensulfuron, iofensulfuron-sodium, lactofen, lancotrione, linuron, rimisoxafen, rimsulfuron, lenacil, 2,2,2-trichloroacetic acid (TCA) (including a salt of sodium, calcium, ammonia, or the like), 2,3,6-trichlorobenzoic acid (2,3,6-TBA), 2,4,5-trichlorophenoxyacetic acid (2,4,5-T), 2,4-dichlorophenoxyacetic acid (2,4-D) (including a salt of amine, diethylamine, triethanolamine, isopropylamine, dimethylammonium, diolamine, dodecylammonium, heptylammonium, tetradecylammonium, triethylammonium, tris(2-hydroxypropyl)ammonium, trolamine, choline, sodium, lithium, or the like, or ester of butotyl ester, 2-butoxypropyl ester, 2-ethylhexyl ester, methyl ester, ethyl ester, butyl ester, isobutyl ester, octyl ester, pentyl ester, propyl ester, isooctyl ester, isopropyl ester, meptyl ester, tefuryl ester, or the like), 2,4-dichlorophenoxybutyric acid (2,4-DB) (including a salt of amine, diethylamine, triethanolamine, isopropylamine, dimethylammonium, choline, sodium, lithium, or the like, or ester of isoctyl ester or the like), 2-amino-3-chloro-1,4-naphthoquinone (ACN), 2-methyl-4-chlorophenoxyacetic acid (MCPA) (including a salt of sodium, dimethylammonium, choline, or the like, or ester of 2-ethylhexyl ester, isoctyl ester, ethyl ester, or the like), 2-methyl-4-chlorophenoxyacetic acid (MCPB) (including a sodium salt, ethyl ester, or the like), 4-(2,4-dichlorophenoxy)butyric acid (2,4-DB), 4,6-dinitro-O-creso (DNOC) (including a salt of amine, sodium, or the like), (5S)-3-(3,5-difluorophenyl)-N-[rel-(3R,5R)-5-(trifluoromethylsulfonylcarbamoyl)tetrahydrofuran-3-yl]-5-vinyl-4H-isoxazole-5-carboxamide (Chemical name, CAS registry number: 2266183-40-6) (WO 2018/228986 A and WO 2020/114934 A), N4-(2,6-difluorophenyl)-6-(1-fluoro-1-methylethyl)-1,3,5-triazine-2,4-diamine (Chemical name, CAS registry number: 1606999-43-2) (WO 2014/064094 A and WO 2015/162164 A), (5S)-3-(3,5-difluorophenyl)-N-[(3R)-5-(methylsulfonylcarbamoyl)-2,3-dihydrofuran-3-yl]-5-vinyl-4H-isoxazole-5-carboxamide (Chemical name, CAS registry number: 2266190-06-9) (WO 2018/228986 A and WO 2020/114934 A), (5R)-3-(3,5-difluorophenyl)-5-methyl-N-[rel-(3R,5R)-5-(methylsulfonylcarbamoyl)tetrahydrofuran-3-yl]-4H-isoxazole-5-carboxamide (Chemical name, CAS registry number: 2266164-36-5) (WO 2018/228986 A and WO 2020/114934 A), (5R)-3-(3,5-difluorophenyl)-N-[(3R)-5-(methoxycarbamoyl)-2,3-dihydrofuran-3-yl]-5-methyl-4H-isoxazole-5-carboxamide (Chemical name, CAS registry number: 2266170-31-2) (WO 2018/228986 A and WO 2020/114934 A), 4-hydroxy-1-methyl-3-[4-(trifluoromethyl)-2-pyridyl]imidazolidin-2-one (Chemical name, CAS registry number: 1708087-22-2) (WO 2015/059262 A and WO 2018/015476 A), 6-(1-fluorocyclopentyl)-N4-(2,3,5,6-tetrafluorophenyl)-1,3,5-triazine-2,4-diamine (Chemical name, CAS registry number: 1820807-75-7) (WO 2015/162164 A), 6-(1-fluoro-1-methylethyl)-N4-(2,3,5,6-tetrafluorophenyl)-1,3,5-triazine-2,4-diamine (Chemical name, CAS registry number: 1606999-21-6) (WO 2014/064094 A, WO 2015/162164 A), (5S)-3-(3-fluoro-5-methylphenyl)-N-[rel-(3R,5R)-5-(methoxycarbamoyl)tetrahydrofuran-3-yl]-5-vinyl-4H-isoxazole-5-carboxamide (Chemical name, CAS registry number: 2266292-43-5) (WO 2018/228986 A and WO 2020/114934 A), 6-(1-methylcyclobutyl)-N4-(2,3,5,6-tetrafluorophenyl)-1,3,5-triazine-4,4-diamine (Chemical name, CAS registry number: 1607001-97-7) (WO 2014/064094 A and WO 2015/162164 A), AE-F-150944 (code number), IR-6396 (code number), MCPA-thioethyl, SYP-298 (code number), SYP-300 (code number), S-ethyldipropylthiocarbamate (EPTC), S-metolachlor, S-9750 (code number), MSMA, and HW-02 (code number).

Plant growth regulators:

1-naphthylacetamide, 1-methylcyclopropene, 1,3-diphenylurea, 2,3,5-triiodobenzoic acid, 2-methyl-4-chlorophenoxyacetic acid (MCPB) [including a sodium salt, ethyl ester, or the like], 2-(naphthalene-1-yl)acetamide, 2,6-diisopropylnaphthalene, 3-[(6-chloro-4-phenylquinazoline-2-yl)amino]propane-1-ol, 4-oxo-4-(2-phenylethyl)aminobutyric acid (Chemical name, CAS registry number: 1083-55-2), 4-chlorophenoxyacetic acid (4-CPA), 5-aminolevulinic acid hydrochloride, methyl 5-(trifluoromethyl)benzo[b]thiofen-2-carboxylate, aminoethoxyvinylglycine (AVG), n-decanol, anisiflupurin, aviglycine, ancymidol, abscisic acid, isoprothiolane, inabenfide, indole acetic acid, indole butyric acid, uniconazole, uniconazole-P, Ecolyst, ethychlozate, ethephon, epocholeone, calcium chloride, choline chloride, oxine-sulfate, kinetin, calcium peroxide, carvone, calcium formate, cloxyfonac, cloxyfonac-potassium, cloprop, chlormequat, chlormequat-chloride, chlorpropham, choline, cytokinins, oxydized glutathione, cyanamide, sodium cyanate, cyclanilide, dichlorprop (including a salt of dimethylammonium, potassium, sodium, choline, or the like, or ester of butotyl ester, 2-ethylhexyl ester, isoctyl ester, methyl ester, or the like), dichlorprop-P (including a salt of sodium, potassium, dimethylammonium, or the like, or 2-ethylhexyl ester), diquat, diquat dibromide, dikegulac, gibberellinic acid, gibberellin A4, gibberellin A7, dimethipin, sintofen, jasmone, cis-jasmone, jasmonic acid, methyl jasmonate, streptomycin, calcium polysulfide, daminozide, calcium carbonate, thidiazuron, decan-1-ol, triacontanol, triapenthenol, trinexapac-ethyl, tribufos, paclobutrazol, paraffin, bispyribac-sodium, hymexazol, butralin, fluthiacet-methyl, pyraflufen-ethyl, flumetralin, flurprimidol, flurenol, pronitridine, prohydrojasmon, prohexadione-calcium, heptamaloxyloglucan, 6-benzylaminopurine, pendimethalin, forchlorfenuron, formononetin, maleic hydrazide, mepiquat chloride, mefluidide, lipochitooligosaccharides (for example, lipochitooligosaccharides SP104), and calcium sulfate.

Next, examples of known phytotoxicity alleviating compounds that can be mixed or used in combination are shown below:

Phytotoxicity alleviating compounds:

isoxadifen, isoxadifen-ethyl, oxabetrinil, octane-1,8-diamine, cloquintocet, cloquintcet-mexyl, dietholate, cyometrinil, dichlormid, dicyclonone, cyprosulfamide, daimuron, 1,8-naphthalic anhydride, fenchlorazole, fenchlorazole-O-ethyl, fenclorim, furilazole, fluxofenim, flurazole, benoxacor, metcamifen, mephenate, mefenpyr, mefenpyr-ethyl, mefenpyr-diethyl, lower alkyl-substituted benzoic acid, 2,2-dichloro-N-(1,3-dioxan-2-ylmethyl)-N-(2-propenyl)acetamide (PPG-1292), 2-dichloromethyl-2-methyl-1,3-dioxane (MG-191), 3-dichloroacetyl-2,2,5-trimethyl-1,3-oxazolidine (R-29148), 4-dichloroacetyl-1-oxa-4-azaspiro[4.5]decane (AD-67), 4-carboxy-3,4-dihydro-2H-1-benzopyran-4-acetic acid (CL-304415, code number), MON4660 (code number), metcamifen, N1,N2-diallyl-N2-dichloroacetyl glycineamide (DKA-24, code number), 1-bromo-4-[(chloromethyl) sulfonyl]benzene (CSB), 2-propenyl-1-oxa-4-azaspiro[4,5]decan-4-carbodithioate (MG-838, code number), 3-(dichloroacetyl)-2,2-dimethyl-1,3-oxazolidine (R-28725, code number), R-29148 (code number), and 1-(dichloroacetyl)azepane (TI-35, code number).

Next, examples of known biological pesticides that can be mixed or used in combination are shown.

Biological pesticides:

Haplothrips brevitubus, Franklinothrips vespiformis, Diglyphus isaea, Encarsia formosa, Amblyseius cucumeris, Pseudaphycus malinus, Amblyseius womersleyi, Aphidius colemani, Eretmocerus eremicus, Aphidoletes aphidimyza, Amblyseius swirskii, Orius strigicollis, Phytoseiulus persimilis, Amblyseius degenerans, Phytoseiulus persimilis, Orius sauteri, Dacnusa sibirica, Amblyseius californicus, Chrysoperla nipponensis, and Anicetus beneficus.

Next, examples of known agricultural materials that can be mixed or used in combination are shown.

Agricultural materials:

ethylene, hypochlorous acid water (limited to those obtained by electrolyzing hydrochloric acid or an aqueous potassium chloride solution), sodium bicarbonate, vinegar, humic substances, humic acid, fulvic acid, seaweed extract, polysaccharide, amino acid, a microbial material, an animal and plant-derived functional component, microbial metabolite, a microbial activator, a soil spreading agent, a soil permeability control material, a soil water retention material, and a biostimulant.

Next, examples of known agricultural fertilizer components that can be mixed or used in combination are shown below. Examples of the fertilizer include an inorganic fertilizer and an organic fertilizer.

Agricultural fertilizer components:

ammonium chloride, ammonium sulfate, ammonium nitrate, ammonium dihydrogen phosphate, urea ammonium nitrate, urea, lime nitrogen, potassium nitrate, superphosphate of lime, double superphosphate of lime, potassium dihydrogen phosphate, potassium phosphite, potassium chloride, potassium sulfate, potassium carbonate, potassium silicate, oil cake, fish powder, rice bran, bat guano, and fermented chicken manure.

Specific examples of diseases that can be controlled by the agricultural and horticultural plant disease control agent and the method for controlling agricultural and horticultural plant diseases are described below, but the diseases are not limited thereto: blast disease of rice (Pyricularia oryzae), leaf spot (Cochliobolus miyabeanus), sheath blight disease (Rhizoctonia solani), bakanae disease (Gibberella fujikuroi), damping-off (Fusarium spp., Pythium spp., Rhizopus spp., Trichoderma sp.), damping-off disease (Burkholderia plantarii), bacterial leaf spot (Xanthomonas oryzae), bacterial grain rot of rice (Burkholderia glumae), bacterial brown stripe of rice (Acidovorax avenae), bacterial palea browning (Erwinia ananas), powdery mildew of wheat (Erysiphe graminis f.sp. hordei, f.sp. tritici), mottle leaf (Pyrenophora graminea), net blotch (Pyrenophora teres), scab (Gibberella zeae), rust (Puccinia striiformis, Puccinia graminis, Puccinia recondita, Puccinia hordei), snow mold (Typhula incarnata, Typhula ishikariensis, Micronectriella nivalis, Microdochium nivale), loose smut (Ustilago tritici, U. nuda), eye spot (Pseudocercosporella herpotrichoides), leaf blotch (Rhynchosporium secalis), leaf spot (Septoria tritici), glume blotch (Septoria nodorum, Leptosphaeria nodorum), snow mold caused by the genus Pythium (Pythium iwayamai or the like), downy mildew of grape (Plasmopara viticola), powdery mildew (Uncinula necator), sphaceloma scab (Elsinoe ampelina), ripe rot (Glomerella cingulata), rust (Phakopsora ampelopsidis), black rot (Guignardia bidwellii), leaf spot (Phomopsis viticola), powdery mildew of apple (Podosphaera leucotricha), black spot (Venturia inaequalis), leaf spot (Alternaria alternata), rust (Gymnosporangium yamadae), monilinia mali (Sclerotinia mali), brown canker (Valsa ceratospema), ring spot (Physalospora piricola), black spot of pear (Alternaria kikuchiana), black spot (Venturia nashicola), rust (Gymnosporangium haraeanum), blight (Phomopsis fukushii), blight of western pear (Phytophthora cactorum), brown rot of peach (Monilinia fructicola), black spot (Cladosporium carpophilum), phomopsis black rot (Phomopsis sp.), blight (Phytophthora sp.), anthracnose of persimmon (Colletotrichum gloeosporioides), leaf spot (Pseudocercospora kaki, Mycosphaerella nawae), black rot of mandarin (Diaporthe citri), scab (Elsinoe fawcetti), downy mildew of gourds (Pseudoperonospora cubensis), blight (Phytophthora parasitica, Phytophthora melonis, Phytophthora nicotianae, Phytophthora drechsleri, Phytophthora capsici, or the like), powdery mildew (Sphaerotheca fuliginea, Sphaerotheca cucurbitae), anthracnose (Colletotrichum lagenarium), gummy stem blight (Mycosphaerella melonis), fusarium wilt (Fusarium oxysporum), damping-off (Pythium debaryanum), brown spot (Corynespora cassiicola), gray mold (Botrytis cinerea), bacterial blight (Pseudomonas syringae), blight of tomato (Phytophthora infestans), damping-off (Pythium vexans, Rhizoctonia solani), ring spot (Alternaria solani), damping-off (Pythium myriotylum, Pythium dissotocum), anthracnose (Colletotrichum phomoides), leaf mold (Cladosporium fulvum), canker (Corynebacterium michiganense), powdery mildew of eggplant (Sphaerotheca fuliginea), blight (Phytophthora infestans), brown rot (Phytophthora capsici), bacterial wilt (Ralstonia solanacearum), stem rot of rapeseed (Sclerotinia scerotiorum), downy mildew (Peronospora brassicae), black spot of brassicaceae vegetable (Alternaria japonica), silvertop (Pseudocercosporella capsellae), clubroot (Plasmodiophora brassicae), downy mildew (Peronospora brassicae), white blight of green onion (Phytophthora porri), rust (Puccinia allii), soft rot of Chinese cabbage (Erwinia carotovora), root blight of soybean (Phytophthora megasperma), rust (Phakopsora pachyrhizi), stem rot (Sclerotinia sclerotiorum), downy mildew (Peronospora manshurica), purpura (Cercospora kikuchii), sphaceloma scab (Elsinoe glycines), black rot (Diaporthe phaseololum), anthracnose of wax bean (Colletotrichum lindemuthianum), black rust of peanut (Mycosphaerella berkeleyii), brown spot (Mycosphaerella arachidis), powdery mildew of pea (Erysiphe pisi), downy mildew (Peronospora pisi), blight of potato (Phytophthora infestans), early blight (Alternaria solani), net blister blight (Exobasidium reticulatum), white scab (Elsinoe leucospila), rust of tabacco (Alternaria alternata (tabacco pathotype)), powdery mildew (Erysiphe cichoracearum), anthracnose (Colletotrichum tabacum), brown spot of beet (Cercospora beticola), downy mildew (Peronospora schachtii), black spot of rose (Diplocarpon rosae), powdery mildew (Sphaerotheca pannosa), brown spot of chrysanthemum (Septoria chrysanthemi-indici), white rust (Puccinia horiana), powdery mildew of strawberry (Sphaerotheca macularis), blight (Phytophthora nicotianae), fruit soft rot (Pythium ultimum), anthracnose (Colletotrichum fragariae), black Sigatoka disease of banana (Mycosphaerella fijiensis), yellow Sigatoka disease (Mycosphaerella musicola), fusarium wilt (Fusarium oxysporum), gray mold of cucumber, tomato, green bean, strawberry, grape, or the like (Botrytis cinerea), stem rot

(Sclerotinia sclerotiorum), grass brownpatch (Rhizoctonia solani), dollar spot (Sclerotinia homoeocarpa), curvularia leaf spot (Curvularia geniculata), rust (Puccinia zoysiae), helminthosporium leaf spot (Cochliobolus sp.), leaf blotch (Rhynchosporium secalis), root rot (Gaeumannomyces graminis), anthracnose (Colletotrichum graminicola), typhula brown stem rot (Typhula incarnata), typhula black stem rot (Typhula ishikariensis), sclerotinia stem rot (Sclerotinia borealis), fairy ring (Marasmius oreades or the like), and pythium blight (Pythium aphanidermatum or the like).

The agricultural and horticultural plant disease control agent of the present invention also exhibits a control effect on the plant diseases and the like exemplified above, which have acquired resistance to the existing agricultural and horticultural plant disease control agent. Furthermore, the agricultural and horticultural plant disease control agent of the present invention can be used for plants that have acquired characteristics such as pest tolerance, disease tolerance, herbicide tolerance, or the like by gene recombination, new breeding techniques such as genome editing, artificial mating, or the like.

Next, the production methods, the formulation methods, and the applications of the compound of the present invention will be described in detail with reference to the following Examples. However, the present invention is not limited to these Examples. The melting point which is a physical property value of the compound of the present invention was determined by a MP-500V micro melting point measuring apparatus manufactured by Yanaco Corporation. The refractive index was determined by using an Abbe refractometer manufactured by ATAGO CO., LTD. $^1$H NMR spectrum was determined by using JNM-LA400 (400 MHz) or JNM-ECS300 (300 MHz) manufactured by JEOL Ltd. and tetramethylsilane (TMS) as the internal standard.

A method for producing a production intermediate of the compound of the present invention is also described.

Example 1

Production of N-benzyl-N-{4-[5-(trifluoromethyl)-1,2,4-oxadiazol-3-yl]phenyl}formamide (Compound of Present Invention: A-0096)

(1) Production of N-{4-[5-(trifluoromethyl)-1,2,4-oxadiazol-3-yl]phenyl}formamide (Compound of Present Invention: A-0001)

8.70 g (37.96 mmol) of 4-[5-(trifluoromethyl)-1,2,4-oxadiazol-3-yl]phenylamine produced by the method described in Example 188 of WO 2007/111212 A and 2.80 g (41.17 mmol) of sodium formate were dissolved in 100 ml of formic acid, and the mixture was heated and refluxed for 3 hours. The reaction mixture was cooled to room temperature, the solvent was distilled in vacuo, ethyl acetate was added, the organic layer was washed with a saturated sodium bicarbonate aqueous solution and brine, drying with anhydrous magnesium sulfate was performed, and the solvent was distilled in vacuo. The residue was purified by silica gel column chromatography (developing solvent, n-hexane : ethyl acetate = 3 : 1) to obtain 8.30 g of a desired product (yield: 85%).
$^1$H-NMR data (300MHz, DMSO-$d_6$/TMS $\delta$(ppm)): 10.50-10.60 (1H,m), 8.32-9.01 (1H,m), 7.99-8.06 (2H,m), 7.20-7.84 (2H,m)

(2) Production of N-benzyl-N-{4-[5-(trifluoromethyl)-1,2,4-oxadiazol-3-yl]phenyl}formamide (Compound of Present Invention: A-0096)

0.40 g (1.56 mmol) of N-{4-[5-(trifluoromethyl)-1,2,4-oxadiazol-3-yl]phenyl}formamide was dissolved in 20 ml of acetonitrile, and 0.43 g (3.11 mmol) of potassium carbonate, 0.53 g (3.10 mmol) of benzyl bromide, and 0.026 g (0.16 mmol) of potassium iodide were sequentially added at room temperature, and the mixture was stirred for 40 hours. Water was added at room temperature, and extraction was performed with ethyl acetate. Washing was performed with brine, drying with anhydrous sodium sulfate was performed, and the solvent was distilled in vacuo. The residue was purified by silica gel column chromatography (developing solvent, n-hexane : ethyl acetate = 3 : 1) to obtain 0.46 g of a desired product (yield: 85%) .
$^1$H-NMR data (300MHz, $CDCl_3$/TMS $\delta$(ppm)): 8.61-8.73 (1H,m), 8.05-8.12 (2H,m), 7.22-7.38 (7H,m), 4.94-5.08 (2H,m)

Example 2

Production of N-{4-[5-(trifluoromethyl)-1,2,4-oxadiazol-3-yl]phenyl}formamide (Compound of Present Invention: A-0001)

40.00 g (0.12 mol) of t-butyl {4-[5-(trifluoromethyl)-1,2,4-oxadiazol-3-yl]phenyl}carbamate produced by the method described in Example 188 of WO 2007/111212 A and 8.14 g (0.12 mol) of sodium formate were dissolved in 80 ml of formic acid, and the mixture was heated and refluxed for 3 hours. Cooling was performed at room temperature, 200 ml of water was added dropwise, and stirring was performed for 1 hour. The precipitated crystals were collected by

filtration and washed with water. The crystals were dissolved in 250 ml of ethyl acetate, drying with anhydrous magnesium sulfate was performed, and the solvent was distilled in vacuo, thereby obtaining 30.00 g of a desired product (yield: 96%).
$^1$H-NMR data (300MHz, DMSO-$d_6$/TMS δ (ppm)): 10.50-10.60 (1H,m), 8.32-9.01 (1H,m), 7.99-8.06 (2H,m), 7.20-7.84 (2H,m)

Example 3

Production of N-(quinolin-3-ylmethyl)-N-{4-[5-(trifluoromethyl)-1,2,4-oxadiazol-3-yl]phenyl}formamide

(Compound of Present Invention: B-0004)

(1) Production of 1-(quinolin-3-yl)-N-{4-[5-(trifluoromethyl)-1,2,4-oxadiazol-3-yl]phenyl}methenamine

0.40 g (1.75 mmol) of 4-[5-(trifluoromethyl)-1,2,4-oxadiazol-3-yl]phenylamine and 0.27 g (1.72 mmol) of quinolin-3-carbaldehyde were dissolved in 10 ml of toluene, and the mixture was heated and refluxed for 5 hours. The reaction mixture was cooled, and the solvent was distilled in vacuo, thereby obtaining 0.62 g of a desired product (yield: 96%).
$^1$H-NMR data (300MHz, $CDCl_3$/TMS δ (ppm)): 9.52 (1H,d),8.70 (1H,s), 8.62 (1H,d), 8.20 (3H,m), 7.96 (1H,dd), 7.84 (1H,ddd), 7.64 (1H,ddd), 7.39-7.42 (2H,m)

(2) Production of N-(quinolin-3-ylmethyl)-N-{4-[5-(trifluoromethyl)-1,2,4-oxadiazol-3-yl]phenyl}amine (Compound of Present Invention: G-0004)

0.50 g (1.36 mmol) of 1-(quinolin-3-yl)-N-{4-[5-(trifluoromethyl)-1,2,4-oxadiazol-3-yl]phenyl}methenamine was suspended in 10 ml of ethanol, 0.08 g (1.27 mmol) of sodium cyanoborohydride was added, and the mixture was stirred at room temperature for 1 hour. Thereafter, 0.1 ml of acetic acid was added, and the mixture was stirred at room temperature for 3 hours. 30 ml of a saturated sodium bicarbonate aqueous solution was added, the mixture was stirred for 0.5 hours, and extraction with ethyl acetate was performed. The organic layer was washed with brine and dried with anhydrous magnesium sulfate, the solvent was distilled in vacuo, and the residue was purified by silica gel column chromatography (developing solvent, n-hexane : ethyl acetate = 1 : 1), thereby obtaining 0.24 g of a desired product (yield: 48%).
$^1$H-NMR data (300MHz, DMSO-$d_6$/TMS δ (ppm)): 8.94 (1H,d), 8.27 (1H,d), 7.03-7.94 (2H,m), 7.70-7.81 (3H,m), 7.59 (1H,ddd), 7.27 (1H,t), 6.81 (2H,d), 4.60 (2H,d)

(3) Production of N-(quinolin-3-ylmethyl)-N-{4-[5-(trifluoromethyl)-1,2,4-oxadiazol-3-yl]phenyl}formamide

(Compound of Present Invention: B-0004)

0.17 g (0.46 mmol) of N-(quinolin-3-ylmethyl)-N-{4-[5-(trifluoromethyl)-1,2,4-oxadiazol-3-yl]phenylamine and 0.03 g (0.46 mmol) of sodium formate were dissolved in 1.0 ml of formic acid, and the mixture was heated and refluxed for 0.5 hours. Cooling was performed at room temperature, 30 ml of water was added, extraction with ethyl acetate was performed, and washing with a saturated sodium bicarbonate aqueous solution was performed. The organic layer was dried with anhydrous magnesium sulfate, and the solvent was distilled in vacuo, thereby obtaining 0.17 g of a desired product (yield: 93%) .
$^1$H-NMR data (300MHz, DMSO-$d_6$/TMS δ(ppm)): 8.80-8.95 (2H,m), 8.19-8.26 (1H,m), 7.90-8.05 (4H,m), 7.68-7.75 (3H,m), 7.58 (1H,ddd), 5.35 (2H,s)

Example 4

Production of N-[(tetrahydrofuran-2-yl)methyl]-N-{4-[5-(trifluoromethyl)-1,2,4-oxadiazol-3-yl]phenyl}formamide

(Compound of Present Invention: B-0810)

(1) Production of 4-{[(tetrahydrofuran-2-yl)methyl]amino}benzonitrile

3.00 g (24.81 mmol) of 4-fluorobenzonitrile and 2.51 g (24.81 mmol) of (tetrahydrofuran-2-yl)methanamine were dissolved in 30 ml of DMF, 3.43 g (24.81 mmol) of potassium carbonate was added, and the mixture was stirred at 120°C for 5 hours. Cooling was performed at room temperature, water was added, and extraction with ethyl acetate was performed. Drying with anhydrous magnesium sulfate was performed, the solvent was distilled in vacuo, and the

residue was purified by silica gel column chromatography (developing solvent, n-hexane : ethyl acetate = 1 : 1), thereby obtaining 2.40 g of a desired product (yield: 48%).
$^1$H-NMR data (300MHz, $CDCl_3$/TMS δ(ppm)): 7.39-7.44 (2H,m), 6.55-6.60 (2H,m), 4.55 (1H,s), 4.07-4.15 (1H,m), 3.76-3.93 (2H,m), 3.27-3.34 (1H,m), 3.06-3.14 (1H,m), 1.90-2.09 (3H,m), 1.60-1.67 (1H,m)

(2) Production of N-(4-cyanophenyl)-N-[(tetrahydrofuran-2-yl)methyl]formamide

2.40 g (11.87 mmol) of 4-{[(tetrahydrofuran-2-yl)methyl]amino}benzonitrile and 0.81 g (11.87 mmol) of sodium formate were dissolved in 10 ml of formic aid, and the mixture was heated and refluxed for 0.5 hours. Cooling was performed at room temperature, water was added, and extraction with ethyl acetate was performed. Washing with a saturated sodium bicarbonate aqueous solution was performed, drying with anhydrous magnesium sulfate was performed, and the solvent was distilled in vacuo, thereby obtaining 2.20 g of a desired product (yield: 81%).
$^1$H-NMR data (300MHz, $CDCl_3$/TMS δ(ppm)): 8.38-8.55 (1H,m), 7.68-7.72 (2H,m), 7.40-7.61 (2H,m), 3.97-4.27 (2H,m), 3.67-3.89 (3H,m), 1.80-2.10 (3H,m), 1.48-1.62 (1H,m)

(3) Production of N'-hydroxy-4-{N-[(tetrahydrofuran-2-yl)methyl]formamid}benzimidamide

2.20 g (9.55 mmol) of N-(4-cyanophenyl)-N-[(tetrahydrofuran-2-yl)methyl]formamide and 0.80 g (11.46 mmol) of hydroxylamine hydrochloride were added to 30 ml of ethanol, 1.16 g (11.46 mmol) of triethylamine was further added, and the mixture was heated and refluxed for 4 hours. Cooling was performed at room temperature, water was added, and extraction with ethyl acetate was performed. Washing with brine was performed, drying with anhydrous magnesium sulfate was performed, and the solvent was distilled in vacuo, thereby obtaining 1.30 g of a desired product (yield: 52%).
$^1$H-NMR data (300MHz, DMSO-$d_6$/TMS δ (ppm)): 9.64-9.66 (1H,m), 8.30-8.46 (1H,m), 7.66-7.72 (2H,m), 7.36-7.40 (2H,m), 5.83 (2H,s), 3.52-3.96 (5H,m), 1.42-1.91 (4H,m)

(4) Production of N-[(tetrahydrofuran-2-yl)methyl]-N-{4-[5-(trifluoromethyl)-1,2,4-oxadiazol-3-yl]phenyl}formamide

(Compound of Present Invention: B-0810)

1.15 g (4.37 mmol) of N'-hydroxy-4-{N-[(tetrahydrofuran-2-yl)methyl]formamid}benzimidamide was dissolved in 30 ml of tetrahydrofuran, 1.01 g (4.80 mmol) of trifluoroacetic anhydride was added under ice cooling, and the mixture was stirred at 0°C for 2 hours. The solvent was distilled in vacuo, water was added, and extraction with ethyl acetate was performed.

Washing with a saturated sodium bicarbonate aqueous solution was performed, drying with anhydrous magnesium sulfate was performed, the solvent was distilled in vacuo, and the residue was purified by silica gel column chromatography (developing solvent, n-hexane : ethyl acetate = 1 : 3), thereby obtaining 0.75 g of a desired product (yield: 50%).
$^1$H-NMR data (300MHz, DMSO-$d_6$/TMS δ(ppm)): 8.38-8.64 (1H,m), 7.98-8.16 (2H,m), 7.64-7.73 (2H,m), 3.52-4.06 (5H,m), 1.68-1.94 (3H,m), 1.49-1.53 (1H,m)

Example 5

Production of 4-[5-(trifluoromethyl)-1,2,4-oxadiazol-3-yl]phenylamine

(1) Production of N-hydroxy-4-nitrobenzimidamide

15.00 g (0.10 mol) of 4-nitrobenzonitrile and 21.10 g (0.30 mol) of hydroxylamine hydrochloride were added to 200 ml of ethanol, 50.60 g (0.50 mol) of triethylamine was further added, and the mixture was heated and refluxed for 2 hours. Cooling was performed at room temperature, water was added, and extraction with ethyl acetate was performed. Washing with brine was performed, drying with anhydrous magnesium sulfate was performed, and the solvent was distilled in vacuo, thereby obtaining 18.34 g of a desired crude product.
$^1$H-NMR data (300MHz, DMSO-$d_6$/TMS δ(ppm)): 10.13 (1H,s), 8.21-8.26 (2H,m), 7.92-7.97 (2H,s), 6.07 (1H,brs)

(2) Production of 3-(4-nitrophenyl)-5-(trifluoromethyl)-1,2,4-oxadiazole

18.00 g (0.10 mol) of crude N-hydroxy-4-nitrobenzimidamide was dissolved in 300 ml of toluene, 42.00 g (0.20 mol) of trifluoroacetic anhydride was added at room temperature, and the mixture was heated and refluxed for 2 hours. Cooling was performed, and the solvent was distilled in vacuo. The residue was purified by silica gel column chromatography (developing solvent, n-hexane : ethyl acetate = 10 : 1) to obtain 26.28 g of a desired product (yield: 100%).
$^1$H-NMR data (300MHz, $CDCl_3$/TMS δ(ppm)): 8.32-8.43 (4H,m)

(3) Production of 4-[5-(trifluoromethyl)-1,2,4-oxadiazol-3-yl]aniline

4.96 g (19.14 mmol) of 3-(4-nitrophenyl)-5-(trifluoromethyl)-1,2,4-oxadiazole and 0.62 g (1.92 mmol) of tetrabutylammonium bromide were dissolved in a mixed solution of 40 mL of tetrahydrofuran and 40 mL of water, 8.88 g (51.01 mmol) of sodium dithionite was added under ice cooling, and the mixture was stirred under ice cooling for 2 hours. 2.00 g (11.49 mmol) of sodium dithionite was further added under ice cooling, and the mixture was stirred under ice cooling for 1 hour. At room temperature, water was added, and extraction with ethyl acetate was performed.
The organic layer was dried with anhydrous magnesium sulfate, the solvent was distilled in vacuo, and the residue was purified by silica gel column chromatography (developing solvent, n-hexane : ethyl acetate = 1 : 1), thereby obtaining 2.38 g of a desired product (yield: 54%).
$^1$H-NMR data (300MHz, $CDCl_3$/TMS δ(ppm)): 7.88-7.93 (2H,m), 6.72-6.77 (2H,m), 4.06(1H,brs)

Example 6

Production of N-(2-bromobenzyl)-N-{4-[5-(trifluoromethyl)-1,2,4-oxadiazol-3-yl]phenyl}thioformamide (Compound of Present Invention: C-0162)

(1) Production of N-(2-bromobenzyl)-N-{4-[5-(trifluoromethyl)-1,2,4-oxadiazol-3-yl]phenyl}formamide

(Compound of Present Invention: A-0162)

0.35 g (1.36 mmol) of N-{4-[5-(trifluoromethyl)-1,2,4-oxadiazol-3-yl]phenyl}formamide was dissolved in 20 ml of DMF, 0.082 g (purity: about 60%, 2.05 mmol) of sodium hydride was added under ice cooling, and the mixture was stirred for 0.5 hours. 0.68 g (2.72 mmol) of 1-bromo-2-(bromomethyl)benzene was added under ice cooling, the temperature was raised to room temperature, and the mixture was stirred for 2 hours. The reaction mixture was cooled to 0°C, ice water was added, the mixture was stirred for 0.5 hours, ethyl acetate was added, the organic layer was washed with brine, drying with anhydrous sodium sulfate, and the solvent was distilled in vacuo. The residue was purified by silica gel column chromatography (developing solvent, n-hexane : ethyl acetate = 10 : 1) to obtain 0.58 g of a desired product (yield: 100%).
$^1$H-NMR data (300MHz, $CDCl_3$/TMS δ(ppm)): 8.61-8.82 (1H,m), 8.06-8.13 (2H,m), 7.09-7.60 (6H,m), 5.01-5.18 (2H,m)

(2) Production of N-(2-bromobenzyl)-N-{4-[5-(trifluoromethyl)-1,2,4-oxadiazol-3-yl]phenyl}thioformamide

(Compound of Present Invention: C-0162)

0.34 g (0.80 mmol) of N-(2-bromobenzyl)-N-{4-[5-(trifluoromethyl)-1,2,4-oxadiazol-3-yl]phenyl}formamide was dissolved in 20 ml of tetrahydrofuran, 0.21 g (0.52 mmol) of a Lawesson's reagent was added, and the mixture was stirred at room temperature for 24 hours. The solvent was distilled in vacuo, and the residue was purified by silica gel column chromatography (developing solvent, n-hexane : ethyl acetate = 10 : 1), thereby obtaining 0.33 g of a desired product (yield: 94%) .

$^1$H-NMR data (300MHz, $CDCl_3$/TMS δ(ppm)): 9.85-10.02 (1H,m), 8.11-8.16 (2H,m), 7.55-7.58 (1H,dd), 7.34-7.39 (2H,m), 7.11-7.28 (3H,m), 5.17-5.69 (2H,m)
Melting point (°C): 122 to 123

Example 7

Production of ethyl {4-[5-(trifluoromethyl)-1,2,4-oxadiazol-3-yl]phenyl}glycine (Compound of Present Invention: F-0031)

2.30 g (10.04 mmol) of 4-[5-(trifluoromethyl)-1,2,4-oxadiazol-3-yl]aniline was dissolved in 30 ml of N,N-dimethylacetamide, 1.40 g (10.13 mmol) of potassium carbonate, 1.79 g (10.72 mmol) of ethyl bromoacetate, and 0.50 g (3.01 mmol) of potassium iodide were added, and the mixture was stirred at 130°C for 2 hours. Cooling was performed at room temperature, water was added, and extraction with ethyl acetate was performed. Washing with brine was performed, drying with anhydrous magnesium sulfate was performed, the solvent was distilled in vacuo, and the residue was purified by silica gel column chromatography (developing solvent, n-hexane : ethyl acetate = 5 : 1), thereby obtaining 0.65 g of

a desired product (yield: 21%).
$^1$H-NMR data (300MHz, $CDCl_3$/TMS δ(ppm)): 7.92-7.96 (2H,m), 6.65-6.69 (2H,m), 4.74 (1H,brs), 4.25-4.32 (2H,q), 3.97 (2H,s) 1.30-1.34 (3H,s)

Example 8

Production of N-[2-(trifluoromethyl)benzyl]-N-{4-[5-(trifluoromethyl)-1,2,4-oxadiazol-3-yl]phenyl}amine (Compound of Present Invention: F-0213)

(1) Production of t-butyl N-[2-(trifluoromethyl)benzyl]-N-{4-[5-(trifluoromethyl)-1,2,4-oxadiazol-3-yl]phenyl}carbamate

0.40 g (1.21 mmol) of t-butyl {4-[5-(trifluoromethyl)-1,2,4-oxadiazol-3-yl]phenyl}carbamate was dissolved in 5 ml of N,N-dimethylformamide, 0.06 g (purity: about 60%, 1.50 mmol) of sodium hydride was added under ice cooling, and the mixture was stirred for 0.5 hours. 0.32 g (1.34 mmol) of 2-(trifluoromethyl)benzyl bromide was added, the temperature was raised to room temperature, and the mixture was stirred for 15 hours. Thereafter, water was added, and extraction with ethyl acetate was performed. Drying with anhydrous magnesium sulfate was performed, the solvent was distilled in vacuo, and the residue was purified by silica gel column chromatography (developing solvent, n-hexane : ethyl acetate = 9 : 1), thereby obtaining 0.54 g of a desired product (yield: 91%).
$^1$H-NMR data (300MHz, $CDCl_3$/TMS δ(ppm)): 8.01-8.06 (2H,m), 7.65-7.72 (1H,m), 7.29-7.58 (5H,m), 5.10-5.14 (2H,m), 1.36-1.41 (9H,m)

(2) Production of N-[2-(trifluoromethyl)benzyl]-N-{4-[5-(trifluoromethyl)-1,2,4-oxadiazol-3-yl]phenyl}amine (Compound of Present Invention: F-0213)

0.54 g (1.11 mmol) of t-butyl N-[2-(trifluoromethyl)benzyl]-N-{4-[5-(trifluoromethyl)-1,2,4-oxadiazol-3-yl]phenyl} carbamate was dissolved in 20 ml of dichloromethane, 4.8 ml of trifluoroacetic acid was added under ice cooling, and the mixture was stirred for 2 hours. 60 mL of a saturated sodium bicarbonate aqueous solution was added, extraction with dichloromethane was performed, washing with brine was performed, drying with anhydrous magnesium sulfate was performed, and the solvent was distilled in vacuo. The residue was purified by silica gel column chromatography (developing solvent, n-hexane : ethyl acetate = 7 : 3) to obtain 0.23 g of a desired product (yield: 54%).
$^1$H-NMR data (300MHz, $CDCl_3$/TMS δ (ppm)): 7.88-7.93 (2H,m), 7.71 (1H,d), 7.49-7.61 (2H,m), 7.40 1H,t), 6.64-6.91 (2H,m), 4.62-4.64 (3H,m)

The physical property values (the melting point, the refractive index, and the $^1$H-NMR spectrum data) of the compound [I] of the present invention synthesized according to each Example and the values in each Example are shown in Tables 203 to 218, the physical property values (the melting point, the refractive index, and the $^1$H-NMR spectrum data) of the compound [II] of the present invention are also shown in Tables 219 to 222, and the physical property values (the melting point, the refractive index, and the $^1$H-NMR spectrum data) of the compound [III] of the present invention are also shown in Tables 223 to 225. The compound numbers and symbols in the tables are as defined above.

[Table 203]

| Compound Number | Physical property value | |
|---|---|---|
| | Melting point (°C) | Refractive index ($n_D^{20}$) |
| A-0001 | 130-132 | |
| A-0002 | 71-72 | |
| A-0003 | 56-57 | |
| A-0004 | | 1.5196 |
| A-0006 | | 1.5153 |
| A-0007 | 64-66 | |
| A-0011 | | 1.5111 |
| A-0012 | | 1.5085 |
| A-0014 | 52-54 | |

(continued)

| Compound Number | Physical property value | |
|---|---|---|
| | Melting point (°C) | Refractive index ($n_D^{20}$) |
| A-0018 | 67-69 | |
| A-0026 | 62-65 | |
| A-0027 | | 1.4979 |
| A-0029 | | 1.4541 |
| A-0032 | 31-34 | |
| A-0034 | 95-98 | |
| A-0035 | 108-110 | |
| A-0038 | 122-125 | |
| A-0039 | | 1.5041 |
| A-0041 | 93-95 | |
| A-0042 | 154-157 | |
| A-0045 | 167-169 | |
| A-0057 | 91-94 | |
| A-0064 | 68-71 | |
| A-0066 | | 1.5177 |
| A-0067 | | 1.5098 |
| A-0072 | 81-84 | |
| A-0074 | 40-43 | |
| A-0075 | 33-34 | |
| A-0080 | | 1.5009 |
| A-0081 | | 1.4995 |
| A-0082 | | 1.5692 |
| A-0095 | | 1.5267 |
| A-0096 | 64-65 | |
| A-0097 | | 1.5558 |
| A-0098 | 35-38 | |
| A-0099 | | 1.5504 |
| A-0103 | 87-90 | |
| A-0104 | 145-147 | |
| A-0105 | 168-171 | |
| A-0107 | | 1.5443 |
| A-0144 | | 1.5564 |

[Table 204]

| Compound Number | Physical property value | |
|---|---|---|
| | Melting point (°C) | Refractive index ($n_D^{20}$) |
| A-0151 | | 1.5786 |
| A-0156 | 67-68 | |
| A-0157 | 55-57 | |
| A-0158 | 67-70 | |
| A-0159 | 75-78 | |
| A-0160 | 53-56 | |
| A-0161 | 104-105 | |
| A-0162 | 73-76 | |
| A-0163 | 75-77 | |
| A-0164 | 122-125 | |
| A-0165 | 99-102 | |
| A-0166 | 113-116 | |
| A-0167 | 120-123 | |
| A-0168 | 102-103 | |
| A-0169 | 77-79 | |
| A-0170 | 102-103 | |
| A-0204 | | 1.5611 |
| A-0205 | 53-54 | |
| A-0206 | 71-74 | |
| A-0216 | 65-68 | |
| A-0217 | 78-80 | |
| A-0218 | 83-85 | |
| A-0219 | | 1.4995 |
| A-0220 | 57-59 | |
| A-0221 | 83-85 | |
| A-0240 | 93-95 | |
| A-0241 | 87-90 | |
| A-0242 | 85-88 | |
| A-0255 | 94-96 | |
| A-0256 | 85-88 | |
| A-0257 | 112-113 | |
| A-0259 | 58-60 | |
| A-0260 | 91-94 | |
| A-0279 | | 1.5602 |
| A-0280 | 84-87 | |
| A-0281 | 120-122 | |
| A-0282 | | 1.5548 |

(continued)

| Compound Number | Physical property value | |
|---|---|---|
| | Melting point (°C) | Refractive index ($n_D^{20}$) |
| A-0283 | | 1.5479 |
| A-0284 | 104-107 | |
| A-0315 | 108-110 | |
| A-0316 | 87-89 | |

[Table 205]

| Compound Number | Physical property value | |
|---|---|---|
| | Melting point (°C) | Refractive index ( $n_D^{20}$) |
| A-0317 | 125-127 | |
| A-0334 | 63-66 | |
| A-0335 | 120-123 | |
| A-0351 | 54-57 | |
| A-0352 | 48-50 | |
| A-0353 | | 1.4727 |
| A-0354 | | 1.5200 |
| A-0355 | 57-60 | |
| A-0356 | 73-76 | |
| A-0481 | 98-100 | |
| A-0482 | | 1.5414 |
| A-0483 | 108-110 | |
| A-0484 | | 1.5580 |
| A-0485 | 114-116 | |
| A-0486 | | 1.5362 |
| A-0487 | 126-127 | |
| A-0488 | 70-71 | |
| A-0490 | 57-60 | |
| A-0491 | 154-157 | |
| A-0492 | 98-100 | |
| A-0493 | 191-193 | |
| A-0494 | 124-126 | |
| A-0497 | 155-157 | |
| A-0498 | 124-127 | |
| A-0504 | 119-122 | |
| A-0505 | | 1.5410 |
| A-0506 | | 1.5468 |
| A-0507 | | 1.5426 |

(continued)

| Compound Number | Physical property value | |
|---|---|---|
| | Melting point (°C) | Refractive index ( $n_D^{20}$) |
| A-0508 | 97-100 | |
| A-0509 | 89-91 | |
| A-0510 | 69-72 | |
| A-0512 | 48-51 | |
| A-0515 | | 1.5498 |
| A-0516 | 82-84 | |
| A-0517 | 72-75 | |
| A-0518 | 78-80 | |
| A-0519 | 103-104 | |
| A-0520 | 116-117 | |
| A-0521 | 71-73 | |
| A-0522 | 68-70 | |
| A-0523 | 100-103 | |

[Table 206]

| Compound Number | Physical property value | |
|---|---|---|
| | Melting point(°C) | Refractive index($n_D^{20}$) |
| A-0524 | 59-61 | |
| A-0525 | 127-130 | |
| A-0527 | 72-74 | |
| A-0528 | 76-79 | |
| A-0537 | 122-124 | |
| A-0538 | 126-129 | |
| A-0539 | 100-103 | |
| A-0546 | 169-172 | |
| A-0549 | | 1.4961 |
| A-0551 | | 1.5120 |
| A-0552 | | 1.5122 |
| A-0553 | 55-57 | |
| A-0554 | 119-121 | |
| A-0557 | 128-130 | |
| A-0560 | 110-113 | |
| A-0561 | 83-86 | |
| A-0562 | 97-99 | |
| A-0563 | 141-142 | |
| A-0564 | 130-133 | |

(continued)

| Compound Number | Physical property value | |
|---|---|---|
| | Melting point(°C) | Refractive index($n_D^{20}$) |
| A-0566 | 112-115 | |
| A-0567 | 124-126 | |
| A-0569 | 114-116 | |
| A-0572 | 97-99 | |
| A-0573 | 97-100 | |
| A-0574 | 77-80 | |
| A-0575 | 140-143 | |
| A-0576 | 114-117 | |
| A-0577 | 142-145 | |
| A-0578 | 42-45 | |
| A-0580 | 81-84 | |
| A-0582 | 103-106 | |
| A-0583 | 97-100 | |
| A-0584 | 80-83 | |
| A-0585 | 85-87 | |
| A-0586 | 105-108 | |
| A-0588 | 144-147 | |
| A-0590 | | 1.5489 |
| A-0591 | 129-132 | |
| A-0592 | 106-108 | |
| A-0593 | 104-107 | |
| A-0594 | 90-93 | |

[Table 207]

| Compound Number | Physical property value | |
|---|---|---|
| | Melting point (°C) | Refractive index ( $n_D^{20}$) |
| A-0595 | 98-100 | |
| A-0596 | | 1.5295 |
| A-0597 | 61-64 | |
| A-0598 | | 1.5150 |
| A-0599 | 141-144 | |
| A-0600 | 147-149 | |
| A-0601 | 149-151 | |
| A-0602 | 96-98 | |
| A-0603 | 141-143 | |
| A-0604 | 156-159 | |

(continued)

| Compound Number | Physical property value | |
|---|---|---|
| | Melting point (°C) | Refractive index ( $n_D^{20}$) |
| A-0605 | 164-167 | |
| A-0608 | 165-168 | |
| A-0609 | 181-184 | |
| A-0613 | 136-139 | |
| A-0619 | 173-176 | |
| A-0622 | 107-109 | |
| A-0623 | 137-140 | |
| A-0624 | 187-189 | |
| A-0626 | 114-117 | |
| A-0651 | | 1.5093 |
| A-0652 | 60-63 | |
| A-0654 | 35-36 | |
| A-0657 | | 1.4937 |
| A-0658 | | 1.5160 |
| A-0660 | 52-55 | |
| A-0667 | 77-79 | |
| A-0706 | 131-133 | |
| A-0717 | 63-64 | |
| A-0718 | 86-89 | |
| A-0719 | | 1.5067 |
| A-0720 | | 1.5060 |
| A-0729 | 42-45 | |
| A-0734 | | 1.5085 |
| A-0736 | - | 1.5070 |
| A-0739 | 87-90 | |
| A-0742 | 61-62 | |
| A-0743 | | 1.5177 |
| A-0744 | 35-37 | |
| A-0753 | 111-114 | |
| A-0754 | 121-122 | |
| A-0757 | 124-127 | |

[Table 208]

| Compound Number | Physical property value | |
|---|---|---|
| | Melting point (°C) | Refractive index ( $n_D^{20}$) |
| A-0758 | 142-144 | |

(continued)

| Compound Number | Physical property value | |
|---|---|---|
| | Melting point (°C) | Refractive index ( $n_D^{20}$) |
| A-0767 | 91-94 | |
| A-0774 | 50-53 | |
| A-0797 | 62-65 | |
| A-0798 | 77-80 | |
| A-0799 | 57-60 | |
| A-0800 | 65-68 | |
| A-0801 | | ’1.5252 |
| A-0810 | 116-119 | |
| A-0812 | 82-84 | |
| A-0814 | | 1.4983 |
| A-0857 | 166-169 | |
| A-0859 | 159-162 | |
| A-0860 | 72-74 | |
| A-0861 | 74-76 | |
| A-0862 | 121-124 | |
| A-0863 | | 1.5121 |
| A-0864 | 55-57 | |
| A-0865 | 64-67 | |
| A-0866 | 82-84 | |
| A-0867 | 70-72 | |
| A-0868 | 62-65 | |
| A-0869 | 92-93 | |
| A-0870 | 70-73 | |
| A-0872 | | ’ 1.5129 |
| A-0873 | 147-150 | |
| A-0874 | 143-145 | |
| A-0875 | 127-130 | |
| A-0876 | | ’ 1.4967 |
| A-0877 | 83-86 | |
| A-0878 | 90-93 | |

[Table 209]

| Compound Number | Physical property value $^1$H-NMR data [TMS δ(ppm)] | |
|---|---|---|
| A-0023 | 300 MHz $CDCl_3$ | 8.52(1H, s), 8.16-8.21(2H, m), 7.37-7.41(2H, m), 3.76-3.79(2H, m), 0.95-1.09(1H, m), 0.18-0.59(4H, m) |
| A-0065 | 300 MHz $CDCl_3$ | 8.70(1H, s), 8.14-8.20(2H, m), 7.46-7.51(2H, m), 5.07-5.24(2H, m), 3.64-3.71(2H, m), 1.22-1.27(3H, m) |

(continued)

| Compound Number | Physical property value $^1$H-NMR data [TMS δ(ppm)] | |
|---|---|---|
| A-0550 | 300 MHz $CDCl_3$ | 8.52(1H, s), 8.16-8.19(2H, m), 7.50-7.52(2H, m), 5.69(1H, s), 4.19-4.27 (4H, m), 1.19-1.27(6H, m) |
| A-0871 | 300 MHz DMSO-$d_6$ | 8.64-8.72(1H, m), 8.07-8.11(2H, m), 7.52-7.61(2H, m), 4.11-4.15(2H, t), 3.85-3.91(2H, m), 1.32-1.42(9H, m) |

[Table 210]

| Compound Number | Physical property value | |
|---|---|---|
| | Melting point (°C) | Refractive index ( $n_D^{20}$) |
| B-0001 | 130-133 | |
| B-0002 | 131-134 | |
| B-0003 | 154-156 | |
| B-0004 | 115-117 | |
| B-0007 | 152-155 | |
| B-0008 | 102-105 | |
| B-0009 | 96-99 | |
| B-0010 | 71-73 | |
| B-0011 | 94-96 | |
| B-0012 | 77-80 | |
| B-0013 | 82-85 | |
| B-0014 | 96-99 | |
| B-0016 | 103-106 | |
| B-0017 | 76-78 | |
| B-0018 | 97-100 | |
| B-0019 | 74-77 | |
| B-0020 | 110-112 | |
| B-0021 | 90-91 | |
| B-0025 | 82-84 | |
| B-0029 | 72-73 | |
| B-0033 | 120-123 | |
| B-0041 | 105-107 | |
| B-0042 | 54-55 | |
| B-0044 | 59-61 | |
| B-0046 | 93-96 | |
| B-0047 | 95-98 | |
| B-0049 | | 1.5708 |
| B-0050 | 76-79 | |
| B-0051 | 98-100 | |
| B-0055 | 104-105 | |

(continued)

| Compound Number | Physical property value | |
|---|---|---|
| | Melting point (°C) | Refractive index ( $n_D^{20}$) |
| B-0059 | 82-83 | |
| B-0063 | 123-126 | |
| B-0067 | 77-80 | |
| B-0072 | 55-57 | |
| B-0076 | | 1.5672 |
| B-0077 | | 1.5671 |
| B-0088 | 100-103 | |
| B-0104 | 62-65 | |
| B-0127 | 74-77 | |
| 8-0133 | 85-86 | |
| 8-0135 | 116-119 | |

[Table 211]

| Compound Number | Physical property value | |
|---|---|---|
| | Melting point (°C) | Refractive index ( $n_D^{20}$) |
| B-0143 | 132-134 | |
| B-0150 | 118-120 | |
| 8-0189 | 63-66 | |
| B-0214 | | 1.5729 |
| B-0237 | 68-70 | |
| B-0260 | 65-68 | |
| B-0310 | | 1.4834 |
| B-0316 | 75-77 | |
| B-0341 | 73-74 | |
| B-0359 | 89-92 | |
| B-0375 | 81-84 | |
| B-0377 | 80-82 | |
| B-0418 | 106-109 | |
| B-0436 | 116-118 | |
| B-0454 | 107-109 | |
| B-0470 | 114-117 | |
| B-0488 | 87-90 | |
| B-0508 | 105-107 | |
| B-0531 | 133-135 | |
| B-0533 | 84-85 | |
| B-0549 | 68-71 | |

(continued)

| Compound Number | Physical property value | |
|---|---|---|
| | Melting point (°C) | Refractive index ( $n_D^{20}$) |
| B-0565 | | 1.5783 |
| B-0569 | 83-86 | |
| B-0619 | | 1.5766 |
| B-0630 | 87-89 | |
| B-0646 | 45-46 | |
| B-0662 | 103-104 | |
| B-0678 | 74-75 | |
| B-0744 | 117-120 | |
| B-0751 | 78-80 | |
| B-0762 | 161-164 | |
| B-0770 | 65-68 | |
| B-0779 | 51-53 | |
| B-0782 | 75-77 | |
| B-0794 | | 1.4898 |
| B-0797 | | 1.5240 |
| B-0810 | | 1.5273 |
| B-0812 | | 1.5280 |
| B-0814 | 122-125 | |
| B-0823 | | 1.4781 |
| B-0835 | 50-52 | |

[Table 212]

| Compound Number | Physical property value | |
|---|---|---|
| | Melting point (°C) | Refractive index ( $n_D^{20}$) |
| B-0837 | 73-75 | |
| B-0838 | 57-60 | |
| B-0850 | 86-88 | |
| B-0851 | 71-74 | |
| B-0852 | 114-116 | |

[Table 213]

| Compound Number | Physical property value $^1$H-NMR data [TMS δ(ppm)] | |
|---|---|---|
| B-0048 | 300 MHz DMSO-$d_6$ | 8.68-8.91(1H, m), 8.30-8.32(1H, dd), 8.01-8.08(2H, m), 7.71-7.78(1H, dd), 7.62-7.67(2H, m), 7.33-7.40(1H, dd), 5.12-5.19(2H, m) |

[Table 214]

| Compound Number | Physical property value | |
|---|---|---|
| | Melting point (°C) | Refractive index ($n_D^{20}$) |
| C-0001 | 203-206 | |
| C-0002 | 76-79 | |
| C-0003 | 65-67 | |
| C-0004 | 68-70 | |
| C-0038 | 102-104 | |
| C-0067 | 55-56 | |
| C-0080 | 50-51 | |
| C-0081 | 37-39 | |
| C-0096 | | 1.6172 |
| C-0156 | 85-87 | |
| C-0157 | 91-94 | |
| C-0162 | 122-123 | |
| C-0163 | | 1.6313 |
| C-0164 | 122-123 | |
| C-0166 | 90-93 | |
| C-0167 | 142-143 | |
| C-0216 | 88-90 | |
| C-0218 | | 1.5703 |
| C-0219 | 90-92 | |
| C-0281 | 102-104 | |
| C-0355 | | 1.5981 |
| C-0356 | | 1.5945 |
| C-0490 | | 1.6319 |
| C-0504 | | 1.5250 |
| C-0517 | 83-88 | |
| C-0549 | | 1.5364 |
| C-0552 | 68-69 | |
| C-0554 | 116-118 | |
| C-0557 | 132-134 | |
| C-0563 | 152-155 | |
| C-0564 | 107-109 | |
| C-0751 | 82-85 | |

[Table 215]

| Compound Number | Physical property value $^1$H-NMR data [TMS δ(ppm)] | |
|---|---|---|
| C-0011 | 300 MHz $CDCl_3$ | 9.69(1H, s), 8.14-8.27(2H, m), 7.37-7.42(2H, m), 4.33-4.38(2H, m), 0.85-1.76(9H, m) |
| C-0525 | 300 MHz $CDCl_3$ | 9.81(1H, s), 8.15-8.20(2H, m), 7.38-7.74(2H, m), 5.26(2H, s), 2.05-2.09 (1H, m), 1.17-1.20(2H, m), 1.02-1.06(2H, m) |

[Table 216]

| Compound Number | Physical property value | |
|---|---|---|
| | Melting point (°C) | Refractive index ( $n_D^{20}$) |
| D-0002 | 128-131 | |
| D-0003 | 140-143 | |
| 0-0008 | | 1.6193 |
| D-0010 | 75-78 | |
| D-0011 | 92-93 | |
| D-0012 | 97-99 | |
| D-0017 | 92-94 | |
| D-0104 | | 1.6154 |
| D-0135 | 119-121 | |
| D-0508 | 102-104 | |
| D-0531 | 107-110 | |
| D-0662 | | 1.5859 |
| D-0838 | 112-114 | |

[Table 217]

| Compound Number | Physical property value $^1$H-NMR data [TMS δ(ppm)] | |
|---|---|---|
| D-0569 | 300 MHz $CDCl_3$ | 9.68(1H, s), 8.19-8.22(2H, m), 7.71(1H, s), 7.35-7.38(2H, m), 5.43-5.66 (2H, m) |

[Table 218]

| Compound Number | Physical property value | |
|---|---|---|
| | Melting point (°C) | Refractive index ( $n_D^{20}$) |
| E-0001 | | 1.5421 |
| E-0199 | | 1.5455 |
| E-0397 | | 1.5793 |
| E-0496 | 121-122 | |
| E-0595 | 88-90 | |
| E-0793 | 127-129 | |
| E-1156 | | 1.5847 |

[Table 219]

| Compound Number | Physical property value | |
|---|---|---|
| | Melting point (°C) | Refractive index ( $n_D^{20}$) |
| F-0025 | 63-66 | |
| F-0031 | 94-97 | |
| F-0038 | 132-134 | |
| F-0059 | | 1.6242 |
| F-0074 | 86-88 | |
| F-0114 | 107-108 | |
| F-0115 | 59-62 | |
| F-0116 | 80-82 | |
| F-0117 | | 1.5873 |
| F-0118 | 59-60 | |
| F-0119 | 93-95 | |
| F-0126 | 80-82 | |
| F-0127 | | 1.5750 |
| F-0128 | 98-100 | |
| F-0162 | 99-102 | |
| F-0163 | 62-64 | |
| F-0164 | 53-55 | |
| F-0177 | 41-44 | |
| F-0178 | | 1.5547 |
| F-0200 | 153-156 | |
| F-0213 | 85-88 | |
| F-0241 | 89-91 | |
| F-0274 | 124-126 | |
| F-0292 | 106-107 | |
| F-0309 | 70-72 | |
| F-0313 | 79-82 | |
| F-0491 | 107-108 | |
| F-0650 | | 1.5512 |

[Table 220]

| Compound Number | Physical property value $^1$H-NMR data [TMS $\delta$(ppm)] | |
|---|---|---|
| F-0497 | 300 MHz $CDCl_3$ | 7.89-7.95(2H, d), 6.48-6.76(2H, m), 4.23-4.30(1H, m), 3.75-3.94(2H, m), 0.72-0.79(4H, m) |

[Table 221]

| Compound Number | Physical property value | |
|---|---|---|
| | Melting point (°C) | Refractive index ( $n_D^{20}$) |
| G-0004 | 127-130 | |
| G-0007 | 178-180 | |
| G-0012 | 122-125 | |
| G-0017 | 96-99 | |
| G-0025 | 96-98 | |
| G-0042 | 127-129 | |
| G-0072 | 137-139 | |
| G-0 135 | 107-109 | |
| G-0237 | 43-45 | |
| G-0310 | 104-106 | |
| G-0339 | 108-110 | |
| G-0533 | 77-80 | |
| G-0710 | 72-73 | |
| G-0838 | 60-62 | |

[Table 222]

| Compound Number | Physical property value $^1$H-NMR data [TMS δ(ppm)] | |
|---|---|---|
| G-0850 | 300 MHz $CDCl_3$ | 7.91-7.94(2H, m), 7.67-7.74(1H, t), 7.34-7.36(1H, d), 7.21-7.23(1H, d), 6.73-6.76(2H, m), 5.38-5.44(1H, m), 4.62(2H, s), 4.50-4.52(2H, d), 3.51 (3H, s) |

[Table 223]

| Compound Number | Physical property value | |
|---|---|---|
| | Melting point (°C) | Refractive index ( $n_D^{20}$) |
| H-0032 | 125-128 | |
| H-0038 | 110-113 | |
| H-0045 | | 1.5950 |
| H-0067 | 90-93 | |
| H-0096 | 50-53 | |
| H-0 156 | 150-153 | |
| H-0157 | 121-124 | |
| H-0 158 | 153-155 | |
| H-0159 | 177-180 | |
| H-0160 | 147-148 | |
| H-0161 | 134-137 | |
| H-0168 | 195-198 | |

(continued)

| Compound Number | Physical property value | |
|---|---|---|
| | Melting point (°C) | Refractive index ( $n_D^{20}$) |
| H-0169 | 129-130 | |
| H-0170 | 132-135 | |
| H-0204 | 183-186 | |
| H-0205 | 104-107 | |
| H-0206 | 119-122 | |
| H-0219 | 130-133 | |
| H-0220 | 132-135 | |
| H-0255 | 140-143 | |
| H-0257 | 152-155 | |
| H-0283 | 107-110 | |
| H-0316 | 145-148 | |
| H-0351 | 166-169 | |
| H-0355 | 134-137 | |
| H-0495 | 164-167 | |
| H-0496 | 167-170 | |
| H-0527 | 130-133 | |
| H-0530 | 120-122 | |
| H-0533 | 130-133 | |
| H-0537 | 165-168 | |
| H-0539 | 186-189 | |
| H-0602 | 165-168 | |
| H-0682 | 197-200 | |

[Table 224]

| Compound Number | Physical property value | |
|---|---|---|
| | Melting point (°C) | Refractive index ( $n_D^{20}$) |
| I-0012 | 168-170 | |
| I-0013 | 151-154 | |
| I-0017 | 129-131 | |
| I-0042 | 190-193 | |
| I-0072 | 171-174 | |
| I-0237 | 136-139 | |
| I-0678 | 139-142 | |
| I-0810 | 148-150 | |
| I-0838 | 86-89 | |
| I-0855 | 230-233 | |

[Table 225]

| Compound Number | Physical property value $^1$H-NMR data [TMS $\delta$(ppm)] | |
|---|---|---|
| 1-0025 | 300 MHz DMSO-$d_6$ | 9.62-9.65(1H, m), 8.58-8.75(1H, m), 7.58-7.67(3H, m), 7.38-7.41(2H, m), 7.02-7.11 (2H, m), 5.77-5.82(2H, m), 5.03-5.08(2H, m), 2.24(s, 3H) |
| 1-0812 | 300 MHz DMSO-$d_6$ | 9.67-9.68(1H, m), 8.36-8.52(1H, m), 7.69-7.74(2H, m), 7.36-7.41(2H, m), 5.86(2H, s), 3.36-3.91(5H, m), 3.30-3.36(1H, m), 2.27-2.36(1H, m), 1.73-1.87(1H, m), 1.44-1.55(1H, m) |

Next, Formulation Examples of the agricultural and horticultural plant disease control agent of the present invention using the formamide derivative of the present invention produced as described above, or the agriculturally acceptable salt thereof will be specifically described. It is to be noted that the compound, the type and mixing ratios of compounds and additives are not limited thereto, and can be changed in a wide range. In the following description, "part(s)" mean part(s) by mass.

[Formulation Example 1] Emulsifiable concentrate

| | |
|---|---|
| Each compound described in Tables 1 to 225 | 10 parts |
| Cyclohexanone | 30 parts |
| Polyoxyethylene alkyl aryl ether | 11 parts |
| Calcium alkylbenzenesulfonate | 4 parts |
| Methylnaphthalene | 45 parts |

The above components were uniformly dissolved to obtain an emulsifiable concentrate.

[Formulation Example 2] Wettable powder

| | |
|---|---|
| Each compound described in Tables 1 to 225 | 10 parts |
| Naphthalenesulfonic acid formalin condensate sodium salt | 0.5 parts |
| Polyoxyethylene alkyl aryl ether | 0.5 parts |
| Diatomaceous earth | 24 parts |
| Clay | 65 parts |

The above components were uniformly mixed and pulverized to obtain a wettable powder.

[Formulation Example 3] Powder

| | |
|---|---|
| Each compound described in Tables 1 to 225 | 2 parts |
| Diatomaceous earth | 5 parts |
| Clay | 93 parts |

The above components were uniformly mixed and pulverized to obtain a powder.

[Formulation Example 4] Granules

| | |
|---|---|
| Each compound described in Tables 1 to 225 | 5 parts |
| Sodium salt of lauryl alcohol sulfuric acid ester | 2 parts |
| Sodium ligninsulfonate | 5 parts |
| Carboxymethylcellulose | 2 parts |
| Clay | 86 parts |

The above components were uniformly mixed and pulverized. An amount corresponding to 20 parts of water was added to the mixture. The mixture was kneaded and processed into a granular shape of 14 to 32 mesh using an extrusion granulator, followed by drying to obtain granules.

[Formulation Example 5] Flowable concentrate

| | |
|---|---|
| Each compound described in Tables 1 to 225 | 20 parts |
| Polyoxyethylene styrenated phenyl ether sulfate | 4 parts |
| Ethylene glycol | 7 parts |
| Silicone AF-118N (manufactured by Asahi Kasei Corporation) | 0.02 parts |
| Water | 68.98 parts |

The above components were mixed for 30 minutes with a highspeed stirrer, and pulverized with a wet pulverizer to obtain a flowable concentrate.

[Formulation Example 6] Wettable granules

| | |
|---|---|
| Each compound described in Tables 1 to 225 | 10 parts |
| Sodium ligninsulfonate | 5 parts |
| Polyoxyethylene alkyl aryl ether | 1 part |
| Sodium polycarboxylate | 3 parts |
| White carbon | 5 parts |
| Pre-gelatinized starch | 1 part |
| Calcium carbonate | 65 parts |
| Water | 10 parts |

The above components were mixed, kneaded, and pressed to obtain a granule. The obtained granule was dried in a fluidized bed drier to obtain wettable granules.
Next, the effect exhibited by the disease control agent of the present invention will be described with reference to Test Examples.

[Test Example 1] Soybean rust (Phakopsora pachyrhizi) control effect test

Soybean seeds were sown in a pot and sprayed on the grown seedlings so that the amount of the active ingredient was 100 g a.i./ha. Thereafter, a soybean rust fungus was spray-inoculated, the pot was transferred to a wet room and was kept under dark conditions, and invasion of pathogens was promoted. Thereafter, the pot was managed in a greenhouse, after 12 to 14 days, a degree of disease severity for each first leaf was examined according to the criteria in Table 226 to determine a degree of disease severity by a calculation formula of Equation 1, and a control value was determined by a calculation formula of Equation 2.

[Table 226]

| Disease severity index | |
|---|---|
| 0: | The infection was not observed. |

(continued)

| Disease severity index | |
|---|---|
| 1 : | The infection area was less than 25% of the area of the leaf. |
| 2: | The infection area was 25% or more and less than 50% of the area of the leaf. |
| 3: | The infection area was 50% or more and less than 75% of the area of the leaf. |
| 4: | The infection area was 75% or more of the area of the leaf. |

[Equation 1]

$$\text{Degree of disease severity} = \frac{n0 \times 0 + n1 \times 1 + n2 \times 2 + n3 \times 3 + n4 \times 4}{4N} \times 100$$

[in the formula, N represents the total number of examined leaves, n0 represents the number of leaves having a disease severity index of 0, n1 represents the number of leaves having a disease severity index of 1, n2 represents the number of leaves having a disease severity index of 2, n3 represents the number of leaves having a disease severity index of 3, and n4 represents the number of leaves having a disease severity index of 4.]

[Equation 2]

$$\text{Control value} = \left(1 - \frac{\text{Degree of disease severity of treated section}}{\text{Degree of disease severity of untreated section}} \times 100\right)$$

The comparative compound in the table is the compound 7 of the present invention described in Table 1 of [0060] of WO 2017/110862 A, and has the following structure.

[Chem. 18]

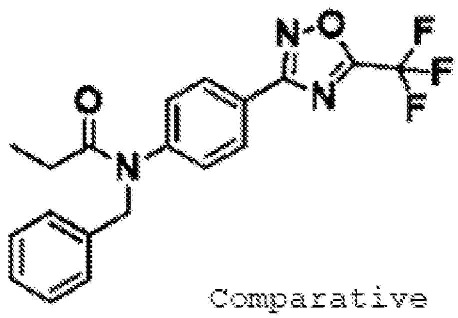

Comparative compound

As a result of the test, the control value of the comparative compound was less than 60, the control value of each of A-0002, A-0006, A-0011, A-0014, A-0026, A-0064, A-0098, A-0103, A-0151, A-0216, A-0256, A-0315, A-0483,

A-0486, A-0490, A-0494, A-0515, A-0549, A-0553, A-0567, A-0574, A-0575, A-0577, A-0585, A-0593, A-0594, A-0601, A-0608, A-0609, A-0652, A-0657, A-0667, A-0706, A-0717, A-0739, A-0758, A-0774, A-0814, A-0859, A-0862, A-0870, A-0871, A-0874, A-0876, A-0877, A-0921, B-0003, B-0004, B-0009, B-0067, B-0762, B-0782, B-0823, B-0835, B-0837, B-0852, C-0080, C-0163, C-0552, D-0012, E-0199, and G-0025 was 60 or more and less than 80, and the control value of each of A-0001, A-0007, A-0018, A-0027, A-0029, A-0032, A-0034, A-0035, A-0038, A-0039, A-0041, A-0042, A-0045, A-0057, A-0065, A-0066, A-0067, A-0072, A-0074, A-0075, A-0082, A-0095, A-0096, A-0097, A-0099, A-0107, A-0144, A-0156, A-0157, A-0158, A-0159, A-0160, A-0161, A-0168, A-0169, A-0170, A-0204, A-0205, A-0206, A-0219, A-0220, A-0240, A-0241, A-0242, A-0259, A-0260, A-0279, A-0282, A-0283, A-0316, A-0334, A-0351, A-0352, A-0353, A-0354, A-0355, A-0356, A-0482, A-0484, A-0491, A-0492, A-0497, A-0498, A-0505, A-0506, A-0507, A-0508, A-0512, A-0516, A-0517, A-0518, A-0519, A-0520, A-0521, A-0522, A-0523, A-0524, A-0525, A-0527, A-0528, A-0537, A-0539, A-0550, A-0551, A-0552, A-0554, A-0557, A-0560, A-0561, A-0563, A-0566, A-0569, A-0573, A-0592, A-0595, A-0596, A-0597, A-0598, A-0599, A-0600, A-0602, A-0603, A-0604, A-0613, A-0619, A-0622, A-0623, A-0626, A-0651, A-0654, A-0658, A-0660, A-0718, A-0719, A-0720, A-0729, A-0734, A-0736, A-0744, A-0753, A-0754, A-0757, A-0767, A-0797, A-0798, A-0799, A-0800, A-0801, A-0810, A-0812, A-0860, A-0861, A-0864, A-0867, A-0872, A-0873, B-0008, B-0011, B-0012, B-0013, B-0014, B-0016, B-0017, B-0018, B-0019, B-0020, B-0021, B-0025, B-0029, B-0033, B-0042, B-0044, B-0046, B-0047, B-0048, B-0049, B-0050, B-0051, B-0055, B-0059, B-0063, B-0072, B-0076, B-0077, B-0088, B-0104, B-0127, B-0133, B-0135, B-0143, B-0150, B-0189, B-0214, B-0237, B-0260, B-0310, B-0316, B-0341, B-0359, B-0375, B-0377, B-0418, B-0436, B-0454, B-0470, B-0488, B-0508, B-0531, B-0533, B-0549, B-0565, B-0569, B-0630, B-0646, B-0662, B-0678, B-0744, B-0751, B-0770, B-0779, B-0794, B-0797, B-0810, B-0812, B-0814, B-0838, B-0850, B-0851, C-0001, C-0038, C-0067, C-0096, C-0156, C-0157, C-0219, C-0355, C-0517, C-0525, C-0549, C-0554, C-0557, C-0563, C-0564, D-0008, D-0017, D-0104, D-0135, D-0508, D-0531, D-0662, D-0838, E-0397, E-0496, E-0595, E-0793, F-0038, G-0533, and G-0710 was 80 or more.

[Test Example 2] Wheat rust (Puccinia recondita) control effect test

Wheat seeds were sown in a pot, and a chemical solution was sprayed on the grown seedlings so that the concentration of the active ingredient was 50 ppm. Thereafter, a wheat rust fungus was spray-inoculated, the pot was transferred to a wet room and was kept under dark conditions, and invasion of pathogens was promoted. Thereafter, the pot was managed in a greenhouse, after 9 days, a degree of disease severity for each first leaf was examined according to the criteria in Table 220 to determine a degree of disease severity by a calculation formula of Equation 1, and a control value was determined by a calculation formula of Equation Formula 2.

[Table 227]

| Disease severity index | |
|---|---|
| 0: | The infection was not observed. |
| 1 : | The infection area was less than 5% of the area of the leaf. |
| 2: | The infection area was 5% or more and less than 33% of the area of the leaf. |
| 3: | The infection area was 33% or more and less than 67% of the area of the leaf. |
| 4: | The infection area was 67% or more of the area of the leaf. |

As a result of the test, the control value of the comparative compound was 12.5 or less, the control value of each of A-0002, A-0080, A-0103, A-0104, A-0105, A-0162, A-0165, A-0204, A-0216, A-0221, A-0255, A-0256, A-0257, A-0260, A-0280, A-0283, A-0284, A-0316, A-0317, A-0335, A-0351, A-0352, A-0481, A-0487, A-0504, A-0507, A-0510, A-0523, A-0575, A-0576, A-0577, A-0582, A-0584, A-0623, A-0624, A-0706, B-0007, B-0619, B-0751, C-0167, C-0219, C-0281, C-0355, D-0002, D-0011, F-0025,F-0059, F-0119, F-0164, F-0177, F-0292, F-0313, G-0004, and G-0838 was more than 12.5 and 50 or less, the control value of each of A-0026, A-0027, A-0029, A-0035, A-0039, A-0081, A-0095, A-0098, A-0099, A-0144, A-0151, A-0159, A-0163, A-0166, A-0167, A-0168, A-0219, A-0220, A-0279, A-0281, A-0282, A-0334, A-0354, A-0355, A-0483, A-0490, A-0492, A-0493, A-0505, A-0506, A-0522,A-0524, A-0537, A-0538, A-0550, A-0566, A-0569, A-0574, A-0583, A-0588, A-0591, A-0592, A-0593, A-0594, A-0605, A-0608, A-0609, A-0622, A-0626, A-0667, A-0797, A-0801, A-0859, A-0860, A-0864, B-0002, B-0003, B-0009, B-0011, B-0018, B-0048, B-0049, B-0454, B-0569, B-0782, C-0011, C-0081, C-0552, C-0751, D-0010, G-0017, G-0310, and G-0850 was more than 50 and 75 or less, and the control value of each of A-0001, A-0003, A-0004, A-0006, A-0007, A-0011, A-0012, A-0014, A-0018, A-0023, A-0032, A-0034, A-0038, A-0041, A-0042, A-0045, A-0057, A-0065, A-0066, A-0067, A-0072, A-0074, A-0075, A-0082, A-0096, A-0097, A-0156, A-0157, A-0158, A-0164, A-0169, A-0170, A-0205, A-0206, A-0240, A-0241, A-0242,

A-0259, A-0315, A-0353, A-0356, A-0482, A-0484, A-0485, A-0491, A-0494, A-0497, A-0498, A-0508, A-0509, A-0512, A-0515, A-0516, A-0517, A-0518, A-0519, A-0520, A-0521, A-0525, A-0527, A-0528, A-0539, A-0551, A-0552, A-0553, A-0554, A-0557, A-0560, A-0561, A-0562, A-0564, A-0567, A-0572, A-0573, A-0585, A-0586, A-0590, A-0595, A-0598, A-0599, A-0604, A-0613, A-0619, A-0651, A-0652, A-0654, A-0657, A-0658, A-0660, A-0717, A-0718, A-0719, A-0720, A-0729, A-0734, A-0736, A-0739, A-0742, A-0743, A-0744, A-0753, A-0754, A-0767, A-0774, A-0798, A-0799, A-0800, A-0812, A-0814, A-0862, A-0875, A-0876, A-0921, B-0004, B-0012, B-0013, B-0014, B-0016, B-0017, B-0019, B-0020, B-0021, B-0025, B-0033, B-0041, B-0042, B-0044, B-0046, B-0047, B-0050, B-0051, B-0055, B-0059, B-0063, B-0067, B-0072, B-0076, B-0077, B-0088, B-0104, B-0127, B-0133, B-0135, B-0143, B-0150, B-0189, B-0214, B-0237, B-0310, B-0316, B-0341, B-0359, B-0375, B-0377, B-0418, B-0436, B-0470, B-0488, B-0508, B-0531, B-0533, B-0549, B-0565, B-0630, B-0646, B-0662, B-0678, B-0744, B-0762, B-0770, B-0794, B-0797, B-0810, B-0812, B-0814, B-0823, B-0837, B-0838, B-0850, B-0851, B-0852, C-0001, C-0002, C-0003, C-0004, C-0038, C-0067, C-0080, C-0156, C-0157, C-0517, C-0525, C-0549, C-0554, C-0557, C-0564, D-0012, D-0017, D-0104, D-0135, D-0508, D-0531, D-0662, D-0838, E-0397, G-0012, G-0135, G-0533, and G-0710 was more than 75.

[Test Example 3] Rice sheath blight disease (Rhizoctonia solani) control effect test

Rice seeds were sown in a pot, and a chemical solution was sprayed on the grown seedlings so that the concentration of the active ingredient was 50 ppm. Thereafter, a bran medium in which a rice sheath blight fungi was cultured was inoculated into the strain, the bran medium was transferred to a wet room, and invasion of pathogens was promoted. Thereafter, the pot was managed in a greenhouse, after 5 days, a lesion height was examined according to the criteria in Table 221 to determine a degree of disease severity by a calculation formula of Equation 1, and a control value was determined by a calculation formula of Equation 2.

[Table 228]

| Disease severity index | |
|---|---|
| 0: | The infection was not observed. |
| 1 : | The lesion height was less than 25% of that of the untreated section. |
| 2: | The lesion height was 25% or more and less than 50% of that of the untreated section. |
| 3: | The lesion height was 50% or more and less than 75% of that of the untreated section. |
| 4: | The lesion height was 75% or more of that of the untreated section. |

As a result of the test, the control value of the comparative compound was 12.5 or less, the control value of each of A-0041, A-0042, A-0072, A-0151, A-0156, A-0204, A-0217, A-0279, A-0315, A-0316, A-0317, A-0334, A-0352, A-0353, A-0484, A-0490, A-0505, A-0508, A-0562, A-0564, A-0567, A-0572, A-0575, A-0591, A-0592, A-0597, A-0605, A-0667, A-0706, A-0718, A-0743, A-0799, A-0864, A-0872, A-0876, A-0921, B-0003, B-0011, B-0044, B-0046, B-0048, B-0049, B-0050, B-0310, B-0454, B-0569, B-0770, B-0814, B-0838, C-0038, C-0525, E-0199, E-0595, E-1156, F-0038, F-0491, H-0169, H-0351, and I-0678 was more than 12.5 and 50 or less, the control value of each of A-0075, A-0081, A-0097, A-0159, A-0168, A-0220, A-0280, A-0284, A-0491, A-0497, A-0507, A-0518, A-0522, A-0537, A-0561, A-0578, A-0582, A-0593, A-0594, A-0598, A-0622, A-0623, A-0624, A-0658, A-0729, A-0742, A-0744, A-0757, A-0798, A-0801, A-0810, A-0860, A-0862, A-0875, B-0020, B-0051, B-0104, B-0127, B-0143, B-0359, B-0549, B-0565, B-0852, C-0156, C-0355, C-0557, D-0011, D-0662, F-0213, G-0042, and 1-0042 was more than 50 and 75 or less, and the control value of each of A-0035, A-0080, A-0096, A-0098, A-0144, A-0157, A-0158, A-0160, A-0169, A-0170, A-0205, A-0206, A-0240, A-0241, A-0256, A-0259, A-0283, A-0335, A-0355, A-0356, A-0492, A-0498, A-0509, A-0517, A-0519, A-0520, A-0523, A-0525, A-0527, A-0528, A-0554, A-0557, A-0566, A-0569, A-0595, A-0599, A-0600, A-0603, A-0608, A-0609, A-0619, A-0758, A-0767, A-0774, A-0859, A-0870, A-0873, A-0874, B-0004, B-0009, B-0012, B-0014, B-0016, B-0017, B-0018, B-0019, B-0021, B-0025, B-0041, B-0047, B-0055, B-0059, B-0067, B-0076, B-0077, B-0088, B-0135, B-0150, B-0237, B-0341, B-0375, B-0418, B-0533, B-0762, B-0850, B-0851, C-0281, C-0554, C-0564, D-0012, D-0017, D-0135, D-0531, E-0496, and E-0793 was more than 75.

Industrial Applicability

The present invention provides a novel compound having excellent bactericidal activity and a production intermediate thereof, is useful in pesticide fields and agricultural fields, and has industrial applicability.

## Claims

1. A formamide derivative of formula [I] or an agriculturally acceptable salt thereof,

[Chem. 1]

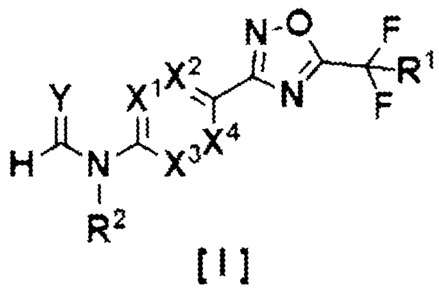

[I]

wherein in the formula [I],
$X^1$, $X^2$, $X^3$, and $X^4$ each independently represent a carbon atom (the carbon atom is optionally substituted with $R^3$) or a nitrogen atom,
Y represents an oxygen atom or a sulfur atom,
$R^1$ represents a hydrogen atom or a halogen atom,
$R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C3-C6)cycloalkyl (the (C3-C6)cycloalkyl is optionally mono-substituted or poly-substituted with $R^4$), a (C6-C12)aryl (the (C6-C12)aryl is optionally mono-substituted or poly-substituted with $R^5$), a 3- to 6-membered heterocyclyl (the 3- to 6-membered heterocyclyl is optionally mono-substituted or poly-substituted with $R^5$), or a 5- to 12-membered heteroaryl (the 5- to 12-membered heteroaryl is optionally mono-substituted or poly-substituted with $R^5$),
$R^3$ each independently represent a hydrogen atom, a halogen atom, or a (C1-C6)alkyl,
$R^4$ each independently represent a hydrogen atom, a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (Cl-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (Cl-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, an amino, a hydroxy, a thiol, a formyl, an oxo, a (C1-C6)alkyl, a (C2-C6)alkenyl, a (C2-C6)alkynyl, a (C3-C6)cycloalkyl, a (C1-C6)haloalkyl, a (C1-C6)alkoxy, a (C1-C6)haloalkoxy, a (C1-C6)alkoxy (C1-C6)alkyl, a (C1-C6)haloalkoxy (C1-C6)alkyl, a cyano (C1-C6)alkyl, a cyano (C3-C6)cycloalkyl, a (C1-C6)alkylthio, a (C1-C6)haloalkylthio, a (C1-C6)alkylsulfinyl, a (C1-C6)haloalkylsulfinyl, a (C1-C6)alkylsulfonyl, a (C1-C6)haloalkylsulfonyl, a (C1-C6)alkylcarbonylthio, a (C1-C6)alkylamino (the amino moiety is optionally substituted with $R^6$), a (C1-C6)haloalkylamino (the amino moiety is optionally substituted with $R^6$), a formylamino (the amino moiety is optionally substituted with $R^6$), a (C1-C6)alkylcarbonylamino (the amino moiety is optionally substituted with $R^6$), a carbamoyl, a (C1-C6)alkylaminocarbonyl (the amino moiety is optionally substituted with $R^6$), a carboxyl, a (C1-C6)alkylcarbonyl, a (C1-C6)haloalkylcarbonyl, a (C1-C6)alkoxycarbonyl, a hydroxyimino (C1-C6)alkyl, a (C1-C6)alkoxyimino (C1-C6)alkyl, a (C6-C12)aryl (the (C6-C12)aryl is optionally mono-substituted or poly-substituted with $R^7$), a (C6-C12)aryl (C1-C6)alkyl (the (C6-C12)aryl moiety is optionally mono-substituted or poly-substituted with $R^7$), a (C6-C12)arylcarbonyl (the (C6-C12)arylcarbonyl is optionally mono-substituted or poly-substituted with $R^7$), a (C6-C12)aryloxy (the (C6-C12)aryloxy is optionally mono-substituted or poly-substituted with $R^7$), a (C6-C12)arylthio (the (C6-C12)arylthio is optionally mono-substituted or poly-substituted with $R^7$), a (C6-C12)arylsulfinyl (the (C6-C12)arylsulfinyl is optionally mono-substituted or poly-substituted with $R^7$), a (C6-C12)arylsulfonyl (the (C6-C12)arylsulfonyl is optionally mono-substituted or poly-substituted with $R^7$), a (C6-C12)arylamino

(the (C6-C12)aryl moiety is optionally mono-substituted or poly-substituted with $R^7$, and the amino moiety is optionally substituted with $R^6$), a 3- to 6-membered heterocyclyl (the 3- to 6-membered heterocyclyl is optionally mono-substituted or poly-substituted with $R^7$), a 3- to 6-membered heterocyclyl (C1-C6)alkyl (the 3- to 6-membered heterocyclyl moiety is optionally mono-substituted or poly-substituted with $R^7$), a 3- to 6-membered heterocyclylcarbonyl (the 3- to 6-membered heterocyclylcarbonyl is optionally mono-substituted or poly-substituted with $R^7$), a 3- to 6-membered heterocyclyloxy (the 3- to 6-membered heterocyclyloxy is optionally mono-substituted or poly-substituted with $R^7$), a 3- to 6-membered heterocyclylthio (the 3- to 6-membered heterocyclylthio is optionally mono-substituted or poly-substituted with $R^7$), a 3- to 6-membered heterocyclylsulfinyl (the 3- to 6-membered heterocyclylsulfinyl is optionally mono-substituted or poly-substituted with $R^7$), 3- to 6-membered heterocyclylsulfonyl (the 3- to 6-membered heterocyclylsulfonyl is optionally mono-substituted or poly-substituted with $R^7$), a 3- to 6-membered heterocyclylamino (the 3- to 6-membered heterocyclyl moiety is optionally mono-substituted or poly-substituted with $R^7$, and the amino moiety is optionally substituted with $R^6$), a 5- to 12-membered heteroaryl (the 5- to 12-membered heteroaryl is optionally mono-substituted or poly-substituted with $R^7$), a 5- to 12-membered heteroaryl (C1-C6)alkyl (the 5- to 12-membered heteroaryl moiety is optionally mono-substituted or poly-substituted with $R^7$), a 5- to 12-membered heteroarylcarbonyl (the 5- to 12-membered heteroarylcarbonyl is optionally mono-substituted or poly-substituted with $R^7$), a 5- to 12-membered heteroaryloxy (the 5- to 12-membered heteroaryloxy is optionally mono-substituted or poly-substituted with $R^7$), a 5- to 12-membered heteroarylthio (the 5- to 12-membered heteroarylthio is optionally mono-substituted or poly-substituted with $R^7$), a 5- to 12-membered heteroarylsulfinyl (the 5- to 12-membered heteroarylsulfinyl is optionally mono-substituted or poly-substituted with $R^7$), a 5- to 12-membered heteroarylsulfonyl (the 5- to 12-membered heteroarylsulfonyl is optionally mono-substituted or poly-substituted with $R^7$), or a 5- to 12-membered heteroarylamino (the 5- to 12-membered heteroaryl moiety is optionally mono-substituted or poly-substituted with $R^7$, and the amino moiety is optionally substituted with $R^6$),

$R^6$ each independently represent a hydrogen atom, a (C1-C6)alkyl, a (C1-C6)haloalkyl, a (C3-C6)cycloalkyl, a (C1-C6)alkoxy (C1-C6)alkyl, a (C1-C6)haloalkoxy (C1-C6)alkyl, a cyano (C1-C6)alkyl, a (C1-C6)alkylcarbonyl, a (C1-C6)haloalkylcarbonyl, a (C1-C6)alkoxycarbonyl, or a (C6-C12)arylcarbonyl (the (C6-C12)arylcarbonyl is optionally mono-substituted or poly-substituted with $R^7$),

$R^7$ each independently represent a hydrogen atom, a halogen atom, a cyano, a (C1-C6)alkyl, or a (C1-C6)alkoxy, and

$R^8$ each independently represent a halogen atom, a cyano, a (C1-C6)haloalkyl, a (C3-C6)cycloalkyl, a (C1-C6)alkoxy, a (C1-C6)alkylcarbonyl, a (C1-C6)alkoxycarbonyl, a (C1-C6)haloalkoxycarbonyl, a (C6-C12)aryl (the (C6-C12)aryl is optionally mono-substituted or poly-substituted with $R^7$), a 3- to 6-membered heterocyclyl (the 3- to 6-membered heterocyclyl is optionally mono-substituted or poly-substituted with $R^7$), or a 5- to 12-membered heteroaryl (the 5- to 12-membered heteroaryl is optionally mono-substituted or poly-substituted with $R^7$).

**2.** The formamide derivative of formula [I] or the agriculturally acceptable salt thereof according to claim 1, wherein in the formula [I], $R^2$ represents a (C1-C6)alkyl (the (C1-C6)alkyl is optionally mono-substituted or poly-substituted with $R^4$), a (C3-C6)cycloalkyl (the (C3-C6)cycloalkyl is optionally mono-substituted or poly-substituted with $R^4$), or a 3- to 6-membered heterocyclyl (the 3- to 6-membered heterocyclyl is optionally mono-substituted or poly-substituted with $R^5$), and

$R^4$ each independently represent a (C3-C6)cycloalkyl, a (C3-C6)halocycloalkyl, a (C6-C12)aryl (the (C6-C12)aryl is optionally mono-substituted or poly-substituted with $R^5$), a 3- to 6-membered heterocyclyl (the 3- to 6-membered heterocyclyl is optionally mono-substituted or poly-substituted with $R^5$), a 5- to 12-membered heteroaryl (the 5- to 12-membered heteroaryl is optionally mono-substituted or poly-substituted with $R^5$), or a 1,3-benzodioxole (the 1,3-benzodioxole is optionally mono-substituted or poly-substituted with $R^5$).

**3.** The formamide derivative of formula [I] or the agriculturally acceptable salt thereof according to claim 2, wherein in the formula [I], $R^2$ represents a (C1-C6)alkyl (the (C1-C6)alkyl is optionally mono-substituted or poly-substituted with $R^4$), and

$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl.

**4.** The formamide derivative of formula [I] or the agriculturally acceptable salt thereof according to claim 1, wherein in the formula [I], $R^4$ each independently represent a halogen atom, a cyano, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino

moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (Cl-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (Cl-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino

moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

5. The formamide derivative of formula [I] or the agriculturally acceptable salt thereof according to claim 4, wherein in the formula [I], $R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C6-C12)aryl (the (C6-C12)aryl is optionally mono-substituted or poly-substituted with $R^5$), or a 3- to 6-membered heterocyclyl (the 3- to 6-membered heterocyclyl is optionally mono-substituted or poly-substituted with $R^5$),

$R^4$ each independently represent a halogen atom, a cyano, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with $R^6$), or a pentafluorosulfanyl,
$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl, and
$R^6$ each independently represent a hydrogen atom, a (C1-C6)alkyl, a (C1-C6)haloalkylcarbonyl, or a (C1-C6)alkoxycarbonyl.

6. The formamide derivative of formula [I] or the agriculturally acceptable salt thereof according to claim 2, wherein in the formula [I], $X^1$, $X^2$, $X^3$, and $X^4$ each independently represent a carbon atom (the carbon atom is optionally substituted with $R^3$), and $R^1$ is a halogen atom.

7. The formamide derivative of formula [I] or the agriculturally acceptable salt thereof according to claim 2, wherein in the formula [I], $R^3$ is a hydrogen atom.

8. The formamide derivative of formula [I] or the agriculturally acceptable salt thereof according to claim 2, wherein in the formula [I], $R^2$ represents a (C1-C6)alkyl mono-substituted or poly-substituted with substituent(s) selected from a substituent group $R^4$.

9. The formamide derivative of formula [I] or the agriculturally acceptable salt thereof according to claim 4, wherein in the formula [I], $X^1$, $X^2$, $X^3$, and $X^4$ each independently represent a carbon atom (the carbon atom is optionally substituted with $R^3$), and $R^1$ is a halogen atom.

10. The formamide derivative of formula [I] or the agriculturally acceptable salt thereof according to claim 4, wherein in General Formula [I], $R^3$ is a hydrogen atom.

11. The formamide derivative of formula [I] or the agriculturally acceptable salt thereof according to claim 4, wherein in the formula [I], $R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C3-C6)cycloalkyl (the (C3-C6)cycloalkyl is optionally mono-substituted or poly-substituted with $R^4$), a (C6-C12)aryl (the (C6-C12)aryl is optionally mono-substituted or poly-substituted with $R^5$), or a 5- to 12-membered heteroaryl (the 5- to 12-membered heteroaryl is optionally mono-substituted or poly-substituted with $R^5$).

12. The formamide derivative of formula [I] or the agriculturally acceptable salt thereof according to claim 4, wherein in the formula [I], $R^2$ represents a hydrogen atom or a (C1-C6)alkyl mono-substituted or poly-substituted with substituent(s) selected from a substituent group $R^4$.

13. An agricultural and horticultural plant disease control agent comprising the formamide derivative of formula [I] or the agriculturally acceptable salt thereof according to any one of claims 1 to 12 as an active ingredient.

14. The agricultural and horticultural plant disease control agent according to claim 13, wherein phytopathogenic fungi are basidiomycetes, ascomycetes, deuteromycetes, oomycetes, and/or zygomycetes.

15. An agricultural and horticultural plant disease control agent composition comprising the formamide derivative of formula [I] or the agriculturally acceptable salt thereof according to any one of claims 1 to 12, and an agriculturally acceptable carrier.

16. A method for controlling a disease of a useful plant, the method comprising a step of spraying an effective amount of the formamide derivative of formula [I] or the agriculturally acceptable salt thereof according to any one of claims 1 to 12 on the useful plant, a part of the useful plant, or a place where the useful plant grows.

17. The method according to claim 16, wherein phytopathogenic fungi are basidiomycetes, ascomycetes, deuteromycetes, oomycetes, and/or zygomycetes.

18. A use of the formamide derivative of formula [I] or the agriculturally acceptable salt thereof according to any one of claims 1 to 12 as an agricultural and horticultural plant disease control agent for a useful plant.

19. An aniline derivative of formula [II] or an agriculturally acceptable salt thereof,

[Chem. 2]

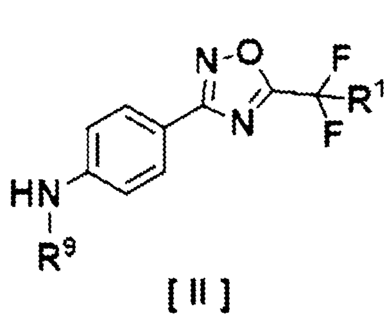

[II]

wherein in the formula [II],
$R^1$ represents a hydrogen atom or a halogen atom,
$R^9$ represents a (C3-C6)alkyl, a (C1-C6)alkyl mono-substituted or poly-substituted with substituent(s) selected from a substituent group $R^{10}$, a (C2-C6)alkenyl mono-substituted or poly-substituted with substituent(s) selected from a substituent group $R^{11}$, or a (C2-C6)alkynyl mono-substituted or poly-substituted with substituent(s) selected from a substituent group $R^{12}$,
$R^{10}$ and $R^{12}$ each independently represent a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycar-

bonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^{11}$ each independently represent a halogen atom, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or

poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl,

a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, an amino, a hydroxy, a thiol, a formyl, an oxo, a (C1-C6)alkyl, a (C2-C6)alkenyl, a (C2-C6)alkynyl, a (C3-C6)cycloalkyl, a (C1-C6)haloalkyl, a (C1-C6)alkoxy, a (C1-C6)haloalkoxy, a (C1-C6)alkoxy (C1-C6)alkyl, a (C1-C6)haloalkoxy (C1-C6)alkyl, a cyano (C1-C6)alkyl, a cyano (C3-C6)cycloalkyl, a (C1-C6)alkylthio, a (C1-C6)haloalkylthio, a (C1-C6)alkylsulfinyl, a (C1-C6)haloalkylsulfinyl, a (C1-C6)alkylsulfonyl, a (C1-C6)haloalkylsulfonyl, a (C1-C6)alkylcarbonylthio, a (C1-C6)alkylamino (the amino moiety is optionally substituted with $R^6$), a (C1-C6)haloalkylamino (the amino moiety is optionally substituted with $R^6$), a formylamino (the amino moiety is optionally substituted with $R^6$), a (C1-C6)alkylcarbonylamino (the amino moiety is optionally substituted with $R^6$), a carbamoyl, a (C1-C6)alkylaminocarbonyl (the amino moiety is optionally substituted with $R^6$), a carboxyl, a (C1-C6)alkylcarbonyl, a (C1-C6)haloalkylcarbonyl, a (C1-C6)alkoxycarbonyl, a hydroxyimino (C1-C6)alkyl, a (C1-C6)alkoxyimino (C1-C6)alkyl, a (C6-C12)aryl (the (C6-C12)aryl is optionally mono-substituted or poly-substituted with $R^7$), a (C6-C12)aryl (C1-C6)alkyl (the (C6-C12)aryl moiety is optionally mono-substituted or poly-substituted with $R^7$), a (C6-C12)arylcarbonyl (the (C6-C12)arylcarbonyl is optionally mono-substituted or poly-substituted with $R^7$), a (C6-C12)aryloxy (the (C6-C12)aryloxy is optionally mono-substituted or poly-substituted with $R^7$), a (C6-C12)arylthio (the (C6-C12)arylthio is optionally mono-substituted or poly-substituted with $R^7$), a (C6-C12)arylsulfinyl (the (C6-C12)arylsulfinyl is optionally mono-substituted or poly-substituted with $R^7$), a (C6-C12)arylsulfonyl (the (C6-C12)arylsulfonyl is optionally mono-substituted or poly-substituted with $R^7$), a (C6-C12)arylamino (the (C6-C12)aryl moiety is optionally mono-substituted or poly-substituted with $R^7$, and the amino moiety is optionally substituted with $R^6$), a 3- to 6-membered heterocyclyl (the 3- to 6-membered heterocyclyl is optionally mono-substituted or poly-substituted with $R^7$), a 3- to 6-membered heterocyclyl (C1-C6)alkyl (the 3- to 6-membered heterocyclyl moiety is optionally mono-substituted or poly-substituted with $R^7$), a 3- to 6-membered heterocyclylcarbonyl (the 3- to 6-membered heterocyclylcarbonyl is optionally mono-substituted or poly-substituted with $R^7$), a 3- to 6-membered heterocyclyloxy (the 3- to 6-membered heterocyclyloxy is optionally mono-substituted or poly-substituted with $R^7$), a 3- to 6-membered heterocyclylthio (the 3- to 6-membered heterocyclylthio is optionally mono-substituted or poly-substituted with $R^7$), a 3- to 6-membered heterocyclylsulfinyl (the 3- to 6-membered heterocyclylsulfinyl is optionally mono-substituted or poly-substituted with $R^7$), 3- to 6-membered heterocyclylsulfonyl (the 3- to 6-membered heterocyclylsulfonyl is optionally mono-substituted or poly-substituted with $R^7$), a 3- to 6-membered heterocyclylamino (the 3- to 6-membered heterocyclyl moiety is optionally mono-substituted or poly-substituted with $R^7$, and the amino moiety is optionally substituted with $R^6$), a 5- to 12-membered heteroaryl (the 5- to 12-membered heteroaryl is optionally mono-substituted or poly-substituted with $R^7$), a 5- to 12-membered heteroaryl (C1-C6)alkyl (the 5- to 12-membered heteroaryl moiety is optionally mono-substituted or poly-substituted with $R^7$), a 5- to 12-membered heteroarylcarbonyl (the 5- to 12-membered

heteroarylcarbonyl is optionally mono-substituted or poly-substituted with $R^7$), a 5- to 12-membered heteroaryloxy (the 5- to 12-membered heteroaryloxy is optionally mono-substituted or poly-substituted with $R^7$), a 5- to 12-membered heteroarylthio (the 5- to 12-membered heteroarylthio is optionally mono-substituted or poly-substituted with $R^7$), a 5- to 12-membered heteroarylsulfinyl (the 5- to 12-membered heteroarylsulfinyl is optionally mono-substituted or poly-substituted with $R^7$), a 5- to 12-membered heteroarylsulfonyl (the 5- to 12-membered heteroarylsulfonyl is optionally mono-substituted or poly-substituted with $R^7$), or a 5- to 12-membered heteroarylamino (the 5- to 12-membered heteroaryl moiety is optionally mono-substituted or poly-substituted with $R^7$, and the amino moiety is optionally substituted with $R^6$),

$R^6$ each independently represent a hydrogen atom, a (C1-C6)alkyl, a (C1-C6)haloalkyl, a (C3-C6)cycloalkyl, a (C1-C6)alkoxy (C1-C6)alkyl, a (C1-C6)haloalkoxy (C1-C6)alkyl, a cyano (C1-C6)alkyl, a (C1-C6)alkylcarbonyl, a (C1-C6)haloalkylcarbonyl, a (C1-C6)alkoxycarbonyl, or a (C6-C12)arylcarbonyl (the (C6-C12)arylcarbonyl is optionally mono-substituted or poly-substituted with $R^7$),

$R^7$ each independently represent a hydrogen atom, a halogen atom, a cyano, a (C1-C6)alkyl, or a (C1-C6)alkoxy, and

$R^8$ each independently represent a halogen atom, a cyano, a (C1-C6)haloalkyl, a (C3-C6)cycloalkyl, a (C1-C6)alkoxy, a (C1-C6)alkylcarbonyl, a (C1-C6)alkoxycarbonyl, a (C1-C6)haloalkoxycarbonyl, a (C6-C12)aryl (the (C6-C12)aryl is optionally mono-substituted or poly-substituted with $R^7$), a 3- to 6-membered heterocyclyl (the 3- to 6-membered heterocyclyl is optionally mono-substituted or poly-substituted with $R^7$), or a 5- to 12-membered heteroaryl (the 5- to 12-membered heteroaryl is optionally mono-substituted or poly-substituted with $R^7$).

**20.** The aniline derivative of formula [II] or the agriculturally acceptable salt thereof according to claim 19, wherein in the formula [II], $R^9$ represents a (C1-C6)alkyl mono-substituted or poly-substituted with substituent(s) selected from a substituent group $R^{10}$, and

$R^{10}$ each independently represent a (C3-C6)cycloalkyl, a (C3-C6)halocycloalkyl, a (C6-C12)aryl (the (C6-C12)aryl is optionally mono-substituted or poly-substituted with $R^5$), a 3- to 6-membered heterocyclyl (the 3- to 6-membered heterocyclyl is optionally mono-substituted or poly-substituted with $R^5$), a 5- to 12-membered heteroaryl (the 5- to 12-membered heteroaryl is optionally mono-substituted or poly-substituted with $R^5$), or a 1,3-benzodioxole (the 1,3-benzodioxole is optionally mono-substituted or poly-substituted with $R^5$).

**21.** The aniline derivative of formula [II] or the agriculturally acceptable salt thereof according to claim 20, wherein in the formula [II], $R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl.

**22.** The aniline derivative of formula [II] or the agriculturally acceptable salt thereof according to claim 19, wherein in the formula [II], $R^{10}$ and $R^{12}$ each independently represent a halogen atom, a cyano, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-

substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered

heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^{11}$ each independently represent a halogen atom, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylami-

nocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino

moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl group.

**23.** The aniline derivative of formula [II] or the agriculturally acceptable salt thereof according to claim 22, wherein in the formula [II], $R^{10}$ and $R^{12}$ each independently represent a halogen atom, a cyano, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-

substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with $R^6$), or a pentafluorosulfanyl,

$R^{11}$ each independently represent a halogen atom, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with

$R^6$), or a pentafluorosulfanyl,
$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl, and
$R^6$ each independently represent a hydrogen atom, a (C1-C6)alkyl, a (C1-C6)haloalkylcarbonyl, or a (C1-C6)alkoxycarbonyl.

**24.** An amidoxime derivative of formula [III] or an agriculturally acceptable salt thereof,

[Chem. 3]

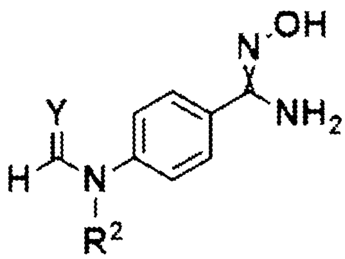

[ III ]

wherein in the formula [III],
Y represents an oxygen atom or a sulfur atom,
$R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C3-C6)cycloalkyl (the (C3-C6)cycloalkyl is optionally mono-substituted or poly-substituted with $R^4$), a (C6-C12)aryl (the (C6-C12)aryl is optionally mono-substituted or poly-substituted with $R^5$), a 3- to 6-membered heterocyclyl (the 3- to 6-membered heterocyclyl is optionally mono-substituted or poly-substituted with $R^5$), or a 5- to 12-membered heteroaryl (the 5- to 12-membered heteroaryl is optionally mono-substituted or poly-substituted with $R^5$),
$R^4$ each independently represent a hydrogen atom, a halogen atom, a cyano, a thiocyanato, a thiol, a hydroxy, an amino, a tri((C1-C6)alkyl)silyl, a (C3-C6)cycloalkyl, a (C3-C6)halocycloalkyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)ar-

ylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryl (the (C6-C12)aryl is optionally mono-substituted or poly-substituted with $R^5$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyl (the 3- to 6-membered heterocyclyl is optionally mono-substituted or poly-substituted with $R^5$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryl (the 5- to 12-membered heteroaryl is optionally mono-substituted or poly-substituted with $R^5$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a 1,3-benzodioxole (the 1,3-benzodioxole is optionally mono-substituted or poly-substituted with $R^5$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally substituted with $R^6$), a hydrazinocarbonyl (the hydrazino moiety is optionally substituted with $R^6$), or a pentafluorosulfanyl,

$R^5$ each independently represent a hydrogen atom, a halogen atom, a cyano, a nitro, an amino, a hydroxy, a thiol, a formyl, an oxo, a (C1-C6)alkyl, a (C2-C6)alkenyl, a (C2-C6)alkynyl, a (C3-C6)cycloalkyl, a (C1-C6)haloalkyl, a (C1-C6)alkoxy, a (C1-C6)haloalkoxy, a (C1-C6)alkoxy (C1-C6)alkyl, a (C1-C6)haloalkoxy (C1-C6)alkyl, a cyano (C1-C6)alkyl, a cyano (C3-C6)cycloalkyl, a (C1-C6)alkylthio, a (C1-C6)haloalkylthio, a (C1-C6)alkylsulfinyl, a (C1-C6)haloalkylsulfinyl, a (C1-C6)alkylsulfonyl, a (C1-C6)haloalkylsulfonyl, a (C1-C6)alkylcarbonylthio, a (C1-C6)alkylamino (the amino moiety is optionally substituted with $R^6$), a (C1-C6)haloalkylamino (the amino moiety is optionally substituted with $R^6$), a formylamino (the amino moiety is optionally substituted with $R^6$), a (C1-C6)alkylcarbonylamino (the amino moiety is optionally substituted with $R^6$), a carbamoyl, a (C1-C6)alkylaminocarbonyl (the amino moiety is optionally substituted with $R^6$), a carboxyl, a (C1-C6)alkylcarbonyl, a (C1-C6)haloalkylcarbonyl, a (C1-C6)alkoxycarbonyl, a hydroxyimino (C1-C6)alkyl, a (C1-C6)alkoxyimino (C1-C6)alkyl, a (C6-C12)aryl (the (C6-C12)aryl is optionally mono-substituted or poly-substituted with $R^7$), a (C6-C12)aryl (C1-C6)alkyl (the (C6-C12)aryl moiety is optionally mono-substituted or poly-substituted with $R^7$), a (C6-C12)arylcarbonyl (the (C6-C12)arylcarbonyl is optionally mono-substituted or poly-substituted with $R^7$), a

(C6-C12)aryloxy (the (C6-C12)aryloxy is optionally mono-substituted or poly-substituted with $R^7$), a (C6-C12)arylthio (the (C6-C12)arylthio is optionally mono-substituted or poly-substituted with $R^7$), a (C6-C12)arylsulfinyl (the (C6-C12)arylsulfinyl is optionally mono-substituted or poly-substituted with $R^7$), a (C6-C12)arylsulfonyl (the (C6-C12)arylsulfonyl is optionally mono-substituted or poly-substituted with $R^7$), a (C6-C12)arylamino (the (C6-C12)aryl moiety is optionally mono-substituted or poly-substituted with $R^7$, and the amino moiety is optionally substituted with $R^6$), a 3- to 6-membered heterocyclyl (the 3- to 6-membered heterocyclyl is optionally mono-substituted or poly-substituted with $R^7$), a 3- to 6-membered heterocyclyl (C1-C6)alkyl (the 3- to 6-membered heterocyclyl moiety is optionally mono-substituted or poly-substituted with $R^7$), a 3- to 6-membered heterocyclylcarbonyl (the 3- to 6-membered heterocyclylcarbonyl is optionally mono-substituted or poly-substituted with $R^7$), a 3- to 6-membered heterocyclyloxy (the 3- to 6-membered heterocyclyloxy is optionally mono-substituted or poly-substituted with $R^7$), a 3- to 6-membered heterocyclylthio (the 3- to 6-membered heterocyclylthio is optionally mono-substituted or poly-substituted with $R^7$), a 3- to 6-membered heterocyclylsulfinyl (the 3- to 6-membered heterocyclylsulfinyl is optionally mono-substituted or poly-substituted with $R^7$), 3- to 6-membered heterocyclylsulfonyl (the 3- to 6-membered heterocyclylsulfonyl is optionally mono-substituted or poly-substituted with $R^7$), a 3- to 6-membered heterocyclylamino (the 3- to 6-membered heterocyclyl moiety is optionally mono-substituted or poly-substituted with $R^7$, and the amino moiety is optionally substituted with $R^6$), a 5- to 12-membered heteroaryl (the 5- to 12-membered heteroaryl is optionally mono-substituted or poly-substituted with $R^7$), a 5- to 12-membered heteroaryl (C1-C6)alkyl (the 5- to 12-membered heteroaryl moiety is optionally mono-substituted or poly-substituted with $R^7$), a 5- to 12-membered heteroarylcarbonyl (the 5- to 12-membered heteroarylcarbonyl is optionally mono-substituted or poly-substituted with $R^7$), a 5- to 12-membered heteroaryloxy (the 5- to 12-membered heteroaryloxy is optionally mono-substituted or poly-substituted with $R^7$), a 5- to 12-membered heteroarylthio (the 5- to 12-membered heteroarylthio is optionally mono-substituted or poly-substituted with $R^7$), a 5- to 12-membered heteroarylsulfinyl (the 5- to 12-membered heteroarylsulfinyl is optionally mono-substituted or poly-substituted with $R^7$), a 5- to 12-membered heteroarylsulfonyl (the 5- to 12-membered heteroarylsulfonyl is optionally mono-substituted or poly-substituted with $R^7$), or a 5- to 12-membered heteroarylamino (the 5- to 12-membered heteroaryl moiety is optionally mono-substituted or poly-substituted with $R^7$, and the amino moiety is optionally substituted with $R^6$), $R^6$ each independently represent a hydrogen atom, a (C1-C6)alkyl, a (C1-C6)haloalkyl, a (C3-C6)cycloalkyl, a (C1-C6)alkoxy (C1-C6)alkyl, a (C1-C6)haloalkoxy (C1-C6)alkyl, a cyano (C1-C6)alkyl, a (C1-C6)alkylcarbonyl, a (C1-C6)haloalkylcarbonyl, a (C1-C6)alkoxycarbonyl, or a (C6-C12)arylcarbonyl (the (C6-C12)arylcarbonyl is optionally mono-substituted or poly-substituted with $R^7$),

$R^7$ each independently represent a hydrogen atom, a halogen atom, a cyano, a (C1-C6)alkyl, or a (C1-C6)alkoxy, and

$R^8$ each independently represent a halogen atom, a cyano, a (C1-C6)haloalkyl, a (C3-C6)cycloalkyl, a (C1-C6)alkoxy, a (C1-C6)alkylcarbonyl, a (C1-C6)alkoxycarbonyl, a (C1-C6)haloalkoxycarbonyl, a (C6-C12)aryl (the (C6-C12)aryl is optionally mono-substituted or poly-substituted with $R^7$), a 3- to 6-membered heterocyclyl (the 3- to 6-membered heterocyclyl is optionally mono-substituted or poly-substituted with $R^7$), or a 5- to 12-membered heteroaryl (the 5- to 12-membered heteroaryl is optionally mono-substituted or poly-substituted with $R^7$).

**25.** The amidoxime derivative of formula [III] or the agriculturally acceptable salt thereof according to claim 24, wherein in the formula [III], $R^2$ represents a (C1-C6)alkyl (the (C1-C6)alkyl is optionally mono-substituted or poly-substituted with $R^4$), a (C3-C6)cycloalkyl (the (C3-C6)cycloalkyl is optionally mono-substituted or poly-substituted with $R^4$), or a 3- to 6-membered heterocyclyl (the 3- to 6-membered heterocyclyl is optionally mono-substituted or poly-substituted with $R^5$), and
$R^4$ each independently represent a (C3-C6)cycloalkyl, a (C3-C6)halocycloalkyl, a (C6-C12)aryl (the (C6-C12)aryl is optionally mono-substituted or poly-substituted with $R^5$), a 3- to 6-membered heterocyclyl (the 3- to 6-membered heterocyclyl is optionally mono-substituted or poly-substituted with $R^5$), a 5- to 12-membered heteroaryl (the 5- to 12-membered heteroaryl is optionally mono-substituted or poly-substituted with $R^5$), or a 1,3-benzodioxole (the 1,3-benzodioxole is optionally mono-substituted or poly-substituted with $R^5$).

**26.** The amidoxime derivative of formula [III] or the agriculturally acceptable salt thereof according to claim 25, wherein in the formula [III], $R^2$ represents a (C1-C6)alkyl (the (C1-C6)alkyl is optionally mono-substituted or poly-substituted with $R^4$), and $R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl.

**27.** The amidoxime derivative of formula [III] or the agriculturally acceptable salt thereof according to claim 24, wherein

in the formula [III], $R^4$ each independently represent a halogen atom, a cyano, a thiocyanato, a thiol, a hydroxy, an amino, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a (C2-C6)alkenylamino (the amino moiety is optionally substituted with $R^6$), a (C2-C6)alkynylamino (the amino moiety is optionally substituted with $R^6$), a (C3-C6)cycloalkylamino (the (C3-C6)cycloalkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C2-C6)alkenylcarbonyl, a (C2-C6)alkynylcarbonyl, a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 3- to 6-membered heterocyclylaminocarbonyl (the 3- to 6-membered heterocyclyl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a formylamino (the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C2-C6)alkenylcarbonylamino (the amino moiety is optionally substituted with $R^6$), a (C2-C6)alkynylcarbonylamino (the amino moiety is optionally substituted with $R^6$), a (C3-C6)cycloalkylcarbonylamino (the (C3-C6)cycloalkyl moiety is optionally mono-substituted or poly-substituted with $R^8$, and the amino moiety is optionally substituted with $R^6$), a (C6-C12)arylcarbonylamino (the (C6-C12)aryl moiety is optionally mono-substituted or poly-substituted with $R^5$, and the amino moiety is optionally substituted with $R^6$), a 3- to 6-membered heterocyclylcarbonylamino (the 3- to 6-membered heterocyclyl moiety is optionally mono-substituted or poly-substituted with $R^5$, and the amino moiety is optionally substituted with $R^6$), a 5- to 12-membered heteroarylcarbonylamino (the 5- to 12-membered heteroaryl moiety is optionally mono-substituted or poly-substituted with $R^5$, and the amino moiety is optionally substituted with $R^6$), a formyloxy, a (C1-C6)alkylcarbonyloxy (the (C1-C6)alkylcarbonyloxy is optionally mono-substituted or poly-substituted with $R^8$), a (C2-C6)alkenylcarbonyloxy, a (C2-C6)alkynylcarbonyloxy, a (C3-C6)cycloalkylcarbonyloxy (the (C3-C6)cycloalkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a 3- to 6-membered heterocyclylcarbonyloxy (the 3- to 6-membered heterocyclylcarbonyloxy is optionally mono-substituted or poly-substituted with $R^5$), a 5- to 12-membered heteroarylcarbonyloxy (the 5- to 12-membered heteroarylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)haloalkoxy, a (C2-C6)alkenyloxy, a (C2-C6)alkynyloxy, a (C3-C6)cycloalkyloxy (the (C3-C6)cycloalkyloxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C2-C6)alkenylsulfonylamino (the amino moiety is optionally substituted with $R^6$), a (C2-C6)alkynylsulfonylamino (the amino moiety is optionally substituted with $R^6$), a (C3-C6)cycloalkylsulfonylamino (the (C3-C6)cycloalkyl moiety is optionally mono-substituted or poly-substituted with $R^8$, and the amino moiety is optionally substituted with $R^6$), a (C6-C12)arylsulfonylamino (the (C6-C12)aryl moiety is optionally mono-substituted or poly-substituted with $R^5$, and the amino moiety is optionally substituted with $R^6$), a 3- to 6-membered heterocyclylsulfonylamino (the 3- to 6-membered heterocyclyl moiety is optionally mono-substituted or poly-substituted with $R^5$, and the amino moiety is optionally substituted with $R^6$), a 5- to 12-membered heteroarylsulfonylamino (the 5- to 12-membered heteroaryl moiety is optionally mono-substituted or poly-substituted with $R^5$, and the amino moiety is optionally substituted with $R^6$), a (C1-C6)alkylaminosulfonyl (the (C1-C6)alkyl moiety is optionally mono-substituted or poly-substituted with $R^8$, and the amino moiety

is optionally substituted with $R^6$), a (C2-C6)alkenylaminosulfonyl (the amino moiety is optionally substituted with $R^6$), a (C2-C6)alkynylaminosulfonyl (the amino moiety is optionally substituted with $R^6$), a (C3-C6)cycloalkylaminosulfonyl (the (C3-C6)cycloalkyl moiety is optionally mono-substituted or poly-substituted with $R^8$, and the amino moiety is optionally substituted with $R^6$), a (C6-C12)arylaminosulfonyl (the (C6-C12)aryl moiety is optionally mono-substituted or poly-substituted with $R^5$, and the amino moiety is optionally substituted with $R^6$), a 3- to 6-membered heterocyclylaminosulfonyl (the 3- to 6-membered heterocyclyl moiety is optionally mono-substituted or poly-substituted with $R^5$, and the amino moiety is optionally substituted with $R^6$), a 5- to 12-membered heteroarylaminosulfonyl (the 5- to 12-membered heteroaryl moiety is optionally mono-substituted or poly-substituted with $R^5$, and the amino moiety is optionally substituted with $R^6$), a (C1-C6)alkylcarbonylthio, a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C1-C6)haloalkylsulfinyl, a (C1-C6)haloalkylsulfonyl, a (C2-C6)alkenylthio, a (C2-C6)alkenylsulfinyl, a (C2-C6)alkenylsulfonyl, a (C2-C6)alkynylthio, a (C2-C6)alkynylsulfinyl, a (C2-C6)alkynylsulfonyl, a (C3-C6)cycloalkylthio (the (C3-C6)cycloalkylthio is optionally mono-substituted or poly-substituted with $R^8$), a (C3-C6)cycloalkylsulfinyl (the (C3-C6)cycloalkylsulfinyl is optionally mono-substituted or poly-substituted with $R^8$), a (C3-C6)cycloalkylsulfonyl (the (C3-C6)cycloalkylsulfonyl is optionally mono-substituted or poly-substituted with $R^8$), a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C6-C12)arylsulfinyl (the (C6-C12)arylsulfinyl is optionally mono-substituted or poly-substituted with $R^5$), a (C6-C12)arylsulfonyl (the (C6-C12)arylsulfonyl is optionally mono-substituted or poly-substituted with $R^5$), a (C6-C12)arylamino (the (C6-C12)aryl moiety is optionally mono-substituted or poly-substituted with $R^5$, and the amino moiety is optionally substituted with $R^6$), a 3- to 6-membered heterocyclyloxy (the 3- to 6-membered heterocyclyloxy is optionally mono-substituted or poly-substituted with $R^5$), a 3- to 6-membered heterocyclylthio (the 3- to 6-membered heterocyclylthio is optionally mono-substituted or poly-substituted with $R^5$), a 3- to 6-membered heterocyclylsulfinyl (the 3- to 6-membered heterocyclylsulfinyl is optionally mono-substituted or poly-substituted with $R^5$), a 3- to 6-membered heterocyclylsulfonyl (the 3- to 6-membered heterocyclylsulfonyl is optionally mono-substituted or poly-substituted with $R^5$), a 3- to 6-membered heterocyclylamino (the 3- to 6-membered heterocyclyl moiety is optionally mono-substituted or poly-substituted with $R^5$, and the amino moiety is optionally substituted with $R^6$), a 5- to 12-membered heteroaryloxy (the 5- to 12-membered heteroaryloxy is optionally mono-substituted or poly-substituted with $R^5$), a 5- to 12-membered heteroarylthio (the 5- to 12-membered heteroarylthio is optionally mono-substituted or poly-substituted with $R^5$), a 5- to 12-membered heteroarylsulfinyl (the 5- to 12-membered heteroarylsulfinyl is optionally mono-substituted or poly-substituted with $R^5$), a 5- to 12-membered heteroarylsulfonyl (the 5- to 12-membered heteroarylsulfonyl is optionally mono-substituted or poly-substituted with $R^5$), a 5- to 12-membered heteroarylamino (the 5- to 12-membered heteroaryl moiety is optionally mono-substituted or poly-substituted with $R^5$, and the amino moiety is optionally substituted with $R^6$), a hydroxyimino (C1-C6)alkyl, a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C2-C6)alkynyloxyimino (C1-C6)alkyl, a (C3-C6)cycloalkyloxyimino (C1-C6)alkyl (the (C3-C6)cycloalkyl moiety is optionally mono-substituted or poly-substituted with $R^8$), a (C6-C12)aryloxyimino (C1-C6)alkyl (the (C6-C12)aryl moiety is optionally mono-substituted or poly-substituted with $R^5$), a 3- to 6-membered heterocyclyloxyimino (C1-C6)alkyl (the 3- to 6-membered heterocyclyl moiety is optionally mono-substituted or poly-substituted with $R^5$), a 5- to 12-membered heteroaryloxyimino (C1-C6)alkyl (the 5- to 12-membered heteroaryl moiety is optionally mono-substituted or poly-substituted with $R^5$), a (C1-C6)alkoxycarbonyloxy (the (C1-C6)alkoxycarbonyloxy is optionally mono-substituted or poly-substituted with $R^8$), a (C2-C6)alkenyloxycarbonyloxy, a (C2-C6)alkenyloxycarbonyloxy, a (C3-C6)cycloalkyloxycarbonyloxy (the (C3-C6)cycloalkyloxycarbonyloxy is optionally mono-substituted or poly-substituted with $R^8$), a (C6-C12)aryloxycarbonyloxy (the(C6-C12)aryloxycarbonyloxy is optionally mono-substituted or poly-substituted with $R^5$), a 3- to 6-membered heterocyclyloxycarbonyloxy (the 3- to 6-membered heterocyclyloxycarbonyloxy is optionally mono-substituted or poly-substituted with $R^5$), a 5- to 12-membered heteroaryloxycarbonyloxy (the 5- to 12-membered heteroaryloxycarbonyloxy is optionally mono-substituted or poly-substituted with $R^5$), a (C1-C6)alkylaminocarbonylamino (the (C1-C6)alkyl moiety is optionally mono-substituted or poly-substituted with $R^8$, and the amino moiety is optionally substituted with $R^6$), a (C2-C6)alkenylaminocarbonylamino (the amino moiety is optionally substituted with $R^6$), a (C2-C6)alkynylaminocarbonylamino (the amino moiety is optionally substituted with $R^6$), a (C3-C6)cycloalkylaminocarbonylamino (the (C3-C6)cycloalkyl moiety is optionally mono-substituted or poly-substituted with $R^8$, and the amino moiety is optionally substituted with $R^6$), a (C6-C12)arylaminocarbonylamino (the (C6-C12)aryl moiety is optionally mono-substituted or poly-substituted with $R^5$, and the amino moiety is optionally substituted with $R^6$), a 3- to 6-membered heterocyclylaminocarbonylamino (the 3- to 6-membered heterocyclyl moiety is optionally mono-substituted or poly-substituted with $R^5$, and the amino moiety is optionally substituted with $R^6$), a 5- to 12-membered heteroarylaminocarbonylamino (the 5- to 12-membered heteroaryl moiety is optionally mono-substituted or poly-substituted with $R^5$, and the amino moiety is optionally

substituted with $R^6$), a hydrazinocarbonyl (the hydrazino moiety is optionally substituted with $R^6$), or a pentafluorosulfanyl group.

**28.** The amidoxime derivative of formula [III] or the agriculturally acceptable salt thereof according to claim 27, wherein in the gormula [III], $R^2$ represents a hydrogen atom, a (C1-C6)alkyl (the (C1-C6)alkyl is optionally mono-substituted or poly-substituted with $R^4$), a (C2-C6)alkenyl (the (C2-C6)alkenyl is optionally mono-substituted or poly-substituted with $R^4$), a (C2-C6)alkynyl (the (C2-C6)alkynyl is optionally mono-substituted or poly-substituted with $R^4$), a (C6-C12)aryl (the (C6-C12)aryl is optionally mono-substituted or poly-substituted with $R^5$), or a 3- to 6-membered heterocyclyl (the 3- to 6-membered heterocyclyl is optionally mono-substituted or poly-substituted with $R^5$),

$R^4$ each independently represent a halogen atom, a cyano, a thiol, a hydroxy, a tri((C1-C6)alkyl)silyl, a (C1-C6)alkylamino (the (C1-C6)alkyl moiety is optionally mono-substituted or poly-substituted with $R^8$, and the amino moiety is optionally substituted with $R^6$), a formyl, a (C1-C6)alkylcarbonyl (the (C1-C6)alkylcarbonyl is optionally mono-substituted or poly-substituted with $R^8$), a (C3-C6)cycloalkylcarbonyl (the (C3-C6)cycloalkylcarbonyl is optionally mono-substituted or poly-substituted with $R^8$), a (C6-C12)arylcarbonyl (the (C6-C12)arylcarbonyl is optionally mono-substituted or poly-substituted with $R^5$), a 3- to 6-membered heterocyclylcarbonyl (the 3- to 6-membered heterocyclylcarbonyl is optionally mono-substituted or poly-substituted with $R^5$), a 5- to 12-membered heteroarylcarbonyl (the 5- to 12-membered heteroarylcarbonyl is optionally mono-substituted or poly-substituted with $R^5$), a carboxyl, a (C1-C6)alkoxycarbonyl (the (C1-C6)alkoxycarbonyl is optionally mono-substituted or poly-substituted with $R^8$), a (C2-C6)alkenyloxycarbonyl, a (C2-C6)alkynyloxycarbonyl, a (C3-C6)cycloalkyloxycarbonyl (the (C3-C6)cycloalkyloxycarbonyl is optionally mono-substituted or poly-substituted with $R^8$), a (C6-C12)aryloxycarbonyl (the (C6-C12)aryloxycarbonyl is optionally mono-substituted or poly-substituted with $R^5$), a 3- to 6-membered heterocyclyloxycarbonyl (the 3- to 6-membered heterocyclyloxycarbonyl is optionally mono-substituted or poly-substituted with $R^5$), a 5- to 12-membered heteroaryloxycarbonyl (the 5- to 12-membered heteroaryloxycarbonyl is optionally mono-substituted or poly-substituted with $R^5$), a carbamoyl, a (C1-C6)alkylaminocarbonyl (the (C1-C6)alkyl moiety is optionally mono-substituted or poly-substituted with $R^8$, and the amino moiety is optionally substituted with $R^6$), a (C2-C6)alkenylaminocarbonyl (the amino moiety is optionally substituted with $R^6$), a (C2-C6)alkynylaminocarbonyl (the amino moiety is optionally substituted with $R^6$), a (C3-C6)cycloalkylaminocarbonyl (the (C3-C6)cycloalkyl moiety is optionally mono-substituted or poly-substituted with $R^8$, and the amino moiety is optionally substituted with $R^6$), a (C6-C12)arylaminocarbonyl (the (C6-C12)aryl moiety is optionally mono-substituted or poly-substituted with $R^5$, and the amino moiety is optionally substituted with $R^6$), a 5- to 12-membered heteroarylaminocarbonyl (the 5- to 12-membered heteroaryl moiety is optionally mono-substituted or poly-substituted with $R^5$, and the amino moiety is optionally substituted with $R^6$), a (C1-C6)alkoxycarbonylamino (the amino moiety is optionally substituted with $R^6$), a (C1-C6)alkylcarbonylamino (the (C1-C6)alkyl moiety is optionally mono-substituted or poly-substituted with $R^8$, and the amino moiety is optionally substituted with $R^6$), a (C1-C6)alkylcarbonyloxy (the (C1-C6)alkylcarbonyloxy is optionally mono-substituted or poly-substituted with $R^8$), a (C6-C12)arylcarbonyloxy (the (C6-C12)arylcarbonyloxy is optionally mono-substituted or poly-substituted with $R^5$), a (C1-C6)alkoxy (the (C1-C6)alkoxy is optionally mono-substituted or poly-substituted with $R^8$), a (C1-C6)alkylsulfonylamino (the (C1-C6)alkyl moiety is optionally mono-substituted or poly-substituted with $R^8$, and the amino moiety is optionally substituted with $R^6$), a (C1-C6)alkylthio (the (C1-C6)alkylthio is optionally mono-substituted or poly-substituted with $R^8$), a (C1-C6)alkylsulfinyl (the (C1-C6)alkylsulfinyl is optionally mono-substituted or poly-substituted with $R^8$), a (C1-C6)alkylsulfonyl (the (C1-C6)alkylsulfonyl is optionally mono-substituted or poly-substituted with $R^8$), a (C1-C6)haloalkylthio, a (C6-C12)aryloxy (the (C6-C12)aryloxy is optionally mono-substituted or poly-substituted with $R^5$), a (C6-C12)arylthio (the (C6-C12)arylthio is optionally mono-substituted or poly-substituted with $R^5$), a (C1-C6)alkoxyimino (C1-C6)alkyl (the (C1-C6)alkoxy moiety is optionally mono-substituted or poly-substituted with $R^8$), a (C2-C6)alkenyloxyimino (C1-C6)alkyl, a (C1-C6)alkoxycarbonyloxy (the (C1-C6)alkoxycarbonyloxy is optionally mono-substituted or poly-substituted with $R^8$), a hydrazinocarbonyl (the hydrazino moiety is optionally substituted with $R^6$), or a pentafluorosulfanyl,
$R^5$ each independently represent a hydrogen atom, a halogen atom, a cyano, a nitro, a formyl, an oxo, a (C1-C6)alkyl, a (C1-C6)haloalkyl, a (C1-C6)alkoxy, a (C1-C6)haloalkoxy, a (C1-C6)alkoxy (C1-C6)alkyl, or a (C1-C6)alkoxycarbonyl, and
$R^6$ each independently represent a hydrogen atom, a (C1-C6)alkyl, a (C1-C6)haloalkylcarbonyl, or a (C1-C6)alkoxycarbonyl.

## INTERNATIONAL SEARCH REPORT

International application No.
**PCT/JP2022/027824**

**A. CLASSIFICATION OF SUBJECT MATTER**

***C07D 271/06***(2006.01)i; ***A01N 43/836***(2006.01)i; ***A01P 3/00***(2006.01)i; ***C07C 259/18***(2006.01)i; ***C07D 413/04***(2006.01)i; ***C07D 413/12***(2006.01)i
FI: C07D271/06 CSP; A01N43/836; A01P3/00; C07C259/18; C07D413/04; C07D413/12

According to International Patent Classification (IPC) or to both national classification and IPC

**B. FIELDS SEARCHED**

Minimum documentation searched (classification system followed by classification symbols)

C07D271/06; A01N43/836; A01P3/00; C07C259/18; C07D413/04; C07D413/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2017/081309 A1 (BASF SE) 18 May 2017 (2017-05-18) claims, table A, no. I-1, I-93, I-185, I-277, I-369, I-461, I-553, I-645 | 1-6, 8-9, 11-18 |
| Y | claims, table A, no. I-1, I-93, I-185, I-277, I-369, I-461, I-553, I-645, p. 31, line 2 to p. 33, line 2 | 7, 10, 19-28 |
| Y | JP 2019-151553 A (SUMITOMO CHEMICAL CO) 12 September 2019 (2019-09-12) claims, formula (1), R3, R4, R5-R6, definition, examples, test examples | 7, 10, 19-28 |
| Y | WO 2019/131867 A1 (NIHON NOHYAKU CO LTD) 04 July 2019 (2019-07-04) claims, general formula (Ⅰ), Ｘ1, Ｘ2, Ｘ3-Ｘ4, definition, examples, test examples | 7, 10, 19-28 |
| A | WO 2017/110862 A1 (SUMITOMO CHEMICAL CO) 29 June 2017 (2017-06-29) entire text, in particular, compound 7 present invention | 1-28 |
| A | WO 2017/081310 A1 (BASF SE) 18 May 2017 (2017-05-18) entire text | 1-28 |
| A | JP 2019-537553 A (SYNGENTA PARTICIPATIONS AG) 26 December 2019 (2019-12-26) entire text | 1-28 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 October 2022** | **11 October 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/027824**

**C. DOCUMENTS CONSIDERED TO BE RELEVANT**

| Category* | Citation of document, with indication, where appropriate, of the relevant passages | Relevant to claim No. |
|---|---|---|
| A | JP 63-162680 A (CIBA GEIGY AG) 06 July 1988 (1988-07-06)<br>entire text | 1-28 |
| P, X | WO 2022/129190 A1 (BAYER AKTIENGESELLSCHAFT) 23 June 2022 (2022-06-23)<br>table 1, example no. I.004, I.017, I.025, I.026 | 19-21 |
| P, A | CN 113754607 A (SHENYANG UNIVERSITY OF CHEMICAL TECHNOLOGY) 07 December 2021 (2021-12-07)<br>examples 16, 17 | 19-23 |

Form PCT/ISA/210 (second sheet) (January 2015)

**INTERNATIONAL SEARCH REPORT**
**Information on patent family members**

International application No.
**PCT/JP2022/027824**

| Patent document cited in search report | Publication date (day/month/year) | Patent family member(s) | Publication date (day/month/year) |
|---|---|---|---|
| WO 2017/081309 A1 | 18 May 2017 | US 2018/0343864 A1<br>EP 3373735 A1<br>BR 112018009539 A | |
| JP 2019-151553 A | 12 September 2019 | WO 2018/056340 A1<br>claims, formula (１), Ｒ3, Ｒ4, Ｒ5-Ｒ6, definition, examples, test examples<br>AR 109689 A | |
| WO 2019/131867 A1 | 04 July 2019 | US 2021/0037823 A1<br>claims, general formula (Ｉ), X1, X2, X3-X4, definition, examples, test examples<br>EP 3733653 A1<br>CN 111566097 A<br>BR 112020010780 A | |
| WO 2017/110862 A1 | 29 June 2017 | AR 107187 A | |
| WO 2017/081310 A1 | 18 May 2017 | US 2018/0354920 A1<br>entire text<br>EP 3373732 A1<br>BR 112018009566 A | |
| JP 2019-537553 A | 26 December 2019 | US 2020/0022370 A1<br>entire text<br>WO 2018/055135 A1<br>EP 3515908 A1<br>CN 109923112 A<br>BR 112019005656 A | |
| JP 63-162680 A | 06 July 1988 | US 4871753 A<br>entire text<br>EP 276432 A2<br>NZ 222868 A<br>PT 86329 A<br>AU 8246587 A<br>BR 8706756 A<br>DK 650787 A<br>ZA 8709329 A<br>IL 84776 D<br>KR 10-1988-0007500 A<br>ZA 879329 B<br>DK 650787 A0 | |
| WO 2022/129190 A1 | 23 June 2022 | (Family: none) | |
| CN 113754607 A | 07 December 2021 | WO 2021/244244 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 63162680 A
- WO 2017081309 A
- WO 2017081310 A
- WO 2009019656 A
- WO 2013080120 A
- WO 2017222951 A
- WO 2020070610 A
- WO 2020254494 A
- WO 2017110862 A
- EP 3187497 A
- WO 2007111212 A
- WO 2020109391 A
- WO 2020127780 A
- WO 2018228986 A
- WO 2020114934 A
- WO 2014064094 A
- WO 2015162164 A
- WO 2015059262 A
- WO 2018015476 A


### Non-patent literature cited in the description


- *European Journal of Organic Chemistry,* 2019, vol. 28, 4, , 539
- *European Journal of Organic Chemistry,* 2015, vol. 34, 7, , 472
- **PETER G.M. WUTS.** GREEN'S PROTECTIVE GROUPS in Organic Synthesis. John Wiley and Sons, 2014
- *CHEMICAL ABSTRACTS,* 2435594-93-5
- *CHEMICAL ABSTRACTS,* 2446124-68-9
- *CHEMICAL ABSTRACTS,* 2435603-06-6
- *CHEMICAL ABSTRACTS,* 2435600-09-0
- *CHEMICAL ABSTRACTS,* 2435603-15-7
- *CHEMICAL ABSTRACTS,* 2446123-98-2
- *CHEMICAL ABSTRACTS,* 2266183-40-6
- *CHEMICAL ABSTRACTS,* 1606999-43-2
- *CHEMICAL ABSTRACTS,* 2266190-06-9
- *CHEMICAL ABSTRACTS,* 2266164-36-5
- *CHEMICAL ABSTRACTS,* 2266170-31-2
- *CHEMICAL ABSTRACTS,* 1708087-22-2
- *CHEMICAL ABSTRACTS,* 1820807-75-7
- *CHEMICAL ABSTRACTS,* 1606999-21-6
- *CHEMICAL ABSTRACTS,* 2266292-43-5
- *CHEMICAL ABSTRACTS,* 1607001-97-7
- *CHEMICAL ABSTRACTS,* 1083-55-2